# EUROPEAN PATENT APPLICATION

(11) **EP 2 302 036 A2**
(43) Date of publication of application: **30.03.2011**
(21) Application number: 10184689.7
(22) Date of filing: 26.05.2006
(51) Int. Cl.: C12N 5/073

(54) **Amniotic fluid derived cells**

(30) Priority: 27.05.2005 US 685607 P; 27.03.2006 US 743821 P
(62) Divisional of application: 06252751.0
(71) Applicant: Lifescan, Inc., Milpitas, CA 95035 (US)
(72) Inventor: Rezania, Alireza, Hillsborough, NJ 08844 (US); Xu, Jean, Hillsborough, NJ 08844 (US)
(74) Representative: Goodfellow, Hugh Robin

(57) **Abstract**

This invention relates to an expandable population of amniotic fluid-derived cells that can be differentiated into a β-cell lineage. This invention also provides methods for isolating and expanding such amniotic fluid-derived cells, as well as related methods and compositions for utilizing such cells in the therapeutic treatment of diabetes.

## Description

*Related* US application data: Provisional application No 60/685,607, filed 27/05/2005 and Provisional application No 60/743,821, filed 27/03/2006.

### FIELD OF THE INVENTION

This invention relates to an expandable population of amniotic fluid-derived cells that can be differentiated into a β-cell lineage. This invention also provides methods for isolating and expanding such amniotic fluid-derived cells, as well as related methods and compositions for utilizing such cells in the therapeutic treatment of diabetes.

### BACKGROUND

Loss of organ function can result from congenital defects, injury or disease. One example of a disease causing loss of organ function is diabetes mellitus, or diabetes. Most cases of diabetes fall into two clinical types: Type 1, also known as juvenile-onset diabetes, or insulin dependent diabetes mellitus (IDDM), and Type 2, also known as adult-onset diabetes. Each type has a different prognosis, treatment, and cause. Both types are characterized by the patient's inability to regulate their blood glucose levels. As a consequence, blood glucose levels rise to high values because glucose cannot enter cells to meet metabolic demands. This inability to properly metabolize blood sugar causes a complex series of early and late-stage symptomologies, beginning with, for example, hyperglycemia, abnormal hunger, thirst, polyuria, and glycouria, and then escalating to, for example, neuropathy, macro-vascular disease, and micro-vascular disease.

A common method of treatment of Type 1 diabetes involves the exogenous administration of insulin, typically by injection with either a syringe or a pump. This method does not completely normalize blood glucose levels and is often associated with an increased risk of hypoglycemia. More effective glycemic control can be achieved if the function of the pancreas can be restored or rejuvenated via transplantation or cell-based therapies.

There are many transplantation therapies currently used to treat diabetes: One such treatment involves transplanting isolated islets of Langerhans into the diabetic patient. One of the main hurdles to human islet transplantation has been the lack of sufficient number of islets to treat the large number of diabetic patients. One possible solution to the shortage of islets is the generation of islets from alternate cellular sources.

It has been documented that progenitor cells derived from adult tissues are capable of differentiation into a pancreatic β-celt phenotype. See, for example, WO2004/087885 A2, Hess et al. (Nature Biotechnology 21, 763 - 770, 2003), and Ianus et al. (J. Clin. Invest. 111: 843-850, 2003), which report the capacity of adult bone marrow-derived cells (mesenchymal and hematopoetic cells) to differentiate into cells having characteristics of a pancreatic β-cell *in vitro,* or secrete trophic factors that help regenerate a damaged pancreas *in vivo*.

Among other sources of progenitor cells that can be differentiated into pancreatic cells include rodent liver oval stem cells (WO03/033697) and post-partum placenta (U.S. Published Application 2004/0161419 A1).

The endocrine cells of the islets of Langerhans, including β-cells, are constantly turning over by processes of apoptosis and the proliferation of new islet cells (neogenesis). As such, the pancreas is thought to be a source of progenitor cells that are capable of differentiating into pancreatic hormone producing cells. There are three distinct tissue types, isolated from a pancreas, that are a potential source of pancreatic progenitor cells: an islet rich fraction, a ductal cell rich fraction, and an acinar cell rich fraction.

Isolation of progenitor cells or partially differentiated cells from crude pancreatic tissue extracts may be achieved using antibodies raised against cell surface markers. For example, U.S. Published Application 2004/0241761 discloses isolation of murine cells that expressed ErbB2, ErbB3, ErbB4, Msx-2, PDX-1 and insulin.

Gershengom et al. (Science 306: 2261-2264, 2004) teach the production of proliferating cells that were able to form islet-like cell aggregates. The cells were derived from a heterogeneous population of adherent cells that emerged from the culture of isolated human pancreatic islets *in vitro.* The isolated islets of Langerhans were initially seeded onto tissue culture dishes and cultured in medium containing 10% serum. Fibroblast-like cells were observed to migrate out of the cultured islets and form a monolayer. These cells expressed Nestin, smooth muscle actin and vimentin.

Pancreatic progenitor cells may also arise from the culture of pancreatic islet and ductal tissue that has been dissociated into single cells, as disclosed by Seaberg et al. (Nature Biotechnology 22: 1115 - 1124, 2004). The murine progenitor cells disclosed by Seaberg *et al.* expressed Nestin during proliferation.

U.S. Published Application 2003/0082155 discloses methods to isolate and identify a population of cells from the islets of Langerhans of human pancreas, which have the functional and molecular characteristics of stem cells. In particular, these cells were characterized by Nestin-positive staining, Nestin gene expression, GLP-1 R-positive staining, GLP- I R gene expression, ABCG2 positive staining, ABCG2 gene expression, Oct3/4 positive staining, Oct3/4 gene expression, latrophilin (type 2) positive staining, latrophilin (type 2) gene expression, Hes-1 positive staining, Hes-1 gene expression, Integrin subunits α6 and β1 positive staining, Integrin subunits α6 and β1 gene expression, c-kit positive staining, c-kit gene expression, MDR-1 positive staining, MDR-1 gene expression, SST-R, 2, 3, 4 positive staining, SST-R, 2, 3, 4 gene expression, SUR-1 positive staining, SUR-1 gene expression, Kir 6.2 positive staining, Kir 6.2 gene expression, CD34 negative staining, CD45 negative staining, CD133 negative staining, MHC class I negative staining, MHC class II negative staining, cytokeratin-19 negative staining, long-term proliferation in culture, and the ability to differentiate into pseudo-islets in culture.

In another approach, as disclosed in U.S. Patent 5,834,308, U.S. Patent 6,001,647 and U.S. Patent 6,703,017, crude preparations of islet cultures from NOD mice may be used to establish epithelial-like cultures, which can be maintained in growing cultures for greater than 1 year and which appear to demonstrate the ability to differentiate into islet-like clusters, capable of secreting insulin.

Islet-like structures may be generated from fractions of digested human pancreata enriched for ductal tissue, as disclosed in Bonner-Weir et al. (Proc Nat Acad Sci 97: 7999-8004, 2000) and U.S. Patent 6,815,203 B1. Islet-like clusters disclosed in these publications stained positive for cytokeratin-19 and showed immunoreactivity for insulin.

WO2004/011621 discloses the generation of insulin negative adherent cells from human pancreatic ductal fragments.

WO03/102134 discloses the generation of an epithelial cell positive for cytokeratin-19 from an acinar fraction of a human pancreatic digest. The cells generated are capable of limited expansion and differentiate into an insulin-producing cell in the presence of an induction media.

U.S. Published Application 2004/015805 A1 reports that a subset of human pancreatic stem cells may be isolated using ligands to the cell surface marker CD56 (also known as NCAM). These cells can differentiate into insulin producing cells and insulin producing aggregates.

It has been documented that progenitor cells, derived from fetal or embryonic tissues, have the potential to differentiate into a pancreatic hormone-producing cell. See, for example, U.S. Patent 6,436,704, WO03/062405, WO02/092756 and EP 0 363 125 A2, which report the potential of human fetal and embryonic derived cells to differentiate into a β-cell lineage.

Human Embryonic Stem cells (hES) are derived from the inner cell mass of the blastocyst, the earliest stage of embryonic development of the fertilized egg. The blastocyst is a preimplantation stage of the embryo, a stage before the embryo would implant in the uterine wall. When cultured on an inactivated feeder layer of cells according to conditions described by Thompson and colleagues (Thomson, et al. (Proc. Natl. Acad. Sci. U.S.A. 92: 7844-7848, 1995); Thomson, et al. (Science 282:1145-1147, 1998), Marshall, et al., (Methods Mol. Biol. 158:11-18, 2001), the inner layer cells of the blastocyst may be grown *in vitro* indefinitely in an undifferentiated state. Properly propagated hES cells have unlimited potential to double while maintaining their pluiripotency; namely their capacity of differentiating into the three layers of the embryo, Ectoderm (Ec), Mesoderm (Me) and Endoderm (En). When grown as pluripotent hES, the cells maintain a euploid karyotype and are not prone to senescence.

Human embryonic stem cells display a distinct group of cell surface antigens, SSEA-3, SSEA-4, TRA-2-54 (alkaline phosphatase), TRA-1-60 and TRA-1-81, in addition to expressing specific transcription factors OCT-4, NANOG, SOX-2, FGF-4 and REX-1 (Henderson, et al., (Stem Cells 20:329-337, 2002), Draper, et al., (J. Anat. 200:249-258, 2002), Mitsui et al., (Cell 113:631-642, 2003), Chambers et al., (Cell 113:643-655, 2003).

It is important to note from these publications, however, that human embryonic cells often require a feeder layer for expansion and maintenance of pluripotency or combination of a complex extracellular matrix, such as, for example, MATRIGEL™, plus conditioned media. These conditions do not allow the facile scale up of cells and an eventual cell therapy for treating diabetes.

Researchers have found that non-embryonic types of stem cells ("adult stem cells") are not as capable of differentiating into many different tissue types, as are embryonic stem cells, so embryonic stem cells still have many advantages over the use of adult stem cells. However, one obstacle with the isolation of embryonic stem cells is that the cells are derived from embryos at the "blastocyst" stage. Human embryonic stem cell research is encumbered by an emotionally charged political and ethics debate and is likely to remain so for years to come.

Additionally, human embryonic stem cells (hES) have been found to be tumorigenic when injected into immunologically impaired animals, i.e. in the context of post-natal tissues, whereas adult stem cells are not. The tumorigenic attributes of hES cells are not frequently addressed, though this issue may burden their use in replacement cell therapy in the future. The political, moral and ethical issues around hES cells and their tumorigenic properties, as well as the perceived difficulties of expanding undifferentiated adult stem cells in culture, while maintaining a genetically normal genome, are major barriers in the development of human cell replacement therapy.

Pluripotent or multipotent stem cells have been isolated from chorionic villus, and amniotic fluid. Many amniotic and placental cells share a common origin, namely the inner cell mass of the morula, which gives rise to the embryo itself, the yolk sac, the mesenchymal core of the chorionic villi, the chorion and the amnion (Crane & Cheung, Prenatal Diagnosis 8: 119-129, 1988). Embryonic and fetal cells from all three germ layers have long been identified in the amniotic fluid (Milunsky, Genetic Disorder of the Fetus. New York: Plenum Press, 75-84,1979; Hoehn & Salk, Methods in Cell Biology 26, 11-34, 1982; Gosden, British Medical Bulletin 39, 348-354, 1983; Prusa et al, Human Reproduction 18, 1489-1493, 2003). Thus, amniotic fluid may provide the least invasive access to embryonic-like and fetal-like stem cells.

Amniotic fluid derived cells have been routinely used for detecting chromosomal abnormality of the fetus. Amniotic fluid is typically sampled during the 2nd trimester (16 to 22 weeks of gestation). Previous art clearly demonstrates presence of three subpopulation with distinct cell morphologies: "fibroblastic" (F), "amniotic fluid" (AF) cells, and "epithelial" (E) cells. The F and AF cells rapidly expand whereas the E cells display a much slower growth curve and have poor clonal efficiency.

For example, PCT application W02003/042405 discloses isolation of c-Kit positive stem cells from chorionic villus, amniotic fluid and placenta (Cell 1, **Table I).**

In another example, U.S. Published Application 2005/0054093 discloses the isolation of stem cells from amniotic fluid. These cells express stage-specific embryonic antigen 3 (SSEA3), stage-specific embryonic antigen 4 (SSEA4), Tral-60, Tral-81, Tra2-54, Oct-4, HLA class I, CD13, CD44 CD49b and CD105 (Cell 2, **Table I).**

In another example, fetal cells have been isolated from amniotic fluid (in't Anker et al, Blood 102, 1548-1549, 2003). The cells disclosed were positive for expression of the following markers: CD44, CD73, CD90, CD105, CD106, HLA-A,B, & C. The cells were negative for expression of the following markers: c-Kit (CD 117), CD11, CD31, CD34, CD45 and HLA-D (Cell 3, **Table I).**

A population of mesenchymal stem cells isolated from amniotic fluid has also been reported in a publication to Tsai *et al* (Tsai et al, Human Reproduction 19, 1450-1456, 2004). The cells disclosed were positive for expression of the following markers: CD29, CD44, CD73, CD90, HLA-A,B, & C. The cells were also positive for the embryonic transcription factor Oct-4. The cells were negative for expression of the following markers: c-Kit (CD117), CD34 and HLA-D (Cell 4, **Table I).**

Although recent publications and patents have suggested that within the fibroblastic, amniotic fluid, or epithelial subpopulations there exists a cell population that display some characteristics of human embryonic cells, such as expression of surface markers SSEA3 and -4, expression of transcription factor Oct-4, strong expansion potential, and differentiation into multiple cell types; none of the previously published art has demonstrated the existence of a subpopulation of the cells that display expression of key early endodermal markers, such as HNF-1 beta, HNF-3 beta, SOX-17, and GATA-6, while maintaining expression of ES markers SSEA-4.

Co expression of HNF-1 beta, HNF-3 beta (also known as FOXa2), SOX-17, and GATA-6 is regarded as the key step to define the formation of definitive endoderm during gastrulation. Thus, expression of these markers may be key in the generation of a pancreatic β-cell population, or a population of pancreatic hormone-producing cells, or a gut hormone-producing cell from an amniotic fluid-derived cell.

Therefore, there still remains a significant need to develop culture conditions for establishing amniotic fluid-derived cell lines that can be expanded to address the current clinical needs, while retaining the potential to differentiate into definitive endoderm, or a population of pancreatic hormone-producing cells, or a gut hormone-producing cell, or a β-cell lineage.

### SUMMARY

In one embodiment, the present invention provides a method for isolating mammalian amniotic fluid-derived cells. According to the present invention, amniotic fluid-derived cells are obtained from amniotic fluid samples of about 14 to about 23 weeks gestation. Alternatively, the amniotic fluid-derived cells are obtained from amniotic fluid samples of about 23 to about 40 weeks gestation.

In one embodiment, the cultures are left undisturbed for at least 5 to 10 days under hypoxic conditions (3% O₂). Alternatively, the cultures are left undisturbed for at least 5 to 10 days under normoxic conditions (approximately 20% O₂).

In an alternate embodiment, amniotic fluid-derived cells are obtained from amniotic fluid samples from the second trimester of gestation. Alternatively, the amniotic fluid-derived cells are obtained from amniotic fluid samples from the third trimester of gestation.

In one embodiment, the cultured amniotic fluid-derived cells are isolated as single cells, and clonally expanded.

The amniotic fluid-derived isolated according to the methods of the present invention can be contacted, for example, with an agent (such as an antibody) that specifically recognizes a protein marker expressed by amniotic fluid cells, to identify and select amniotic fluid-derived cells, thereby obtaining a substantially pure population of amniotic fluid-derived cells, i.e., wherein a recognized protein marker is expressed in at least 50% of the cell population.

In one embodiment, the resulting amniotic fluid-derived cell population is substantially positive for at least one of the following markers: HNF-1 beta, HNF-3 beta, SOX-17, or GATA-6. The amniotic fluid-derived cell population is substantially negative for at least one of the following markers: CD117, Oct-4, or Tra2-54. The amniotic fluid-derived cell population can be expanded for more than 50 population doublings without losing the capacity to express HNF-1 beta, HNF-3 beta, SOX-17, or GATA-6.

In one embodiment, the amniotic fluid-derived cell population is substantially positive for the following markers: SSEA4 and CD44. The amniotic fluid-derived cell population can be expanded for more than 50 population doublings without losing the capacity to express HNF-1 beta, HNF-3 beta, SOX-17, or GATA-6.

In one embodiment, the amniotic fluid-derived cell population isolated according to the methods of the present invention is substantially negative for at least one of the following markers: SOX-17, CD117, Oct-4, or Tra2-54. The amniotic fluid-derived cell population is substantially positive for the following markers: SSEA4 and CD44. The amniotic fluid-derived cell population can be expanded for more than 50 population doublings.

In one embodiment, the amniotic fluid-derived cell population isolated according to the methods of the present invention is substantially negative for cytokeratin and at least one of the following markers: SOX-17, CD117, Oct-4, or Tra2-54. The amniotic fluid-derived cell population is substantially positive for the following markers: SSEA4 and CD44. The amniotic fluid-derived cell population can be expanded for more than 50 population doublings.

In one embodiment, the amniotic fluid-derived cell population isolated according to the methods of the present invention is substantially negative for SOX-17. The amniotic fluid-derived cell population is substantially positive for the following markers: SSEA4 and CD44. The amniotic fluid-derived cell population can be expanded for more than 50 population doublings.

In one embodiment, the amniotic fluid-derived cell population isolated according to the methods of the present invention is substantially negative for the following markers: cytokeratin, and SOX-17. The amniotic fluid-derived cell population is substantially positive for the following markers: SSEA4 and CD44. The amniotic fluid-derived cell population can be expanded for more than 50 population doublings.

In one embodiment, the amniotic fluid-derived cell population isolated according to the methods of the present invention is substantially negative for SOX-17. The amniotic fluid-derived cell population is further negative for at least one of the following markers: CD 117, Oct-4, or Tra2-54. The amniotic fluid-derived cell population is substantially positive for the following markers: SSEA4 and CD44. The amniotic fluid-derived cell population can be expanded for more than 50 population doublings.

In one embodiment, the amniotic fluid-derived cell population isolated according to the methods of the present invention is substantially negative for the following markers: cytokeratin, and SOX-17. The amniotic fluid-derived cell population is further negative for at least one of the following markers: CD 117, Oct-4, or Tra2-54. The amniotic fluid-derived cell population is substantially positive for the following markers: SSEA4 and CD44. The amniotic fluid-derived cell population can be expanded for more than 50 population doubling.

In another embodiment, the present invention provides an isolated pure population of amniotic fluid-derived cells that are substantially negative for at least one of the following markers: CD117, Oct-4, or Tra2-54.

In another embodiment, the present invention provides an isolated pure population of amniotic fluid-derived cells that are substantially negative for at least one of the following markers: SOX-17, CD117, Oct-4, or Tra2-54.

In another embodiment, the present invention provides an isolated pure population of amniotic fluid-derived cells that are substantially negative for SOX-17.

In another embodiment, the present invention provides an isolated pure population of amniotic fluid-derived cells that are substantially negative for SOX-17, and substantially negative for at least one of the following markers: CD117, Oct-4, or Tra2-54.

In one embodiment, the amniotic fluid-derived cells isolated according to the methods of the present invention may also express at least one of the following: Musashi-1 and Hes1.

The amniotic fluid-derived cells isolated and expanded according to the present invention can be induced to differentiate into cells of the β cell lineage under appropriate *in vitro* or *in vivo* conditions. Accordingly, the amniotic fluid-derived cells selected and expanded according to the present invention, as well as the differentiated cells derived from the amniotic fluid-derived cells, are useful for treating Type 1 and 2 diabetes.

The amniotic fluid-derived cells isolated and expanded according to the present invention can be induced to gut hormone-producing cells under appropriate *in vitro* or *in vivo* conditions. In one embodiment, the amniotic fluid-derived cells isolated and expanded according to the present invention can be induced to gut hormone-producing cells under appropriate *in vitro* or *in vivo* conditions and may express insulin in a glucose responsive manner.

### BRIEF DESCRIPTION OF THE DRAWINGS

**Figure 1** shows the isolation and culturing steps used to isolate the amniotic fluid derived cells of the present invention.
**Figure 2** shows three distinct morphologies of cells isolated from an amniotic fluid sample at passage 0. a) AF morphology, b) epithelial morphology, and c) fibroblast morphology.
**Figure 3** depicts the expression of cell surface markers on AF-I cells derived from amniotic fluid. The markers are indicated on panels a-n.
**Figure 4** depicts the expression of cell surface markers on F cells derived from amniotic fluid. The markers are indicated on panels a-l.
**Figure 5** depicts the expression of cell surface markers on E cells derived from amniotic fluid. The markers are indicated on panels a-m.
**Figure 6** depicts immunofluoresence images of the F cells derived from amniotic fluid samples. F cells stained positive for a) vimentin, b) SSEA-4, and c) beta III tubulin.
**Figure 7** depicts immunofluoresence images of the E cells derived from amniotic fluid samples. E cells stained positive for a) vimentin and nestin, b) SSEA-4, c) beta III tubulin, d) pan-cytokeratin, e) smooth muscle actin, and f) cytokeratin 19.
**Figure 8** depicts immunofluoresence images of the AF-I cells derived from amniotic fluid samples. AF-I cells stained positive for a) vimentin and nestin, b) beta III tubulin, c) cytokeratin 19 and HES-1, d) pan-cytokeratin, e) SSEA-4, f) SOX-17 and ZO-1, g) GATA-6, h) HNF-1 beta, i) smooth muscle actin and HES-2.
**Figure 9** shows the expression profile of AF-I, AF-II, and AF-III cells of the present invention.
**Figure 10** depicts the population doubling curve of early passage AF-I cells.

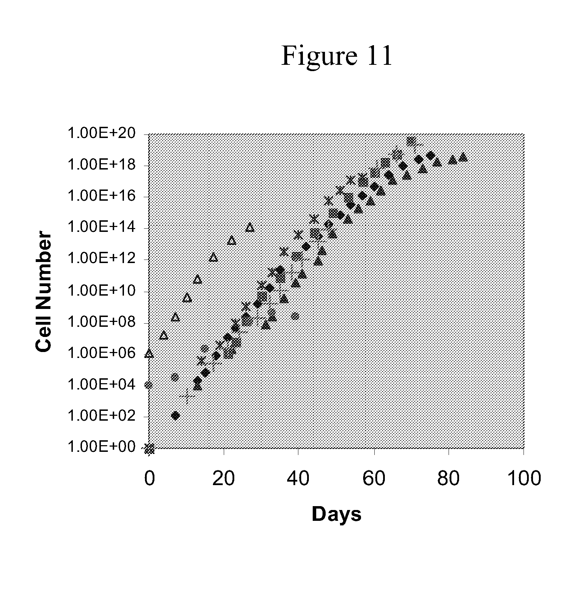

**Figure 11** depicts the expansion potential of AF, F, or E cell derived from different donors. ◆ shows the cell number of amniotic fluid-derived cells with AF-I morphology obtained from amniotic fluid from one donor at 14-23 weeks gestation. Cells were cultured in media number 5 **(Table II).** ▲ shows the cell number of amniotic fluid-derived cells with AF-I morphology obtained from amniotic fluid from a second donor at 14-23 weeks gestation. Cells were cultured in media number 5 **(Table II).** ■ shows the cell number of amniotic fluid-derived cells with F morphology obtained from amniotic fluid from a third donor at 14-23 weeks gestation. Cells were cultured in media number 15 **(Table II).** * shows the cell number of amniotic fluid-derived cells with F morphology obtained from amniotic fluid from a fourth donor at 14-23 weeks gestation.
Cells were cultured in media number 16 **(Table II).** • shows the cell number of amniotic fluid-derived cells with E morphology obtained from amniotic fluid from a donor at 14-23 weeks gestation. Cells were cultured in media number 5. + shows the cell number of amniotic fluid-derived cells with AF-II morphology obtained from amniotic fluid from a second donor at 14-23 weeks gestation. Cells were cultured in media number 5 **(Table II).** △ shows the cell number of amniotic fluid-derived cells with AF-III morphology obtained from amniotic fluid from a second donor at 14-23 weeks gestation. Cells were cultured in media number 5 **(Table II)**

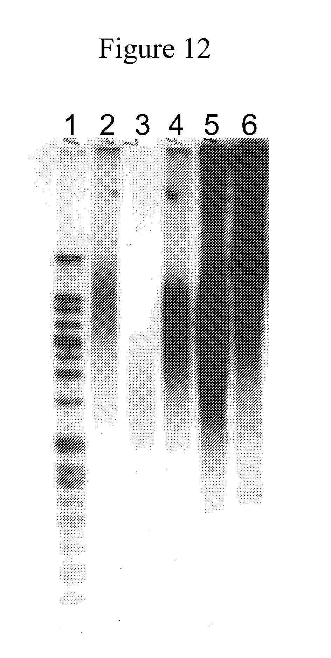

**Figure 12** depicts the telomere length of an AF-I cell line cultured either in AMNIOMAX or DM-LG + 10 % FBS at an intermediate passage level (approximately 40 population doublings). Lane I is the molecular weight ladder, lane 2 is the high telomere length control, lane 3 is the low telomere length control, lane 4 is amniotic fluid-derived cells from a donor at Passage 12, cultured in DMEM-LG + 10% FBS, lane 5 is amniotic fluid-derived cells from the same donor at passage 12, cultured in media #5, and lane 6 is an embryonic carcinoma cell line (NTERA cells) that serves as a positive control.

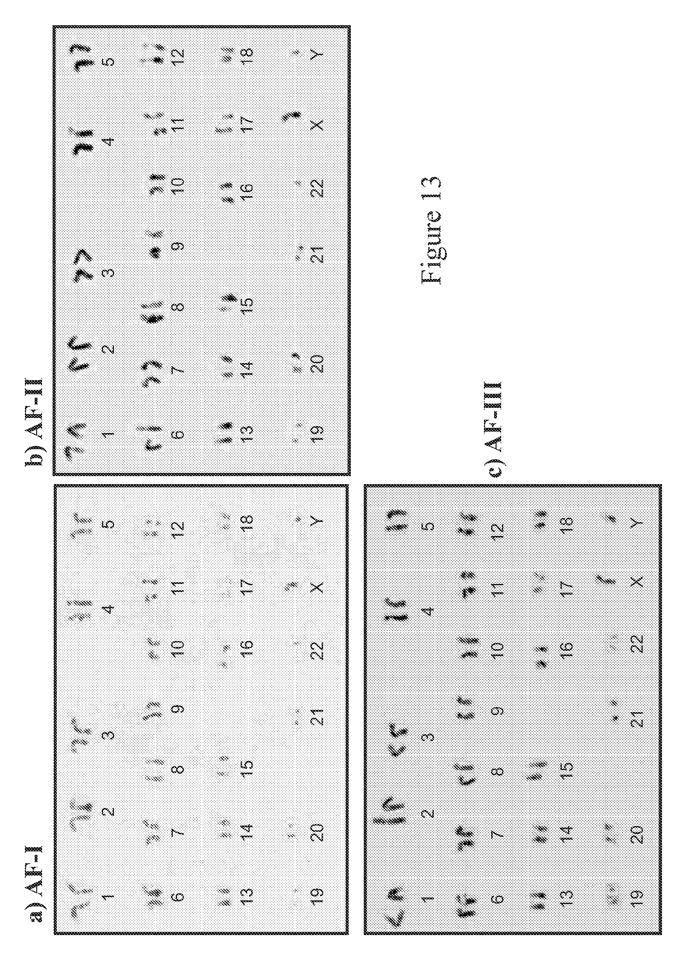

**Figure 13** shows the karyotype of a) AF-I, b) AF-II, and c) AF-III cells cultured at passage 7-9 (approximately 30-35 population doublings).

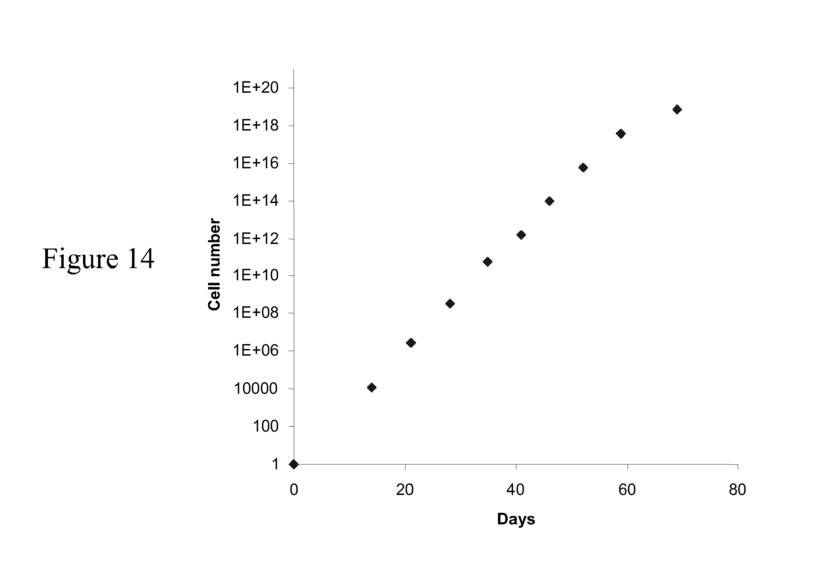

Figure 14 depicts the expansion potential of a single AF derived cell from one donor at term (approximately 38 weeks). Cells were cultured in media number 5 **(Table II).**

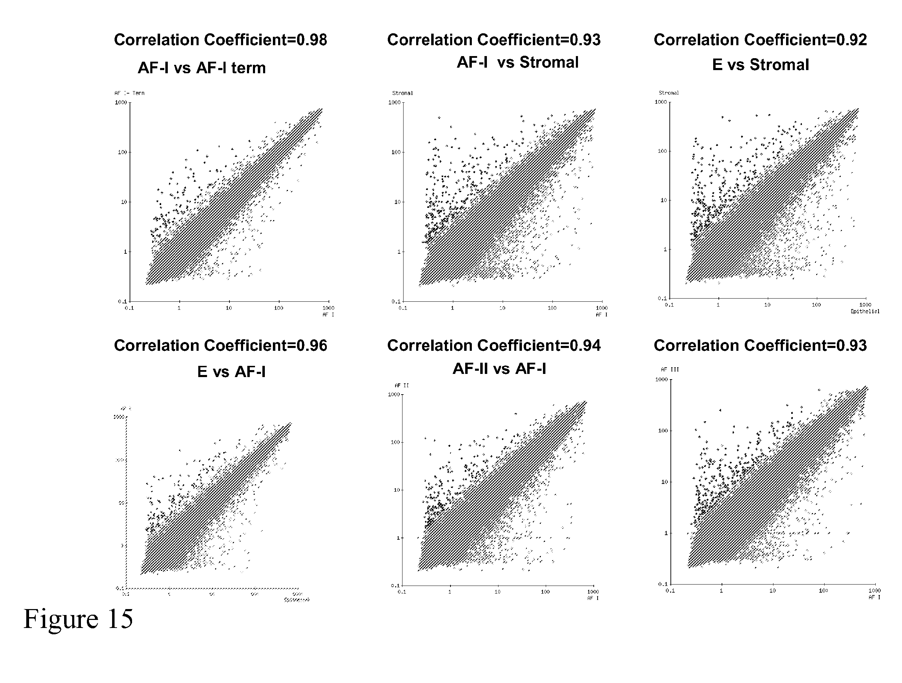

**Figure 15** depicts the scatter plot gene expression profiles between the different amniotic fluid cell types. The Pearson correlation coefficient for each plot is also listed.

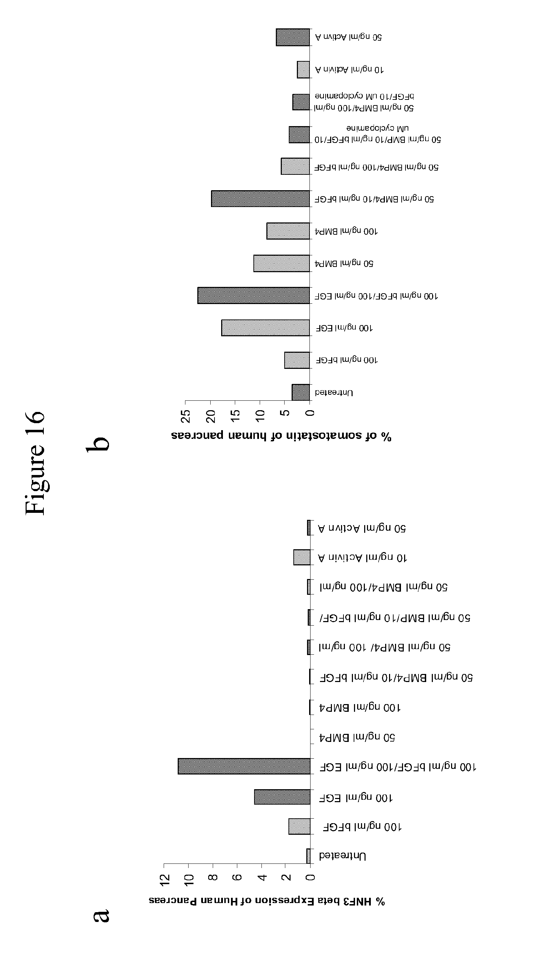

**Figure 16** shows the effects of growth factors on gene expression in amniotic fluid-derived cells. Amniotic fluid-derived cells were obtained from a single donor and cultured for 12 days in conditioned media that was obtained from cultures of PANC-1 cells. The media was supplemented with the growth factors indicated. The levels of expression of HNF-3 beta and somatostatin were determined by real-time PCR. Human pancreas total RNA was included as a calibrator. Panel a shows the changes in HNF-3 beta expression. Panel b shows the changes in somatostatin expression.

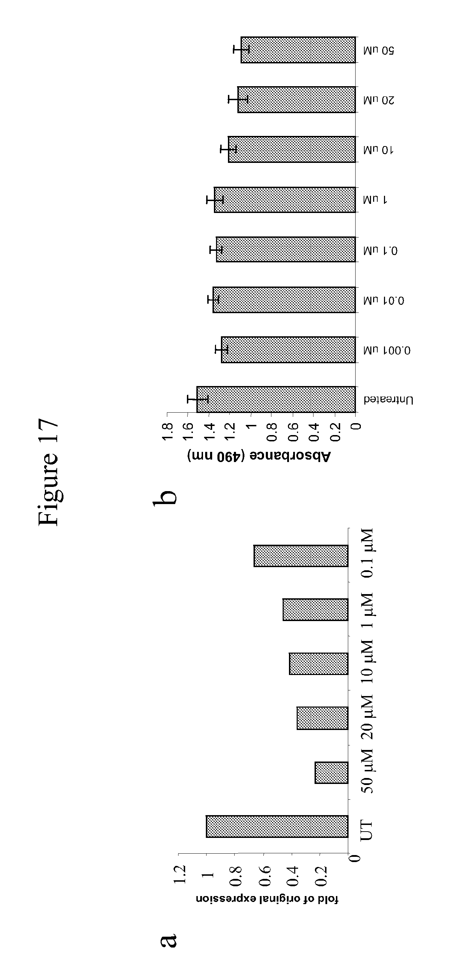

**Figure 17** shows the effects of L685,458 on cultured amniotic fluid-derived cells having the AF morphology. Panel a shows the relative differences in RNA expression of human Hes-1 in cultured AF cells treated with the concentrations of L685,458 indicated. Panel b shows the effects of L685,458 on the viability of the cultured cells following a treatment with L685,458. Cells were treated for three days, at the concentrations indicated. Changes in viability, corresponding to cytotoxicity were detected using an MTS assay, where a decrease in cell viability corresponds to a decrease in A490nm.

### DETAILED DESCRIPTION

For clarity of disclosure, and not by way of limitation, the detailed description of the invention is divided into the following subsections that describe or illustrate certain features, embodiments or applications of the present invention.

The present invention is directed to methods for isolating an amniotic fluid-derived cell population that is highly proliferative, and displays embryonic-like characteristics. Similar cells may also be present in the chorionic villus. Some of the embodiments of the invention disclosed herein describe three morphologically distinct populations of amniotic fluid-derived cells: "fibroblastic" (F), epithelial" (E) cells, and "amniotic fluid" (AF) cells.

### Definitions

"β-cell lineage" refer to cells with positive gene expression for the transcription factor PDX-1 and at least one of the following transcription factors: NGN-3, Nkx2.2, Nkx6.1, NeuroD, Isl-1, HNF-3 beta, MAFA, Pax4, and Pax6. Characteristics of cells of the beta cell lineage are well known to those skilled in the art, and additional characteristics of the beta cell lineage continue to be identified. These transcription factors are well established in prior art for identification of endocrine cells (Nature Reviews Genetics, Vol3, 524-632, 2002).

"Pancreatic islet-like structure" refers to a three-dimensional clusters of cells derived by practicing the methods of the invention, which has the appearance of a pancreatic islet. The cells in a pancreatic islet-like structure express at least the PDX-1 gene and one hormone selected from the list glucagon, somatostatin, or insulin.

The term "hypoxic" refers to oxygen levels less than 20%, preferably less than 10%, and more preferably less than 5% but more than 1%.

The term "normoxia" refers to atmospheric oxygen levels of about 20%.

The term "substantially positive," when used in connection with a population of cells with respect to the expression of certain marker (such as a membrane receptor, cytoplasmic or nuclear protein, or a transcription factor), means that the marker is present or expressed in at least about 50%, alternatively at least about 60%, and alternatively at least about 70%, of the total cell population.

The term "substantially negative," when used in connection with a population of cells with respect to the expression of certain marker (such as a membrane receptor, cytoplasmic or nuclear protein, or a transcription factor), means that the marker is not present or expressed in at least about 70%, alternatively about 80%, alternatively about 90%, of the total cell population.

A "stem cell" as used herein refers to an undifferentiated cell that is capable of extensive propagation either *in vivo* or *ex vivo* and capable of differentiation to other cell types.

A "progenitor cell" refers to a cell that is derived from a stem cell by differentiation and is capable of further differentiation to more mature cell types. Progenitor cells typically have more restricted proliferation capacity as compared to stem cells.

"Expandable population" refers to the ability of an isolated cell population to be propagated through at least 50 or more cell divisions in a cell culture system.

By "undifferentiated cells," when used in connection with cells isolated from a amniotic fluid, are meant a population of amniotic fluid-derived cells that are substantially negative for the expression of PDX- 1, or insulin.

By "differentiated cells," when used in connection with cells isolated from amniotic fluid, are meant a population of amniotic fluid-derived cells that are substantially positive for the expression of PDX-1, or insulin.

"Markers" as used herein, are nucleic acid or polypeptide molecules that are differentially expressed in a cell of interest. In this context, differential expression means an increased level of the marker for a positive marker, and a decreased level for a negative marker. The detectable level of the marker nucleic acid or polypeptide is sufficiently higher or lower in the cells of interest, compared to other cells, such that the cell of interest can be identified and distinguished from other cells, using any of a variety of methods known in the art.

"c-Kit" and "CD1 17" both refer to a cell surface receptor tyrosine kinase having a sequence disclosed in Genbank Accession No. X06182, or a naturally occurring variant sequence thereof (e.g., allelic variant).

"CD9" is also referred to as "Motility-related protein-1 (MRP-1)" and is a transmembrane glycoprotein that has been implicated in cell adhesion, motility, proliferation, and differentiation.

"CD10" is also referred to as "Common Acute Lymphocytic Leukemia Antigen (CALLA)". CD10 is a cell surface enzyme with neutral metalloendopeptidase activity and it is expressed in lymphoblastic, Burkitt's, and follicular germinal center lymphomas and in patients with chronic myelocytic leukemia. It is also expressed on the surface of normal early lymphoid progenitor cells, immature B Cells within adult bone marrow and germinal center B Cells within lymphoid tissue. CD10 is also present on breast myoepithelial cells, bile canaliculi, fibroblasts, brush border of kidney and gut epithelial cells.

"CD44" is also referred to as "Hermes antigen" and is the main cell surface receptor for hyaluronan. This CD is primarily expressed in most cell types, except for tissues/cells such as hepatocytes, some epithelial cells, and cardiac muscle.

"CD49f' is also referred to as "a6 integrin" and "VLA-6," and associates with integrin subunit beta 1 to bind laminin. CD49f is expressed primarily on epithelial cells, trophoblasts, platelets, and monocytes.

"CD73" is also referred to as "ecto-5'-nucleotidase" and is primarily expressed on a subset of-B and T cells, bone marrow stromal cells, various epithelial cells, fibroblasts, and endothelial cells.

"CD90" is also referred to as "Thy-1" and is primarily expressed on hematopoietic stem cells, connective tissue cells, and various fibroblastic and stromal cells.

"SSEA-1" (Stage Specific Embryonic Antigen-1) is a glycolipid surface antigen present on the surface of murine teratocarcinoma stem cells (EC), murine and human embryonic germ cells (EG), and murine embryonic stem cells (ES).

"SSEA-3" (Stage Specific Embryonic Antigen-3) is a glycolipid surface antigen present on the surface of human teratocarcinoma stem cells (EC), human embryonic germ cells (EG), and human embryonic stem cells (ES).

"SSEA-4" (Stage Specific Embryonic Antigen-4) is a glycolipid surface antigen present on the surface of human teratocarcinoma stem cells (EC), human embryonic germ cells (EG), and human embryonic stem cells (ES).

"TRA1-60" is a keratin sulfate related antigen that is expressed on the surface of human teratocarcinoma stem cells (EC), human embryonic germ cells (EG), and human embryonic stem cells (ES).

"TRA1-81" is a keratin sulfate related antigen that is expressed on the surface of human teratocarcinoma stem cells (EC), human embryonic germ cells (EG), and human embryonic stem cells (ES).

"TRA2-49" is an alkaline phosphatase isozyme expressed on the surface of human teratocarcinoma stem cells (EC), and human embryonic stem cells (ES).

"Oct-4" is a member of the POU-domain transcription factor and is widely regarded as a hallmark of pluripotent stem cells. The relationship of Oct-4 to pluripotent stem cells is indicated by its tightly restricted expression to undifferentiated pluripotent stem cells. Upon differentiation to somatic lineages, the expression of Oct-4 disappears rapidly.

"EPCAM" " is also referred to as "Epithelial Cell Adhesion Molecule" is broadly expressed on cells of epithelial origin and epithelial derived tumor cells.

"Rex-1" is a developmentally regulated acidic zinc finger gene (*Zfp-42*). Rex-1 message level is high in embryonic stem cells and reduced upon induction of differentiation. As expected for a stem-cell-specific message, Rex-1 mRNA is present in the inner cell mass (ICM) of blastocyst, polar trophoblast of the blastocyst and later in the ectoplacental cone and extraembryonic ectoderm of the egg cylinder (trophoblast-derived tissues), but its abundance is much reduced in the embryonic ectoderm, which is directly descended from the ICM.

"HNF-1 alpha", "HNF-1 beta" and "HNF-3 beta" belong to the hepatic nuclear factor family of transcription factors, which is characterized by a highly conserved DNA binding domain and two short carboxy-terminal domains.

"GATA-4" and "GATA-6" are members of the GATA transcription factor family. This family of transcription factors are induced by TGF β signaling and contribute to the maintenance of early endoderm markers, Sox 17α and HNF-1 beta, and the later marker HNF-3 beta.

"SOX-17" is a transcription factor, which is implicated in the formation of endoderm during embryogenesis.

By "basic defined cell culture medium" is meant a serum free or serum containing, chemically defined cell growth medium. Such medium includes, but is not limited to, Dulbecco's Modified Eagle's Medium (DMEM), alpha modified Minimum Essential Medium (alpha MMEM), Basal Medium Essential (BME), CMRL-1066, RPMI 1640, M199 medium. Ham's F10 nutrient medium, KNOCKOUT™ DMEM, Advanced DMEM, MCDB based media such as MCDB -151, -153, -201, and -302 (Sigma, MO), and DMEM/P12. These and other useful media are available from GIBCO, Grand Island, New York, U.S.A., for example. A number of these media are reviewed in Methods in Enzymology, Volume LVIII, "Cell Culture," pp. 62-72, edited by William B. Jakoby and Ira H. Pastan, published by Academic Press Inc.

"Hes-1 ", also known as "hairy/enhancer of split-1" is a transcription factor that may influence cell fate determination.

"Musashi-1" is a member of a subfamily of RNA binding proteins that are highly conserved across species. Musashi-1 expression is highly enriched in proliferative cells within the developing central nervous system, and may be a stem cell marker in intestinal cells.

"Pharmaceutical carrier" refers to a biodegradable or non-degradable porous or nonporous matrix that can act as a carrier for transplantation of mammalian cells.

"Transplantation" as used herein, can include the steps of introducing a cell or a population of cells or tissue into a mammal such as a human patient. "Transplantation" may also include incorporating cells or tissue into a pharmaceutical carrier, and implanting the carrier in a mammal such as a human patient.

### Isolation of Amniotic Fluid-Derived Cells

In one aspect of the present invention, amniotic fluid-derived cells are isolated by a multi-stage method, which essentially involves:
- Isolation of amniotic fluid,
- Centrifugation of the amniotic fluid, followed by removal of the supernatant,
- Resuspending the cell pellet in growth medium,
- Culturing the tissues and cells in a low oxygen environment,
- Leaving the culture undisturbed for about 5 to 10 days without any media changes,
- Isolation of distinct colonies using cloning rings,
- Culturing the isolated colonies in growth media
- Serial dilution cloning and identification of single cells that give rise to proliferating colonies, and
- Culturing the clones in growth media.

In an alternate embodiment, amniotic fluid-derived cells are isolated by a multi-stage method, which essentially involves:
- Isolation of amniotic fluid,
- Centrifugation of the amniotic fluid, followed by removal of the supernatant,
- Resuspending the cell pellet in growth medium,
- Culturing the tissues and cells in a normoxic environment,
- Leaving the culture undisturbed for about 5 to 10 days without any media changes,
- Isolation of distinct colonies using cloning rings,
- Culturing the isolated colonies in growth media,
- Serial dilution cloning and identification of single cells that give rise to proliferating colonies, and
- Culturing the clones in growth media.

The culture plates may be pre-coated with agents such as, for example, fibronectin, vitronectin, laminin, collagen, gelatin, thrombospondin, placenta extracts, MATRIGEL™, tenascin, human serum, or combinations thereof.

If desirable, the amniotic fluid may be exposed, for example, to an agent (such as an antibody) that specifically recognizes a protein marker expressed by amniotic fluid cells, to identify and select amniotic fluid-derived cells, thereby obtaining a substantially pure population of amniotic fluid-derived cells.

Amniotic fluid-derived cells may be cultured in AMNIOMAX™ complete medium (Invitrogen). Alternatively, the cells may be cultured in Chang B/C medium (Irvine Scientific). Alternatively, the cells may be cultured in low glucose DMEM, supplemented with insulin-transferrin-selenium-X (ITS-X, Invitrogen, CA), 2% fetal bovine serum (FBS), 1% penicillin/streptomycin (P/S) + 25 ng/ml bFGF. Alternatively, the cells may be cultured in, DM-KNOCKOUT™ media (Invitrogen, CA), supplemented with 20% KNOCKOUT™ serum replacement (Invitrogen, CA), 10 ng/ml bFGF. Alternatively, the cells may be cultured in Williams' medium E supplemented with 2% defined FBS, 2mM L-glutamine, ITS, 55 µM 2-mercaptoethanol, 10ng/ml EGF, 4ng/ml bFGF, and 4ng/ml dexamethasone. Alternatively, the cells may be cultured in 1:1 DMEM-LG/MCDB 201, 2% FBS, ITS-X, βme 55 µM, 100 µM ascorbic acid-2-phosphate, 4ng/ml bFGF, 10ng/ml EGF, and 4ng/ml dexamethasone. Alternatively, the cells may be cultured in low glucose DMEM, supplemented with 20% FBS. Alternatively, the cell may be cultured in low glucose DMEM, supplemented with 5% FBS. The cells may also be cultured in low glucose DMEM/MCDB 201 medium (1:1), supplemented with 2% defined FBS, ITS-X, 1nM dexamethasone, 100 mM ascorbic acid 2-phosphate, 10ng/ml EGF, 10nglml PDGF-bb and 100 mM 2-mercaptoethanol. The media may be supplemented with bFGF, at concentrations from about 5 ng/ml to about 100 ng/ml. Alternatively, the cells may be cultured in 20% KNOCKOUT™ serum replacement + 80% KNOCKOUT™ DMEM, supplemented with 1 mM L-glutamine, 1% non-essential amino acids and 0.1 mM 2-mercaptoethanol. The medium may be conditioned overnight, on human or murine embryonic fibroblasts, human bone marrow derived stromal cells, or human placenta derived cells and supplemented with 4 ng/ml bFGF. Alternatively, the cells may be cultured in high glucose DMEM, supplemented with 20% defined FBS with 0.1 mM 2- mercaptoethanol. **Table II** lists the various media formulations used to culture the amniotic fluid-derived cells of the present invention.

During culture in growth media, the cells may be cultured under hypoxic or normoxic conditions. Under hypoxic conditions, oxygen levels are lower than 20%, alternatively lower than 10%, alternatively lower than 5%, but more than 1%.

Preferably, the culture should be maintained in the growth media undisturbed for about 5 to 14 days without any media changes, at which point the cells have typically become adherent to the culture substrate used. At which point, cells may be sub-cultured.

Subculture can be achieved with any of the enzymatic solutions well known to those skilled in the art. An example of an enzymatic solution suitable for use in the present invention is TrypLE EXPRESS™ (Invitrogen, Ca).

Furthermore, the amniotic fluid-derived cells may be expanded by culturing in a defined growth media containing agent(s) that stimulate the proliferation of the cells of the present invention. These factors may include, for example, nicotinamide, members of TGF-β family, including TGF-β1, 2, and 3, bone morphogenic proteins (BMP-2, -4, 6, - 7, -11, -12, and -13), serum albumin, fibroblast growth factor family, platelet-derived growth factor-AA, and -BB, platelet rich plasma, insulin growth factor (IGF-I, II) growth differentiation factor (GDF-5, -6, -8, -10, 11), glucagon like peptide-I and II (GLP-I and II), GLP-1 and GLP-2 mimetobody, Exendin-4, retinoic acid, parathyroid hormone, insulin, progesterone, testosterone, estrogen, aprotinin, hydrocortisone, ethanolamine, beta mercaptoethanol, epidermal growth factor (EGF), gastrin I and II, copper chelators such as triethylene pentamine, TGF-α, forskolin, Na-Butyrate, activin, betacellulin, noggin, neuron growth factor, nodal, insulin/transferrin/selenium (ITS), hepatocyte growth factor (HGF), keratinocyte growth factor (KGF), bovine pituitary extract, islet neogenesis-associated protein (INGAP), proteasome inhibitors, notch pathway inhibitors, sonic hedgehog inhibitors, GSK-3 beta inhibitors, or combinations thereof. Alternatively, the amniotic fluid-derived cells may be expanded by culturing in conditioned media. By "conditioned media" is meant that a population of cells is grown in a basic defined cell culture medium and contributes soluble factors to the medium. In one such use, the cells are removed from the medium, while the soluble factors the cells produce remain. This medium is then used to nourish a different population of cells.

In certain embodiments, the amniotic fluid-derived cells are cultured on standard tissue culture plates. Alternatively, the culture plates may be coated with extracellular matrix proteins, such as, for example, MATRIGEL ®, growth factor reduced MATRIGEL ®, laminin, collagen, gelatin, tenascin, fibronectin, vitronectin, thrombospondin, placenta extracts, human serum, or combinations thereof.

### Characterization of The Isolated Amniotic Fluid-Derived Cells

Methods for assessing expression of protein and nucleic acid markers in cultured or isolated cells are standard in the art. These include quantitative reverse transcriptase polymerase chain reaction (RT-PCR), Northern blots, *in situ* hybridization (see, e.g., *Current Protocols in Molecular Biology* (Ausubel *et al.,* eds. 2001 supplement)), and immunoassays, such as immunohistochemical analysis of sectioned material, Western blotting, and for markers that are accessible in intact cells, flow cytometry analysis (FACS) (see, e.g., Harlow and Lane, Using Antibodies: A Laboratory Manual, New York: Cold Spring Harbor Laboratory Press (1998)).

Examples of antibodies useful for detecting certain protein markers are listed in **Table III.** It should be noted that other antibodies directed to the same markers that are recognized by the antibodies listed in **Table III** are available, or can be readily developed. Such other antibodies can also be employed for assessing expression of markers in the cells isolated in accordance with the present invention.

Characteristics of cells of the β-cell lineage are well known to those skilled in the art, and additional characteristics of the β-cell lineage continue to be identified. These characteristics can be used to confirm that the amniotic fluid-derived cells isolated in accordance with the present invention have differentiated to acquire the properties characteristic of the β-cell lineage. β-cell lineage specific characteristics include the expression of one or more transcription factors such as, for example, PDX-1 (pancreatic and duodenal homeobox gene-1), NGN-3 (neurogenin-3), HIxb9, Nkx6, Isll, Pax6, NeuroD, Hnfla, Hnf6, Hnf3 Beta, and Mafa, among others. These transcription factors are well established in the art for identification of endocrine cells. See, e.g., Edlund (Nature Reviews Genetics 3: 524-632 (2002)).

Characteristics of cells of the intestinal cell lineage are well known to those skilled in the art, and additional characteristics of this lineage continue to be identified. These characteristics can be used to confirm that the differentiated or undifferentiated amniotic fluid-derived cells isolated in accordance with the present invention have some of the properties characteristic of the intestinal cell lineage. Intestinal cell lineage characteristics include the expression of one or more transcription factors such as, for example, Hews-1 (hairy/enhancer of split-1), NGN-3, Pax6, NeuroD, Math-1, and Musashi-1, among others. In addition, gut cells express hormones such as secretin, cholecystokinin, GLP-1, neurotensin, gastric inhibitory peptide (GIP), serotonin, somatostatin, and gastrin, among others. These transcription factors and gut hormones are well established in the art for identification of intestinal cells. See, e.g., Schonhoff (Endocrinology 145: 2639-2644 (2004)).

The present inventors have identified and isolated a population of amniotic fluid-derived cells that is highly proliferative, and displays embryonic cell-like characteristics, and may express at least one of the following markers: HNF- beta, HNF-3 beta, SOX-17, or GATA 6. In particular, the amniotic fluid-derived cells isolated in accordance with the present invention are characterized as, *inter alia,* substantially lacking at least one of the following protein markers: CD117, Oct-4 or Tra2-54. Populations of amniotic fluid-derived cells with these characteristics are referred to herein as AF-I (ATCC accession number PTA-6975).

Under the above growth conditions for expansion, the amniotic fluid cells isolated in accordance with the present invention may be expanded for more than 50 population doublings, while maintaining the potential to express at least one of the following markers: HNF-1 beta, HNF-3 beta, SOX-17, or GATA-6.

The present inventors have also identified and isolated populations of amniotic fluid-derived cells that is highly proliferative, displays embryonic cell-like characteristics, and do not express at least one of following markers: HNF-3 beta, SOX-17, GATA-4, CD117, Oct-4 or Tra2-54. In particular, the amniotic fluid-derived cells isolated in accordance with the present invention are characterized as, *inter alia,* substantially lacking at least one of the following protein markers: CD117, Oct-4 or Tra2-54. Populations of amniotic fluid-derived cells with these characteristics are referred to herein as AF-II.

The present inventors have also identified and isolated populations of amniotic fluid-derived cells that is highly proliferative, displays embryonic cell-like characteristics, and do not express any of the following markers: HNF-3beta, SOX-17, GATA-4, CO117, Oct-4 or Tra2-54. In particular, the amniotic fluid-derived cells isolated in accordance with the present invention are characterized as, *inter alia,* substantially lacking at least one of the following protein markers: CD1 17, Oct-4 or Tra2-54. Populations of amniotic fluid-derived cells with these characteristics are referred to herein as AF-II.

The present inventors have also identified and isolated populations of amniotic fluid-derived cells that is highly proliferative, displays embryonic cell-like characteristics, and do not express cytokeratin and at least one of following markers: HNF-3 beta, SOX-17, GATA-4, CD117, Oct-4 or Tra2-54. In particular, the amniotic fluid-derived cells isolated in accordance with the present invention are characterized as, *inter alia,* substantially lacking at least one of the following protein markers: CD117, Oct-4 or Tra2-54. Populations of amniotic fluid-derived cells with these characteristics are refered to herein as AF-III.

The present inventors have also identified and isolated populations of amniotic fluid-derived cells that is highly proliferative, displays embryonic cell-like characteristics, and do not express any of the following markers: cytokeratin, HNF-3beta, SOX-17, GATA-4, CD117, Oct-4 or Tra2-54. In particular, the amniotic fluid-derived cells isolated in accordance with the present invention are characterized as, *inter alia,* substantially lacking at least one of the following protein markers: CD117, Oct-4 or Tra2-54. Populations of amniotic fluid-derived cells with these characteristics are refered to herein as AF-III.

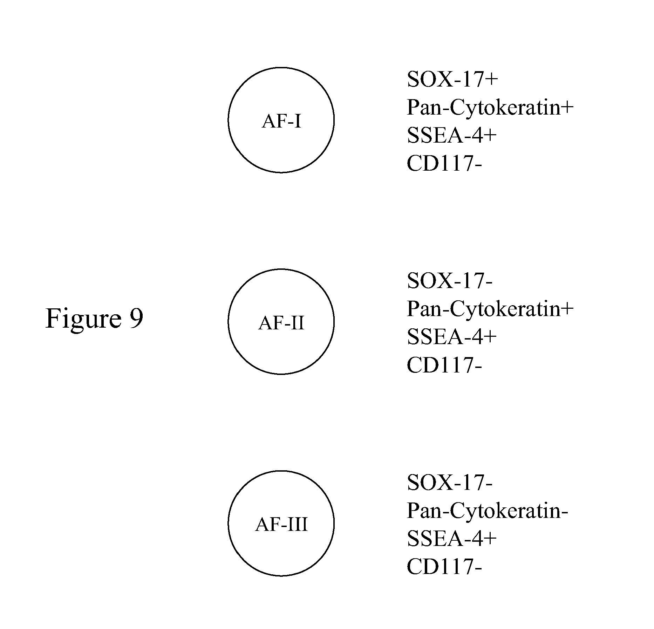

A summay of the expression profile of AF-I, AF-II and AF-III cells is shown in **Figure 9****.**

Amniotic fluid-derived cells of the present invention may be expanded for more than 50 population doublings, while maintaining the potential to differentiate into definitive endoderm, or cells with characteristics of a pancreatic β- cell lineage, or the capacity to differentiate into a gut hormone-producing cell.

### Differentiation Of Amniotic Fluid-Derived Cells

In one aspect, the present invention provides compositions capable of differentiating the expanded amniotic fluid-derived cells of this invention into cells bearing markers characteristic of the βcell lineage.

In another aspect, the present invention provides compositions capable of differentiating the expanded amniotic fluid-derived cells of this invention into cells bearing markers characteristic of definitive endoderm.

In another aspect, the present invention provides compositions capable of differentiating the expanded amniotic fluid-derived cells of this invention into cells bearing markers characteristic of a gut hormone-producing cell.

A basic defined culture medium, when supplied with one or more components, that support the growth of amniotic fluid-derived cells, supplemented with differentiation-inducing amounts of one or more growth factors, is referred to as an "induction medium." In accordance with the present invention, the induction medium contains less than or equal to 20% serum. In one embodiment, fetal calf serum may be used. Alternatively, fetal bovine serum may be replaced by serum from any mammal, or by albumin, bovine albumin or other compounds that permit or enhance differentiation of amniotic fluid-derived cells to the β cell lineage. Alternatively, the induction medium may be conditioned medium.

Factors appropriate for use in the induction medium may include, for example, nicotinamide, members of TGF-β family, including TGF-β1, 2, and 3, bone morphogenic proteins (BMP-2, -4, 6, -7, -11, -12, and -13), serum albumin, fibroblast growth factor family, platelet-derived growth factor-AA, and -BB, platelet rich plasma, insulin growth factor (IGF-I, II) growth differentiation factor (GDF-5, -6, -8, -10, 11), glucagon like peptide-I and II (GLP-I and II), GLP-1 and GLP-2 mimetobody, Exendin-4, retinoic acid, parathyroid hormone, insulin, progesterone, aprotinin, hydrocortisone, ethanolamine, beta mercaptoethanol, epidermal growth factor (EGF), gastrin I and II, copper chelators such as triethylene pentamine, TGF-α, forskolin, Na-Butyrate, activin, betacellulin, ITS, noggin, neurite growth factor, nodal, valporic acid, trichostatin A, sodium butyrate, hepatocyte growth factor (HGF), sphingosine-1, Wnt proteins such as Wnt- 1, -3, -3a, 07a, and -8, keratinocyte growth factor (KGF), Dickkopf protein family, bovine pituitary extract, islet neogenesis-associated protein (INGAP), Indian hedgehog, sonic hedgehog, proteasome inhibitors, notch pathway inhibitors, sonic hedgehog inhibitors, or combinations thereof.

In one aspect of the present invention, a combination of growth factors and chemical agents, including bFGF, Activin-A, FGF5, N2 and B27 supplements (Gibco, CA), steroid alkaloid such as, for example, cyclopamine (EMD, CA) that inhibits sonic hedgehog signaling, and a proteasome inhibitor such as, for example MG132 (EMD, CA), is supplied to a basic defined medium to support differentiation of amniotic fluid-derived cells into a β-cell lineage. In one aspect, the cells are cultured in an induction media composed of DMEM (low glucose, 5.5 mM) containing 10 micromolar MG-132 for 1-2 days, followed by additional incubation for 3-7 days in an induction media supplemented with 1X B27 (Gibco, CA) and 1X N2 (Gibco, CA) and further supplemented with Cyclopamine (10 µM; EMD, CA), bFGF (20 ng/ml; R&D Systems, MN), Activin A (20 nM; R&D Systems, MN) or FGF5 (20 ng/ml; R&D Systems, MN) for an additional five days.

In another aspect of the invention, the cells of the current invention can be treated with conditioned media isolated from cultures of primary fetal intestinal or pancreatic rudiments to induce further differentiation into the intestinal or pancreatic lineages, respectively. The cells may also be induce to differentiate with conditioned media from pancreatic cells lines such as PANC-1, CAPAN-1, BxPC-3, HPAF-II, hepatic cell lines such as HepG2, and intestinal cell lines such as, for example, FHs 74 and HS738. Altrenatively, the cells of the present invention can be treated with conditioned media isolated from human or mouse embryonic stem cells indiced to differentiate into an endodermal lineage. These cell lines can be purchased from the ATCC (VA).

The combination and concentrations of growth factors, the length of culture, and other culture conditions can be optimized by those skilled in the art to achieve effective differentiation by, e.g., monitoring the percentage of cells that have differentiated into cells characteristic of the β-cell lineage. The one or more growth factors may be added in an amount sufficient to induce the differentiation of the amniotic fluid-derived cells of the present invention into cells bearing markers of a β-cell lineage over a time period of about one to four weeks.

### Therapeutic Use of The Cells of The Present Invention.

In one aspect, the present invention provides a method for treating a patient suffering from, or at risk of developing Typel diabetes. This method involves isolating and culturing amniotic fluid-derived cells, expanding the isolated population of cells, differentiating the cultured cells *in vitro* into a β-cell lineage, and implanting the differentiated cells either directly or in a pharmaceutical carrier into the patient. If appropriate, the patient can be further treated with pharmaceutical agents or bioactives that facilitate the survival and function of the transplanted cells. These agents may include, for example, insulin, members of the TGF-β family, including TGF-β1, 2, and 3, bone morphogenic proteins (BMP-2, -3, -4, -5, -6, -7, -11, -12, and -13), fibroblast growth factors-1 and -2, platelet-derived growth factor-AA, and -BB, platelet rich plasma, insulin growth factor (IGF-I, II) growth differentiation factor (GDF-5, -6, -8, -10, -15), vascular endothelial cell-derived growth factor (VEGF), pleiotrophin, endothelin, among others. Other pharmaceutical compounds can include, for example, nicotinamide, glucagon like peptide-I (GLP-1) and II, GLP-1 and 2 mimetibody, Exendin-4, retinoic acid, parathyroid hormone, MAPK inhibitors, such as, for example, compounds disclosed in U.S. Published Application 2004/0209901 and U.S. Published Application 2004/0132729.

In yet another aspect, this invention provides a method for treating a patient suffering from, or at risk of developing Type 2 diabetes. The method involves isolating and culturing amniotic fluid-derived cells according to the present invention, expanding the isolated population of cells, differentiating the cultured cells *in vitro* into a β-cell lineage, and implanting the differentiated cells either directly or in a pharmaceutical carrier into said patient.

In yet another embodiment, the amniotic fluid-derived cells of the present invention may be cryopreserved using commercially available medium containing DMSO (dimethylsulfoxide) or glycerol. The banked and frozen cells may be stored in the vapor phase of a liquid nitrogen storage tank until needed.

In yet another embodiment, the amniotic fluid-derived cells of the present invention may be transplanted with mature islets of the same or different animal species to enhance the survival of the amniotic fluid-derived cells or to induce further differentiation of the amniotic fluid-derived cells into a pancreatic β cell lineage.

The source of amniotic fluid from which the cells are isolated may be autologous in relation to the patient undergoing the therapeutic treatment. Alternatively, the source may be allogeneic, or xenogeneic. Cells to be administered to a patient may also be genetically modified to enhance proliferation and/or differentiation or prevent or lessen the risk of immune rejection. Alternatively, the amniotic fluid-derived cells obtained in accordance with the present invention can be used to modulate the recipient's immune response, prior to transplantation of differentiated cells prepared in accordance with the present invention. See, for example, U.S. Patent 6,328,960, U.S. Patent 6,281,012.

The amniotic fluid-derived cells of the present invention may be differentiated into an insulin-producing cell prior to transplantation into a recipient. In a specific embodiment, the amniotic fluid-derived cells of the present invention are fully differentiated into β-cells, prior to transplantation into a recipient. Alternatively, the amniotic fluid-derived cells of the present invention may be transplanted into a recipient in an undifferentiated or partially differentiated state. Further differentiation may take place in the recipient.

The amniotic fluid-derived cells of the present invention may be genetically modified. For example, the cells may be engineered to over express markers characteristic of a cell l of a β-cell lineage, such as, for example, PDX-1 or insulin. The cells may be engineered to over express with any suitable gene of interest. Furthermore, the cells may be engineered to over express markers characteristic of an intestinal cell, such as MATH-1. Alternatively, the cells of the present invention can be differentiated into a GIP expressing cell population and further modified with an insulin gene under control of the GIP promoter to become glucose responsive and insulin-producing cell population. Techniques useful to genetically modify the amniotic fluid-derived cells of the present invention can be found, for example, in standard textbooks and reviews in cell biology. Methods in molecular genetics and genetic engineering are described, for example, in Molecular Cloning: A Laboratory Manual, 2nd Ed. (Sambrook et al., 1989); Oligonucleotide Synthesis (M. J. Gait, ed., 1984); Animal Cell Culture (R. I. Freshney, ed., 1987); the series Methods in Enzymology (Academic Press, Inc.); Gene Transfer Vectors for Mammalian Cells (I. M. Miller &M. P. Calos, eds. , 1987); Current Protocols in Molecular Biology and Short Protocols in Molecular Biology, 3rd Edition (F. M. Ausubel et al., eds., 1987 &1995); and Recombinant DNA Methodology II (R. Wu ed. , Academic Press 1995).

The nucleic acid molecule, encoding the gene of interest may be stably integrated into the genome of the host amniotic fluid-derived cell, or the nucleic acid molecule may be present as an extrachromosomal molecule, such as a vector or plasmid. Such an extrachromosomal molecule may be auto- replicating. The term "transfection," as used herein, refers to a process for introducing heterologous nucleic acid into the host amniotic fluid-derived cell.

The cells, undifferentiated or otherwise, may be used as dispersed cells or formed into clusters that may be infused into the hepatic portal vein. Alternatively, the cells may be provided in biocompatible degradable polymeric supports, porous non-degradable devices or encapsulated to protect from host immune response. The cells may be implanted into an appropriate site in a recipient. The implantation sites include, for example, the liver, natural pancreas, renal subcapsular space, omentum, peritoneum, subserosal space, intestine, stomach, or a subcutaneous pocket.

To enhance further differentiation, survival or activity of implanted cells, additional factors, such as growth factors, antioxidants or anti-inflammatory agents, can be administered before, simultaneously with, or after the administration of the cells. In certain embodiments, growth factors are utilized to differentiate the administered cells *in vivo.* These factors can be secreted by endogenous cells and exposed to the administered amniotic fluid-derived cells *in situ.* Implanted amniotic fluid-derived cells can be induced to differentiate by any combination of endogenous and exogenously administered growth factors known in the art.

The amount of cells used in implantation depends on a number of factors including the patient's condition and response to the therapy, and can be determined by one skilled in the art.

In one aspect, this invention provides a method for treating a patient suffering from, or at risk of developing diabetes. The method includes isolating and culturing amniotic fluid-derived cells according to the present invention, expanding the isolated population of cells, differentiating *in vitro* the cultured amniotic fluid-derived cells into a β-cell lineage, and incorporating the cells into a three-dimensional support. The cells can be maintained *in vitro* on this support prior to implantation into the patient. Alternatively, the support containing the cells can be directly implanted in the patient without additional *in vitro* culturing. The support can optionally be incorporated with at least one pharmaceutical agent that facilitates the survival and function of the transplanted cells.

Support materials suitable for use for purposes of the present invention include tissue templates, conduits, barriers, and reservoirs useful for tissue repair. In particular, synthetic and natural materials in the form of foams, sponges, gels, hydrogels, textiles, and nonwoven structures, which have been used *in vitro* and *in vivo* to reconstruct or regenerate biological tissue, as well as to deliver chemotactic agents for inducing tissue growth, are suitable for use in practicing the methods of the present invention. See, e.g., the materials disclosed in U.S. Patent 5,770,417, U.S. Patent 6,022,743, U.S. Patent 5,567,612, U.S. Patent 5,759,830, U.S. Patent 6,626,950, U.S. Patent 6,534,084, U.S. Patent 6,306,424, U.S. Patent 6,365,149, U.S. Patent 6,599,323, U.S. Patent 6,656,488, and U.S. Patent 6,333,029. Exemplary polymers suitable for use in the present invention are disclosed in U.S. Published Application 2004/0062753 A1 and U.S. Patent 4,557,264.

To form a support incorporated with a pharmaceutical agent, the pharmaceutical agent can be mixed with the polymer solution prior to forming the support. Alternatively, a pharmaceutical agent could be coated onto a fabricated support, preferably in the presence of a pharmaceutical carrier. The pharmaceutical agent may be present as a liquid, a finely divided solid, or any other appropriate physical form. Alternatively, excipients may be added to the support to alter the release rate of the pharmaceutical agent. In an alternate embodiment, the support is incorporated with at least one pharmaceutical compound that is an anti-inflammatory compound, such as, for example compounds disclosed in U.S. Patent 6,509,369.

In one embodiment, the support is incorporated with at least one pharmaceutical compound that is an anti-apoptotic compound, such as, for example, compounds disclosed in U.S. Patent 6,793,945.

In another embodiment, the support is incorporated with at least one pharmaceutical compound that is an inhibitor of fibrosis, such as, for example, compounds disclosed in U.S. Patent 6,331,298.

In a further embodiment, the support is incorporated with at least one pharmaceutical compound that is capable of enhancing angiogenesis, such as, for example, compounds disclosed in U.S. Published Application 2004/0220393 and U.S. Published Application 2004/0209901.

In still another embodiment, the support is incorporated with at least one pharmaceutical compound that is an immunosuppressive compound, such as, for example, compounds disclosed in U.S. Published Application 2004/0171623.

In a further embodiment, the support is incorporated with at least one pharmaceutical compound that is a growth factor, such as, for example, members of the TGF-β family, including TGF-β1,2, and 3, bone morphogenic proteins (BMP-2, -3, -4, -5, -6, -7, -11, - 12, and -13), fibroblast growth factors-1 and -2, platelet-derived growth factor-AA, and - BB, platelet rich plasma, insulin growth factor (IGF-I, II) growth differentiation factor (GDF-5, -6, -8, -10, -15), vascular endothelial cell-derived growth factor (VEGF), pleiotrophin, endothelin, among others. Other pharmaceutical compounds can include, for example, nicotinamide, hypoxia inducible factor 1-alpha, glucagon like peptide-I (GLP-1), GLP-1 and GLP-2 mimetibody, and II, Exendin-4, nodal, noggin, NGF, retinoic acid, parathyroid hormone, tenascin-C, tropoelastin, thrombin-derived peptides, cathelicidins, defensins, laminin, biological peptides containing cell- and heparin-binding domains of adhesive extracellular matrix proteins such as fibronectin and vitronectin, MAPK inhibitors, such as, for example, compounds disclosed in U.S. Published Application 2004/0209901 and U.S. Published Application 2004/0132729.

The incorporation of the cells of the present invention into a scaffold can be achieved by the simple depositing of cells onto the scaffold. Cells can enter into the scaffold by simple diffusion (J. Pediatr. Surg. 23 (1 Pt 2): 3-9 (1988)). Several other approaches have been developed to enhance the efficiency of cell seeding. For example, spinner flasks have been used in seeding of chondrocytes onto polyglycolic acid scaffolds (Biotechnol. Prog. 14(2): 193-202 (1998)). Another approach for seeding cells is the use of centrifugation, which yields minimum stress to the seeded cells and enhances seeding efficiency. For example, Yang *et al.* developed a cell seeding method (J. Biomed. Mater. Res. 55(3): 379-86 (2001)), referred to as Centrifugational Cell Immobilization (CCI).

The present invention is further illustrated, but not limited by, the following examples.

### Example 1

### The Establishment of Human Amniotic Fluid-Derived Cell Lines

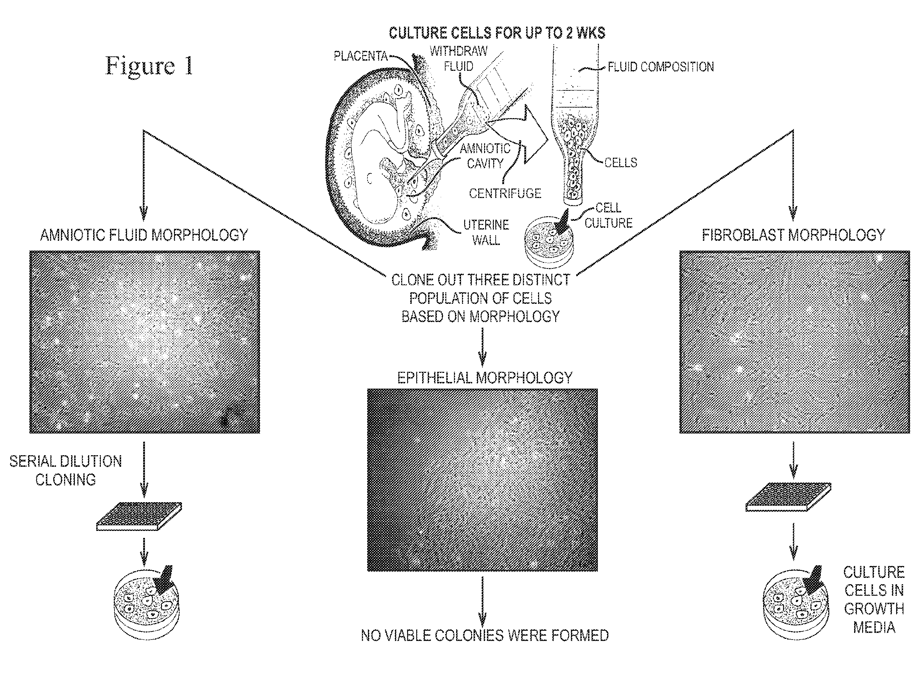

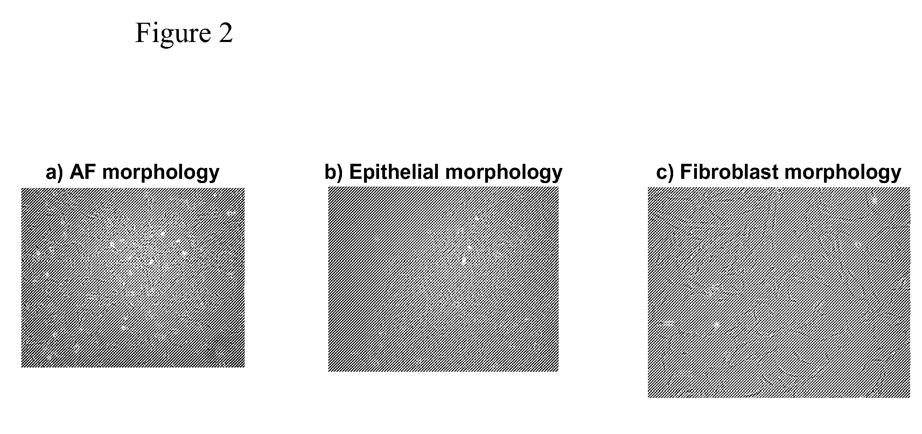

Amniotic fluid used to isolate the cells of the present invention was taken from samples taken from routine amniocentesis performed at 16 to 22 weeks of gestation for fetal karyotyping. The multi-stage method used to isolate the amniotic fluid-derived cells is outlined in **Figure 1**. The amniotic fluid was centrifuged for 7 minutes at 400 *x g* and the supernatant removed. The resulting cell pellet was resuspended in the growth media indicated in **Table III** for the amniotic fluid samples used in the present invention. The cells were cultured either on collagen type IV (1mg/100 mm plate), or on collagen type I (1 microgram/cm²), vitronectin (10 microgram/ml) or fibronectin (10 micrograms/ml) coated plates. The cell yield from amniotic fluid samples had a large variation (8000-300000 cell/sample) and some samples also contained a significant number of blood cell contamination. The cultures were left undisturbed for at least 5-10 days under hypoxic conditions (3% 02). In parallel, cultures were established under similar conditions in normoxic conditions. Next, the cultures were fed with the same growth media and cultured until the cultures reached 70-80% confluency. Cells at this stage were referred to as "P0". In some cultures, colonies of cells were isolated by a cloning ring and sub cultured into a different culture plate. Distinct colonies were present with morphologies characteristic of fibroblast (F), amniotic fluid (AF), and epithelial (E) cells **(****Figure 2****).** Cells were released from P0 culture by using TrypLE Express™ (Invitrogen) and seeded into fibronectin, vitronectin, or collagen type IV coated flaks/dishes/plates at various densities (50-10,000 cell/cm2). Some of the P0 cells were used for serial dilution cloning. The population doubling time of the fastest growing cells was approximately 24 hrs at early passages. The expanded cells cultured under various media conditions **(Table II)** were analyzed for cell surface markers **(Table III).** Cells were typically split at ∼70% confluency and reseeded at 100-10000 cells/cm². RNA was collected at various stages of cell growth and analyzed for embryonic and germ layer markers **(Table V).**

Amniotic fluid cells of the present invention were present at various gestational ages. **Table VI** lists the presence or absence of AF, E, and F morphologies in amniotic fluid samples obtained at 17 weeks to 41 weeks of gestation.

Amniotic fluid cells of the present invention were also obtained from amniotic fluid obtained at term (approximately 40 wks of gestation). Amniotic fluid samples were obtained from 38-40 wk deliveries and cultured according to the protocols outlined above. The resulting adherent cell populations displayed very similar characteristics to the cells isolated from 16-22 wks of gestation.

### Example 2

### Clonal Expansion of the Cells of the Present Invention

Using methods described in **Example 1,** cells with AF-like morphologies were harvested from P0 cultures using cloning rings. Three distinct populations of cells exhibiting different expression of surface receptors, cytoskeletal proteins, and transcription factors were identified. For sake of clarity, these populations are referred to as AF-I, AF-II, and AF-III cells. Subsequent examples highlight the differences between AF-I, -II, and -III populations.

### Example 3

### Fluorescence-Activated Cell Sorting (FACS) Analysis

Adhered cells were removed from culture plates by five-minute incubation with the TRYPLE™ express solution (Gibco, CA). Released cells were resuspended in DMEM supplemented with 10% FBS and recovered by centrifugation, followed by washing and resuspending the cells in a staining buffer consisting of 2% BSA, 0.05% sodium azide (Sigma, MO) in PBS. If appropriate, the cells were Fc-receptor blocked using a 0.1 % γ-globulin (Sigma) solution for 15 min. Aliquots (approximately 10⁵ cells) were incubated with either phycoerythirin (PE) or allophycocyanin (APC) conjugated monoclonal antibodies (5 µl antibody per 10⁶ cells), as indicated in **Table III-A,** or with an unconjugated primary antibody. Controls included appropriate isotype matched antibodies, non-stained cells, and cells only stained with secondary conjugated antibody. All incubations with antibodies were performed for 30 mins at 4°C, after which the cells were washed with the staining buffer. Samples that were stained with unconjugated primary antibodies were incubated for additional 30 mins at 4°C with secondary conjugated PE or -APC labeled antibodies. See **Table III-B** for a list of secondary antibodies used. Washed cells were pelleted and resuspended in the staining buffer and the cell surface molecules were identified by using a FACS Array (BD Biosciences) by collecting at least 10,000 events.

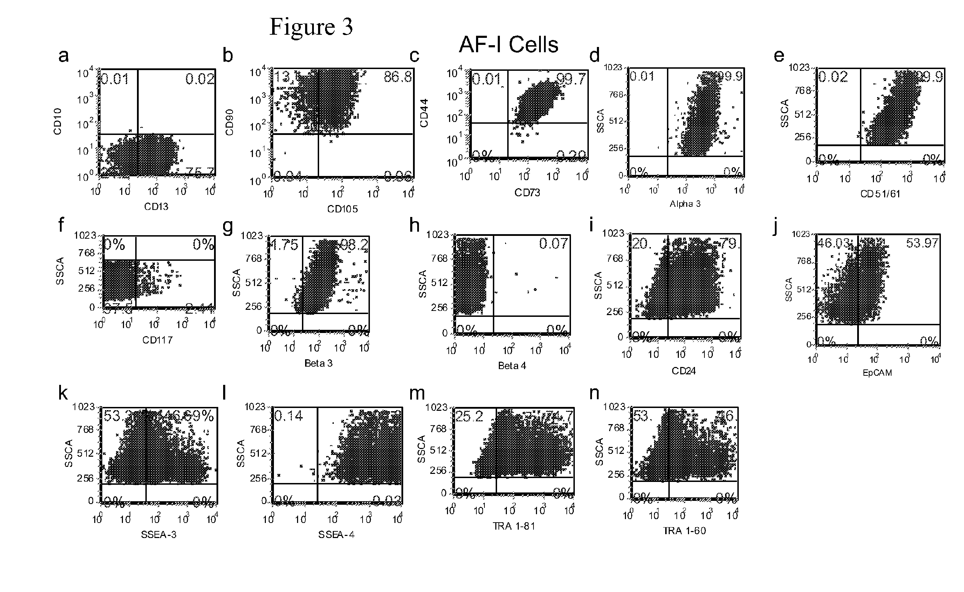

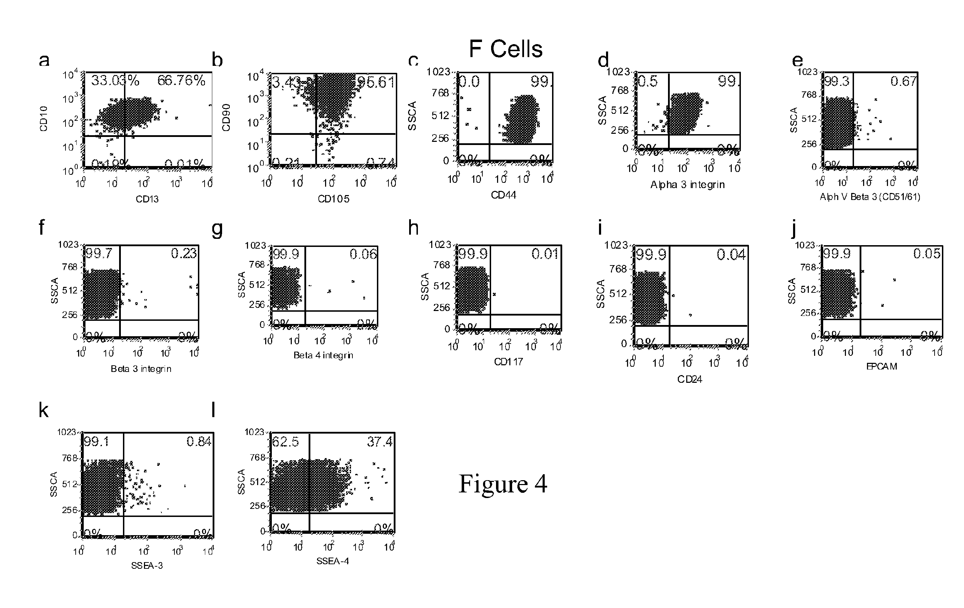

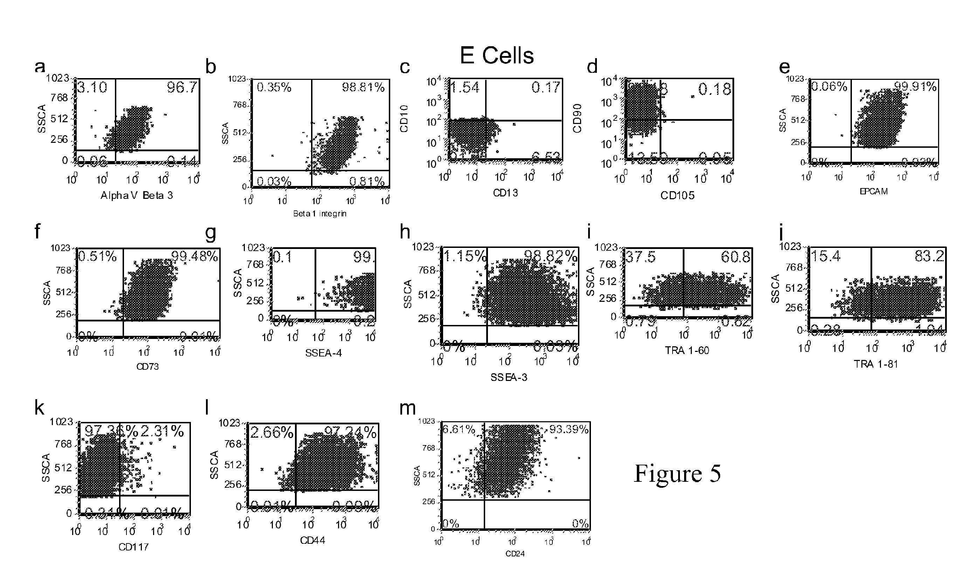

For intracellular staining, cells were first fixed for 10 mins with 4% parafor-maldheyde, followed by two rinses in the staining buffer, centrifugation of cells and resuspension of the cells in a permeabilization buffer containing 0.5% Triton-X (Sigma) in PBS for 5 mins at room temperature (RT). The permeabilized cells were rinsed twice with a rinsing buffer, centrifuged, and resuspended in the staining buffer and incubated with an appropriate conjugated antibody (5 µl antibody per 10⁶ cells), for 30 mins at 4°C. Samples that were stained with unconjugated primary antibodies were incubated for additional 30 mins at 4°C with secondary conjugated PE or-APC labeled antibodies **(Table III B).** Washed cells were pelleted and resuspended in the staining buffer and the internal proteins were identified by using a FACSArray (BD Biosciences) by collecting at least 10,000 events. The expression level of examined surface and internal markers is listed in **Table IV A and B.** FACS analysis allowed identification of signature markers to distinguish amniotic fluid cells (AF-I, -II, and -III), fibroblasts (F), and epithelial cells (E) **(****Figures 3-5****).** Table **IV C** lists the cell surface expression profile of AF-I cells isolated from term (38-40 wks) amniotic fluid. The expression level of cell surface receptors is very similar to AF-I cells isolated from 16-22 wks amniotic fluid.

### Example 4

### Immunostaining of Undifferentiated Cells

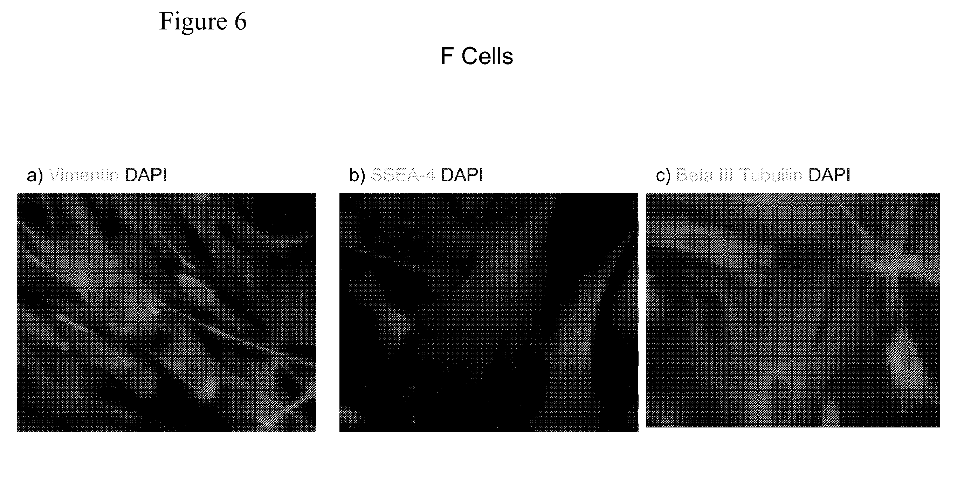

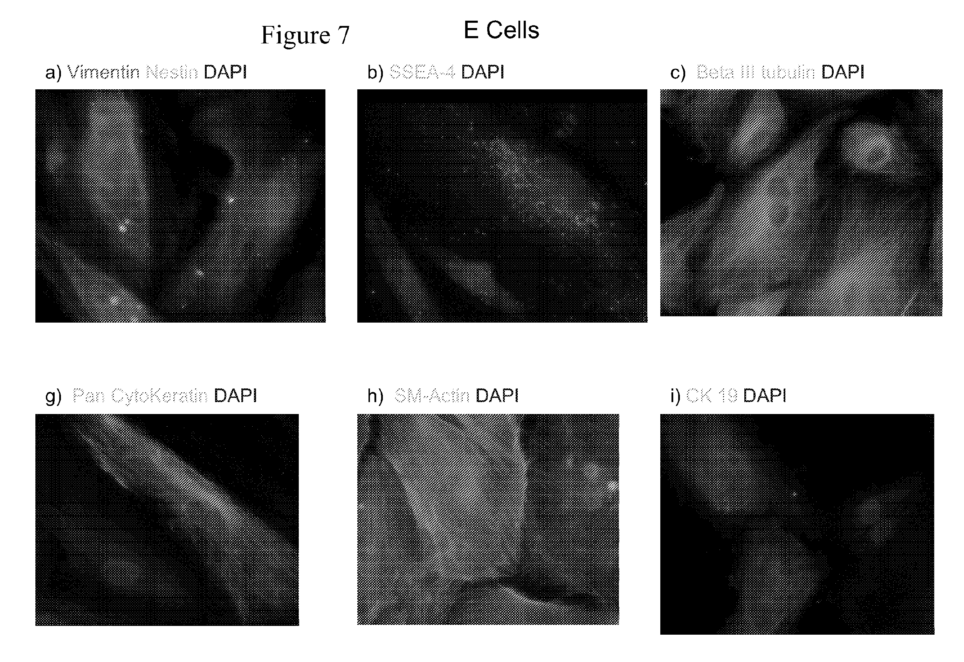

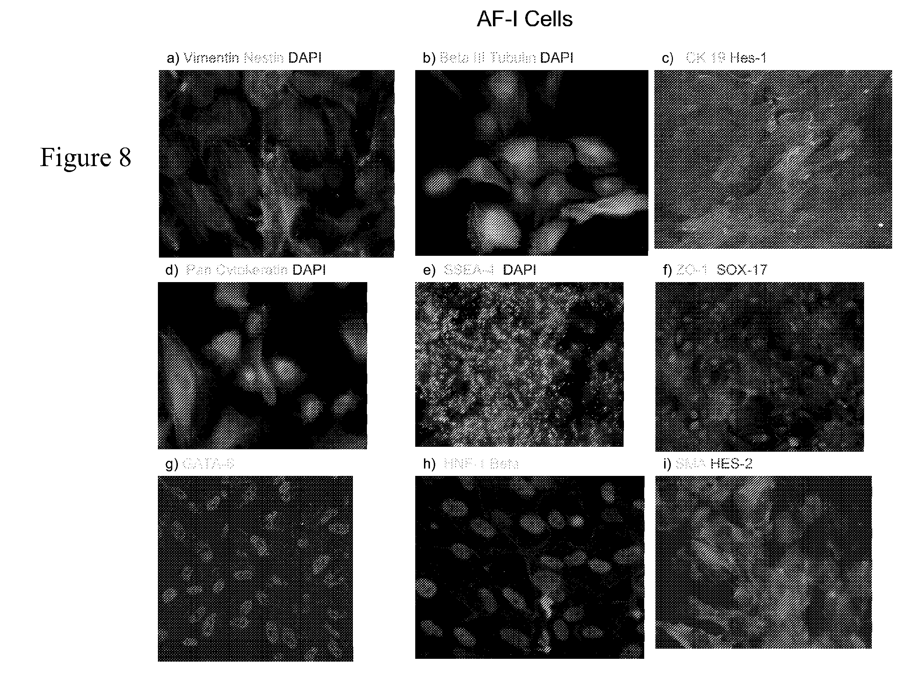

10,000 cells/cm² cells, cultured according to **Example 1,** were seeded into glass bottom 35 mm microwell dishes (Matek Corp, MA) in various growth media. Following three days in culture, the cells were fixed for 10 mins with 4% paraformaldheyde, followed by two rinses in the PBS, and addition of a permeabilization buffer containing 0.5% Triton-X (Sigma) for 5 mins at room temperature (RT) followed by additional three rinses with PBS. The fixed and permeabilized cells were blocked with either 1% bovine serum albumin (BSA) or 4% sera from the species where the secondary antibody was raised in (Goat, donkey, or rabbit). Control samples included reactions with the primary antibody omitted or where the primary antibody was replaced with corresponding immunoglobulins at the same concentration as the primary antibodies. Stained samples were rinsed with a PROLONG® antifade reagent (Invitrogen, CA) containing diamidino-2-phenylindole, dihydrochloride (DAPI) to counter stain the nucleus. Images were acquired using a Nikon Confocal Eclipse C-1 inverted microscope (Nikon, Japan) and a 10-60X objective **(****Figures 6 - 8****).**

### Example 5

### PCR Analysis Of Undifferentiated Cells

RNA was extracted from cells cultured in the growth media. Total RNA from human pancreas, liver, brain ,gut (Ambion, INC.) NTERA cells (human embryonic carcinoma cells line, ATCC), HEK293 cells (ATCC), and human airway epithelia cells (Cambrex) were used as positive controls. Bone marrow derived mesenchymal cells (Cambrex, MD) were used as negative controls for the expression of key genes involved in pancreatic development.

*RNA extraction, purification, and cDNA synthesis.* RNA samples were purified through its binding to a silica-gel membrane (Rneasy Mini Kit, Qiagen, CA) in the presence of an ethanol-containing, high-salt buffer; while contaminants were washed away. The RNA was further purified while bound to the column by treatment with DNase I (Qiagen, CA) for 15 min. High-quality RNA was then eluted in water. Yield and purity were assessed by A260 and A280 readings on the spectrophotometer. cDNA copies were made from purified RNA using an ABI (ABI, CA) high capacity cDNA archive kit.

*Real-time PCR amplification and quantitative analysis.* Unless otherwise stated, all reagents were purchased from Applied Biosystems. Real-time PCR reactions were performed using the ABI PRISM® 7000 Sequence Detection System. TAQMAN® UNIVERSAL PCR MASTER MIX® (ABI, CA) was used with 20 ng of reverse transcribed RNA in a total reaction volume of 20 µl. Each cDNA sample was run in duplicate to correct for pipetting errors. Primers and FAM-labeled TAQMAN®probes were used at concentrations of 200 nM. The level of expression of each target gene was normalized using the pre-developed Applied Biosystem's 18S ribosomal RNA or human glyceraldehydes-3-phosphate dehydrogenase (GAPDH) endogenous control kit. Primers and probes were either designed using ABI PRISM PRIMER EXPRESS™ software or used pre-developed ABI gene analysis kit. For each gene, either one of the primers or the probe were designed to be exon-boundary spanning. This eliminated the possibility of the primers/probe binding to any genomic DNA present. The primer and probe sets are listed as following Nkx2.2 (Hs00159616), Pdx-1 (Hs00426216), Nkx6.1 (Hs00232355), Ngn3 (Hs00360700), Pax4 (Hs00173014), Pax6 (Hs00240871), Insulin (Hs00355773), Glu2 (Hs00165775), glucagon (Hs00174967), Isl-1 (Hs00158126), somatostatin (Hs00174949), FoxA2 (HNF 3-beta) (Hs00232764), HlxB9 (Hs00232128), GATA-4 (Hs00171403), HNF1β (Hs00172123), Musashi Homolog 1 (Msi-1) (Hs00159291), Hes-1 (Hs00172878), Neurotensin (NTS) (Hs00175048), Cholecystokinin (Hs00174937), AFP (Hs00173490), Secretin (Hs00360814), GIP (Hs00175030), GFAP (Hs00157674), MAP2 (Hs00159041), Olig2 (Hs0037782), Oct-4 (CGACCATCTGCCGCTTTGAG (SEQ ID NO: 1) and CCCCCTGTCCCCCA TTCCTA (SEQ ID NO: 2)); Rex-1 (CAGATCCTAAACAGCTCGCAGAAT (SEQ ID NO: 3), and GCGTACGCAAATTAAACTCCAGA(SEQ ID NO: 4); Sox 17: TGGCGCAGCAGATACCA (SEQ ID NO:5), AGCGCCTTCCACGACTTG (SEQ ID NO:6) and CCAGCATCTTGCTCAACTCGGCG (SEQ ID NO:7); ABCG-2: GTTTATCCGTGGTGTGTCTGG (SEQ ID NO: 8) and CTGAGCTATAGAGGCCTGGG (SEQ ID NO: 9); SOX2: ATGCACCGCTACGACGTGA (SEQ ID NO:10) and CTTTTGCACCCCTCCCATTT (SEQ ID NO: 11). The remaining primers were designed by using the PRIMERS program (ABI, CA) and are listed in **Table V.** After an initial 50°C for 2 min, and 95°C for 10 min, samples were cycled 40 times in two stages - a denaturation step at 95°C for 15 sec, followed by an annealing/extension step at 60°C for I min. Data analysis was carried out using GENEAMP®7000 Sequence Detection System software. For each primer/probe set, a Cₜ value was determined as the cycle number at which the fluorescence intensity reached a specific value in the middle of the exponential region of amplification. Relative gene expression levels were calculated using the comparative Cₜ method. Briefly, for each cDNA sample, the endogenous control Cₜ value was subtracted from the gene of interest Cₜ to give the delta Cₜ value (ΔCₜ). The normalized amount of target was calculated as 2^{-ΔCt}, assuming amplification to be 100% efficiency. Final data were expressed relative to a calibrator sample. The comparative Cₜ method is only valid if target and endogenous control amplification efficiencies are approximately equal. Preliminary validation experiments were therefore performed for each primer/probe set by amplifying serially diluted cDNA samples and determining the ΔCₜ values. These ΔCₜ values should remain constant across the range of dilutions if amplification efficiencies are equal **(Table V).**

### Example 6

### Population doubling time

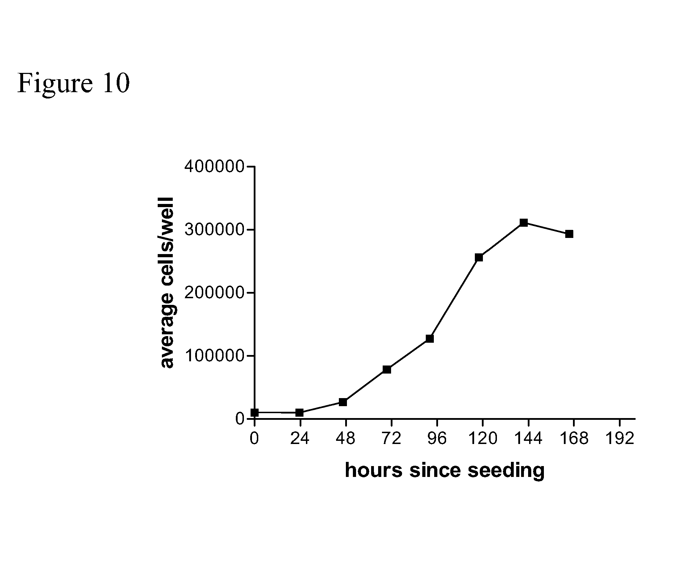

Passage 6 amniotic fluid cells (AF-1 type), isolated and expanded according to Example1 were seeded at 10000 cells/well of a 24-well tissue culture plate (Corning, MA) in growth media #11. At various time points, cells were removed from three wells of the plate using TRYPLE™ Express (Invitrogen, CA) and counted using a Guava PCA-96 cell analysis system and the VIACOUNT® reagent (Guava, CA). **Figure 10** depicts the growth curve of passage 6 cells cultured under hypoxic conditions (3% 02). The linear phase of the log plot was used to estimate the population doubling time of the cells. Population doubling time of passage 6 cells was 31 hrs.

The growth potential of the three cell populations (fibroblast, AF, and epithelial morphology) were compared over long-term cultures. **Figure 11** depicts the growth potential of AF-I, AF-II, AF-III, F, and E cells cultured in media #5. It is clear that F ("fibroblastic" amniotic fluid-derived) cells and AF cells can expand well above 50 population doublings and represent a scalable source for cell therapy applications.

### Example 7

### Telomere length of AF-I cells

The telomere length of an AF-I line isolated from a single cell by limited serial dilution was analyzed at passage 12 (approximately 50 population doublings) by using the *Telo TAGGG* Telomere Length Assay (Roche, IN) and following the manufacturer's instruction. The telomere length was analyzed for cells cultured in DMEM-LG + 10 % FBS and cells cultured in Amniomax™ (Gibco) see **Figure 12****.** DNA from NTERA cells served as a positive control.

### Example 8

### Karyotype analysis

The karyotype of AF cells, isolated from multiple donors at passage 8-10 (approximately 30 population doublings), was determined by G-band analysis. Five karyotypes were prepared and cytogenetic analysis showed that the cells had a normal autosomes and a modal chromosome number of 46. All cells analyzed also contained the X and Y-chromosomes confirming their fetal origin. **Figure 13** depicts karyotypes of amniotic fluid-derived cells (AF-I, AF-II, and AF-III) isolated from amniotic fluid obtained from 16-22 weeks of gestation.

### Example 9

### Expansion potential of AF cells derived from term amniotic fluid

**Figure 14** depicts the expansion potential of an AF-I cell morphology derived from term amniotic fluid (∼38 weeks) and cultured in media #5. The expansion potential is very similar to the AF-I cells isolated from 16-22 wks amniotic fluid.

### Example 10

### Microarray analysis of fibroblast, epithelial, and amniotic fluid morphology cells

Total RNA was isolated from passage 9-11 amniotic fluid-derived fibroblast cells (F), amniotic fluid-derived epithelial cells (E), amniotic fluid-derived amniotic fluid cells (AF-I, -II, and -III lines), and amniotic fluid at term (AF term) using an RNeasy mini kit (Qiagen). The sample preparation, hybridization, and image analysis was performed according to the CodeLink™ System (GE Healthcare, Amersham Biosciences, NJ). Codelink™ Human Whole Genome arrays were used. It is comprised of approximately 55 000 30-mer probes designed to conserved exons across the transcripts of targeted genes. The chip contains ∼45000 unique Unigene IDs. Following normalization and a log transformation, data analysis was performed using OmniViz® software (MA) and GENESIFTER (VizXLabs, WA). The variance stabilizing transformation along with cross sample normalization was applied to the log transformed array dataset. The variability within each cell line and among the different cell lines was compared using the Pearson correlation coefficient. For all the samples analyzed, the correlation coefficient within a cell line was higher as compared to those between the lines. Variance in gene expression profiles between the different cell types along with the correlation coefficient between the lines are depicted in **Figure 15****.** Significant differences in gene expression between the cell types were evaluated using analysis of variance and an F-test with adjusted P-value (Benjamini and Hochberg correction) of ≤ 0.05. **Tables VII A-G** list the genes that are differentially expressed at least 5-fold between the various cell types.

### Example 11

### Differentiation of cells into intestinal-like cells

The AF-I line, AFCA007 Clone A passage 14, was cultured at 10,000 cells/cm² in AMNIOMAX^{™}. At confluency, the cells were further treated for 2 weeks with a daily dose of 10 micro molar retinoic acid (RA) in AMNIOMAX^{™} media. RNA was collected at day 14 and expression of intestinal hormones (secretin, neurotensin, gastric inhibitory peptide (GIP), cholecystokinin, somatostatin, and gastrin) was assessed by using real-titne PCR as outlined in Example 4. Intestinal RNA (Ambion) was used to assess relative levels of expression using the ΔΔCₜ method. **Table VIII** lists the Cₜ values and the relative level of expression of the intestinal hormones in treated and untreated samples. As shown in **Table VIII,** addition of RA enhanced expression of the gut hormones.

### Example 12

### Differentiation of cells into multiple endocrine lineages

Cells from the cell line AFCA007 Clone A (AF-1) at passage 8 were embedded in collagen type I (Becton Dickinson, CA) with 1% growth-factor reduced matrigel matrix (Becton Dickinson), and seeded into 6-well transwell insert at 5x10⁵ cells per well. The bottom well was seeded with human aortic endothelial cells passage 6 (Cambrex. MD). Cells were cultured with DMEM medium supplemented with 5% FBS and growth factors, which includes Cyclopamine, bFGF, EGF, BMP4-7, Activin A, Exendin 4, FGF4, all-trans retinoic acid and γ-secretase inhibitors for 14 days. Cultures were fed every other day. Cells treated by all-trans retinoic acid showed the up-regulation of alpha-fetoprotein (AFP). Treatment of cells with Activin A, BMP4, or the γ-secretase inhibitor L-685,458 up-regulated the expression of HNF-3 beta. Treatment of cells with BMPs at high concentration, 50 ng/ml, also up-regualted the GATA4 expression. Treatment of cells with FGF4 at 50 ng/ml showed an up-regulation of PDX-1 expression

### (Table IX).

Cells from the cell line AFCA004 (E morphology) at passage 6 were seeded at 5x10⁵ cells per well of 6-well culture plates and treated with conditioned medium from confluent PANC-1 cells (ATCC, VA) in combination with different growth factors. Basic FGF, EGF and combination of bFGF and EGF enhanced the expression of HNF-3 beta ∼100 fold over untreated cells. Basic FGF, EGF and BMPs also stimulated somatostatin expression after 14 days treatment **(****Figure 16****, panels a&b).**

Taken together, these results suggest that AF cells could be differentiated into pancreatic, hepatic or intestinal lineage by treating the cells with different growth factors.

### Example 13

### Modulation of the expression of endoderm makers by inhibiting the Notch pathway

Cells from the cell line AFCA007 (AF-I) at passage 8 were treated with a range of concentrations of the notch pathway inhibitor L-685,458 (Sigma, MO) for 3 to 5 days. The cytotoxcity of L-685,458 was determined by measuring cell viability by a MTS assay (Promega, WI). Real-time PCR analysis was performed to analyze Hes-1 expression after the treatment. We found that L-685,458 showed a dose-dependent inhibitory effect on Hes-1 expression, Hes-1 is the downstream direct target of Notch pathway **(****Figure 17****, panel a).** No effect on cell viability, as determined by the MTS assay, was observed following L-685,458 treatment of up to 10µM for 5 days **(****Figure 17****, panel b).**

### Example 14

### Cytokine antibody array analysis for AF I and AF II cells

AFCA007 A (AF-I) and AFCA015 C (AF-II) at passage 10 were grown to approximately 70 % confluency and then cell lystaes was collected using mammalian cell lysis kit (Sigma-Aldrich, MO). Cytokine array analyss was completed using Cytokine Array panels provided by RayBiotech, GA (http://www.raybiotech.com/). **Table X** lists cytokine, cytokine and growth factor receeptor expression following normalization of the data and background subtraction. For each panel, positive and negative controls are also included. The panels were run for two different samples per cell type.

**TABLE I: COMPARISON OF THE CELL OF THE PRESENT INVENTION WITH AMNIOTIC FLUID-DERIVED CELLS OF THE ART.**

| Marker | Cell 1 | Cell 2 | Cell 3 | Cell 4 |
|---|---|---|---|---|
| CD10 | | | | - |
| CD105 | + | + | + | Weak |
| CD11 | | | - | - |
| CD117 | Weak | - | - | - |
| CD13 | | + | | |
| CD29 (Beta | | | | + |
| 1 integrin) | | | | |
| CD31 | | | - | |
| CD34 | Weak | - | - | - |
| CD44 | | + | + | + |
| CD45 | | - | - | |
| CD49b | | + | | |
| (Alpha 2 | | | | |
| integrin) | | | | |
| CD49e | | | + | |
| (Alpha 5 | | | | |
| integrin) | | | | |
| | | | | |
| CD73 | | | + | + |
| CD 166 | | | + | |
| CD90 | + | | + | + |
| HLA ABC | + | + | + | + |
| H LA D | | - | - | - |
| Oct-4 | + | + | | + |
| SSEA-1 | - | - | | |
| SSEA-3 | + | + | | |
| SSEA-4 | + | + | | |
| Stro-1 | | - | | |
| Telomerase | + | | | |
| TRA 1-60 | | + | | |
| TRA 1-81 | | + | | |
| TRA 1-85 | | | | |
| TRA 2-49 | | | | |
| TRA 2-54 | | + | | |

**TABLE II. MEDIA FORMULATIONS USED TO CULTURE AIVINIOTIC-FLUID DERIVED CELLS.**

| Media formulation | |
|---|---|
| 1 | DMEM-LG + ITS-X + 1% P/S + 2% FBS + 25 ng/ml bFGF |
| 2 | Advanced DMEM + ITS-X + 1% P/S + 1% FBS + 25 ng/ml bFGF |
| 3 | Alpha MEM: MCDB 153 (1:1) + ITS-X + 1% P/S + 1% FBS + 25 ng/ml bFGF |
| 4 | Defined Keratinocyte growth media (Gibco, NY) |
| 5 | AMNIOMAX™ complete media (Gibco, NY) |
| 6 | Chang B/C (Irvine Scientific, CA) |
| 7 | Chang D (Irvine Scientific, CA) |
| 8 | DM-KNOCKOUT™ media (lnvitrogen, CA), supplemented with 20% KNOCKOUT™ serum replacement (Invitrogen, CA) ,10 ng/ml bFGF |
| 9 | DMEM- Low glucose, supplemented with 20% FBS |
| 10 | DMEM- Low glucose, supplemented with 5% FBS |
| 11 | DMEM- low glucose /MCDB 201 medium (1:1), supplemented with 2% defined FBS, ITS-X, 1nM dexamethasone, 100 mM ascorbic acid 2-phosphate, 10ng/ml EGF, 10ng/ml PDGF-bb and 100 mM 2-mercaptoethanol |
| 12 | 20% KNOCKOUT™ serum replacement + 80% KNOCKOUT™ DMEM, supplemented with 1 mM L-glutamine, 1% non-essential amino acids and 0.1 mM 2-mercaptoethanol |
| 13 | The medium may be conditioned overnight, on human or murine embryonic fibroblasts, human bone marrow derived stromal cells, or human placenta derived cells and supplemented with 4 ng/ml bFGF |
| 14 | high glucose DMEM, supplemented with 20% defined FBS with 0.1 mM 2- mercaptoethanol |
| 15 | Williams' E medium, 2% FBS, 1X ITS, 55uM βme, 1X Glutamax (Gibco), 1%P/S, 10ng/ml EGF, 4ng/ml bFGF, 4ng/ml dexamethasone |
| 16 | 1:1 DMEM-LG/MCDB 201, 2% FBS, ITS-X, βme 55uM,100uM ascorbic acid-2-phosphate, 4ng/ml bFGF, 10ng/ml EGF, 4ng/ml dexamethasone |

**TABLE III A: ANTIBODIES TO SURFACE RECEPTORS.**

| Antibody | Supplier | Isotype | Clone |
|---|---|---|---|
| CD117 (c-Kit) | Santa Cruz Biotechnology (CA) | Goat IgG | M-14 |
| CD117 (c-Kit) | Santa Cruz Biotechnology (CA) | Mouse IgG1 | 104D2 |
| CD117 (c-Kit) | BD Pharmingen (CA) | Mouse IgG1, kappa | YB5.B8 |
| CD24 | BD Pharmingen (CA) | Mouse IgG2A, Kappa | ML5 |
| CD44 | BD Pharmingen (CA) | Mouse IgG2b, Kappa | G44-26 |
| CD45 | BD Phamingen (CA) | Mouse IgG1, Kappa | Hi30 |
| CD49f | BD Pharmingen (CA) | Rat IgG2A, Kappa | G0H3 |
| CD73 | BD Pharmingen (CA) | Mouse IgG1, Kappa | AD2 |
| CD10 | BD Pharmingen (CA) | Mouse IgG1, Kappa | HI10a |
| CD105 | Santa Cruz (CA) | Mouse IgG1 | P3D1 |
| CD49b (Alpha 2 | BD Pharmingen | Mouse IgG2a, | 121-H6 |

| integrin) | (CA) | Kappa | |
|---|---|---|---|
| Alpha 3 integrin | Santa Cruz (CA) | Mouse IgG1 | P1B5 |
| Alpha 4 integrin | BD Pharmingen | Mouse IgG1, | 9P10 |
| (CD49d) | (CA) | Kappa | |
| Alpha 5 intgerin | BD Pharmingen | Mouse IgG1, | IIA1 |
| (CD49e) | (CA) | Kappa | |
| CD49f (Alpha 6 | BD Pharmingen | Rat IgG2a | GoH3 |
| integrin) | (CA) | | |
| CD29 (Beta 1 | BD Pharmingen | Mouse IgG1, | MAR4 |
| integrin) | (CA) | Kappa | |
| Beta 3 integrin | Santa Cruz (CA) | Mouse IgG1 | Y2/51 |
| Alpha V Beta 3 | BD Pharmingen | Mouse IgG1, | 23C6 |
| integrin (CD51/61) | (CA) | Kappa | |
| SSEA-3 | Chemicon (CA) | Mouse IgG3 | MC-631 |
| SSEA-4 | Chemicon (CA) | Rat IgM | MC-813-70 |
| TRA 1-60 | Chemicon (CA) | Mouse IgM | TRA 1-60 |
| TRA 1-81 | Chemicon (CA) | Mouse IgM | TRA 1-81 |
| TRA 1-85 | Chemicon (CA) | Mouse IgG1 | TRA 1-85 |
| TRA 2-54 | Chemicon (CA) | Mouse IgG1 | TRA 2-54 |
| EGF r | BD Pharmingen (CA) | Mouse IgG2b, Kappa | EGFR1 |
| EpCAM | BD Pharmingen (CA) | Mouse IgG1 | EBA-1 |
| HLA ABC | BD Pharmingen (CA) | Mouse IgGl, Kappa | G46-2.6 |
| HLA DR | BD Pharmingen (CA) | Mouse IgG2b, Kappa | TU36 |
| CD90 | BD Pharmingen (CA) | Mouse IgG1, kappa | 5E10 |

**TABLE III B: LIST OF SECONDARY CONJUGATED ANTIBODIES USED FOR FACS AND IMMUNOSTAINININGANALYSIS.**

| Secondary conjugated | Supplier | Dilution |
|---|---|---|
| antibody | | |
| Goat Anti-Mouse IgG APC | Jackson ImmunoResearch | 1 :200 |
| conjugated | (PA) | |
| Goat Anti-Mouse IgG PE | Jackson ImmunoResearch | 1:200 |
| conjugated | (PA) | |
| Donkey anti-rabbit PE or - | Jackson ImmunoResearch | 1:200 |
| APC conjugated | (PA) | |
| Donkey anti-goat PE or - | Jackson ImmunoResearch | 1 :200 |
| APC conjugated | (PA) | |
| Goat anti-mouse IgM PE | SouthernBiotech (AL) | 1:200 |
| Goat anti-Rat IgM PE | SouthernBiotech (AL) | 1:200 |
| Goat anti-mouse IgG3 PE | SouthernBiotech (AL) | 1:200 |

**TABLE III C: LIST OF PRIMARY ANTIBODIES USED FOR IMMUNOSTAINININGANALYSIS.**

| Antibody | Supplier | Dilution |
|---|---|---|
| Beta III Tubulin | R&D Systems (MN) | 1:100 |
| C-Kit | Santa Cruz Biotechnology (CA) | 1:100 |
| Cytokeratin 18 | Sigma (MO) | 1:100 |
| Cytokeratin 8 | Sigma (MO) | 1:100 |
| Cytokeratin-7 | Santa Cruz Biotechnology (CA) | 1:100 |
| Cytokeratin 19 | Sigma (MO) | 1:100 |
| E-Cadherin | BD Transduction Laboratories (CA) | 1:100 |
| FOXAI | Chemicon International (CA) | 1:100 |
| GATA-6 | R&D Systems (MN) | 1:100 |
| HES-1 | Chemicon International (CA) | 1:100 |
| HES-2 | Chemicon International (CA) | 1:100 |
| FINF-1 alpha | BD Transduction Laboratories (CA) | 1:100 |
| HNF-1 beta | BD Transduction Laboratories (CA) | 1:100 |
| Musashi-1 | Chemicon International (CA) | 1:100 |
| Nestin | R&D Systems (MN) | 1:100 |
| Pan-Cytokeratin (CK, 4, 5, | Santa Cruz Biotechnology (CA) | 1:100 |
| 6, 10, 13, and 18) | | |
| SOX-17 | R&D Systems (MN) | 1:100 |
| SSEA-4 | Santa Cruz Biotechnology (CA) | 1:100 |
| TRA 1-81 | Santa Cruz Biotechnology (CA) | 1:100 |
| Vimentin | Santa Cruz Biotechnology (CA) | 1:100 |
| ZO-1 | BD Transduction Laboratories (CA) | 1:100 |

**TABLE IV A: CELL SURFACE RECEPTORS ON FIBROBLAST, AMNIOTIC FLUID, AND EPITHELIAL CELLS DERIVED FROM ANMNIOTIC FLUID AND GROWN UNDER HYPOXIC CONDITIONS**

| | Fibroblast cells (F) | Amniotic fluid cells (AF-I) | Amniotic fluid cells (AF-II) | Amniotic fluid cells (AF-III) | Epithelial cells (E) |
|---|---|---|---|---|---|
| Alpha 3 | + | + | + | + | + |
| integrin | | | | | |
| Beta 1 | + | + | + | + | + |
| integrin | | | | | |
| Beta 3 | - | + | Weak | Weak | + |
| integrin | | | | | |
| Alpha V - | | + | Weak | Weak | + |
| Beta 3 | | | | | |
| (CD51/ | | | | | |
| 61) | | | | | |
| C-Met | | + | + | + | |
| CD24 | Weak | + | Weak | Weak | + |
| CD10 | + | Weak | - | - | - |
| CD105 | + | Weak | + | + | - |
| CD117 | - | - | - | - | - |
| CD13 | + | Weak | + | + | - |
| CD44 | + | + | + | + | + |
| CD73 | + | + | + | + | + |
| CD90 | + | + | + | + | + |
| EpCAM - | | + | - | - | + |
| HLA | + | + | + | + | + |
| ABC | | | | | |
| HLA II- | - | - | - | - | - |
| DR | | | | | |
| TRA2- | - | - | - | - | - |
| 49 | | | | | |
| SSEA-4 | + | + | + | + | + |
| SSEA-3 - | | + | - | - | + |
| TRA1- | - | + | - | - | + |
| 60 | | | | | |
| TRA 1- | - | + | - | - | + |
| 81 | | | | | |

**TABLE IV B: INTRACELLULAR PROTEINS OF VARIOUS AMNIOTIC FLUID CELL TYPES GROWN IN MEDIA #5**

| Marker | Fibroblast cells (F) | Amniotic fluid cells (AF-I) | Amniotic fluid cells (AF-II) | Amniotic fluid cells (AF-III) | Epithelial cells (E) |
|---|---|---|---|---|---|
| CK-19 | - | + | - | - | + |
| Vimentin | + | + | + | + | + |
| Smooth | Weak | Weak | Weak | Weak | + |
| muscle actin | | | | | |
| Beta III | + | + | + | + | + |
| tubulin | | | | | |
| Pan | - | + | + | - | + |
| cytokeratin | | | | | |
| Cytokeratin | - | + | | | |
| 7 | | | | | |
| Cytokeratin | - | + | | | |
| 8 | | | | | |
| Cytokeratin | - | + | | | |
| 18 | | | | | |
| Nestin | - | + | + | + | Weak |

**TABLE IV C: CELL SURFACE RECEPTORS ON AMNIOTIC FLUID CELLS DERIVED FROM TERM ANMNIOTIC FLUID AND GROWN UNDER HYPOXIC CONDITIONS**

| | Amniotic fluid cells (AF-I) |
|---|---|
| Alpha 3 | + |
| integrin | |
| Beta I | + |
| integrin | |
| Beta 3 | + |
| integrin | |
| Alpha V | + |
| Beta 3 | |
| (CD51/61) | |
| CD10 | Weak |
| CD 105 | Weak |
| CD117 | - |
| CD13 | Weak |
| CD44 | + |
| CD73 | + |
| CD90 | + |
| EpCAM | + |
| HLA ABC | + |
| HLA II- | - |
| DR | |
| SSEA-4 | + |
| TRA1-60 | + |
| TRA 1-81 | + |

**TABLE V: PCR ANALYSIS OF THE AMNIOTIC FLUID-DERIVED CELLS OF THE PRESENT INVENTION a. Pluripotency markers**

| **Markers** | **Fibroblast Cells** | **Epithelial cells** | **AF-I Cells** | **AF-II Cells** | **AF-III Cells** | **Ntera-I** |
|---|---|---|---|---|---|---|
| Oct3/4 | - | - | - | - | - | + |
| Sox-2 | - | - | - | - | - | + |
| Rex-1 | - | + | +/- | | | + |
| hTERT | - | +/- | - | - | - | + |

### b. Endocrine markers

| Cell Markers | Fibroblast cells | Epithelial cells | AF_I cells | AF_II cells | AF_III cells |
|---|---|---|---|---|---|
| Sox 17 | Weak | + | + | - | - |
| GATA-6 | - | + | + | + | + |
| GATA-4 | Weak | + | Weak | - | - |
| HNF 1 Beta | - | + | + | + | - |
| HNF3 beta | - | + | Weak | - | - |
| Pdx-1 | - | - | - | - | - |
| NGN-3 | - | - | - | - | - |
| Musashi-1 | + | + | + | | |
| HES-1 | + | + | + | + | + |
| NeuroD1 | - | - | - | - | - |
| Pax 4 | - | - | - | | |
| Pax6 | + | + | + | - | - |
| | | | | | |
| Secretin | - | - | - | - | - |
| Gastric | | | | | |
| inhibitory | | + | | - | - |
| peptide (GIP) | + | | + | | |
| Glucagon | - | - | - | - | - |
| Somatostatin | + | + | + | - | - |
| | | | | | |
| Cholecystokinin | - | | - | - | - |
| Gastrin | - | - | - | - | - |
| Insulin | | - | Weak | - | - |
| Nestin | + | + | + | + | + |

| | | | | | |
|---|---|---|---|---|---|
| *Weak: CT values of 35-38 cycles +: CT value <35 cycles | | | | | |

**TABLE VI: PRESENCE OF ALL AF, E, AND F MORPHOLOGIES AT VARIOUS GESTATIONAL AGES IN SECOND TRIMESTER AMNIOTIC FLUID SAMPLES.**

| AF designation | Gestation age | Presence of AF-like morphology | Presence of E-like morphology | Presence of F-like morphology |
|---|---|---|---|---|
| AFCA001 | 17 wks | + | + | + |
| AFCA002 | 18 wks | + | + | - |
| AFCA004 | 18 wks | + | + | - |
| AFCA008 | 18 wks | + | + | - |
| AFDX001 | 19 wks | + | + | + |
| AFDX021 | 20 wks | + | + | + |
| AFDX022 | 20 wks | + | + | + |
| AFCA007 | 19 wks | + | + | - |
| AFCA009 | 18 wks | + | + | - |
| AFCA010 | 18 wks | + | + | - |
| AFCA011 | 19 wks | + | + | - |
| AFCA017 | 16 wks | + | + | - |
| AFPN003 | 20 wks | + | + | - |
| AFPN004 | 20 wks | + | + | - |
| AFND001 | 41 wks | + | + | - |

**TABLE VII A: GENES THAT WERE DIFFERENTIALLY EXPRESSED AT LEAST 5 FOLD IN FIBROBLAST VERSUS AF-I CELLS**

| Gene Identifier | Gene Name | Average fold change in fibroblast versus AF cells | Direction adj. p- value |
|---|---|---|---|
| | | | |
| NM_002421 | Homo sapiens matrix metalloproteinase 1 (interstitial collagenase) (MMP1), mRNA | 434.28 UP | 1.09E-03 |
| NM_144594 | Homo sapiens hypothetical protein FLJ32942 (FLJ32942), mRNA | 207.74 UP | 2.52E-03 |
| NM_020927 | Homo sapiens KIAA1576 protein (KIAA1576), mRNA | 200.11 UP | 5.06E-04 |
| NM_001451 | Homo sapiens forkhead box F1 (FOXF1), mRNA | 183.69 UP | 1_52E-03 |
| AK021543 | Homo sapiens cDNA FLJ11481 fis, clone HEMBA1001803 | 131.1 UP | 3.11 E-03 |
| NM_007036 | Homo sapiens endothelial cell-specific molecule 1 (ESM1), mRNA | 120.15 UP | 2.07E-03 |
| NM_002448 | Homo sapiens msh homeo box homolog 1 (Drosophila) (MSX1), mRNA | 112.45 UP | 7.69E-04 |
| NM_152270 | Homo sapiens hypothetical protein FLJ34922 (FLJ34922), mRNA | 109.54 UP | 1.63E-03 |
| NM_000474 | Homo sapiens twist homolog 1 (acrocephalosyndactyly 3; Saethre-Chotzen syndrome) (Drosophila) | 102.84 UP | 1.72E-03 |
| AK122739 | (TWIST1), mRNA Homo sapiens cDNA FLJ16260 fis, clone IMR322006947, highly similar to Rattus norvegicus m RNA for dHand protein | 98.04 UP | 4.82E-03 |
| AV702977 | AV702977 ADB Homo sapiens cDNA clone ADBCVD08 5, mRNA sequence | 93.67 UP | 2.91 E-03 |
| AK026784 | Homo sapiens cDNA: FLJ23131 fis, clone LNG08502 | 87.77 UP | 1.52E-03 |
| NM_000710 | Homo sapiens bradykinin receptor B1 (BDKRB1), mRNA | 86.86 UP | 4.77E-03 |
| NM_000609 | Homo sapiens chemokine (C-X-C motif) ligand 12 (stromal cell-derived factor 1) (CXCL12), mRNA | 77.16 UP | 2.97E-03 |
| NM_030781 | Homo sapiens collectin sub-family member 12 (COLEC12), transcript variant II, mRNA | 64.54 UP | 8.99E-05 |
| NM_032638 | Homo sapiens GATA binding protein 2 (GATA2), mRNA | 63.54 UP | 3.35E-03 |
| NM_000362 | Homo sapiens tissue inhibitor of metalloproteinase 3 (Sorsby fundus dystrophy, pseudoinflammatory) (TIMP3), mRNA | 61.32 UP | 5.06E-03 |
| NM_198148 | Homo sapiens carboxypeptidase X (M14 family), member 2 (CPXM2), mRNA | 57.01 UP | 4.00E-03 |
| AL831863 | Homo sapiens mRNA; cDNA DKFZp761J2017 (from clone DKFZp761J2017) | 55.07 UP | 7.57E-03 |
| NM_002091 | Homo sapiens gastrin-releasing peptide (GRP), mRNA | 53.77 UP | 9.01 E-03 |
| NM_020404 | Homo sapiens CD164 sialomucin-like 1(CD164L1), mRNA | 51.44 UP | 2.22E-04 |
| NM_014178 | Homo sapiens syntaxin binding protein 6 (amisyn) (STXBP6), mRNA | 47.27 UP | 7.17E-03 |
| NM_205855 | Homo sapiens HWKM1940 (UNQ1940), mRNA | 42.6 UP | 2.31 E-03 |
| BX089019 | BX089019 Soares_testis_NHT Homo sapiens cDNA clone IMAGp998K243513 ; IMAGE:1391375, mRNA sequence | 42.17 UP | 8.50E-03 |
| Al124557 | am58g02.x1 Johnston frontal cortex Homo sapiens cDNA clone IMAGE:1539794 3, mRNA sequence | 37.2 UP | 8.24E-03 |
| NM_139211 | Homo sapiens homeodomain-only protein (HOP), transcript variant 2, mRNA | 34.14 UP | 1.45E-04 |
| NM_002593 | Homo sapiens procollagen C-endopeptidase enhancer (PCOLCE), mRNA | 30.85 UP | 2.64E-03 |
| BQ020357 | UI-H-EDO-axk-p-07-0-UI.sl NCI_CGAP_ED0 Homo sapiens cDNA clone IMAGE:5830134 3, mRNA sequence | 30.74 UP | 5.91 E-03 |
| CA843592 | ir49c12.x1 HR85 islet Homo sapiens cDNA clone IMAGE:6548544 3, mRNA sequence | 29.57 UP | 6.22E-05 |
| NM_002852 | Homo sapiens pentaxin-related gene, rapidly induced by IL-1 beta (PTX3), mRNA | 29.35 UP | 3.55E-03 |
| NM_000089 | Homo sapiens collagen, type I, atpha2(COL1A2), mRNA | 28.94 UP | 4.61 E-04 |
| CD677332 | ho15f06.y1 Human Trabecular meshwork cDNA: hohphq Homo sapiens cDNA clone ho15f06 5, mRNA sequence | 28.67 UP | 1.15E-04 |
| BC030692 | Homo sapiens ELAV (embryonic lethal, abnormal vision, Drosophila)-like 2 (Hu antigen B), mRNA (cDNA clone MGC:26319 IMAGE:4826082), complete cds | 28.22 UP | 7.64E-03 |
| AK021543 | Homo sapiens cDNA FLJ11481 fis, clone HEMBA1001803 | 27.4 UP | 1.70E-02 |
| AI962169 | wq45c10.x1 NCI_CGAP_GC6 Homo sapiens cDNA clone IMAGE:2474226 3, mRNA sequence | 27.1 UP | 8.11E-03 |
| NM_000685 | Homo sapiens angiotensin II receptor, type 1 (AGTR1), transcript variant 1, mRNA | 26.59 UP | 4.86E-03 |
| AI422199 | tf58d04.x1 NCI_CGAP_Brn23 Homo sapiens cDNA clone IMAGE:2103463 3, mRNA sequence | 26.11 UP | 1.04E-02 |
| | Homo sapiens GATA binding protein | 24.62 UP | 3.24E-03 |
| NM_0010022 95 | 3 (GATA3), transcript variant 1, mRNA | | |
| NM_000396 | Homo sapiens cathepsin K (pycnodysostosis) (CTSK), mRNA | 24.58 UP | 4.37E-05 |
| NM_001442 | Homo sapiens fatty acid binding protein 4, adipocyte (FABP4), mRNA | 24.37 UP | 8.07E-03 |
| NM_004460 | Homo sapiens fibroblast activation protein, alpha (FAP), mRNA | 24.15 UP | 1.93E-04 |
| AB067499 | Homo sapiens mRNA for KIAA1912 protein, partial cds | 23.83 UP | 2.51 E-03 |
| NM_032777 | Homo sapiens G protein-coupled receptor 124 (GPR124), mRNA | 22.29 UP | 3.83E-03 |
| U83115 | Human non-lens beta gamma-crystallin like protein (AIM1) mRNA, partial cds | 21.93 UP | 5.28E-04 |
| AY335938 | Homo sapiens homeodomain protein IRXA1 (IRX1) mRNA, complete cds | 21.54 UP | 2.02E-02 |
| NM_006350 | Homo sapiens follistatin (FST), transcript variant FST317, mRNA | 21.53 UP | 2.20E-03 |
| W38393 | zb15c07.r1 Soares_fetal_lung_NbHL19W Homo sapiens cDNA clone IMAGE:302124 5, mRNA sequence | 21.16 UP | 8.46E-05 |
| NM_021637 | Homo sapiens transmembrane protein 35 (TMEM35), mRNA | 20.78 UP | 3.74E-04 |
| AK091731 | Homo sapiens cDNA FLJ34412 fis, clone HEART2002432 | 20.71 UP | 1.19E-02 |
| NM_032883 | Homo sapiens chromosome 20 open reading frame 100 (C20orf100), mRNA | 20.29 UP | 2.33E-04 |
| NM_005110 | Homo sapiens glutamine-fructose-6-phosphate transaminase 2 (GFPT2), mRNA | 19.69 UP | 9.13E-05 |
| NM_024633 | Homo sapiens chromosome 14 open reading frame 139 (C14orf139), mRNA | 19.44 UP | 1.05E-02 |
| NM_004811 | Homo sapiens leupaxin (LPXN), mRNA | 18.74 UP | 1.74E-05 |
| NM_153183 | Homo sapiens nudix (nucleoside diphosphate linked moiety X)-type motif 10 (NUDT10), mRNA | 18.51 UP | 9.88E-03 |
| NM_014459 | Homo sapiens protocadherin 17 (PCDH17), mRNA | 18.39 UP | 1.39E-03 |
| BX115659 | BX115659 Soares_total_fetus_Nb2HF8_9w Homo sapiens cDNA clone IMAGp998C204119 ; IMAGE:1623883, mRNA sequence | 18.15 UP | 4.07E-04 |
| NM_018013 | Homo sapiens hypothetical protein FLJ10159 (FLJ10159), mRNA | 17.64 UP | 1.54E-02 |
| BX112628 | BX112628 Soares_fetal_lung_NbHL19W Homo sapiens cDNA clone IMAGp998A09669 ; IMAGE:299024, mRNA sequence | 16.09 UP | 2.35E-02 |
| NM_016428 | Homo sapiens ABI gene family, member 3 (ABI3), mRNA | 16.07 UP | 1.15E-02 |
| NM_012449 | Homo sapiens six transmembrane epithelial antigen of the prostate (STEAP), mRNA | 16.07 UP | 1.49E-04 |
| NM_006350 | Homo sapiens follistatin (FST), transcript variant FST317, mRNA | 15.98 UP | 6.30E-05 |
| AF052115 | Homo sapiens clone 23688 mRNA sequence | 15.92 UP | 1.56E-02 |
| NM_004787 | Homo sapiens slit homolog 2 (Drosophila) (SLIT2), mRNA | 15.9 UP | 4.75E-04 |
| AK091336 | Homo sapiens cDNA FLJ34017 fis, clone FCBBF2002626 | 15.59 UP | 2.68E-02 |
| NM_006329 | Homo sapiens fibulin 5 (FBLN5), mRNA | 15.59 UP | 7.87E-03 |
| NM_000963 | Homo sapiens prostaglandin-endoperoxide synthase 2 (prostaglandin G/H synthase and cyclooxygenase) (PTGS2), mRNA | 15.57 UP | 8.59E-04 |
| NM_001146 | Homo sapiens angiopoietin 1 (ANGPT1), transcript variant 1, mRNA | 14.89 UP | 9.40E-04 |
| NM_018431 | Homo sapiens docking protein 5 (DOK5), transcript variant 1, mRNA | 14.56 UP | 8.69E-03 |
| NM_017577 | Homo sapiens hypothetical protein DKFZp434C0328 (DKFZp434C0328), mRNA | 14.47 UP | 4.20E-03 |
| H85497 | yv88b07.r1 Soares melanocyte 2NbHM Homo sapiens cDNA clone IMAGE:249781 5, mRNA sequence | 13.8 UP | 1.13E-04 |
| NM_001147 | Homo sapiens angiopoietin 2 (ANGPT2), mRNA | 13.76 UP | 2.64E-02 |
| NM_000090 | Homo sapiens collagen, type III, alpha 1 (Ehlers-Danlos syndrome type IV, autosomal dominant) (COL3A1), mRNA | 13.63 UP | 1.65E-02 |
| NM_007289 | Homo sapiens membrane metallo-endopeptidase (neutral endopeptidase, enkephalinase, CALLA, CD10) (MME), transcript variant 2b, mRNA | 13.35 UP | 1.86E-02 |
| NM_016229 | Homo sapiens cytochrome b5 reductase b5R.2 (CYB5R2), transcript variant 1, mRNA | 13.35 UP | 9.80E-03 |
| NM_000810 | Homo sapiens gamma-aminobutyric acid (GABA) A receptor, alpha 5 (GABRA5), mRNA | 13.07 UP | 2.71 E-02 |
| NM_002518 | Homo sapiens neuronal PAS domain protein 2 (NPAS2), mRNA | 12.99 UP | 1 _04E-02 |
| AK093256 | Homo sapiens cDNA FLJ35937 fis, clone TESTI2011480 | 12.93 UP | 2.09E-03 |
| NM_005127 | Homo sapiens C-type (calcium dependent, carbohydrate-recognition domain) lectin, superfamily member 2 (activation-induced) (CLECSF2), mRNA | 12.82 UP | 2.71 E-02 |
| NM_006209 | Homo sapiens ectonucleotide pyrophosphatase/phosphodiesteras e 2 (autotaxin) (ENPP2), mRNA | 12.62 UP | 2.60E-02 |
| W03013 | za02c04.r1 Soares melanocyte 2NbHM Homo sapiens cDNA clone IMAGE:291366 5 similar to contains THR.t3 THR repetitive element ;, mRNA sequence | 12.43 UP | 7.69E-04 |
| AF131813 | Homo sapiens clone 24970 mRNA sequence | 12.17 UP | 2.57E-02 |
| BQ025821 | UI-1-BB1p-aye-f-10-0-UI.s1 NCI_CGAP_PI6 Homo sapiens cDNA clone UI-1-BB1 p-aye-f-10-0-UI 3, mRNA sequence | 12.15 UP | 1.30E-02 |
| AW445209 | UI-H-BI3-akc-g-11-0-UI.s1 NCI_CGAP_Sub5 Homo sapiens cDNA clone IMAGE:2733908 3, mRNA sequence | 12.09 UP | 8.91 E-04 |
| NM_033292 | Homo sapiens caspase 1, apoptosis-related cysteine protease | 11.63 UP | 2.67E-02 |
| | (interleukin 1, beta, convertase) (CASP1), transcript variant alpha, mRNA | | |
| NM_170744 | Homo sapiens unc-5 homolog B (C. elegans) (UNC5B), mRNA | 11.62 UP | 3.68E-02 |
| NM_012204 | Homo sapiens general transcription factor IIIC, polypeptide 4, 90kDa (GTF3C4), mRNA | 11.59 UP | 3.11E-02 |
| NM_002531 | Homo sapiens neurotensin receptor 1 (high affinity) (NTSR1), mRNA | 11.33 UP | 2.65E-02 |
| BG118019 | 602351269F1 NIH_MGC_90 Homo sapiens cDNA clone IMAGE:4446065 5, mRNA sequence | 11.16 UP | 8.51E-05 |
| NM_014802 | Homo sapiens KIAA0528 gene product (KIAA0528), mRNA | 11.05 UP | 2.84E-02 |
| BX647541 | Homo sapiens mRNA; cDNA DKFZp686P0492 (from clone DKFZp686P0492) | 10.9 UP | 1.65E-03 |
| NM_020809 | Homo sapiens Rho GTPase activating protein 20 (ARHGAP20), mRNA | 10.85 UP | 2.74E-02 |
| NM_016307 | Homo sapiens paired related homeobox 2 (PRRX2), mRNA | 10.85 UP | 7.71 E-05 |
| NM_057179 | Homo sapiens twist homolog 2 (Drosophila) (TWIST2), mRNA | 10.83 UP | 7.22E-05 |
| AK128325 | Homo sapiens cDNA FLJ46467 fis, clone THYMU3022668 | 10.82 UP | 1.20E-02 |
| BE866150 | 601679068F1 NIH_MGC_53 Homo sapiens cDNA clone IMAGE:3961768 5, mRNA sequence | 10.74 UP | 2.16E-02 |
| BX109483 | BX109483 NCI_CGAP_Ov23 Homo sapiens cDNA clone IMAGp998C165481 ; IMAGE:2216391, mRNA sequence | 10.72 UP | 2.65E-02 |
| C02345 | HUMGS0007544 Human adult (K.Okubo) Homo sapiens cDNA, mRNA sequence | 10.64 UP | 2.15E-03 |
| NM_002961 | Homo sapiens S100 calcium binding protein A4 (calcium protein, calvasculin, metastasin, murine placental homolog) (S100A4), transcript variant 1, mRNA | 10.61 UP | 1.87E-04 |
| NM_031908 | Homo sapiens C1q and tumor necrosis factor related protein 2 (C1QTNF2), mRNA | 10.13 UP | 3.04E-02 |
| NM_013387 | Homo sapiens ubiquinol-cytochrome c reductase complex (7.2 kD) (HSPC051), transcript variant 1, mRNA | 9.95 UP | 4.17E-04 |
| BC039369 | Homo sapiens, clone IMAGE:5271073, mRNA, partial cds | 9.76 UP | 4.82E-03 |
| BQ934941 | AGENCOURT_8810373 NIH_MGC_101 Homo sapiens cDNA clone IMAGE:6429485 5, mRNA sequence | 9.53 UP | 2.21 E-02 |
| NM_006670 | Homo sapiens trophoblast glycoprotein (TPBG), mRNA | 9.37 UP | 2.37E-04 |
| BC046364 | Homo sapiens flavoprotein oxidoreductase MICAL3, mRNA (cDNA clone IMAGE:5737121), with apparent retained intron | 9.09 UP | 2.55E-02 |
| NM_024600 | Homo sapiens chromosome 16 open reading frame 30 (C16orf30), mRNA | 9.09 UP | 4.32E-02 |
| AK023647 | Homo sapiens cDNA FLJ13585 fis, clone PLACE1009150 | 8.81 UP | 2.25E-03 |
| NM_017805 | Homo sapiens Ras interacting protein 1 (RASIP1 mRNA | 8.78 UP | 2.38E-03 |
| NM_152399 | Homo sapiens hypothetical protein FLJ30834 (FLJ30834), mRNA | 8.73 UP | 2.18E-02 |
| NM_002851 | Homo sapiens protein tyrosine phosphatase, receptor-type, Z polypeptide 1 (PTPRZ1), mRNA | 8.61 UP | 5.08E-02 |
| AK024653 | Homo sapiens cDNA: FLJ21000 fis, clone CAE03359 | 8.51 UP | 6.29E-03 |
| NM_020226 | Homo sapiens PR domain containing 8 (PRDM8), mRNA | 8.48 UP | 1.49E-04 |
| NM_012307 | Homo sapiens erythrocyte membrane protein band 4.1-like 3 (EPB41L3), mRNA | 8.41 UP | 7.37E-03 |
| NM_002203 | Homo sapiens integrin, alpha 2 (CD49B, alpha 2 subunit of VLA-2 receptor) (ITGA2), mRNA | 8.3 UP | 8.37E-05 |
| NM_203370 | Homo sapiens similar to RIKEN cDNA 6530418L21 (LOC389119), mRNA | 8.27 UP | 8.21 E-05 |
| NM_015192 | Homo sapiens phospholipase C, beta 1 (phosphoinositide-specific) (PLCB1), transcript variant 1, mRNA | 8.26 UP | 2.64E-03 |
| AK056725 | Homo sapiens cDNA FLJ32163 fis, clone PLACE6000371 | 8.22 UP | 1.68E-04 |
| NM_005328 | Homo sapiens hyaluronan synthase | 8.2 UP | 4.39E-02 |
| NM_012294 | 2 (HAS2), mRNA Homo sapiens Rap guanine nucleotide exchange factor (GEF) 5 (RAPGEF5), mRNA | 8.08 UP | 4.94E-02 |
| AA187037 | zp58e04.r1 Stratagene endothelial cell 937223 Homo sapiens cDNA clone IMAGE:624414 5, mRNA sequence | 7.95 UP | 1.09E-02 |
| NM_001463 | Homo sapiens frizzled-related protein (FRZB), mRNA | 7.94 UP | 1.31 E-03 |
| BM468332 | AGENCOURT_6432296 NIH_MGC_71 Homo sapiens cDNA clone IMAGE:5535773 5, mRNA sequence | 7.94 UP | 7.42E-05 |
| AK096661 | Homo sapiens cDNA FLJ39342 fis, clone OCBBF2018873 | 7.82 UP | 2.30E-03 |
| NM_001849 | Homo sapiens collagen, type VI, alpha 2 (COL6A2), transcript variant 2C2, mRNA | 7.67 UP | 1.10E-04 |
| BC071787 | Homo sapiens cDNA clone IMAGE:4610527, partial cds | 7.59 UP | 1.14E-04 |
| NM_012105 | Homo sapiens beta-site APP-cleaving enzyme 2 (BACE2), transcript variant a, mRNA | 7.58 UP | 2.00E-04 |
| NM_014217 | Homo sapiens potassium channel, subfamily K, member 2 (KCNK2), mRNA | 7.58 UP | 4.61 E-02 |
| N28431 | yx35c03.r1 Soares melanocyte 2NbHM Homo sapiens cDNA clone IMAGE:263716 5 similar to SP:PIR:S32603 S32603 collagen | 7.47 UP | 1.75E-02 |
| | alpha 1(VI) chain - mouse;, mRNA sequence | | |
| NM_012445 | Homo sapiens spondin 2, extracellular matrix protein (SPON2), mRNA | 7.22 UP | 4.03E-02 |
| NM_000304 | Homo sapiens peripheral myelin protein 22 (PMP22), transcript variant 1, mRNA | 7.02 UP | 4.58E-02 |
| NM_004791 | Homo sapiens integrin, beta-like 1 (with EGF-like repeat domains) (ITGBL1), mRNA | 7.02 UP | 2.64E-02 |
| NM_053044 | Homo sapiens serine protease HTRA3 (HTRA3), mRNA | 6.86 UP | 1.13E-03 |
| BC028245 | Homo sapiens, Similar to hypothetical gene LOC130797, clone IMAGE:5395354, mRNA | 6.86 UP | 1.28E-02 |
| NM_023003 | Homo sapiens transmembrane 6 superfamily member 1 (TM6SF1), mRNA | 6.86 UP | 2.12E-03 |
| AJ420536 | Homo sapiens mRNA full length insert cDNA clone EUROIMAGE 994183 | 6.79 UP | 3.53E-04 |
| NM_152996 | Homo sapiens sialyltransferase 7 ((alpha-N-acetylneuraminyl-2,3-beta-galactosyl-1,3)-N-acetyl galactosaminide alpha-2,6-sialyltransferase) C (SIAT7C), mRNA | 6.79 UP | 4.08E-02 |
| NM_005583 | Homo sapiens lymphoblastic leukemia derived sequence 1 (LYL1), mRNA | 6.77 UP | 1.92E-02 |
| NM_016270 | Homo sapiens Kruppel-like factor 2 (lung) (KLF2), mRNA | 6.55 UP | 5.02E-02 |
| NM_005397 | Homo sapiens podocalyxin-like (PODXL), mRNA | 6.53 UP | 3.68E-03 |
| NM_006182 | Homo sapiens discoidin domain receptor family, member 2 (DDR2), mRNA | 6.52 UP | 8.46E-05 |
| U79271 | Human clones 23920 and 23921 mRNA sequence | 6.31 UP | 8.59E-05 |
| BC036034 | Homo sapiens endothelial differentiation, lysophosphatidic acid G-protein-coupled receptor, 2, transcript variant 2, mRNA (cDNA clone MGC:33157 IMAGE:5272431), complete cds | 6.3 UP | 3.07E-04 |
| NM_152314 | Homo sapiens hypothetical protein MGC34830 (MGC34830), mRNA | 6.29 UP | 2.93E-04 |
| AK095791 | Homo sapiens cDNA FLJ38472 fis, clone FEBRA2022148 | 6.27 UP | 1.74E-03 |
| NM_004840 | Homo sapiens Rac/Cdc42 guanine nucleotide exchange factor (GEF) 6 (ARHGEF6), mRNA | 6.2 UP | 1.32E-03 |
| AL833655 | Homo sapiens mRNA; cDNA DKFZp667O0320 (from clone DKFZp667O0320) | 6.18 UP | 3.01 E-02 |
| NM_032270 | Homo sapiens factor for adipocyte differentiation 158 (FAD158), mRNA | 6.1 UP | 6.22E-05 |
| NM_021643 | Homo sapiens tribbles homolog 2 (Drosophila) (TRIB2), mRNA | 5.97 UP | 8.59E-05 |
| AW044647 | wy78e09.x1 Soares_NSF_F8_9W_OT_PA_P_S1 Homo sapiens cDNA clone IMAGE:2554696 3, mRNA sequence | 5.94 UP | 4.09E-02 |
| AK125284 | Homo sapiens cDNA FLJ43294 fis, clone MESTC1000042 | 5.85 UP | 3.94E-03 |
| NM_005069 | Homo sapiens single-minded homolog 2 (Drosophila) (SIM2), transcript variant SIM2, mRNA | 5.84 UP | 8.35E-05 |
| NM_018836 | Homo sapiens transmembrane protein SHREW1 (SHREW1), mRNA | 5.83 UP | 2.71 E-02 |
| NM_014932 | Homo sapiens neuroligin 1 (NLGN1), mRNA | 5.83 UP | 2.45E-03 |
| NM_006307 | Homo sapiens sushi-repeat-containing protein, X-linked (SRPX), mRNA | 5.77 UP | 4.30E-04 |
| NM_000210 | Homo sapiens integrin, alpha 6 (ITGA6), mRNA | 5.77 UP | 7.49E-05 |
| BG208475 | RST27977 Athersys RAGE Library Homo sapiens cDNA, mRNA sequence | 5.72 UP | 1.46E-03 |
| N36786 | yy34e08.s1 Soares melanocyte 2NbHM Homo sapiens cDNA clone IMAGE:273158 3 similar to contains element MSR1 repetitive element ;, mRNA sequence | 5.67 UP | 9.13E-05 |
| NM_005308 | Homo sapiens G protein-coupled receptor kinase 5 (GRK5), mRNA | 5.58 UP | 1.35E-02 |
| AB037722 | Homo sapiens mRNA for KIAA1301 protein, partial cds | 5.58 UP | 9.18E-04 |
| NM_012242 | Homo sapiens dickkopf homolog 1 (Xenopus laevis) (DKK1 mRNA | 5.57 UP | 1.68E-04 |
| NM_003068 | Homo sapiens snail homolog 2 (Drosophila) (SNA12), mRNA | 5.53 UP | 7.42E-05 |
| NM_080806 | Homo sapiens collagen, type XIII, alpha 1 (COL13A1), transcript variant 10, mRNA | 5.52 UP | 8.65E-05 |
| NM_173553 | Homo sapiens hypothetical protein FLJ25801 (FLJ25801 mRNA | 5.48 UP | 1.77E-02 |
| A1085016 | ow88e06.s1 Soares_fetaUiver _spleen_1 NFLS_ S1 Homo sapiens cDNA clone IMAGE:1653922 3, mRNA sequence | 5.45 UP | 4.77E-03 |
| NM_182728 | Homo sapiens solute carrier family 7 (cationic amino acid transporter, y+ system), member 8 (SLC7A8), transcript variant 2, mRNA | 5.34 UP | 2.70E-02 |
| BC035066 | Homo sapiens, clone IMAGE:5259543, mRNA | 5.29 UP | 2.24E-02 |
| NM_016247 | Homo sapiens interphotoreceptor matrix proteoglycan 2 (IMPG2), mRNA | 5.22 UP | 2.97E-03 |
| NM_001704 | Homo sapiens brain-specific angiogenesis inhibitor 3 (BA13), mRNA | 5.19 UP | 1.08E-02 |
| NM_005226 | Homo sapiens endothelial differentiation, sphingolipid G-protein-coupled receptor, 3 (EDG3), mRNA | 5.18 UP | 1.53E-04 |
| NM_017413 | Homo sapiens apelin, AGTRL1 ligand (APLN), mRNA | 5.14 UP | 2.48E-02 |
| NM_000576 | Homo sapiens interleukin 1, beta (IL1B), mRNA | 5.1 UP | 3.32E-03 |
| NM_012307 | Homo sapiens erythrocyte membrane protein band 4.1 -like 3 (EPB41 L3), mRNA | 5.09 UP | 4.93E-04 |
| AL831835 | Homo sapiens mRNA; cDNA DKFZp547A0515 (from clone DKFZp547A0515) | 5.04 UP | 4.14E-02 |
| NM_000955 | Homo sapiens prostaglandin E receptor 1 (subtype EP1), 42kDa (PTGER1), mRNA | 5.02 UP | 3.12E-03 |
| NM_018658 | Homo sapiens potassium inwardly-rectifying channel, subfamily J, member 16 (KCNJ16), transcript variant 1, mRNA | 334.17 Down | 8.59E-05 |
| NM_007038 | Homo sapiens a disintegrin-like and metalloprotease (reprolysin type) with thrombospondin type 1 motif, 5 (aggrecanase-2) (ADAMTS5), mRNA | 250.9 Down | 3.32E-05 |
| BG219729 | RST39494 Athersys RAGE Library Homo sapiens cDNA, mRNA sequence | 182.25 Down | 6.10E-05 |
| AB032980 | Homo sapiens mRNA for KIAA1154 protein, partial cds | 159.9 Down | 7.49E-05 |
| NM_022454 | Homo sapiens SRY (sex determining region Y)-box 17 (SOX17), mRNA | 151.72 Down | 3.05E-05 |
| AK130281 | Homo sapiens cDNA FLJ26771 fis, clone PRS03189 | 148.34 Down | 6.10E-05 |
| NM_003238 | Homo sapiens transforming growth factor, beta 2 (TGFB2), mRNA | 139.18 Down | 7.00E-05 |
| BU734212 | UI-E-CQ1-agd-e-21-0-Ul.s1 UI-E-CQ1 Homo sapiens cDNA clone UI-E-CQ1-agd-e-21-0-UI 3, mRNA sequence | 135.35 Down | 7.49E-05 |
| BM993116 | UI-H-DTO-aty-f-17-0-ULs1 NCI_CGAP_DT0 Homo sapiens cDNA clone IMAGE:5866000 3, mRNA sequence | 134.03 Down | 8.59E-05 |
| BX648964 | Homo sapiens mRNA; cDNA DKFZp686J0156 (from clone DKFZp686J0156) | 133.35 Down | 1.19E-04 |
| AI765021 | wh56c02.x1 NCI_CGAP_Kid11 Homo sapiens cDNA clone IMAGE:2384738 3, mRNA sequence | 131.86 Down | 6.22E-05 |
| D29453 | HUMNK566 Human epidermal keratinocyte Homo sapiens cDNA clone 566, mRNA sequence | 128.83 Down | 6.30E-05 |
| NM_002521 | Homo sapiens natriuretic peptide precursor B (NPPB), mRNA | 128.09 Down | 1.42E-04 |
| BX102632 | BX102632 NCI_CGAP_Co3 Homo sapiens cDNA clone IMAGp998J052307 ; IMAGE:928228, mRNA sequence | 123.34 Down | 1.15E-04 |
| NM_024508 | Homo sapiens zinc finger, BED domain containing 2 (ZBED2), mRNA | 118.4 Down | 5.18E-05 |
| NM_153026 | Homo sapiens prickle-like 1 (Drosophila) (PRICKLE1 mRNA | 106.78 Down | 6.10E-05 |
| NM_006228 | Homo sapiens prepronociceptin (PNOC), mRNA | 102.73 Down | 2.87E-04 |
| NM_005560 | Homo sapiens laminin, alpha 5 (LAMA5), mRNA | 99.24 Down | 7.49E-05 |
| NM_023942 | Homo sapiens hypothetical protein MGC3036 (MGC3036), mRNA | 91.38 Down | 6.30E-05 |
| AK096481 | Homo sapiens cDNA FLJ39162 fis, clone OCBBF2002376 | 85.12 Down | 7.45E-05 |
| A1335277 | tb29h06.x1 NCI_CGAP_Kid12 Homo sapiens cDNA clone IMAGE:2055803 3, mRNA sequence | 84.58 Down | 6.22E-05 |
| BF798098 | RC1-CI0045-021000-021-f02 CI0045 Homo sapiens cDNA, mRNA sequence | 81.7 Down | 8.59E-05 |
| AK023631 | Homo sapiens cDNA FLJ13569 fis, clone PLACE1008369 | 77.57 Down | 7.00E-05 |
| NM_000927 | Homo sapiens ATP-binding cassette, sub-family B (MDR/TAP), member 1 (ABCB1), mRNA | 77.16 Down | 2.21 E-04 |
| BG197054 | RST16291 Athersys RAGE Library Homo sapiens cDNA, mRNA sequence | 76.09 Down | 1.03E-04 |
| AK058012 | Homo sapiens cDNA FLJ25283 fis, clone STM06716 | 75.4 Down | 6.22E-05 |
| NM_000104 | Homo sapiens cytochrome P450, family 1, subfamily B, polypeptide 1 (CYP1B1),mRNA | 70.8 Down | 6.24E-05 |
| NM_004617 | Homo sapiens transmembrane 4 superfamily member 4 (TM4SF4), mRNA | 63.32 Down | 1.39E-04 |
| NM_000990 | Homo sapiens ribosomal protein L27a (RPL27A), mRNA | 62.1 Down | 5.32E-05 |
| NM_002899 | Homo sapiens retinol binding protein 1, cellular (RBP1), mRNA | 62.01 Down | 2.87E-04 |
| NM_000582 | Homo sapiens secreted phosphoprotein 1 (osteopontin, bone sialoprotein I, early T-lymphocyte activation 1)(SPP1),mRNA | 60.53 Down | 1.18E-04 |
| NM_002423 | Homo sapiens matrix metalloproteinase 7 (matrilysin, uterine) (MMP7), mRNA | 60.01 Down | 1.56E-04 |
| H89053 | yw24c06.r1 Morton Fetal Cochlea Homo sapiens cDNA clone IMAGE:253162 5, mRNA sequence | 59.02 Down | 7.45E-05 |
| AK090808 | Homo sapiens cDNA FLJ33489 fis, clone BRAMY2003585 | 58.15 Down | 7.49E-05 |
| NM_001200 | Homo sapiens bone morphogenetic protein 2 (BMP2), mRNA | 57.05 Down | 6.24E-05 |
| NM_001453 | Homo sapiens forkhead box C1 (FOXC1), mRNA | 56.24 Down | 6.10E-05 |
| AK092245 | Homo sapiens cDNA FLJ34926 fis, clone NT2RP7003319, highly similar to Mus musculus neuralin mRNA | 56.11 Down | 2.55E-04 |
| NM_005329 | Homo sapiens hyaluronan synthase 3 (HAS3), transcript variant 1, mRNA | 55.44 Down | 7.22E-05 |
| AI244954 | qj93h05.x1 NCI_CGAP_Kid3 Homo sapiens cDNA clone IMAGE:1867065 3, mRNA sequence | 54.38 Down | 6.22E-05 |
| NM_003287 | Homo sapiens tumor protein D52-like 1 (TPD52L1), transcript variant 1, mRNA | 53.91 Down | 7.45E-05 |
| BF696790 | 602125323F1 NIH_MGC_56 Homo sapiens cDNA clone IMAGE:4282540 5, mRNA sequence | 49.46 Down | 1.09E-04 |
| NM_001202 | Homo sapiens bone morphogenetic protein 4 (BMP4), transcript variant 1, mRNA | 47.81 Down | 8.21 E-05 |
| BC042028 | Homo sapiens, clone IMAGE:4794726, mRNA | 47.5 Down | 7.49E-05 |
| AL833276 | Homo sapiens mRNA; cDNA DKFZp451 D088 (from clone DKFZp451 D088) | 43.28 Down | 7.28E-05 |
| NM_184087 | Homo sapiens tripartite motif-containing 55 (TRIM55), transcript variant 4, mRNA | 42.9 Down | 7.42E-05 |
| NM_003494 | Homo sapiens dysferlin, limb girdle muscular dystrophy 2B (autosomal recessive) (DYSF), mRNA | 42.2 Down | 7.31 E-05 |
| NM_024422 | Homo sapiens desmocollin 2 (DSC2), transcript variant Dsc2a, mRNA | 41.3 Down | 1.96E-04 |
| AI249696 | qj64a03.x1 NCLCGAP_Kid3 Homo sapiens cDNA clone IMAGE:1864204 3, mRNA sequence | 41.1 Down | 1.50E-04 |
| AL833166 | Homo sapiens mRNA; cDNA DKFZp686I2118 (from clone DKFZp686I2118) | 39.95 Down | 1.89E-04 |
| NM_173505 | Homo sapiens ankyrin repeat domain 29 (ANKRD29), mRNA | 39.16 Down | 9.13E-05 |
| NM_130435 | Homo sapiens protein tyrosine phosphatase, receptor type, E (PTPRE), transcript variant 2, mRNA | 39.16 Down | 2.54E-04 |
| NM_016356 | Homo sapiens doublecortin domain containing 2 (DCDC2), mRNA | 38.33 Down | 2.10E-04 |
| AF318382 | Homo sapiens pp9974 mRNA, complete cds | 38.03 Down | 8.59E-05 |
| NM_018265 | Homo sapiens hypothetical protein FLJ10901 (FLJ10901), mRNA | 37.23 Down | 1.50E-04 |
| NM_152487 | Homo sapiens hypothetical protein FLJ31842 (FLJ31842), mRNA | 37 Down | 1.56E-04 |
| AK000075 | Homo sapiens cDNA FLJ20068 fis, clone COL01755 | 35.55 Down | 2.21 E-04 |
| NM_012464 | Homo sapiens tolloid-like 1 (TLL1), mRNA | 35.05 Down | 2.09E-03 |
| NM_184087 | Homo sapiens tripartite motif-containing 55 (TRIM55), transcript variant 4, mRNA | 34.38 Down | 7.49E-05 |
| BU680661 | UI-CF-DU1-aaz-f-04-0-UI.s1 UI-CF-DU1 Homo sapiens cDNA clone UI-CF-DU1-aaz-f-04-0-UI 3, mRNA sequence | 34.34 Down | 6.22E-05 |
| BF431030 | 7o18c06.x1 NCI_CGAP_Kid11 Homo sapiens cDNA clone IMAGE:3574283 3, mRNA sequence | 34.25 Down | 1.86E-04 |
| NM_006722 | Homo sapiens microphthalmia-associated transcription factor (MITF), transcript variant 3, mRNA | 34.23 Down | 6.30E-05 |
| NM_152284 | Homo sapiens Snf7 homologue associated with Alix 3 (Shax3), mRNA | 33.99 Down | 7.71 E-05 |
| BC037316 | Homo sapiens, clone IMAGE:5259432, mRNA | 33.46 Down | 2.03E-04 |
| NM_002345 | Homo sapiens lumican (LUM), mRNA | 33.34 Down | 8.46E-05 |
| NM_080743 | Homo sapiens serine-arginine repressor protein (35 kDa) (SRrp35), mRNA | 32.96 Down | 5.23E-05 |
| NM_004221 | Homo sapiens natural killer cell transcript 4 (NK4), mRNA | 32.74 Down | 1.45E-04 |
| NM_004496 | Homo sapiens forkhead box A1 (FOXA1), mRNA | 32.31 Down | 2.57E-04 |
| AA738254 | nx13b02.s1 NCI_CGAP _GC3 Homo sapiens cDNA clone IMAGE:1255947 3, mRNA sequence | 32.23 Down | 6.10E-05 |
| BC045828 | Homo sapiens zinc finger protein 608, mRNA (cDNA clone IMAGE:5262896), partial cds | 32.18 Down | 7.49E-05 |
| NM_000599 | Homo sapiens insulin-like growth factor binding protein 5 (IGFBP5), mRNA | 32.18 Down | 9.81 E-05 |
| AF055376 | Homo sapiens short form transcription factor C-MAF (c-maf) mRNA, complete cds | 31.81 Down | 2.01 E-04 |
| NM_015236 | Homo sapiens latrophilin 3 (LPHN3), mRNA | 31.68 Down | 9.69E-05 |
| NM_007069 | Homo sapiens HRAS-like suppressor 3 (HRASLS3), mRNA | 31.67 Down | 7.49E-05 |
| BG221364 | RST41175 Athersys RAGE Library Homo sapiens cDNA, m RNA sequence | 31.63 Down | 2.22E-04 |
| AI819186 | wj32d10.x1 NCLCGAP_Kid12 Homo sapiens cDNA clone IMAGE:2404531 3, mRNA sequence | 31.49 Down | 1.80E-04 |
| BF512544 | UI-H-BW1-amf-c-08-0-UI.s1 NCI_CGAP_Sub7 Homo sapiens cDNA clone IMAGE:3069687 3, mRNA sequence | 30.8 Down | 7.49E-05 |
| NM_020873 | Homo sapiens leucine rich repeat neuronal 1 (LRRN1), mRNA | 30.67 Down | 2.01 E-04 |
| CA425961 | UI-H-FE1-beg-p-18-0-UI.s1 NCI_CGAP_FE1 Homo sapiens cDNA clone UI_H_FE1_beg_p_18_0_UI 3, mRNA sequence | 30.15 Down | 1.88E-04 |
| AK000776 | Homo sapiens cDNA FLJ20769 fis, clone COL06674 | 30.11 Down | 1.15E-04 |
| NM_001562 | Homo sapiens interleukin 18 (interferon-gamma-inducing factor) (IL18), mRNA | 29.97 Down | 7.65E-04 |
| NM_014333 | Homo sapiens immunoglobulin superfamily, member 4 (IGSF4), mRNA | 29.77 Down | 5.82E-04 |
| NM_030583 | Homo sapiens matrilin 2 (MATN2), transcript variant 2, mRNA | 29.05 Down | 8.46E-05 |
| W93585 | zd95g01.s1 Soares_fetal_heart_NbHH19W Homo sapiens cDNA clone IMAGE:357264 3, mRNA sequence | 28.93 Down | 6.22E-05 |
| NM_012198 | Homo sapiens grancalcin, EF-hand calcium binding protein (GCA), mRNA | 28.84 Down | 9.13E-05 |
| NM_001448 | Homo sapiens glypican 4 (GPC4), mRNA | 28.72 Down | 6.36E-04 |
| AK056882 | Homo sapiens cDNA FLJ32320 fis, clone PROST2003537 | 28.6 Down | 4.45E-04 |
| AB033048 | Homo sapiens mRNA for KIAA1222 protein, partial cds | 28.27 Down | 2.41 E-04 |
| NM_025074 | Homo sapiens Fraser syndrome 1 (FRAS1), transcript variant 1, mRNA | 28.13 Down | 8.92E-05 |
| NM_019000 | Homo sapiens hypothetical protein FLJ20152 (FLJ20152), mRNA | 27.54 Down | 1.07E-04 |
| BU729783 | UI-E-CK1-afh-h-18-0-UI.s1 UI-E-CK1 Homo sapiens cDNA clone UI-E-CK1-afh-h-18-0-UI 3, mRNA sequence | 26.13 Down | 6.30E-05 |
| NM_004862 | Homo sapiens lipopolysaccharide-induced TNF factor (LITAF), mRNA | 25.95 Down | 8.99E-05 |
| AB011539 | Homo sapiens mRNA for MEGF6 protein (KIAA0815), partial cds | 25.67 Down | 3.12E-04 |
| BC043195 | Homo sapiens cDNA clone IMAGE:5288757, partial cds | 25.58 Down | 2.22E-04 |
| NM_152864 | Homo sapiens chromosome 20 open reading frame 58 (C20orf58), mRNA | 25.35 Down | 3.00E-04 |
| AK125608 | Homo sapiens cDNA FLJ43620 fis, clone SPLEN2021701, highly similar to HLA CLASS I HISTOCOMPATIBILITY ANTIGEN, A-2 ALPHA CHAIN PRECURSOR | 25.33 Down | 2.03E-04 |
| AL080103 | Homo sapiens mRNA; cDNA DKFZp564N2216 (from clone DKFZp564N2216) | 25.17 Down | 2.03E-04 |
| NM_173549 | Homo sapiens hypothetical protein FLJ39553 (FLJ39553), mRNA | 24.71 Down | 1.67E-04 |
| BG211832 | RST31404 Athersys RAGE Library Homo sapiens cDNA, mRNA sequence | 24.57 Down | 1.02E-04 |
| NM_024726 | Homo sapiens IQ motif containing with AAA domain (IQCA), mRNA | 24.08 Down | 7.49E-05 |
| NM_002559 | Homo sapiens purinergic receptor P2X, ligand-gated ion channel, 3 (P2RX3), mRNA | 24.04 Down | 2.63E-04 |
| NM_018168 | Homo sapiens chromosome 14 open reading frame 105 (C14orf105). mRNA | 23.69 Down | 1.50E-04 |
| NM_000599 | Homo sapiens insulin-like growth factor binding protein 5 (IGFBP5), mRNA | 23.5 Down | 4.02E-04 |
| NM_003551 | Homo sapiens non-metastatic cells 5, protein expressed in (nucleoside-diphosphate kinase) (NME5), mRNA | 23.23 Down | 1.41E-04 |
| NM_032471 | Homo sapiens protein kinase (cAMP-dependent, catalytic) inhibitor beta (PKIB), transcript variant 3, mRNA | 22.83 Down | 1.54E-04 |
| NM_005949 | Homo sapiens metallothionein 1F (functional) (MT1F), mRNA | 22.22 Down | 8.81 E-05 |
| NM_018242 | Homo sapiens hypothetical protein FLJ10847 (FLJ10847), mRNA | 22.1 Down | 2.03E-04 |
| AW151660 | xf67d04.x1 NCI_CGAP_Gas4 Homo sapiens cDNA clone IMAGE:2623111 3, mRNA sequence | 21.81 Down | 2.71E-04 |
| AK096975 | Homo sapiens cDNA FLJ39656 fis, clone SMINT2005956 | 21.78 Down | 2.52E-04 |
| CA306881 | UI-H-FT1-bht-n-22-0-UI.s1 NCI_CGAP_FTI Homo sapiens cDNA clone UI-H-FT1-bht-n-22-0-UI 3, mRNA sequence | 21.69 Down | 1.30E-04 |
| NM_016212 | Homo sapiens TP53TG3 protein (TP53TG3), mRNA | 21.67 Down | 9.03E-05 |
| NM_178470 | Homo sapiens WD repeat domain 40B (WDR40B), mRNA | 21.63 Down | 7.59E-05 |
| NM_014243 | Homo sapiens a disintegrin-like and metalloprotease (reprolysin type) with thrombospondin type 1 motif, 3 (ADAMTS3), mRNA | 21.47 Down | 8.24E-05 |
| AK126467 | Homo sapiens cDNA FLJ44503 fis, clone UTERU3001158 | 21.42 Down | 6.22E-05 |
| AW248516 | 2820632.3prime NIH_MGC_7 Homo sapiens cDNA clone IMAGE:2820632 3, mRNA sequence | 21.37 Down | 1.23E-04 |
| NM_001263 | Homo sapiens CDP-diacylglycerol synthase (phosphatidate cytidylyltransferase) 1 (CDS1), mRNA | 21.12 Down | 1.10E-04 |
| BM669002 | UI-E-CK1-afn-m-04-0-UI.s2 UI-E-CK1 Homo sapiens cDNA clone UI-E-CK1-afn-m-04-0-UI 3, mRNA sequence | 20.96 Down | 7.28E-05 |
| NM_000170 | Homo sapiens glycine dehydrogenase (decarboxylating; glycine decarboxylase, glycine cleavage system protein P) (GLDC), mRNA | 20.78 Down | 1.19E-04 |
| NM_014585 | Homo sapiens solute carrier family 40 (iron-regulated transporter), member 1 (SLC40A1), mRNA | 20.73 Down | 8.46E-05 |
| NM_031426 | Homo sapiens chromosome 9 open reading frame 58 (C9orf58), transcript variant 1, mRNA | 20.62 Down | 2.00E-04 |
| BM728728 | UI-E-EO1-aiv-c-02-0-UI.r1 UI-E-EO1 Homo sapiens cDNA clone UI-E-EO1-aiv-c-02-0-UI 5, mRNA sequence | 20.59 Down | 1.14E-04 |
| NM_152369 | Homo sapiens hypothetical protein MGC45474 (MGC45474), mRNA | 20.57 Down | 9.78E-05 |
| NM_175056 | Homo sapiens hypothetical protein LOC131368 (LOC131368), mRNA | 20.51 Down | 5.42E-04 |
| NM_004524 | Homo sapiens lethal giant larvae homolog 2 (Drosophila) (LLGL2), mRNA | 20.3 Down | 7.45E-05 |
| AI436290 | th81c01.x1 Soares_NhHMPu_S1 Homo sapiens cDNA clone IMAGE:2125056 3, mRNA sequence | 19.93 Down | 1.23E-04 |
| AW268540 | xv51e10.x1 NCI_CGAP_Lu28 Homo sapiens cDNA clone IMAGE:2816682 3, mRNA sequence | 19.89 Down | 1.09E-04 |
| BC015159 | Homo sapiens cDNA clone IMAGE:3885734, partial cds | 19.47 Down | 7.49E-05 |
| NM_020130 | Homo sapiens chromosome 8 open reading frame 4 (C8orf4), mRNA | 19.43 Down | 2.83E-04 |
| BM979825 | UI-CF-DU1-adt-f-12-0-UI.s1 UI-CF-DU1 Homo sapiens cDNA clone UI-CF-DU1-adt-f-12-0-UI 3, mRNA sequence | 19.41 Down | 1.01 E-04 |
| BM712072 | UI-E-DW1-ahc-b-11-0-UI.r1 UI-E-DW1 Homo sapiens cDNA clone UI-E-DW1-ahc-b-11-0-UI 5, mRNA sequence | 19.33 Down | 1.19E-04 |
| CB135276 | K-EST0187371 L5HLK1 Homo sapiens cDNA clone L5HLK1-32-B12 5, mRNA sequence | 18.98 Down | 8.97E-05 |
| T53523 | ya89h12.r1 Stratagene placenta (#937225) Homo sapiens cDNA clone IMAGE:68903 5, mRNA sequence | 18.87 Down | 1.96E-04 |
| NM_138432 | Homo sapiens serine dehydratase-like (SDSL), mRNA | 18.84 Down | 1.32E-04 |
| NM_023915 | Homo sapiens G protein-coupled receptor 87 (GPR87), mRNA | 18.74 Down | 2.72E-04 |
| NM_017549 | Homo sapiens upregulated in colorectal cancer gene 1 (UCC1), mRNA | 18.73 Down | 8.83E-05 |
| BG436244 | 602508665F1 NIH_MGC_79 Homo sapiens cDNA clone IMAGE:460561 5, mRNA sequence | 18.7 Down | 1.49E-04 |
| NM_024901 | Homo sapiens hypothetical protein FLJ22457 (FLJ22457), mRNA | 18.68 Down | 1.35E-04 |
| NM_005712 | Homo sapiens HERV-H LTR-associating 1 (HHLA1), mRNA | 18.63 Down | 1.08E-04 |
| NM_198488 | Homo sapiens FLJ46072 protein (FLJ46072), mRNA | 18.58 Down | 7.49E-05 |
| NM_020349 | Homo sapiens ankyrin repeat domain 2 (stretch responsive muscle) (ANKRD2), mRNA | 18.49 Down | 2.00E-04 |
| NM_002148 | Homo sapiens homeo box D10 (HOXD10), mRNA | 18.41 Down | 3.05E-05 |
| N78460 | yz76h06.r1 Soares_multiple_sclerosis_2NbHMS P Homo sapiens cDNA clone lMAGE:289019 5, mRNA sequence | 18.4 Down | 4.02E-04 |
| NM_006598 | Homo sapiens solute carrier family 12 (potassium/chloride transporters), member 7 (SLC12A7), mRNA | 18.13 Down | 8.59E-05 |
| BF431041 | nab31g02.x1 Soares_NSF_F8_9W_OT_PA_P_S1 Homo sapiens cDNA clone IMAGE:3267627 3, mRNA sequence | 18.11 Down | 6.10E-05 |
| AK126467 | Homo sapiens cDNA FLJ44503 fis, clone UTERU3001158 | 17.84 Down | 1.31 E-04 |
| NM_182487 | Homo sapiens olfactomedin-like 2A (OLFML2A), mRNA | 17.77 Down | 1.06E-04 |
| AK058012 | Homo sapiens cDNA FLJ25283 fis, clone STM06716 | 17.76 Down | 8.82E-05 |
| NM_001252 | Homo sapiens tumor necrosis factor (ligand) superfamily, member 7 (TNFSF7), mRNA | 17.71 Down | 2.46E-04 |
| NM_006252 | Homo sapiens protein kinase, AMP-activated, alpha 2 catalytic subunit (PRKAA2), mRNA | 17.63 Down | 6.57E-04 |
| NM_030899 | Homo sapiens zinc finger protein 323 (ZNF323), mRNA | 17.62 Down | 1.77E-04 |
| NM_006722 | Homo sapiens microphthalmia-associated transcription factor (MITF), transcript variant 3, mRNA | 17.53 Down | 1.61 E-04 |
| AK056431 | Homo sapiens cDNA FLJ31869 fis, clone NT2RP7002151 | 17.29 Down | 8.46E-05 |
| BM719937 | UI-E-EJO-ahu-a-10-0-UI.r1 UI-E-EJO Homo sapiens cDNA clone UI-E-EJ0-ahu-a-10-0-UI 5, mRNA sequence | 17.11 Down | 4.37E-05 |
| NM_005855 | Homo sapiens receptor (calcitonin) activity modifying protein 1 (RAMP1), mRNA | 16.97 Down | 1.56E-04 |
| AK124873 | Homo sapiens cDNA FLJ42883 fis, clone BRHIP3006683 | 16.87 Down | 3.99E-04 |
| NM_001977 | Homo sapiens glutamyl aminopeptidase (aminopeptidase A) (ENPEP), mRNA | 16.61 Down | 6.12E-04 |
| AK074181 | Homo sapiens mRNA for FLJ00254 protein | 16.54 Down | 6.10E-05 |
| NM_000557 | Homo sapiens growth differentiation factor 5 (cartilage-derived morphogenetic protein-1) (GDF5), mRNA | 16.47 Down | 1.49E-04 |
| AK026966 | Homo sapiens cDNA: FLJ23313 fis, clone HEP11919 | 16.36 Down | 8.92E-05 |
| BX113319 | BX113319 NCI_CGAP_Gas4 Homo sapiens cDNA clone IMAGp998G205398 ; IMAGE:2184619, mRNA sequence | 16.19 Down | 6.30E-05 |
| AK055362 | Homo sapiens cDNA FLJ30800 fis, clone FEBRA2001197 | 16.02 Down | 3.05E-05 |
| NM_021102 | Homo sapiens serine protease inhibitor, Kunitz type, 2 (SPINT2), mRNA | 15.94 Down | 2.71 E-04 |
| NM_002354 | Homo sapiens tumor-associated calcium signal transducer 1 (TACSTD1), mRNA | 15.93 Down | 9.13E-05 |
| BQ003401 | UI-H-EI1-azd-j-23-0-UI.s1 NCI_CGAP_EI1 Homo sapiens cDNA clone IMAGE:5847286 3, m RNA sequence | 15.78 Down | 6.30E-05 |
| NM_033641 | Homo sapiens collagen, type IV, alpha 6 (COL4A6), transcript variant B, mRNA | 15.77 Down | 5.35E-05 |
| N38753 | yy42d01.s1 Soares melanocyte 2NbHM Homo sapiens cDNA clone IMAGE:273889 3, mRNA sequence | 15.77 Down | 3.67E-05 |
| AI420213 | te92g09.x1 NCI_CGAP_Pr28 Homo sapiens cDNA clone IMAGE:2094208 3, mRNA sequence | 15.7 Down | 2.35E-04 |
| AK095776 | Homo sapiens cDNA FLJ38457 fis, clone FEBRA2020400 | 15.48 Down | 3.04E-04 |
| NM_006378 | Homo sapiens sema domain, immunoglobulin domain (lg), transmembrane domain (TM) and short cytoplasmic domain, (semaphorin) 4D (SEMA4D), mRNA | 15.33 Down | 6.10E-05 |
| AK021801 | Homo sapiens cDNA FLJ11739 fis, clone HEMBA1005497 | 15.33 Down | 1.38E-04 |
| BX538226 | Homo sapiens mRNA; cDNA DKFZp686E1944 (from clone DKFZp686E1944) | 15.23 Down | 2.37E-04 |
| NM_001847 | Homo sapiens collagen, type IV, alpha 6 (COL4A6), transcript variant A, mRNA | 15.22 Down | 7.42E-05 |
| NM_017640 | Homo sapiens leucine rich repeat containing 16 (LRRC16), mRNA | 15.21 Down | 6.39E-05 |
| NM_007072 | Homo sapiens HERV-H LTR-associating 2 (HHLA2), mRNA | 15.04 Down | 1.06E-04 |
| NM_052947 | Homo sapiens heart alpha-kinase (HAK), mRNA | 14.96 Down | 2.23E-04 |
| NM_005139 | Homo sapiens annexin A3 (ANXA3), mRNA | 14.95 Down | 6.71 E-05 |
| N63415 | yy60d04.s1 Soares_multiple_sclerosis_2NbHMS P Homo sapiens cDNA clone IMAGE:277927 3 similar to contains L1.b3 L1 repetitive element,, mRNA sequence | 14.79 Down | 2.23E-05 |
| BC052289 | Homo sapiens carboxypeptidase A4, mRNA (cDNA clone MGC:59749 IMAGE:6106874), complete cds | 14.76 Down | 7.22E-05 |
| NM_153715 | Homo sapiens homeo box A10 (HOXA10), transcript variant 2, mRNA | 14.76 Down | 8.35E-05 |
| NM_014936 | Homo sapiens ectonucleotide pyrophosphatase/phosphodiesteras e 4 (putative function) (ENPP4), mRNA | 14.67 Down | 8.19E-04 |
| NM_021192 | Homo sapiens homeo box D11 (HOXD11), mRNA | 14.47 Down | 4.53E-04 |
| BX118238 | BX118238 NCI_CGAP_Kid3 Homo sapiens cDNA clone IMAGp998L153800 ; IMAGE:1501598, mRNA sequence | 14.44 Down | 5.07E-04 |
| NM_024969 | Homo sapiens TGF-beta induced apotosis protein 2 (TAIP-2), mRNA | 14.11 Down | 1.57E-04 |
| AK125490 | Homo sapiens cDNA FLJ43501 fis, clone PEBLM2004497 | 14.06 Down | 9.71 E-04 |
| R99527 | yq79b11.s1 Soares fetal liver spleen 1 NFLS Homo sapiens cDNA clone IMAGE:201981 3, mRNA sequence | 13.98 Down | 8.39E-04 |
| NM_001873 | Homo sapiens carboxypeptidase E (CPE), mRNA | 13.91 Down | 9.16E-05 |
| NM_003761 | Homo sapiens vesicle-associated membrane protein 8 (endobrevin) (VAMP8), mRNA | 13.87 Down | 3.12E-04 |
| NM_020808 | Homo sapiens signal-induced proliferation-associated 1 like 2 (SIPA1L2), mRNA | 13.84 Down | 5.47E-04 |
| NM_152573 | Homo sapiens RAS and EF hand domain containing (RASEF), mRNA | 13.82 Down | 5.13E-04 |
| NM_032866 | Homo sapiens cingulin-like 1 (CGNL1), mRNA | 13.78 Down | 6.30E-05 |
| BU740051 | UI-E-EO0-ahw-n-18-0-UI.s1 UI-E-EO0 Homo sapiens cDNA clone UI-E-EO0-ahw-n-18-0-UI 3, mRNA sequence | 13.74 Down | 4.63E-04 |
| NM_198389 | Homo sapiens lung type-I cell membrane-associated glycoprotein (T1A-2), transcript variant 2, mRNA | 13.71 Down | 3.62E-04 |
| CA777268 | ip05d09.y1 HR85 islet Homo sapiens cDNA clone IMAGE:6134849 5, mRNA sequence | 13.63 Down | 1.39E-04 |
| H00617 | yj25f02.s1 Soares placenta Nb2HP Homo sapiens cDNA clone IMAGE:149787 3, mRNA sequence | 13.45 Down | 3.33E-04 |
| NM_024898 | Homo sapiens family with sequence similarity 31, member C (FAM31C), mRNA | 13.39 Down | 2.17E-03 |
| NM_000094 | Homo sapiens collagen, type VII, alpha 1 (epidermolysis bullosa, dystrophic, dominant and recessive) (COL7A1),mRNA | 13.3 Down | 4.93E-04 |
| NM_002837 | Homo sapiens protein tyrosine phosphatase, receptor type, B (PTPRB), mRNA | 13.25 Down | 6.84E-04 |
| NM_018728 | Homo sapiens myosin VC (MY05C), mRNA | 13.15 Down | 1.64E-04 |
| NM_018659 | Homo sapiens cytokine-like protein C17 (C17), mRNA | 13.11 Down | 1.45E-04 |
| AL834140 | Homo sapiens mRNA; cDNA DKFZp434A2029 (from clone DKFZp434A2029) | 13.08 Down | 9.99E-05 |
| BX103476 | BX103476 NCI_CGAP_Lu5 Homo sapiens cDNA clone IMAGp998C053946 ; IMAGE:1557436, mRNA sequence | 13.02 Down | 1.71 E-04 |
| BG545305 | 602572521F1 NIH_MGC_77 Homo sapiens cDNA clone IMAGE:4700644 5, mRNA sequence | 12.86 Down | 6.30E-05 |
| BX647876 | Homo sapiens mRNA; cDNA DKFZp313A1525 (from clone DKFZp313A1525) | 12.83 Down | 7.95E-05 |
| NM_030949 | Homo sapiens protein phosphatase 1, regulatory (inhibitor) subunit 14C (PPP1R14C), mRNA | 12.82 Down | 3.35E-04 |
| NM_024423 | Homo sapiens desmocollin 3 (DSC3), transcript variant Dsc3b, mRNA | 12.81 Down | 7.71 E-05 |
| NM_014452 | Homo sapiens tumor necrosis factor receptor superfamily, member 21 (TNFRSF21), mRNA | 12.8 Down | 2.20E-04 |
| NM_002246 | Homo sapiens potassium channel, subfamily K, member 3 (KCNK3), mRNA | 12.76 Down | 7.59E-05 |
| NM_002427 | Homo sapiens matrix metalloproteinase 13 (collagenase 3) (MMP13), mRNA | 12.65 Down | 4.08E-04 |
| NM_003328 | Homo sapiens TXK tyrosine kinase (TXK), mRNA | 12.61 Down | 2.45E-04 |
| NM_014422 | Homo sapiens phosphatidylinositol (4,5) bisphosphate 5-phosphatase, A (PIB5PA), transcript variant 1, mRNA | 12.48 Down | 8.97E-05 |
| NM_014333 | Homo sapiens immunoglobulin superfamily, member 4 (IGSF4), mRNA | 12.4 Down | 1.04E-04 |
| AA888443 | nw74f10.s1 NCI_CGAP_Pr12 Homo sapiens cDNA clone IMAGE:1252363, mRNA sequence | 12.37 Down | 3.96E-04 |
| BX110418 | BX110418 NCI_CGAP_Kid3 Homo sapiens cDNA clone IMAGp998C224149 ; IMAGE:1635405, mRNA sequence | 12.35 Down | 1.54E-04 |
| NM_019102 | Homo sapiens homeo box A5 (HOXA5), mRNA | 12.33 Down | 7.59E-05 |
| NM_016463 | Homo sapiens CXXC finger 5 (CXXC5), mRNA | 12.32 Down | 1.96E-04 |
| NM_004572 | Homo sapiens plakophilin 2 (PKP2), transcript variant 2b, mRNA | 12.28 Down | 4.15E-04 |
| N25875 | yw78d12.s1 Soares_placenta_8to9weeks_2NbH P8to9W Homo sapiens cDNA clone IMAGE:258359 3, mRNA sequence | 12.27 Down | 1.02E-04 |
| NM_152694 | Homo sapiens zinc finger, CCHC domain containing 5 (ZCCHC5), mRNA | 12.22 Down | 3.38E-04 |
| NM_021012 | Homo sapiens potassium inwardly-rectifying channel, subfamily J, member 12 (KCNJ12), mRNA | 12.22 Down | 9.23E-04 |
| NM_017671 | Homo sapiens chromosome 20 open reading frame 42 (C20orf42), mRNA | 12.21 Down | 1.06E-04 |
| NM_002031 | Homo sapiens fyn-related kinase (FRK), mRNA | 12.11 Down | 2.14E-04 |
| BM976385 | UI-CF-EN1-acz-f-03-0-UI.s1 UI-CF-EN1 Homo sapiens cDNA clone UI-CF-EN1-acz-f-03-0-UI 3, mRNA sequence | 12.02 Down | 8.11E-04 |
| NM_005296 | Homo sapiens G protein-coupled receptor 23 (GPR23), mRNA | 11.98 Down | 1.50E-04 |
| NM_000817 | Homo sapiens glutamate decarboxylase 1 (brain, 67kDa) (GAD1), transcript variant GAD67, mRNA | 11.92 Down | 6.22E-05 |
| AI220066 | qg84d01.x1 Soares_NFL_T_GBC_S1 Homo sapiens cDNA clone IMAGE:1841857 3, mRNA sequence | 11.88 Down | 1.09E-04 |
| NM_005737 | Homo sapiens ADP-ribosylation factor-like 7 (ARL7), mRNA | 11.83 Down | 1.58E-04 |
| NM_000147 | Homo sapiens fucosidase, alpha-L-1, tissue (FUCA1), mRNA | 11.82 Down | 1.74E-03 |
| AK127437 | Homo sapiens cDNA FLJ45529 fis, clone BRTHA2027546 | 11.82 Down | 1.68E-04 |
| NM_178033 | Homo sapiens cytochrome P450, family 4, subfamily X, polypeptide 1 (CYP4X1), mRNA | 11.81 Down | 2.10E-04 |
| NM_173567 | Homo sapiens abhydrolase domain containing 7 (ABHD7), mRNA | 11.76 Down | 1.75E-04 |
| AL049974 | Homo sapiens mRNA; cDNA DKFZp564B222 (from clone DKFZp564B222) | 11.74 Down | 5.82E-04 |
| NM_000519 | Homo sapiens hemoglobin, delta (HBD), mRNA | 11.7 Down | 9.70E-04 |
| AL359567 | Homo sapiens mRNA; cDNA DKFZp547D023 (from clone DKFZp547D023) | 11.66 Down | 3.95E-04 |
| BF510493 | UI-H-BI4-apa-b-08-0-UI.s1 NCI_CGAP_Sub8 Homo sapiens cDNA clone IMAGE:3086558 3, mRNA sequence | 11.66 Down | 6.30E-05 |
| NM_000961 | Homo sapiens prostaglandin 12 (prostacyclin) synthase (PTGIS), mRNA | 11.63 Down | 5.06E-04 |
| NM_025151 | Homo sapiens RAB11 family interacting protein 1 (class I) (RAB11FIP1 transcript variant 1, mRNA | 11.6 Down | 1.14E-04 |
| BM712945 | UI-E-EJO-ahi-c-16-0-UI.r1 UI-E-EJ0 Homo sapiens cDNA clone UI-E-EJ0-ahi-c-16-0-UI 5, mRNA sequence | 11.6 Down | 9.48E-05 |
| AW451831 | UI-H-B13-alk-e-12-0-UI.s1 NCI_CGAP_Sub5 Homo sapiens cDNA clone IMAGE:2737246 3, mRNA sequence | 11.54 Down | 8.69E-04 |
| BC040701 | Homo sapiens cDNA clone IMAGE:5736259, partial cds | 11.53 Down | 1.23E-04 |
| AL359058 | Homo sapiens mRNA full length insert cDNA clone EUROIMAGE 592473 | 11.44 Down | 1.27E-03 |
| NM_001854 | Homo sapiens collagen, type XI, alpha 1 (COL11A1), transcript variant A, mRNA | 11.39 Down | 7.56E-04 |
| NM_000227 | Homo sapiens laminin, alpha 3 (LAMA3), transcript variant 2, mRNA | 11.39 Down | 8.59E-05 |
| NM_033256 | Homo sapiens protein phosphatase 1, regulatory (inhibitor) subunit 14A (PPP1R14A), mRNA | 11.36 Down | 1.26E-04 |
| AB011538 | Homo sapiens mRNA for MEGF5, partial cds | 11.35 Down | 4.81 E-04 |
| AL389942 | Homo sapiens mRNA full length insert cDNA clone EUROIMAGE 2005635 | 11.33 Down | 4.78E-04 |
| NM_174900 | Homo sapiens zinc finger protein 42 (ZFP42), mRNA | 11.23 Down | 1.29E-04 |
| BG622707 | 602647476F1 NIH_MGG_79 Homo sapiens cDNA clone IMAGE:4768963 5, mRNA sequence | 11.21 Down | 1.49E-04 |
| NM_178177 | Homo sapiens nicotinamide nucleotide adenylyltransferase 3 (NMNAT3), mRNA | 11.19 Down | 3.57E-04 |
| AA099748 | z178c09.s1 Stratagene colon (#937204) Homo sapiens cDNA clone IMAGE:510736 3, mRNA sequence | 11.16 Down | 9.87E-05 |
| H89526 | yw28b04.r1 Morton Fetal Cochlea Homo sapiens cDNA clone IMAGE:253519 5, mRNA sequence | 11.06 Down | 2.94E-04 |
| NM_178868 | Homo sapiens chemokine-like factor super family 8 (CKLFSF8), mRNA | 10.96 Down | 9.23E-04 |
| BE788763 | 601475864F1 NIH_MGC_68 Homo sapiens cDNA clone IMAGE:3879014 5, mRNA sequence | 10.88 Down | 6.22E-05 |
| NM_000076 | Homo sapiens cyclin-dependent kinase inhibitor 1C (p57, Kip2) (CDKN1C), mRNA | 10.87 Down | 7.28E-05 |
| NM_152768 | Homo sapiens hypothetical protein FLJ25378 (FLJ25378), mRNA | 10.81 Down | 7.31 E-05 |
| M60502 | Human profilaggrin mRNA, 3 end | 10.81 Down | 8.46E-05 |
| NM_181847 | Homo sapiens amphoterin induced gene 2 (AMIGO2), mRNA | 10.78 Down | 1.86E-03 |
| NM_005331 | Homo sapiens hemoglobin, theta 1 (HBQ1), mRNA | 10.77 Down | 3.32E-04 |
| NM_032367 | Homo sapiens zinc finger, BED domain containing 3 (ZBED3), mRNA | 10.61 Down | 7.71 E-05 |
| NM_004574 | Homo sapiens peanut-like 2 (Drosophila) (PNUTL2), transcript variant 1, mRNA | 10.6 Down | 3.18E-04 |
| NM_014421 | Homo sapiens dickkopf homolog 2 (Xenopus laevis) (DKK2), mRNA | 10.52 Down | 7.31 E-05 |
| NM_052923 | Homo sapiens zinc finger protein 452 (ZNF452), mRNA | 10.43 Down | 1.06E-04 |
| NM_006379 | Homo sapiens sema domain, immunoglobulin domain (lg), short basic domain, secreted, (semaphorin) 3C (SEMA3C), mRNA | 10.39 Down | 6.30E-05 |
| AL137488 | Homo sapiens mRNA; cDNA DKFZp434N2030 (from clone DKFZp434N2030) | 10.37 Down | 2.60E-04 |
| BX088936 | BX088936 Soares_testis_NHT Homo sapiens cDNA clone IMAGp998G123255 IMAGE:1292195, mRNA sequence | 10.34 Down | 1.20E-03 |
| AB041269 | Homo sapiens mRNA for keratin 19, partial cds, isolate:K19-141 | 10.33 Down | 4.02E-03 |
| AK091933 | Homo sapiens cDNA FLJ34614 fis, clone KIDNE2014268 | 10.3 Down | 1.49E-04 |
| AF269162 | Homo sapiens c21orf7 form B mRNA, complete cds | 10.3 Down | 1.78E-03 |
| NM_001935 | Homo sapiens dipeptidylpeptidase 4 (CD26, adenosine deaminase complexing protein 2) (DPP4), mRNA | 10.27 Down | 1.10E-04 |
| NM_001850 | Homo sapiens collagen, type VIII, alpha 1 (COL8A1), transcript variant 1, mRNA | 10.23 Down | 2.23E-05 |
| AI469032 | ti70a01.x1 NCI_CGAP_Kid11 Homo sapiens cDNA clone IMAGE:2137320 3, mRNA sequence | 10.2 Down | 7.42E-05 |
| NM_003081 | Homo sapiens synaptosomal-associated protein, 25kDa (SNAP25), transcript variant 1, mRNA | 10.05 Down | 2.34E-03 |
| BG570144 | 602591134F1 NIH_MGC_77 Homo sapiens cDNA clone IMAGE:4717761 5, mRNA sequence | 9.99 Down | 1.09E-03 |
| BM969191 | UI-CF-ENO-acp-e-22-0-UI.s1 UI-CF-EN0 Homo sapiens cDNA clone UI-CF-ENO-acp-e-22-0-UI 3, mRNA sequence | 9.99 Down | 1.00E-03 |
| NM_006622 | Homo sapiens polo-like kinase 2 (Drosophila) (PLK2), mRNA | 9.99 Down | 1.45E-04 |
| NM_000860 | Homo sapiens hydroxyprostaglandin dehydrogenase 15-(NAD) (HPGD), mRNA | 9.98 Down | 6.39E-05 |
| AJ318805 | AJ318805 Homo sapiens adipose tissue Homo sapiens cDNA clone 2040, mRNA sequence | 9.97 Down | 1.92E-04 |
| NM_002735 | Homo sapiens protein kinase, cAMP-dependent, regulatory, type I, beta (PRKAR1B), mRNA | 9.93 Down | 8.51 E-06 |
| AK092114 | Homo sapiens cDNA FLJ34795 fis, clone NT2NE2005921 | 9.91 Down | 1.57E-03 |
| NM_024608 | Homo sapiens nei endonuclease Vill-like 1 (E. coli) (NEIL1), mRNA | 9.89 Down | 3.05E-05 |
| BE464407 | hx89g05.x1 NCI_CGAP_Kid11 Homo sapiens cDNA clone IMAGE:3195032 3, mRNA sequence | 9.88 Down | 1.05E-03 |
| BF431460 | 7o14b05.x1 NCI_CGAP_Kid11 Homo sapiens cDNA clone IMAGE:3573849 3, mRNA sequence | 9.85 Down | 1.19E-03 |
| BF509573 | UI-H-B14-apf-b-11-0-UI.s1 NCI_CGAP_Sub8 Homo sapiens cDNA clone IMAGE:3086949 3, mRNA sequence | 9.82 Down | 2.57E-04 |
| T78754 | yd01f08.r1 Soares infant brain 1NIB Homo sapiens cDNA clone IMAGE:24180 5, mRNA sequence | 9.79 Down | 1.51E-03 |
| NM_153256 | Homo sapiens chromosome 10 open reading frame 47 (C10orf47), mRNA | 9.78 Down | 2.10E-04 |
| NM_207482 | Homo sapiens FLJ44048 protein (FLJ44048), mRNA | 9.76 Down | 2.14E-04 |
| NM_002276 | Homo sapiens keratin 19 (KRT19), mRNA | 9.74 Down | 3.70E-03 |
| N70752 | za72d02.s1 Soares_fetal_lung_NbHL19W Homo sapiens cDNA clone IMAGE:298083 3, mRNA sequence | 9.73 Down | 1.49E-04 |
| NM_020962 | Homo sapiens likely ortholog of mouse neighbor of Punc E11 (NOPE), mRNA | 9.73 Down | 8.91 E-04 |
| NM_016276 | Homo sapiens serum/glucocorticoid regulated kinase 2 (SGK2), transcript variant 2, mRNA | 9.7 Down | 4.41 E-04 |
| AI355761 | qt94a11.x1 NCI_CGAP_Co14 Homo sapiens cDNA clone IMAGE:1962908 3 similar to gb:X74929 KERATIN, TYPE II CYTOSKELETAL 8 (HUMAN);, mRNA sequence | 9.7 Down | 9.99E-05 |
| NM_003264 | Homo sapiens toll-like receptor 2 (TLR2), mRNA | 9.69 Down | 1.86E-04 |
| NM_198495 | Homo sapiens CTAGE family, member 4 (CTAGE4), mRNA | 9.66 Down | 3.12E-04 |
| BX097888 | BX097888 Soares_parathyroid_tumor_NbHPA Homo sapiens cDNA clone IMAGp998K064187; IMAGE:1650173, mRNA sequence | 9.62 Down | 6.10E-05 |
| BU567804 | AGENCOURT_10398872 NIH_MGC_82 Homo sapiens cDNA clone IMAGE:6614502 5, mRNA sequence | 9.62 Down | 7.71 E-05 |
| NM_152423 | Homo sapiens melanoma associated antigen (mutated) 1-like 1 (MUM1L1), mRNA | 9.61 Down | 2.45E-03 |
| NM_018330 | Homo sapiens KIAA1598 (KIAA1598), mRNA | 9.6 Down | 1.68E-04 |
| BU584197 | 2513030T6 LIVRTUT04 Homo sapiens cDNA clone 2513030 3, mRNA sequence | 9.56 Down | 8.83E-04 |
| NM_005264 | Homo sapiens GDNF family receptor alpha 1 (GFRA1), transcript variant 1, m RNA | 9.56 Down | 1.19E-04 |
| NM_002245 | Homo sapiens potassium channel, subfamily K, member 1 (KCNK1), mRNA | 9.53 Down | 2.05E-03 |
| NM_178550 | Homo sapiens hypothetical protein MGC48998 (MGC48998), mRNA | 9.51 Down | 9.03E-04 |
| NM_006863 | Homo sapiens leukocyte immunoglobulin-like receptor, subfamily A (with TM domain), member 1 (LILRA1), mRNA | 9.48 Down | 6.22E-05 |
| NM_173354 | Homo sapiens SNF1-like kinase (SNF1LK),mRNA | 9.47 Down | 8.25E-04 |
| BC016962 | Homo sapiens, clone IMAGE:4182947, mRNA | 9.43 Down | 2.15E-04 |
| AB046810 | Homo sapiens mRNA for KIAA1590 protein, partial cds | 9.38 Down | 6.30E-05 |
| BX116347 | BX116347 NCI_CGAP_Kid12 Homo sapiens cDNA clone IMAGp998B215967 IMAGE:2401844, mRNA sequence | 9.33 Down | 1.54E-04 |
| BU633163 | UI-H-FL1-bgt-n-07-0-UI.s1 NCI_CGAP_FL1 Homo sapiens cDNA clone UI-H-FL1-bgt-n-07-0-UI 3, mRNA sequence | 9.31 Down | 1.46E-04 |
| AI289329 | qw28c09.x1 NCI_CGAP_Ut4 Homo sapiens cDNA clone IMAGE:1992400 3 similar to contains L1.b2 L1 repetitive element ;, mRNA sequence | 9.28 Down | 4.80E-04 |
| BU951469 | in60a05.x3 HR85 islet Homo sapiens cDNA clone IMAGE:6126249 3, mRNA sequence | 9.21 Down | 2.54E-03 |
| NM_002338 | Homo sapiens limbic system-associated membrane protein (LSAMP), mRNA | 9.19 Down | 1.30E-03 |
| AL833346 | Homo sapiens mRNA; cDNA DKFZp686M2234 (from clone DKFZp686M2234) | 9.17 Down | 3.02E-04 |
| BC040293 | Homo sapiens, clone IMAGE:4820330, mRNA | 9.1 Down | 1.09E-03 |
| NM_003810 | Homo sapiens tumor necrosis factor | 9.04 Down | 5.06E-04 |
| | (ligand) superfamily, member 10 | | |
| | (TNFSF10), mRNA | | |
| NM_014373 | Homo sapiens G protein-coupled | 9.01 Down | 1.59E-04 |
| | receptor 160 (GPR160), mRNA | | |
| CA425405 | UI-H-FE1-bef-g-08-0-UI.s1 | 8.89 Down | 1.74E-05 |
| | NCI_CGAP_FE1 Homo sapiens | | |
| | cDNA clone UI-H-FE1-bef-g-08-0-UI | | |
| | 3, mRNA sequence | | |
| A1953708 | wq47d09.x1 NCI_CGAP_GC6 Homo | 8.87 Down | 9.89E-03 |
| | sapiens cDNA clone | | |
| | IMAGE:2474417 3, mRNA sequence | | |
| NM_017641 | Homo sapiens kinesin family | 8.86 Down | 7.22E-05 |
| | member 21A (KIF21A), mRNA | | |
| NM_153377 | Homo sapiens leucine-rich repeats | 8.86 Down | 2.71 E-04 |
| | and immunoglobulin-like domains 3 | | |
| | (LRIG3), mRNA | | |
| NM_000236 | Homo sapiens lipase, hepatic | 8.83 Down | 1.93E-03 |
| | (LIPC), mRNA | | |
| BC046362 | Homo sapiens voltage-dependent | 8.8 Down | 4.71 E-04 |
| | calcium channel gamma subunit-like | | |
| | protein, mRNA (cDNA clone | | |
| | MGC:50757 IMAGE:5221396), | | |
| | complete cds | | |
| AJ697972 | Homo sapiens chromosome 3 cDNA | 8.8 Down | 7.35E-04 |
| BF509074 | UI-H-B14-aou-b-08-0-UI.s1 | 8.8 Down | 1.24E-04 |
| | NCI_CGAP_Sub8 Homo sapiens | | |
| | cDNA clone IMAGE:3086150 3, | | |
| | mRNA sequence | | |
| NM_001080 | Homo sapiens aldehyde | 8.79 Down | 8.46E-05 |
| | dehydrogenase 5 family, member A1 | | |
| | (succinate-semialdehyde | | |
| | dehydrogenase) (ALDH5A1), | | |
| | nuclear gene encoding mitochondrial | | |
| | protein, transcript variant 2, mRNA | | |
| NM_002247 | Homo sapiens potassium large | 8.78 Down | 7.45E-05 |
| | conductance calcium-activated | | |
| | channel, subfamily M, alpha member | | |
| | 1 (KCNMA1), mRNA | | |
| NM_022154 | Homo sapiens solute carrier family | 8.77 Down | 4.65E-05 |
| | 39 (zinc transporter), member 8 | | |
| | (SLC39A8), mRNA | | |
| NM_144707 | Homo sapiens prominin 2 (PROM2), | 8.73 Down | 2.01 E-04 |
| | mRNA | | |
| AB095949 | Homo sapiens mRNA for KIAA2029 | 8.69 Down | 9.23E-04 |
| | protein | | |
| AV728294 | AV728294 HTC Homo sapiens | 8.68 Down | 8.46E-05 |
| | cDNA clone HTCBIE09 5, mRNA | | |
| | sequence | | |
| NM_003851 | Homo sapiens cellular repressor of | 8.68 Down | 2.10E-04 |
| | E1A-stimulated genes 1 (CREG1), | | |
| | mRNA | | |
| NM_014181 | Homo sapiens HSPC159 protein | 8.67 Down | 1.10E-04 |
| | (HSPC159), mRNA | | |
| NM_000349 | Homo sapiens steroidogenic acute | 8.66 Down | 1.03E-04 |
| | regulator (STAR), nuclear gene | | |
| | encoding mitochondrial protein, | | |
| | transcript variant 1, mRNA | | |
| AK026740 | Homo sapiens cDNA: FLJ23087 fis, | 8.65 Down | 7.45E-04 |
| | clone LNG06994, highly similar to | | |
| | AF161368 Homo sapiens HSPC105 | | |
| | mRNA | | |
| T56535 | yb33g07.r1 Stratagene fetal spleen | 8.63 Down | 1.23E-04 |
| | (#937205) Homo sapiens cDNA | | |
| | clone IMAGE:73020 5, mRNA | | |
| | sequence | | |
| NM_178868 | Homo sapiens chemokine-like factor | 8.63 Down | 6.22E-05 |
| | super family 8 (CKLFSF8), mRNA | | |
| NM_005130 | Homo sapiens fibroblast growth | 8.62 Down | 1.15E-04 |
| | factor binding protein 1 (FGFBP1), | | |
| | mRNA | | |
| NM_032488 | Homo sapiens cornifelin (CNFN), | 8.6 Down | 3.05E-05 |
| | mRNA | | |
| NM_052960 | Homo sapiens retinol binding protein | 8.6 Down | 2.05E-04 |
| | 7, cellular (RBP7), mRNA | | |
| NM_207517 | Homo sapiens ADAMTS-like 3 | 8.57 Down | 2.22E-04 |
| | (ADAMTSL3), mRNA | | |
| AK127644 | Homo sapiens cDNA FLJ45742 fis, | 8.54 Down | 3.24E-03 |
| | clone KIDNE2016327 | | |
| BU727096 | UI-E-CRO-ach-e-12-0-UI.s1 UI-E- | 8.53 Down | 9.29E-04 |
| | CRO Homo sapiens cDNA clone UI- | | |
| | E-CRO-ach-e-12-0-UI 3, mRNA | | |
| | sequence | | |
| NM_015973 | Homo sapiens galanin (GAL), mRNA | 8.53 Down | 6.30E-05 |
| NM_033514 | Homo sapiens LIM and senescent | 8.51 Down | 3.30E-04 |
| | cell antigen-like domains 3 (LIMS3), | | |
| | mRNA | | |
| NM_138811 | Homo sapiens chromosome 7 open | 8.47 Down | 8.35E-05 |
| | reading frame 31 (C7orf31), mRNA | | |
| NM_005302 | Homo sapiens G protein-coupled | 8.47 Down | 2.19E-03 |
| | receptor 37 (endothelin receptor | | |
| | type B-like) (GPR37), mRNA | | |
| NM_173462 | Homo sapiens papilin, proteoglycan- | 8.46 Down | 1.70E-04 |
| | like sulfated glycoprotein (PAPLN), | | |
| | mRNA | | |
| NM_032148 | Homo sapiens solute carrier family | 8.44 Down | 9.03E-05 |
| | 41, member 2 (SLC41A2), mRNA | | |
| NM_000856 | Homo sapiens guanylate cyclase 1, | 8.37 Down | 1.17E-03 |
| | soluble, alpha 3 (GUCY1A3), mRNA | | |
| AW591723 | xt85h10.x1 NCI_CGAP_Ut1 Homo | 8.36 Down | 4.86E-04 |
| | sapiens cDNA clone | | |
| | IMAGE:2793283 3 similar to | | |
| | contains element MER32 repetitive | | |
| | element mRNA sequence | | |
| NM_032024 | Homo sapiens chromosome 10 open | 8.25 Down | 1.80E-04 |
| | reading frame 11 (C10orf11), mRNA | | |
| AK024850 | Homo sapiens cDNA: FLJ21197 fis, | 8.21 Down | 7.71 E-05 |
| | clone COL00201 | | |
| NM_002206 | Homo sapiens integrin, alpha 7 | 8.2 Down | 6.30E-05 |
| | (ITGA7), mRNA | | |
| A1963999 | wt87g07_x1 NCI_CGAP_GC6 Homo | 8.19 Down | 1.61 E-03 |
| | sapiens cDNA clone | | |
| | IMAGE:2514492 3, mRNA sequence | | |
| NM_005567 | Homo sapiens lectin, galactoside- | 8.18 Down | 8.76E-05 |
| | binding, soluble, 3 binding protein | | |
| | (LGALS3BP), mRNA | | |
| D87454 | Human mRNA for KIAA0265 gene, | 8.14 Down | 1.08E-03 |
| | partial cds | | |
| BG564960 | 602583930F1 NIH_MGC_76 Homo | 8.12 Down | 7.71 E-05 |
| | sapiens cDNA clone | | |
| | IMAGE:4711807 5, mRNA sequence | | |
| NM_022168 | Homo sapiens interferon induced | 8.12 Down | 1.46E-04 |
| | with helicase C domain 1 (IFIH1), mRNA | | |
| AK027541 | Homo sapiens cDNA FLJ14635 fis, | 8.12 Down | 2.08E-04 |
| | clone NT2RP2001196 | | |
| NM_024997 | Homo sapiens activating | 8.11 Down | 1.65E-03 |
| | transcription factor 7 interacting | | |
| | protein 2 (ATF7lP2), mRNA | | |
| A1926616 | wo48e04.x1 NCI_CGAP_Gas4 | 8.08 Down | 6.30E-05 |
| | Homo sapiens cDNA clone | | |
| | IMAGE:2458590 3 similar to | | |
| | contains MER27.b2 MER27 | | |
| | repetitive element mRNA | | |
| | sequence | | |
| S70348 | Homo sapiens integrin beta 3 | 8.06 Down | 2.21 E-04 |
| | mRNA, partial cds, alternatively | | |
| | spliced | | |
| NM_005725 | Homo sapiens tetraspan 2 (TSPAN- | 8.05 Down | 3.97E-03 |
| | 2), mRNA | | |
| CA311343 | UI-CF-FNO-aff-b-19-0-UI.s1 UI-CF- | 8.02 Down | 8.51E-06 |
| | FNO Homo sapiens cDNA clone UI- | | |
| | CF-FNO-aff-b-19-0-UI 3, mRNA | | |
| | sequence | | |
| NM_001710 | Homo sapiens B-factor, properdin | 8.01 Down | 1.30E-03 |
| | (BF), mRNA | | |
| NM_002247 | Homo sapiens potassium large | 8 Down | 1.29E-03 |
| | conductance calcium-activated | | |
| | channel, subfamily M, alpha member | | |
| | 1 (KCNMA1),mRNA | | |
| BG149255 | nad25d01.x1 NCI_CGAP_Lu24 | 8 Down | 3.85E-04 |
| | Homo sapiens cDNA clone | | |
| | IMAGE:3366553 3, mRNA sequence | | |
| NM_153634 | Homo sapiens copine VIII (CPNE8), mRNA | 7.97 Down | 3.57E-04 |
| NM_004390 | Homo sapiens cathepsin H (CTSH), | 7.97 Down | 7.71 E-05 |
| | transcript variant 1, mRNA | | |
| NM_016613 | Homo sapiens hypothetical protein | 7.97 Down | 3.72E-04 |
| | DKFZp434L142 (DKFZp434L142), | | |
| | mRNA | | |
| NM_001845 | Homo sapiens collagen, type IV, | 7.93 Down | 8.65E-05 |
| | alpha 1 (COL4A1), mRNA | | |
| NM_005502 | Homo sapiens ATP-binding | 7.93 Down | 5.18E-04 |
| | cassette, sub-family A (ABC1), | | |
| | member 1 (ABCA1), mRNA | | |
| NM_018650 | Homo sapiens MAP/microtubule | 7.92 Down | 1.23E-04 |
| | affinity-regulating kinase 1 (MARK1), | | |
| | mRNA | | |
| D62831 | HUM330B12B Clontech human | 7.92 Down | 9.23E-04 |
| | aorta polyA+ mRNA (#6572) Homo | | |
| | sapiens cDNA clone GEN-330B12 5, | | |
| | mRNA sequence | | |
| CA313095 | UI-CF-FNO-aex-f-01-0-UI.s1 UI-CF- | 7.91 Down | 7.42E-04 |
| | FNO Homo sapiens cDNA clone UI- | | |
| | CF-FNO-aex-f-01-0-UI 3, mRNA | | |
| | sequence | | |
| BC038556 | Homo sapiens, clone | 7.91 Down | 2.57E-03 |
| | IMAGE:3446976, mRNA | | |
| A1493349 | tg70f04.x1 Soares_NhHMPu_S1 | 7.9 Down | 1.14E-04 |
| | Homo sapiens cDNA clone | | |
| | IMAGE:2114143 3, mRNA sequence | | |
| BC015108 | Homo sapiens, Similar to otoconin | 7.9 Down | 1.01 E-04 |
| | 90, clone IMAGE:4044247, mRNA | | |
| AI819863 | wj45h05.x1 NCI_CGAP_Lu19 Homo | 7.87 Down | 2.39E-03 |
| | sapiens cDNA clone IMAGE:2405817 3, mRNA sequence | | |
| BM802920 | AGENCOURT_6457446 | 7.85 Down | 1.60E-03 |
| | NIH_MGC_88 Homo sapiens cDNA | | |
| | clone IMAGE:5560288 5, mRNA | | |
| | sequence | | |
| AK057113 | Homo sapiens cDNA FLJ32551 fis, | 7.83 Down | 1.20E-03 |
| | clone SPLEN1000087 | | |
| NM_005025 | Homo sapiens serine (or cysteine) | 7.82 Down | 221 E-04 |
| | proteinase inhibitor, clade I | | |
| | (neuroserpin), member 1 | | |
| | (SERPINI1), mRNA | | |
| AA195328 | zr34f08.s1 Soares_NhHMPu_S1 | 7.81 Down | 2.37E-04 |
| | Homo sapiens cDNA clone | | |
| | IMAGE:665319 3, mRNA sequence | | |
| NM_014358 | Homo sapiens C-type (calcium | 7.81 Down | 5.03E-03 |
| | dependent, carbohydrate-recognition | | |
| | domain) lectin, superfamily member | | |
| | 9 (CLECSF9), mRNA | | |
| BI493986 | df106g12.y1 Morton Fetal Cochlea | 7.8 Down | 6.69E-04 |
| | Homo sapiens cDNA clone | | |
| | IMAGE:2538815 5, mRNA sequence | | |
| NM_152366 | Homo sapiens kelch/ankyrin repeat | 7.79 Down | 8.97E-05 |
| | containing cyclin A1 interacting | | |
| | protein (KARCA1 transcript variant | | |
| | 1, mRNA | | |
| NM_015678 | Homo sapiens neurobeachin | 7.78 Down | 2.12E-04 |
| | (NBEA), mRNA | | |
| NM_018424 | Homo sapiens erythrocyte | 7.77 Down | 1.23E-04 |
| | membrane protein band 4.1 like 4B | | |
| | (EPB41L4B), mRNA | | |
| CA310979 | UI-CF-FNO-afc-c-21-0-UI.s1 UI-CF- | 7.76 Down | 1.86E-04 |
| | FN0 Homo sapiens cDNA clone UI- | | |
| | CF-FNO-afc-c-21-0-UI 3, mRNA | | |
| | sequence | | |
| NM_031476 | Homo sapiens hypothetical protein | 7.75 Down | 2.20E-03 |
| | DKFZp434B044 (DKFZP434B044), | | |
| | mRNA | | |
| NM_004086 | Homo sapiens coagulation factor C | 7.74 Down | 2.21 E-04 |
| | homolog, cochlin (Limulus | | |
| | polyphemus) (COCH), mRNA | | |
| NM_003617 | Homo sapiens regulator of G-protein | 7.73 Down | 3.85E-04 |
| | signalling 5 (RGS5), mRNA | | |
| BF966833 | 602286668T1 NIH_MGC_95 Homo | 7.73 Down | 6.10E-05 |
| | sapiens cDNA clone | | |
| | IMAGE:4375360 3, mRNA sequence | | |
| AK024270 | Homo sapiens cDNA FLJ14208 fis, | 7.71 Down | 1.13E-03 |
| | clone NT2RP3003264 | | |
| NM_144587 | Homo sapiens chromosome 10 open | 7.71 Down | 6.03E-04 |
| | reading frame 87 (C10orf87), mRNA | | |
| AK096288 | Homo sapiens cDNA FLJ38969 fis, | 7.7 Down | 1.49E-04 |
| | clone NT2RI2002359 | | |
| BE968596 | 601649770F1 NIH_MGC_74 Homo | 7.69 Down | 2.39E-03 |
| | sapiens cDNA clone | | |
| | IMAGE:3933472 5, mRNA sequence | | |
| AY358775 | Homo sapiens clone DNA170212 | 7.66 Down | 2.16E-03 |
| | WGAR9166 (UNQ9166) mRNA, | | |
| | complete cds | | |
| BX103949 | BX103949 NCI_CGAP_Co3 Homo | 7.64 Down | 3.18E-04 |
| | sapiens cDNA clone | | |
| | IMAGp998C112296 ; | | |
| | IMAGE:923842, mRNA sequence | | |
| NM_013410 | Homo sapiens adenylate kinase 3 | 7.64 Down | 3.07E-04 |
| | (AK3), nuclear gene encoding | | |
| | mitochondrial protein, transcript | | |
| | variant 2, mRNA | | |
| NM_005204 | Homo sapiens mitogen-activated | 7.63 Down | 6.82E-04 |
| | protein kinase kinase kinase 8 | | |
| | (MAP3K8), mRNA | | |
| NM_032211 | Homo sapiens lysyl oxidase-like 4 | 7.6 Down | 3.30E-04 |
| | (LOXL4), mRNA | | |
| AA325746 | EST28794 Cerebellum II Homo | 7.57 Down | 6.30E-05 |
| | sapiens cDNA 5 end, mRNA | | |
| | sequence | | |
| H94320 | yv18b10.s1 Soares fetal liver spleen | 7.55 Down | 3.35E-03 |
| | 1 NFLS Homo sapiens cDNA clone | | |
| | IMAGE:243067 3, mRNA sequence | | |
| BX640973 | Homo sapiens mRNA; cDNA | 7.55 Down | 7.89E-04 |
| | DKFZp686B15184 (from clone | | |
| | DKFZp686B15184) | | |
| BQ015616 | UI-1-BC1-ajb-g-04-0-UI.s1 | 7.54 Down | 5.28E-04 |
| | NCI_CGAP_PI2 Homo sapiens | | |
| | cDNA clone UI-1-BC1-ajb-g-04-0-UI | | |
| | 3, mRNA sequence | | |
| AK128715 | Homo sapiens cDNA FLJ46882 fis, | 7.53 Down | 2.30E-04 |
| | clone UTERU3015844 | | |
| NM_015068 | Homo sapiens paternally expressed | 7.52 Down | 7.71 E-05 |
| | 10 (PEG10), mRNA | | |
| BU754480 | UI-1-BBIp-axz-h-11-0-UI.s1 | 7.49 Down | 6.24E-04 |
| | NCI_CGAP_PI6 Homo sapiens | | |
| | cDNA clone UI-1-BB1p-axz-h-11-0- | | |
| | UI 3, mRNA sequence | | |
| AK074097 | Homo sapiens mRNA for FLJ00168 protein | 7.48 Down | 1.93E-03 |
| BC033567 | Homo sapiens, clone | 7.48 Down | 1.96E-04 |
| | IMAGE:4822266, mRNA | | |
| NM_018894 | Homo sapiens EGF-containing | 7.47 Down | 2.67E-04 |
| | fibulin-like extracellular matrix | | |
| | protein 1 (EFEMP1), transcript | | |
| | variant 2, mRNA | | |
| BM701989 | UI-E-CQ1-aex-j-06-0-UI.r1 UI-E- | 7.47 Down | 4.53E-04 |
| | CQ1 Homo sapiens cDNA clone UI- | | |
| | E-CQ1-aex-j-06-D-UI 5, mRNA | | |
| | sequence | | |
| NM_016423 | Homo sapiens zinc finger protein | 7.46 Down | 1.61 E-04 |
| | 219 (ZNF219), mRNA | | |
| BX117317 | BX117317 NCI_CGAP_Co3 Homo | 7.45 Down | 1.13E-03 |
| | sapiens cDNA, clone | | |
| | IMAGp998E242234 ; | | |
| | IMAGE:900095, mRNA sequence | | |
| NM_001993 | Homo sapiens coagulation factor III | 7.45 Down | 5.82E-04 |
| | (thromboplastin, tissue factor) (F3), | | |
| | mRNA | | |
| CA502991 | UI-CF-FN0-afp-g-01-0-UI.s1 UI-CF- | 7.43 Down | 2.24E-02 |
| | FNO Homo sapiens cDNA clone UI- | | |
| | CF-FN0-afp-g-01-0-UI 3, mRNA | | |
| | sequence | | |
| AI686890 | tp90h02.x1 NCI_CGAP_Ut3 Homo | 7.41 Down | 1.49E-04 |
| | sapiens cDNA clone | | |
| | IMAGE:2206611 3, mRNA sequence | | |
| AW137001 | UI-H-BI1-acu-c-05-0-UI.s1 | 7.4 Down | 7.95E-05 |
| | NCI_CGAP_Sub3 Homo sapiens | | |
| | cDNA clone IMAGE:2715632 3, | | |
| | m RNA sequence | | |
| BQ021695 | UI-H-DH1-axi-f-22-0-UI.s1 | 7.39 Down | 6.10E-05 |
| | NCI_CGAP_DH1 Homo sapiens | | |
| | cDNA clone IMAGE:5829141 3, | | |
| | mRNA sequence | | |
| BX640643 | Homo sapiens mRNA; cDNA | 7.36 Down | 5.18E-05 |
| | DKFZp686024114 (from clone | | |
| | DKFZp686024114) | | |
| NM_004669 | Homo sapiens chloride intracellular | 7.35 Down | 4.30E-03 |
| | channel 3 (CLIC3), mRNA | | |
| NM_001955 | Homo sapiens endothelin 1 (EDN1), | 7.33 Down | 7.72E-04 |
| | mRNA | | |
| AB007974 | Homo sapiens mRNA, chromosome | 7.33 Down | 5.29E-03 |
| | 1 specific transcript KIAA0505 | | |
| NM_198174 | Homo sapiens transcription factor | 7.32 Down | 4.82E-03 |
| | CP2-like 4 (TFCP2L4), transcript | | |
| | variant 3, mRNA | | |
| BX098521 | BX098521 Soares fetal liver spleen | 7.3 Down | 5.51 E-03 |
| | 1 NFLS Homo sapiens cDNA clone | | |
| | IMAGp998L05118 ; IMAGE:123412, | | |
| | mRNA sequence | | |
| NM_004154 | Homo sapiens pyrimidinergic | 7.28 Down | 3.41E-03 |
| | receptor P2Y, G-protein coupled, 6 | | |
| | (P2RY6), transcript variant 4, mRNA | | |
| AK123617 | Homo sapiens cDNA FLJ41623 fis, | 7.27 Down | 2.26E-03 |
| | clone CTONG3009227 | | |
| BF111903 | 7138d07.x1 | 7.25 Down | 1.34E-03 |
| | Soares_NSF_F8_9W_OT_PA_P_S1 | | |
| | Homo sapiens cDNA clone | | |
| | IMAGE:3523644 3, mRNA sequence | | |
| NM_018655 | Homo sapiens lens epithelial protein | 7.24 Down | 1.07E-04 |
| | (LENEP), mRNA | | |
| NM_139161 | Homo sapiens crumbs homolog 3 | 7.23 Down | 8.46E-04 |
| | (Drosophila) (CRB3), transcript | | |
| | variant 2, mRNA | | |
| BC060805 | Homo sapiens hypothetical protein | 7.22 Down | 7.45E-05 |
| | FLJ12788, mRNA (cDNA clone | | |
| | IMAGE:5266931 partial cds | | |
| CA413744 | UI-H-EZO-bat-h-12-0-UI.s1 | 7.22 Down | 6.09E-04 |
| | NCI_CGAP_Ch1 Homo sapiens | | |
| | cDNA clone UI-H-EZO-bat-h-12-0-UI | | |
| | 3, mRNA sequence | | |
| NM_018986 | Homo sapiens SH3 domain and | 7.21 Down | 2.68E-03 |
| | tetratricopeptide repeats 1 | | |
| | (SH3TC1), mRNA | | |
| NM_024677 | Homo sapiens hypothetical protein | 7.21 Down | 1.14E-04 |
| | FLJ14001 (FLJ14001), mRNA | | |
| AL117454 | Homo sapiens mRNA; cDNA | 7.2 Down | 2.93E-04 |
| | DKFZp586J1717 (from clone | | |
| | DKFZp586J 1717) | | |
| AW450938 | UI-H-BI3-all-g-05-0-UI.s1 | 7.19 Down | 1.19E-04 |
| | NCI_CGAP_Sub5 Homo sapiens | | |
| | cDNA clone IMAGE:2737329 3, | | |
| | mRNA sequence | | |
| NM_017899 | Homo sapiens hypothetical protein | 7.16 Down | 4.80E-03 |
| | FLJ20607 (TSC), mRNA | | |
| S81734 | tissue transglutaminase homologue | 7.16 Down | 8.76E-05 |
| | {alternatively spliced} [human, | | |
| | erythroleukemia cell line HEL | | |
| | GM06141A, mRNA, 2362 nt] | | |
| BU616749 | UI-H-FH1-bfj-a-11-0-UI.s1 | 7.14 Down | 2.21E-04 |
| | NCI_CGAP_FH1 Homo sapiens | | |
| | cDNA clone UI-H-FH1-bfj-a-11-0-UI | | |
| | 3, mRNA sequence | | |
| NM_006681 | Homo sapiens neuromedin U | 7.1 Down | 6.52E-03 |
| | (NMU), mRNA | | |
| AK022598 | Homo sapiens cDNA FLJ12536 fis, | 7.09 Down | 1.15E-04 |
| | clone NT2RM4000265 | | |
| AW137116 | UI-H-B11-acp-f-03-0-UI.s1 | 7.07 Down | 3.50E-03 |
| | NCI_CGAP_Sub3 Homo sapiens | | |
| | cDNA clone IMAGE:2715029 3, | | |
| | mRNA sequence | | |
| BX647876 | Homo sapiens mRNA; cDNA | 7.06 Down | 1.41E-03 |
| | DKFZp313A1525 (from clone | | |
| | DKFZp313A1525) | | |
| NM_000597 | Homo sapiens insulin-like growth | 7.04 Down | 2.99E-03 |
| | factor binding protein 2, 36kDa | | |
| | (IGFBP2), mRNA | | |
| CN478597 | UI-CF-FNO-aeo-g-21-0-UI.s1 UI-CF- | 7.04 Down | 1.43E-03 |
| | FNO Homo sapiens cDNA clone UI- | | |
| | CF-FNO-aeo-g-21-0-UI 3, mRNA | | |
| | sequence | | |
| CN478714 | UI-CF-FNO-afu-c-19-0-UI.s1 UI-CF- | 7.04 Down | 3.55E-03 |
| | FNO Homo sapiens cDNA clone UI- | | |
| | CF-FNO-afu-c-19-0-UI 3, mRNA | | |
| | sequence | | |
| AB020640 | Homo sapiens mRNA for KIAA0833 | 7 Down | 2.45E-03 |
| | protein, partial cds | | |
| NM_022746 | Homo sapiens hypothetical protein | 6.99 Down | 9.12E-04 |
| | FLJ22390 (FLJ22390), mRNA | | |
| AI905628 | CM-BT094-050299-147 BT094 | 6.98 Down | 1.54E-04 |
| | Homo sapiens cDNA, mRNA | | |
| | sequence | | |
| NM_002193 | Homo sapiens inhibin, beta B | 6.96 Down | 7.59E-04 |
| | (activin AB beta polypeptide) | | |
| | (INHBB), mRNA | | |
| NM_004490 | Homo sapiens growth factor | 6.94 Down | 1.98E-03 |
| | receptor-bound protein 14 (GRB14), | | |
| | mRNA | | |
| NM_003985 | Homo sapiens tyrosine kinase, non- | 6.93 Down | 2.10E-04 |
| | receptor, 1 (TNK1), mRNA | | |
| NM_000480 | Homo sapiens adenosine | 6.92 Down | 1.06E-04 |
| | monophosphate deaminase (isoform | | |
| | E) (AMPD3), mRNA | | |
| AK094292 | Homo sapiens cDNA FLJ36973 fis, | 6.92 Down | 2.03E-03 |
| | clone BRACE2006249 | | |
| BC033124 | Homo sapiens, clone | 6.88 Down | 7.22E-05 |
| | IMAGE:2960615, mRNA | | |
| NM_000612 | Homo sapiens insulin-like growth | 6.88 Down | 2.60E-04 |
| | factor 2 (somatomedin A) (IGF2), | | |
| | mRNA | | |
| BC042976 | Homo sapiens cDNA clone | 6.88 Down | 2.39E-03 |
| | IMAGE:5295023, partial cds | | |
| BF445031 | nad20f02.x1 NCI_CGAP_Lu24 | 6.87 Down | 5.18E-04 |
| | Homo sapiens cDNA clone | | |
| | IMAGE:3366266 3, mRNA sequence | | |
| NM_004170 | Homo sapiens solute carrier family 1 | 6.87 Down | 3.18E-04 |
| | (neuronal/epithelial high affinity | | |
| | glutamate transporter, system Xag), | | |
| | member 1 (SLC1A1), mRNA | | |
| NM_199169 | Homo sapiens transmembrane, | 6.84 Down | 1.45E-03 |
| | prostate androgen induced RNA | | |
| | (TMEPAI), transcript variant 2, | | |
| | mRNA | | |
| NM_019644 | Homo sapiens ankyrin repeat | 6.83 Down | 8.99E-05 |
| | domain 7 (ANKRD7), mRNA | | |
| W20132 | zb40c10.r1 | 6.82 Down | 6.79E-03 |
| | Soares_parathyroid_tumor_NbHPA | | |
| | Homo sapiens cDNA clone | | |
| | IMAGE:306066 5, mRNA sequence | | |
| BC033116 | Homo sapiens chromodomain | 6.82 Down | 8.03E-05 |
| | helicase DNA binding protein 7, | | |
| | mRNA (cDNA clone | | |
| | IMAGE:3352674), partial cds | | |
| NM_018650 | Homo sapiens MAP/microtubule | 6.8 Down | 4.97E-03 |
| | affinity-regulating kinase 1 (MARK1), | | |
| | mRNA | | |
| BU563992 | AGENCOURT_10371176 | 6.79 Down | 1.29E-02 |
| | NIH_MGC_141 Homo sapiens | | |
| | cDNA clone IMAGE:6601829 5, | | |
| | mRNA sequence | | |
| AL119769 | DKFZp761 E1224_r1 761 (synonym: | 6.78 Down | 2.52E-03 |
| | hamy2) Homo sapiens cDNA clone | | |
| | DKFZp761E1224 5, mRNA | | |
| | sequence | | |
| NM_052997 | Homo sapiens ankyrin repeat | 6.78 Down | 2.45E-03 |
| | domain 30A (ANKRD30A), mRNA | | |
| NM_024704 | Homo sapiens chromosome 20 open | 6.76 Down | 7.45E-04 |
| | reading frame 23 (C20orf23), mRNA | | |
| NM_000495 | Homo sapiens collagen, type IV, | 6.74 Down | 4.77E-03 |
| | alpha 5 (Alport syndrome) | | |
| | (COL4A5), transcript variant 1, | | |
| | mRNA | | |
| BX102895 | BX102895 Soares fetal liver spleen | 6.7 Down | 3.04E-04 |
| | 1NFLS Homo sapiens cDNA clone | | |
| | IMAGp998I18520 ; IMAGE:242009, | | |
| | mRNA sequence | | |
| BU753362 | UI-1-BB1-air-h-09-0-UI.s1 | 6.7 Down | 7.42E-05 |
| | NCI_CGAP_PI5 Homo sapiens | | |
| | cDNA clone UI-1-BB1-air-h-09-0-UI | | |
| | 3, mRNA sequence | | |
| AA190552 | zp86b11.s1 Stratagene HeLa cell s3 | 6.67 Down | 1.26E-03 |
| | 937216 Homo sapiens cDNA clone | | |
| | IMAGE:627069 3, mRNA sequence | | |
| NM_004024 | Homo sapiens activating | 6.66 Down | 3.12E-03 |
| | transcription factor 3 (ATF3), mRNA | | |
| NM_144650 | Homo sapiens alcohol | 6.65 Down | 1.45E-04 |
| | dehydrogenase, iron containing, 1 | | |
| | (ADHFE1), mRNA | | |
| NM_002800 | Homo sapiens proteasome | 6.63 Down | 1.70E-04 |
| | (prosome, macropain) subunit, beta | | |
| | type, 9 (large multifunctional | | |
| | protease 2) (PSMB9), transcript | | |
| | variant 1, mRNA | | |
| NM_021977 | Homo sapiens solute carrier family | 6.62 Down | 2.05E-03 |
| | 22 (extraneuronal monoamine | | |
| | transporter), member 3 (SLC22A3), | | |
| | mRNA | | |
| AW516579 | xq01f06.x1 | 6.62 Down | 4.65E-03 |
| | Soares_NHCeC_cervicat_tumor | | |
| | Homo sapiens cDNA clone | | |
| | IMAGE:2748611 3, mRNA sequence | | |
| AI311296 | ta48d10.x2 NCI_CGAP_Lu25 Homo | 6.61 Down | 1.72E-03 |
| | sapiens cDNA clone | | |
| | IMAGE:2047315 3, mRNA sequence | | |
| BC040204 | Homo sapiens, clone | 6.61 Down | 5.66E-04 |
| | IMAGE:4821815, mRNA | | |
| AA340011 | EST45155 Fetal skin Homo sapiens | 6.58 Down | 6.30E-05 |
| | cDNA 5 end, mRNA sequence | | |
| NM_025044 | Homo sapiens bicaudal C homolog 1 | 6.57 Down | 6.30E-05 |
| | (Drosophila) (BICC1), mRNA | | |
| BX111520 | BX111520 Soares placenta Nb2HP | 6.57 Down | 1.70E-03 |
| | Homo sapiens cDNA clone | | |
| | IMAGp998L15208 ; IMAGE:141470, | | |
| | mRNA sequence | | |
| AW444925 | UI-H-BI3-ajz-f-09-0-UI.s1 | 6.52 Down | 2.55E-03 |
| | NCI_CGAP_Sub5 Homo sapiens | | |
| | cDNA clone IMAGE:2733473 3, | | |
| | mRNA sequence | | |
| NM_003475 | Homo sapiens chromosome 11 open | 6.5 Down | 5.88E-04 |
| | reading frame 13 (C11orf13), mRNA | | |
| AK124778 | Homo sapiens cDNA FLJ42788 fis, | 6.48 Down | 8.61 E-04 |
| | clone BRAWH3007129 | | |
| NM_024563 | Homo sapiens hypothetical protein | 6.44 Down | 4.72E-03 |
| | FLJ14054 (FLJ14054), mRNA | | |
| NM_002214 | Homo sapiens integrin, beta 8 | 6.43 Down | 8.46E-05 |
| | (ITGB8), mRNA | | |
| NM_178814 | Homo sapiens adaptor-related | 6.42 Down | 1.78E-04 |
| | protein complex 1, sigma 3 subunit | | |
| | (AP1S3), mRNA | | |
| AI831068 | wj62d12.x1 NCI_CGAP_Lu19 Homo | 6.41 Down | 3.72E-04 |
| | sapiens cDNA clone | | |
| | IMAGE:2407415 3, mRNA sequence | | |
| NM_000186 | Homo sapiens complement factor H | 6.39 Down | 1.74E-03 |
| | (CFH), mRNA | | |
| NM_000216 | Homo sapiens Kallmann syndrome 1 | 6.37 Down | 8.59E-05 |
| | sequence (KAL1), mRNA | | |
| NM_015478 | Homo sapiens 1(3)mbt-like | 6.36 Down | 7.22E-05 |
| | (Drosophila) (L3MBTL), transcript | | |
| | variant I, mRNA | | |
| BX096609 | BX096609 Soares retina N2b4HR | 6.36 Down | 1.24E-03 |
| | Homo sapiens cDNA clone | | |
| | IMAGp998L12439 ; IMAGE:221339, | | |
| | m RNA sequence | | |
| NM_030970 | Homo sapiens hypothetical protein | 6.34 Down | 1.69E-04 |
| | MGC3771 (MGC3771), mRNA | | |
| BQ007085 | UI-H-EII-azc-k-11-0-UI.s1 | 6.33 Down | 1.19E-04 |
| | NCI_CGAP_EI1 Homo sapiens | | |
| | cDNA clone IMAGE:5846914 3, | | |
| | mRNA sequence | | |
| BC039329 | Homo sapiens, clone | 6.33 Down | 6.61 E-03 |
| | IMAGE:5267606, mRNA | | |
| NM_001165 | Homo sapiens baculoviral IAP | 6.33 Down | 3.42E-03 |
| | repeat-containing 3 (BIRC3), | | |
| | transcript variant 1, mRNA | | |
| NM_004165 | Homo sapiens Ras-related | 6.33 Down | 4.68E-03 |
| | associated with diabetes (RRAD), | | |
| | mRNA | | |
| NM_001928 | Homo sapiens D component of | 6.32 Down | 3.68E-03 |
| | complement (adipsin) (DF), mRNA | | |
| NM_018670 | Homo sapiens mesoderm posterior | 6.31 Down | 5.76E-03 |
| | 1 (MESP1), mRNA | | |
| NM_153229 | Homo sapiens hypothetical protein | 6.31 Down | 2.57E-03 |
| | FLJ33318 (FLJ33318), mRNA | | |
| AA912845 | ol32a12.s1 | 6.29 Down | 1.13E-03 |
| | Soares_NFL_T_GBC_S1 Homo | | |
| | sapiens cDNA clone | | |
| | IMAGE:1525150 3, mRNA sequence | | |
| AK127437 | Homo sapiens cDNA FLJ45529 fis, | 6.28 Down | 3.37E-04 |
| | clone BRTHA2027546 | | |
| NM_020836 | Homo sapiens brain-enriched | 6.27 Down | 6.60E-03 |
| | guanylate kinase-associated protein | | |
| | (KIAA1446), mRNA | | |
| AL512697 | Homo sapiens mRNA; cDNA | 6.25 Down | 1.36E-03 |
| | DKFZp547F134 (from clone | | |
| | DKFZp547F134) | | |
| NM_005211 | Homo sapiens colony stimulating | 6.24 Down | 5.07E-04 |
| | factor 1 receptor, formerly | | |
| | McDonough feline sarcoma viral (v- | | |
| | fms) oncogene homolog (CSF1R), | | |
| | mRNA | | |
| AK124776 | Homo sapiens cDNA FLJ42786 fis, | 6.2 Down | 1.15E-04 |
| | clone BRAWH3006761 | | |
| NM_173662 | Homo sapiens hypothetical protein | 6.18 Down | 1.05E-02 |
| | LOC285533 (LOC285533), mRNA | | |
| NM_014391 | Homo sapiens ankyrin repeat | 6.18 Down | 3.52E-03 |
| | domain 1 (cardiac muscle) | | |
| | (ANKRD1), mRNA | | |
| NM_005860 | Homo sapiens follistatin-like 3 | 6.18 Down | 5.23E-05 |
| | (secreted glycoprotein) (FSTL3), | | |
| | mRNA | | |
| NM_001045 | Homo sapiens solute carrier family 6 | 6.17 Down | 5.46E-03 |
| | (neurotransmitter transporter, | | |
| | serotonin), member 4 (SLC6A4), | | |
| | mRNA | | |
| NM_002147 | Homo sapiens homeo box B5 | 6.17 Down | 1.05E-04 |
| | (HOXB5), mRNA | | |
| AI288404 | qv89b01.x1 NCI_CGAP_Ut2 Homo | 6.16 Down | 3.74E-04 |
| | sapiens cDNA clone | | |
| | IMAGE:1988713 3, mRNA sequence | | |
| NM_173584 | Homo sapiens hypothetical protein | 6.16 Down | 1.32E-04 |
| | MGC45840 (MGC45840), mRNA | | |
| N62729 | yz76g05.s1 | 6.12 Down | 1.37E-03 |
| | Soares_multiple_sclerosis_2NbHMS | | |
| | P Homo sapiens cDNA clone | | |
| | IMAGE:289016 3, mRNA sequence | | |
| AK024238 | Homo sapiens cDNA FLJ14176 fis, | 6.11 Down | 9.69E-05 |
| | clone NT2RP2003101 | | |
| NM_001843 | Homo sapiens contactin 1 (CNTN1), | 6.1 Down | 4.80E-03 |
| | transcript variant 1, mRNA | | |
| NM_152433 | Homo sapiens kelch repeat and BTB | 6.1 Down | 2.39E-03 |
| | (POZ) domain containing 3 | | |
| | (KBTBD3), transcript variant 1, | | |
| | mRNA | | |
| BX649112 | Homo sapiens mRNA; cDNA | 6.1 Down | 2.24E-04 |
| | DKFZp686E02109 (from clone | | |
| | DKFZp686E02109) | | |
| AA411988 | zt65g11.s1 Soares_testis_NHT | 6.09 Down | 1.40E-03 |
| | Homo sapiens cDNA clone | | |
| | IMAGE:727268 3, mRNA sequence | | |
| R80806 | yi94f01.s1 Soares placenta Nb2HP | 6.08 Down | 1.54E-04 |
| | Homo sapiens cDNA clone | | |
| | IMAGE:146905 3, mRNA sequence | | |
| NM_002843 | Homo sapiens protein tyrosine | 6.08 Down | 6.54E-03 |
| | phosphatase, receptor type, J | | |
| | (PTPRJ), mRNA | | |
| NM_003979 | Homo sapiens G protein-coupled | 6.06 Down | 8.75E-03 |
| | receptor, family C, group 5, member | | |
| | A (GPCR5A), mRNA | | |
| AW129281 | xf23a03.x1 NCI_CGAP_Kid8 Homo | 6.06 Down | 1.70E-04 |
| | sapiens cDNA clone | | |
| | IMAGE:2618860 3 similar to | | |
| | gb:X58295_rna1 PLASMA | | |
| | GLUTATHIONE PEROXIDASE | | |
| | PRECURSOR (HUMAN);, mRNA | | |
| | sequence | | |
| NM_015345 | Homo sapiens dishevelled | 6.05 Down | 1.09E-03 |
| | associated activator of | | |
| | morphogenesis 2 (DAAM2), mRNA | | |
| L07615 | Human neuropeptide Y receptor Y1 | 6.05 Down | 5.51 E-03 |
| | (NPYY1) mRNA, exon 2-3 and | | |
| | complete cds | | |
| NM_016179 | Homo sapiens transient receptor | 6.04 Down | 8.59E-05 |
| | potential cation channel, subfamily | | |
| | C, member 4 (TRPC4), mRNA | | |
| NM_182797 | Homo sapiens phospholipase C, | 6.03 Down | 4.40E-04 |
| | beta 4 (PLCB4), transcript variant 2, | | |
| | mRNA | | |
| AB032945 | Homo sapiens mRNA for KIAA1119 | 6.03 Down | 1.11 E-02 |
| | protein, partial cds | | |
| CA444471 | UI-H-DPO-avv-a-16-0-UI.s1 | 6.02 Down | 1.02E-03 |
| | NCI_CGAP_Fs1 Homo sapiens | | |
| | cDNA clone UI-H-DPO-avv-a-16-0-UI | | |
| | 3, mRNA sequence | | |
| AK127421 | Homo sapiens cDNA FLJ45513 fis, | 6.01 Down | 1.60E-04 |
| | clone BRTHA2021450 | | |
| AV736303 | AV736303 CB Homo sapiens cDNA | 6.01 Down | 8.97E-05 |
| | clone CBCAJD04 5, mRNA | | |
| | sequence | | |
| BX537613 | Homo sapiens mRNA; cDNA | 6 Down | 4.89E-04 |
| | DKFZp686E11117 (from clone | | |
| | DKFZp686E11117) | | |
| AB023211 | Homo sapiens mRNA for KIAA0994 | 5.99 Down | 5.50E-04 |
| | protein, partial cds | | |
| NM_018349 | Homo sapiens multiple C2-domains | 5.99 Down | 2.48E-04 |
| | with two transmembrane regions 2 | | |
| | (MCTP2), mRNA | | |
| NM_000087 | Homo sapiens cyclic nucleotide | 5.98 Down | 3.29E-03 |
| | gated channel alpha 1 (CNGA1), | | |
| | mRNA | | |
| BF939416 | nad89c02.x1 NCI_CGAP_Pr28 | 5.97 Down | 9.03E-04 |
| | Homo sapiens cDNA clone | | |
| | IMAGE:3410667 3, mRNA sequence | | |
| NM_013951 | Homo sapiens paired box gene 8 | 5.97 Down | 2.57E-03 |
| | (PAX8), transcript variant PAX8B, | | |
| | mRNA | | |
| AW195474 | xn38g09.x1 NCI_CGAP_Kid11 | 5.97 Down | 1.68E-04 |
| | Homo sapiens cDNA clone | | |
| | IMAGE:2696032 3, mRNA sequence | | |
| R40050 | yf68h07.s1 Soares infant brain 1NIB | 5.94 Down | 5.78E-03 |
| | Homo sapiens cDNA clone | | |
| | IMAGE:27726 3, mRNA sequence | | |
| NM_021101 | Homo sapiens claudin 1 (CLDN1), | 5.9 Down | 2.60E-04 |
| | mRNA | | |
| AA602964 | no97c02.s1 NCI_CGAP_Pr2 Homo | 5.9 Down | 1.23E-03 |
| | sapiens cDNA clone | | |
| | IMAGE:1114754, mRNA sequence | | |
| NM_006255 | Homo sapiens protein kinase C, eta | 5.89 Down | 2.78E-04 |
| | (PRKCH), mRNA | | |
| NM_024103 | Homo sapiens solute carrier family | 5.89 Down | 7.42E-05 |
| | 25 (mitochondrial carrier; phosphate | | |
| | carrier), member 23 (SLC25A23), mRNA | | |
| AK093870 | Homo sapiens cDNA FLJ36551 fis, | 5.88 Down | 2.28E-02 |
| | clone TRACH2008127 | | |
| BE070450 | QV4-BT0407-020300-122-d08 | 5.85 Down | 3.53E-04 |
| | BT0407 Homo sapiens cDNA, | | |
| | mRNA sequence | | |
| NM_003706 | Homo sapiens phospholipase A2, | 5.85 Down | 7.81 E-04 |
| | group IVC (cytosolic, calcium- | | |
| | independent) (PLA2G4C), mRNA | | |
| NM_023927 | Homo sapiens HCV NS3- | 5.85 Down | 2.64E-03 |
| | transactivated protein 2 (NS3TP2), | | |
| | mRNA | | |
| AF519622 | Homo sapiens noncoding mRNA | 5.84 Down | 7.70E-03 |
| | sequence | | |
| NM_198582 | Homo sapiens FLJ43374 protein | 5.84 Down | 4.05E-03 |
| | (FLJ43374), mRNA | | |
| AK026283 | Homo sapiens cDNA: FLJ22630 fis, | 5.84 Down | 5.32E-04 |
| | clone HSI06250 | | |
| CB115754 | K-EST0159876 L8SCK0 Homo | 5.81 Down | 9.71 E-04 |
| | sapiens cDNA clone L8SCKO-8-H08 | | |
| | 5, mRNA sequence | | |
| BQ188285 | UI-E-EJ1-ajp-n-20-0-UI.r1 UI-E-EJ1 | 5.8 Down | 5.47E-03 |
| | Homo sapiens cDNA clone UI-E- | | |
| | EJ1-ajp-n-20-0-UI 5, mRNA | | |
| | sequence | | |
| NM_003662 | Homo sapiens pirin (iron-binding | 5.78 Down | 1.71 E-04 |
| | nuclear protein) (PIR), mRNA | | |
| BX098660 | BX098660 Soares placenta Nb2HP | 5.77 Down | 6.00E-04 |
| | Homo sapiens cDNA clone | | |
| | IMAGp998L03214 ; IMAGE:143762, mRNA sequence | | |
| NM_005360 | Homo sapiens v-maf | 5.77 Down | 2.24E-04 |
| | musculoaponeurotic fibrosarcoma | | |
| | oncogene homolog (avian) (MAF), | | |
| | mRNA | | |
| NM_031847 | Homo sapiens microtubule- | 5.77 Down | 1.34E-03 |
| | associated protein 2 (MAP2), | | |
| | transcript variant 4, mRNA | | |
| NM_003057 | Homo sapiens solute carrier family | 5.75 Down | 2.42E-04 |
| | 22 (organic cation transporter), | | |
| | member 1 (SLC22A1 transcript | | |
| | variant 1, mRNA | | |
| AW242323 | xm96f03.x1 NCI_CGAP_Kid11 | 5.74 Down | 1.92E-04 |
| | Homo sapiens cDNA clone | | |
| | IMAGE:2692061 3 similar to | | |
| | contains Alu repetitive element;, | | |
| | mRNA sequence | | |
| NM_052890 | Homo sapiens peptidoglycan | 5.73 Down | 1.14E-04 |
| | recognition protein 2 (PGLYRP2), | | |
| | mRNA | | |
| NM_000593 | Homo sapiens transporter 1, ATP- | 5.72 Down | 4.86E-03 |
| | binding cassette, sub-family B | | |
| | (MDR/TAP) (TAP1), mRNA | | |
| NM_033132 | Homo sapiens Zic family member 5 | 5.71 Down | 1.76E-04 |
| | (odd-paired homolog, Drosophila) | | |
| | (ZIC5), mRNA | | |
| AL832380 | Homo sapiens mRNA; cDNA | 5.71 Down | 1.54E-04 |
| | DKFZp451L157 (from clone | | |
| | DKFZp451L157) | | |
| NM_183376 | Homo sapiens arrestin domain | 5.71 Down | 5.88E-04 |
| | containing 4 (ARRDC4), mRNA | | |
| NM_018700 | Homo sapiens tripartite motif- | 5.7 Down | 1.32E-02 |
| | containing 36 (TRIM36), mRNA | | |
| NM_000782 | Homo sapiens cytochrome P450, | 5.69 Down | 1.82E-02 |
| | family 24, subfamily A, polypeptide 1 | | |
| | (CYP24A1), nuclear gene encoding | | |
| | mitochondrial protein, mRNA | | |
| BC035805 | Homo sapiens caspase recruitment | 5.68 Down | 4.00E-03 |
| | domain family, member 9, mRNA | | |
| | (cDNA clone IMAGE:5745585), | | |
| | partial cds | | |
| BC051727 | Homo sapiens cDNA clone | 5.68 Down | 5.13E-04 |
| | IMAGE:5265929, partial cds | | |
| NM_022842 | Homo sapiens CUB domain- | 5.68 Down | 1.85E-03 |
| | containing protein 1 (CDCP1), | | |
| | transcript variant 1, m RNA | | |
| NM_004335 | Homo sapiens bone marrow stromal | 5.67 Down | 6.01 E-03 |
| | cell antigen 2 (BST2), mRNA | | |
| NM_053039 | Homo sapiens UDP | 5.66 Down | 8.07E-03 |
| | glycosyltransferase 2 family, | | |
| | polypeptide B28 (UGT2B28), mRNA | | |
| NM_004529 | Homo sapiens myeloid/lymphoid or | 5.66 Down | 4.02E-04 |
| | mixed-lineage leukemia (trithorax | | |
| | homolog, Drosophila); translocated | | |
| | to, 3 (MLLT3), mRNA | | |
| AK093069 | Homo sapiens cDNA FLJ35750 fis, | 5.65 Down | 1.10E-03 |
| | clone TEST12004539, weakly similar | | |
| | to Homo sapiens adlican mRNA | | |
| NM_005141 | Homo sapiens fibrinogen, B beta | 5.63 Down | 7.89E-04 |
| | polypeptide (FGB), mRNA | | |
| NM_000682 | Homo sapiens adrenergic, alpha-2B- | 5.61 Down | 1.14E-04 |
| | , receptor (ADRA2B), mRNA | | |
| BF512326 | UI-H-BW1-amb-g-12-0-UI.s1 | 5.6 Down | 1.49E-04 |
| | NCI_CGAP_Sub7 Homo sapiens | | |
| | cDNA clone IMAGE:3069503 3, | | |
| | mRNA sequence | | |
| NM_003726 | Homo sapiens src family associated | 5.6 Down | 2.42E-03 |
| | phosphoprotein 1 (SCAP1), mRNA | | |
| AK024261 | Homo sapiens cDNA FLJ14199 fis, | 5.6 Down | 4.42E-04 |
| | clone NT2RP3002713 | | |
| AK125695 | Homo sapiens cDNA FLJ43707 fis, | 5.59 Down | 1.10E-02 |
| | clone TESOP2001865 | | |
| NM_006074 | Homo sapiens tripartite motif- | 5.58 Down | 1.94E-03 |
| | containing 22 (TRIM22), mRNA | | |
| AW591461 | x192h06.x1 NCI_CGAP_Ut1 Homo | 5.58 Down | 1.95E-02 |
| | sapiens cDNA clone | | |
| | IMAGE:2682203 3, mRNA sequence | | |
| NM_022128 | Homo sapiens ribokinase (RBKS), | 5.58 Down | 1.87E-04 |
| | mRNA | | |
| N66105 | yy65e06.s1 | 5.57 Down | 2.84E-04 |
| | Soares_multiple_sclerosis_2NbHMS | | |
| | P Homo sapiens cDNA clone | | |
| | IMAGE:278434 3, mRNA sequence | | |
| BC035116 | Homo sapiens cDNA clone | 5.56 Down | 5.49E-04 |
| | IMAGE:5263177, partial cds | | |
| BM988642 | UI-H-DHO-arx-p-21-0-UI.s1 | 5.56 Down | 2.48E-03 |
| | NCI_CGAP_DH0 Homo sapiens | | |
| | cDNA clone IMAGE:5855492 3, | | |
| | mRNA sequence | | |
| NM_003947 | Homo sapiens huntingtin-associated | 5.56 Down | 9.71 E-04 |
| | protein interacting protein (duo) | | |
| | (HAPIP), mRNA | | |
| AI697906 | we18f06.x1 NCI_CGAP_Lu24 Homo | 5.56 Down | 3.52E-03 |
| | sapiens cDNA clone | | |
| | IMAGE:2341475 3, mRNA sequence | | |
| AK095399 | Homo sapiens cDNA FLJ38080 fis, | 5.55 Down | 7.43E-03 |
| | clone CTONG2016185 | | |
| AL110252 | Homo sapiens mRNA; cDNA | 5.54 Down | 3.44E-03 |
| | DKFZp566A1046 (from clone | | |
| | DKFZp566A1046) | | |
| NM_024572 | Homo sapiens UDP-N-acetyl-alpha- | 5.54 Down | 5.93E-03 |
| | D-galactosamine:polypeptide N- | | |
| | acetylgalactosaminyltransferase 14 | | |
| | (GaINAc-T14) (GALNT14), mRNA | | |
| NM_000775 | Homo sapiens cytochrome P450, | 5.53 Down | 1.57E-03 |
| | family 2, subfamily J, polypeptide 2 | | |
| | (CYP2J2), mRNA | | |
| NM_018317 | Homo sapiens hypothetical protein | 5.51 Down | 5.10E-04 |
| | FLJ11082 (FLJ11082), mRNA | | |
| NM_030915 | Homo sapiens likely ortholog of | 5.51 Down | 6.86E-04 |
| | mouse limb-bud and heart gene | | |
| | (LBH), mRNA | | |
| NM_139241 | Homo sapiens FYVE, RhoGEF and | 5.5 Down | 9.42E-03 |
| | PH domain containing 4 (FGD4), | | |
| | mRNA | | |
| CB047092 | NISC_gf08f03.x1 NCI_CGAP_Kid12 | 5.5 Down | 5.38E-04 |
| | Homo sapiens cDNA clone | | |
| | IMAGE:325301 3, mRNA sequence | | |
| NM_005711 | Homo sapiens EGF-like repeats and | 5.5 Down | 6.04E-04 |
| | discoidin I-like domains 3 (EDIL3), | | |
| | mRNA | | |
| AA974968 | on02e08.s1 NCI_CGAP_Kid3 Homo | 5.49 Down | 1.63E-03 |
| | sapiens cDNA clone | | |
| | IMAGE:1555526 3, mRNA sequence | | |
| NM_000129 | Homo sapiens coagulation factor | 5.49 Down | 1.09E-02 |
| | XIII, A1 polypeptide (F13A1), mRNA | | |
| NM_007366 | Homo sapiens phospholipase A2 | 5.47 Down | 4.66E-03 |
| | receptor 1, 180kDa (PLA2R1), | | |
| | transcript variant 1, mRNA | | |
| AL353944 | Homo sapiens mRNA; cDNA | 5.46 Down | 5.38E-04 |
| | DKFZp761J1112 (from clone | | |
| | DKFZp761J1112) | | |
| AK095647 | Homo sapiens cDNA FLJ38328 fis, | 5.46 Down | 1.61 E-03 |
| | clone FCBBF3025142 | | |
| BQ188860 | UI-E-EJ1-ajx-h-03-0-UI.r1 UI-E-EJ1 | | |
| | Homo sapiens cDNA clone UI-E- | | |
| | EJ1-ajx-h-03-0-UI 5, mRNA | | |
| | sequence | | |
| NM_017594 | Homo sapiens DIRAS family, GTP- | 5.44 Down | 1.22E-02 |
| | binding RAS-like 2 (DIRAS2), mRNA | | |
| NM_014399 | Homo sapiens transmembrane 4 | 5.43 Down | 2.64E-04 |
| | superfamily member 13 (TM4SF13), | | |
| | mRNA | | |
| NM_033255 | Homo sapiens epithelial stromal | 5.43 Down | 1.73E-03 |
| | interaction 1 (breast) (EPSTI1), | | |
| | mRNA | | |
| AW269270 | xs35c11.x1 NCI_CGAP_Kid11 | 5.42 Down | 1.33E-03 |
| | Homo sapiens cDNA clone | | |
| | IMAGE:2771636 3 similar to | | |
| | contains L1.t3 L1 repetitive element | | |
| | mRNA sequence | | |
| AK092456 | Homo sapiens cDNA FLJ35137 fis, | 5.42 Down | 1.09E-04 |
| | clone PLACE6009419 | | |
| NM_031935 | Homo sapiens hemicentin (FIBL-6), | 5.42 Down | 7.69E-04 |
| | mRNA | | |
| BU751966 | UI-1-BBO-acy-c-09-0-ULs1 | 5.4 Down | 1.61 E-03 |
| | NCI_CGAP_P14 Homo sapiens | | |
| | cDNA clone UI-1-BBO-acy-c-09-0-UI | | |
| | 3, mRNA sequence | | |
| NM_001747 | Homo sapiens capping protein (actin | 5.4 Down | 1.33E-03 |
| | filament), gelsolin-like (CAPG), | | |
| | mRNA | | |
| NM_002260 | Homo sapiens killer cell lectin-like | 5.4 Down | 1.21 E-02 |
| | receptor subfamily C, member 2 | | |
| | (KLRC2), mRNA | | |
| AA732841 | zg77f01.s1 | 5.4 Down | 3.48E-03 |
| | Soares_fetal_heark_NbHH19W | | |
| | Homo sapiens cDNA clone | | |
| | IMAGE:399385 3, mRNA sequence | | |
| AK055468 | Homo sapiens cDNA FLJ30906 fis, | 5.4 Down | 1.36E-03 |
| | clone FEBRA2006055 | | |
| AI080164 | oz48c05.x1 Soares_NhHMPu_S1 | 5.38 Down | 2.44E-03 |
| | Homo sapiens cDNA clone | | |
| | IMAGE:1678568 3, mRNA sequence | | |
| NM_005562 | Homo sapiens laminin, gamma 2 | 5.38 Down | 3.78E-04 |
| | (LAMC2), transcript variant 1, mRNA | | |
| NM_080659 | Homo sapiens similar to RIKEN | 5.36 Down | 3.81 E-03 |
| | cDNA 2310030G06 gene | | |
| | (MGC14839), mRNA | | |
| NM_015085 | Homo sapiens GTPase activating | 5.36 Down | 1.86E-04 |
| | Rap/RanGAP domain-like 4 | | |
| | (GARNL4), mRNA | | |
| BX097034 | BX097034 Soares infant brain 1NIB | 5.36 Down | 8.59E-05 |
| | Homo sapiens cDNA clone | | |
| | IMAGp998F14169; IMAGE:39685, | | |
| | mRNA sequence | | |
| NM_002147 | Homo sapiens homeo box B5 | 5.35 Down | 8.21 E-05 |
| | (HOXB5), mRNA | | |
| NM_181785 | Homo sapiens hypothetical protein | 5.33 Down | 3.48E-04 |
| | LOC283537 (LOC283537), mRNA | | |
| NM_032857 | Homo sapiens lactamase, beta | 5.33 Down | 2.45E-04 |
| | (LACTB), nuclear gene encoding | | |
| | mitochondrial protein, transcript | | |
| | variant 1, mRNA | | |
| BX113144 | BX113144 Scares placenta Nb2HP | 5.32 Down | 1.53E-03 |
| | Homo sapiens cDNA clone | | |
| | IMAGp998N07225 ; IMAGE:148038, | | |
| | mRNA sequence | | |
| NM_014905 | Homo sapiens glutaminase (GLS), | 5.31 Down | 1.63E-03 |
| | mRNA | | |
| NM_138396 | Homo sapiens membrane- | 5.31 Down | 1.39E-04 |
| | associated RING-CH protein IX | | |
| | (MARCH-IX), mRNA | | |
| NM_004433 | Homo sapiens E74-like factor 3 (ets | 5.3 Down | 5.32E-05 |
| | domain transcription factor, | | |
| | epithelial-specific ) (ELF3), mRNA | | |
| AA553553 | nk78d11.s1 NCI_CGAP_Sch1 Homo | 5.27 Down | 1.69E-03 |
| | sapiens cDNA clone | | |
| | IMAGE:1019637 3, mRNA sequence | | |
| AW188195 | xj93e12.x1 | 5.27 Down | 5.32E-05 |
| | Soares_NFL_T_GBC_S1 Homo | | |
| | sapiens cDNA clone | | |
| | IMAGE:2664814 3 similar to | | |
| | contains element THR repetitive | | |
| | element;, mRNA sequence | | |
| NM_006169 | Homo sapiens nicotinamide N- | 5.26 Down | 7.45E-05 |
| | methyltransferase (NNMT), mRNA | | |
| NM_000071 | Homo sapiens cystathionine-beta- | 5.26 Down | 1.49E-03 |
| | synthase (CBS), mRNA | | |
| NM_001958 | Homo sapiens eukaryotic translation | 5.25 Down | 5.20E-03 |
| | elongation factor 1 alpha 2 | | |
| | (EEF1A2), mRNA | | |
| AK055356 | Homo sapiens cDNA FLJ30794 fis, | 5.25 Down | 8.65E-04 |
| | clone FEBRA2001093, weakly | | |
| | similar to MONOCARBOXYLATE | | |
| | TRANSPORTER 4 | | |
| NM_014578 | Homo sapiens ras homolog gene | 5.24 Down | 5.43E-03 |
| | family, member D (RHOD), mRNA | | |
| NM_013230 | Homo sapiens CD24 antigen (small | 5.22 Down | 7.65E-04 |
| | cell lung carcinoma cluster 4 | | |
| | antigen) (CD24), mRNA | | |
| AF086158 | Homo sapiens full length insert | 5.2 Down | 6.04E-03 |
| | cDNA clone ZB72E12 | | |
| NM_004932 | Homo sapiens cadherin 6, type 2, K- | 5.18 Down | 2.11E-04 |
| | cadherin (fetal kidney) (CDH6), | | |
| | mRNA | | |
| NM_020466 | Homo sapiens hypothetical protein | 5.18 Down | 2.99E-03 |
| | dJ122O8.2 (DJ12208.2), mRNA | | |
| AL117425 | Homo sapiens mRNA; cDNA | 5.17 Down | 8.64E-03 |
| | DKFZp566L203 (from clone | | |
| | DKFZp566L203) | | |
| AI732568 | zo23d12.x5 Stratagene colon | 5.17 Down | 9.19E-04 |
| | (#937204) Homo sapiens cDNA | | |
| | clone IMAGE:587735 3, mRNA | | |
| | sequence | | |
| AI150192 | qf34d12.x1 Soares_testis_NHT | 5.17 Down | 1.21E-02 |
| | Homo sapiens cDNA clone | | |
| | IMAGE:1751927 3, mRNA sequence | | |
| AK125852 | Homo sapiens cDNA FLJ43864 fis, | 5.17 Down | 4.82E-03 |
| | clone TEST14007799 | | |
| NM_017912 | Homo sapiens hect domain and RLD | 5.15 Down | 3.63E-04 |
| | 6 (HERC6), mRNA | | |
| NM_006174 | Homo sapiens neuropeptide Y | 5.14 Down | 1.13E-02 |
| | receptor Y5 (NPY5R), mRNA | | |
| AK025281 | Homo sapiens cDNA: FLJ21628 fis, | 5.14 Down | 9.71 E-04 |
| | clone COL08076 | | |
| NM_153742 | Homo sapiens cystathionase | 5.12 Down | 1.27E-04 |
| | (cystathionine gamma-lyase) (CTH), | | |
| | transcript variant 2, mRNA | | |
| NM_003107 | Homo sapiens SRY (sex | 5.11 Down | 3.48E-04 |
| | determining region Y)-box 4 (SOX4), | | |
| | mRNA | | |
| NM_003948 | Homo sapiens cyclin-dependent | 5.11 Down | 2.05E-03 |
| | kinase-like 2 (CDC2-related kinase) | | |
| | (CDKL2), mRNA | | |
| NM_173660 | Homo sapiens hypothetical protein | 5.11 Down | 1.30E-03 |
| | FLJ33718 (FLJ33718), mRNA | | |
| A1693580 | wd12d01.x1 NCI_CGAP_Co3 Homo | 5.1 Down | 6.19E-03 |
| | sapiens cDNA clone | | |
| | IMAGE:2327905 3, mRNA sequence | | |
| NM_004482 | Homo sapiens UDP-N-acetyl-alpha- | 5.1 Down | 2.31 E-03 |
| | D-galactosamine:polypeptide N- | | |
| | acetylgalactosaminyltransferase 3 | | |
| | (GaINAc-T3) (GALNT3), mRNA | | |
| NM_030965 | Homo sapiens sialyltransferase 7 | 5.1 Down | 2.61 E-04 |
| | ((alpha-N-acetylneuraminyl-2,3-beta- | | |
| | galactosyl-1,3)-N-acetyl | | |
| | galactosaminide alpha-2,6- | | |
| | sialyltransferase) E (SIAT7E), | | |
| | mRNA | | |
| NM_016147 | Homo sapiens protein phosphatase | 5.09 Down | 9.71 E-04 |
| | methylesterase-1 (PME-1), mRNA | | |
| NM_006259 | Homo sapiens protein kinase, | 5.08 Down | 1.36E-03 |
| | cGMP-dependent, type II (PRKG2), | | |
| | mRNA | | |
| NM_002993 | Homo sapiens chemokine (C-X-C | 5.07 Down | 2.32E-03 |
| | motif) ligand 6 (granulocyte | | |
| | chemotactic protein 2) (CXCL6), | | |
| | mRNA | | |
| BE671338 | 7e49f03.x1 NCI_CGAP_Lu24 Homo | 5.07 Down | 1.28E-02 |
| | sapiens cDNA clone | | |
| | IMAGE:3285821 3, mRNA sequence | | |
| BX647256 | Homo sapiens mRNA; cDNA | 5.07 Down | 2.05E-02 |
| | DKFZp686K0753 (from clone | | |
| | DKFZp686K0753) | | |
| BG186566 | RST5534 Athersys RAGE Library | 5.05 Down | 8.91 E-04 |
| | Homo sapiens cDNA, mRNA | | |
| | sequence | | |
| AV652758 | AV652758 GLC Homo sapiens | 5.04 Down | 1.05E-03 |
| | cDNA clone GLCDDG05 3, mRNA | | |
| | sequence | | |
| A1672441 | wa03c03.x1 NCI_CGAP_Kid11 | 5.04 Down | 1.11E-02 |
| | Homo sapiens cDNA clone | | |
| | IMAGE:2296996 3, mRNA sequence | | |
| AA769642 | ob20h04.s1 NCI-CGAP-Kid5 Homo | 5.02 Down | 1.59E-02 |
| | sapiens cDNA clone | | |
| | IMAGE:1324279 3, mRNA sequence | | |
| AL706653 | DKFZp686E1543_r1 686 (synonym: | 5.02 Down | 1.09E-03 |
| | hlcc3) Homo sapiens cDNA clone | | |
| | DKFZp686E1543 5, mRNA | | |
| | sequence | | |
| AW296834 | UI-H-BI2-ahz-a-10-0-UI.s1 | 5.01 Down | 5.93E-03 |
| | NCI_CGAP_Sub4 Homo sapiens | | |
| | cDNA clone IMAGE:2728243 3, | | |
| | mRNA sequence | | |

**TABLE VII B: GENES THAT WERE DIFFERENTIALLY EXPRESSED AT LEAST 5 FOLD IN EPITHELIAL VERSUS FIBROBLAST CELLS**

| Gene Identifier | Gene Name | fold change in Epithelial versus fibroblast cells | Direction adj. p- value |
|---|---|---|---|
| | | | |
| NM_018658 | Homo sapiens potassium inwardly-rectifying | 481.28 UP | 2.64E-05 |
| | channel, subfamily J, member 16 (KCNJ16), | | |
| | transcript variant 1, mRNA | | |
| NM_014358 | Homo sapiens C-type (calcium dependent, | 375.12 UP | 6.56E-05 |
| | carbohydrate-recognition domain) lectin, | | |
| | superfamily member 9 (CLECSF9), mRNA | | |
| AB032980 | Homo sapiens mRNA for KIAA1154 protein, | 331.92 UP | 1.60E-05 |
| | partial cds | | |
| BG219729 | RST39494 Athersys RAGE Library Homo | 316.78 UP | 8.39E-06 |
| | sapiens cDNA, mRNA sequence | | |
| NM_002899 | Homo sapiens retinol binding protein 1, cellular | 314.6 UP | 5.37E-05 |
| | (RBP1), mRNA | | |
| NM_007038 | Homo sapiens a disintegrin-like and | 280.99 UP | 1.26E-05 |
| | metalloprotease (reprolysin type) with | | |
| | thrombospondin type 1 motif, 5 (aggrecanase- | | |
| | 2) (ADAMTS5), mRNA | | |
| NM_002423 | Homo sapiens matrix metalloproteinase 7 | 269.82 UP | 8.65E-06 |
| | (matrilysin, uterine) (MMP7), mRNA | | |
| NM_001977 | Homo sapiens glutamyl aminopeptidase | 242.27 UP | 5.43E-05 |
| | (aminopeptidase A) (ENPEP), mRNA | | |
| D29453 | HUMNK566 Human epidermal keratinocyte | 208.36 UP | 1.72E-05 |
| | Homo sapiens cDNA clone 566, mRNA | | |
| | sequence | | |
| NM_024508 | Homo sapiens zinc finger, BED domain | 168.99 UP | 8.39E-06 |
| | containing 2 (ZBED2), mRNA | | |
| AI335277 | tb29h06.x1 NCI_CGAP_Kid12 Homo sapiens | 165.99 UP | 8.46E-06 |
| | cDNA clone IMAGE:2055803 3, mRNA | | |
| | sequence | | |
| BE877764 | 601486331 F1 NIH_MGC_69 Homo sapiens | 145.52 UP | 2.36E-05 |
| | cDNA clone IMAGE:3888943 5, mRNA | | |
| | sequence | | |
| A1765021 | wh56c02.x1 NCI_CGAP_Kid11 Homo sapiens | 145.09 UP | 1.15E-05 |
| | cDNA clone IMAGE:2384738 3, mRNA | | |
| | sequence | | |
| NM_024726 | Homo sapiens IQ motif containing with AAA | 143.09 UP | 8.39E-06 |
| | domain (IQCA), mRNA | | |
| NM_004221 | Homo sapiens natural killer cell transcript 4 | 142.15 UP | 1.88E-05 |
| | (NK4), mRNA | | |
| NM_019000 | Homo sapiens hypothetical protein FLJ20152 | 131.23 UP | 1.88E-05 |
| | (FLJ20152), mRNA | | |
| NM_000104 | Homo sapiens cytochrome P450, family 1, | 127.7 UP | 1.51 E-05 |
| | subfamily B, polypeptide 1 (CYP1B1), mRNA | | |
| BM993116 | UI-H-DT0-aty-f-17-0-UI.s1 NCI_CGAP_DTO | 127.35 UP | 4.13E-05 |
| | Homo sapiens cDNA clone IMAGE:5866000 3, | | |
| | mRNA sequence | | |
| NM_031426 | Homo sapiens chromosome 9 open reading | 116.09 UP | 3.82E-05 |
| | frame 58 (C9orf58), transcript variant 1, mRNA | | |
| NM_152369 | Homo sapiens hypothetical protein MGC45474 | 112.28 UP | 2.18E-05 |
| | (MGC45474), mRNA | | |
| NM_003810 | Homo sapiens tumor necrosis factor (ligand) | 112.19 UP | 2.96E-05 |
| | superfamily, member 10 (TNFSF10), mRNA | | |
| NM_023942 | Homo sapiens hypothetical protein MGC3036 | 111.27 UP | 1.15E-05 |
| | (MGC3036), mRNA | | |
| NM_005560 | Homo sapiens laminin, alpha 5 (LAMA5), | 110.51 UP | 1.37E-05 |
| | mRNA | | |
| NM_001453 | Homo sapiens forkhead box C1(FOXC1). | 107.21 UP | 1.15E-05 |
| | mRNA | | |
| BU734212 | UI-E-CQ1-agd-e-21-0-UI.s1 UI-E-CQ1 Homo | 103.23 UP | 2.89E-05 |
| | sapiens cDNA clone UI-E-CQ1-agd-e-21-0-UI | | |
| | 3, mRNA sequence | | |
| AK026966 | Homo sapiens cDNA: FLJ23313 fis, clone | 103.17 UP | 1.78E-05 |
| | HEP11919 | | |
| CA425961 | UI-H-FE1-beg-p-18-0-UI.s1 NCI_CGAP_FEI | 102.53 UP | 6.21E-05 |
| | Homo sapiens cDNA clone UI-H-FE1-beg-p-18- | | |
| | 0-UI 3, mRNA sequence | | |
| AK075003 | Homo sapiens cDNA FLJ90522 fis, clone | 101.8 UP | 2.31 E-04 |
| | NT2RP4000108, highly similar to Human gene | | |
| | for neurofilament subunit NF-L | | |
| NM_018265 | Homo sapiens hypothetical protein FLJ10901 | 99.41 UP | 3.66E-05 |
| | (FLJ10901), mRNA | | |
| AK058012 | Homo sapiens cDNA FLJ25283 fis, clone | 98.88 UP | 9.08E-06 |
| | STM06716 | | |
| NM_004098 | Homo sapiens empty spiracles homolog 2 | 97.96 UP | 4.13E-05 |
| | (Drosophila) (EMX2), mRNA | | |
| AK096481 | Homo sapiens cDNA FLJ39162 fis, clone | 94.93 UP | 2.83E-05 |
| | OCBBF2002376 | | |
| NM_003238 | Homo sapiens transforming growth factor, beta | 91.63 UP | 2.31 E-05 |
| | 2 (TGFB2), mRNA | | |
| AI244954 | qj93h05.x1 NCI_CGAP_Kid3 Homo sapiens | 85.24 UP | 1.78E-05 |
| | cDNA clone IMAGE:1867065 3, mRNA | | |
| | sequence | | |
| NM_173505 | Homo sapiens ankyrin repeat domain 29 | 82.89 UP | 3.53E-05 |
| | (ANKRD29), mRNA | | |
| AK130281 | Homo sapiens cDNA FLJ26771 fis, clone | 82.02 UP | 3.66E-06 |
| | PRS03189 | | |
| NM_153026 | Homo sapiens prickle-like 1 (Drosophila) | 81.73 UP | 1.78E-05 |
| | (PRICKLE1), mRNA | | |
| AK092401 | Homo sapiens cDNA FLJ35082 fis, clone | 79.69 UP | 1.70E-05 |
| | PLACE6005351 | | |
| AB011539 | Homo sapiens mRNA for MEGF6 protein | 79.31 UP | 8.41 E-05 |
| | (KIAA0815), partial cds | | |
| NM_016356 | Homo sapiens doublecortin domain containing | 78.04 UP | 6.98E-05 |
| | 2 (DCDC2), mRNA | | |
| AK023631 | Homo sapiens cDNA FLJ13569 fis, clone | 77.68 UP | 2.09E-05 |
| | PLACE 1008369 | | |
| NM_024422 | Homo sapiens desmocollin 2 (DSC2), transcript | 73.14 UP | 7.38E-05 |
| | variant Dsc2a, mRNA | | |
| BX648964 | Homo sapiens mRNA; cDNA DKFZp686J0156 | 70.68 UP | 6.92E-05 |
| | (from clone DKFZp686J0156) | | |
| T56535 | yb33g07.r1 Stratagene fetal spleen (#937205) | 69.74 UP | 2.03E-05 |
| | Homo sapiens cDNA clone IMAGE:73020 5, | | |
| | mRNA sequence | | |
| BG197054 | RST16291 Athersys RAGE Library Homo | 69.48 UP | 4.96E-05 |
| | sapiens cDNA, mRNA sequence | | |
| NM_005949 | Homo sapiens metallothionein 1 F (functional) | 68.1 UP | 2.03E-05 |
| | (MT1F), mRNA | | |
| AK090808 | Homo sapiens cDNA FLJ33489 fis, clone | 65.29 UP | 2.87E-05 |
| | BRAMY2003585 | | |
| CA306881 | UI-H-FT1-bht-n-22-0-UI.s1 NCI_CGAP_FTI | 64.98 UP | 2.93E-05 |
| | Homo sapiens cDNA clone UI-H-FT1-bht-n-22- | | |
| | 0-UI 3, mRNA sequence | | |
| NM_001448 | Homo sapiens glypican 4 (GPC4), mRNA | 64.85 UP | 2.45E-04 |
| AL833276 | Homo sapiens mRNA; cDNA DKFZp451D088 | 64.7 UP | 1.98E-05 |
| | (from clone DKFZp451 D088) | | |
| BC044843 | Homo sapiens hypothetical protein | 63.8 UP | 7.59E-05 |
| | LOC339535, mRNA (cDNA clone | | |
| | IMAGE:5186761), partial cds | | |
| BF798098 | RC1-Ct0045-021000-021-f02 CI0045 Homo | 58.58 UP | 3.04E-05 |
| | sapiens cDNA, mRNA sequence | | |
| H89526 | yw28b04.r1 Morton Fetal Cochlea Homo | 56.92 UP | 3.82E-05 |
| | sapiens cDNA clone IMAGE:253519 5, mRNA | | |
| | sequence | | |
| BF509573 | UI-H-BI4-apf-b-11-0-UI.s1 NCI_CGAP_Sub8 | 56.79 UP | 2.83E-05 |
| | Homo sapiens cDNA clone IMAGE:3086949 3, | | |
| | mRNA sequence | | |
| NM_000990 | Homo sapiens ribosomal protein L27a | 56.36 UP | 1.73E-05 |
| | (RPL27A), mRNA | | |
| BC042028 | Homo sapiens, clone IMAGE:4794726, mRNA | 55.75 UP | 1.64E-05 |
| NM_003287 | Homo sapiens tumor protein D52-like 1 | 55.25 UP | 8.46E-06 |
| | (TPD52L1 transcript variant 1, mRNA | | |
| NM_018168 | Homo sapiens chromosome 14 open reading | 54.64 UP | 4.79E-05 |
| | frame 105 (C14orf105), mRNA | | |
| NM_152284 | Homo sapiens Snf7 homologue associated with | 54.16 UP | 9.09E-06 |
| | Alix 3 (Shax3), mRNA | | |
| AK096975 | Homo sapiens cDNA FLJ39656 fis, clone | 54.14 UP | 8.37E-05 |
| | SMINT2005956 | | |
| A1249696 | qj64a03.x1 NCI_CGAP_Kid3 Homo sapiens | 53.24 UP | 7.14E-05 |
| | cDNA clone IMAGE:1864204 3, mRNA | | |
| | sequence | | |
| NM_003551 | Homo sapiens non-metastatic cells 5, protein | 53.01 UP | 4.75E-05 |
| | expressed in (nucleaside-diphosphate kinase) | | |
| | (NME5), mRNA | | |
| BX118238 | BX118238 NCI_CGAP_Kid3 Homo sapiens | 51.99 UP | 5.64E-05 |
| | cDNA clone IMAGp998L153800; | | |
| | IMAGE:1501598, mRNA sequence | | |
| BX102632 | BX102632 NCI_CGAP_Co3 Homo sapiens | 51.79 UP | 9.10E-05 |
| | cDNA clone IMAGp998J052307; | | |
| | IMAGE:928228, mRNA sequence | | |
| AF055376 | Homo sapiens short form transcription factor C- | 51.22 UP | 3.94E-05 |
| | MAF (c-maf) mRNA, complete cds | | |
| NM_012464 | Homo sapiens tolloid-like 1 (TLL1), mRNA | 51.19 UP | 6.57E-04 |
| NM_030949 | Homo sapiens protein phosphatase 1, | 49.96 UP | 5.80E-05 |
| | regulatory (inhibitor) subunit 14C (PPP1R14C), | | |
| | mRNA | | |
| BM669002 | UI-E-CK1-afn-m-04-0-UI.s2 UI-E-CK1 Homo | 49.05 UP | 1.78E-05 |
| | sapiens cDNA clone UI-E-CK1-afn-m-04-0-UI 3, | | |
| | mRNA sequence | | |
| NM_000582 | Homo sapiens secreted phosphoprotein 1 | 46.79 UP | 6.18E-05 |
| | (osteopontin, bone sialoprotein I, early T- | | |
| | lymphocyte activation 1) (SPP1), mRNA | | |
| NM_006722 | Homo sapiens microphthalmia-associated | 46.73 UP | 1.26E-05 |
| | transcription factor (MITF), transcript variant 3, | | |
| | mRNA | | |
| NM_152423 | Homo sapiens melanoma associated antigen | 46.24 UP | 1.48E-04 |
| | (mutated) 1-like 1 (MUM1L1), mRNA | | |
| AA075748 | zm89e04.r1 Stratagene ovarian cancer | 45.76 UP | 4.39E-05 |
| | (#937219) Homo sapiens cDNA clone | | |
| | IMAGE:545118 5, mRNA sequence | | |
| NM_017549 | Homo sapiens upregulated in colorectal cancer | 45.18 UP | 1.24E-05 |
| | gene 1 (UCC1), mRNA | | |
| BF431030 | 7o18c06.x1 NCI_CGAP_Kid11 Homo sapiens | 44.98 UP | 7.75E-05 |
| | cDNA clone IMAGE:3574283 3, mRNA | | |
| | sequence | | |
| NM_002245 | Homo sapiens potassium channel, subfamily K, | 44.3 UP | 4.65E-05 |
| | member 1 (KCNK1), mRNA | | |
| NM_003494 | Homo sapiens dysferlin, limb girdle muscular | 44.23 UP | 1.78E-05 |
| | dystrophy 2B (autosomal recessive) (DYSF), | | |
| | mRNA | | |
| AK074097 | Homo sapiens mRNA for FLJ00168 protein | 43.88 UP | 7.31 E-05 |
| NM_013410 | Homo sapiens adenylate kinase 3 (AK3), | 42.34 UP | 2.96E-05 |
| | nuclear gene encoding mitochondrial protein, | | |
| | transcript variant 2, mRNA | | |
| AK000075 | Homo sapiens cDNA FLJ20068 fis, clone | 42.18 UP | 1.35E-04 |
| | COL01755 | | |
| NM_001200 | Homo sapiens bone morphogenetic protein 2 | 41.13 UP | 2.11 E-05 |
| | (BMP2), mRNA | | |
| NM_032782 | Homo sapiens hepatitis A virus cellular receptor | 41.13 UP | 2.83E-05 |
| | 2 (HAVCR2), mRNA | | |
| AW 151660 | xf67d04.x1 NCI_CGAP_Gas4 Homo sapiens | 40.6 UP | 8.65E-05 |
| | cDNA clone IMAGE:2623111 3, mRNA | | |
| | sequence | | |
| NM_130435 | Homo sapiens protein tyrosine phosphatase, | 40.41 UP | 1.62E-04 |
| | receptor type, E (PTPRE), transcript variant 2, | | |
| | mRNA | | |
| AA738254 | nx13b02.s1 NCI_CGAP_GC3 Homo sapiens | 40.24 UP | 2.43E-05 |
| | cDNA clone IMAGE:1255947 3, mRNA | | |
| | sequence | | |
| AK124873 | Homo sapiens cDNA FLJ42883 fis, clone | 40.2 UP | 1.25E-04 |
| | BRHIP3006683 | | |
| NM_000170 | Homo sapiens glycine dehydrogenase | 40.06 UP | 4.98E-05 |
| | (decarboxylating; glycine decarboxylase, | | |
| | glycine cleavage system protein P) (GLDC), | | |
| | mRNA | | |
| AK125490 | Homo sapiens cDNA FLJ43501 fis, clone | 39.93 UP | 1.64E-04 |
| | PEBLM2004497 | | |
| NM_004862 | Homo sapiens lipopolysaccharide-induced TNF | 39.52 UP | 1.37E-05 |
| | factor (LITAF), mRNA | | |
| NM_004617 | Homo sapiens transmembrane 4 superfamily | 39.3 UP | 9.32E-05 |
| | member 4 (TM4SF4), mRNA | | |
| NM_001263 | Homo sapiens CDP-diacylglycerol synthase | 39.26 UP | 3.44E-05 |
| | (phosphatidate cytidylyltransferase) 1 (CDS1), | | |
| | mRNA | | |
| AK000776 | Homo sapiens cDNA FLJ20769 fis, clone | 38.12 UP | 2.83E-05 |
| | COL06674 | | |
| AL833166 | Homo sapiens mRNA; cDNA DKFZp68612118 | 37.72 UP | 1.14E-04 |
| | (from clone DKFZp68612118) | | |
| NM_198488 | Homo sapiens FLJ46072 protein (FLJ46072), | 37.66 UP | 2.46E-05 |
| | mRNA | | |
| NM_005562 | Homo sapiens laminin, gamma 2 (LAMC2), | 37.4 UP | 8.46E-06 |
| | transcript variant 1, mRNA | | |
| AW268540 | xv51e10.x1 NCI_CGAP_Lu28 Homo sapiens | 37.26 UP | 5.82E-05 |
| | cDNA clone IMAGE:2816682 3, mRNA | | |
| | sequence | | |
| NM_012198 | Homo sapiens grancalcin, EF-hand calcium | 36.52 UP | 2.13E-05 |
| | binding protein (GCA), mRNA | | |
| NM_020808 | Homo sapiens signal-induced proliferation- | 36.28 UP | 9.39E-05 |
| | associated 1 like 2 (SIPA1L2), mRNA | | |
| NM_000927 | Homo sapiens ATP-binding cassette, sub- | 35.89 UP | 2.20E-04 |
| | family B (MDR/TAP), member 1 (ABCB1), | | |
| | mRNA | | |
| AK127644 | Homo sapiens cDNA FLJ45742 fis, clone | 35.81 UP | 3.60E-04 |
| | KIDNE2016327 | | |
| NM_006158 | Homo sapiens neurofilament, light polypeptide | 35.78 UP | 2.64E-05 |
| | 68kDa (NEFL), mRNA | | |
| AK074181 | Homo sapiens mRNA for FLJ00254 protein | 35.75 UP | 8.46E-06 |
| BC043195 | Homo sapiens cDNA clone IMAGE:5288757, | 35.12 UP | 1.24E-04 |
| | partial cds | | |
| AL389942 | Homo sapiens mRNA full length insert cDNA | 34.31 UP | 1.37E-04 |
| | clone EUROIMAGE 2005635 | | |
| BC045828 | Homo sapiens zinc finger protein 608, mRNA | 34.02 UP | 1.88E-05 |
| | (cDNA clone IMAGE:5262896), partial cds | | |
| NM_153229 | Homo sapiens hypothetical protein FLJ33318 | 34.02 UP | 9.39E-05 |
| | (FLJ33318),mRNA | | |
| NM_184087 | Homo sapiens tripartite motif-containing 55 | 33.68 UP | 2.18E-05 |
| | (TRIM55), transcript variant 4, mRNA | | |
| NM_000216 | Homo sapiens Kallmann syndrome 1 sequence | 33.32 UP | 1.78E-05 |
| | (KAL1), mRNA | | |
| BF696790 | 602125323F1 NIH_MGC_56 Homo sapiens | 32.7 UP | 5.00E-05 |
| | cDNA clone IMAGE:4282540 5, mRNA | | |
| | sequence | | |
| NM_006598 | Homo sapiens solute carrier family 12 | 32.41 UP | 8.39E-06 |
| | (potassium/chloride transporters), member 7 | | |
| | (SLC12A7), mRNA | | |
| BQ003401 | UI-H-EI1-azd-j-23-0-UI.s1 NCI_CGAP_EI1 | 32.41 UP | 1.72E-05 |
| | Homo sapiens cDNA clone IMAGE:5847286 3, | | |
| | mRNA sequence | | |
| NM_003222 | Homo sapiens transcription factor AP-2 gamma | 32.33 UP | 4.75E-05 |
| | (activating enhancer binding protein 2 gamma) | | |
| | (TFAP2C), mRNA | | |
| NM_000599 | Homo sapiens insulin-like growth factor binding | 32.1 UP | 5.66E-05 |
| | protein 5 (IGFBP5), mRNA | | |
| NM_198389 | Homo sapiens lung type-I cell membrane- | 31.44 UP | 1.03E-04 |
| | associated glycoprotein (T1A-2), transcript | | |
| | variant 2, m RNA | | |
| NM_180991 | Homo sapiens solute carrier organic anion | 30.86 UP | 3.05E-04 |
| | transporter family, member 4C1 (SLC04C1), | | |
| | mRNA | | |
| NM_031311 | Homo sapiens carboxypeptidase, vitellogenic- | 29.97 UP | 4.30E-05 |
| | like (CPVL), transcript variant 1, mRNA | | |
| NM_052947 | Homo sapiens heart alpha-kinase (HAK), | 29.53 UP | 4.34E-05 |
| | mRNA | | |
| H89053 | yw24c06.r1 Morton Fetal Cochlea Homo | 29.27 UP | 2.78E-05 |
| | sapiens cDNA clone IMAGE:253162 5, mRNA | | |
| | sequence | | |
| AI819186 | wj32d10.x1 NCI_CGAP _Kid12 Homo sapiens | 29.14 UP | 1.21 E-04 |
| | cDNA clone IMAGE:2404531 3, mRNA | | |
| | sequence | | |
| NM_003761 | Homo sapiens vesicle-associated membrane | 28.5 UP | 7.47E-05 |
| | protein 8 (endobrevin) (VAMP8), mRNA | | |
| NM_080743 | Homo sapiens serine-arginine repressor protein | 27.83 UP | 1.02E-05 |
| | (35 kDa) (SRrp35), mRNA | | |
| NM_007069 | Homo sapiens HRAS-like suppressor 3 | 27.51 UP | 6.86E-06 |
| | (HRASLS3), mRNA | | |
| NM_002345 | Homo sapiens lumican (LUM), mRNA | 27.17 UP | 2.20E-05 |
| BU569937 | AGENCOURT_1039981 NIH_MGC_82 Homo | 27.05 UP | 3.20E-05 |
| | sapiens cDNA clone IMAGE:6618011 5, mRNA | | |
| | sequence | | |
| NM_024901 | Homo sapiens hypothetical protein FLJ22457 | 27.04 UP | 5.37E-05 |
| | (FLJ22457), mRNA | | |
| N25875 | yw78d12.s1 | 26.94 UP | 3.36E-05 |
| | Soares_placenta_8to9weeks_2NbHP8to9W | | |
| | Homo sapiens cDNA clone IMAGE:258359 3, | | |
| | mRNA sequence | | |
| NM_152573 | Homo sapiens RAS and EF hand domain | 26.91 UP | 4.75E-05 |
| | containing (RASEF), mRNA | | |
| NM_018728 | Homo sapiens myosin VC (MY05C), mRNA | 26.81 UP | 4.47E-05 |
| AB033048 | Homo sapiens mRNA for KIAA1222 protein, | 26.76 UP | 1.90E-04 |
| | partial cds | | |
| NM_153256 | Homo sapiens chromosome 10 open reading | 26.61 UP | 2.48E-05 |
| | frame 47 (C10orf47), mRNA | | |
| BG221364 | RST41175 Athersys RAGE Library Homo | 26.25 UP | 2.06E-04 |
| | sapiens cDNA, mRNA sequence | | |
| NM_000094 | Homo sapiens collagen, type Vil, alpha 1 | 26.16 UP | 6.51 E-05 |
| | (epidermolysis bullosa, dystrophic, dominant | | |
| | and recessive) (COL7A1), mRNA | | |
| NM_001854 | Homo sapiens collagen, type XI, alpha 1 | 25.88 UP | 1.36E-04 |
| | (COL11A1), transcript variant A, mRNA | | |
| BU740051 | UI-E-EO0-ahw-n-18-0-UI.s1 UI-E-EO0 Homo | 25.64 UP | 5.14E-05 |
| | sapiens cDNA clone UI-E-EO0-ahw-n-18-0-UI | | |
| | 3, mRNA sequence | | |
| NM_014936 | Homo sapiens ectonucleotide | 25.62 UP | 2.82E-04 |
| | pyrophosphatase/phosphodiesterase 4 | | |
| | (putative function) (ENPP4), mRNA | | |
| NM_024898 | Homo sapiens family with sequence similarity | 25.44 UP | 7.17E-04 |
| | 31, member C (FAM31C), mRNA | | |
| W93585 | zd95g01.s1 Soares_fetal_heart_NbHH19W | 25.41 UP | 1.78E-05 |
| | Homo sapiens cDNA clone IMAGE:357264 3, | | |
| | mRNA sequence | | |
| BF510493 | UI-H-BI4-apa-b-08-0-UI.s1 NCI_CGAP_Sub8 | 25.28 UP | 2.11E-05 |
| | Homo sapiens cDNA clone IMAGE:3086558 3, | | |
| | mRNA sequence | | |
| NM_020962 | Homo sapiens likely ortholog of mouse | 25.23 UP | 7.99E-05 |
| | neighbor of Punc E11 (NOPE), mRNA | | |
| NM_016212 | Homo sapiens TP53TG3 protein (TP53TG3), | 25.14 UP | 4.07E-05 |
| | mRNA | | |
| NM_021012 | Homo sapiens potassium inwardly-rectifying | 24.7 UP | 2.10E-04 |
| | channel, subfamily J, member 12 (KCNJ12), | | |
| | mRNA | | |
| NM_001935 | Homo sapiens dipeptidylpeptidase 4 (CD26, | 24.67 UP | 8.46E-06 |
| | adenosine deaminase complexing protein 2) | | |
| | (DPP4), mRNA | | |
| NM_001993 | Homo sapiens coagulation factor III | 24.47 UP | 6.12E-05 |
| | (thromboplastin, tissue factor) (F3), mRNA | | |
| NM_184087 | Homo sapiens tripartite motif-containing 55 | 24.38 UP | 8.46E-06 |
| | (TRIM55), transcript variant 4, mRNA | | |
| AK056431 | Homo sapiens cDNA FLJ31869 fis, clone | 24.34 UP | 2.73E-05 |
| | NT2RP7002151 | | |
| NM_000775 | Homo sapiens cytochrome P450, family 2, | 24.04 UP | 3.53E-05 |
| | subfamily J, polypeptide 2 (CYP2J2), mRNA | | |
| NM_006722 | Homo sapiens microphthalmia-associated | 23.95 UP | 5.23E-06 |
| | transcription factor (MITF), transcript variant 3, | | |
| | mRNA | | |
| NM_001977 | Homo sapiens glutamyl aminopeptidase | 23.91 UP | 1.88E-05 |
| | (aminopeptidase A) (ENPEP), mRNA | | |
| AK058012 | Homo sapiens cDNA FLJ25283 fis, clone | 23.55 UP | 1.88E-05 |
| | STM06716 | | |
| BU680661 | UI-CF-DU1-aaz-f-04-0-UI.s1 UI-CF-DU1 Homo | 23.17 UP | 1.82E-05 |
| | sapiens cDNA clone UI-CF-DU1-aaz-f-04-0-UI | | |
| | 3, mRNA sequence | | |
| NM_002246 | Homo sapiens potassium channel, subfamily K, | 22.96 UP | 2.27E-05 |
| | member 3 (KCNK3), mRNA | | |
| NM_052923 | Homo sapiens zinc finger protein 452 | 22.72 UP | 3.77E-05 |
| | (ZNF452), mRNA | | |
| NM_014373 | Homo sapiens G protein-coupled receptor 160 | 22.71 UP | 3.94E-05 |
| | (GPR160), mRNA | | |
| BX538226 | Homo sapiens mRNA; cDNA DKFZp686E1944 | 22.58 UP | 1.05E-04 |
| | (from clone DKFZp686E1944) | | |
| NM_173660 | Homo sapiens hypothetical protein FLJ33718 | 22.46 UP | 2.83E-05 |
| | (FLJ33718), mRNA | | |
| NM_002354 | Homo sapiens tumor-associated calcium signal | 21.97 UP | 2.83E-05 |
| | transducer 1 (TACSTD1), mRNA | | |
| NM_005822 | Homo sapiens Down syndrome critical region | 21.78 UP | 2.21 E-04 |
| | gene 1-like 1 (DSCR1L1), mRNA | | |
| W20132 | zb40c10.r1 Soares_parathyroid_tumor_NbHPA | 21.76 UP | 4.41E-04 |
| | Homo sapiens cDNA clone IMAGE:306066 5, | | |
| | mRNA sequence | | |
| NM_001680 | Homo sapiens FXYD domain containing ion | 21.75 UP | 3.26E-05 |
| | transport regulator 2 (FXYD2), transcript variant | | |
| | a, mRNA | | |
| NM_006379 | Homo sapiens sema domain, immunoglobulin | 21.57 UP | 3.15E-05 |
| | domain (Ig), short basic domain, secreted, | | |
| | (semaphorin) 3C (SEMA3C), mRNA | | |
| H23441 | ym52f11.s1 Soares infant brain 1NIB Homo | 21.43 UP | 3.51 E-05 |
| | sapiens cDNA clone IMAGE:51888 3, mRNA | | |
| | sequence | | |
| BG211832 | RST31404 Athersys RAGE Library Homo | 21.35 UP | 5.06E-05 |
| | sapiens cDNA, mRNA sequence | | |
| N51335 | yz15e08.s1 | 21.19 UP | 6.92E-03 |
| | Soares_multiple sclerosis_2NbHMSP Homo | | |
| | sapiens cDNA clone IMAGE:283142 3, mRNA | | |
| | sequence | | |
| NM_022843 | Homo sapiens protocadherin 20 (PCDH20), | 21.17 UP | 2.03E-05 |
| | mRNA | | |
| NM_002521 | Homo sapiens natriuretic peptide precursor B | 21.01 UP | 4.54E-04 |
| | (NPPB), mRNA | | |
| NM_021101 | Homo sapiens claudin 1 (CLDN1), mRNA | 20.92 UP | 3.04E-05 |
| NM_004165 | Homo sapiens Ras-related associated with | 20.76 UP | 6.04E-04 |
| | diabetes (RRAD), mRNA | | |
| NM_005329 | Homo sapiens hyaluronan synthase 3 (HAS3), | 20.73 UP | 4.13E-05 |
| | transcript variant 1, mRNA | | |
| NM_138432 | Homo sapiens serine dehydratase-like (SDSL), | 20.41 UP | 7.27E-05 |
| | mRNA | | |
| NM_004591 | Homo sapiens chemokine (C-C motif) ligand 20 | 20.34 UP | 3.29E-04 |
| | (CCL20), mRNA | | |
| NM_003063 | Homo sapiens sarcolipin (SLN), mRNA | 20.26 UP | 1.18E-04 |
| BU951469 | in60a05.x3 HR85 islet Homo sapiens cDNA | 20.02 UP | 4.52E-04 |
| | clone IMAGE:6126249 3, mRNA sequence | | |
| AI436290 | th81c01.x1 Soares_NhHMPu_S1 Homo | 19.92 UP | 9.28E-05 |
| | sapiens cDNA clone IMAGE:2125056 3, mRNA | | |
| | sequence | | |
| NM_014452 | Homo sapiens tumor necrosis factor receptor | 19.39 UP | 2.96E-05 |
| | superfamily, member 21 (TNFRSF21), mRNA | | |
| AA099748 | zI78c09.s1 Stratagene colon (#937204) Homo | 19.2 UP | 1.78E-05 |
| | sapiens cDNA clone IMAGE:510736 3, mRNA | | |
| | sequence | | |
| NM_018354 | Homo sapiens chromosome 20 open reading | 18.87 UP | 5.05E-04 |
| | frame 46 (C20orf46), mRNA | | |
| NM_194298 | Homo sapiens solute carrier family 16 | 18.87 UP | 9.82E-03 |
| | (monocarboxylic acid transporters), member 9 | | |
| | (SLC16A9), mRNA | | |
| NM_033554 | Homo sapiens major histocompatibility | 18.6 UP | 5.61 E-04 |
| | complex, class II, DP alpha 1 (HLA-DPA1), | | |
| | mRNA | | |
| NM_004524 | Homo sapiens lethal giant larvae homolog 2 | 18.45 UP | 2.11 E-05 |
| | (Drosophila) (LLGL2), mRNA | | |
| NM_032293 | Homo sapiens GTPase activating Rap/RanGAP | 18.19 UP | 2.24E-04 |
| | domain-like 3 (GARNL3), mRNA | | |
| NM_022842 | Homo sapiens CUB domain-containing protein | 18.14 UP | 1.64E-05 |
| | 1 (CDCP1), transcript variant 1, mRNA | | |
| NM_000227 | Homo sapiens laminin, alpha 3 (LAMA3), | 18.04 UP | 3.92E-05 |
| | transcript variant 2, mRNA | | |
| NM_000214 | Homo sapiens jagged 1 (Alagille syndrome) | 18.03 UP | 1.26E-04 |
| | (JAG1), mRNA | | |
| AK096580 | Homo sapiens cDNA FLJ39261 fis, clone | 17.89 UP | 3.05E-04 |
| | OCBBF2009391 | | |
| NM_014243 | Homo sapiens a disintegrin-like and | 17.86 UP | 1.78E-05 |
| | metalloprotease (reprolysin type) with | | |
| | thrombospondin type 1 motif, 3 (ADAMTS3), | | |
| | mRNA | | |
| NM_000856 | Homo sapiens guanylate cyclase 1, soluble, | 17.85 UP | 1.19E-04 |
| | alpha 3 (GUCY1A3), mRNA | | |
| NM_018404 | Homo sapiens centaurin, alpha 2 (CENTA2), | 17.8 UP | 3.05E-04 |
| | mRNA | | |
| NM_001165 | Homo sapiens baculoviral IAP repeat- | 17.74 UP | 2.56E-04 |
| | containing 3 (BIRC3), transcript variant 1, | | |
| | mRNA | | |
| NM_003706 | Homo sapiens phospholipase A2, group IVC | 17.65 UP | 4.77E-05 |
| | (cytosolic, calcium-independent) (PLA2G4C), | | |
| | mRNA | | |
| AK056882 | Homo sapiens cDNA FLJ32320 fis, clone | 17.65 UP | 4.33E-04 |
| | PROST2003537 | | |
| NM_017640 | Homo sapiens leucine rich repeat containing 16 | 17.43 UP | 1.04E-04 |
| | (LRRC16), mRNA | | |
| NM_000480 | Homo sapiens adenosine monophosphate | 17.4 UP | 1.15E-05 |
| | deaminase (isoform E) (AMPD3), mRNA | | |
| BX103476 | BX103476 NCI_CGAP_Lu5 Homo sapiens | 17.29 UP | 7.13E-05 |
| | cDNA clone IMAGp998C053946; | | |
| | IMAGE:1557436, mRNA sequence | | |
| NM_013427 | Homo sapiens Rho GTPase activating protein 6 | 17.27 UP | 1.48E-04 |
| | (ARHGAP6), transcript variant 1, mRNA | | |
| S70348 | Homo sapiens integrin beta 3 mRNA, partial | 17.24 UP | 3.37E-05 |
| | cds, alternatively spliced | | |
| NM_032024 | Homo sapiens chromosome 10 open reading | 16.95 UP | 3.03E-05 |
| | frame 11 (C10orf11), mRNA | | |
| BM988642 | UI-H-DH0-arx-p-21-0-UI.s1 NCI_CGAP_DHO | 16.94 UP | 1.94E-04 |
| | Homo sapiens cDNA clone IMAGE:5855492 3, | | |
| | mRNA sequence | | |
| NM_021102 | Homo sapiens serine protease inhibitor, Kunitz | 16.94 UP | 1.85E-04 |
| | type, 2 (SPINT2), mRNA | | |
| A1632692 | wa33b05.x1 NCI_CGAP_Kid11 Homo sapiens | 16.88 UP | 7.19E-04 |
| | cDNA clone IMAGE:2299857 3, mRNA | | |
| | sequence | | |
| NM_152487 | Homo sapiens hypothetical protein FLJ31842 | 16.75 UP | 2.55E-04 |
| | (FLJ31842), mRNA | | |
| NM_173567 | Homo sapiens abhydrolase domain containing | 16.66 UP | 2.13E-05 |
| | 7 (ABHD7), mRNA | | |
| CA502927 | UI-CF-FN0-afq-j-12-0-UI.s1 UI-CF-FNO Homo | 16.59 UP | 2.35E-04 |
| | sapiens cDNA clone UI-CF-FNO-afq-j-12-0-UI 3, | | |
| | mRNA sequence | | |
| NM_015888 | Homo sapiens hook homolog 1 (Drosophila) | 16.44 UP | 4.12E-05 |
| | (HOOK1), mRNA | | |
| NM_178177 | Homo sapiens nicotinamide nucleotide | 16.43 UP | 1.56E-04 |
| | adenylyltransferase 3 (NMNAT3), mRNA | | |
| NM_005181 | Homo sapiens carbonic anhydrase III, muscle | 16.42 UP | 4.93E-05 |
| | specific (CA3), mRNA | | |
| NM_000599 | Homo sapiens insulin-like growth factor binding | 16.27 UP | 4.97E-04 |
| | protein 5 (IGFBP5), mRNA | | |
| NM_006393 | Homo sapiens nebulette (NEBL), transcript | 16.13 UP | 6.06E-04 |
| | variant 1, mRNA | | |
| NM_006252 | Homo sapiens protein kinase, AMP-activated, | 16.12 UP | 5.64E-04 |
| | alpha 2 catalytic subunit (PRKAA2), mRNA | | |
| NM_002338 | Homo sapiens limbic system-associated | 16.11 UP | 2.81 E-04 |
| | membrane protein (LSAMP), mRNA | | |
| NM_006378 | Homo sapiens sema domain, immunoglobulin | 16.09 UP | 1.78E-05 |
| | domain (Ig), transmembrane domain (TM) and | | |
| | short cytoplasmic domain, (semaphorin) 4D | | |
| | (SEMA4D), mRNA | | |
| AI819863 | wj45h05.x1 NCI_CGAP_Lu19 Homo sapiens | 15.95 UP | 3.04E-04 |
| | cDNA clone IMAGE:2405817 3, mRNA | | |
| | sequence | | |
| BM979825 | UI-CF-DU1-adt-f-12-0-UI.s1 UI-CF-DU1 Homo | 15.9 UP | 2.77E-05 |
| | sapiens cDNA clone UI-CF-DU1-adt-f-12-0-UI | | |
| | 3, mRNA sequence | | |
| BX647876 | Homo sapiens mRNA; cDNA OKFZp313A1525 | 15.83 UP | 4.39E-05 |
| | (from clone DKFZp313A1525) | | |
| NM_003730 | Homo sapiens ribonuclease T2 (RNASET2), | 15.6 UP | 2.41E-05 |
| | mRNA | | |
| AL117425 | Homo sapiens mRNA; cDNA DKFZp566L203 | 15.59 UP | 3.31 E-04 |
| | (from clone DKFZp566L203) | | |
| AL833346 | Homo sapiens mRNA; cDNA DKFZp686M2234 | 15.59 UP | 2.99E-05 |
| | (from clone DKFZp686M2234) | | |
| NM_199169 | Homo sapiens transmembrane, prostate | 15.58 UP | 1.34E-04 |
| | androgen induced RNA (TMEPAI), transcript | | |
| | variant 2, mRNA | | |
| NM_002559 | Homo sapiens purinergic receptor P2X, ligand- | 15.51 UP | 9.39E-05 |
| | gated ion channel, 3 (P2RX3), mRNA | | |
| NM_032148 | Homo sapiens solute carrier family 41, member | 15.46 UP | 2.38E-05 |
| | 2 (SLC41A2), mRNA | | |
| BC021684 | Homo sapiens, clone IMAGE:3827252, mRNA | 15.41 UP | 2.31 E-05 |
| BF000009 | 7h15g04.x1 NCI_CGAP_Co16 Homo sapiens | 15.33 UP | 2.09E-05 |
| | cDNA clone IMAGE:3316086 3, mRNA | | |
| | sequence | | |
| NM_000147 | Homo sapiens fucosidase, alpha-L-1, tissue | 15.31 UP | 9.99E-04 |
| | (FUCA1), mRNA | | |
| NM_001252 | Homo sapiens tumor necrosis factor (ligand) | 14.88 UP | 1.85E-04 |
| | superfamily, member 7 (TNFSF7), mRNA | | |
| NM_013322 | Homo sapiens sorting nexin 10 (SNX10), | 14.84 UP | 2.64E-05 |
| | mRNA | | |
| NM_022440 | Homo sapiens mal, T-cell differentiation protein | 14.65 UP | 2.14E-04 |
| | (MAL), transcript variant d, mRNA | | |
| NM_178470 | Homo sapiens WD repeat domain 40B | 14.53 UP | 3.34E-05 |
| | (WDR40B), mRNA | | |
| NM_001873 | Homo sapiens carboxypeptidase E (CPE), | 14.51 UP | 6.23E-05 |
| | mRNA | | |
| BM719937 | UI-E-EJ0-ahu-a-10-0-UI.r1 UI-E-EJ0 Homo | 14.46 UP | 1.64E-05 |
| | sapiens cDNA clone UI-E-EJO-ahu-a-10-0-UI 5, | | |
| | mRNA sequence | | |
| NM_032551 | Homo sapiens G protein-coupled receptor 54 | 14.45 UP | 5.64E-04 |
| | (GPR54), mRNA | | |
| T53523 | ya89h12.r1 Stratagene placenta (#937225) | 14.42 UP | 1.81E-04 |
| | Homo sapiens cDNA clone IMAGE:68903 5, | | |
| | mRNA sequence | | |
| NM_173354 | Homo sapiens SNF1-like kinase (SNF1LK), | 14.19 UP | 5.72E-05 |
| | mRNA | | |
| NM_014585 | Homo sapiens solute carrier family 40 (iron- | 14.17 UP | 4.06E-05 |
| | regulated transporter), member 1 (SLC40A1), | | |
| | mRNA | | |
| N38753 | yy42d01.s1 Soares melanocyte 2NbHM Homo | 14.02 UP | 1.15E-05 |
| | sapiens cDNA clone IMAGE:273889 3, mRNA | | |
| | sequence | | |
| NM_001710 | Homo sapiens B-factor, properdin (BF), mRNA | 13.95 UP | 2.05E-04 |
| BC038556 | Homo sapiens, clone IMAGE:3446976, mRNA | 13.9 UP | 4.45E-04 |
| AK125608 | Homo sapiens cDNA FLJ43620 fis, clone | 13.88 UP | 2.56E-04 |
| | SPLEN2021701, highly similar to HLA CLASS | | |
| | I HISTOCOMPATIBILITY ANTIGEN, A-2 | | |
| | ALPHA CHAIN PRECURSOR | | |
| AW248516 | 2820632.3prime NIH_MGC_7 Homo sapiens | 13.79 UP | 2.22E-04 |
| | cDNA clone IMAGE:2820632 3, mRNA | | |
| | sequence | | |
| NM_000600 | Homo sapiens interleukin 6 (interferon, beta 2) | 13.62 UP | 1.64E-05 |
| | (IL6), mRNA | | |
| AK125852 | Homo sapiens cDNA FLJ43864 fis, clone | 13.46 UP | 1.66E-04 |
| | TEST14007799 | | |
| BM728728 | UI-E-EO1-aiv-c-02-0-UI.r1 UI-E-EO1 Homo | 13.46 UP | 2.77E-04 |
| | sapiens cDNA clone UI-E-EO1-aiv-c-02-0-UI 5, | | |
| | mRNA sequence | | |
| NM_018650 | Homo sapiens MAP/microtubule affinity- | 13.45 UP | 4.39E-05 |
| | regulating kinase 1 (MARK1), mRNA | | |
| NM_022154 | Homo sapiens solute carrier family 39 (zinc | 13.39 UP | 4.78E-05 |
| | transporter), member 8 (SLC39A8), mRNA | | |
| AA908815 | og77h08.s1 NCI_CGAP_Ov8 Homo sapiens | 13.23 UP | 3.11E-05 |
| | cDNA clone IMAGE:1454367 3, mRNA | | |
| | sequence | | |
| NM_016463 | Homo sapiens CXXC finger 5 (CXXC5), mRNA | 13.22 UP | 9.67E-05 |
| AB011538 | Homo sapiens mRNA for MEGF5, partial cds | 13.19 UP | 3.38E-04 |
| CA413744 | UI-H-EZ0-bat-h-12-0-UI.s1 NCI_CGAP_Ch1 | 13.17 UP | 2.09E-05 |
| | Homo sapiens cDNA clone UI-H-EZ0-bat-h-12- | | |
| | 0-UI 3, mRNA sequence | | |
| BM712072 | UI-E-DW1-ahc-b-11-0-UI.r1 UI-E-DW1 Homo | 13.16 UP | 9.39E-05 |
| | sapiens cDNA clone UI-E-DW1-ahc-b-11-0-UI | | |
| | 5, mRNA sequence | | |
| BU727096 | UI-E-CRO-ach-e-12-0-UI.s1 UI-E-CR0 Homo | 13.14 UP | 4.30E-05 |
| | sapiens cDNA clone UI-E-CR0-ach-e-12-0-UI 3, | | |
| | RNA sequence | | |
| NM_032858 | Homo sapiens hypothetical protein FLJ14904 | 13.09 UP | 1.83E-05 |
| | (FLJ14904), mRNA | | |
| BU729783 | UI-E-CK1-afh-h-18-0-UI.s1 UI-E-CK1 Homo | 12.92 UP | 8.39E-06 |
| | sapiens cDNA clone UI-E-CK1-afh-h-18-0-UI 3, | | |
| | mRNA sequence | | |
| AB011095 | Homo sapiens mRNA for KIAA0523 protein, | 12.91 UP | 3.44E-04 |
| | partial cds | | |
| BC033116 | Homo sapiens chromodomain helicase DNA | 12.87 UP | 2.31 E-05 |
| | binding protein 7, mRNA (cDNA clone | | |
| | IMAGE:3352674), partial cds | | |
| CB135276 | K-EST0187371 L5HLK1 Homo sapiens cDNA | 12.75 UP | 4.93E-05 |
| | clone L5HLK1-32-B12 5, mRNA sequence | | |
| NM_182920 | Homo sapiens a disintegrin-like and | 12.73 UP | 9.10E-05 |
| | metalloprotease (reprolysin type) with | | |
| | thrombospondin type 1 motif, 9 (ADAMTS9), | | |
| | transcript variant 1, mRNA | | |
| NM_000236 | Homo sapiens lipase, hepatic (LIPC), mRNA | 12.71 UP | 1.16E-03 |
| NM_152694 | Homo sapiens zinc finger, CCHC domain | 12.66 UP | 4.93E-05 |
| | containing 5 (ZCCHC5), mRNA | | |
| AB020640 | Homo sapiens mRNA for KIAA0833 protein, | 12.51 UP | 2.31 E-04 |
| | partial cds | | |
| NM_018330 | Homo sapiens KIAA1598 (KIAA1598), mRNA | 12.46 UP | 6.01 E-05 |
| NM_000064 | Homo sapiens complement component 3 (C3), | 12.42 UP | 5.12E-04 |
| | mRNA | | |
| NM_152864 | Homo sapiens chromosome 20 open reading | 12.4 UP | 1.66E-03 |
| | frame 58 (C20orf58), mRNA | | |
| AW591723 | xt85h10.x1 NCI_CGAP_Ut1 Homo sapiens | 12.4 UP | 3.71 E-05 |
| | cDNA clone IMAGE:2793283 3 similar to | | |
| | contains element MER32 repetitive element ;, | | |
| | m RNA sequence | | |
| NM_152768 | Homo sapiens hypothetical protein FLJ25378 | 12.28 UP | 2.03E-05 |
| | (FLJ25378), mRNA | | |
| NM_152366 | Homo sapiens kelch/ankyrin repeat containing | 12.19 UP | 2.62E-05 |
| | cyclin A1 interacting protein (KARCA1), | | |
| | transcript variant 1, mRNA | | |
| AW300043 | xs45a09.x1 NCI_CGAP_Kid11 Homo sapiens | 12.13UP | 2.31E-05 |
| | cDNA clone IMAGE:2772568 3, mRNA | | |
| | sequence | | |
| NM_006741 | Homo sapiens protein phosphatase 1, | 12.12 UP | 2.10E-04 |
| | regulatory (inhibitor) subunit 1A (PPP1R1A), | | |
| | mRNA | | |
| NM_024726 | Homo sapiens IQ motif containing with AAA | 12.08 UP | 5.33E-03 |
| | domain (IQCA), mRNA | | |
| NM_194284 | Homo sapiens claudin 23 (CLDN23), mRNA | 12.02 UP | 2.87E-05 |
| AL049974 | Homo sapiens mRNA; cDNA DKFZp564B222 | 12 UP | 1.48E-04 |
| | (from clone DKFZp564B222) | | |
| AI420213 | te92g09.x1 NCI_CGAP_Pr28 Homo sapiens | 11.95 UP | 3.59E-04 |
| | cDNA clone IMAGE:2094208 3, mRNA | | |
| | sequence | | |
| NM_002800 | Homo sapiens proteasome (prosome, | 11.85 UP | 4.69E-05 |
| | macropain) subunit, beta type, 9 (large | | |
| | multifunctional protease 2) (PSMB9), transcript | | |
| | variant 1, mRNA | | |
| CA391258 | cs13a10.x1 Human Retinal pigment | 11.85 UP | 3.64E-05 |
| | epithelium/choroid cDNA (Un-normalized, | | |
| | unamplified): cs Homo sapiens cDNA clone | | |
| | cs13a10 3, mRNA sequence | | |
| NM_013430 | Homo sapiens gamma-glutamyltransferase 1 | 11.79 UP | 1.38E-04 |
| | (GGT1 transcript variant 3, mRNA | | |
| TM_199169 | Homo sapiens transmembrane, prostate | 11.79 UP | 8.39E-06 |
| | androgen induced RNA (TMEPAI), transcript | | |
| | variant 2, mRNA | | |
| NM_032471 | Homo sapiens protein kinase (cAMP- | 11.73 UP | 1.28E-04 |
| | dependent, catalytic) inhibitor beta (PKIB), | | |
| | transcript variant 3, mRNA | | |
| NM_016269 | Homo sapiens lymphoid enhancer-binding | 11.72 UP | 4.57E-05 |
| | factor 1 (LEF1), mRNA | | |
| NM_003851 | Homo sapiens cellular repressor of E1A- | 11.61 UP | 1.83E-05 |
| | stimulated genes 1 (CREG1), mRNA | | |
| NM_003236 | Homo sapiens transforming growth factor, | 11.61 UP | 6.51 E-05 |
| | alpha (TGFA), mRNA | | |
| BX110418 | BX110418 NCI_CGAP_Kid3 Homo sapiens | 11.59 UP | 1.89E-05 |
| | cDNA clone IMAGp998C224149 ; | | |
| | IMAGE:1635405, mRNA sequence | | |
| NM_006033 | Homo sapiens lipase, endothelial (LIPG), | 11.55 UP | 8.79E-04 |
| | mRNA | | |
| NM_025151 | Homo sapiens RAB11 family interacting protein | 11.5 UP | 2.65E-05 |
| | 1 (class I) (RAB11FIP1), transcript variant 1, | | |
| | mRNA | | |
| B1759570 | 603046987F1 NIH_MGC_116 Homo sapiens | 11.49 UP | 3.71 E-05 |
| | cDNA clone IMAGE:5187285 5, mRNA | | |
| | sequence | | |
| NM_013261 | Homo sapiens peroxisome proliferative | 11.3 UP | 2.83E-04 |
| | activated receptor, gamma, coactivator 1, alpha | | |
| | (PPARGC1A), mRNA | | |
| A1830524 | wh52c02.x1 NCI_CGAP_Kid11 Homo sapiens | 11.29 UP | 8.65E-04 |
| | cDNA clone IMAGE:2384354 3, mRNA | | |
| | sequence | | |
| NM_139161 | Homo sapiens crumbs homolog 3 (Drosophila) | 11.15 UP | 1.22E-04 |
| | (CRB3), transcript variant 2, mRNA | | |
| AK055362 | Homo sapiens cDNA FLJ30800 fis, clone | 11.13 UP | 1.02E-05 |
| | FEBRA2001197 | | |
| BF055156 | 7j75f03.x1 | 11.11 UP | 4.94E-04 |
| | Soares_NSF_F8_9W_OT_PA_P_S1 Homo | | |
| | sapiens cDNA clone IMAGE:3392285 3, mRNA | | |
| | sequence | | |
| BC016962 | Homo sapiens, clone IMAGE:4182947, mRNA | 11.05 UP | 1.06E-04 |
| AY358775 | Homo sapiens clone DNA170212 WGAR9166 | 11.05 UP | 2.32E-04 |
| | (UNQ9166) mRNA, complete cds | | |
| NM_175056 | Homo sapiens hypothetical protein LOC131368 | 10.98 UP | 1.33E-03 |
| | (LOC131368), mRNA | | |
| AI311296 | ta48d10.x2 NCI_CGAP_Lu25 Homo sapiens | 10.93 UP | 2.19E-04 |
| | cDNA clone IMAGE:2047315 3, mRNA | | |
| | sequence | | |
| AW296834 | UI-H-B12-ahz-a-10-0-UI.s1 NCI_CGAP_Sub4 | 10.91 UP | 1.71E-04 |
| | Homo sapiens cDNA clone IMAGE:2728243 3, | | |
| | mRNA sequence | | |
| NM_014464 | Homo sapiens tubulointerstitial nephritis | 10.89 UP | 3.87E-04 |
| | antigen (TINAG), mRNA | | |
| AK024270 | Homo sapiens cDNA FLJ14208 fis, clone | 10.88 UP | 3.05E-04 |
| | NT2RP3003264 | | |
| H00617 | yj25f02.s1 Soares placenta Nb2HP Homo | 10.87 UP | 2.09E-04 |
| | sapiens cDNA clone IMAGE:149787 3, mRNA | | |
| | sequence | | |
| AK126467 | Homo sapiens cDNA FLJ44503 fis, clone | 10.87 UP | 5.06E-05 |
| | UTERU3001158 | | |
| NM_181847 | Homo sapiens amphoterin induced gene 2 | 10.86 UP | 8.91 E-04 |
| | (AMIGO2), mRNA | | |
| NM_014751 | Homo sapiens metastasis suppressor 1 | 10.86 UP | 2.68E-05 |
| | (MTSS1), mRNA | | |
| N70752 | za72d02.s1 Soares_fetal_lung_NbHL19W | 10.84 UP | 1.34E-04 |
| | Homo sapiens cDNA clone IMAGE:298083 3, | | |
| | mRNA sequence | | |
| NM_005302 | Homo sapiens G protein-coupled receptor 37 | 10.84 UP | 2.63E-03 |
| | (endothelin receptor type B-like) (GPR37), | | |
| | mRNA | | |
| NM_003264 | Homo sapiens toll-like receptor 2 (TLR2), | 10.83 UP | 1.01 E-04 |
| | mRNA | | |
| NM_017594 | Homo sapiens DIRAS family, GTP-binding | 10.81 UP | 7.23E-04 |
| | RAS-like 2 (DIRAS2), mRNA | | |
| AI688800 | wd41b03.x1 Soares_NFL_T_GBC_S1 Homo | 10.8 UP | 8.56E-04 |
| | sapiens cDNA clone IMAGE:2330669 3, mRNA | | |
| | sequence | | |
| H08012 | y191b08.r1 Soares infant brain 1NIB Homo | 10.8 UP | 3.26E-04 |
| | sapiens cDNA clone IMAGE:45474 5, mRNA | | |
| | sequence | | |
| AW014126 | UI-H-BI0-aaj-a-05-0-UI.s1 NCI_CGAP_Sub1 | 10.79 UP | 1.88E-05 |
| | Homo sapiens cDNA clone IMAGE:2709393 3, | | |
| | mRNA sequence | | |
| NM_014422 | Homo sapiens phosphatidylinositol (4,5) | 10.73 UP | 4.47E-05 |
| | bisphosphate 5-phosphatase, A (PIB5PA), | | |
| | transcript variant 1, mRNA | | |
| H62713 | yr28c08.r1 Soares fetal liver spleen NFLS | 10.73 UP | 1.01 E-03 |
| | Homo sapiens cDNA clone IMAGE:206606 5 | | |
| | similar to gb:X01683 ALPHA-1-ANTITRYPSIN | | |
| | PRECURSOR (HUMAN);, mRNA sequence | | |
| AI870547 | wl47a04.x1 NCI_CGAP_Ut1 Homo sapiens | 10.65 UP | 4.82E-03 |
| | cDNA clone IMAGE:2428014 3, mRNA | | |
| | sequence | | |
| NM_025202 | Homo sapiens EF hand domain containing 1 | 10.64 UP | 5.59E-05 |
| | (EFHD1), mRNA | | |
| N78460 | yz76h06.r1 | 10.63 UP | 3.90E-04 |
| | Soares_multiple_sclerosis_2NbHMSP Homo | | |
| | sapiens cDNA clone IMAGE:289019 5, mRNA | | |
| | sequence | | |
| NM_020661 | Homo sapiens activation-induced cytidine | 10.63 UP | 7.27E-05 |
| | deaminase (AICDA), mRNA | | |
| NM_173549 | Homo sapiens hypothetical protein FLJ39553 | 10.57 UP | 3.11E-04 |
| | (FLJ39553), mRNA | | |
| AI767472 | wh27a07.x1 NCI_CGAP_Kid11 Homo sapiens | 10.56 UP | 8.39E-06 |
| | cDNA clone IMAGE:2381940 3 similar to | | |
| | contains L1.t3 L1 repetitive element ;, mRNA | | |
| | sequence | | |
| NM_145804 | Homo sapiens ankyrin repeat and BTB (POZ) | 10.55 UP | 1.41E-06 |
| | domain containing 2 (ABTB2), mRNA | | |
| NM_013230 | Homo sapiens CD24 antigen (small cell lung | 10.54 UP | 1.12E-04 |
| | carcinoma cluster 4 antigen) (CD24), mRNA | | |
| NM_000758 | Homo sapiens colony stimulating factor 2 | 10.51 UP | 7.66E-04 |
| | (granulocyte-macrophage) (CSF2), mRNA | | |
| AB032945 | Homo sapiens mRNA for KIAA1119 protein, | 10.48 UP | 1.32E-03 |
| | partial cds | | |
| NM_024572 | Homo sapiens UDP-N-acetyl-alpha-D- | 10.46 UP | 2.62E-04 |
| | galactosamine:polypeptide N- | | |
| | acetylgalactosaminyltransferase 14 (GaINAc- | | |
| | T14) (GALNT14), mRNA | | |
| NM_181332 | Homo sapiens neuroligin 4, X-linked | 10.46 UP | 1.95E-04 |
| | (NLGN4X), transcript variant 2, mRNA | | |
| NM_022128 | Homo sapiens ribokinase (RBKS), mRNA | 10.45 UP | 2.73E-05 |
| AB046810 | Homo sapiens mRNA for KIAA1590 protein, | 10.39 UP | 2.36E-05 |
| | partial cds | | |
| NM_022168 | Homo sapiens interferon induced with helicase | 10.38 UP | 2.83E-05 |
| | C domain 1 (IFIH1), mRNA | | |
| NM_153715 | Homo sapiens homeo box A10 (HOXA10), | 10.37 UP | 5.50E-05 |
| | transcript variant 2, mRNA | | |
| BF090392 | QV3-NT0023-120900-324-b04 NT0023 Homo | 10.35 UP | 1.25E-03 |
| | sapiens cDNA, mRNA sequence | | |
| NM_000880 | Homo sapiens interleukin 7 (IL7), mRNA | 10.34 UP | 3.60E-04 |
| NM_018650 | Homo sapiens MAP/microtubule affinity- | 10.29 UP | 1.57E-04 |
| | regulating kinase 1 (MARK1), mRNA | | |
| NM_017899 | Homo sapiens hypothetical protein FLJ20607 | 10.27 UP | 6.79E-04 |
| | (TSC), mRNA | | |
| NM_032367 | Homo sapiens zinc finger, BED domain | 10.27 UP | 4.12E-05 |
| | containing 3 (ZBED3), mRNA | | |
| NM_012206 | Homo sapiens hepatitis A virus cellular receptor | 10.23 UP | 8.15E-04 |
| | 1 (HAVCR1),mRNA | | |
| AK092245 | Homo sapiens cDNA FLJ34926 fis, clone | 10.21 UP | 1.92E-03 |
| | NT2RP7003319, highly similar to Mus | | |
| | musculus neuralin mRNA | | |
| NM_031469 | Homo sapiens SH3 domain binding glutamic | 10.18 UP | 1.63E-05 |
| | acid-rich protein like 2 (SH3BGRL2), mRNA | | |
| CA311343 | UI-CF-FN0-aff-b-19-0-UI.s1 UI-CF-FNO Homo | 10.15 UP | 1.89E-05 |
| | sapiens cDNA clone UI-CF-FNO-aff-b-19-0-UI | | |
| | 3, mRNA sequence | | |
| NM_030923 | Homo sapiens hypothetical protein | 10.15 UP | 2.23E-05 |
| | DKFZp566N034 (DKFZP566N034), mRNA | | |
| AK024261 | Homo sapiens cDNA FLJ14199 fis, clone | 10.14 UP | 2.08E-04 |
| | NT2RP3002713 | | |
| NM_174900 | Homo sapiens zinc finger protein 42 (ZFP42), | 10.1 UP | 5.14E-05 |
| | mRNA | | |
| NM_198495 | Homo sapiens CTAGE family, member 4 | 10.07 UP | 4.65E-05 |
| | (CTAGE4), mRNA | | |
| BX105791 | BX105791 Soares_testis_NHT Homo sapiens | 10.06 UP | 2.15E-03 |
| | cDNA clone IMAGp998I101824 ; | | |
| | IMAGE:742737, mRNA sequence | | |
| AI672441 | wa03c03.x1 NCI_CGAP_Kid11 Homo sapiens | 10.05 UP | 2.40E-04 |
| | cDNA clone IMAGE:2296996 3, mRNA | | |
| | sequence | | |
| AK002097 | Homo sapiens cDNA FLJ11235 fis, clone | 10.01 UP | 2.75E-05 |
| | PLACE1008488 | | |
| BX647256 | Homo sapiens mRNA; cDNA DKFZp686K0753 | 9.97 UP | 1.81 E-03 |
| | (from clone DKFZp686K0753) | | |
| AK092456 | Homo sapiens cDNA FLJ35137 fis, clone | 9.96 UP | 5.02E-05 |
| | PLACE6009419 | | |
| NM_001853 | Homo sapiens collagen, type IX, alpha 3 | 9.96 UP | 2.87E-05 |
| | (COL9A3), mRNA | | |
| NM_004946 | Homo sapiens dedicator of cytokinesis 2 | 9.94 UP | 3.94E-05 |
| | (DOCK2), mRNA | | |
| NM_005139 | Homo sapiens annexin A3 (ANXA3), mRNA | 9.92 UP | 5.14E-05 |
| NM_002260 | Homo sapiens killer cell lectin-like receptor | 9.92 UP | 5.95E-04 |
| | subfamily C, member 2 (KLRC2), mRNA | | |
| BX649112 | Homo sapiens mRNA; cDNA | 9.89 UP | 6.13E-05 |
| | DKFZp686E02109 (from clone | | |
| | DKFZp686E02109) | | |
| AK127437 | Homo sapiens cDNA FLJ45529 fis, clone | 9.85 UP | 2.09E-05 |
| | BRTHA2027546 | | |
| NM_080659 | Homo sapiens similar to RIKEN cDNA | 9.81 UP | 1.33E-03 |
| | 2310030G06 gene (MGC14839), mRNA | | |
| NM_014867 | Homo sapiens KIAA0711 gene product | 9.81 UP | 4.12E-05 |
| | (KIAA0711), mRNA | | |
| BG149255 | nad25d01.x1 NCI_CGAP_Lu24 Homo sapiens | 9.7 UP | 1.54E-04 |
| | cDNA clone IMAGE:3366553 3, mRNA | | |
| | sequence | | |
| AK022598 | Homo sapiens cDNA FLJ12536 fis, clone | 9.7 UP | 1.38E-02 |
| | NT2RM4000265 | | |
| A1792194 | ov03c02.y5 NCI_CGAP_Kid3 Homo sapiens | 9.65 UP | 6.10E-04 |
| | cDNA clone IMAGE:1636226 5, mRNA | | |
| | sequence | | |
| NM_005502 | Homo sapiens ATP-binding cassette, sub- | 9:65 UP | 4.03E-04 |
| | family A (ABC1), member 1 (ABCA1), mRNA | | |
| BE070450 | QV4-BT0407-020300-122-d08 BT0407 Homo | 9.62 UP | 5.52E-05 |
| | sapiens cDNA, mRNA sequence | | |
| NM_006255 | Homo sapiens protein kinase C, eta (PRKCH), | 9.56 UP | 2.09E-05 |
| | mRNA | | |
| NM_005114 | Homo sapiens heparan sulfate (glucosamine) | 9.55 UP | 1.44E-04 |
| | 3-O-sulfotransferase 1 (HS3ST1), mRNA | | |
| AK123427 | Homo sapiens cDNA FLJ41433 fis, clone | 9.46 UP | 1.99E-04 |
| | BRHIP2007307 | | |
| NM_013430 | Homo sapiens gamma-glutamyltransferase 1 | 9.4 UP | 1.15E-04 |
| | (GGT1), transcript variant 3, mRNA | | |
| AK094353 | Homo sapiens cDNA FLJ37034 fis, clone | 9.4 UP | 3.08E-05 |
| | BRACE2011478 | | |
| NM_016584 | Homo sapiens interleukin 23, alpha subunit p19 | 9.38 UP | 3.77E-05 |
| | (IL23A), mRNA | | |
| NM_002089 | Homo sapiens chemokine (C-X-C motif) ligand | 9.38 UP | 4.55E-04 |
| | 2 (CXCL2), mRNA | | |
| NM_032023 | Homo sapiens Ras association (RaIGDS/AF-6) | 9.38 UP | 5.39E-05 |
| | domain family 4 (RASSF4), transcript variant 1, | | |
| | mRNA | | |
| D62831 | HUM330B12B Clontech human aorta polyA+ | 9.32 UP | 9.48E-05 |
| | mRNA (#6572) Homo sapiens cDNA clone | | |
| | GEN-330B12 5, mRNA sequence | | |
| AI963999 | wt87g07.x1 NCI_CGAP_GC6 Homo sapiens | 9.31 UP | 8.57E-04 |
| | cDNA clone IMAGE:2514492 3, mRNA | | |
| | sequence | | |
| NM_005978 | Homo sapiens S100 calcium binding protein A2 | 9.31 UP | 1.64E-05 |
| | (S100A2), mRNA | | |
| NM_006622 | Homo sapiens polo-like kinase 2 (Drosophila) | 9.3 UP | 2.93E-05 |
| | (PLK2), mRNA | | |
| NM_033495 | Homo sapiens kelch-like 13 (Drosophila) | 9.25 UP | 2.38E-05 |
| | (KLHL13), mRNA | | |
| L07615 | Human neuropeptide Y receptor Y1 (NPYY1) | 9.23 UP | 1.09E-03 |
| | mRNA, exon 2-3 and complete cds | | |
| NM_018076 | Homo sapiens armadillo repeat containing 4 | 9.16 UP | 5.96E-04 |
| | (ARMC4), mRNA | | |
| CA778369 | ip17f03.y1 HR85 islet Homo sapiens cDNA | 9.12 UP | 5.40E-05 |
| | clone IMAGE:6217493 5, mRNA sequence | | |
| NM_024554 | Homo sapiens piggyBac transposable element | 9.12 UP | 2.63E-04 |
| | derived 5 (PGBD5), mRNA | | |
| NM_182487 | Homo sapiens olfactomedin-like 2A | 9.04 UP | 1.64E-04 |
| | (OLFML2A), mRNA | | |
| T78754 | yd01f08.r1 Soares infant brain 1NIB Homo | 9.01 UP | 1.09E-03 |
| | sapiens cDNA clone IMAGE:24180 5, mRNA | | |
| | sequence | | |
| NM_004024 | Homo sapiens activating transcription factor 3 | 9 UP | 1.50E-03 |
| | (ATF3), mRNA | | |
| NM_030583 | Homo sapiens matrilin 2 (MATN2), transcript | 8.98 UP | 2.15E-04 |
| | variant 2, mRNA | | |
| NM_145032 | Homo sapiens F-box and leucine-rich repeat | 8.97 UP | 1.78E-05 |
| | protein 13 (FBXL13), mRNA | | |
| NM_020130 | Homo sapiens chromosome 8 open reading | 8.93 UP | 4.79E-04 |
| | frame 4 (C8orf4), mRNA | | |
| NM_005737 | Homo sapiens ADP-ribosylation factor-like 7 | 8.87 UP | 1.79E-04 |
| | (ARL7), mRNA | | |
| BG675167 | 602621444F1 NCI_CGAP_Skn3 Homo sapiens | 8.86 UP | 7.41 E-04 |
| | cDNA clone IMAGE:4755106 5, mRNA | | |
| | sequence | | |
| NM_194463 | Homo sapiens ring finger protein 128 | 8.85 UP | 8.97E-05 |
| | (RNF128), transcript variant 1, mRNA | | |
| NM_018349 | Homo sapiens multiple C2-domains with two | 8.81 UP | 8.37E-05 |
| | transmembrane regions 2 (MCTP2), mRNA | | |
| AK026740 | Homo sapiens cDNA: FLJ23087 fis, clone | 8.8 UP | 3.17E-04 |
| | LNG06994, highly similar to AF161368 Homo | | |
| | sapiens HSPC105 mRNA | | |
| NM_000693 | Homo sapiens aldehyde dehydrogenase 1 | 8.78 UP | 4.72E-04 |
| | family, member A3 (ALDH1A3), mRNA | | |
| NM_005459 | Homo sapiens guanylate cyclase activator 1C | 8.77 UP | 1.57E-04 |
| | (GUCA1C), mRNA | | |
| BF509074 | UI-H-BI4-aou-b-08-0-UI.s1 NCI_CGAP_Sub8 | 8.74 UP | 2.11E-05 |
| | Homo sapiens cDNA clone IMAGE:3086150 3, | | |
| | m RNA sequence | | |
| AL117425 | Homo sapiens mRNA; cDNA DKFZp566L203 | 8.69 UP | 2.83E-04 |
| | (from clone DKFZp566L203) | | |
| NM_006257 | Homo sapiens protein kinase C, theta | 8.64 UP | 1.78E-05 |
| | (PRKCQ), mRNA | | |
| NM_005567 | Homo sapiens lectin, galactoside-binding, | 8.64 UP | 1.26E-05 |
| | soluble, 3 binding protein (LGALS3BP), mRNA | | |
| NM_173508 | Homo sapiens solute carrier family 35, member | 8.62 UP | 1.04E-04 |
| | F3 (SLC35F3), mRNA | | |
| AK025281 | Homo sapiens cDNA: FLJ21628 fis, clone | 8.48 UP | 7.96E-05 |
| | COL08076 | | |
| NM_145252 | Homo sapiens similar to common salivary | 8.48 UP | 1.94E-04 |
| | protein 1 (LOC124220), mRNA | | |
| AI764969 | wh57b02.x1 NCI_CGAP_Kid11 Homo sapiens | 8.48 UP | 6.82E-05 |
| | cDNA clone IMAGE:2384811 3, mRNA | | |
| | sequence | | |
| NM_002313 | Homo sapiens actin binding LIM protein 1 | 8.48 UP | 3.03E-05 |
| | (ABL1M1), transcript variant 1, mRNA | | |
| NM_017641 | Homo sapiens kinesin family member 21A | 8.42 UP | 2.43E-05 |
| | (KIF21A), mRNA | | |
| AA564703 | nj22h06.s1 NCI_CGAP_AA1 Homo sapiens | 8.4 UP | 6.11 E-05 |
| | cDNA clone IMAGE:993275 3, mRNA | | |
| | sequence | | |
| NM_001323 | Homo sapiens cystatin E/M (CST6), mRNA | 8.37 UP | 1.02E-04 |
| NM_014333 | Homo sapiens immunoglobulin superfamily, | 8.35 UP | 1.82E-03 |
| | member 4 (IGSF4), mRNA | | |
| NM_022454 | Homo sapiens SRY (sex determining region Y)- | 8.33 UP | 6.04E-05 |
| | box17(SOX17), mRNA | | |
| NM_000186 | Homo sapiens complement factor H (CFH), | 8.32 UP | 1.06E-03 |
| | mRNA | | |
| BF512544 | UI-H-BW1-amf-c-08-0-UI.s1 NCI_CGAP_Sub7 | 8.31 UP | 1.09E-04 |
| | Homo sapiens cDNA clone IMAGE:3069687 3, | | |
| | mRNA sequence | | |
| AF036977 | Homo sapiens clone HCG IV.9 unknown mRNA | 8.27 UP | 1.12E-03 |
| AK095500 | Homo sapiens cDNA FLJ38181 fis, clone | 8.19 UP | 6.56E-05 |
| | FCBBF1000125 | | |
| NM_001955 | Homo sapiens endothelin 1 (EDN1), mRNA | 8.18 UP | 4.43E-04 |
| NM_002053 | Homo sapiens guanylate binding protein 1, | 8.17 UP | 8.39E-06 |
| | interferon-inducible, 67kDa (GBP1), mRNA | | |
| NM_031476 | Homo sapiens hypothetical protein | 8.12 UP | 8.06E-04 |
| | DKFZp434B044 (DKFZP434B044), mRNA | | |
| AA844712 | ai70e12.s1 Soares_testis_NHT Homo sapiens | 8.11 UP | 2.36E-04 |
| | cDNA clone IMAGE:1376206 3, mRNA | | |
| | sequence | | |
| BM976385 | UI-CF-EN1-acz-f-03-0-UI.s1 UI-CF-EN1 Homo | 8.11 UP | 7.27E-04 |
| | sapiens cDNA clone UI-CF-EN1-acz-f-03-0-UI | | |
| | 3, mRNA sequence | | |
| BX647876 | Homo sapiens mRNA; cDNA DKFZp313A1525 | 8.11 UP | 6.87E-04 |
| | (from clone DKFZp313A1525) | | |
| NM_001845 | Homo sapiens collagen, type IV, alpha 1 | 8.06 UP | 2.72E-05 |
| | (COL4A1), mRNA | | |
| NM_014900 | Homo sapiens COBL-like 1 (COBLL1), mRNA | 8.04 UP | 9.67E-05 |
| NM_203306 | Homo sapiens hypothetical protein MGC39606 | 7.98 UP | 1.95E-03 |
| | (MGC39606), mRNA | | |
| BI963896 | ie66c06.x1 Melton Normalized Human Islet 4 | 7.97 UP | 2.73E-05 |
| | N4-HIS 1 Homo sapiens cDNA clone | | |
| | IMAGE:5671691 3, mRNA sequence | | |
| NM_005025 | Homo sapiens serine (or cysteine) proteinase | 7.97 UP | 3.03E-05 |
| | inhibitor, clade I (neuroserpin), member 1 | | |
| | (SERPINI1), mRNA | | |
| AK024238 | Homo sapiens cDNA FLJ14176 fis, clone | 7.96 UP | 4.75E-05 |
| | NT2RP2003101 | | |
| NM_005951 | Homo sapiens metallothionein 1 H (MT1 H), | 7.95 UP | 3.19E-03 |
| | mRNA | | |
| CF887677 | UI-CF-FNO-aev-o-22-18-UI.r18 UI-CF-FNO | 7.93 UP | 4.30E-05 |
| | Homo sapiens cDNA clone UI-CF-FNO-aev-o- | | |
| | 22-18-UI 5, mRNA sequence | | |
| NM_024423 | Homo sapiens desmocollin 3 (DSC3), transcript | 7.91 UP | 1.23E-04 |
| | variant Dsc3b, mRNA | | |
| AI355761 | qt94a11.x1 NCI_CGAP_Co14 Homo sapiens | 7.91 UP | 6.82E-05 |
| | cDNA clone IMAGE:1962908 3 similar to | | |
| | gb:X74929 KERATIN, TYPE II | | |
| | CYTOSKELETAL 8 (HUMAN);, mRNA | | |
| | sequence | | |
| BF445031 | nad20f02.x1 NCI_CGAP_Lu24 Homo sapiens | 7.91 UP | 1.78E-05 |
| | cDNA clone IMAGE:3366266 3, mRNA | | |
| | sequence | | |
| CA444471 | UI-H-DPO-avv-a-16-0-UI.s1 NCI_CGAP_Fs1 | 7.89 UP | 7.69E-05 |
| | Homo sapiens cDNA clone UI-H-DPO-avv-a-16- | | |
| | 0-UI 3, mRNA sequence | | |
| NM_014942 | Homo sapiens ankyrin repeat domain 6 | 7.89 UP | 2.49E-05 |
| | (ANKRD6), mRNA | | |
| AK021801 | Homo sapiens cDNA FLJ11739 fis, clone | 7.86 UP | 1.15E-04 |
| | HEMBA1005497 | | |
| NM_001766 | Homo sapiens CD1D antigen, d polypeptide | 7.85 UP | 5.10E-04 |
| | (CD1D), mRNA | | |
| NM_005360 | Homo sapiens v-maf musculoaponeurotic | 7.81 UP | 7.59E-05 |
| | fibrosarcoma oncogene homolog (avian) | | |
| | (MAF), mRNA | | |
| NM_018700 | Homo sapiens tripartite motif-containing 36 | 7.8 UP | 2.55E-05 |
| | (TRIM36), mRNA | | |
| BX640643 | Homo sapiens mRNA; cDNA | 7.79 UP | 2.59E-05 |
| | DKFZp686O24114 (from clone | | |
| | DKFZp686O24114) | | |
| NM_005331 | Homo sapiens hemoglobin, theta 1 (HBQ1), | 7.77 UP | 3.73E-04 |
| | mRNA | | |
| NM_194298 | Homo sapiens solute carrier family 16 | 7.77 UP | 2.66E-04 |
| | (monocarboxylic acid transporters), member 9 | | |
| | (SLC16A9), mRNA | | |
| NM_012413 | Homo sapiens glutaminyl-peptide | 7.76 UP | 4.60E-04 |
| | cyclotransferase (glutaminyl cyclase) (QPCT), | | |
| | mRNA | | |
| NM_005860 | Homo sapiens follistatin-like 3 (secreted | 7.76 UP | 2.64E-05 |
| | glycoprotein) (FSTL3), mRNA | | |
| AB033029 | Homo sapiens mRNA for KIAA1203 protein, | 7.7 UP | 8.39E-06 |
| | partial cds | | |
| NM_012216 | Homo sapiens midline 2 (MID2), transcript | 7.62 UP | 6.12E-05 |
| | variant 1, mRNA | | |
| NM_018986 | Homo sapiens SH3 domain and | 7.61 UP | 8.69E-04 |
| | tetratricopeptide repeats 1 (SH3TC1), mRNA | | |
| BM665101 | UI-E-CQ1-aew-I-09-0-UI.s1 UI-E-CQ1 Homo | 7.58 UP | 4.09E-05 |
| | sapiens cDNA clone UI-E-CQ1-aew-I-09-0-UI 3, | | |
| | mRNA sequence | | |
| AL706653 | DKFZp686E1543_r1 686 (synonym: hlcc3) | 7.57 UP | 2.36E-05 |
| | Homo sapiens cDNA clone DKFZp686E1543 5, | | |
| | mRNA sequence | | |
| BF920409 | QV2-NT0144-071100-463-b06 NT0144 Homo | 7.55 UP | 6.70E-04 |
| | sapiens cDNA, mRNA sequence | | |
| NM_173462 | Homo sapiens papilin, proteoglycan-like | 7.55 UP | 5.64E-05 |
| | sulfated glycoprotein (PAPLN), mRNA | | |
| NM_031419 | Homo sapiens nuclear factor of kappa light | 7.55 UP | 1.49E-04 |
| | polypeptide gene enhancer in B-cells inhibitor, | | |
| | zeta (NFKBIZ), transcript variant 1, mRNA | | |
| NM_016423 | Homo sapiens zinc finger protein 219 | 7.53 UP | 1.71E-05 |
| | (ZNF219), mRNA | | |
| NM_020859 | Homo sapiens Shroom-related protein (ShrmL), | 7.52 UP | 1.26E-05 |
| | mRNA | | |
| NM_004932 | Homo sapiens cadherin 6, type 2, K-cadherin | 7.52 UP | 1.64E-05 |
| | (fetal kidney) (CDH6), mRNA | | |
| NM_182797 | Homo sapiens phospholipase C, beta 4 | 7.52 UP | 5.49E-05 |
| | (PLCB4), transcript variant 2, mRNA | | |
| AL512697 | Homo sapiens mRNA; cDNA DKFZp547F134 | 7.5 UP | 6.17E-05 |
| | (from clone DKFZp547F134) | | |
| BC035116 | Homo sapiens cDNA clone IMAGE:5263177, | 7.47 UP | 2.32E-05 |
| | partial cds | | |
| AL353944 | Homo sapiens mRNA; cDNA DKFZp761J1112 | 7.45 UP | 1.72E-05 |
| | (from clone DKFZp761J1112) | | |
| NM_152780 | Homo sapiens hypothetical protein FLJ14503 | 7.45 UP | 1.19E-03 |
| | (FLJ14503), mRNA | | |
| AA888443 | nw74f10.s1 NCI_CGAP_Pr12 Homo sapiens | 7.43 UP | 5.18E-04 |
| | cDNA clone IMAGE:1252363, mRNA sequence | | |
| AW450938 | UI-H-BI3-all-g-05-0-UI.s1 NCI_CGAP_Sub5 | 7.4 UP | 4.98E-05 |
| | Homo sapiens cDNA clone IMAGE:2737329 3, | | |
| | mRNA sequence | | |
| NM_053039 | Homo sapiens UDP glycosyltransferase 2 | 7.38 UP | 1.12E-03 |
| | family, polypeptide B28 (UGT2B28), mRNA | | |
| NM_016946 | Homo sapiens F11 receptor (F11 R), transcript | 7.36 UP | 4.65E-05 |
| | variant 1, mRNA | | |
| AW969742 | EST381820 MAGE resequences, MAGK Homo | 7.36 UP | 1.15E-04 |
| | sapiens cDNA, mRNA sequence | | |
| BX117866 | BX117866 NCI_CGAP_GCB1 Homo sapiens | 7.34 UP | 2.20E-04 |
| | cDNA clone IMAGp998N233105 ; | | |
| | IMAGE:1234774, mRNA sequence | | |
| NM_005739 | Homo sapiens RAS guanyl releasing protein 1 | 7.31 UP | 2.20E-04 |
| | (calcium and DAG-regulated) (RASGRP1), | | |
| | mRNA | | |
| CB047287 | NISC_gg01h01.y1 NCI_CGAP_Kid11 Homo | 7.25 UP | 3.52E-05 |
| | sapiens cDNA clone IMAGE:3253464 5, mRNA | | |
| | sequence | | |
| NM_013951 | Homo sapiens paired box gene 8 (PAX8), | 7.25 UP | 5.00E-05 |
| | transcript variant PAX8B, mRNA | | |
| AK125695 | Homo sapiens cDNA FLJ43707 fis, clone | 7.24 UP | 1.62E-03 |
| | TESOP2001865 | | |
| NM_020796 | Homo sapiens sema domain, transmembrane | 7.21 UP | 1.01 E-04 |
| | domain (TM), and cytoplasmic domain, | | |
| | (semaphorin) 6A (SEMA6A), mRNA | | |
| BM929354 | UI-E-EJ1-aje-o-19-0-UI.r1 UI-E-EJ1 Homo | 7.19 UP | 5.49E-05 |
| | sapiens cDNA clone UI-E-EJ1-aje-o-19-0-UI 5, | | |
| | mRNA sequence | | |
| BX103949 | BX103949 NCI_CGAP_Co3 Homo sapiens | 7.16 UP | 6.37E-05 |
| | cDNA clone IMAGp998C112296 ; | | |
| | IMAGE:923842, mRNA sequence | | |
| NM_003475 | Homo sapiens chromosome 11 open reading | 7.14 UP | 1.56E-04 |
| | frame 13 (C11orf13), mRNA | | |
| BE735115 | 601566084F1 NIH_MGC_21 Homo sapiens | 7.12 UP | 1.41 E-03 |
| | cDNA clone IMAGE:3840837 5, mRNA | | |
| | sequence | | |
| NM_207517 | Homo sapiens ADAMTS-like 3 (ADAMTSL3), | 7.11 UP | 1.26E-05 |
| | mRNA | | |
| BU688263 | UI-CF-EC1-aea-g-11-0-UI.s1 UI-CF-EC1 Homo | 7.06 UP | 2.97E-05 |
| | sapiens cDNA clone UI-CF-EC1-aea-g-11-0-UI | | |
| | 3, mRNA sequence | | |
| AK022971 | Homo sapiens cDNA FLJ12909 fis, clone | 7.06 UP | 7.80E-05 |
| | NT2RP2004400 | | |
| NM_001740 | Homo sapiens calbindin 2, 29kDa (calretinin) | 7.05 UP | 1.81 E-04 |
| | (CALB2), transcript variant CALB2, mRNA | | |
| NM_022073 | Homo sapiens egl nine homolog 3 (C. elegans) | 7.04 UP | 5.64E-04 |
| | (EGLN3), mRNA | | |
| BX117230 | BX117230 NCI_CGAP_Kid11 Homo sapiens | 7.03 UP | 1.56E-03 |
| | cDNA clone IMAGp998I235695; | | |
| | IMAGE:2298718, mRNA sequence | | |
| NM_002230 | Homo sapiens junction plakoglobin (JUP), | 7.03 UP | 2.78E-05 |
| | transcript variant 1, mRNA | | |
| AK023793 | Homo sapiens cDNA FLJ13731 fis, clone | 7.02 UP | 4.04E-04 |
| | PLACE3000142 | | |
| AL117598 | Homo sapiens mRNA; cDNA DKFZp564H1663 | 7 UP | 1.59E-04 |
| | (from clone DKFZp564H1663) | | |
| NM_001958 | Homo sapiens eukaryotic translation elongation | 6.99 UP | 5.20E-04 |
| | factor 1 alpha 2 (EEF1A2), mRNA | | |
| NM_007029 | Homo sapiens stathmin-like 2 (STMN2), mRNA | 6.97 UP | 4.47E-04 |
| BE968596 | 601649770F1 NIH_MGC_74 Homo sapiens | 6.97 UP | 8.39E-04 |
| | cDNA clone IMAGE:3933472 5, mRNA | | |
| | sequence | | |
| AK126467 | Homo sapiens cDNA FLJ44503 fis, clone | 6.97 UP | 3.82E-05 |
| | UTERU3001158 | | |
| AA832510 | oe62d06.s1 NCI_CGAP_Lu5 Homo sapiens | 6.93 UP | 9.67E-04 |
| | cDNA clone IMAGE:1416203 3, mRNA | | |
| | sequence | | |
| CF137545 | UI-HF-BNO-ane-d-05-0-UI.r1 NIH_MGC_50 | 6.93 UP | 2.70E-03 |
| | Homo sapiens cDNA clone IMAGE:3092384 5, | | |
| | mRNA sequence | | |
| NM_004895 | Homo sapiens cold autoinflammatory syndrome | 6.9 UP | 1.46E-03 |
| | 1 (CIAS1), transcript variant 1, mRNA | | |
| NM_018424 | Homo sapiens erythrocyte membrane protein | 6.9 UP | 4.68E-05 |
| | band 4.1 like 4B (EPB41 L4B), mRNA | | |
| NM_004390 | Homo sapiens cathepsin H (CTSH), transcript | 6.89 UP | 2.72E-05 |
| | variant 1, mRNA | | |
| NM_024603 | Homo sapiens hypothetical protein FLJ11588 | 6.87 UP | 3.80E-05 |
| | (FLJ11588), mRNA | | |
| AI492941 | qz42h08.x1 NCI_CGAP_Kid11 Homo sapiens | 6.82 UP | 8.39E-04 |
| | cDNA clone IMAGE:2029599 3, mRNA | | |
| | sequence | | |
| NM_003985 | Homo sapiens tyrosine kinase, non-receptor, 1 | 6.8 UP | 4.55E-05 |
| | (TNK1), mRNA | | |
| AK095399 | Homo sapiens cDNA FLJ38080 fis, clone | 6.8 UP | 9.23E-04 |
| | CTONG2016185 | | |
| BQ020597 | UI-H-DPO-avd-a-13-0-UI.s1 NCI_CGAP_Fs1 | 6.8 UP | 4.53E-05 |
| | Homo sapiens cDNA clone IMAGE:5877780 3, | | |
| | mRNA sequence | | |
| NM_001432 | Homo sapiens epiregulin (EREG), mRNA | 6.77 UP | 1.25E-05 |
| NM_005485 | Homo sapiens poly (ADP-ribose) polymerase | 6.76 UP | 6.46E-05 |
| | family, member 3 (PARP3), transcript variant 2, | | |
| | mRNA | | |
| AB058769 | Homo sapiens mRNA for KIAA1866 protein, | 6.76 UP | 9.84E-05 |
| | partial cds | | |
| BU753362 | UI-1-BB1-air-h-09-0-UI.s1 NCI_CGAP_PI5 | 6.75 UP | 1.08E-04 |
| | Homo sapiens cDNA clone UI-1-BB1-air-h-09- | | |
| | 0-UI 3, mRNA sequence | | |
| AL834140 | Homo sapiens mRNA; cDNA DKFZp434A2029 | 6.73 UP | 4.49E-05 |
| | (from clone DKFZp434A2029) | | |
| BX092501 | BX092501 NCI_CGAP_Lu5 Homo sapiens | 6.71 UP | 2.56E-04 |
| | cDNA clone IMAGp998K143946; | | |
| | IMAGE:1557637, mRNA sequence | | |
| BX640973 | Homo sapiens mRNA; cDNA | 6.71 UP | 2.36E-04 |
| | DKFZp686B15184 (from clone | | |
| | DKFZp686B15184) | | |
| NM_015277 | Homo sapiens neural precursor cell expressed, | 6.7 UP | 1.60E-05 |
| | developmentally down-regulated 4-like | | |
| | (NEDD4L), mRNA | | |
| NM_021192 | Homo sapiens homeo box D11 (HOXD11), | 6.69 UP | 8.94E-04 |
| | mRNA | | |
| NM_032621 | Homo sapiens brain expressed X-linked 2 | 6.69 UP | 1.83E-04 |
| | (BEX2), mRNA | | |
| X02851 | Human mRNA for interleukin-1 precursor (pre | 6.68 UP | 3.32E-04 |
| | IL-1) | | |
| NM_014258 | Homo sapiens synaptonemal complex protein 2 | 6.68 UP | 4.96E-02 |
| | (SYCP2), mRNA | | |
| NM_000682 | Homo sapiens adrenergic, alpha-2B-, receptor | 6.67 UP | 3.64E-05 |
| | (ADRA2B), mRNA | | |
| BQ435580 | AGENCOURT_7836890 NIH_MGC_82 Homo | 6.64 UP | 1.64E-04 |
| | sapiens cDNA clone IMAGE:6102371 5, mRNA | | |
| | sequence | | |
| NM_024608 | Homo sapiens nei endonuclease VIII-like 1 (E. | 6.63 UP | 3.23E-05 |
| | coli) (NEIL1), mRNA | | |
| AK057113 | Homo sapiens cDNA FLJ32551 fis, clone | 6.63 UP | 6.06E-04 |
| | SPLEN1000087 | | |
| NM_130897 | Homo sapiens dynein, cytoplasmic, light | 6.62 UP | 2.40E-03 |
| | polypeptide 2B (DNCL2B), mRNA | | |
| N39597 | yy51e04.s1 | 6.62 UP | 8.33E-04 |
| | Soares_multiple_sclerosis_2NbHMSP Homo | | |
| | sapiens cDNA clone IMAGE:277086 3, mRNA | | |
| | sequence | | |
| BU616749 | UI-H-FH1-bfj-a-11-0-UI.s1 NCI_CGAP_FH1 | 6.59 UP | 1.93E-04 |
| | Homo sapiens cDNA clone UI-H-FH1-bfj-a-11- | | |
| | 0-UI 3, mRNA sequence | | |
| AW195474 | xn38g09.x1 NCI_CGAP_Kid11 Homo sapiens | 6.59 UP | 6.82E-05 |
| | cDNA clone IMAGE:2696032 3, mRNA | | |
| | sequence | | |
| AK127437 | Homo sapiens cDNA FLJ45529 fis, clone | 6.57 UP | 1.76E-04 |
| | BRTHA2027546 | | |
| NM_004717 | Homo sapiens diacylglycerol kinase, iota | 6.56 UP | 7.54E-05 |
| | (DGKI), mRNA | | |
| BU584197 | 2513030T6 LIVRTUT04 Homo sapiens cDNA | 6.53 UP | 8.82E-04 |
| | clone 2513030 3, mRNA sequence | | |
| BU634363 | UI-H-FL1-bgx-o-20-0-UI.s1 NCI_CGAP_FL1 | 6.53 UP | 1.24E-04 |
| | Homo sapiens cDNA clone UI-H-FL1-bgx-o-20- | | |
| | 0-UI 3, mRNA sequence | | |
| NM_178868 | Homo sapiens chemokine-like factor super | 6.52 UP | 1.59E-03 |
| | family 8 (CKLFSF8), mRNA | | |
| NM_002247 | Homo sapiens potassium large conductance | 6.49 UP | 4.49E-05 |
| | calcium-activated channel, subfamily M, alpha | | |
| | member 1 (KCNMA1), mRNA | | |
| AW188195 | xj93e12.x1 Soares_NFL_T_GBC_S1 Homo | 6.49 UP | 3.81E-05 |
| | sapiens cDNA clone IMAGE:2664814 3 similar | | |
| | to contains element THR repetitive element ;, | | |
| | mRNA sequence | | |
| NM_153634 | Homo sapiens copine VIII (CPNE8), mRNA | 6.48 UP | 1.72E-04 |
| NM_145205 | Homo sapiens HMG2 like (LOC127540), mRNA | 6.47 UP | 6.39E-05 |
| NM_178868 | Homo sapiens chemokine-like factor super | 6.47 UP | 1.15E-05 |
| | family 8 (CKLFSF8), mRNA | | |
| U63828 | Human tissue plasminogen activator mRNA, | 6.46 UP | 5.51 E-05 |
| | partial cds | | |
| NM_004529 | Homo sapiens myeloid/lymphoid or mixed- | 6.46 UP | 8.21 E-05 |
| | lineage leukemia (trithorax homolog, | | |
| | Drosophila); translocated to, 3 (MLLT3), mRNA | | |
| BX647655 | Homo sapiens mRNA; cDNA DKFZp451A211 | 6.46 UP | 6.94E-05 |
| | (from clone DKFZp451A211) | | |
| AW470401 | xz83g01.x1 NCI_CGAP_Lu24 Homo sapiens | 6.46 UP | 1.30E-04 |
| | cDNA clone IMAGE:2870832 3 similar to | | |
| | contains L1.t2 L1 repetitive element ;, mRNA | | |
| | sequence | | |
| BG570144 | 602591134F1 NIH_MGC_77 Homo sapiens | 6.45 UP | 1.08E-03 |
| | cDNA clone IMAGE:4717761 5, mRNA | | |
| | sequence | | |
| BX115325 | BX115325 NCI_CGAP_Kid11 Homo sapiens | 6.45 UP | 1.04E-04 |
| | cDNA clone IMAGp998K085276 ; | | |
| | IMAGE:2137855, mRNA sequence | | |
| NM_002214 | Homo sapiens integrin, beta 8 (ITGB8), mRNA | 6.44 UP | 6.77E-05 |
| CA314926 | UI-CF-FNO-afi-b-19-0-UI.s1 UI-CF-FNO Homo | 6.43 UP | 6.61 E-05 |
| | sapiens cDNA clone UI-CF-FN0-afi-b-19-0-UI 3, | | |
| | mRNA sequence | | |
| AK093870 | Homo sapiens cDNA FLJ36551 fis, clone | 6.43 UP | 1.28E-03 |
| | TRACH2008127 | | |
| NM_000860 | Homo sapiens hydroxyprostaglandin | 6.42 UP | 1.76E-05 |
| | dehydrogenase 15-(NAD) (HPGD), mRNA | | |
| AI831068 | wj62d12.x1 NCI_CGAP_Lu19 Homo sapiens | 6.41 UP | 1.37E-04 |
| | cDNA clone IMAGE:2407415 3, mRNA | | |
| | sequence | | |
| NM_000349 | Homo sapiens steroidogenic acute regulator | 6.41 UP | 1.26E-04 |
| | (STAR), nuclear gene encoding mitochondrial | | |
| | protein, transcript variant 1, mRNA | | |
| NM_014181 | Homo sapiens HSPC159 protein (HSPC159), | 6.4 UP | 1.67E-04 |
| | mRNA | | |
| NM_024534 | Homo sapiens hypothetical protein FLJ12684 | 6.38 UP | 6.57E-04 |
| | (FLJ12684), mRNA | | |
| NM_002843 | Homo sapiens protein tyrosine phosphatase, | 6.38 UP | 1.32E-03 |
| | receptor type, J (PTPRJ), mRNA | | |
| AI926616 | wo48e04.x1 NCI_CGAP_Gas4 Homo sapiens | 6.37 UP | 7.01 E-03 |
| | cDNA clone IMAGE:2458590 3 similar to | | |
| | contains MER27.b2 MER27 repetitive element | | |
| | ;, mRNA sequence | | |
| NM_002273 | Homo sapiens keratin 8 (KRT8), mRNA | 6.36 UP | 2.91E-03 |
| NM_207303 | Homo sapiens attractin-like 1 (ATRNL1), mRNA | 6.35 UP | 7.85E-04 |
| AK001007 | Homo sapiens cDNA FLJ10145 fis, clone | 6.35 UP | 1.82E-05 |
| | HEMBA1003322 | | |
| CB047092 | NISC_gf08f03.x1 NCI_CGAP_Kid12 Homo | 6.33 UP | 6.92E-05 |
| | sapiens cDNA clone IMAGE:3253013 3, mRNA | | |
| | sequence | | |
| BC060805 | Homo sapiens hypothetical protein FLJ12788, | 6.31 UP | 2.93E-05 |
| | mRNA (cDNA clone IMAGE:5266931), partial | | |
| | cds | | |
| AI359782 | qy41h10.x1 NCI_CGAP_Brn23 Homo sapiens | 6.31 UP | 3.23E-04 |
| | cDNA clone IMAGE:2014627 3, mRNA | | |
| | sequence | | |
| CA426602 | UI-H-FE1-bef-f-11-0-UI.s1 NCI_CGAP_FE1 | 6.29 UP | 2.14E-04 |
| | Homo sapiens cDNA clone UI-H-FE1-bef-f-11- | | |
| | 0-UI 3, mRNA sequence | | |
| AK091686 | Homo sapiens cDNA FLJ34367 fis, clone | 6.27 UP | 1.93E-03 |
| | FEBRA2016621 | | |
| AK091336 | Homo sapiens cDNA FLJ34017 fis, clone | 6.26 UP | 5.49E-05 |
| | FCBBF2002626 | | |
| NM_015068 | Homo sapiens paternally expressed 10 | 6.26 UP | 4.53E-05 |
| | (PEG10), mRNA | | |
| R79128 | yi86c12.r1 Soares placenta Nb2HP Homo | 6.23 UP | 2.04E-04 |
| | sapiens cDNA clone IMAGE:146134 5, mRNA | | |
| | sequence | | |
| H08785 | yl94f04.s1 Soares infant brain 1NIB Homo | 6.22 UP | 1.66E-03 |
| | sapiens cDNA clone IMAGE:45882 3, mRNA | | |
| | sequence | | |
| AI953708 | wq47d09.x1 NCI_CGAP_GC6 Homo sapiens | 6.21 UP | 4.58E-03 |
| | cDNA clone IMAGE:2474417 3, mRNA | | |
| | sequence | | |
| NM_015236 | Homo sapiens latrophilin 3 (LPHN3), mRNA | 6.2 UP | 5.35E-04 |
| AL833609 | Homo sapiens mRNA; cDNA DKFZp68601267 | 6.19 UP | 7.53E-04 |
| | (from clone DKFZp686O1267) | | |
| AK127421 | Homo sapiens cDNA FLJ45513 fis, clone | 6.19 UP | 4.68E-05 |
| | BRTHA2021450 | | |
| NM_015085 | Homo sapiens GTPase activating Rap/RanGAP | 6.18 UP | 6.82E-05 |
| | domain-like 4 (GARNL4), mRNA | | |
| NM_001305 | Homo sapiens claudin 4 (CLDN4), mRNA | 6.17 UP | 3.48E-05 |
| NM_173078 | Homo sapiens SLIT and NTRK-like family, | 6.16 UP | 1.08E-03 |
| | member 4 (SLITRK4), mRNA | | |
| BG165745 | 602344592F1 NIH_MGC_89 Homo sapiens | 6.16 UP | 1.20E-04 |
| | cDNA clone IMAGE:4454470 5, mRNA | | |
| | sequence | | |
| NM_006074 | Homo sapiens tripartite motif-containing 22 | 6.15 UP | 1.15E-03 |
| | (TRIM22), mRNA | | |
| NM_005928 | Homo sapiens milk fat globule-EGF factor 8 | 6.15 UP | 1.63E-02 |
| | protein (MFGE8), mRNA | | |
| BF939703 | nac80e07.x1 NCI_CGAP_Brn23 Homo sapiens | 6.12 UP | 6.35E-05 |
| | cDNA clone IMAGE:3440725 3 similar to | | |
| | contains MER30.t3 MER30 repetitive element ;, | | |
| | mRNA sequence | | |
| BX115301 | BX115301 NCI_CGAP_Kid5 Homo sapiens | 6.11 UP | 8.26E-05 |
| | cDNA clone IMAGp998J093989 ; | | |
| | IMAGE:1574120, mRNA sequence | | |
| NM_018849 | Homo sapiens ATP-binding cassette, sub- | 6.09 UP | 3.23E-05 |
| | family B (MDR/TAP), member 4 (ABCB4), | | |
| | transcript variant B, mRNA | | |
| NM_015478 | Homo sapiens 1(3)mbt-like (Drosophila) | 6.09 UP | 2.43E-05 |
| | (L3MBTL), transcript variant I, mRNA | | |
| NM_015669 | Homo sapiens protocadherin beta 5 (PCDHB5), | 6.08 UP | 7.52E-05 |
| | mRNA | | |
| NM_147189 | Homo sapiens hypothetical protein MGC39325 | 6.06 UP | 3.18E-04 |
| | (MGC39325), mRNA | | |
| BQ018133 | UI-H-DPO-avv-I-18-0-UI.s1 NCI_CGAP_Fs1 | 6.06 UP | 1.78E-05 |
| | Homo sapiens cDNA clone IMAGE:5884961 3, | | |
| | mRNA sequence | | |
| N34295 | yy51e10.s1 | 6.04 UP | 8.29E-05 |
| | Soares_multiple_sclerosis_2NbHMSP Homo | | |
| | sapiens cDNA clone IMAGE:277098 3, mRNA | | |
| | sequence | | |
| NM_006472 | Homo sapiens thioredoxin interacting protein | 6.03 UP | 6.87E-05 |
| | (TXNIP), mRNA | | |
| NM_012472 | Homo sapiens leucine rich repeat containing 6 | 6.02 UP | 2.66E-05 |
| | (LRRC6), mRNA | | |
| AK123319 | Homo sapiens cDNA FLJ41325 fis, clone | 6 UP | 2.55E-04 |
| | BRAMY2046871 | | |
| AK123807 | Homo sapiens cDNA FLJ41813 fis, clone | 5.98 UP | 7.92E-05 |
| | NT2RI2011450 | | |
| NM_017786 | Homo sapiens hypothetical protein FLJ20366 | 5.98 UP | 1.85E-03 |
| | (FLJ20366), mRNA | | |
| NM_000212 | Homo sapiens integrin, beta 3 (platelet | 5.97 UP | 2.73E-05 |
| | glycoprotein IIIa, antigen CD61) (ITGB3), | | |
| | mRNA | | |
| NM_000817 | Homo sapiens glutamate decarboxylase 1 | 5.96 UP | 9.94E-03 |
| | (brain, 67kDa) (GAD1), transcript variant | | |
| | GAD67, mRNA | | |
| AK123617 | Homo sapiens cDNA FLJ41623 fis, clone | 5.92 UP | 1.38E-03 |
| | CTONG3009227 | | |
| BI493986 | df106g12.y1 Morton Fetal Cochlea Homo | 5.91 UP | 3.68E-04 |
| | sapiens cDNA clone IMAGE:2538815 5, mRNA | | |
| | sequence | | |
| NM_005264 | Homo sapiens GDNF family receptor alpha 1 | 5.91 UP | 4.09E-05 |
| | (GFRA1), transcript variant 1, mRNA | | |
| CN478597 | UI-CF-FNO-aeo-g-21-0-UI.s1 UI-CF-FNO Homo | 5.88 UP | 1.13E-03 |
| | sapiens cDNA clone UI-CF-FNO-aeo-g-21-0-UI | | |
| | 3, mRNA sequence | | |
| BQ021695 | UI-H-DH1-axi-f-22-0-UI.s1 NCI_CGAP_DH1 | 5.88 UP | 1.72E-04 |
| | Homo sapiens cDNA clone IMAGE:5829141 3, | | |
| | m RNA sequence | | |
| AL137698 | Homo sapiens mRNA; cDNA DKFZp434C1915 | 5.88 UP | 2.03E-05 |
| | (from clone DKFZp434C1915); partial cds | | |
| NM_014905 | Homo sapiens glutaminase (GLS), mRNA | 5.88 UP | 1.24E-04 |
| AA993234 | ot60a08.s1 Soares_testis_NHT Homo sapiens | 5.87 UP | 2.88E-05 |
| | cDNA clone IMAGE:1621142 3, mRNA | | |
| | sequence | | |
| N38890 | yy81f12.s1 | 5.87 UP | 1.25E-04 |
| | Soares_multiple_sclerosis_2NbHMSP Homo | | |
| | sapiens cDNA clone IMAGE:279983 3 similar | | |
| | to contains OFR.t3 OFR repetitive element ;, | | |
| | mRNA sequence | | |
| NM_197955 | Homo sapiens normal mucosa of esophagus | 5.85 UP | 1.85E-04 |
| | specific 1 (NMES1), transcript variant 1, mRNA | | |
| NM_014399 | Homo sapiens transmembrane 4 superfamily | 5.84 UP | 2.18E-05 |
| | member 13 (TM4SF13), mRNA | | |
| NM_024704 | Homo sapiens chromosome 20 open reading | 5.82 UP | 1.24E-04 |
| | frame 23 (C20orf23), mRNA | | |
| NM_002276 | Homo sapiens keratin 19 (KRT19), mRNA | 5.8 UP | 7.06E-03 |
| CD248442 | AGENCOURT_14095939 NIH_MGC_172 | 5.79 UP | 2.16E-04 |
| | Homo sapiens cDNA 5, mRNA sequence | | |
| AK024850 | Homo sapiens cDNA: FLJ21197 fis, clone | 5.79 UP | 1.45E-04 |
| | COL00201 | | |
| H83499 | ys91f12.r1 Soares retina N2b5HR Homo | 5.77 UP | 1.37E-02 |
| | sapiens cDNA clone IMAGE:222191 5, mRNA | | |
| | sequence | | |
| NM_153377 | Homo sapiens leucine-rich repeats and | 5.77 UP | 2.43E-05 |
| | immunoglobulin-like domains 3 (LRIG3), mRNA | | |
| NM_153267 | Homo sapiens MAM domain containing 2 | 5.76 UP | 2.31 E-04 |
| | (MAMDC2), mRNA | | |
| NM_144595 | Homo sapiens hypothetical protein FLJ30046 | 5.76 UP | 1.38E-03 |
| | (FLJ30046), mRNA | | |
| BF509155 | UI-H-BI4-aov-b-05-0-UI.s1 NCI_CGAP_Sub8 | 5.75 UP | 6.59E-05 |
| | Homo sapiens cDNA clone IMAGE:3086145 3, | | |
| | mRNA sequence | | |
| NM_002735 | Homo sapiens protein kinase, cAMP- | 5.73 UP | 2.73E-05 |
| | dependent, regulatory, type I, beta (PRKAR1B), | | |
| | mRNA | | |
| NM_025044 | Homo sapiens bicaudal C homolog 1 | 5.72 UP | 5.81 E-05 |
| | (Drosophila) (BICC1), mRNA | | |
| BM969191 | UI-CF-ENO-acp-e-22-0-UI.s1 UI-CF-ENO Homo | 5.69 UP | 1.67E-03 |
| | sapiens cDNA clone UI-CF-ENO-acp-e-22-0-UI | | |
| | 3, mRNA sequence | | |
| NM_000203 | Homo sapiens iduronidase, alpha-L- (IDUA), | 5.68 UP | 1.10E-03 |
| | mRNA | | |
| AI693580 | wd12d01.x1 NCI_CGAP_Co3 Homo sapiens | 5.67 UP | 9.23E-04 |
| | cDNA clone IMAGE:2327905 3, mRNA | | |
| | sequence | | |
| NM_006169 | Homo sapiens nicotinamide N- | 5.66 UP | 2.18E-05 |
| | methyltransferase (NNMT), mRNA | | |
| H46176 | yo14a11.s1 Soares adult brain N2b5HB55Y | 5.65 UP | 1.88E-05 |
| | Homo sapiens cDNA clone IMAGE:177884 3, | | |
| | mRNA sequence | | |
| NM_024306 | Homo sapiens fatty acid 2-hydroxylase (FA2H), | 5.63 UP | 4.19E-03 |
| | mRNA | | |
| BX110683 | BX110683 NCI_CGAP_GC6 Homo sapiens | 5.62 UP | 7.42E-04 |
| | cDNA clone IMAGp998E175727 ; | | |
| | IMAGE:2310904, mRNA sequence | | |
| NM_030765 | Homo sapiens UDP-GlcNAc:betaGal beta-1,3- | 5.58 UP | 1.80E-03 |
| | N-acetylglucosaminyltransferase 4 (B3GNT4), | | |
| | mRNA | | |
| NM_003929 | Homo sapiens RAB7, member RAS oncogene | 5.55 UP | 2.14E-05 |
| | family-like 1 (RAB7L1), mRNA | | |
| AI220066 | qg84d01.x1 Soares_NFL_T_GBC_S1 Homo | 5.54 UP | 2.13E-05 |
| | sapiens cDNA clone IMAGE:1841857 3, mRNA | | |
| | sequence | | |
| AA664452 | ae94d02.s1 Human bone marrow stromal cells | 5.54 UP | 4.13E-05 |
| | Homo sapiens cDNA clone IMAGE:1026723 3 | | |
| | similar to contains element LTR4 repetitive | | |
| | element ;, mRNA sequence | | |
| NM_007237 | Homo sapiens SP140 nuclear body protein | 5.53 UP | 2.41 E-04 |
| | (SP140), transcript variant 1, mRNA | | |
| NM_173662 | Homo sapiens hypothetical protein LOC285533 | 5.52 UP | 3.69E-03 |
| | (LOC285533), mRNA | | |
| NM_003020 | Homo sapiens secretory granule, | 5.52 UP | 2.08E-04 |
| | neuroendocrine protein 1 (7B2 protein) | | |
| | (SGNE1), mRNA | | |
| NM_206808 | Homo sapiens citrate lyase beta like (CLYBL), | 5.52 UP | 1.87E-04 |
| | transcript variant 2, mRNA | | |
| NM_004867 | Homo sapiens integral membrane protein 2A | 5.51 UP | 2.13E-05 |
| | (ITM2A), mRNA | | |
| NM_006333 | Homo sapiens nuclear DNA-binding protein | 5.51 UP | 7.84E-04 |
| | (C1D), transcript variant 1, mRNA | | |
| NM_014251 | Homo sapiens solute carrier family 25, member | 5.5 UP | 5.96E-04 |
| | 13 (citrin) (SLC25A13), mRNA | | |
| NM_006863 | Homo sapiens leukocyte immunoglobulin-like | 5.49 UP | 6.51 E-05 |
| | receptor, subfamily A (with TM domain), | | |
| | member 1 (LILRA1), mRNA | | |
| AK057166 | Homo sapiens cDNA FLJ32604 fis, clone | 5.49 UP | 1.85E-03 |
| | STOMA1000133 | | |
| NM_000076 | Homo sapiens cyclin-dependent kinase inhibitor | 5.48 UP | 4.65E-05 |
| | 1C (p57, Kip2) (CDKN1C), mRNA | | |
| BC040701 | Homo sapiens cDNA clone IMAGE:5736259, | 5.47 UP | 1.48E-04 |
| | partial cds | | |
| NM_152765 | Homo sapiens hypothetical protein MGC33510 | 5.46 UP | 4.76E-05 |
| | (MGC33510), mRNA | | |
| NM-019034 | Homo sapiens ras homolog gene family, | 5.46 UP | 2.64E-05 |
| | member F (in filopodia) (RHOF), mRNA | | |
| S81734 | tissue transglutaminase homologue | 5.46 UP | 3.92E-05 |
| | {alternatively spliced} [human, erythroleukemia | | |
| | cell line HEL GM06141A, mRNA, 2362 nt] | | |
| BM670793 | UI-E-DX1-agv-p-03-0-UI.s1 UI-E-DX1 Homo | 5.46 UP | 3.77E-05 |
| | sapiens cDNA clone UI-E-DX1-agv-p-03-0-UI 3, | | |
| | mRNA sequence | | |
| NM_002829 | Homo sapiens protein tyrosine phosphatase, | 5.45 UP | 4.79E-05 |
| | non-receptor type 3 (PTPN3), mRNA | | |
| BM727151 | UI-E-EJO-aij-f-05-0-UI.r1 UI-E-EJO Homo | 5.45 UP | 4.13E-05 |
| | sapiens cDNA clone UI-E-EJO-aij-f-05-0-UI 5, | | |
| | mRNA sequence | | |
| BF966833 | 602286668T1 NIH_MGC_95 Homo sapiens | 5.45 UP | 2.83E-05 |
| | cDNA clone IMAGE:4375360 3, mRNA | | |
| | sequence | | |
| NM_004904 | Homo sapiens cAMP responsive element | 5.44 UP | 1.09E-04 |
| | binding protein 5 (CREB5), mRNA | | |
| AA635788 | nr32h01.s1 NCI_CGAP_Pr22 Homo sapiens | 5.43 UP | 2.02E-03 |
| | cDNA clone IMAGE:1169713 3 similar to | | |
| | contains Alu repetitive element;, mRNA | | |
| | sequence | | |
| BM701989 | UI-E-CQ1-aex-j-06-0-UI.r1 UI-E-CQ1 Homo | 5.42 UP | 7.55E-04 |
| | sapiens cDNA clone UI-E-CQ1-aex-j-06-0-UI 5, | | |
| | mRNA sequence | | |
| NM_020665 | Homo sapiens transmembrane protein 27 | 5.4 UP | 5.46E-05 |
| | (TMEM27), mRNA | | |
| NM_019034 | Homo sapiens ras homolog gene family, | 5.39 UP | 5.72E-05 |
| | member F (in filopodia) (RHOF), mRNA | | |
| NM_017912 | Homo sapiens hect domain and RLD 6 | 5.38 UP | 5.85E-05 |
| | (HERC6), mRNA | | |
| NM_152433 | Homo sapiens kelch repeat and BTB (POZ) | 5.37 UP | 4.29E-04 |
| | domain containing 3 (KBTBD3), transcript | | |
| | variant 1, mRNA | | |
| CA314843 | UI-CF-FNO-afi-a-06-0-UI.s1 UI-CF-FNO Homo | 5.37 UP | 2.05E-04 |
| | sapiens cDNA clone UI-CF-FNO-afi-a-06-0-UI 3, | | |
| | m RNA sequence | | |
| NM_006058 | Homo sapiens TNFAIP3 interacting protein 1 | 5.36 UP | 2.25E-03 |
| | (TNIP1), mRNA | | |
| BU191317 | AGENCOURT_8074912 NIH_MGC_110 Homo | 5.36 UP | 4.45E-02 |
| | sapiens cDNA clone IMAGE:6086274 5, mRNA | | |
| | sequence | | |
| AL049437 | Homo sapiens mRNA; cDNA DKFZp586E1120 | 5.34 UP | 7.78E-04 |
| | (from clone DKFZp586E1120) | | |
| NM_030952 | Homo sapiens likely ortholog of rat SNF1/AMP- | 5.31 UP | 4.21 E-05 |
| | activated protein kinase (SNARK), mRNA | | |
| NM_004170 | Homo sapiens solute carrier family 1 | 5.31 UP | 1.15E-04 |
| | (neuronal/epithelial high affinity glutamate | | |
| | transporter, system Xag), member 1 (SLC1A1), | | |
| | mRNA | | |
| AW269776 | xv45b09.x1 Soares_NFL_T_GBC_S1 Homo | 5.3 UP | 1.97E-04 |
| | sapiens cDNA clone IMAGE:2816057 3, mRNA | | |
| | sequence | | |
| BM977716 | UI-CF-EN1-aef-b-21-0-UI.s1 UI-CF-EN1 Homo | 5.3 UP | 4.01E-03 |
| | sapiens cDNA clone UI-CF-EN1-aef-b-21-0-UI | | |
| | 3, mRNA sequence | | |
| AK023658 | Homo sapiens cDNA FLJ13596 fis, clone | 5.29 UP | 6.83E-04 |
| | PLACE1009637 | | |
| AF196185 | Homo sapiens atypical PKC isotype-specific | 5.28 UP | 4.57E-05 |
| | interacting protein long variant mRNA, | | |
| | complete cds | | |
| AL045014 | DKFZp434F134_s1 434 (synonym: htes3) | 5.27 UP | 2.67E-05 |
| | Homo sapiens cDNA clone DKFZp434F134 3, | | |
| | mRNA sequence | | |
| NM_004496 | Homo sapiens forkhead box A1(FOXA1), | 5.27 UP | 2.88E-03 |
| | mRNA | | |
| NM_033132 | Homo sapiens Zic family member 5 (odd-paired | 5.26 UP | 2.09E-05 |
| | homolog, Drosophila) (ZIC5), mRNA | | |
| NM_198182 | Homo sapiens transcription factor CP2-like 2 | 5.26 UP | 1.35E-02 |
| | (TFCP2L2), transcript variant 2, mRNA | | |
| NM_001657 | Homo sapiens amphiregulin (schwannoma- | 5.26 UP | 3.99E-05 |
| | derived growth factor) (AREG), mRNA | | |
| R92346 | yq06b10.s1 Soares fetal liver spleen 1 NFLS | 5.25 UP | 1.16E-04 |
| | Homo sapiens cDNA clone IMAGE:196123 3, | | |
| | mRNA sequence | | |
| BX537539 | Homo sapiens mRNA; cDNA DKFZp686A1130 | 5.25 UP | 4.80E-04 |
| | (from clone DKFZp686A1130) | | |
| AB041269 | Homo sapiens mRNA for keratin 19, partial cds, | 5.23 UP | 1.15E-02 |
| | isolate:K19-141 | | |
| BX090717 | BX090717 NCI_CGAP_Kid5 Homo sapiens | 5.2 UP | 9.02E-04 |
| | cDNA clone IMAGp9980154699 ; | | |
| | IMAGE:1916390, mRNA sequence | | |
| AK025743 | Homo sapiens cDNA: FLJ22090 fis, clone | 5.2 UP | 1.04E-04 |
| | HEP16084 | | |
| NM_002413 | Homo sapiens microsomal glutathione S- | 5.2 UP | 3.34E-05 |
| | transferase 2 (MGST2), mRNA | | |
| AA780946 | ag99c12.s1 Gessler Wilms tumor Homo | 5.2 UP | 2.40E-04 |
| | sapiens cDNA clone IMAGE:1155286 3, mRNA | | |
| | sequence | | |
| NM_032488 | Homo sapiens cornifelin (CNFN), mRNA | 5.2 UP | 1.78E-05 |
| NM_024997 | Homo sapiens activating transcription factor 7 | 5.19 UP | 2.40E-03 |
| | interacting protein 2 (ATF7IP2), mRNA | | |
| NM_002222 | Homo sapiens inositol 1,4,5-triphosphate | 5.19 UP | 1.41E-04 |
| | receptor, type 1 (ITPR1), mRNA | | |
| NM_000784 | Homo sapiens cytochrome P450, family 27, | 5.17 UP | 7.39E-03 |
| | subfamily A, polypeptide 1 (CYP27A1), nuclear | | |
| | gene encoding mitochondrial protein, mRNA | | |
| NM_005760 | Homo sapiens CCAAT/enhancer binding | 5.16 UP | 5.52E-05 |
| | protein zeta (CEBPZ), mRNA | | |
| BQ011746 | UI-1-BC1p-atk-b-09-0-UI.s1 NCI_CGAP_PI3 | 5.16 UP | 5.86E-04 |
| | Homo sapiens cDNA clone UI-1-BC1p-atk-b- | | |
| | 09-0-UI 3, mRNA sequence | | |
| NM_032728 | Homo sapiens chromosome 9 open reading | 5.15 UP | 1.85E-03 |
| | frame 67 (C9orf67), mRNA | | |
| NM_005755 | Homo sapiens Epstein-Barr virus induced gene | 5.15 UP | 2.33E-04 |
| | 3 (EBI3), mRNA | | |
| AK056852 | Homo sapiens cDNA FLJ32290 fis, clone | 5.15 UP | 1.32E-03 |
| | PROST2000463 | | |
| AK054990 | Homo sapiens cDNA FLJ30428 fis, clone | 5.15 UP | 1.85E-03 |
| | BRACE2008941 | | |
| D54580 | HUM144G01 B Clontech human fetal brain | 5.13 UP | 2.03E-04 |
| | polyA+ mRNA (#6535) Homo sapiens cDNA | | |
| | clone GEN-144G01 5, mRNA sequence | | |
| AL137535 | Homo sapiens mRNA; cDNA DKFZp434H2019 | 5.13 UP | 2.45E-02 |
| | (from clone DKFZp434H2019) | | |
| AL110252 | Homo sapiens mRNA; cDNA DKFZp566A1046 | 5.12 UP | 2.19E-03 |
| | (from clone DKFZp566A1046) | | |
| AI659523 | tt99d12.x1 NCI_CGAP_Pr28 Homo sapiens | 5.1 UP | 1.16E-03 |
| | cDNA clone IMAGE:2249687 3, mRNA | | |
| | sequence | | |
| AW137001 | UI-H-BI1-acu-c-05-0-UI,s1 NCI_CGAP_Sub3 | 5.1 UP | 6.90E-05 |
| | Homo sapiens cDNA clone IMAGE:2715632 3, | | |
| | mRNA sequence | | |
| BM969331 | UI-CF-DU1-aar-g-23-0-UI.s1 UI-CF-DU1 Homo | 5.08 UP | 3.31 E-03 |
| | sapiens cDNA clone UI-CF-DU1-aar-g-23-0-UI | | |
| | 3, mRNA sequence | | |
| NM_178823 | Homo sapiens chromosome 6 open reading | 5.08 UP | 3.66E-05 |
| | frame 165 (C6orf165), mRNA | | |
| NM_018476 | Homo sapiens brain expressed, X-linked 1 | 5.06 UP | 3.05E-05 |
| | (BEX1), mRNA | | |
| BF107212 | 601824290F1 NIH_MGC_79 Homo sapiens | 5.06 UP | 1.75E-04 |
| | cDNA clone IMAGE:4043879 5, mRNA | | |
| | sequence | | |
| NM_052890 | Homo sapiens peptidoglycan recognition | 5.05 UP | 3.95E-05 |
| | protein 2 (PGLYRP2), mRNA | | |
| BX459043 | BX459043 Homo sapiens PLACENTA Homo | 5.03 UP | 2.88E-05 |
| | sapiens cDNA clone CS0DE011YN10 3- | | |
| | PRIME, mRNA sequence | | |
| NM_032857 | Homo sapiens lactamase, beta (LACTB), | 5.02 UP | 1.76E-05 |
| | nuclear gene encoding mitochondrial protein, | | |
| | transcript variant 1, mRNA | | |
| NM_022746 | Homo sapiens hypothetical protein FLJ22390 | 5.02 UP | 4.49E-04 |
| | (FLJ22390), mRNA | | |
| AK025909 | Homo sapiens cDNA: FLJ22256 fis, clone | 5.02 UP | 4.54E-04 |
| | HRC02860 | | |
| NM_002247 | Homo sapiens potassium large conductance | 5.02 UP | 1.33E-03 |
| | calcium-activated channel, subfamily M, alpha | | |
| | member 1 (KCNMA1), mRNA | | |
| AI434849 | ti13b01.x1 NGI_GGAP_Kid11 Homo sapiens | 5.01 UP | 2.82E-04 |
| | cDNA clone IMAGE:2130313 3 similar to | | |
| | contains MER37.b1 MER37 repetitive | | |
| | element ;, mRNA sequence | | |
| AK091933 | Homo sapiens cDNA FLJ34614 fis, clone | 5.01 UP | 2.18E-05 |
| | KIDNE2014268 | | |
| NM_004331 | Homo sapiens BCL2/adenovirus E1 B 19kDa | 5 UP | 3.53E-05 |
| | interacting protein 3-like (BNIP3L), mRNA | | |
| AL119769 | DKFZp761E1224_r1 761 (synonym: hamy2) | 5 UP | 1.92E-03 |
| | Homo sapiens cDNA clone DKFZp761 E1224 5, | | |
| | mRNA sequence | | |
| NM_001451 | Homo sapiens forkhead box F1 (FOXF1), | 465.04 Down | 2.67E-05 |
| | mRNA | | |
| NM_144594 | Homo sapiens hypothetical protein FLJ32942 | 379.94 Down | 1.12E-05 |
| | (FLJ32942), mRNA | | |
| NM_002421 | Homo sapiens matrix metalloproteinase 1 | 365.32 Down | 2.43E-05 |
| | (interstitial collagenase) (MMP1), mRNA | | |
| AK026784 | Homo sapiens cDNA: FLJ23131 fis, clone | 331.17 Down | 8.46E-06 |
| | LNG08502 | | |
| NM_016307 | Homo sapiens paired related homeobox 2 | 309.99 Down | 8.39E-06 |
| | (PRRX2), mRNA | | |
| NM_006350 | Homo sapiens follistatin (FST), transcript | 232.51 Down | 8.39E-06 |
| | variant FST317, mRNA | | |
| NM_000089 | Homo sapiens collagen, type I, alpha 2 | 220.95 Down | 2.96E-05 |
| | (COL1A2), mRNA | | |
| NM_007036 | Homo sapiens endothelial cell-specific | 217.57 Down | 2.01 E-05 |
| | molecule 1 (ESM1), mRNA | | |
| AK021543 | Homo sapiens cDNA FLJ11481 fis, clone | 215.51 Down | 2.31 E-05 |
| | HEMBA1001803 | | |
| NM_004460 | Homo sapiens fibroblast activation protein, | 205.38 Down | 1.15E-05 |
| | alpha (FAP), mRNA | | |
| AK122739 | Homo sapiens cDNA FLJ16260 fis, clone | 190.76 Down | 1.15E-05 |
| | IMR322006947, highly similar to Rattus | | |
| | norvegicus mRNA for dHand protein | | |
| NM_000474 | Homo sapiens twist homolog 1 | 151.94 Down | 5.23E-06 |
| | (acrocephalosyndactyly 3; Saethre-Chotzen | | |
| | syndrome) (Drosophila) (TWIST1), mRNA | | |
| NM_152270 | Homo sapiens hypothetical protein FLJ34922 | 144.24 Down | 1.73E-05 |
| | (FLJ34922), mRNA | | |
| NM_001884 | Homo sapiens hyaluronan and proteoglycan | 134.45 Down | 2.18E-05 |
| | link protein 1 (HAPLN1), mRNA | | |
| NM_000710 | Homo sapiens bradykinin receptor B1 | 132.74 Down | 1.18E-05 |
| | (BDKRB1), mRNA | | |
| NM_021637 | Homo sapiens transmembrane protein 35 | 126.76 Down | 8.39E-06 |
| | (TMEM35), mRNA | | |
| NM_006350 | Homo sapiens follistatin (FST), transcript | 106.55 Down | 2.28E-05 |
| | variant FST317, mRNA | | |
| NM_000362 | Homo sapiens tissue inhibitor of | 102.59 Down | 1.76E-05 |
| | metalloproteinase 3 (Sorsby fundus dystrophy, | | |
| | pseudoinflammatory) (TIMP3), mRNA | | |
| NM_002091 | Homo sapiens gastrin-releasing peptide (GRP), | 101.58 Down | 2.46E-05 |
| | mRNA | | |
| NM_000609 | Homo sapiens chemokine (C-X-C motif) ligand | 97.42 Down | 3.48E-05 |
| | 12 (stromal cell-derived factor 1) (CXCL12), | | |
| | mRNA | | |
| NM_032638 | Homo sapiens GATA binding protein 2 | 84.58 Down | 1.37E-05 |
| | (GATA2), mRNA | | |
| NM_198148 | Homo sapiens carboxypeptidase X (M14 | 77.44 Down | 9.50E-05 |
| | family), member 2 (CPXM2), mRNA | | |
| W38393 | zb15c07.r1 Soares_fetal_lung_NbHL19W | 77.37 Down | 1.26E-05 |
| | Homo sapiens cDNA clone IMAGE:302124 5, | | |
| | mRNA sequence | | |
| AL831863 | Homo sapiens mRNA; cDNA DKFZp761J2017 | 77.09 Down | 2.65E-05 |
| | (from clone DKFZp761J2017) | | |
| AB067499 | Homo sapiens mRNA for KIAA1912 protein, | 76.12 Down | 3.65E-05 |
| | partial cds | | |
| BX089019 | BX089019 Soares_testis_NHT Homo sapiens | 72.98 Down | 1.25E-05 |
| | cDNA clone IMAGp998K243513 ; | | |
| | IMAGE:1391375, mRNA sequence | | |
| AI962169 | wq45c10.x1 NCI_CGAP_GC6 Homo sapiens | 65.52 Down | 4.53E-05 |
| | cDNA clone IMAGE:2474226 3, mRNA | | |
| | sequence | | |
| NM_139211 | Homo sapiens homeodomain-only protein | 64.01 Down | 1.72E-05 |
| | (HOP), transcript variant 2, mRNA | | |
| CD677332 | ho15f06.y1 Human Trabecular meshwork | 63.91 Down | 2.35E-05 |
| | cDNA: hohphq Homo sapiens cDNA clone | | |
| | ho15f06 5, mRNA sequence | | |
| NM_001442 | Homo sapiens fatty acid binding protein 4, | 63.47 Down | 1.37E-05 |
| | adipocyte (FABP4), mRNA | | |
| NM_205855 | Homo sapiens HWKM1940 (UNQ1940), mRNA | 58.05 Down | 1.89E-05 |
| W03013 | za02c04.r1 Soares melanocyte 2NbHM Homo | 53.58 Down | 2.67E-06 |
| | sapiens cDNA clone IMAGE:291366 5 similar | | |
| | to contains THR.t3 THR repetitive element ;, | | |
| | mRNA sequence | | |
| NM_002852 | Homo sapiens pentaxin-related gene, rapidly | 53.13 Down | 8.15E-04 |
| | induced by IL-1 beta (PTX3), mRNA | | |
| NM_024633 | Homo sapiens chromosome 14 open reading | 51.58 Down | 3.58E-05 |
| | frame 139 (C14orf139), mRNA | | |
| AK021543 | Homo sapiens cDNA FLJ11481 fis, clone | 51.28 Down | 1.09E-04 |
| | HEMBA1001803 | | |
| NM_002593 | Homo sapiens procollagen C-endopeptidase | 48.32 Down | 2.03E-05 |
| | enhancer (PCOLCE), mRNA | | |
| AI422199 | tf58d04.x1 NCI_CGAP_Brn23 Homo sapiens | 48.23 Down | 4.98E-05 |
| | cDNA clone IMAGE:2103463 3, mRNA | | |
| | sequence | | |
| NM_014459 | Homo sapiens protocadherin 17 (PCDH17), | 45.53 Down | 2.11E-05 |
| | mRNA | | |
| NM_016588 | Homo sapiens neuritin 1 (NRN1), mRNA | 44.52 Down | 1.78E-05 |
| NM_012242 | Homo sapiens dickkopf homolog 1 (Xenopus | 43.26 Down | 7.16E-06 |
| | laevis) (DKK1), mRNA | | |
| NM_000685 | Homo sapiens angiotensin II receptor, type 1 | 42.51 Down | 2.57E-04 |
| | (AGTR1), transcript variant 1, mRNA | | |
| AK056725 | Homo sapiens cDNA FLJ32163 fis, clone | 42.21 Down | 2.67E-06 |
| | PLACE6000371 | | |
| AI124557 | am58g02.x1 Johnston frontal cortex Homo | 41.42 Down | 8.25E-03 |
| | sapiens cDNA clone IMAGE:1539794 3, mRNA | | |
| | sequence | | |
| NM_020404 | Homo sapiens CD164 sialomucin-like 1 | 39.82 Down | 2.32E-05 |
| | (CD164L1), mRNA | | |
| NM_015170 | Homo sapiens sulfatase 1 (SULF1), mRNA | 38.91 Down | 2.36E-05 |
| CA843592 | ir49c12.x1 HR85 islet Homo sapiens cDNA | 37.67 Down | 1.25E-05 |
| | clone IMAGE:6548544 3, mRNA sequence | | |
| AY335938 | Homo sapiens homeodomain protein IRXA1 | 36.92 Down | 3.15E-05 |
| | (IRX1) mRNA, complete cds | | |
| NM_002961 | Homo sapiens S100 calcium binding protein A4 | 35.31 Down | 6.57E-05 |
| | (calcium protein, calvasculin, metastasin, | | |
| | murine placental homolog) (S100A4), transcript | | |
| | variant 1, mRNA | | |
| AK091731 | Homo sapiens cDNA FLJ34412 fis, clone | 34.47 Down | 2.83E-05 |
| | HEART2002432 | | |
| NM_006329 | Homo sapiens fibulin 5 (FBLN5), mRNA | 34.03 Down | 4.55E-05 |
| BQ020357 | UI-H-EDO-axk-p-07-0-UI.s1 NCI_CGAP_EDO | 33.2 Down | 1.82E-05 |
| | Homo sapiens cDNA clone IMAGE:5830134 3, | | |
| | mRNA sequence | | |
| NM_018013 | Homo sapiens hypothetical protein FLJ10159 | 32.86 Down | 1.15E-05 |
| | (FLJ10159), mRNA | | |
| NM_002402 | Homo sapiens mesoderm specific transcript | 32.83 Down | 2.14E-05 |
| | homolog (mouse) (MEST), transcript variant 1, | | |
| | mRNA | | |
| BQ001571 | UI-H-DH1-awr-i-18-0-UI.s1 NCI_CGAP_DH1 | 32.34 Down | 8.96E-06 |
| | Homo sapiens cDNA clone IMAGE:5893337 3, | | |
| | mRNA sequence | | |
| NM_032777 | Homo sapiens G protein-coupled receptor 124 | 31.96 Down | 1.18E-04 |
| | (GPR124), mRNA | | |
| AK124396 | Homo sapiens cDNA FLJ42405 fis, clone | 31.36 Down | 2.24E-05 |
| | ASTR03000474 | | |
| NM_002448 | Homo sapiens msh homeo box homolog 1 | 31.23 Down | 2.03E-05 |
| | (Drosophila) (MSX1), mRNA | | |
| NM_000090 | Homo sapiens collagen, type III, alpha 1 | 29.77 Down | 3.23E-04 |
| | (Ehlers-Danlos syndrome type IV, autosomal | | |
| | dominant) (COL3A1), mRNA | | |
| NM_001147 | Homo sapiens angiopoietin 2 (ANGPT2), | 29.68 Down | 2.51E-05 |
| | mRNA | | |
| NM_001146 | Homo sapiens angiopoietin 1 (ANGPT1), | 29.26 Down | 6.83E-05 |
| | transcript variant 1, mRNA | | |
| NM_000576 | Homo sapiens interleukin 1, beta (IL1B), mRNA | 29.06 Down | 1.15E-05 |
| AF052115 | Homo sapiens clone 23688 mRNA sequence | 26.15 Down | 1.15E-05 |
| NM_006439 | Homo sapiens mab-21-like 2 (C. elegans) | 25.51 Down | 1.20E-04 |
| | (MAB21 L2), mRNA | | |
| NM_016192 | Homo sapiens transmembrane protein with | 25.46 Down | 2.55E-05 |
| | EGF-like and two follistatin-like domains 2 | | |
| | (TMEFF2), mRNA | | |
| NM_002575 | Homo sapiens serine (or cysteine) proteinase | 24.67 Down | 5.40E-05 |
| | inhibitor, clade B (ovalbumin), member 2 | | |
| | (SERPINB2), mRNA | | |
| NM_007361 | Homo sapiens nidogen 2 (osteonidogen) | 23.56 Down | 2.64E-05 |
| | (NID2), mRNA | | |
| NM_152399 | Homo sapiens hypothetical protein FLJ30834 | 23.42 Down | 6.51 E-05 |
| | (FLJ30834), mRNA | | |
| NM_203370 | Homo sapiens similar to RIKEN cDNA | 22.99 Down | 1.43E-05 |
| | 6530418L21 (LOC389119), mRNA | | |
| | Homo sapiens GATA binding protein 3 | 22.51 Down | 1.41 E-04 |
| NM_00100229 | (GATA3), transcript variant 1, mRNA | | |
| 5 | | | |
| AK093256 | Homo sapiens cDNA FLJ35937 fis, clone | 22.26 Down | 3.04E-05 |
| | TESTI2011480 | | |
| U83115 | Human non-lens beta gamma-crystallin like | 22.18 Down | 3.77E-05 |
| | protein (AIM1) mRNA, partial cds | | |
| NM_006475 | Homo sapiens periostin, osteoblast specific | 21.99 Down | 7.38E-05 |
| | factor (POSTN), mRNA | | |
| NM_014178 | Homo sapiens syntaxin binding protein 6 | 21.69 Down | 3.36E-05 |
| | (amisyn) (STXBP6), mRNA | | |
| NM_005127 | Homo sapiens C-type (calcium dependent, | 21.46 Down | 3.71 E-05 |
| | carbohydrate-recognition domain) lectin, | | |
| | superfamily member 2 (activation-induced) | | |
| | (CLECSF2), mRNA | | |
| NM_022475 | Homo sapiens hedgehog interacting protein | 21.42 Down | 4.57E-05 |
| | (HHIP), mRNA | | |
| BX112628 | BX112628 Soares_fetal_lung_NbHL19W Homo | 20.99 Down | 4.47E-05 |
| | sapiens cDNA clone IMAGp998A09669 ; | | |
| | IMAGE:299024, mRNA sequence | | |
| NM_000810 | Homo sapiens gamma-aminobutyric acid | 20.84 Down | 5.50E-05 |
| | (GABA) A receptor, alpha 5 (GABRA5), mRNA | | |
| BQ934941 | AGENCOURT_8810373 NIH_MGC_101 Homo | 20.45 Down | 2.55E-05 |
| | sapiens cDNA clone IMAGE:6429485 5, mRNA | | |
| | sequence | | |
| BC071787 | Homo sapiens cDNA clone IMAGE:4610527, | 20.44 Down | 1.25E-05 |
| | partial cds | | |
| NM_033292 | Homo sapiens caspase 1, apoptosis-related | 19.88 Down | 6.18E-05 |
| | cysteine protease (interleukin 1, beta, | | |
| | convertase) (CASP1), transcript variant alpha, | | |
| | mRNA | | |
| NM_020809 | Homo sapiens Rho GTPase activating protein | 19.54 Down | 5.26E-05 |
| | 20 (ARHGAP20), mRNA | | |
| BE866150 | 601679068F1 NIH_MGC_53 Homo sapiens | 19.3 Down | 2.38E-05 |
| | cDNA clone IMAGE:3961768 5, mRNA | | |
| | sequence | | |
| AW021686 | df26h11.y1 Morton Fetal Cochlea Homo | 18.86 Down | 2.59E-05 |
| | sapiens cDNA clone IMAGE:2484717 5, mRNA | | |
| | sequence | | |
| BX109483 | BX109483 NCI_CGAP_Ov23 Homo sapiens | 18.74 Down | 9.84E-05 |
| | cDNA clone IMAGp998C165481 ; | | |
| | IMAGE:2216391, mRNA sequence | | |
| NM_001463 | Homo sapiens frizzled-related protein (FRZB), | 18.63 Down | 2.05E-04 |
| | mRNA | | |
| AK128325 | Homo sapiens cDNA FLJ46467 fis, clone | 18.11 Down | 1.78E-05 |
| | THYMU3022668 | | |
| AK096661 | Homo sapiens cDNA FLJ39342 fis, clone | 17.95 Down | 5.14E-05 |
| | OCBBF2018873 | | |
| NM_002531 | Homo sapiens neurotensin receptor 1 (high | 17.64 Down | 5.02E-05 |
| | affinity) (NTSR1), mRNA | | |
| NM_031908 | Homo sapiens C1q and tumor necrosis factor | 17.61 Down | 1.23E-04 |
| | related protein 2 (C1QTNF2), mRNA | | |
| AI085016 | ow88e06.s1 | 17.36 Down | 4.39E-05 |
| | Soares_fetal_liver_spleen_1 NFLS_S1 Homo | | |
| | sapiens cDNA clone IMAGE:1653922 3, mRNA | | |
| | sequence | | |
| AK095791 | Homo sapiens cDNA FLJ38472 fis, clone | 17.31 Down | 2.76E-04 |
| | FEBRA2022148 | | |
| NM_020927 | Homo sapiens KIAA1576 protein (KIAA1576), | 17 Down | 2.13E-05 |
| | mRNA | | |
| NM_024600 | Homo sapiens chromosome 16 open reading | 16.96 Down | 6.00E-04 |
| | frame 30 (C16orf30), mRNA | | |
| NM_012449 | Homo sapiens six transmembrane epithelial | 16.92 Down | 5.06E-05 |
| | antigen of the prostate (STEAP), mRNA | | |
| NM_004787 | Homo sapiens slit homolog 2 (Drosophila) | 16.69 Down | 1.78E-05 |
| | (SLIT2), mRNA | | |
| N95448 | zb81e11.s1 | 16.55 Down | 1.72E-05 |
| | Soares_senescent_fibroblasts_NbHSF Homo | | |
| | sapiens cDNA clone IMAGE:310028 3, mRNA | | |
| | sequence | | |
| NM_004657 | Homo sapiens serum deprivation response | 16.4 Down | 1.60E-05 |
| | (phosphatidylserine binding protein) (SDPR), | | |
| | mRNA | | |
| BC046364 | Homo sapiens flavoprotein oxidoreductase | 16.35 Down | 1.15E-05 |
| | MICAL3, mRNA (cDNA clone | | |
| | IMAGE:5737121 with apparent retained intron | | |
| NM_053044 | Homo sapiens serine protease HTRA3 | 16.29 Down | 3.77E-05 |
| | (HTRA3), mRNA | | |
| BQ011545 | UI-1-BC1p-asi-a-02-0-UI.s1 NCI_CGAP_PI3 | 16.22 Down | 5.21 E-04 |
| | Homo sapiens cDNA clone UI-1-BC1 p-asi-a-02- | | |
| | 0-UI 3, mRNA sequence | | |
| BC017939 | Homo sapiens, clone IMAGE:4275711, mRNA, | 15.98 Down | 5.83E-05 |
| | partial cds | | |
| NM_007289 | Homo sapiens membrane metallo- | 15.83 Down | 3.04E-05 |
| | endopeptidase (neutral endopeptidase, | | |
| | enkephalinase, CALLA, CD10) (MME), | | |
| | transcript variant 2b, mRNA | | |
| CA437861 | UI-H-DHO-aur-k-12-0-UI.s1 NCI_CGAP_DHO | 15.77 Down | 2.56E-04 |
| | Homo sapiens cDNA clone UI-H-DHO-aur-k-12- | | |
| | 0-UI 3, mRNA sequence | | |
| NM_006182 | Homo sapiens discoidin domain receptor | 15.71 Down | 4.79E-05 |
| | family, member 2 (DDR2), mRNA | | |
| NM_145239 | Homo sapiens similar to lymphocyte antigen 6 | 15.64 Down | 3.37E-05 |
| | complex, locus G5B; G5b protein; open reading | | |
| | frame 31 (LOC112476), mRNA | | |
| H15096 | ym29e11.r1 Soares infant brain 1NIB Homo | 15.58 Down | 2.20E-04 |
| | sapiens cDNA clone IMAGE:49250 5, mRNA | | |
| | sequence | | |
| NM_002851 | Homo sapiens protein tyrosine phosphatase, | 15.57 Down | 1.90E-04 |
| | receptor-type, Z polypeptide 1 (PTPRZ1), | | |
| | mRNA | | |
| NM_014217 | Homo sapiens potassium channel, subfamily K, | 15.13 Down | 1.31E-04 |
| | member 2 (KCNK2), mRNA | | |
| NM_000963 | Homo sapiens prostaglandin-endoperoxide | 14.94 Down | 1.88E-05 |
| | synthase 2 (prostaglandin G/H synthase and | | |
| | cyclooxygenase) (PTGS2), mRNA | | |
| BX115659 | BX115659 Soares_total_letus_Nb2HF8_9w | 14.81 Down | 2.66E-04 |
| | Homo sapiens cDNA clone IMAGp998C204119 | | |
| | ; IMAGE:1623883, mRNA sequence | | |
| NM_002729 | Homo sapiens hematopoietically expressed | 14.78 Down | 1.09E-03 |
| | homeobox (HHEX), mRNA | | |
| AI082087 | oz52h09.x1 | 14.73 Down | 3.66E-05 |
| | Soares_senescent_fibroblasts_NbHSF Homo | | |
| | sapiens cDNA clone IMAGE:1679009 3, mRNA | | |
| | sequence | | |
| NM_018431 | Homo sapiens docking protein 5 (DOK5), | 14.67 Down | 8.15E-04 |
| | transcript variant 1, mRNA | | |
| NM_004791 | Homo sapiens integrin, beta-like 1 (with EGF- | 14.42 Down | 2.18E-05 |
| | like repeat domains) (ITGBL1), mRNA | | |
| AK021531 | Homo sapiens cDNA FLJ11469 fis, clone | 14.36 Down | 5.21 E-04 |
| | HEMBA1001658 | | |
| NM_057179 | Homo sapiens twist homolog 2 (Drosophila) | 14.27 Down | 1.25E-05 |
| | (TWIST2), mRNA | | |
| NM_003619 | Homo sapiens protease, serine, 12 | 14.1 Down | 1.78E-05 |
| | (neurotrypsin, motopsin) (PRSS12), mRNA | | |
| NM_002518 | Homo sapiens neuronal PAS domain protein 2 | 14.05 Down | 5.49E-05 |
| | (NPAS2), mRNA | | |
| NM_000396 | Homo sapiens cathepsin K (pycnodysostosis) | 13.81 Down | 2.31 E-04 |
| | (CTSK), mRNA | | |
| NM_016206 | Homo sapiens colon carcinoma related protein | 13.76 Down | 1.81 E-04 |
| | (FLJ38507), mRNA | | |
| NM_021643 | Homo sapiens tribbles homolog 2 (Drosophila) | 13.62 Down | 8.39E-06 |
| | (TRIB2), mRNA | | |
| NM_000955 | Homo sapiens prostaglandin E receptor 1 | 13.52 Down | 1.70E-05 |
| | (subtype EP1), 42kDa (PTGER1), mRNA | | |
| NM_058187 | Homo sapiens chromosome 21 open reading | 13.44 Down | 1.53E-04 |
| | frame 63 (C21orf63), mRNA | | |
| CN479391 | UI-H-DF1-aug-f-02-0-UI.s9 NCI_CGAP_DF1 | 13.26 Down | 8.65E-05 |
| | Homo sapiens cDNA clone UI-H-DF1-aug-f-02- | | |
| | 0-UI 3, mRNA sequence | | |
| NM_006209 | Homo sapiens ectonucleotide | 13.21 Down | 3.28E-04 |
| | pyrophosphatase/phosphodiesterase 2 | | |
| | (autotaxin) (ENPP2), mRNA | | |
| NM_005584 | Homo sapiens mab-21-like 1 (C. elegans) | 12.73 Down | 1.01 E-04 |
| | (MAB21L1), mRNA | | |
| BM468332 | AGENCOURT_6432296 NIH_MGC_71 Homo | 12.64 Down | 2.13E-05 |
| | sapiens cDNA clone IMAGE:5535773 5, mRNA | | |
| | sequence | | |
| NM_020353 | Homo sapiens phospholipid scramblase 4 | 12.39 Down | 2.08E-04 |
| | (PLSCR4), mRNA | | |
| NM_031302 | Homo sapiens gycosyltransferase (LOC83468), | 12.39 Down | 1.88E-03 |
| | mRNA | | |
| NM_005308 | Homo sapiens G protein-coupled receptor | 12.28 Down | 1.18E-04 |
| | kinase 5 (GRK5), mRNA | | |
| NM_032883 | Homo sapiens chromosome 20 open reading | 12.17 Down | 1.78E-05 |
| | frame 100 (C20orf100), mRNA | | |
| NM_000110 | Homo sapiens dihydropyrimidine | 12.16 Down | 2.75E-05 |
| | dehydrogenase (DPYD), mRNA | | |
| BU536871 | AGENCOURT_10224340 NIH_MGC_141 | 12.05 Down | 4.21E-05 |
| | Homo sapiens cDNA clone IMAGE:6565454 5, | | |
| | mRNA sequence | | |
| NM_016270 | Homo sapiens Kruppel-like factor 2 (lung) | 11.98 Down | 1.94E-03 |
| | (KLF2), mRNA | | |
| NM_005110 | Homo sapiens glutamine-fructose-6-phosphate | 11.92 Down | 2.93E-05 |
| | transaminase 2 (GFPT2), mRNA | | |
| AK130711 | Homo sapiens cDNA FLJ27201 fis, clone | 11.81 Down | 5.02E-05 |
| | SYN03133 | | |
| BM991890 | UI-H-DF1-auk-h-02-0-UI.s1 NCI_CGAP_DF1 | 11.78 Down | 2.03E-05 |
| | Homo sapiens cDNA clone IMAGE:5870641 3, | | |
| | mRNA sequence | | |
| NM_000304 | Homo sapiens peripheral myelin protein 22 | 11.77 Down | 1.04E-03 |
| | (PMP22), transcript variant 1, mRNA | | |
| BM926469 | AGENCOURT_6644776 NIH_MGC_122 Homo | 11.53 Down | 7.16E-06 |
| | sapiens cDNA clone IMAGE:5766855 5, mRNA | | |
| | sequence | | |
| NM_001424 | Homo sapiens epithelial membrane protein 2 | 11.39 Down | 5.12E-04 |
| | (EMP2), mRNA | | |
| AK093762 | Homo sapiens cDNA FLJ36443 fis, clone | 11.18 Down | 1.34E-04 |
| | THYMU2012891 | | |
| NM_006288 | Homo sapiens Thy-1 cell surface antigen | 11.17 Down | 4.26E-04 |
| | (THY1), mRNA | | |
| NM_006183 | Homo sapiens neurotensin (NTS), mRNA | 11.14 Down | 8.52E-04 |
| NM_002522 | Homo sapiens neuronal pentraxin I (NPTX1), | 11.08 Down | 3.03E-04 |
| | mRNA | | |
| NM_016428 | Homo sapiens ABI gene family, member 3 | 11.03 Down | 2.83E-05 |
| | (ABI3), mRNA | | |
| NM_001769 | Homo sapiens CD9 antigen (p24) (CD9), | 11 Down | 2.45E-04 |
| | mRNA | | |
| AL833655 | Homo sapiens mRNA; cDNA DKFZp667O0320 | 10.8 Down | 1.73E-05 |
| | (from clone DKFZp667O0320) | | |
| R56121 | yg94d04.s1 Soares infant brain 1NIB Homo | 10.64 Down | 1.04E-04 |
| | sapiens cDNA clone IMAGE:41388 3, mRNA | | |
| | sequence | | |
| NM_012445 | Homo sapiens spondin 2, extracellular matrix | 10.63 Down | 1.36E-04 |
| | protein (SPON2), mRNA | | |
| NM_004811 | Homo sapiens leupaxin (LPXN), mRNA | 10.6 Down | 2.09E-05 |
| NM_002977 | Homo sapiens sodium channel, voltage-gated, | 10.51 Down | 2.09E-05 |
| | type IX, alpha (SCN9A), mRNA | | |
| BI598031 | 603248155F1 NIH_MGC_96 Homo sapiens | 10.5 Down | 3.34E-05 |
| | cDNA clone IMAGE:5300149 5, mRNA | | |
| | sequence | | |
| NM_173553 | Homo sapiens hypothetical protein FLJ25801 | 10.49 Down | 2.32E-05 |
| | (FLJ25801), mRNA | | |
| NM_001311 | Homo sapiens cysteine-rich protein 1 | 10.31 Down | 2.73E-05 |
| | (intestinal) (CRIP1), mRNA | | |
| NM_015916 | Homo sapiens family with sequence similarity | 10.28 Down | 1.59E-04 |
| | 26, member B (FAM26B), mRNA | | |
| NM_005595 | Homo sapiens nuclear factor I/A (NFIA), mRNA | 10.12 Down | 7.43E-04 |
| NM_182728 | Homo sapiens solute carrier family 7 (cationic | 9.98 Down | 6.78E-05 |
| | amino acid transporter, y+ system), member 8 | | |
| | (SLC7A8), transcript variant 2, mRNA | | |
| NM_152996 | Homo sapiens sialyltransferase 7 ((alpha-N- | 9.95 Down | 1.25E-05 |
| | acetylneuraminyl-2,3-beta-galactosyl-1,3)-N- | | |
| | acetyl galactosaminide alpha-2,6- | | |
| | sialyltransferase) C (SIAT7C), mRNA | | |
| AK091713 | Homo sapiens cDNA FLJ34394 fis, clone | 9.92 Down | 2.31 E-05 |
| | HCHON2000676 | | |
| AK022877 | Homo sapiens cDNA FLJ12815 fis, clone | 9.89 Down | 3.15E-05 |
| | NT2RP2002546 | | |
| NM_194250 | Homo sapiens similar to C630007C17Rik | 9.84 Down | 7.08E-05 |
| | protein (LOC91752), mRNA | | |
| BC039450 | Homo sapiens, clone IMAGE:5311619, mRNA | 9.77 Down | 2.60E-04 |
| NM_031894 | Homo sapiens ferritin, heavy polypeptide-like | 9.76 Down | 5.99E-04 |
| | 17 (FTHL17), mRNA | | |
| BI561641 | 603256058F1 NIH_MGC_97 Homo sapiens | 9.68 Down | 1.96E-03 |
| | cDNA clone IMAGE:5298374 5, mRNA | | |
| | sequence | | |
| BC028245 | Homo sapiens, Similar to hypothetical gene | 9.68 Down | 1.88E-05 |
| | LOC130797, clone IMAGE:5395354, mRNA | | |
| NM_031957 | Homo sapiens keratin associated protein 1-5 | 9.66 Down | 2.31 E-05 |
| | (KRTAP1-5), mRNA | | |
| AA947461 | ok20f03.s1 | 9.56 Down | 4.34E-05 |
| | Soares_NSF_F8_9W_OT_PA_P_S1 Homo | | |
| | sapiens cDNA clone I MAG E: 1508381 3, m RNA | | |
| | sequence | | |
| AK127309 | Homo sapiens cDNA FLJ45377 fis, clone | 9.55 Down | 1.17E-04 |
| | BRHIP3019956 | | |
| NM_016247 | Homo sapiens interphotoreceptor matrix | 9.51 Down | 2.09E-05 |
| | proteoglycan 2 (IMPG2), mRNA | | |
| NM_005574 | Homo sapiens LIM domain only 2 (rhombotin- | 9.46 Down | 4.10E-05 |
| | like 1) (LM02), mRNA | | |
| NM_004369 | Homo sapiens collagen, type VI, alpha 3 | 9.36 Down | 2.14E-05 |
| | (COL6A3), transcript variant 1, mRNA | | |
| NM_003973 | Homo sapiens ribosomal protein L14 (RPL14), | 9.3 Down | 6.77E-05 |
| | mRNA | | |
| NM_000459 | Homo sapiens TEK tyrosine kinase, endothelial | 9.25 Down | 3.10E-04 |
| | (venous malformations, multiple cutaneous and | | |
| | mucosal) (TEK), mRNA | | |
| NM_005397 | Homo sapiens podocalyxin-like (PODXL), | 9.06 Down | 6.52E-04 |
| | mRNA | | |
| BU675964 | UI-CF-DU1-aaf-b-24-0-UI.s1 UI-CF-DU1 Homo | 9 Down | 6.82E-05 |
| | sapiens cDNA clone UI-CF-DU1-aaf-b-24-0-UI | | |
| | 3, mRNA sequence | | |
| AI640484 | wa27f01.x1 NCI_CGAP_Kid11 Homo sapiens | 8.92 Down | 8.54E-05 |
| | cDNA clone IMAGE:2299321 3, mRNA | | |
| | sequence | | |
| NM_030781 | Homo sapiens collectin sub-family member 12 | 8.54 Down | 8.65E-05 |
| | (COLEC12), transcript variant II, mRNA | | |
| NM_001998 | Homo sapiens fibulin 2 (FBLN2), transcript | 8.53 Down | 4.60E-04 |
| | variant 2, mRNA | | |
| BC039369 | Homo sapiens, clone IMAGE:5271073, mRNA, | 8.51 Down | 4.85E-04 |
| | partial cds | | |
| AA436084 | zu03a02.r1 Soares_testis_NHT Homo sapiens | 8.51 Down | 8.23E-05 |
| | cDNA clone IMAGE:730730 5 similar to | | |
| | contains element PTR5 repetitive | | |
| | element ;, mRNA sequence | | |
| NM_015192 | Homo sapiens phospholipase C, beta 1 | 8.48 Down | 2.34E-04 |
| | (phosphoinositide-specific) (PLCB1), transcript | | |
| | variant 1, mRNA | | |
| NM_024420 | Homo sapiens phospholipase A2, group IVA | 8.48 Down | 2.23E-03 |
| | (cytosolic, calcium-dependent) (PLA2G4A), | | |
| | mRNA | | |
| BX095887 | BX095887 Soares_total_fetus_Nb2HF8_9w | 8.42 Down | 7.38E-05 |
| | Homo sapiens cDNA clone IMAGp998G124121 | | |
| | ; IMAGE:1624739, mRNA sequence | | |
| NM_006417 | Homo sapiens interferon-induced protein 44 | 8.37 Down | 2.46E-04 |
| | (IFI44), mRNA | | |
| AK022033 | Homo sapiens cDNA FLJ11971 fis, clone | 8.35 Down | 2.06E-04 |
| | HEMBB1001208 | | |
| N69782 | yz60b07.s1 Morton Fetal Cochlea Homo | 8.02 Down | 2.78E-04 |
| | sapiens cDNA clone IMAGE:287413 3, mRNA | | |
| | sequence | | |
| NM_023003 | Homo sapiens transmembrane 6 superfamily | 7.93 Down | 1.20E-04 |
| | member 1 (TM6SF1), mRNA | | |
| BC036034 | Homo sapiens endothelial differentiation, | 7.86 Down | 2.31 E-05 |
| | lysophosphatidic acid G-protein-coupled | | |
| | receptor, 2, transcript variant 2, mRNA (cDNA | | |
| | clone MGC:33157 IMAGE:5272431), complete | | |
| | cds | | |
| NM_005602 | Homo sapiens claudin 11 (oligodendrocyte | 7.84 Down | 2.43E-02 |
| | transmembrane protein) (CLDN11), mRNA | | |
| BC039676 | Homo sapiens, clone IMAGE:5173389, mRNA | 7.74 Down | 3.04E-05 |
| BC030692 | Homo sapiens ELAV (embryonic lethal, | 7.72 Down | 3.59E-05 |
| | abnormal vision, Drosophila)-like 2 (Hu antigen | | |
| | B), mRNA (cDNA clone MGC:26319 | | |
| | IMAGE:4826082), complete cds | | |
| NM_015278 | Homo sapiens SAM and SH3 domain | 7.58 Down | 1.38E-04 |
| | containing 1 (SASH1), mRNA | | |
| NM_031283 | Homo sapiens transcription factor 7-like 1 (T- | 7.57 Down | 4.12E-05 |
| | cell specific, HMG-box) (TCF7L1), mRNA | | |
| BC042378 | Homo sapiens, clone IMAGE:5277693, mRNA | 7.56 Down | 9.91 E-04 |
| NM_013363 | Homo sapiens procollagen C-endopeptidase | 7.49 Down | 5.18E-03 |
| | enhancer 2 (PCOLCE2), mRNA | | |
| NM_005923 | Homo sapiens mitogen-activated protein kinase | 7.48 Down | 2.05E-04 |
| | kinase kinase 5 (MAP3K5), mRNA | | |
| R53688 | yg84h04.r1 Soares infant brain 1NIB Homo | 7.43 Down | 2.31 E-05 |
| | sapiens cDNA clone IMAGE:40175 5, mRNA | | |
| | sequence | | |
| AI216469 | qh07h10.x1 Soares_NFL_T_GBC_S1 Homo | 7.28 Down | 4.92E-05 |
| | sapiens cDNA clone IMAGE:1844035 3, mRNA | | |
| | sequence | | |
| NM_014331 | Homo sapiens solute carrier family 7, (cationic | 7.27 Down | 2.76E-03 |
| | amino acid transporter, y+ system) member 11 | | |
| | (SLC7A11), mRNA | | |
| NM_138801 | Homo sapiens galactose mutarotase (aldose 1- | 7.24 Down | 5.50E-05 |
| | epimerase) (GALM), mRNA | | |
| AL049443 | Homo sapiens mRNA; cDNA DKFZp586N2020 | 7.22 Down | 2.83E-04 |
| | (from clone DKFZp586N2020) | | |
| NM_005328 | Homo sapiens hyaluronan synthase 2 (HAS2), | 7.17 Down | 3.96E-03 |
| | mRNA | | |
| NM_006072 | Homo sapiens chemokine (C-C motif) ligand 26 | 7.12 Down | 6.86E-05 |
| | (CCL26), mRNA | | |
| NM_020987 | Homo sapiens ankyrin 3, node of Ranvier | 7.09 Down | 7.31 E-05 |
| | (ankyrin G) (ANK3), transcript variant 1, mRNA | | |
| BX647541 | Homo sapiens mRNA; cDNA DKFZp686P0492 | 7.08 Down | 4.47E-05 |
| | (from clone DKFZp686P0492) | | |
| AI417595 | tg79h09.x1 Soares_NhHMPu_S1 Homo | 7.07 Down | 7.52E-04 |
| | sapiens cDNA clone IMAGE:2115041 3, mRNA | | |
| | sequence | | |
| AA158235 | zo76b02.s1 Stratagene pancreas (#937208) | 7.06 Down | 1.52E-04 |
| | Homo sapiens cDNA clone IMAGE:592779 3, | | |
| | mRNA sequence | | |
| NM_005583 | Homo sapiens lymphoblastic leukemia derived | 7.03 Down | 4.69E-05 |
| | sequence 1 (LYL1), mRNA | | |
| BU570253 | AGENCOURT_10401698 NIH_MGC_82 Homo | 7 Down | 2.35E-04 |
| | sapiens cDNA clone IMAGE:6618451 5, mRNA | | |
| | sequence | | |
| BM675371 | UI-E-EJO-aht-a-07-0-UI.s1 UI-E-EJO Homo | 6.95 Down | 4.53E-05 |
| | sapiens cDNA clone UI-E-EJO-aht-a-07-0-UI 3, | | |
| | mRNA sequence | | |
| AK024653 | Homo sapiens cDNA:FLJ21000 fis, clone | 6.93 Down | 6.77E-05 |
| | CAE03359 | | |
| W31037 | zb86c06.r1 | 6.91 Down | 4.95E-05 |
| | Soares_senescent_fibroblasts_NbHSF Homo | | |
| | sapiens cDNA clone IMAGE:310474 5, mRNA | | |
| | sequence | | |
| AL831835 | Homo sapiens mRNA; cDNA DKFZp547A0515 | 6.9 Down | 1.53E-03 |
| | (from clone DKFZp547A0515) | | |
| NM_001849 | Homo sapiens collagen, type VI, alpha 2 | 6.85 Down | 3.34E-05 |
| | (COL6A2), transcript variant 2C2, mRNA | | |
| NM_007084 | Homo sapiens SRY (sex determining region Y)- | 6.85 Down | 3.88E-02 |
| | box 21 (SOX21), mRNA | | |
| NM_025107 | Homo sapiens myc target 1 (MYCT1), mRNA | 6.82 Down | 6.89E-05 |
| AA393981 | zt58a03.r1 Soares_testis_NHT Homo sapiens | 6.82 Down | 4.58E-03 |
| | cDNA clone IMAGE:726508 5, mRNA | | |
| | sequence | | |
| NM_170744 | Homo sapiens unc-5 homolog B (C. elegans) | 6.82 Down | 9.92E-04 |
| | (UNC5B), mRNA | | |
| AL133118 | Homo sapiens mRNA; cDNA DKFZp586N0121 | 6.78 Down | 1.42E-03 |
| | (from clone DKFZp586N0121) | | |
| AK090603 | Homo sapiens cDNA FLJ33284 fis, clone | 6.76 Down | 1.36E-04 |
| | ASTRO2009458 | | |
| NM_002203 | Homo sapiens integrin, alpha 2 (CD49B, alpha | 6.74 Down | 4.93E-05 |
| | 2 subunit of VLA-2 receptor) (ITGA2), mRNA | | |
| NM_003713 | Homo sapiens phosphatidic acid phosphatase | 6.73 Down | 1.06E-04 |
| | type 2B (PPAP2B), transcript variant 1, mRNA | | |
| NM_001401 | Homo sapiens endothelial differentiation, | 6.69 Down | 9.75E-04 |
| | lysophosphatidic acid G-protein-coupled | | |
| | receptor, 2 (EDG2), transcript variant 1, mRNA | | |
| NM_020226 | Homo sapiens PR domain containing 8 | 6.68 Down | 2.31 E-04 |
| | (PRDM8), mRNA | | |
| N71963 | yz95e03.s1 Soares melanocyte 2NbHM Homo | 6.63 Down | 2.47E-04 |
| | sapiens cDNA clone IMAGE:290812 3 similar | | |
| | to contains Alu repetitive element;, mRNA | | |
| | sequence | | |
| AK127536 | Homo sapiens cDNA FLJ45629 fis, clone | 6.6 Down | 1.09E-04 |
| | CHONS2000797, highly similar to T-box | | |
| | transcription factor TBX15 | | |
| NM_006206 | Homo sapiens platelet-derived growth factor | 6.55 Down | 6.30E-04 |
| | receptor, alpha polypeptide (PDGFRA), mRNA | | |
| NM_016559 | Homo sapiens peroxisomal biogenesis factor 5- | 6.55 Down | 6.77E-04 |
| | like (PEX5L), mRNA | | |
| NM_012307 | Homo sapiens erythrocyte membrane protein | 6.51 Down | 9.62E-04 |
| | band 4.1-like 3 (EPB41L3), mRNA | | |
| R34294 | yh84b01.s1 Soares placenta Nb2HP Homo | 6.49 Down | 2.78E-05 |
| | sapiens cDNA clone IMAGE:136393 3, mRNA | | |
| | sequence | | |
| N36786 | yy34e08.s1 Soares melanocyte 2NbHM Homo | 6.49 Down | 6.12E-05 |
| | sapiens cDNA clone IMAGE:273158 3 similar | | |
| | to contains element MSR1 repetitive element ;, | | |
| | mRNA sequence | | |
| BX113851 | BX113851 Soares_totaUetus_Nb2HF8_9w | 6.44 Down | 8.41E-05 |
| | Homo sapiens cDNA clone IMAGp998G054116 | | |
| | ; IMAGE:1622812, mRNA sequence | | |
| BG484952 | 602503960F1 NIH_MGC_77 Homo sapiens | 6.43 Down | 9.06E-05 |
| | cDNA clone IMAGE:4617278 5, mRNA | | |
| | sequence | | |
| NM_005434 | Homo sapiens BENE protein (BENE), mRNA | 6.4 Down | 1.64E-05 |
| BX106577 | BX106577 Soares_NhHMPu_S1 Homo sapiens | 6.39 Down | 4.12E-05 |
| | cDNA clone IMAGp998H131854 ; | | |
| | IMAGE:754236, mRNA sequence | | |
| NM_144617 | Homo sapiens heat shock protein, alpha- | 6.37 Down | 2.83E-05 |
| | crystallin-related, B6 (HSPB6), mRNA | | |
| AK093529 | Homo sapiens cDNA FLJ36210 fis, clone | 6.29 Down | 1.52E-04 |
| | THYMU2000155 | | |
| NM_022731 | Homo sapiens nuclear ubiquitous casein kinase | 6.27 Down | 9.06E-05 |
| | and cyclin-dependent kinase substrate | | |
| | (NUCKS), mRNA | | |
| NM_018286 | Homo sapiens hypothetical protein FLJ10970 | 6.26 Down | 1.08E-03 |
| | (FLJ10970), mRNA | | |
| NM_002290 | Homo sapiens laminin, alpha 4 (LAMA4), | 6.19 Down | 6.97E-04 |
| | mRNA | | |
| NM_015184 | Homo sapiens phospholipase C-like 2 (PLCL2), | 6.18 Down | 5.91 E-04 |
| | mRNA | | |
| NM_017577 | Homo sapiens hypothetical protein | 6.17 Down | 3.60E-05 |
| | DKFZp434C0328 (DKFZp434C0328), mRNA | | |
| BQ350534 | RC1-HT0256-120400-019-d06 HT0256 Homo | 6.17 Down | 2.04E-04 |
| | sapiens cDNA, mRNA sequence | | |
| NM_004934 | Homo sapiens cadherin 18, type 2 (CDH18), | 6.17 | Down 3.03E-05 |
| | mRNA | | |
| NM_024769 | Homo sapiens adipocyte-specific adhesion | 6.15 Down | 2.41E-04 |
| | molecule (ASAM), mRNA | | |
| NM_197941 | Homo sapiens similar to ADAMTS-10 precursor | 6.14 Down | 1.06E-04 |
| | (A disintegrin and metalloproteinase with | | |
| | thrombospondin motifs 10) (ADAM-TS 10) | | |
| | (ADAM-TS10) (LOC345667), mRNA | | |
| BG118019 | 602351269F1 NIH_MGC_90 Homo sapiens | 6.14 Down | 5.02E-05 |
| | cDNA clone IMAGE:4446065 5, mRNA | | |
| | sequence | | |
| NM_005531 | Homo sapiens interferon, gamma-inducible | 6.13 Down | 4.46E-04 |
| | protein 16 (IFI16), mRNA | | |
| NM_005613 | Homo sapiens regulator of G-protein signalling | 6.08 Down | 1.04E-04 |
| | 4 (RGS4), mRNA | | |
| NM_002166 | Homo sapiens inhibitor of DNA binding 2, | 6.07 Down | 1.16E-03 |
| | dominant negative helix-loop-helix protein | | |
| | (ID2), mRNA | | |
| BC015720 | Homo sapiens, clone IMAGE:3909165, mRNA | 6.04 Down | 1.15E-05 |
| BF508005 | UI-H-BI4-apw-e-06-0-UI.s1 NCI_CGAP_Sub8 | 6.03 Down | 1.15E-05 |
| | Homo sapiens cDNA clone IMAGE:3088978 3, | | |
| | mRNA sequence | | |
| NM_007101 | Homo sapiens sarcosine dehydrogenase | 6.01 Down | 4.54E-03 |
| | (SARDH), mRNA | | |
| BG025371 | 602276295F1 NIH_MGC_85 Homo sapiens | 5.99 Down | 6.94E-05 |
| | cDNA clone IMAGE:4364351 5, mRNA | | |
| | sequence | | |
| NM_152666 | Homo sapiens hypothetical protein FLJ40773 | 5.99 Down | 4.55E-04 |
| | (FLJ40773), mRNA | | |
| BM542398 | AGENCOURT_6436663 NIH_MGC_72 Homo | 5.97 Down | 3.34E-05 |
| | sapiens cDNA clone IMAGE:5539574 5, mRNA | | |
| | sequence | | |
| AK054783 | Homo sapiens cDNA FLJ30221 fis, clone | 5.97 Down | 2.40E-04 |
| | BRACE2001742 | | |
| Al651524 | wb06g07.x1 NCI_CGAP_GC6 Homo sapiens | 5.93 Down | 3.22E-04 |
| | cDNA clone IMAGE:2304924 3, mRNA | | |
| | sequence | | |
| NM_005905 | Homo sapiens SMAD, mothers against DPP | 5.87 Down | 4.76E-04 |
| | homolog 9 (Drosophila) (SMAD9), mRNA | | |
| BG818762 | 602779092F2 NCI_CGAP_Brn67 Homo | 5.84 Down | 1.07E-04 |
| | sapiens cDNA clone IMAGE:4914502 5, mRNA | | |
| | sequence | | |
| NM_002487 | Homo sapiens necdin homolog (mouse) (NDN), | 5.83 Down | 2.72E-05 |
| | mRNA | | |
| NM_000167 | Homo sapiens glycerol kinase (GK), transcript | 5.8 Down | 5.10E-03 |
| | variant 2, mRNA | | |
| BC070147 | Homo sapiens cDNA clone IMAGE:4672631, | 5.74 Down | 7.92E-04 |
| | containing frame-shift errors | | |
| NM_001711 | Homo sapiens biglycan (BGN), mRNA | 5.71 Down | 4.13E-05 |
| NM_000407 | Homo sapiens glycoprotein lb (platelet), beta | 5.69 Down | 3.03E-05 |
| | polypeptide (GP1BB), mRNA | | |
| N28431 | yx35c03.r1 Soares melanocyte 2NbHM Homo | 5.69 Down | 6.03E-04 |
| | sapiens cDNA clone IMAGE:263716 5 similar | | |
| | to SP:PIR:S32603 S32603 collagen alpha 1(VI) | | |
| | chain - mouse ;, mRNA sequence | | |
| NM_002615 | Homo sapiens serine (or cysteine) proteinase | 5.66 Down | 2.02E-03 |
| | inhibitor, clade F (alpha-2 antiplasmin, pigment | | |
| | epithelium derived factor), member 1 | | |
| | (SERPINF1), mRNA | | |
| H18367 | yn49d06.r1 Soares adult brain N2b5HB55Y | 5.66 Down | 4.41 E-04 |
| | Homo sapiens cDNA clone IMAGE:171755 3, | | |
| | mRNA sequence | | |
| NM_018455 | Homo sapiens uncharacterized bone marrow | 5.64 Down | 3.03E-04 |
| | protein BM039 (BM039), mRNA | | |
| H24359 | ym56b03.s1 Soares infant brain 1NIB Homo | 5.61 Down | 2.57E-04 |
| | sapiens cDNA clone IMAGE:52294 3, mRNA | | |
| | sequence | | |
| BU078105 | im64b02.y1 HR85 islet Homo sapiens cDNA | 5.58 Down | 3.60E-04 |
| | clone IMAGE:6039866 5, mRNA sequence | | |
| NM_022576 | Homo sapiens phosducin (PDC), transcript | 5.55 Down | 9.39E-05 |
| | variant 2, mRNA | | |
| NM_033196 | Homo sapiens hypothetical ZNF-like protein | 5.54 Down | 1.49E-04 |
| | (LOC91120), mRNA | | |
| BC035066 | Homo sapiens, clone IMAGE:5259543, mRNA | 5.54 Down | 1.65E-04 |
| R14261 | yf79d05.r1 Soares infant brain 1NIB Homo | 5.51 Down | 2.36E-04 |
| | sapiens cDNA clone IMAGE:28642 5, mRNA | | |
| | sequence | | |
| BC042140 | Homo sapiens, clone IMAGE:5932306, mRNA | 5.48 Down | 5.05E-05 |
| NM_005654 | Homo sapiens nuclear receptor subfamily 2, | 5.48 Down | 1.02E-05 |
| | group F, member 1 (NR2F1), mRNA | | |
| NM_182746 | Homo sapiens MCM4 minichromosome | 5.48 Down | 9.96E-04 |
| | maintenance deficient 4 (S. cerevisiae) | | |
| | (MCM4), transcript variant 2, mRNA | | |
| BG573337 | 602595107F1 NIH_MGC_79 Homo sapiens | 5.43 Down | 3.72E-04 |
| | cDNA clone IMAGE:4724521 5, mRNA | | |
| | sequence | | |
| NM_194250 | Homo sapiens similar to C630007C17Rik | 5.43 Down | 3.30E-04 |
| | protein (LOC91752), mRNA | | |
| NM_015430 | Homo sapiens regeneration associated muscle | 5.43 Down | 3.49E-03 |
| | protease (DKFZP586H2123), transcript variant | | |
| | 1, mRNA | | |
| NM_006868 | Homo sapiens RAB31, member RAS oncogene | 5.41 Down | 2.11 E-05 |
| | family (RAB31), mRNA | | |
| BI494495 | df110f11.w1 Morton Fetal Cochlea Homo | 5.4 Down | 2.64E-03 |
| | sapiens cDNA clone IMAGE:2539149 3, mRNA | | |
| | sequence | | |
| AW025556 | wu97g10.x1 NCI_CGAP_Kid3 Homo sapiens | 5.36 Down | 1.89E-04 |
| | cDNA clone IMAGE:2528034 3, mRNA | | |
| | sequence | | |
| AI793182 | qz36a11.x5 NCI_CGAP_Kid11 Homo sapiens | 5.34 Down | 8.09E-04 |
| | cDNA clone IMAGE:2028956 3 similar to | | |
| | contains L1.t3 L1 repetitive element ;, mRNA | | |
| | sequence | | |
| NM_006208 | Homo sapiens ectonucleotide | 5.34 Down | 2.36E-04 |
| | pyrophosphatase/phosphodiesterase 1 | | |
| | (ENPP1), mRNA | | |
| C02345 | HUMGS0007544 Human adult (K.Okubo) | 5.33 Down | 3.45E-04 |
| | Homo sapiens cDNA, mRNA sequence | | |
| NM_032297 | Homo sapiens hypothetical protein | 5.32 Down | 3.38E-04 |
| | DKFZp761D112 (DKFZp761D112), mRNA | | |
| BM724062 | UI-E-E01-aiy-a-22-0-UI.r1 UI-E-E01 Homo | 5.25 Down | 2.96E-03 |
| | sapiens cDNA clone UI-E-E01-aiy-a-22-0-UI 5, | | |
| | mRNA sequence | | |
| NM_176891 | Homo sapiens interferon epsilon 1 (IFNE1), | 5.22 Down | 2.67E-04 |
| | mRNA | | |
| BX648959 | Homo sapiens mRNA; cDNA DKFZp686N2348 | 5.22 Down | 4.12E-05 |
| | (from clone DKFZp686N2348) | | |
| CA771688 | io81c08.x1 HR85 islet Homo sapiens cDNA | 5.22 Down | 3.35E-03 |
| | clone IMAGE:6132854 3, mRNA sequence | | |
| BC034315 | Homo sapiens hypothetical protein LOC90529, | 5.22 Down | 1.90E-04 |
| | mRNA (cDNA clone IMAGE:4827425), | | |
| | containing frame-shift errors | | |
| W88428 | zh72g09.s1 | 5.22 Down | 5.95E-04 |
| | Soares_feta_liver_spieen_1NFLS_S1 Homo | | |
| | sapiens cDNA clone IMAGE:417664 3, mRNA | | |
| | sequence | | |
| AI468014 | tj84g05.x1 | 5.21 Down | 9.55E-04 |
| | Soares_NSF_F8_9W_OT_PA_P_S1 Homo | | |
| | sapiens cDNA clone IMAGE:2148248 3 similar | | |
| | to contains element TAR1 repetitive element ;, | | |
| | mRNA sequence | | |
| NM_197941 | Homo sapiens similar to ADAMTS-10 precursor | 5.2 Down | 8.12E-04 |
| | (A disintegrin and metalloproteinase with | | |
| | thrombospondin motifs 10) (ADAM-TS 10) | | |
| | (ADAM-TS10) (LOC345667), mRNA | | |
| U10991 | Human G2 protein mRNA, partial cds | 5.19 Down | 7.92E-05 |
| AI888390 | wn30g10.x1 NCI_CGAP_Gas4 Homo sapiens | 5.19 Down | 2.07E-04 |
| | cDNA clone IMAGE:2447010 3 similar to | | |
| | contains element MER8 repetitive element ;, | | |
| | mRNA sequence | | |
| NM_012411 | Homo sapiens protein tyrosine phosphatase, | 5.19 Down | 3.62E-04 |
| | non-receptor type 22 (lymphoid) (PTPN22), | | |
| | transcript variant 2, mRNA | | |
| NM_030806 | Homo sapiens chromosome 1 open reading | 5.17 Down | 1.88E-05 |
| | frame 21 (C1orf21), mRNA | | |
| NM_005654 | Homo sapiens nuclear receptor subfamily 2, | 5.17 Down | 8.65E-05 |
| | group F, member 1 (NR2F1), mRNA | | |
| NM_153014 | Homo sapiens hypothetical protein FLJ30634 | 5.13 Down | 1.32E-03 |
| | (FLJ30634), mRNA | | |
| AB020691 | Homo sapiens mRNA for KIAA0884 protein, | 5.13 Down | 3.40E-05 |
| | partial cds | | |
| NM_020211 | Homo sapiens RGM domain family, member A | 5.1 Down | 1.51 E-04 |
| | (RGMA), mRNA | | |
| BG215747 | RST35420 Athersys RAGE Library Homo | 5.1 Down | 1.30E-04 |
| | sapiens cDNA, mRNA sequence | | |
| NM_002317 | Homo sapiens lysyl oxidase (LOX), mRNA | 5.09 Down | 1.28E-04 |
| NM_003608 | Homo sapiens G protein-coupled receptor 65 | 5.07 Down | 7.41 E-04 |
| | (GPR65), mRNA | | |
| NM_031305 | Homo sapiens Rho GTPase activating protein | 5.05 Down | 1.10E-04 |
| | 24 (ARHGAP24), mRNA | | |
| AK124391 | Homo sapiens cDNA FLJ42400 fis, clone | 5.03 Down | 9.74E-05 |
| | ASTRO2003581 | | |
| NM_005613 | Homo sapiens regulator of G-protein signalling | 5.02 Down | 7.55E-05 |
| | 4 (RGS4), mRNA | | |
| NM_054027 | Homo sapiens ankylosis, progressive homolog | 5 Down | 2.64E-05 |
| | (mouse) (ANKH), mRNA | | |

**TABLE VII C: GENES THAT WERE DIFFERENTIALLY EXPRESSED AT LEAST 5 FOLD IN EPITHELIAL VERSUS AF-I CELLS**

| Gene Identifier | Gene Name | Average fold change in Epithelial versus AF cells | Direction adj. p- value |
|---|---|---|---|
| AK091336 | Homo sapiens cDNA FLJ34017 fis, clone | 97.64 UP | 6.01 E-03 |
| | FCBBF2002626 | | |
| NM_004098 | Homo sapiens empty spiracles homolog | 83.49 UP | 8.10E-03 |
| | 2 (Drosophila) (EMX2), mRNA | | |
| AV702977 | AV702977 ADB Homo sapiens cDNA | 82.96 UP | 3.74E-03 |
| | clone ADBCVD08 5, mRNA sequence | | |
| BE877764 | 601486331F1 NIH_MGC_69 Homo | 60.78 UP | 2.79E-03 |
| | sapiens cDNA clone IMAGE:3888943 5, | | |
| | mRNA sequence | | |
| AK075003 | Homo sapiens cDNA FLJ90522 fis, clone | 48.1 UP | 1.02E-02 |
| | NT2RP4000108, highly similar to Human | | |
| | gene for neurofilament subunit NF-L | | |
| NM_014358 | Homo sapiens C-type (calcium | 48.04 UP | 1.99E-04 |
| | dependent, carbohydrate-recognition | | |
| | domain) lectin, superfamily member 9 | | |
| | (CLECSF9), mRNA | | |
| AK092401 | Homo sapiens cDNA FLJ35082 fis, clone | 40.58 UP | 1.77E-03 |
| | PLACE6005351 | | |
| NM_007029 | Homo sapiens stathmin-like 2 (STMN2), | 38.31 UP | 1.31 E-02 |
| | mRNA | | |
| AW300043 | xs45a09.x1 NCI_CGAP_Kid11 Homo | 25.26 UP | 1.35E-02 |
| | sapiens cDNA clone IMAGE:2772568 3, | | |
| | mRNA sequence | | |
| NM_006158 | Homo sapiens neurofilament, light | 23.08 UP | 1.76E-03 |
| | polypeptide 68kDa (NEFL), mRNA | | |
| NM_003222 | Homo sapiens transcription factor AP-2 | 22.98 UP | 1.59E-02 |
| | gamma (activating enhancer binding | | |
| | protein 2 gamma) (TFAP2C), mRNA | | |
| AK023647 | Homo sapiens cDNA FLJ13585 fis, clone | 22.77 UP | 7.48E-04 |
| | PLACE1009150 | | |
| BC044843 | Homo sapiens hypothetical protein | 21.74 UP | 4.67E-03 |
| | LOC339535, mRNA (cDNA clone | | |
| | IMAGE:5186761), partial cds | | |
| BQ003501 | UI-H-EI1-azd-p-06-0-UI.s1 | 20.08 UP | 9.54E-04 |
| | NCI_CGAP_EI1 Homo sapiens cDNA | | |
| | clone IMAGE:5847413 3, mRNA | | |
| | sequence | | |
| AA075748 | zm89e04.r1 Stratagene ovarian cancer | 19.94 UP | 7.10E-03 |
| | (#937219) Homo sapiens cDNA clone | | |
| | IMAGE:545118 5, mRNA sequence | | |
| BF000009 | 7h15g04.x1 NCI_CGAP_Co16 Homo | 19.92 UP | 7.67E-03 |
| | sapiens cDNA clone IMAGE:3316086 3, | | |
| | mRNA sequence | | |
| NM_004867 | Homo sapiens integral membrane | 19.86 UP | 2.62E-02 |
| | protein 2A (ITM2A), mRNA | | |
| H85497 | yv88b07.r1 Soares melanocyte 2NbHM | 19.75 UP | 7.71 E-05 |
| | Homo sapiens cDNA clone | | |
| | IMAGE:249781 5, mRNA sequence | | |
| BM713465 | UI-E-EJ0-aho-m-22-0-UI.r1 UI-E-EJ0 | 18.57 UP | 1.68E-02 |
| | Homo sapiens cDNA clone UI-E-EJ0- | | |
| | aho-m-22-0-UI 5, mRNA sequence | | |
| BU569937 | AGENCOURT_10399817 NIH_MGC_82 | 18.3 UP | 1.68E-02 |
| | Homo sapiens cDNA clone | | |
| | IMAGE:6618011 5, mRNA sequence | | |
| NM_032782 | Homo sapiens hepatitis A virus cellular | 18.05 UP | 2.97E-03 |
| | receptor 2 (HAVCR2), mRNA | | |
| NM_013387 | Homo sapiens ubiquinol-cytochrome c | 17.68 UP | 1.30E-04 |
| | reductase complex (7.2 kD) (HSPC051), | | |
| | transcript variant 1, mRNA | | |
| BM988338 | UI-H-DHO-asd-f-10-0-UI.s1 | 17.23 UP | 9.27E-03 |
| | NCI_CGAP_DHO Homo sapiens cDNA | | |
| | clone IMAGE:5857545 3, mRNA | | |
| | sequence | | |
| BQ025821 | UI-1-BB1p-aye-f-10-0-UI.s1 | 15.46 UP | 1.08E-02 |
| | NCI_CGAP_PI6 Homo sapiens cDNA | | |
| | clone UI-1-BB1p-aye-f-10-0-UI 3, mRNA | | |
| | sequence | | |
| BF515657 | UI-H-BW1-anu-e-05-0-UI.s1 | 15.17 UP | 8.95E-03 |
| | NCI_CGAP_Sub7 Homo sapiens cDNA | | |
| | clone IMAGE:3083601 3, mRNA | | |
| | sequence | | |
| NM_001977 | Homo sapiens glutamyl aminopeptidase | 14.59 UP | 5.24E-05 |
| | (aminopeptidase A) (ENPEP), mRNA | | |
| NM_197955 | Homo sapiens normal mucosa of | 14.33 UP | 2.54E-03 |
| | esophagus specific 1 (NMES1), | | |
| | transcript variant 1, mRNA | | |
| NM_180991 | Homo sapiens solute carrier organic | 13.46 UP | 2.20E-02 |
| | anion transporter family, member 4C1 | | |
| | (SLC04C1), mRNA | | |
| R44402 | yg37a01.s1 Soares infant brain 1NIB | 13.17 UP | 1.56E-04 |
| | Homo sapiens cDNA clone | | |
| | IMAGE:34639 3 similar to contains | | |
| | MER35 repetitive element ;, mRNA | | |
| | sequence | | |
| CF137545 | UI-HF-BNO-ane-d-05-0-UI.r1 | 12.96 UP | 6.05E-03 |
| | NIH_MGC_50 Homo sapiens cDNA | | |
| | clone IMAGE:3092384 5, mRNA | | |
| | sequence | | |
| NM_016269 | Homo sapiens lymphoid enhancer- | 12.69 UP | 7.27E-03 |
| | binding factor 1 (LEF1), mRNA | | |
| NM_001680 | Homo sapiens FXYD domain containing | 12.65 UP | 2.38E-03 |
| | ion transport regulator 2 (FXYD2), | | |
| | transcript variant a, mRNA | | |
| NM_003063 | Homo sapiens sarcolipin (SLN), mRNA | 12.54 UP | 3.04E-02 |
| NM_012204 | Homo sapiens general transcription | 12.51 UP | 3.10E-02 |
| | factor IIIC, polypeptide 4, 90kDa | | |
| | (GTF3C4), mRNA | | |
| NM_003810 | Homo sapiens tumor necrosis factor | 12.4 UP | 9.19E-05 |
| | (ligand) superfamily, member 10 | | |
| | (TNFSF10), mRNA | | |
| NM_004591 | Homo sapiens chemokine (C-C motif) | 11.84 UP | 3.08E-02 |
| | ligand 20 (CCL20), mRNA | | |
| NM_020927 | Homo sapiens KIAA1576 protein | 11.77 UP | 6.78E-03 |
| | (KIAA1576), mRNA | | |
| NM_014751 | Homo sapiens metastasis suppressor 1 | 11.5 UP | 6.60E-05 |
| | (MTSS1), mRNA | | |
| NM_031311 | Homo sapiens carboxypeptidase, | 10.36 UP | 8.15E-03 |
| | vitellogenic-like (CPVL), transcript variant | | |
| | 1, mRNA | | |
| NM_005382 | Homo sapiens neurofilament 3 (150kDa | 10.12 UP | 4.45E-02 |
| | medium) (NEF3), mRNA | | |
| NM_033554 | Homo sapiens major histocompatibility | 9.84 UP | 4.35E-02 |
| | complex, class II, DP alpha 1 (HLA- | | |
| | DPA1), mRNA | | |
| AI632692 | wa33b05.x1 NCI_CGAP_Kid11 Homo | 9.7 UP | 3.01 E-02 |
| | sapiens cDNA clone IMAGE:2299857 3, | | |
| | mRNA sequence | | |
| NM_030923 | Homo sapiens hypothetical protein | 9.51 UP | 2.72E-02 |
| | DKFZp566N034 (DKFZP566N034), | | |
| | mRNA | | |
| NM_152550 | Homo sapiens SH3 domain containing | 9.31 UP | 4.41 E-02 |
| | ring finger 2 (SH3RF2), mRNA | | |
| AW445209 | UI-H-BI3-akc-g-11-0-UI.s1 | 9.27 UP | 1.37E-03 |
| | NCI_CGAP_Sub5 Homo sapiens cDNA | | |
| | clone IMAGE:2733908 3, mRNA | | |
| | sequence | | |
| AB011095 | Homo sapiens mRNA for KIAA0523 | 8.77 UP | 1.82E-04 |
| | protein, partial cds | | |
| NM_194463 | Homo sapiens ring finger protein 128 | 8.75 UP | 2.95E-03 |
| | (RNF128), transcript variant 1, mRNA | | |
| NM_013427 | Homo sapiens Rho GTPase activating | 8.73 UP | 7.38E-04 |
| | protein 6 (ARHGAP6), transcript variant | | |
| | 1, mRNA | | |
| NM_012105 | Homo sapiens beta-site APP-cleaving | 8.67 UP | 1.49E-04 |
| | enzyme 2 (BACE2), transcript variant a, | | |
| | mRNA | | |
| NM_000640 | Homo sapiens interleukin 13 receptor, | 8.29 UP | 4.39E-02 |
| | alpha 2 (IL13RA2), mRNA | | |
| NM_001977 | Homo sapiens glutamyl aminopeptidase | 8.16 UP | 6.01E-03 |
| | (aminopeptidase A) (ENPEP), mRNA | | |
| T56535 | yb33g07.r1 Stratagene fetal spleen | 8.08 UP | 1.24E-04 |
| | (#937205) Homo sapiens cDNA clone | | |
| | IMAGE:73020 5, mRNA sequence | | |
| NM_022440 | Homo sapiens mal, T-cell differentiation | 8.01 UP | 4.29E-02 |
| | protein (MAL), transcript variant d, | | |
| | mRNA | | |
| BM711923 | UI-E-CL1-afc-m-16-0-UI.r1 UI-E-CL1 | 7.87 UP | 8.56E-04 |
| | Homo sapiens cDNA clone UI-E-CL1- | | |
| | afc-m-16-0-UI 5, mRNA sequence | | |
| NM_005181 | Homo sapiens carbonic anhydrase III, | 7.69 UP | 1.46E-02 |
| | muscle specific (CA3), mRNA | | |
| NM_030781 | Homo sapiens collectin sub-family | 7.56 UP | 8.07E-04 |
| | member 12 (COLEC12), transcript | | |
| | variant II, mRNA | | |
| NM_001305 | Homo sapiens claudin 4 (CLDN4), | 7.41 UP | 3.62E-04 |
| | mRNA | | |
| NM_000055 | Homo sapiens butyrylcholinesterase | 7.26 UP | 4.51 E-03 |
| | (BCHE), mRNA | | |
| NM_153183 | Homo sapiens nudix (nucleoside | 7.23 UP | 3.53E-02 |
| | diphosphate linked moiety X)-type motif | | |
| | 10 (NUDT10), mRNA | | |
| BI759570 | 603046987F1 NIH_MGC_116 Homo | 7.11 UP | 3.86E-02 |
| | sapiens cDNA clone IMAGE:5187285 5, | | |
| | mRNA sequence | | |
| NM_015559 | Homo sapiens SET binding protein 1 | 6.97 UP | 3.67E-02 |
| | (SETBP1), mRNA | | |
| NM_005562 | Homo sapiens laminin, gamma 2 | 6.95 UP | 2.06E-04 |
| | (LAMC2), transcript variant 1, mRNA | | |
| NM_013430 | Homo sapiens gamma- | 6.9 UP | 3.86E-02 |
| | glutamyltransferase 1 (GGT1), transcript | | |
| | variant 3, mRNA | | |
| AA908815 | og77h08.s1 NCI_CGAP_Ov8 Homo | 6.8 UP | 3.10E-02 |
| | sapiens cDNA clone IMAGE:1454367 3, | | |
| | mRNA sequence | | |
| NM_022843 | Homo sapiens protocadherin 20 | 6.73 UP | 5.39E-03 |
| | (PCDH20), mRNA | | |
| NM_018076 | Homo sapiens armadillo repeat | 6.71 UP | 7.53E-04 |
| | containing 4 (ARMC4), mRNA | | |
| NM_005822 | Homo sapiens Down syndrome critical | 6.67 UP | 2.38E-02 |
| | region gene 1-like 1 (DSCR1L1), mRNA | | |
| NM_006393 | Homo sapiens nebulette (NEBL), | 6.66 UP | 4.24E-02 |
| | transcript variant 1, mRNA | | |
| CA391258 | cs13a10.x1 Human Retinal pigment | 6.56 UP | 9.19E-05 |
| | epithelium/choroid cDNA (Un- | | |
| | normalized, unamplified): cs Homo | | |
| | sapiens cDNA clone cs13a10 3, mRNA | | |
| | sequence | | |
| AK026966 | Homo sapiens cDNA: FLJ23313 fis, | 6.31 UP | 1.30E-04 |
| | clone HEP11919 | | |
| NM_020661 | Homo sapiens activation-induced | 6.12 UP | 3.01 E-02 |
| | cytidine deaminase (AICDA), mRNA | | |
| NM_004840 | Homo sapiens Rac/Cdc42 guanine | 6.09 UP | 1.19E-03 |
| | nucleotide exchange factor (GEF) 6 | | |
| | (ARHGEF6), mRNA | | |
| BC021684 | Homo sapiens, clone IMAGE:3827252, | 6.03 UP | 4.71 E-03 |
| | mRNA | | |
| NM_032551 | Homo sapiens G protein-coupled | 5.96 UP | 4.33E-02 |
| | receptor 54 (GPR54), mRNA | | |
| NM_024726 | Homo sapiens IQ motif containing with | 5.94 UP | 1.84E-04 |
| | AAA domain (IQCA), mRNA | | |
| NM_001657 | Homo sapiens amphiregulin | 5.91 UP | 1.05E-03 |
| | (schwannoma-derived growth factor) | | |
| | (AREG), mRNA | | |
| NM_006033 | Homo sapiens lipase, endothelial (LIPG), | 5.9 UP | 1.76E-04 |
| | mRNA | | |
| NM_002089 | Homo sapiens chemokine (C-X-C motif) | 5.88 UP | 1.13E-04 |
| | ligand 2 (CXCL2), mRNA | | |
| AK074097 | Homo sapiens mRNA for FLJ00168 | 5.87 UP | 1.17E-03 |
| | protein | | |
| BF509573 | UI-H-BI4-apf-b-11-0-UI.s1 | 5.78 UP | 1.40E-04 |
| | NCI_CGAP_Sub8 Homo sapiens cDNA | | |
| | clone IMAGE:3086949 3, mRNA | | |
| | sequence | | |
| H23441 | ym52f11.s1 Soares infant brain 1NIB | 5.77 UP | 6.87E-04 |
| | Homo sapiens cDNA clone | | |
| | IMAGE:51888 3, mRNA sequence | | |
| NM_031426 | Homo sapiens chromosome 9 open | 5.63 UP | 3.32E-05 |
| | reading frame 58 (C9orf58), transcript | | |
| | variant 1, mRNA | | |
| NM_013410 | Homo sapiens adenylate kinase 3 (AK3), | 5.54 UP | 1.14E-04 |
| | nuclear gene encoding mitochondrial | | |
| | protein, transcript variant 2, mRNA | | |
| NM_003236 | Homo sapiens transforming growth | 5.51 UP | 3.09E-02 |
| | factor, alpha (TGFA), mRNA | | |
| NM_152369 | Homo sapiens hypothetical protein | 5.46 UP | 1.02E-04 |
| | MGC45474 (MGC45474), mRNA | | |
| NM_153229 | Homo sapiens hypothetical protein | 5.4 UP | 1.92E-03 |
| | FLJ33318 (FLJ33318), mRNA | | |
| NM_000216 | Homo sapiens Kallmann syndrome 1 | 5.23 UP | 1.13E-04 |
| | sequence (KAL1), mRNA | | |
| H89526 | yw28b04.r1 Morton Fetal Cochlea Homo | 5.15 UP | 9.54E-05 |
| | sapiens cDNA clone IMAGE:253519 5, | | |
| | mRNA sequence | | |
| AL832916 | Homo sapiens mRNA; cDNA | 5.12 UP | 6.74E-03 |
| | DKFZp762I0915 (from clone | | |
| | DKFZp762I0915) | | |
| NM_002899 | Homo sapiens retinol binding protein 1, | 5.07 UP | 1.11E-03 |
| | cellular (RBP1), mRNA | | |
| CA417015 | UI-H-FEO-bbp-d-21-0-UI.s1 | 5.07 UP | 1.77E-03 |
| | NCI_CGAP_FEO Homo sapiens cDNA | | |
| | clone UI-H-FEO-bbp-d-21-0-UI 3, mRNA | | |
| | sequence | | |
| AW263542 | xn80f01.x1 Soares_NFL_T_GBC_S1 | 5.07 UP | 9.70E-03 |
| | Homo sapiens cDNA clone | | |
| | IMAGE:2700793 3, mRNA sequence | | |
| T47612 | yb15h03.s1 Stratagene placenta | 5.02 UP | 7.57E-03 |
| | (#937225) Homo sapiens cDNA clone | | |
| | IMAGE:71285 3, mRNA sequence | | |
| NM_002402 | Homo sapiens mesoderm specific | 83.38 Down | 1.30E-04 |
| | transcript homolog (mouse) (MEST), | | |
| | transcript variant 1, mRNA | | |
| NM_001884 | Homo sapiens hyaluronan and | 71.21 Down | 5.97E-05 |
| | proteoglycan link protein 1 (HAPLN1), | | |
| | mRNA | | |
| NM_006439 | Homo sapiens mab-21-like 2 (C. | 70.58 Down | 7.65E-05 |
| | elegans)(MAB21L2), mRNA | | |
| NM_001202 | Homo sapiens bone morphogenetic | 50.48 Down | 3.32E-05 |
| | protein 4 (BMP4), transcript variant 1, | | |
| | mRNA | | |
| NM_017671 | Homo sapiens chromosome 20 open | 50.28 Down | 4.04E-05 |
| | reading frame 42 (C20orf42), mRNA | | |
| AK124396 | Homo sapiens cDNA FLJ42405 fis, clone | 45.55 Down | 6.98E-05 |
| | ASTR03000474 | | |
| NM_015170 | Homo sapiens sulfatase 1 (SULF1), | 38.04 Down | 7.58E-05 |
| | mRNA | | |
| BC037316 | Homo sapiens, clone IMAGE:5259432, | 34.94 Down | 7.08E-05 |
| | mRNA | | |
| NM_001562 | Homo sapiens interleukin 18 (interferon- | 34.48 Down | 7.58E-05 |
| | gamma-inducing factor) (IL18), mRNA | | |
| AI469032 | ti70a01.x1 NCI_CGAP_Kid11 Homo | 28.72 Down | 4.44E-05 |
| | sapiens cDNA clone IMAGE:2137320 3, | | |
| | mRNA sequence | | |
| NM_016307 | Homo sapiens paired related homeobox | 28.57 Down | 3.32E-05 |
| | 2 (PRRX2), mRNA | | |
| NM_022475 | Homo sapiens hedgehog interacting | 27 Down | 7.14E-05 |
| | protein (HHIP), mRNA | | |
| NM_006072 | Homo sapiens chemokine (C-C motif) | 25.57 Down | 5.83E-05 |
| | ligand 26 (CCL26), mRNA | | |
| NM_007361 | Homo sapiens nidogen 2 (osteonidogen) | 22.81 Down | 5.24E-05 |
| | (NID2), mRNA | | |
| NM_006228 | Homo sapiens prepronociceptin (PNOC), | 21.34 Down | 4.20E-04 |
| | mRNA | | |
| N95448 | zb81e11.s1 | 20.92 Down | 1.24E-04 |
| | Soares_senescent_fibroblasts_NbHSF | | |
| | Homo sapiens cDNA clone | | |
| | IMAGE:310028 3, mRNA sequence | | |
| NM_018242 | Homo sapiens hypothetical protein | 20.83 Down | 6.60E-05 |
| | FLJ10847 (FLJ10847), mRNA | | |
| N63415 | yy60d04.s1 | 19.2 Down | 5.24E-05 |
| | Soares_multiple-sclerosis_2NbHMSP | | |
| | Homo sapiens cDNA clone | | |
| | IMAGE:277927 3 similar to contains | | |
| | L1.b3 L1 repetitive element ;, mRNA | | |
| | sequence | | |
| BQ001571 | UI-H-DH1-awr-i-18-0-UI:s1 | 18.75 Down | 6.98E-05 |
| | NCI_CGAP_DH1 Homo sapiens cDNA | | |
| | clone IMAGE:5893337 3, mRNA | | |
| | sequence | | |
| NM_022454 | Homo sapiens SRY (sex determining | 18.21 Down | 5.83E-05 |
| | region Y)-box 17 (SOX17), mRNA | | |
| BE788763 | 601475864F1 NIH_MGC_68 Homo | 17.84 Down | 1.13E-04 |
| | sapiens cDNA clone IMAGE:3879014 5, | | |
| | m RNA sequence | | |
| BU633163 | UI-H-FL1-bgt-n-07-0-UI.s1 | 17.41 Down | 7.14E-05 |
| | NCI_CGAP_FL1 Homo sapiens cDNA | | |
| | clone UI-H-FL1-bgt-n-07-0-UI 3, mRNA | | |
| | sequence | | |
| AI289329 | qw28c09.x1 NCI_CGAP_Ut4 Homo | 17.1 Down | 6.98E-05 |
| | sapiens cDNA clone IMAGE:1992400 3 | | |
| | similar to contains L1.b2 L1 repetitive | | |
| | element;, ;, mRNA sequence | | |
| AF318382 | Homo sapiens pp9974 mRNA, complete | 16.45 Down | 1.38E-04 |
| | cds | | |
| NM_022350 | Homo sapiens leukocyte-derived | 15.8 Down | 1.10E-04 |
| | arginine aminopeptidase (LRAP), mRNA | | |
| BI561641 | 603256058F1 NIH_MGC_97 Homo | 14.77 Down | 1.94E-04 |
| | sapiens cDNA clone IMAGE:5298374 5, | | |
| | mRNA sequence | | |
| NM_006350 | Homo sapiens follistatin (FST), transcript | 14.55 Down | 7.14E-05 |
| | variant FST317, mRNA | | |
| NM_016588 | Homo sapiens neuritin 1 (NRN1), mRNA | 14.52 Down | 6.98E-05 |
| CA437861 | UI-H-DHO-aur-k-12-0-UI.s1 | 14.45 Down | 1.21 E-04 |
| | NCI_CGAP_DHO Homo sapiens cDNA | | |
| | clone UI-H-DHO-aur-k-12-0-UI 3, mRNA | | |
| | sequence | | |
| NM_020873 | Homo sapiens leucine rich repeat | 13.74 Down | 6.60E-05 |
| | neuronal 1 (LRRN1), mRNA | | |
| NM_003385 | Homo sapiens visinin-like 1 (VSNL1), | 13.06 Down | 7.75E-05 |
| | mRNA | | |
| AI640484 | wa27f01.x1 NCI_CGAP_Kid11 Homo | 11.92 Down | 5.48E-04 |
| | sapiens cDNA clone IMAGE:2299321 3, | | |
| | mRNA sequence | | |
| AK127309 | Homo sapiens cDNA FLJ45377 fis, clone | 11.87 Down | 2.35E-04 |
| | BRHIP3019956 | | |
| M60502 | Human profilaggrin mRNA, 3 end | 11.74 Down | 7.58E-05 |
| NM_006475 | Homo sapiens periostin, osteoblast | 11.74 Down | 1.19E-04 |
| | specific factor (POSTN), mRNA | | |
| NM_207482 | Homo sapiens FLJ44048 protein | 11.63 Down | 1.45E-04 |
| | (FLJ44048), mRNA | | |
| NM_000519 | Homo sapiens hemoglobin, delta (HBD), | 11.61 Down | 3.27E-04 |
| | mRNA | | |
| BU567804 | AGENCOURT_10398872 NIH_MGC_82 | 11.59 Down | 5.24E-05 |
| | Homo sapiens cDNA clone | | |
| | IMAGE:6614502 5, mRNA sequence | | |
| AJ318805 | AJ318805 Homo sapiens adipose tissue | 11.59 Down | 1.76E-04 |
| | Homo sapiens cDNA clone 2040, mRNA | | |
| | sequence | | |
| AW451831 | UI-H-BI3-alk-e-12-0-UI.s1 | 11.57 Down | 1.46E-04 |
| | NCI_CGAP_Sub5 Homo sapiens cDNA | | |
| | clone IMAGE:2737246 3, mRNA | | |
| | sequence | | |
| R99527 | yq79b11.s1 Soares fetal liver spleen | 11.16 Down | 1.13E-04 |
| | 1 NFLS Homo sapiens cDNA clone | | |
| | IMAGE:201981 3, mRNA sequence | | |
| NM_031894 | Homo sapiens ferritin, heavy | 11.12 Down | 4.85E-04 |
| | polypeptide-like 17 (FTHL17), mRNA | | |
| NM_007072 | Homo sapiens HERV-H LTR-associating | 10.94 Down | 1.36E-04 |
| | 2 (HHLA2), mRNA | | |
| NM_005654 | Homo sapiens nuclear receptor | 10.91 Down | 3.32E-05 |
| | subfamily 2, group F, member 1 | | |
| | (NR2F1), mRNA | | |
| NM_004574 | Homo sapiens peanut-like 2 (Drosophila) | 10.46 Down | 3.48E-04 |
| | (PNUTL2), transcript variant 1, mRNA | | |
| NM_005141 | Homo sapiens fibrinogen, B beta | 10.37 Down | 4.85E-04 |
| | polypeptide (FGB), mRNA | | |
| AI905628 | CM-BT094-050299-147 BT094 Homo | 10.36 Down | 1.49E-04 |
| | sapiens cDNA, mRNA sequence | | |
| BG622707 | 602647476F1 NIH_MGC_79 Homo | 10.35 Down | 1.73E-04 |
| | sapiens cDNA clone IMAGE:4768963 5, | | |
| | mRNA sequence | | |
| W76003 | zd58g07.r1 | 10.32 Down | 1.58E-04 |
| | Soares_fetal_neart_NbHH19W Homo | | |
| | sapiens cDNA clone IMAGE:344892 5, | | |
| | mRNA sequence | | |
| H15096 | ym29e11.r1 Soares infant brain 1NIB | 10.23 Down | 1.80E-04 |
| | Homo sapiens cDNA clone | | |
| | IMAGE:49250 5, mRNA sequence | | |
| A1493349 | tg70f04.x1 Soares_NhHMPu_S1 Homo | 9.84 Down | 7.54E-05 |
| | sapiens cDNA clone IMAGE:2114143 3, | | |
| | mRNA sequence | | |
| AK093762 | Homo sapiens cDNA FLJ36443 fis, clone | 9.76 Down | 8.07E-04 |
| | THYMU2012891 | | |
| BU570253 | AGENCOURT_10401698 NIH_MGC_82 | 9.71 Down | 3.65E-04 |
| | Homo sapiens cDNA clone | | |
| | IMAGE:6618451 5, mRNA sequence | | |
| NM_005855 | Homo sapiens receptor (calcitonin) | 9.33 Down | 7.08E-05 |
| | activity modifying protein 1 (RAMP1), | | |
| | mRNA | | |
| NM_003328 | Homo sapiens TXK tyrosine kinase | 9.29 Down | 1.19E-04 |
| | (TXK), mRNA | | |
| NM_003004 | Homo sapiens secreted and | 9.23 Down | 1.76E-04 |
| | transmembrane 1 (SECTM1), mRNA | | |
| NM_005596 | Homo sapiens nuclear factor I/B (NFIB), | 9.23 Down | 4.97E-05 |
| | mRNA | | |
| BC039450 | Homo sapiens, clone IMAGE:5311619, | 9.07 Down | 1.13E-04 |
| | mRNA | | |
| NM_005613 | Homo sapiens regulator of G-protein | 8.99 Down | 7.65E-05 |
| | signalling 4 (RGS4), mRNA | | |
| NM_003973 | Homo sapiens ribosomal protein L14 | 8.98 Down | 6.60E-05 |
| | (RPL14), mRNA | | |
| BM675270 | UI-E-EJO-ahr-j-07-0-UI.s1 UI-E-EJO | 8.96 Down | 1.34E-03 |
| | Homo sapiens cDNA clone UI-E-EJO- | | |
| | ahr-j-07-0-UI 3, mRNA sequence | | |
| NM_001311 | Homo sapiens cysteine-rich protein 1 | 8.91 Down | 7.14E-05 |
| | (intestinal) (CRIP1), mRNA | | |
| NM_000087 | Homo sapiens cyclic nucleotide gated | 8.76 Down | 1.33E-03 |
| | channel alpha 1 (CNGA1), mRNA | | |
| AW021686 | df26h11.y1 Morton Fetal Cochlea Homo | 8.74 Down | 4.04E-05 |
| | sapiens cDNA clone IMAGE:2484717 5, | | |
| | mRNA sequence | | |
| NM_002031 | Homo sapiens fyn-related kinase (FRK), | 8.67 Down | 1.14E-04 |
| | mRNA | | |
| NM_005279 | Homo sapiens G protein-coupled | 8.51 Down | 4.16E-04 |
| | receptor 1 (GPR1), mRNA | | |
| NM_004460 | Homo sapiens fibroblast activation | 8.51 Down | 7.71 E-04 |
| | protein, alpha (FAP), mRNA | | |
| BE904671 | 601498784F1 NIH_MGC_70 Homo | 8.46 Down | 1.73E-04 |
| | sapiens cDNA clone IMAGE:3900717 5, | | |
| | mRNA sequence | | |
| NM_000612 | Homo sapiens insulin-like growth factor 2 | 8.27 Down | 1.19E-04 |
| | (somatomedin A) (IGF2), mRNA | | |
| AL137488 | Homo sapiens mRNA; cDNA | 8.23 Down | 1.13E-04 |
| | DKFZp434N2030 (from clone | | |
| | DKFZp434N2030) | | |
| NM_006288 | Homo sapiens Thy-1 cell surface antigen | 8.18 Down | 1.43E-03 |
| | (THY1), mRNA | | |
| NM_002837 | Homo sapiens protein tyrosine | 8.15 Down | 1.19E-03 |
| | phosphatase, receptor type, B (PTPRB), | | |
| | mRNA | | |
| NM_030899 | Homo sapiens zinc finger protein 323 | 8.08 Down | 9.54E-05 |
| | (ZNF323), mRNA | | |
| NM_002193 | Homo sapiens inhibin, beta B (activin AB | 8.07 Down | 1.90E-04 |
| | beta polypeptide) (INHBB), mRNA | | |
| BX537698 | Homo sapiens mRNA; cDNA | 8.06 Down | 1.34E-03 |
| | DKFZp686F09166 (from clone | | |
| | DKFZp686F09166) | | |
| BC033567 | Homo sapiens, clone IMAGE:4822266, | 8.04 Down | 1.88E-04 |
| | mRNA | | |
| NM_052997 | Homo sapiens ankyrin repeat domain | 7.97 Down | 1.42E-03 |
| | 30A (ANKRD30A), mRNA | | |
| NM_178550 | Homo sapiens hypothetical protein | 7.82 Down | 5.76E-04 |
| | MGC48998 (MGC48998), mRNA | | |
| NM_178033 | Homo sapiens cytochrome P450, family | 7.81 Down | 8.00E-04 |
| | 4, subfamily X, polypeptide 1 (CYP4X1), | | |
| | mRNA | | |
| NM_002924 | Homo sapiens regulator of G-protein | 7.8 Down | 5.38E-04 |
| | signalling 7 (RGS7), mRNA | | |
| NM_012242 | Homo sapiens dickkopf homolog 1 | 7.77 Down | 1.24E-04 |
| | (Xenopus laevis) (DKK1), mRNA | | |
| AA740671 | ob01h05.s1 NCI_CGAP_Kid3 Homo | 7.74 Down | 2.22E-03 |
| | sapiens cDNA clone IMAGE:1322457 3, | | |
| | mRNA sequence | | |
| NM_005712 | Homo sapiens HERV-H LTR-associating | 7.67 Down | 1.80E-04 |
| | 1 (HHLA1), mRNA | | |
| BC040293 | Homo sapiens, clone IMAGE:4820330, | 7.65 Down | 3.10E-04 |
| | mRNA | | |
| NM_000089 | Homo sapiens collagen, type I, alpha 2 | 7.64 Down | 1.17E-03 |
| | (COL1A2), mRNA | | |
| NM_005654 | Homo sapiens nuclear receptor | 7.63 Down | 2.73E-04 |
| | subfamily 2, group F, member 1 | | |
| | (NR2F1), mRNA | | |
| BE465134 | hv75e11.x1 NCI_CGAP_Lu24 Homo | 7.53 Down | 1.28E-03 |
| | sapiens cDNA clone IMAGE:3179276 3, | | |
| | mRNA sequence | | |
| NM_002522 | Homo sapiens neuronal pentraxin I | 7.49 Down | 6.53E-04 |
| | (NPTX1), mRNA | | |
| AK093529 | Homo sapiens cDNA FLJ36210 fis, clone | 7.47 Down | 1.18E-04 |
| | THYMU2000155 | | |
| NM_003662 | Homo sapiens pirin (iron-binding nuclear | 7.29 Down | 6.96E-05 |
| | protein) (PIR), mRNA | | |
| NM_001769 | Homo sapiens CD9 antigen (p24) (CD9), | 7.23 Down | 1.80E-04 |
| | mRNA | | |
| AL359567 | Homo sapiens mRNA; cDNA | 7.21 Down | 1.24E-04 |
| | DKFZp547D023 (from clone | | |
| | DKFZp547D023) | | |
| BX647313 | Homo sapiens mRNA; cDNA | 7.19 Down | 1.19E-04 |
| | DKFZp686N1593 (from clone | | |
| | DKFZp686N1593) | | |
| NM_003979 | Homo sapiens G protein-coupled | 7.18 Down | 1.63E-03 |
| | receptor, family C, group 5, member A | | |
| | (GPCR5A), mRNA | | |
| AI365141 | qx96e07.x1 NCI_CGAP_GC6 Homo | 7.04 Down | 3.01 E-04 |
| | sapiens cDNA clone IMAGE:2010372 3, | | |
| | mRNA sequence | | |
| CA948963 | iq30f05.y1 HR85 islet Homo sapiens | 7.03 Down | 8.55E-04 |
| | cDNA clone IMAGE: 5, mRNA sequence | | |
| NM_002977 | Homo sapiens sodium channel, voltage- | 6.99 Down | 5.83E-05 |
| | gated, type IX, alpha (SCN9A), mRNA | | |
| BC033124 | Homo sapiens, clone IMAGE:2960615, | 6.86 Down | 6.60E-05 |
| | mRNA | | |
| NM_003385 | Homo sapiens visinin-like 1 (VSNL1), | 6.73 Down | 1.26E-04 |
| | mRNA | | |
| NM_013262 | Homo sapiens myosin regulatory light | 6.65 Down | 1.66E-04 |
| | chain interacting protein (MYLIP), mRNA | | |
| NM_001843 | Homo sapiens contactin 1 (CNTN1), | 6.64 Down | 3.37E-03 |
| | transcript variant 1, mRNA | | |
| NM_006727 | Homo sapiens cadherin 10, type 2 (T2- | 6.59 Down | 1.91 E-03 |
| | cadherin) (CDH10), mRNA | | |
| NM_016192 | Homo sapiens transmembrane protein | 6.58 Down | 9.19E-05 |
| | with EGF-like and two follistatin-like | | |
| | domains 2 (TMEFF2), mRNA | | |
| NM_031847 | Homo sapiens microtubule-associated | 6.57 Down | 1.13E-03 |
| | protein 2 (MAP2), transcript variant 4, | | |
| | mRNA | | |
| CB115754 | K-EST0159876 L8SCKO Homo sapiens | 6.55 Down | 8.55E-04 |
| | cDNA clone L8SCK0-8-H08 5, mRNA | | |
| | sequence | | |
| NM_025074 | Homo sapiens Fraser syndrome 1 | 6.52 Down | 4.97E-05 |
| | (FRAS1), transcript variant 1, mRNA | | |
| NM_016206 | Homo sapiens colon carcinoma related | 6.45 Down | 5.30E-04 |
| | protein (FLJ38507), mRNA | | |
| BC015159 | Homo sapiens cDNA clone | 6.35 Down | 3.01 E-04 |
| | IMAGE:3885734, partial cds | | |
| NM_005613 | Homo sapiens regulator of G-protein | 6.33 Down | 1.62E-03 |
| | signalling 4 (RGS4), mRNA | | |
| W88428 | zh72g09.s1 | 6.28 Down | 7.53E-04 |
| | Soares_fetal_liver_spleen_1NFLS_S1 | | |
| | Homo sapiens cDNA clone | | |
| | IMAGE:417664 3, mRNA sequence | | |
| NM_058187 | Homo sapiens chromosome 21 open | 6.16 Down | 5.39E-03 |
| | reading frame 63 (C21orf63), mRNA | | |
| NM_020836 | Homo sapiens brain-enriched guanylate | 6.15 Down | 3.75E-03 |
| | kinase-associated protein (KIAA1446), | | |
| | mRNA | | |
| BX107838 | BX107838 NCI_CGAP_Lu5 Homo | 6.14 Down | 1.77E-03 |
| | sapiens cDNA clone IMAGp998A153853 | | |
| | ; IMAGE:1521686, mRNA sequence | | |
| NM_004496 | Homo sapiens forkhead box A1 | 6.13 Down | 1.94E-04 |
| | (FOXA1), mRNA | | |
| NM_021637 | Homo sapiens transmembrane protein | 6.1 Down | 2.12E-03 |
| | 35 (TMEM35), mRNA | | |
| NM_002521 | Homo sapiens natriuretic peptide | 6.1 Down | 4.86E-04 |
| | precursor B (NPPB), mRNA | | |
| BF431041 | nab31g02.x1 | 6.08 Down | 7.63E-05 |
| | Soares_NSF_F8_9W_OT_PA_P_S1 | | |
| | Homo sapiens cDNA clone | | |
| | IMAGE:3267627 3, mRNA sequence | | |
| NM_003713 | Homo sapiens phosphatidic acid | 6.05 Down | 6.44E-04 |
| | phosphatase type 2B (PPAP2B), | | |
| | transcript variant 1, mRNA | | |
| AV728294 | AV728294 HTC Homo sapiens cDNA | 6.03 Down | 7.71E-05 |
| | clone HTCBIE09 5, mRNA sequence | | |
| NM_002148 | Homo sapiens homeo box D10 | 6.01 Down | 7.14E-05 |
| | (HOXD10), mRNA | | |
| NM_176891 | Homo sapiens interferon epsilon 1 | 6 Down | 7.48E-04 |
| | (IFNE1), mRNA | | |
| NM_002317 | Homo sapiens lysyl oxidase (LOX), | 5.99 Down | 3.17E-04 |
| | mRNA | | |
| NM_016179 | Homo sapiens transient receptor | 5.99 Down | 9.19E-05 |
| | potential cation channel, subfamily C, | | |
| | member 4 (TRPC4), mRNA | | |
| AW134473 | UI-H-BI1-abv-a-11-0-UI.s1 | 5.98 Down | 7.04E-05 |
| | NCI_CGAP_Sub3 Homo sapiens cDNA | | |
| | clone IMAGE:2712885 3, mRNA | | |
| | sequence | | |
| NM_005130 | Homo sapiens fibroblast growth factor | 5.95 Down | 5.89E-04 |
| | binding protein 1 (FGFBP1), mRNA | | |
| AW512111 | xx70e05.x1 NCI_CGAP_Lym12 Homo | 5.93 Down | 4.97E-05 |
| | sapiens cDNA clone IMAGE:2849024 3 | | |
| | similar to contains Alu repetitive | | |
| | element; mRNA sequence | | |
| NM_020349 | Homo sapiens ankyrin repeat domain 2 | 5.91 Down | 1.18E-04 |
| | (stretch responsive muscle) (ANKRD2), | | |
| | mRNA | | |
| NM_001083 | Homo sapiens phosphodiesterase 5A, | 5.88 Down | 3.74E-03 |
| | cGMP-specific (PDE5A), transcript | | |
| | variant 1, mRNA | | |
| H94320 | yv18b10.s1 Soares fetal liver spleen | 5.83 Down | 3.18E-02 |
| | 1NFLS Homo sapiens cDNA clone | | |
| | IMAGE:243067 3, mRNA sequence | | |
| NM_032461 | Homo sapiens SPANX family, member | 5.8 Down | 1.08E-03 |
| | B1 (SPANXB1), mRNA | | |
| BM994473 | UI-H-DH0-aul-I-18-0-UI.s1 | 5.79 Down | 1.10E-04 |
| | NCI_CGAP_DHO Homo sapiens cDNA | | |
| | clone IMAGE:5871137 3, mRNA | | |
| | sequence | | |
| BE540906 | 601063027F1 NIH_MGC_10 Homo | 5.79 Down | 4.45E-05 |
| | sapiens cDNA clone IMAGE:3449455 5, | | |
| | mRNA sequence | | |
| NM_033255 | Homo sapiens epithelial stromal | 5.75 Down | 8.55E-04 |
| | interaction 1 (breast) (EPSTI1), mRNA | | |
| BU616268 | UI-H-DF0-bex-n-12-0-UI.s1 | 5.73 Down | 6.74E-04 |
| | NCI_CGAP_DF0 Homo sapiens cDNA | | |
| | clone UI-H-DF0-bex-n-12-0-UI 3, mRNA | | |
| | sequence | | |
| BX112170 | BX112170 | 5.73 Down | 7.32E-04 |
| | Soares_fetal_heart_NbHH19W Homo | | |
| | sapiens cDNA clone IMAGp998007742 ; | | |
| | IMAGE:327390, mRNA sequence | | |
| BC015108 | Homo sapiens, Similar to otoconin 90, | 5.72 Down | 2.40E-03 |
| | clone IMAGE:4044247, mRNA | | |
| NM_000576 | Homo sapiens interleukin 1, beta (IL 1B), | 5.7 Down | 2.97E-03 |
| | mRNA | | |
| AW470868 | ha34h03.x1 NCI_CGAP_Kid12 Homo | 5.7 Down | 2.31E-03 |
| | sapiens cDNA clone IMAGE:2875637 3, | | |
| | mRNA sequence | | |
| AK093069 | Homo sapiens cDNA FLJ35750 fis, clone | 5.7 Down | 1.11 E-03 |
| | TESTI2004539, weakly similar to Homo | | |
| | sapiens adlican mRNA | | |
| NM_032330 | Homo sapiens calpain, small subunit 2 | 5.68 Down | 4.44E-03 |
| | (CAPNS2), mRNA | | |
| U51694 | HSU51694 Human normal gingiva Homo | 5.62 Down | 1.02E-04 |
| | sapiens cDNA, mRNA sequence | | |
| NM_004335 | Homo sapiens bone marrow stromal cell | 5.61 Down | 2.84E-03 |
| | antigen 2 (BST2), mRNA | | |
| BF431460 | 7o14b05.x1 NCI_CGAP_Kid11 Homo | 5.59 Down | 7.53E-04 |
| | sapiens cDNA clone IMAGE:3573849 3, | | |
| | mRNA sequence | | |
| BC046362 | Homo sapiens voltage-dependent | 5.59 Down | 8.07E-04 |
| | calcium channel gamma subunit-like | | |
| | protein, mRNA (cDNA clone MGC:50757 | | |
| | IMAGE:5221396), complete cds | | |
| NM_000961 | Homo sapiens prostaglandin 12 | 5.57 Down | 1.01 E-03 |
| | (prostacyclin) synthase (PTGIS), mRNA | | |
| AK022877 | Homo sapiens cDNA FLJ12815 fis, clone | 5.56 Down | 4.04E-05 |
| | NT2RP2002546 | | |
| NM_002277 | Homo sapiens keratin, hair, acidic, 1 | 5.51 Down | 3.04E-04 |
| | (KRTHA1), mRNA | | |
| AK092245 | Homo sapiens cDNA FLJ34926 fis, clone | 5.49 Down | 1.40E-03 |
| | NT2RP7003319, highly similar to Mus | | |
| | musculus neuralin mRNA | | |
| NM_002427 | Homo sapiens matrix metalloproteinase | 5.47 Down | 4.45E-04 |
| | 13 (collagenase 3) (MMP13), mRNA | | |
| BM991890 | UI-H-DF1-auk-h-02-0-UI.s1 | 5.39 Down | 7.53E-04 |
| | NCI_CGAP_DF1 Homo sapiens cDNA | | |
| | clone IMAGE:5870641 3, mRNA | | |
| | sequence | | |
| NM_002575 | Homo sapiens serine (or cysteine) | 5.39 Down | 9.60E-03 |
| | proteinase inhibitor, clade B (ovalbumin), | | |
| | member 2 (SERPINB2), mRNA | | |
| NM_000782 | Homo sapiens cytochrome P450, family | 5.36 Down | 1.31 E-02 |
| | 24, subfamily A, polypeptide 1 | | |
| | (CYP24A1), nuclear gene encoding | | |
| | mitochondrial protein, mRNA | | |
| NM_016276 | Homo sapiens serum/glucocorticoid | 5.34 Down | 6.73E-04 |
| | regulated kinase 2 (SGK2), transcript | | |
| | variant 2, m RNA | | |
| AK024689 | Homo sapiens cDNA: FLJ21036 fis, | 5.32 Down | 1.36E-04 |
| | clone CAE09578 | | |
| N78829 | zb17a05.s1 | 5.31 Down | 6.00E-03 |
| | Soares_fetal_lung_NbHL19W Homo | | |
| | sapiens cDNA clone IMAGE:302288 3, | | |
| | mRNA sequence | | |
| AL080103 | Homo sapiens mRNA; cDNA | 5.26 Down | 1.61 E-04 |
| | DKFZp564N2216 (from clone | | |
| | DKFZp564N2216) | | |
| BC035400 | Homo sapiens, clone IMAGE:4822830, | 5.17 Down | 1.13E-02 |
| | mRNA | | |
| AK056725 | Homo sapiens cDNA FLJ32163 fis, clone | 5.13 Down | 3.72E-04 |
| | PLACE6000371 | | |
| AK025595 | Homo sapiens cDNA: FLJ21942 fis, | 5.12 Down | 1.56E-02 |
| | clone HEP04527 | | |
| NM_015236 | Homo sapiens latrophilin 3 (LPHN3), | 5.11 Down | 3.14E-04 |
| | mRNA | | |
| NM_012411 | Homo sapiens protein tyrosine | 5.1 Down | 2.78E-03 |
| | phosphatase, non-receptor type 22 | | |
| | (lymphoid) (PTPN22), transcript variant | | |
| | 2, mRNA | | |
| AI190760 | qd61c05.x1 Soares_testis_NHT Homo | 5.08 Down | 2.24E-03 |
| | sapiens cDNA clone IMAGE:1733960 3, | | |
| | mRNA sequence | | |
| NM_139241 | Homo sapiens FYVE, RhoGEF and PH | 5.04 Down | 6.92E-03 |
| | domain containing 4 (FGD4), mRNA | | |
| NM_033066 | Homo sapiens membrane protein, | 5.04 Down | 7.11E-03 |
| | palmitoylated 4 (MAGUK p55 subfamily | | |
| | member 4) (MPP4), mRNA | | |
| BG573337 | 602595107F1 NIH_MGC_79 Homo | 5.03 Down | 5.60E-03 |
| | sapiens cDNA clone IMAGE:4724521 5, | | |
| | mRNA sequence | | |
| AA158235 | zo76b02.s1 Stratagene pancreas | 5.01 Down | 7.10E-03 |
| | (#937208) Homo sapiens cDNA clone | | |
| | IMAGE:592779 3, mRNA sequence | | |
| BX092004 | BX092004 NCI_CGAP_Kid11 Homo | 5.01 Down | 1.69E-02 |
| | sapiens cDNA clone IMAGp998B195924 | | |
| | ; IMAGE:2385330, mRNA sequence | | |
| AK058040 | Homo sapiens cDNA FLJ25311 fis, clone | 5 Down | 1.76E-03 |
| | SYN01066 | | |

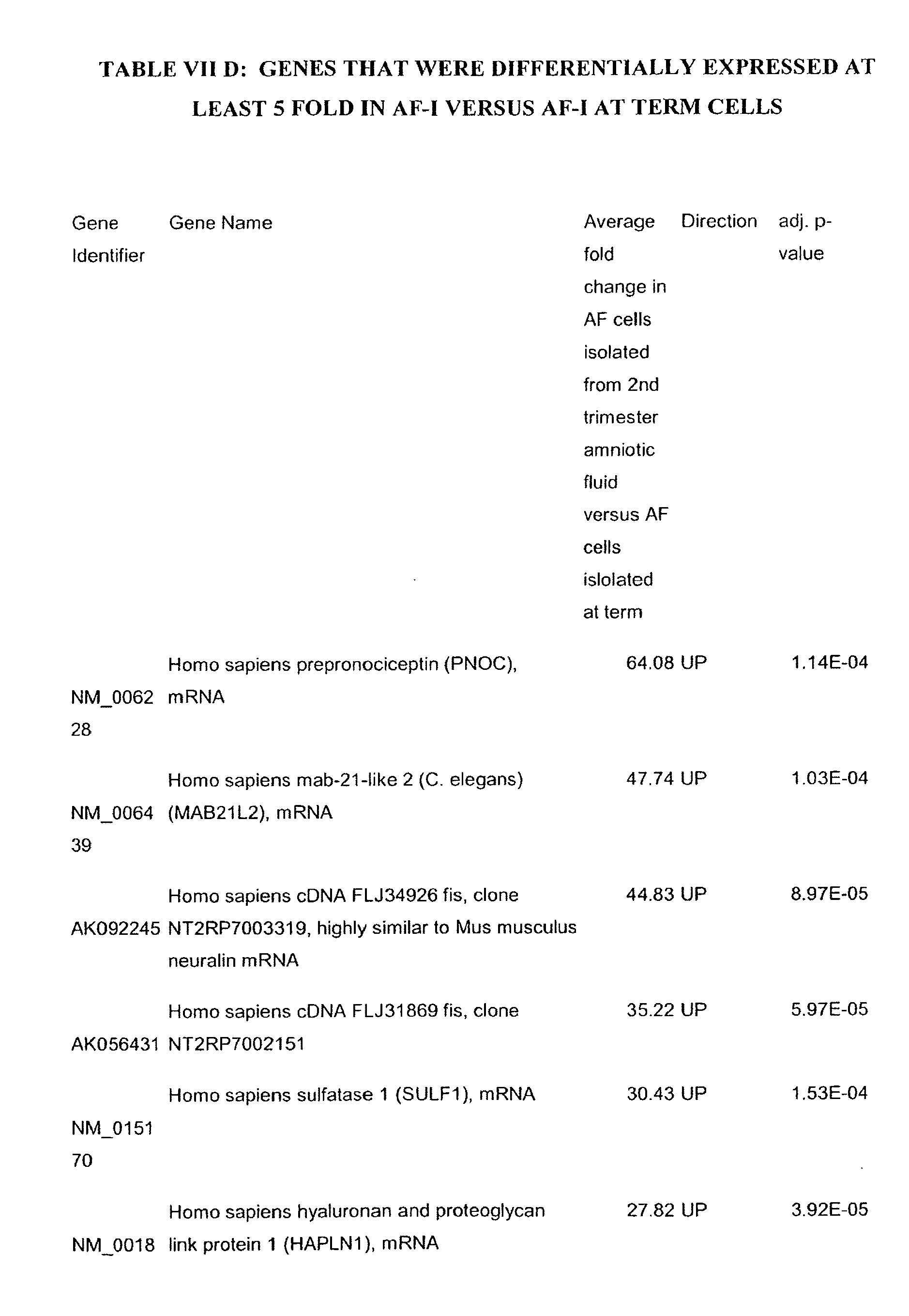

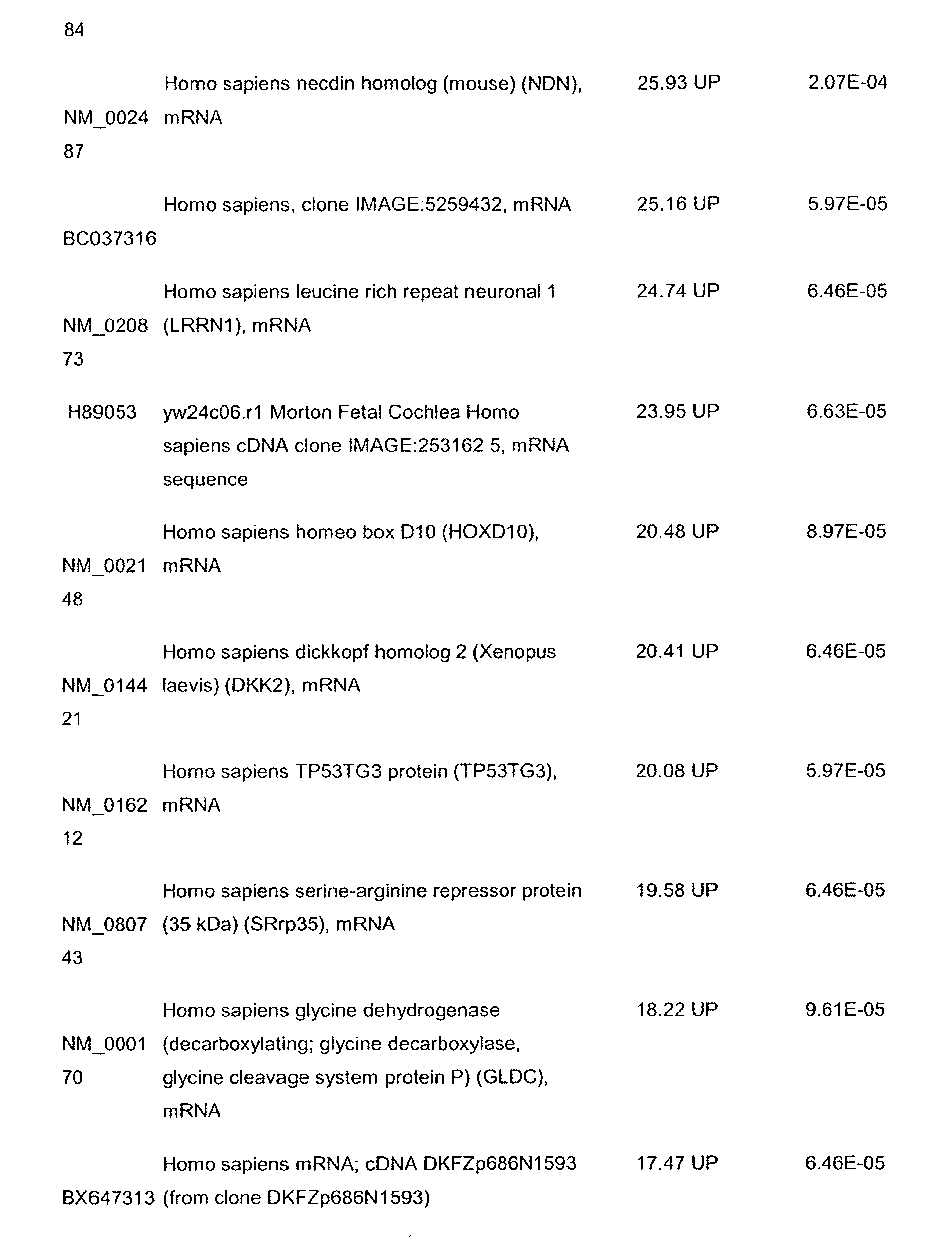

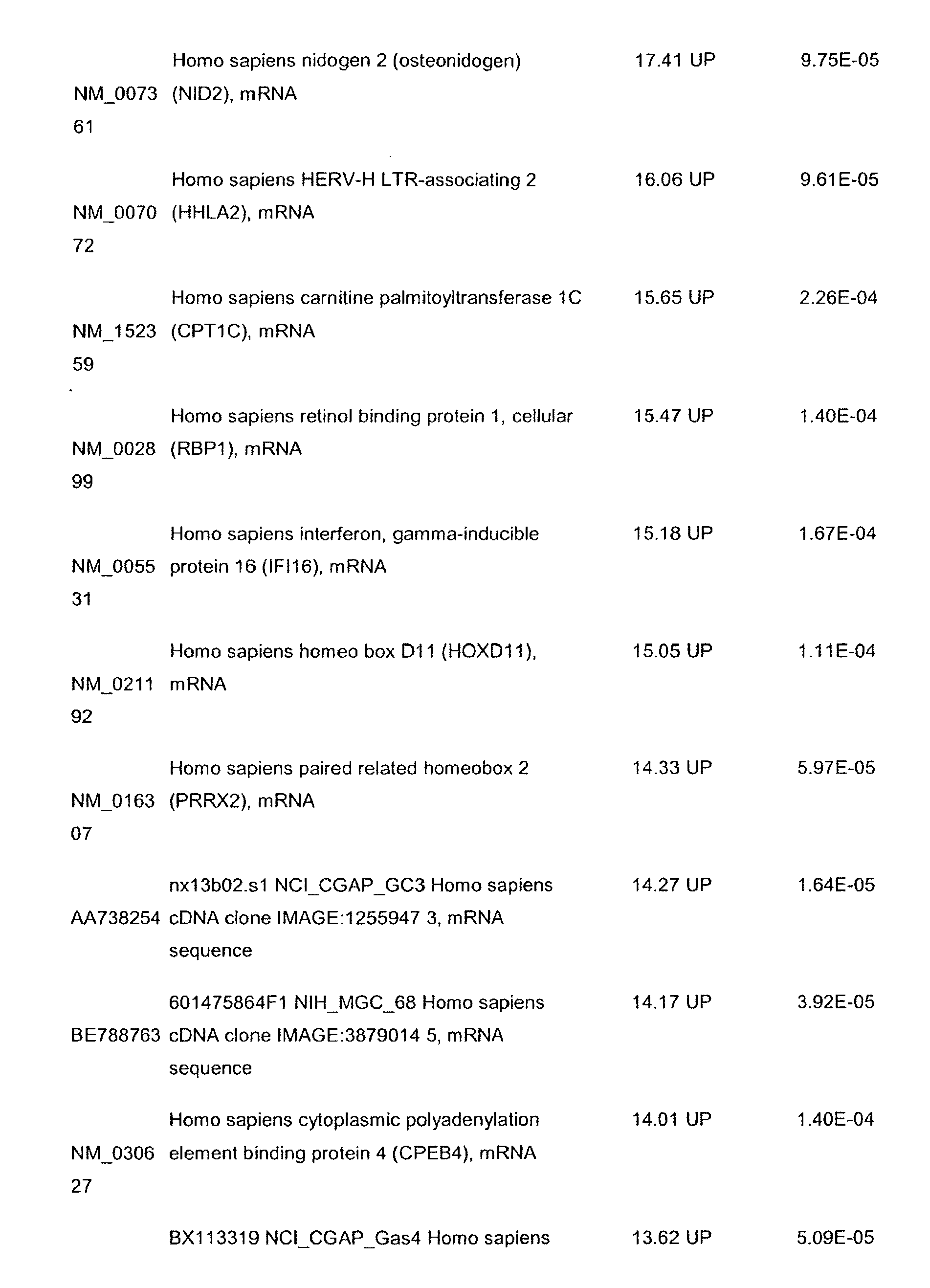

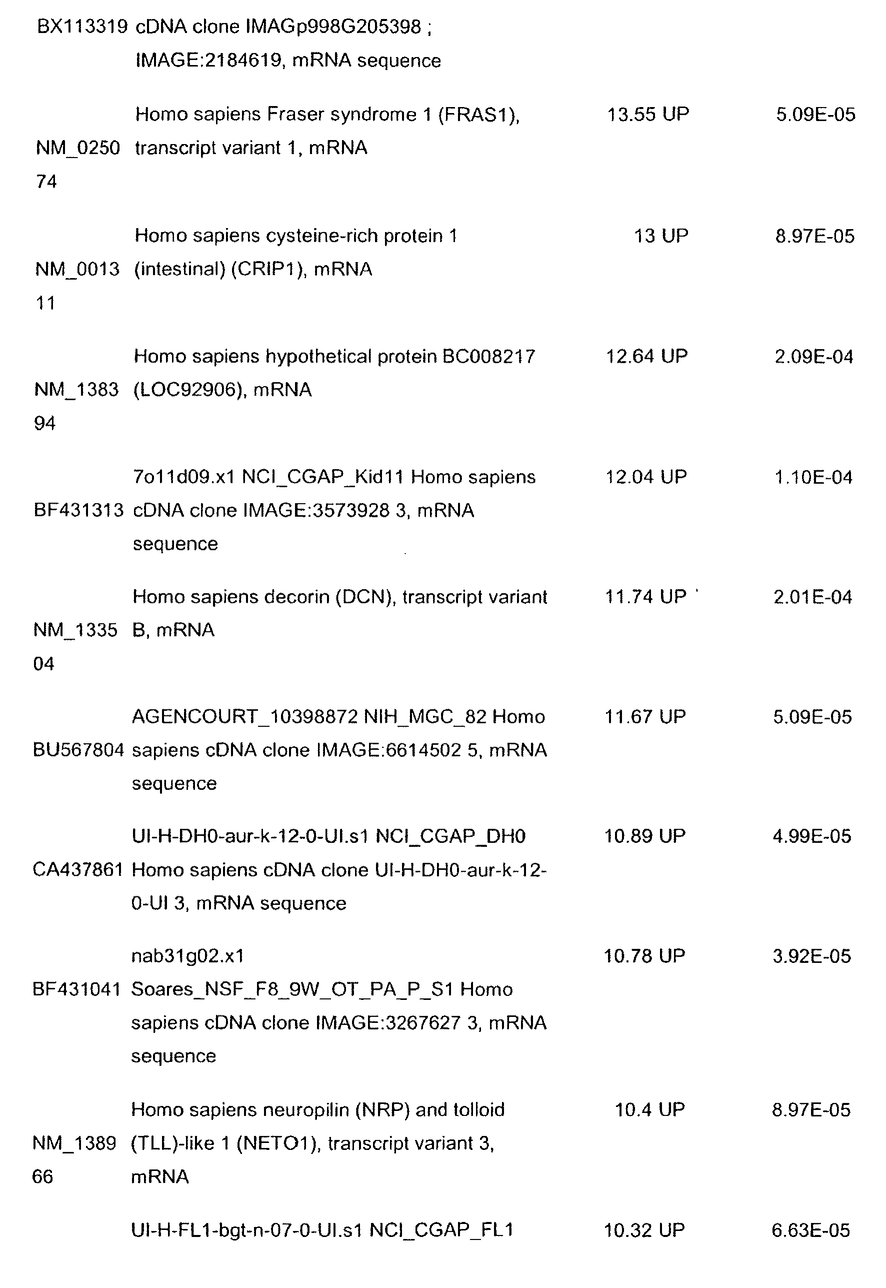

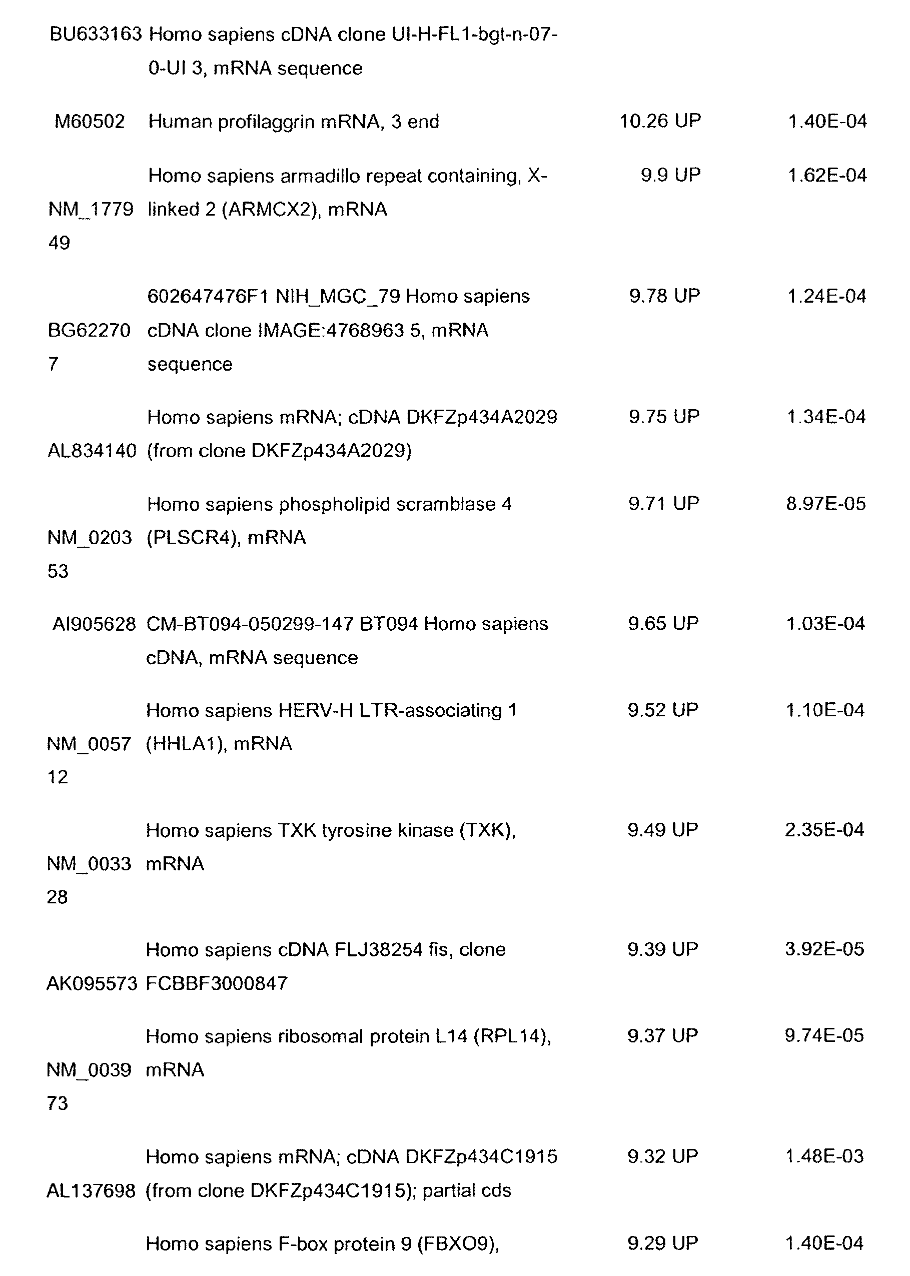

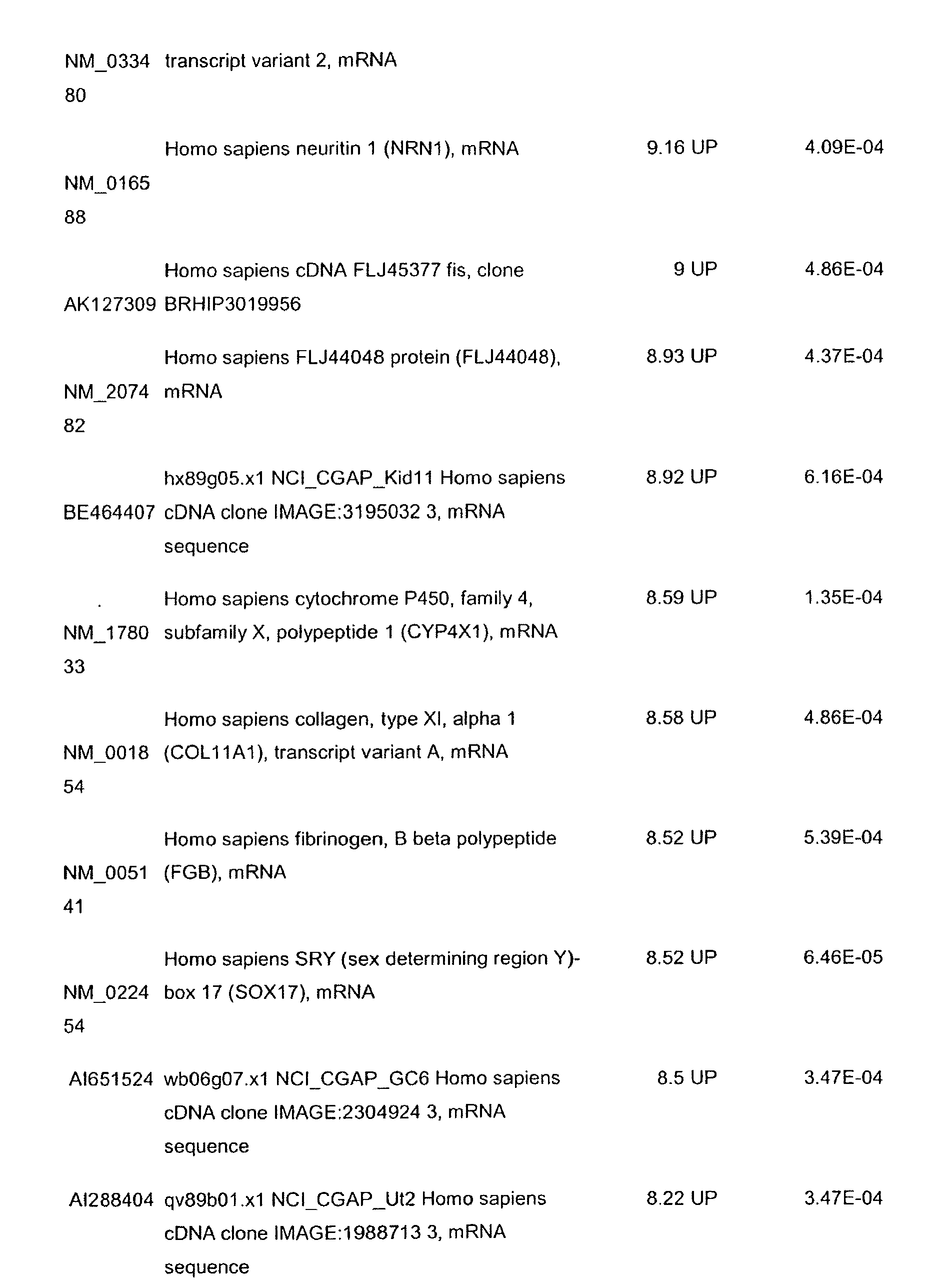

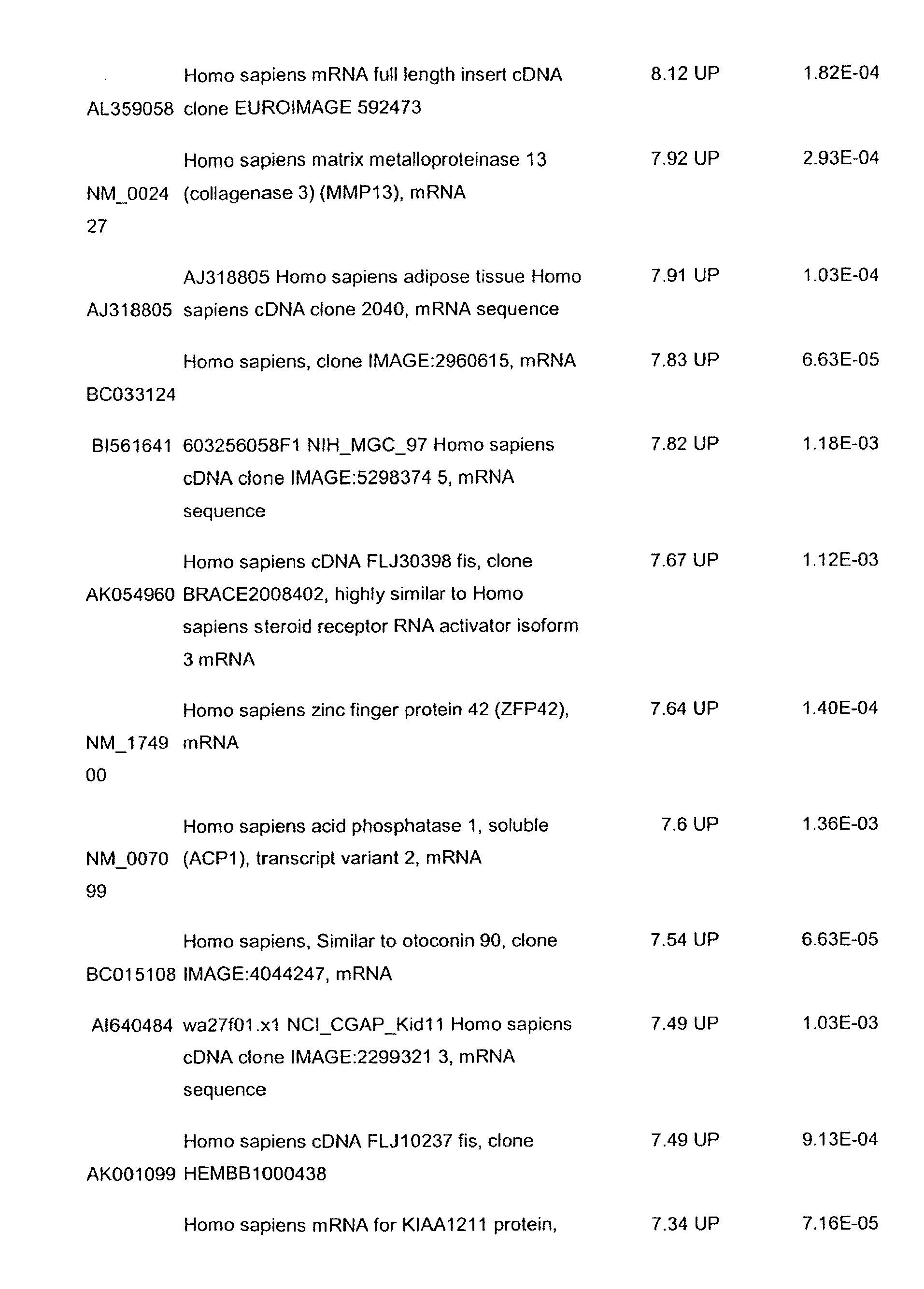

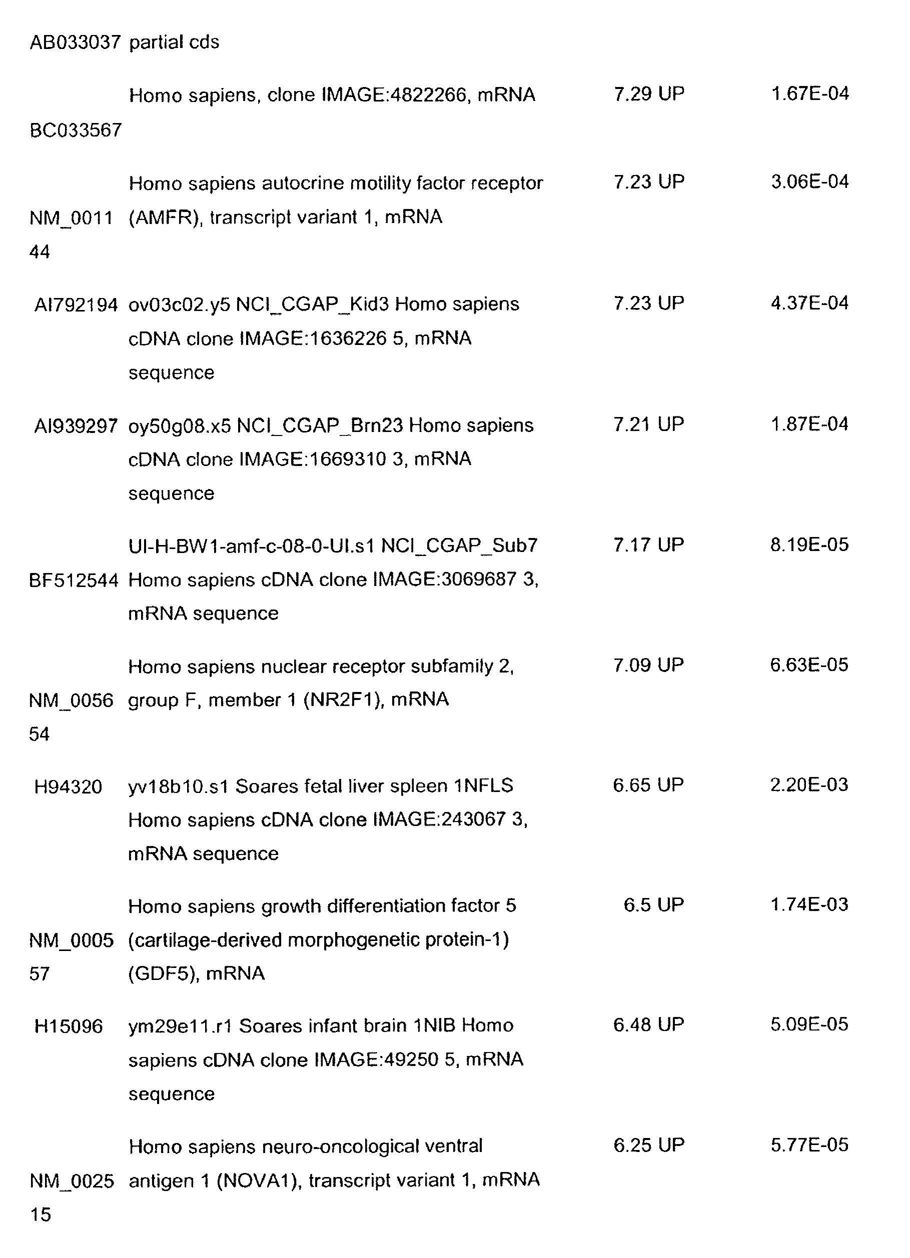

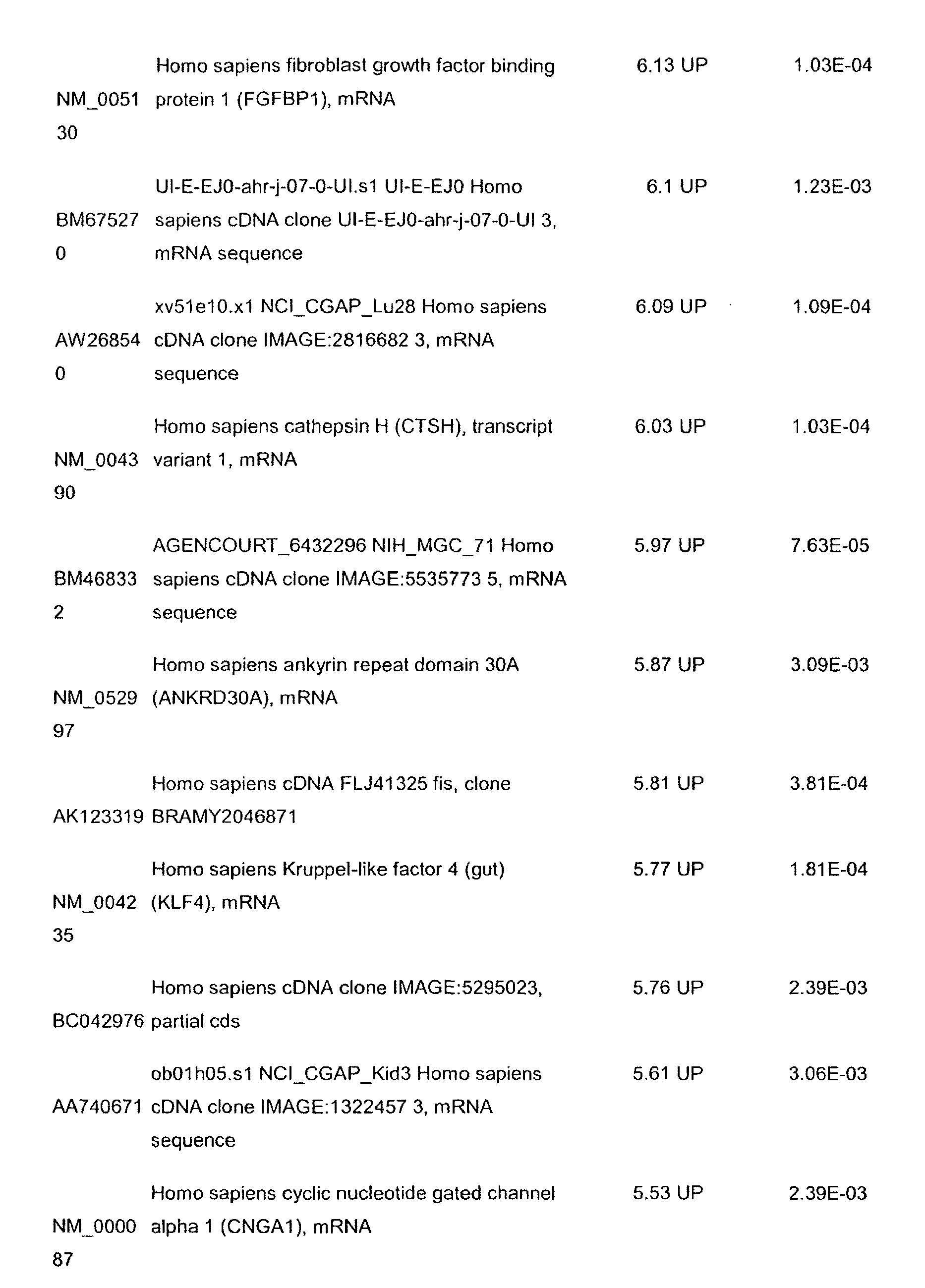

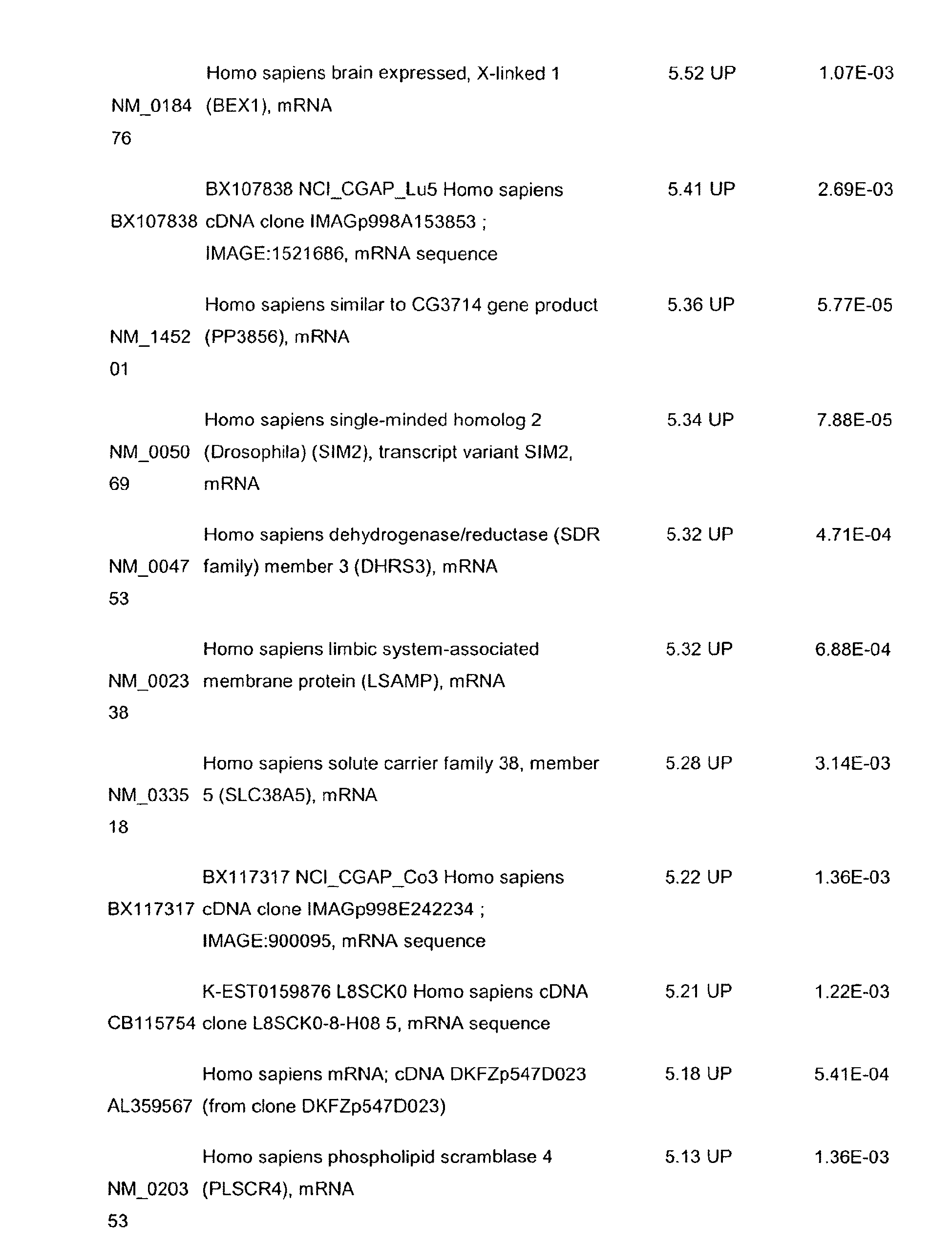

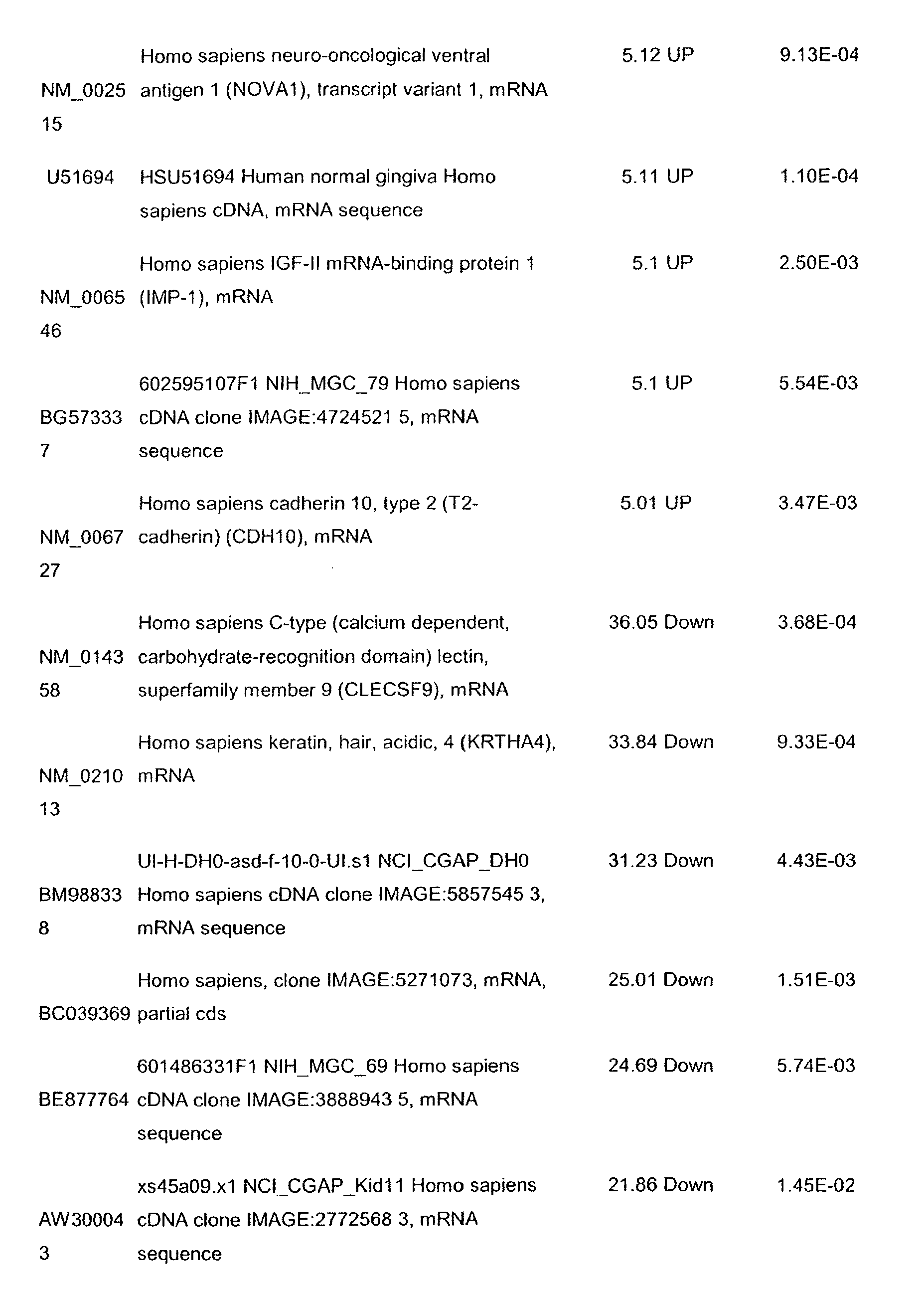

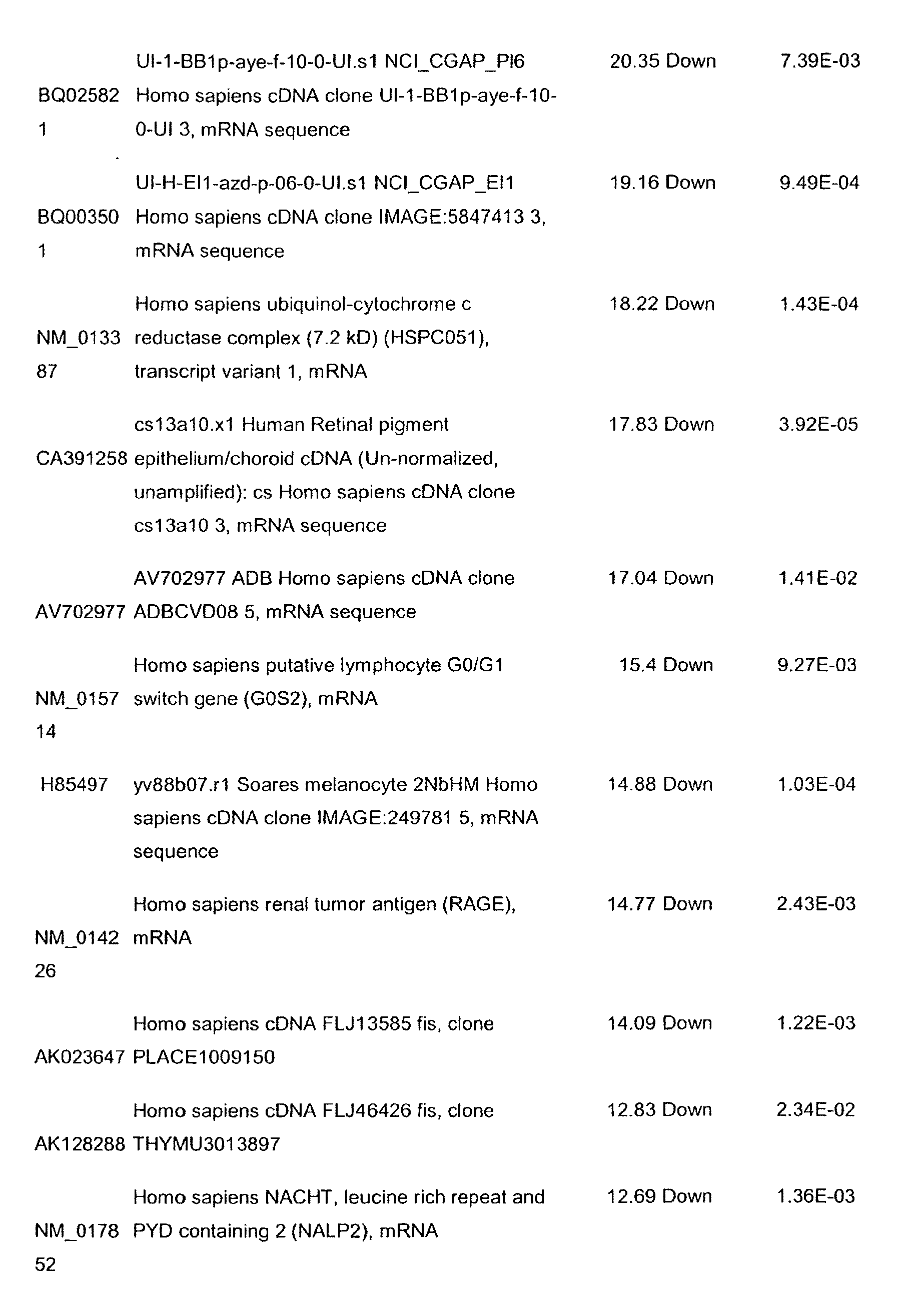

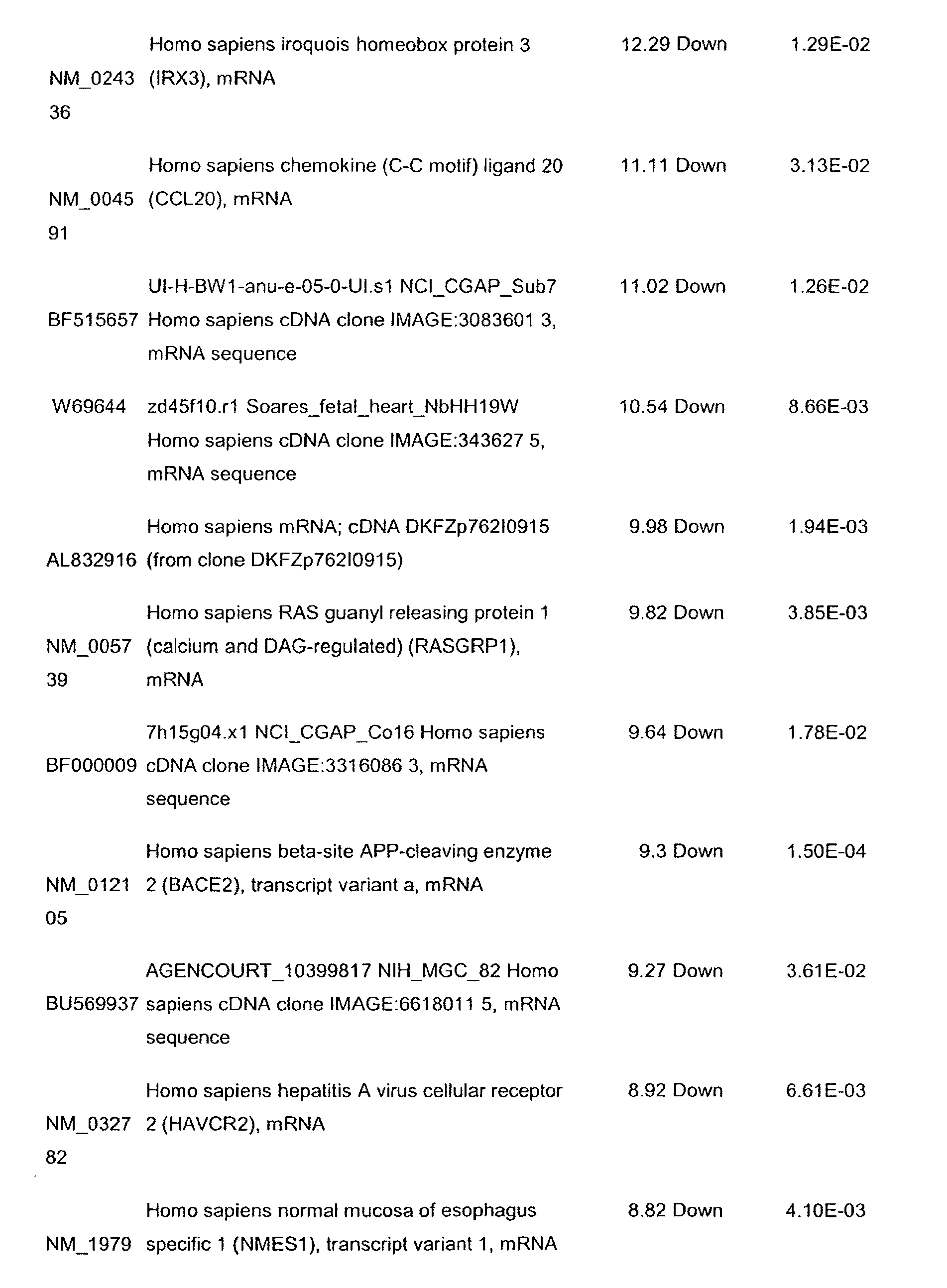

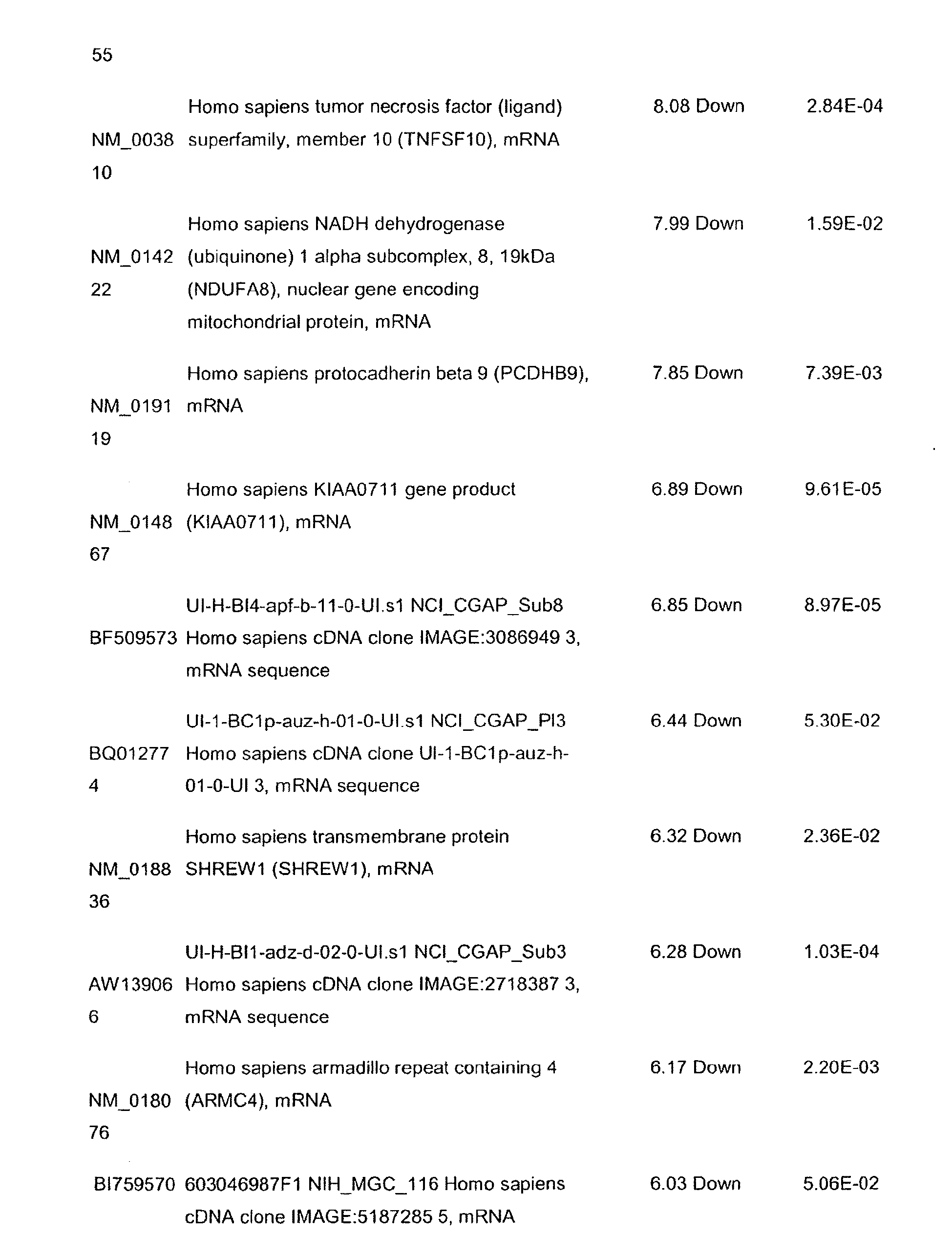

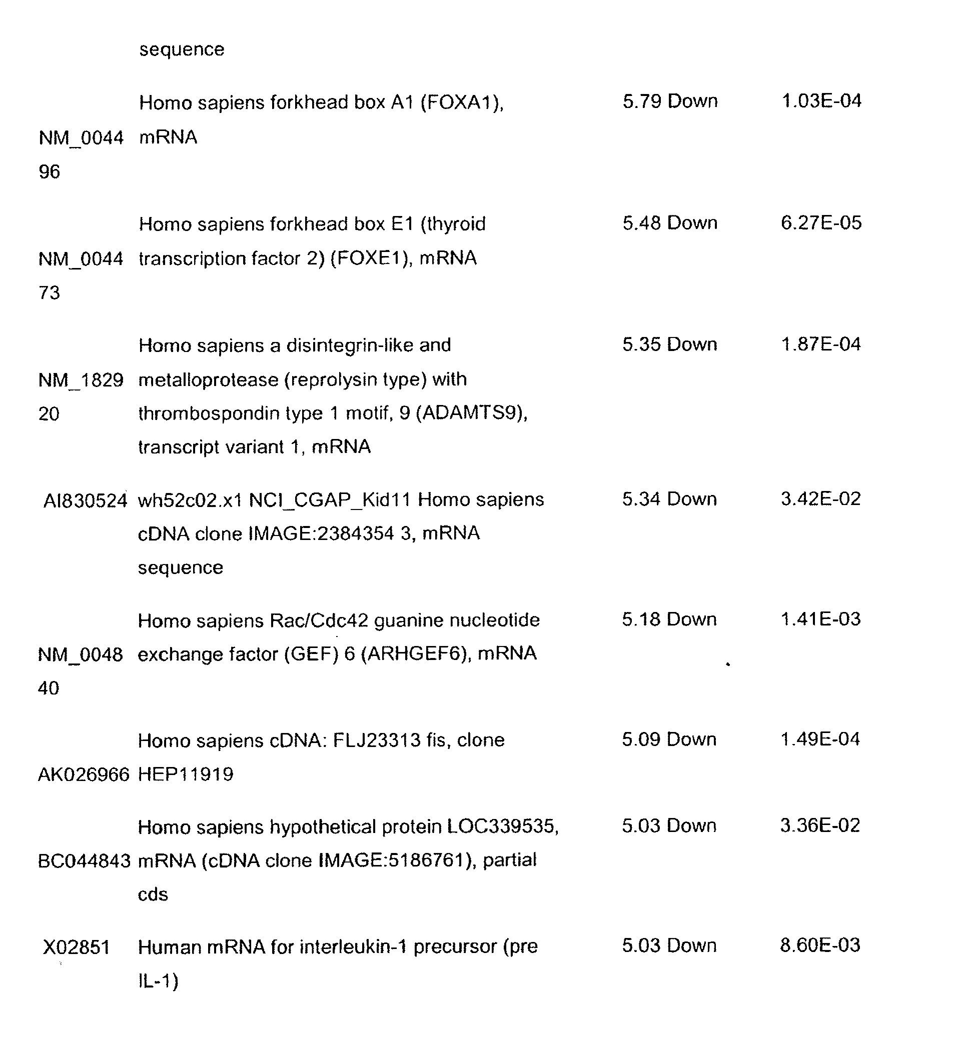

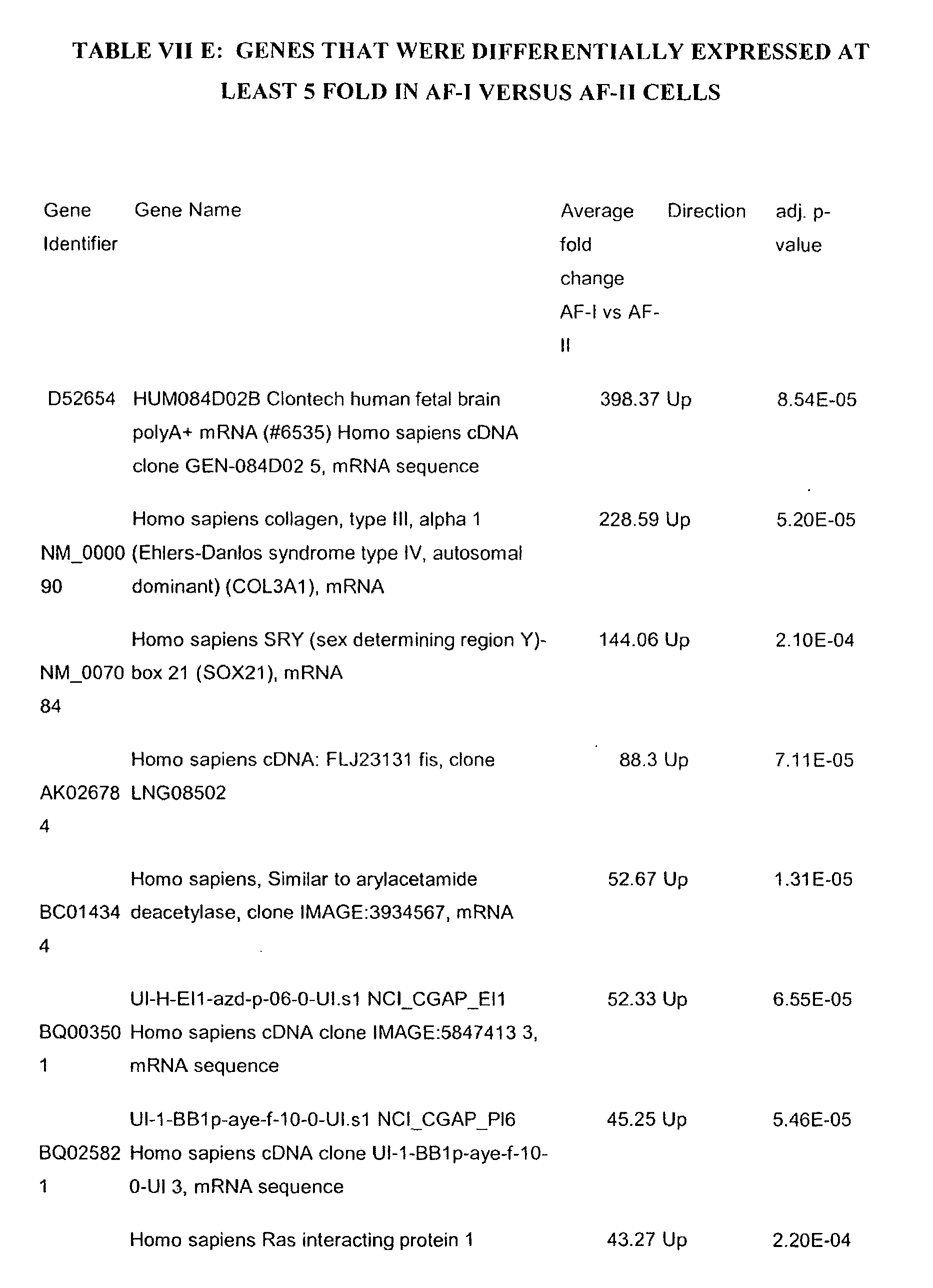

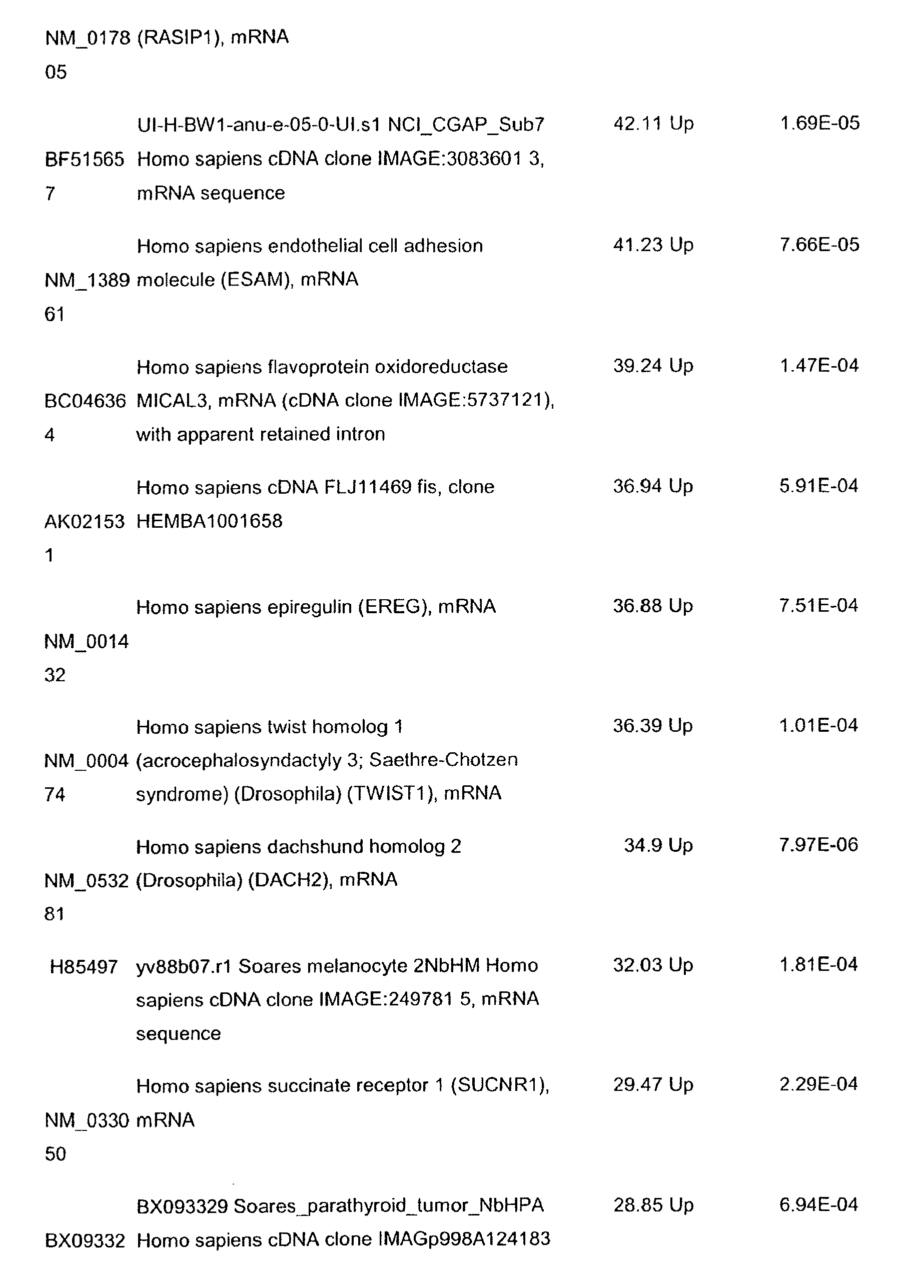

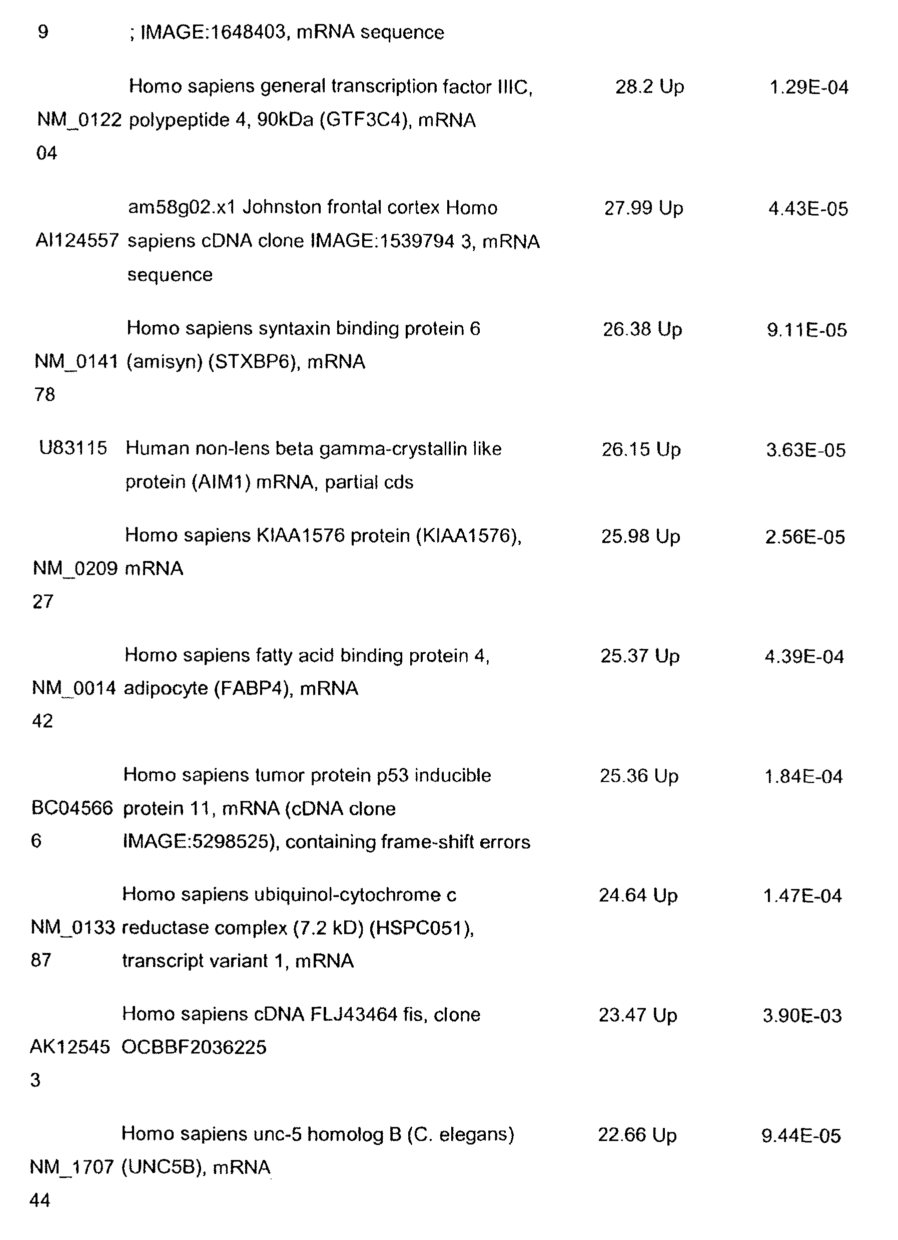

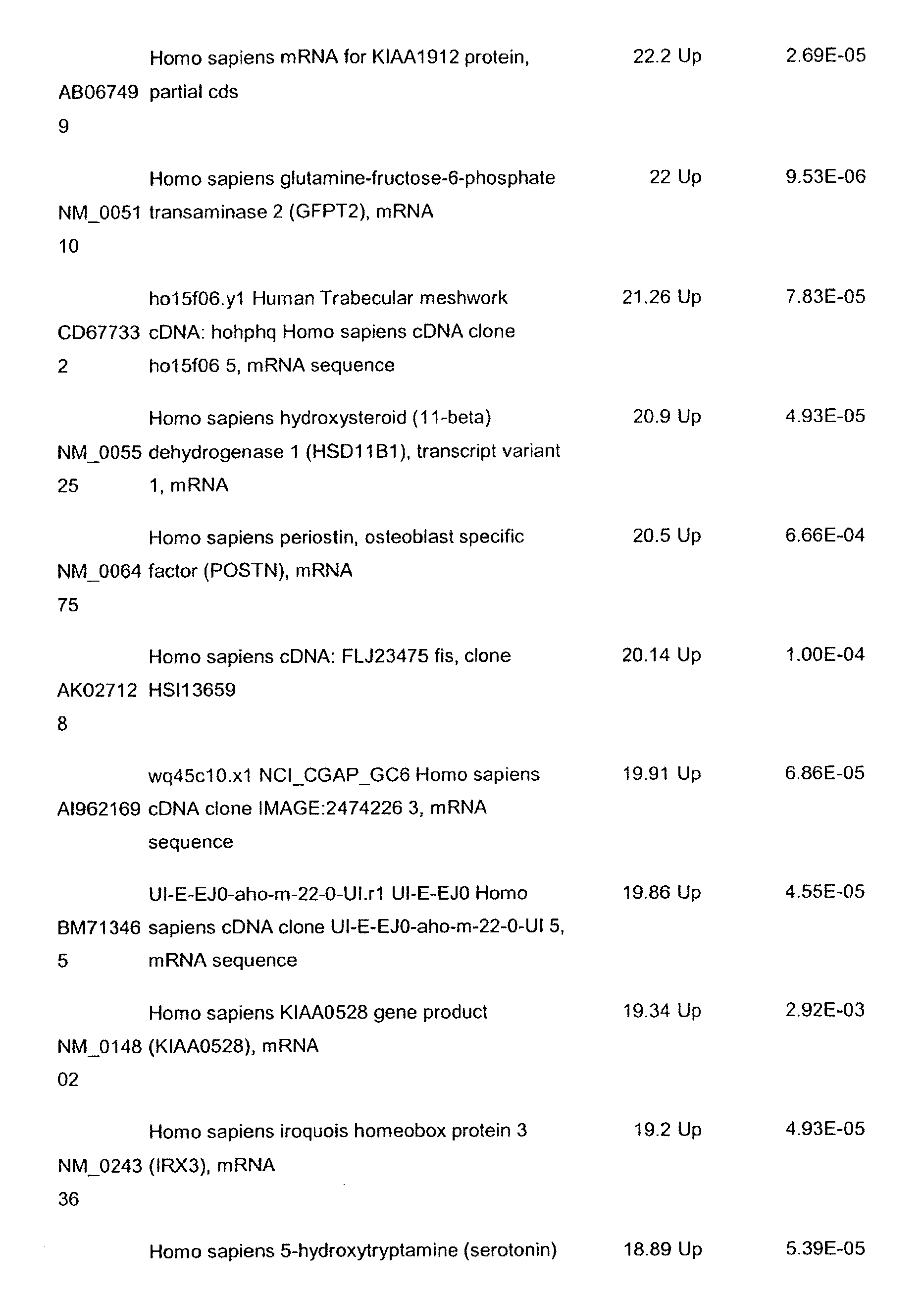

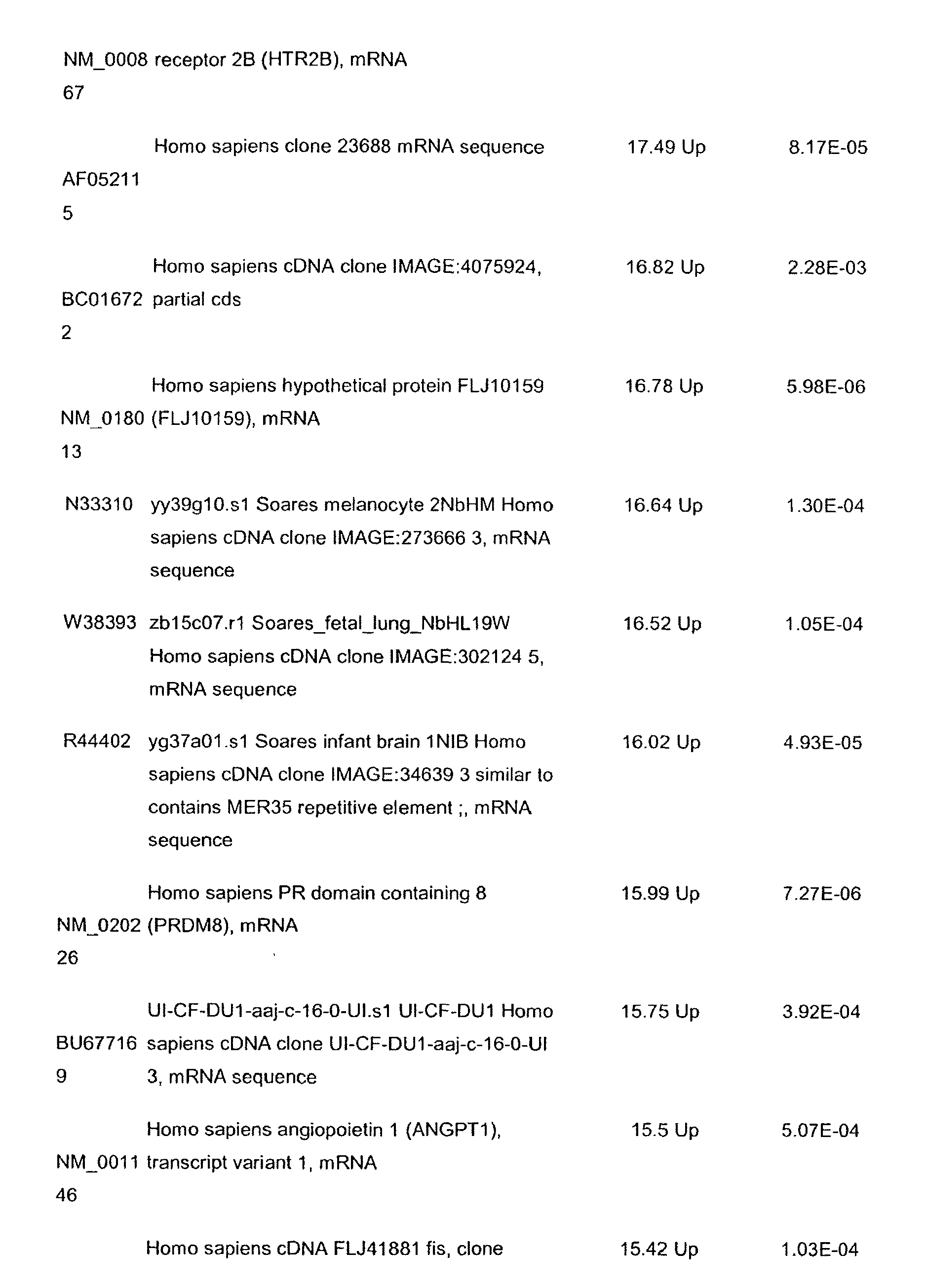

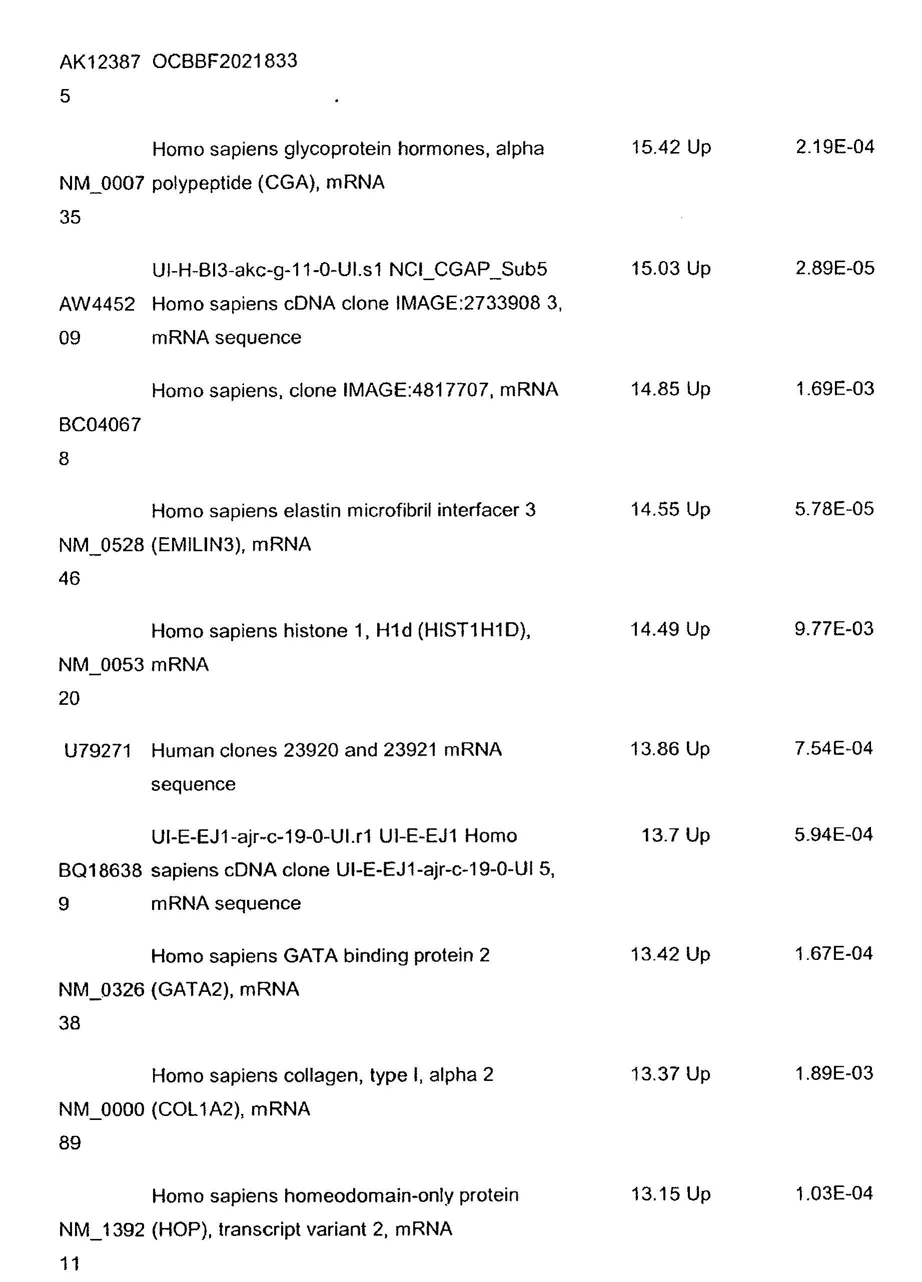

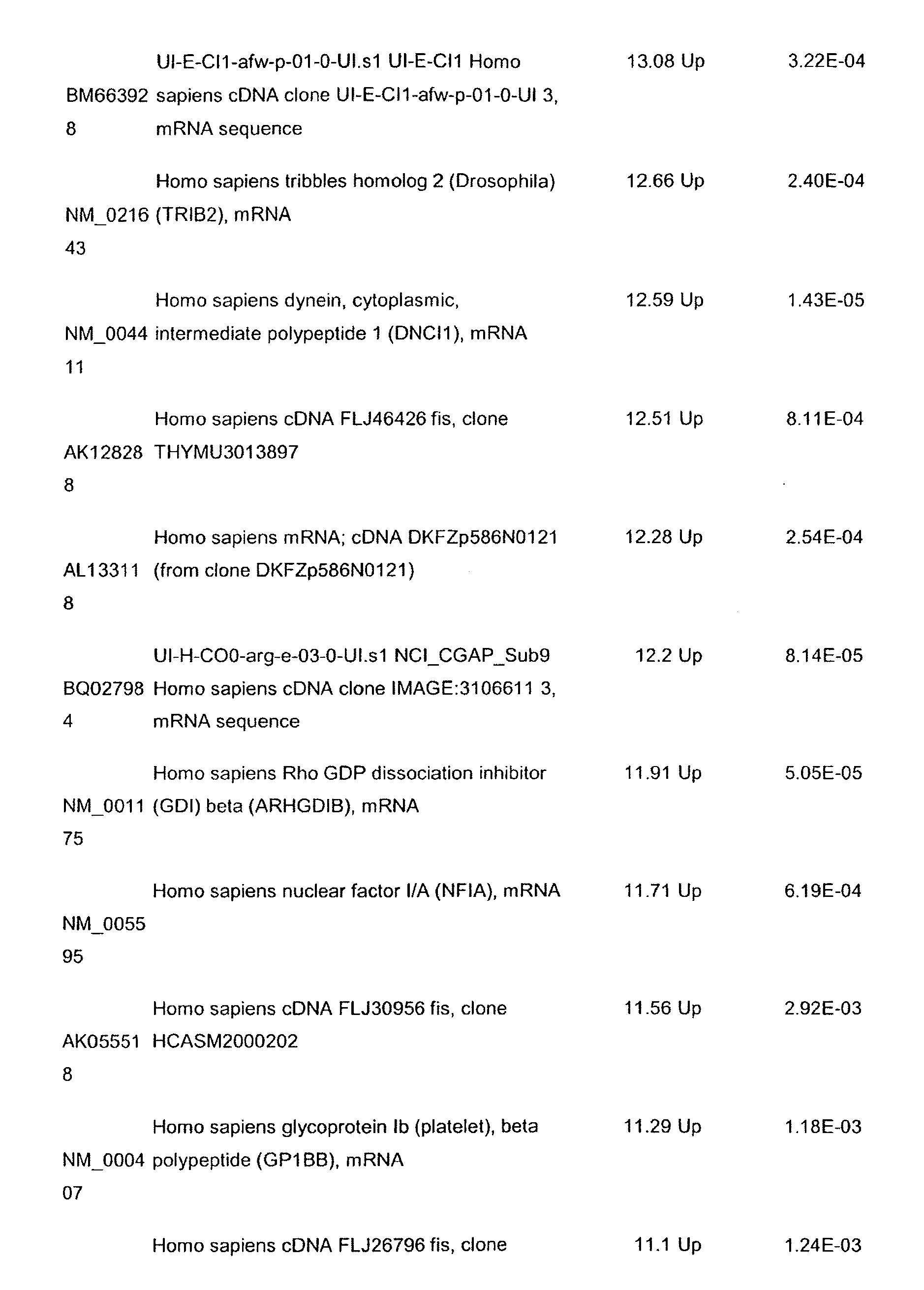

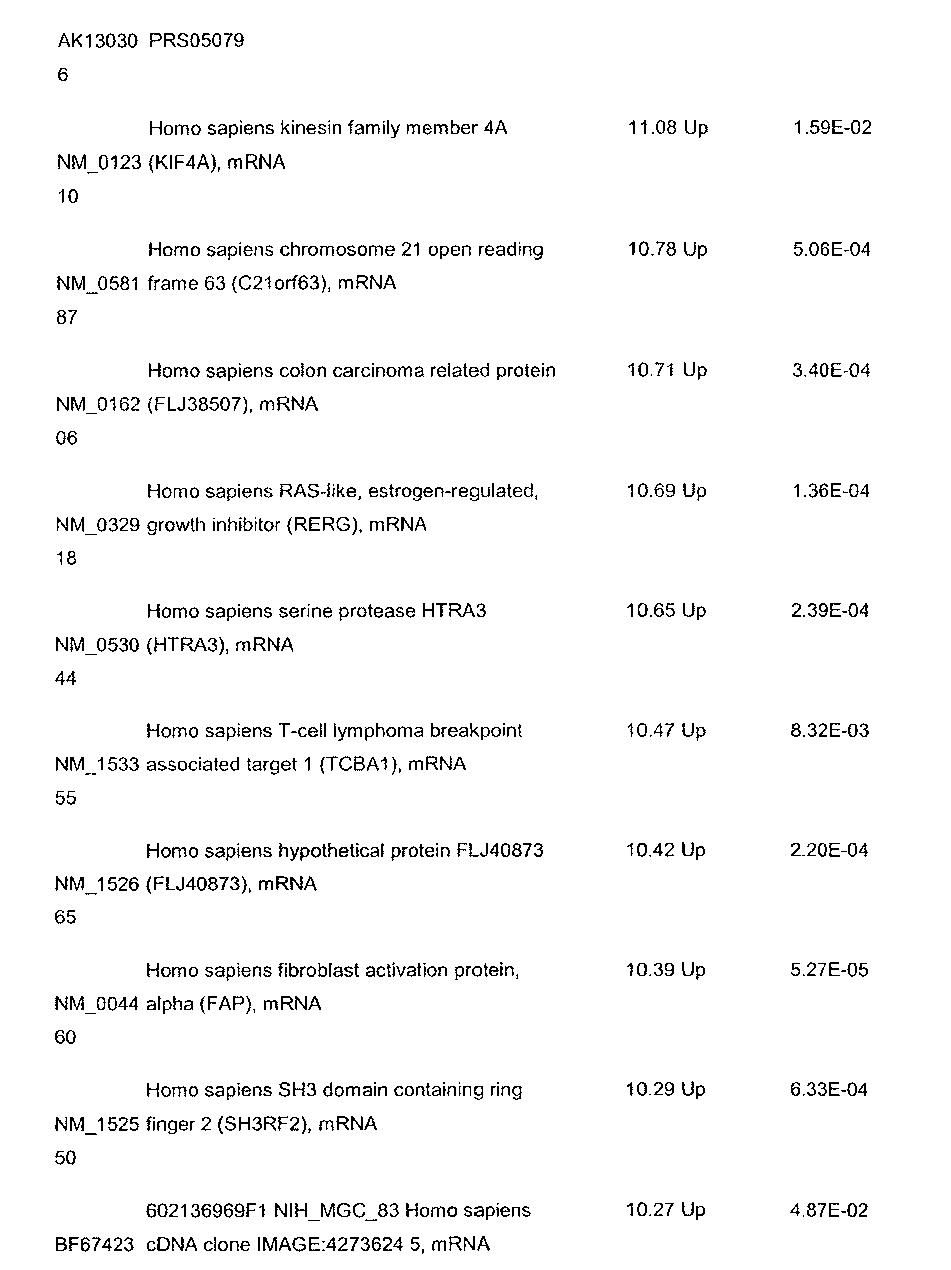

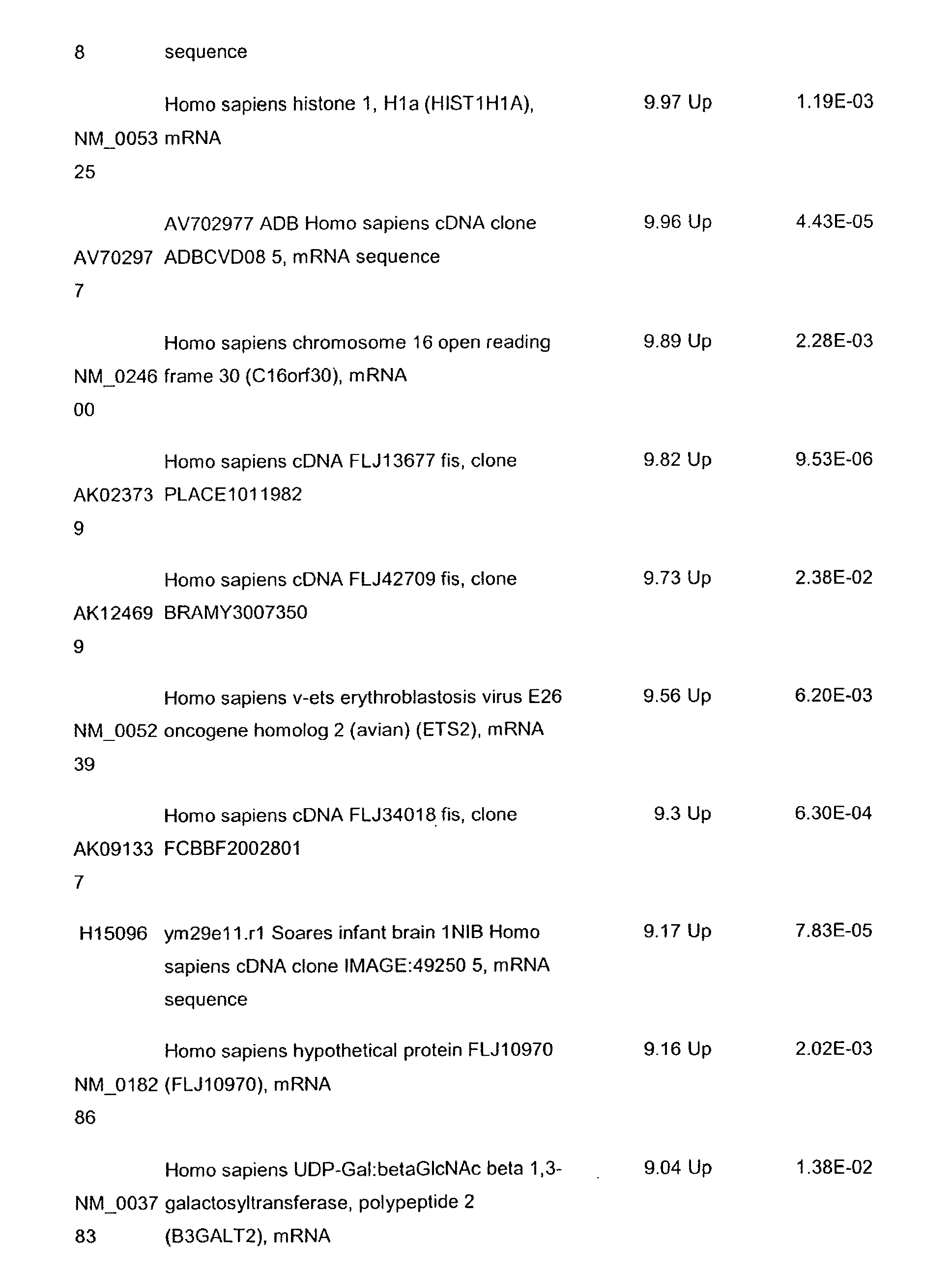

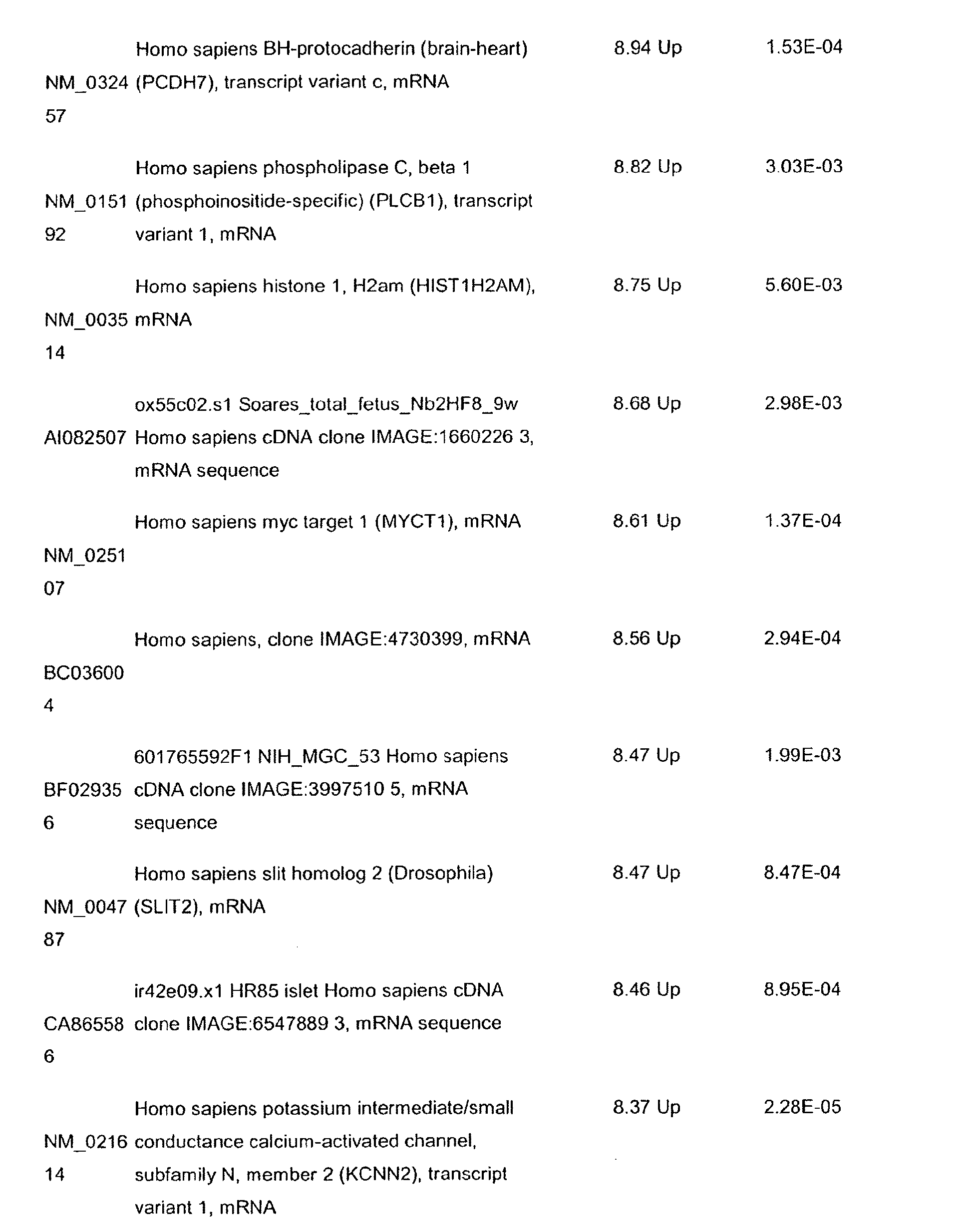

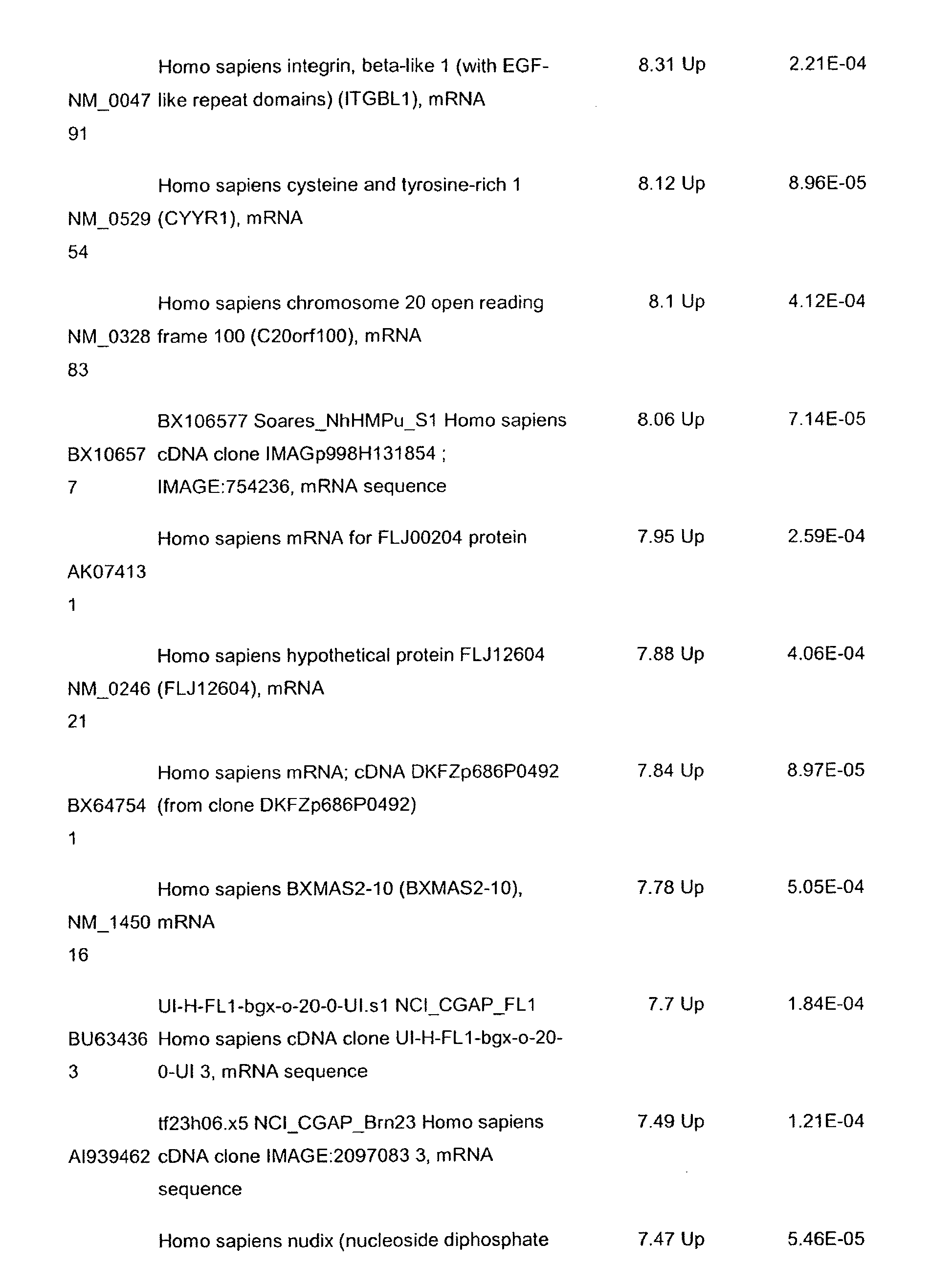

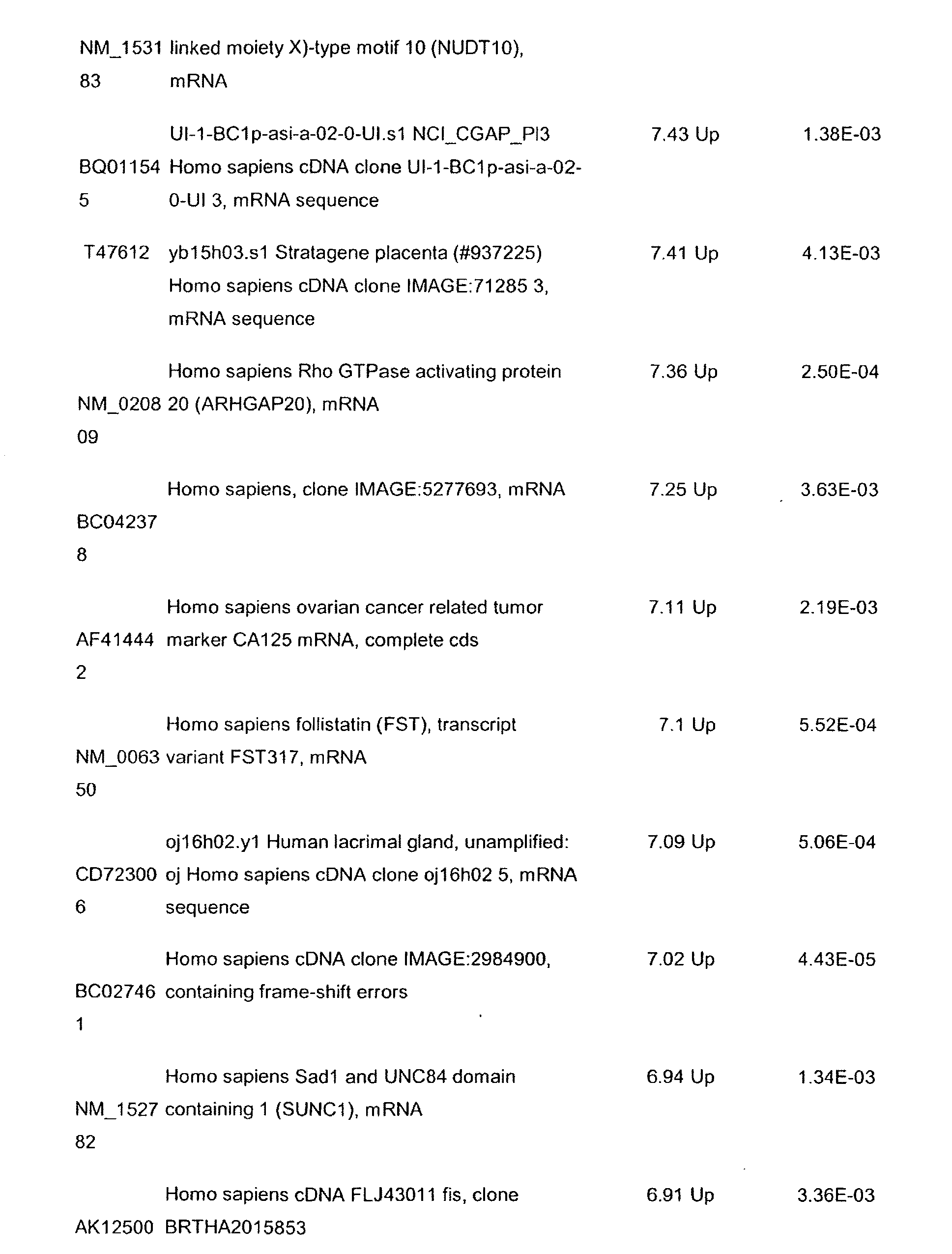

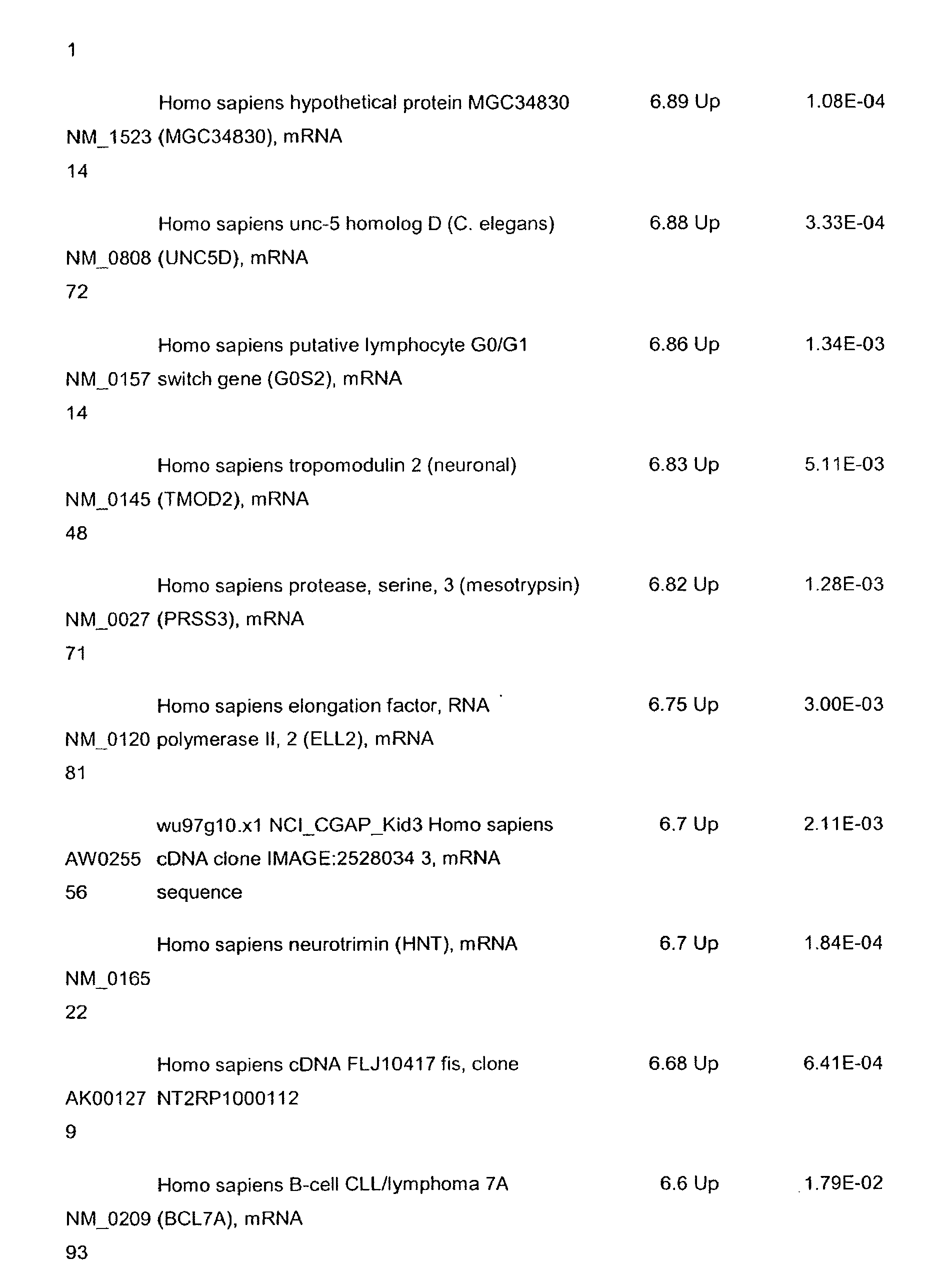

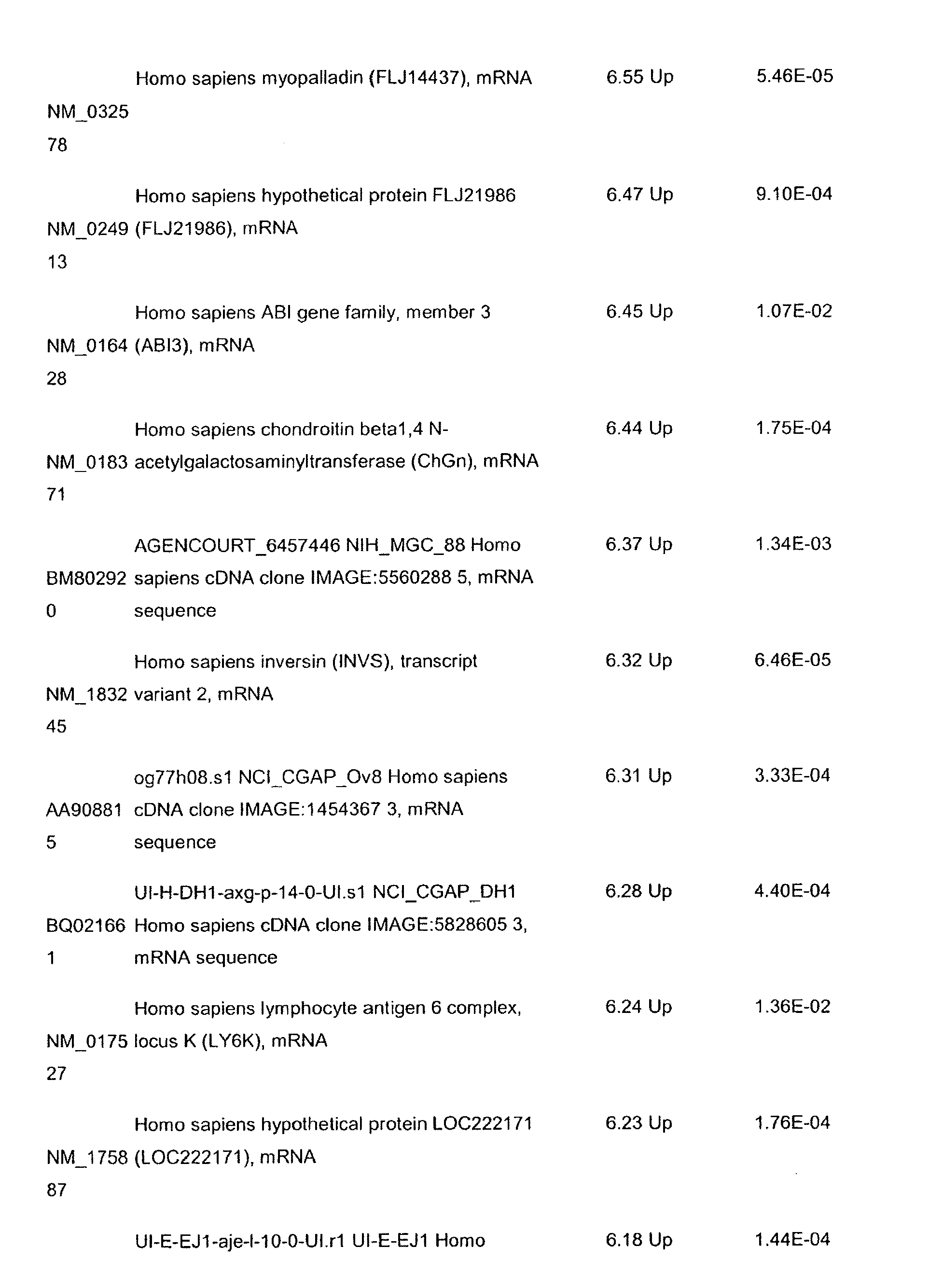

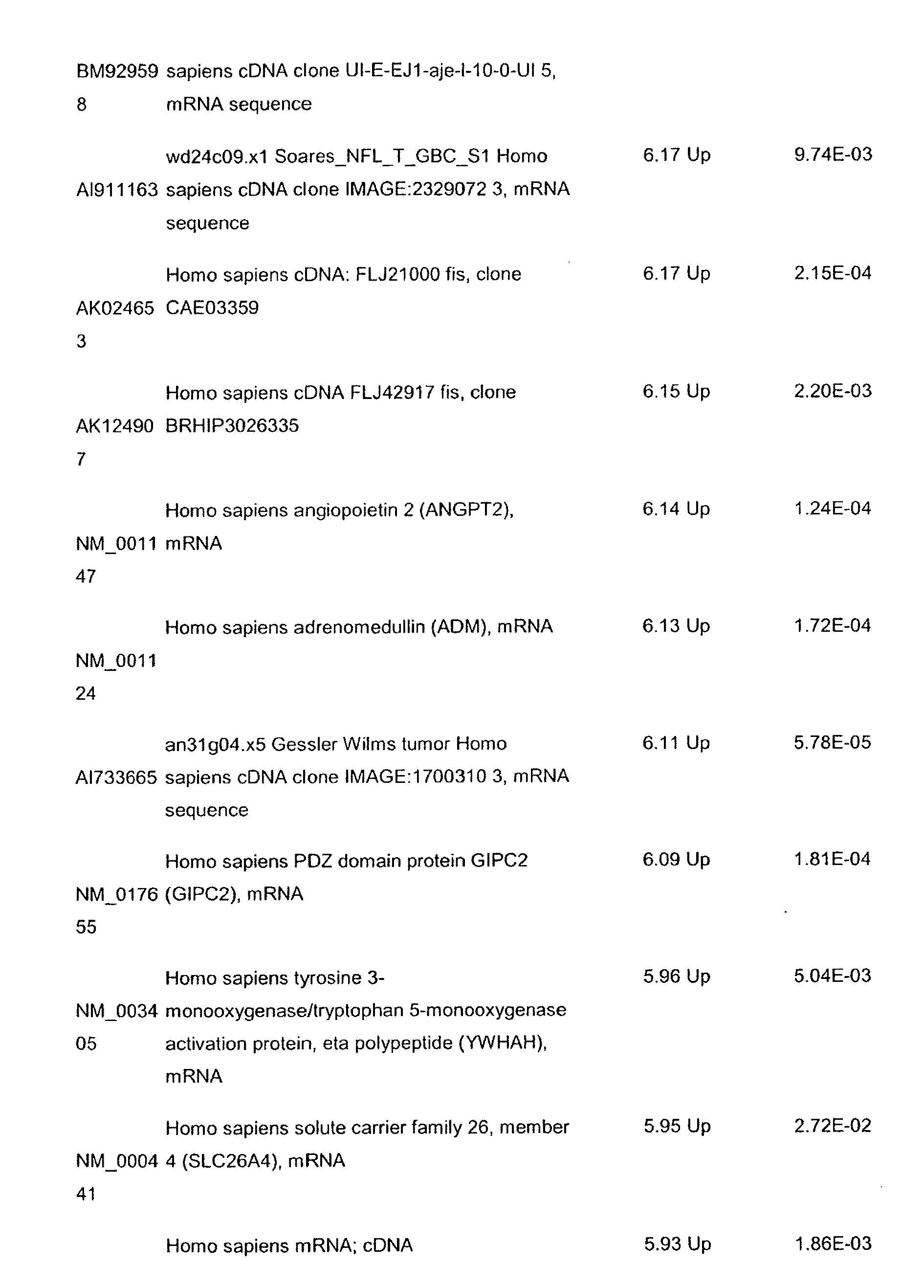

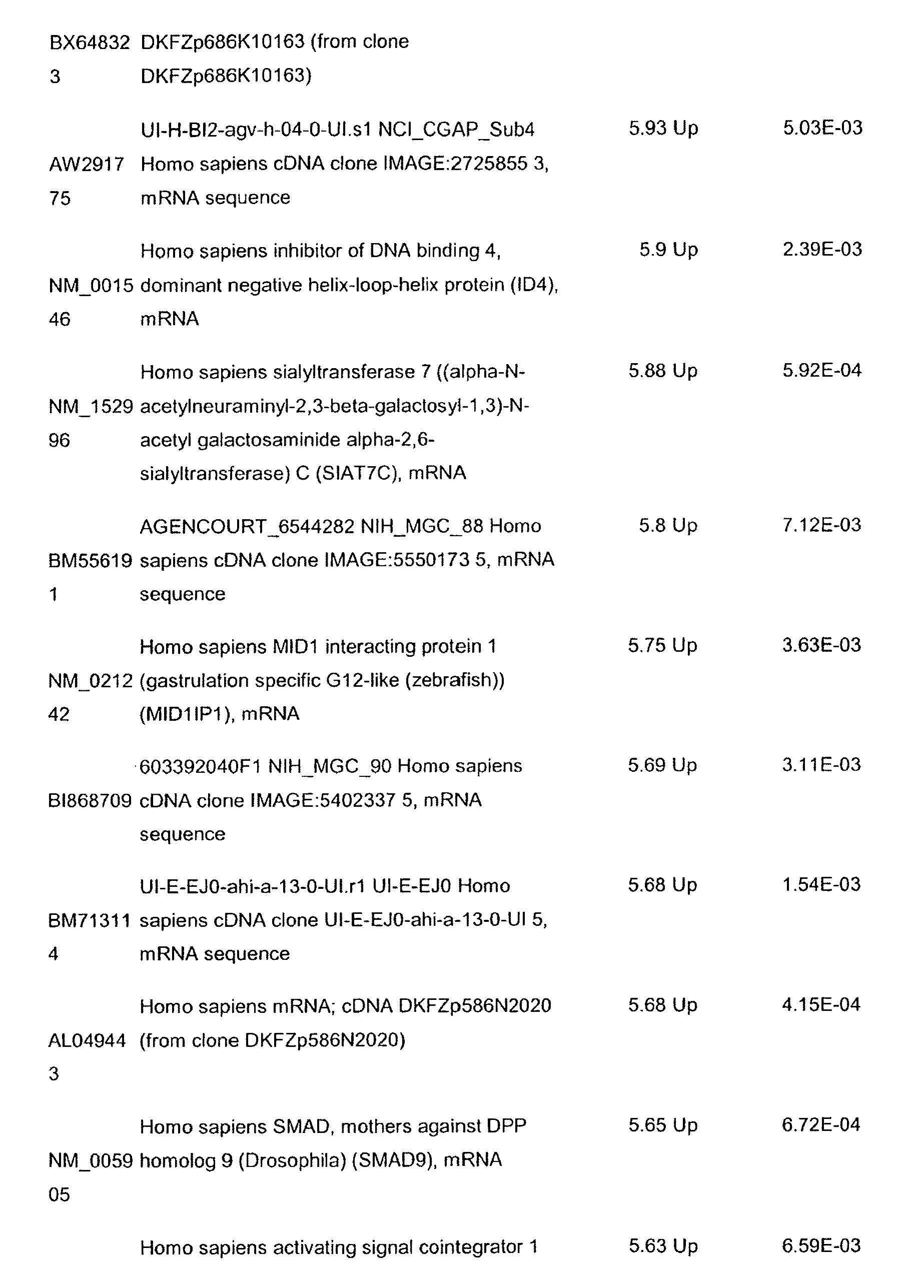

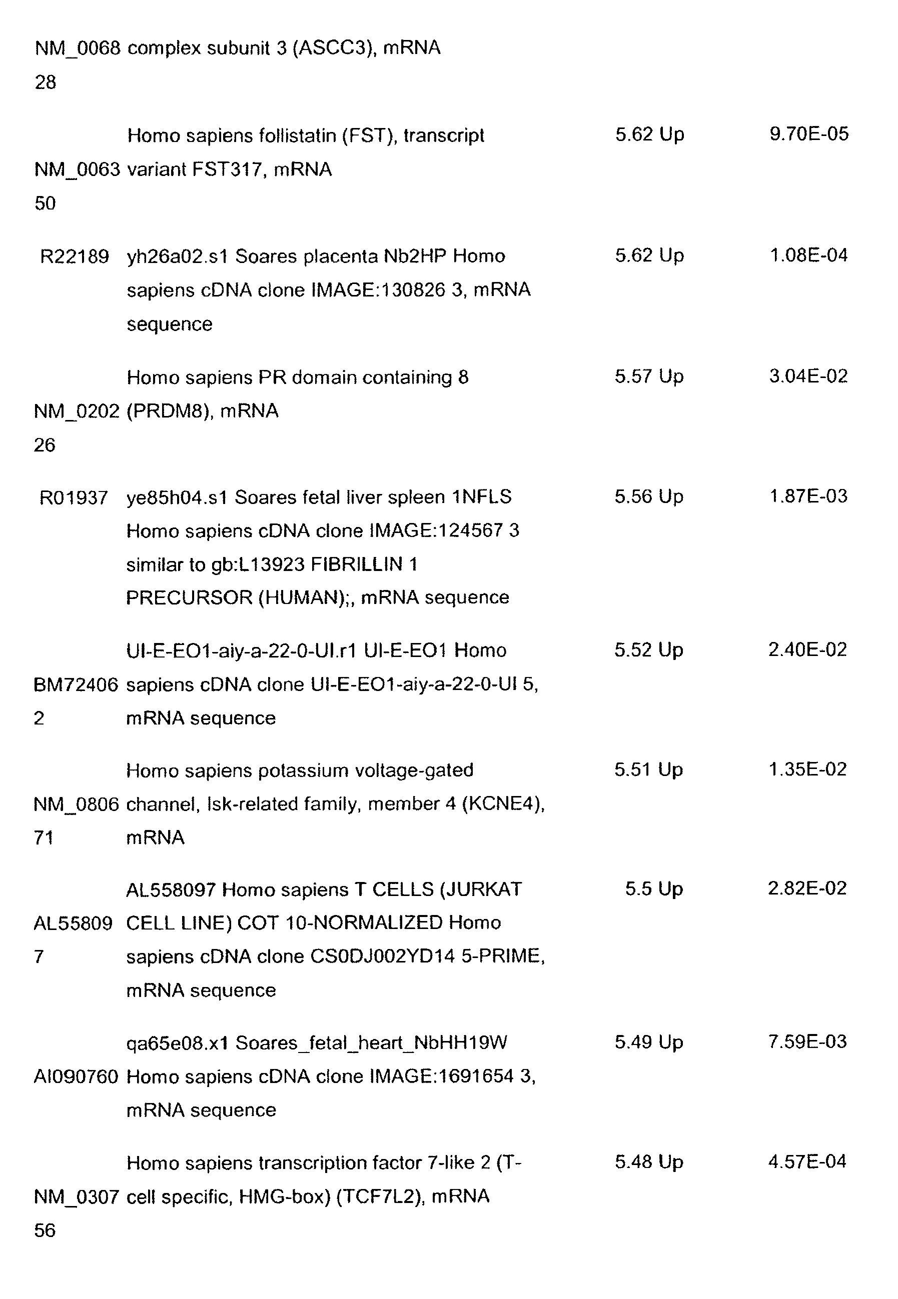

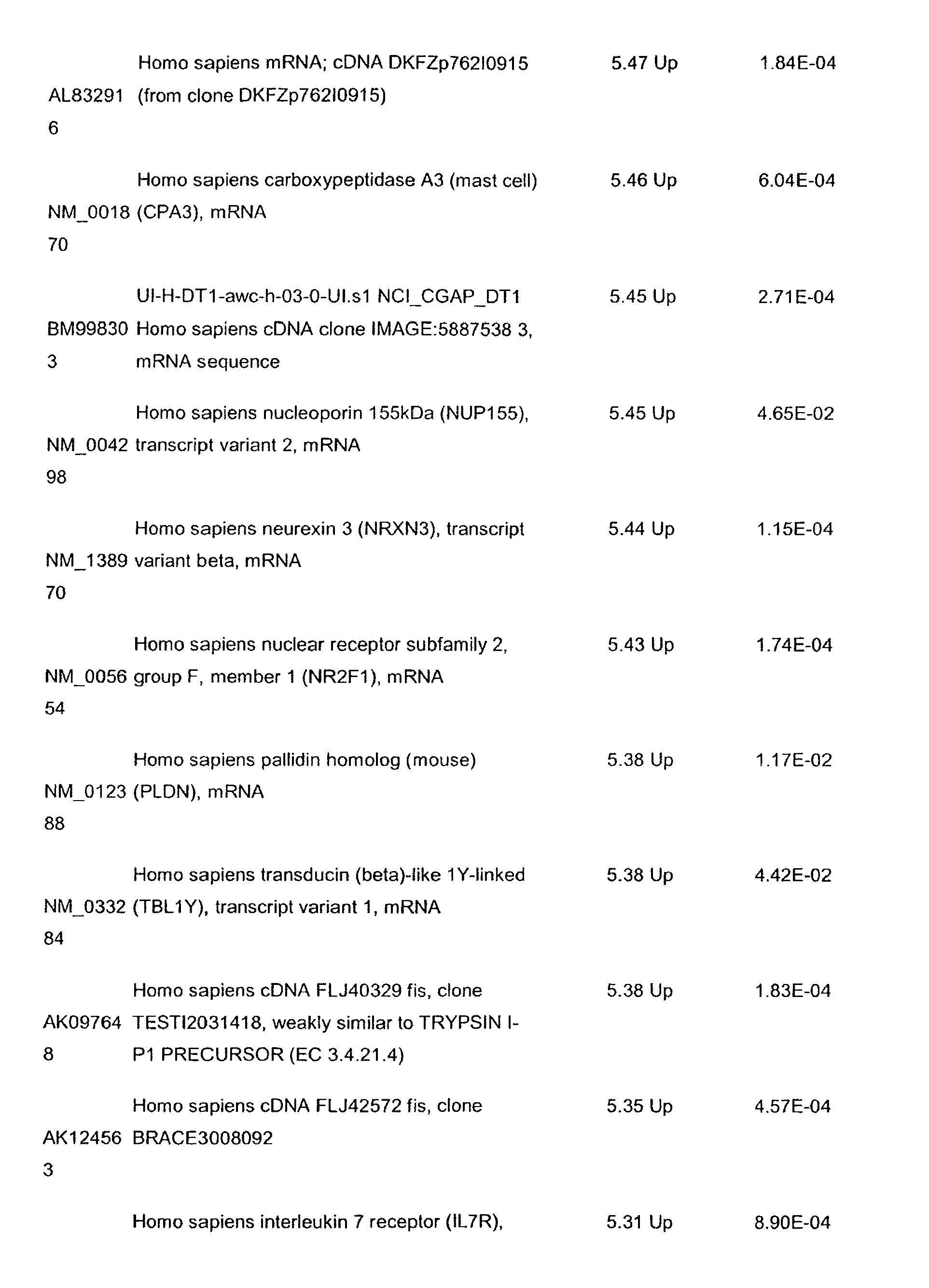

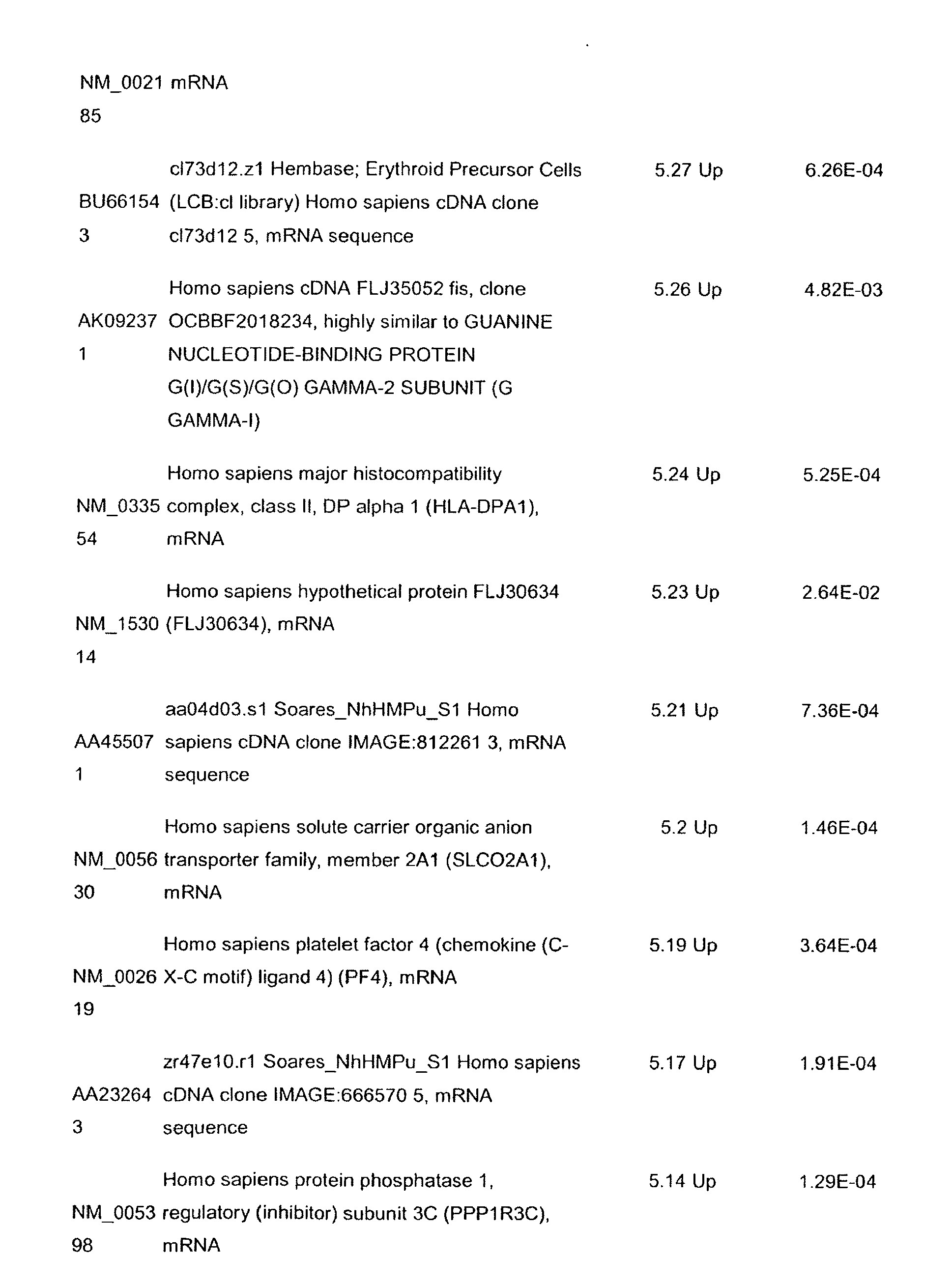

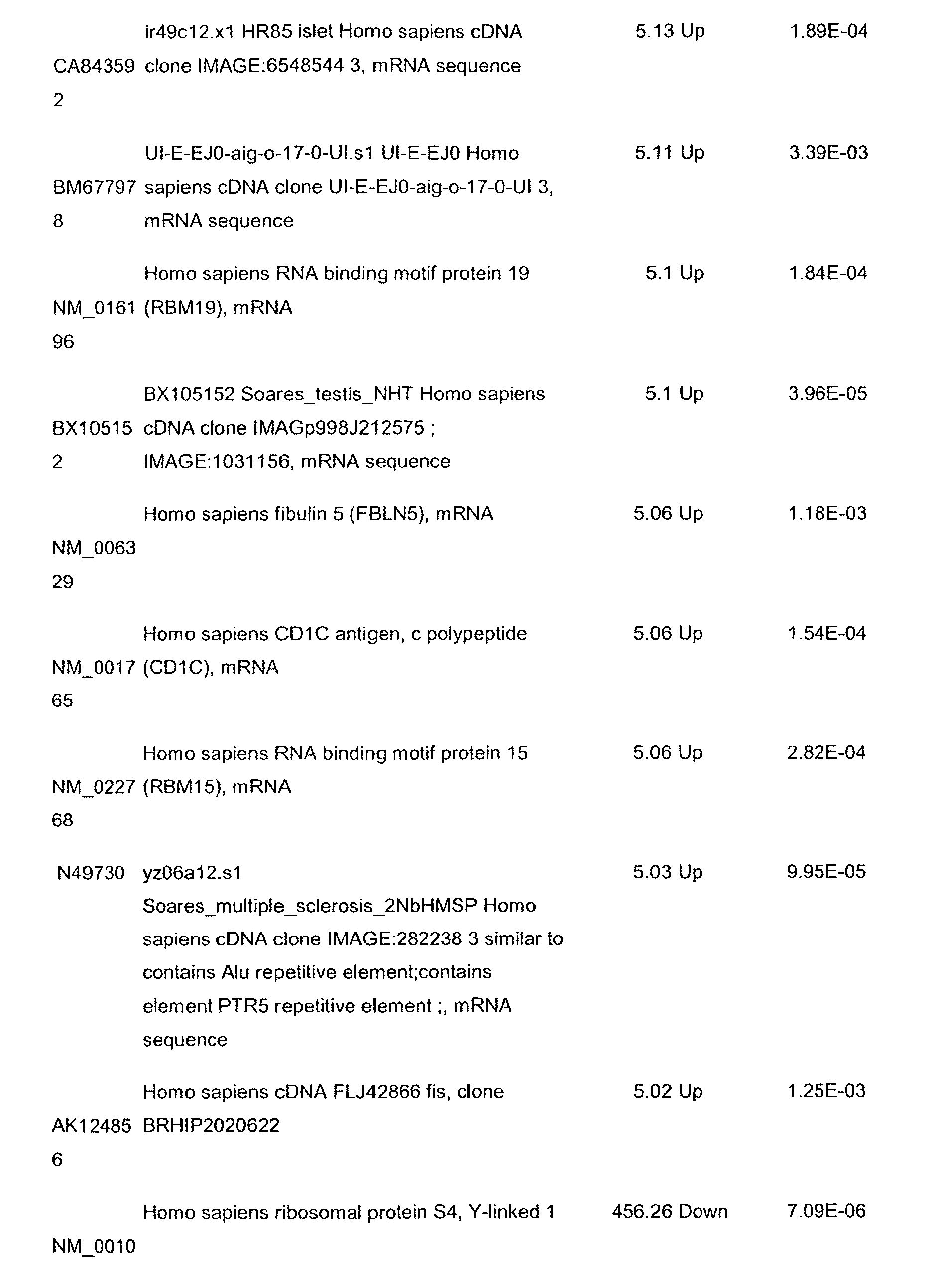

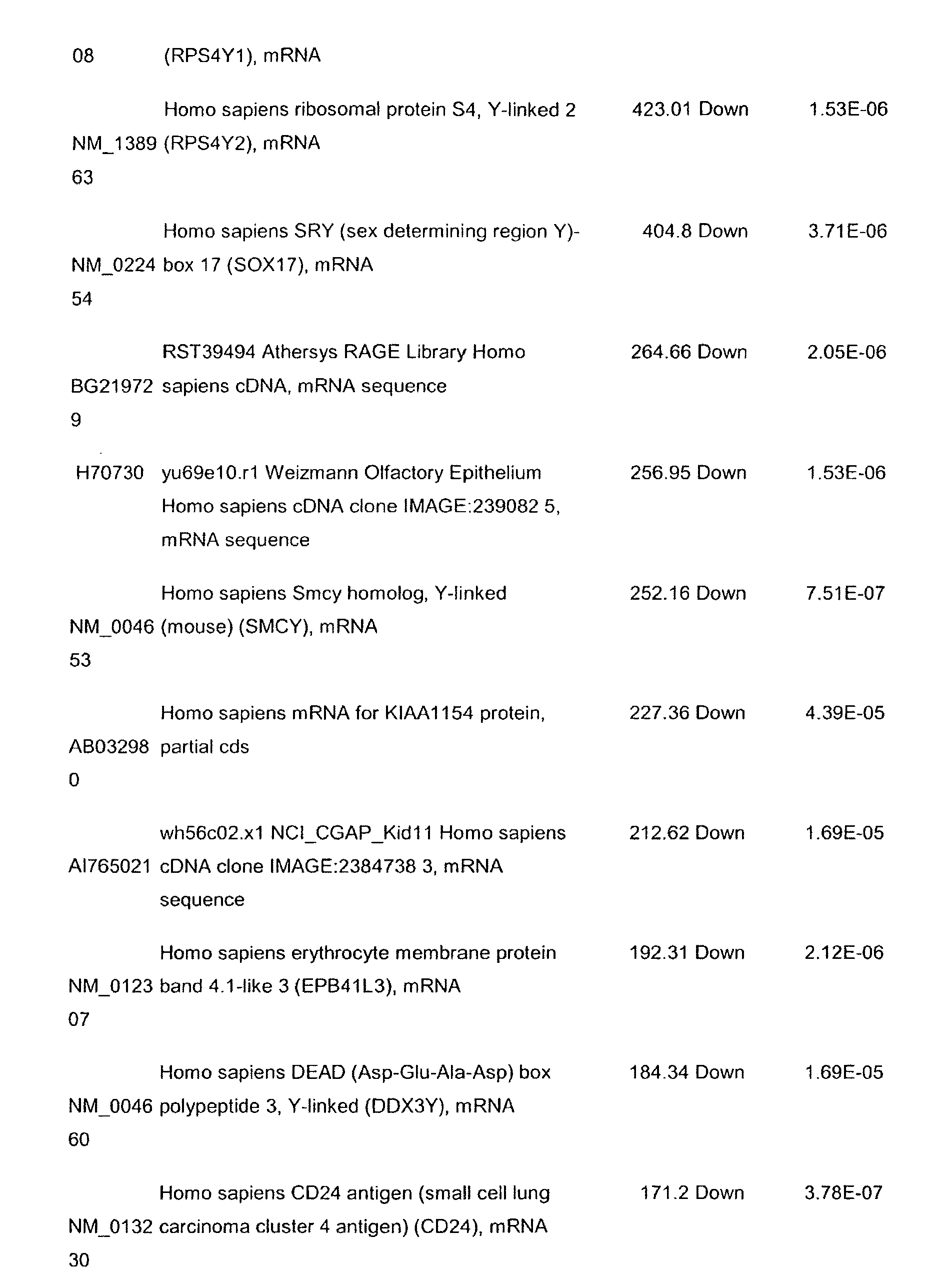

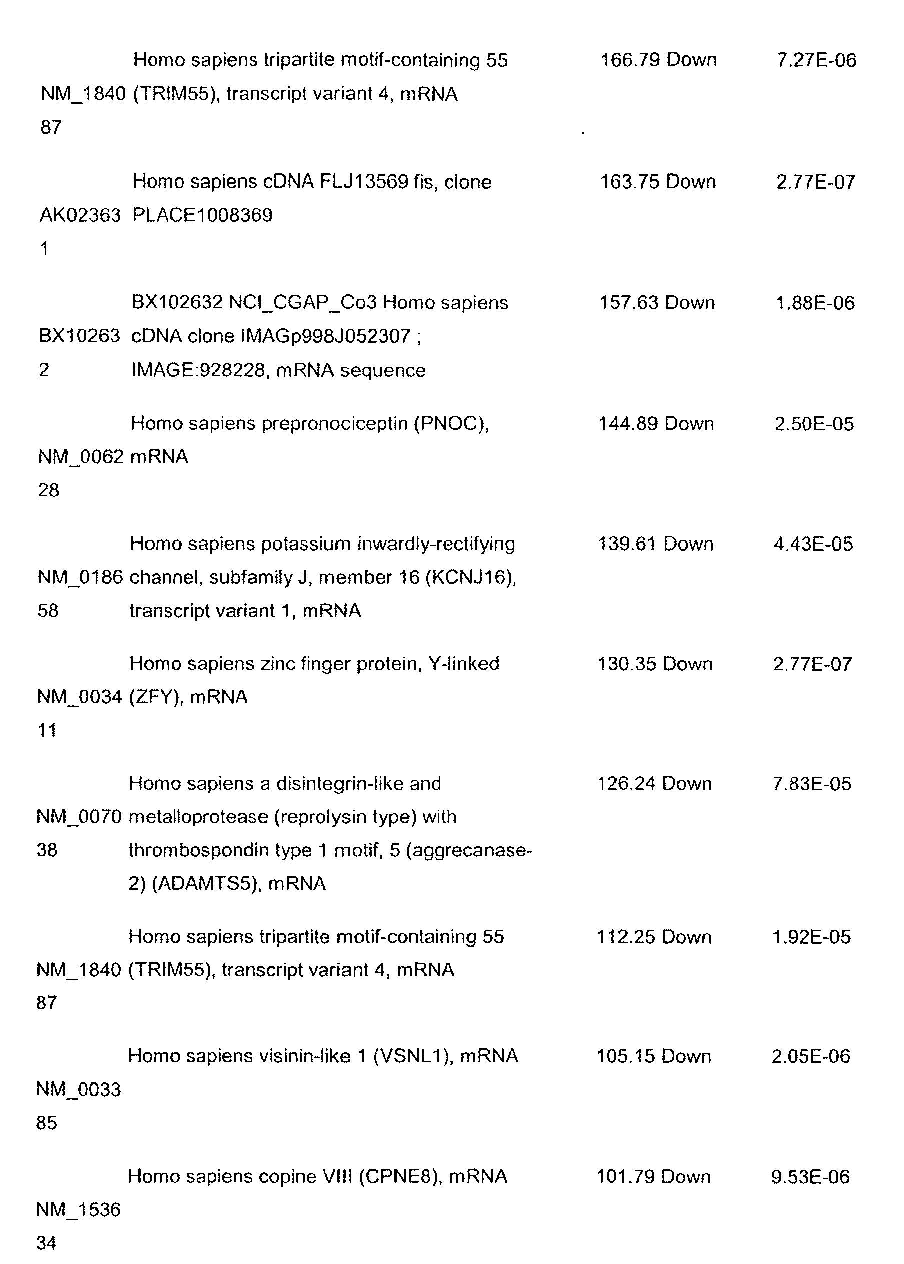

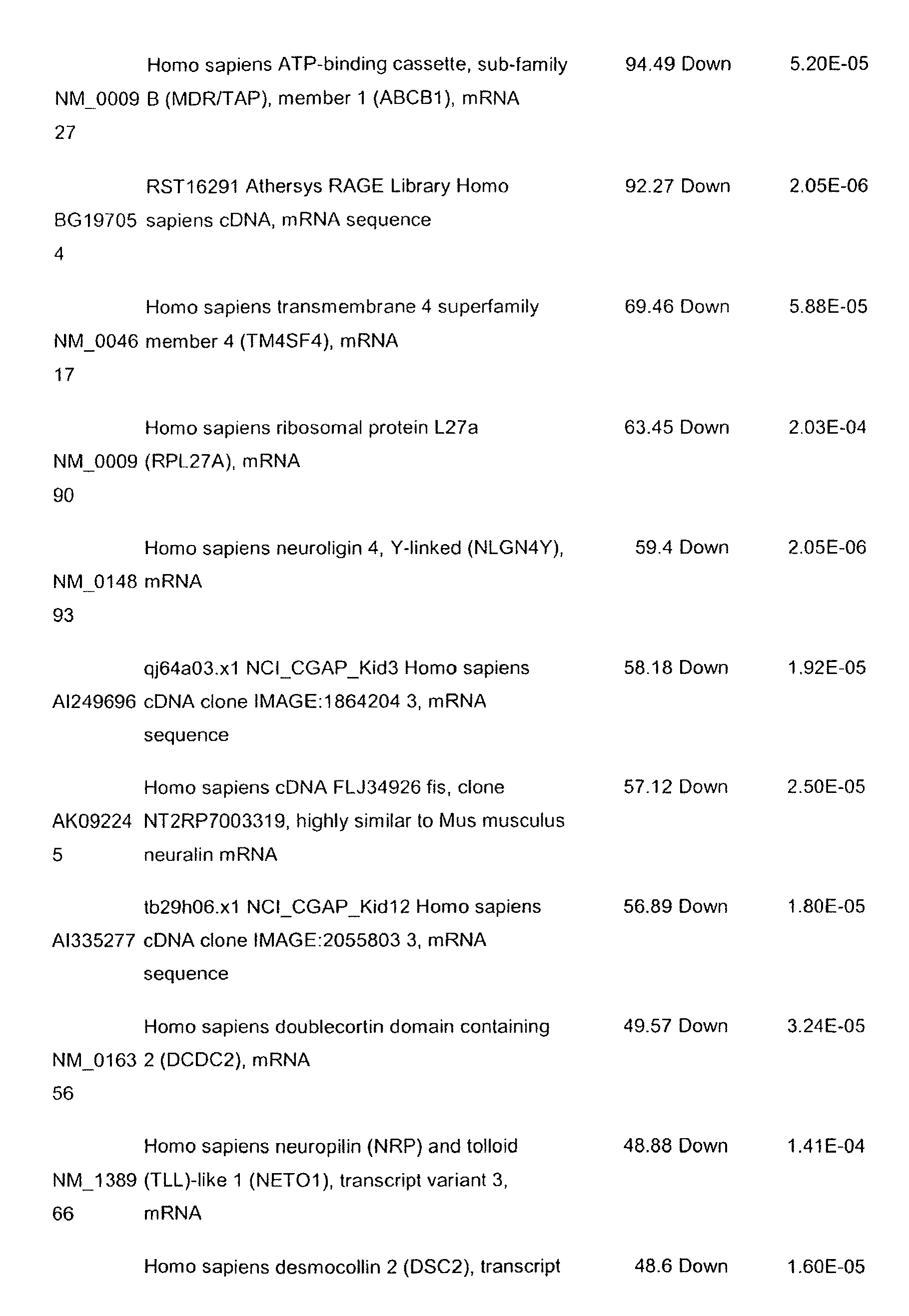

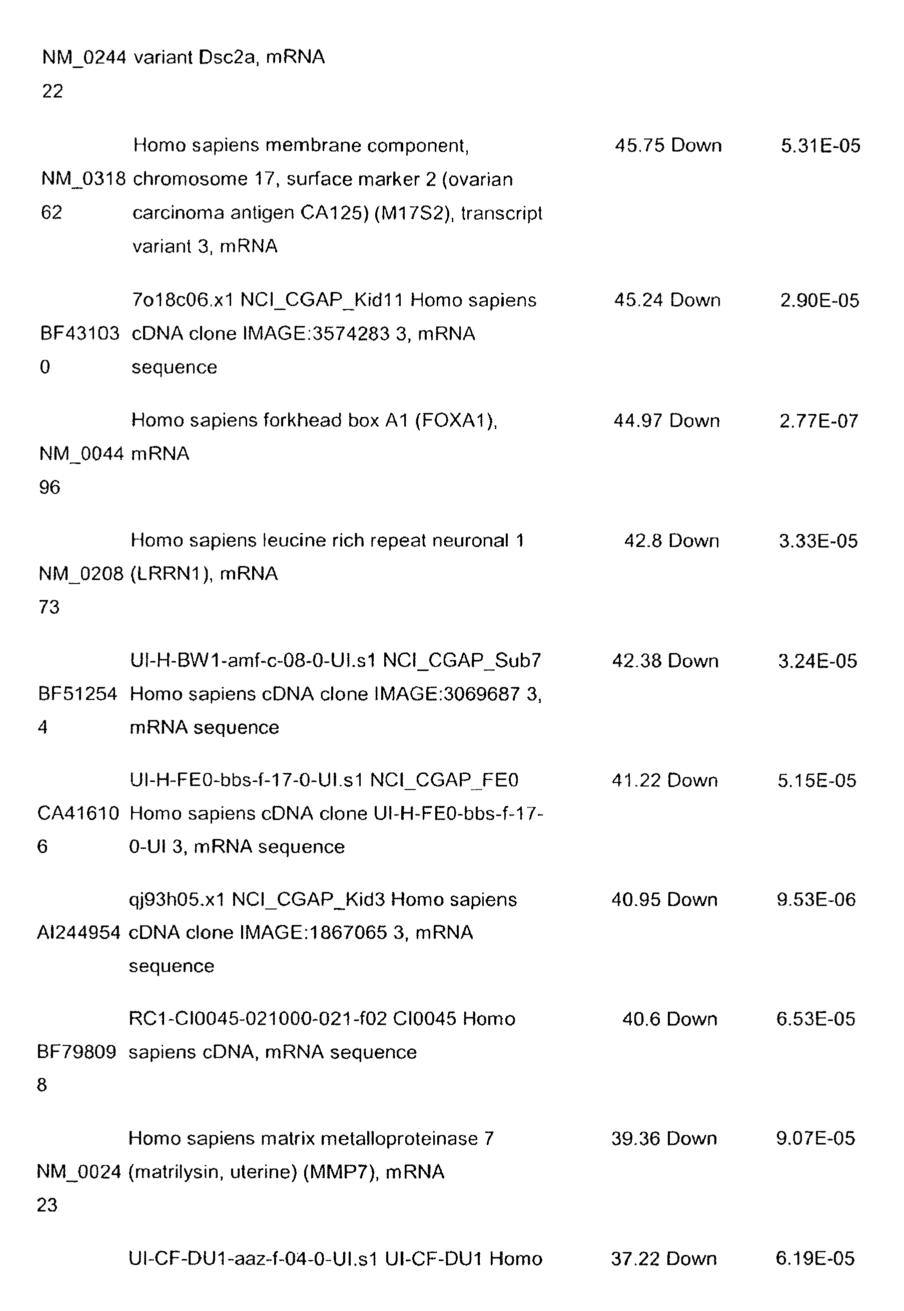

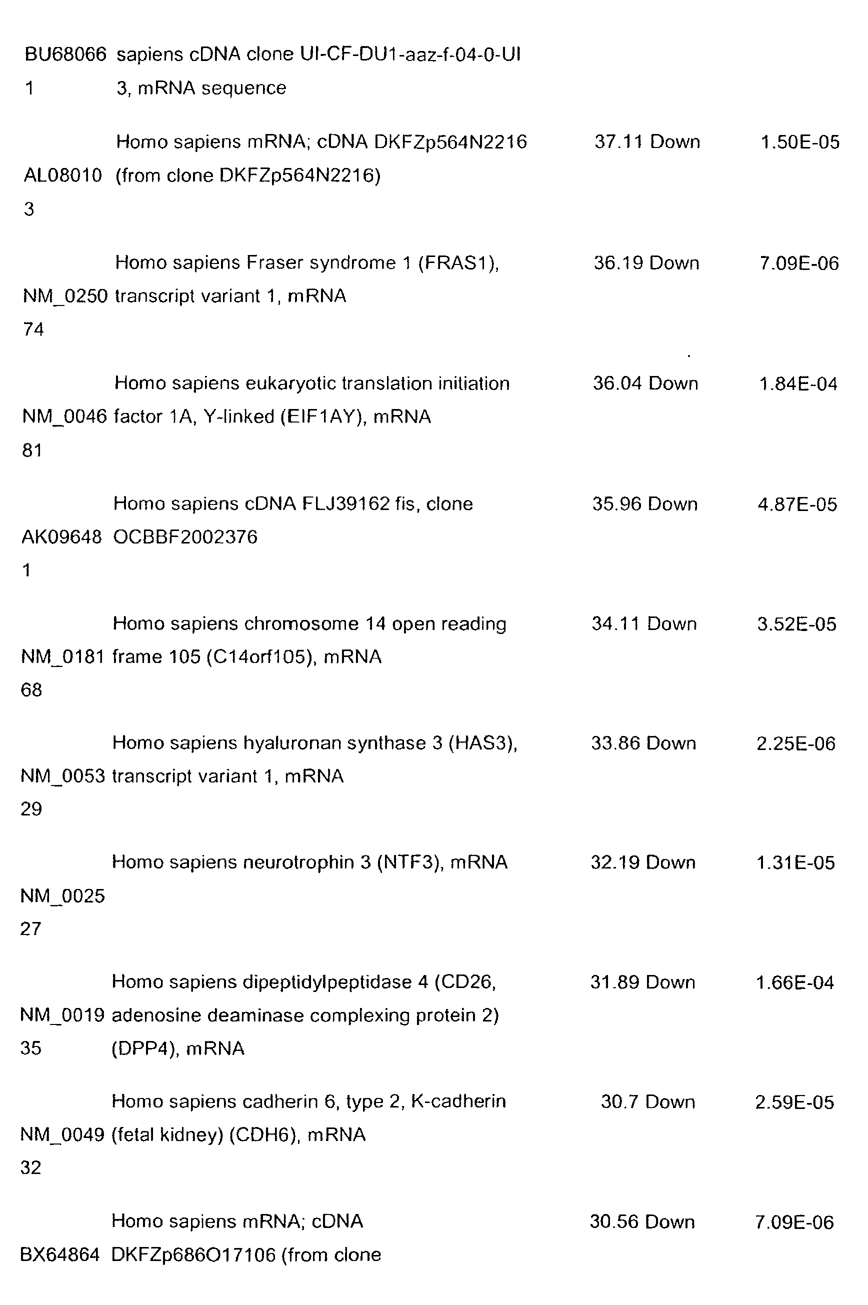

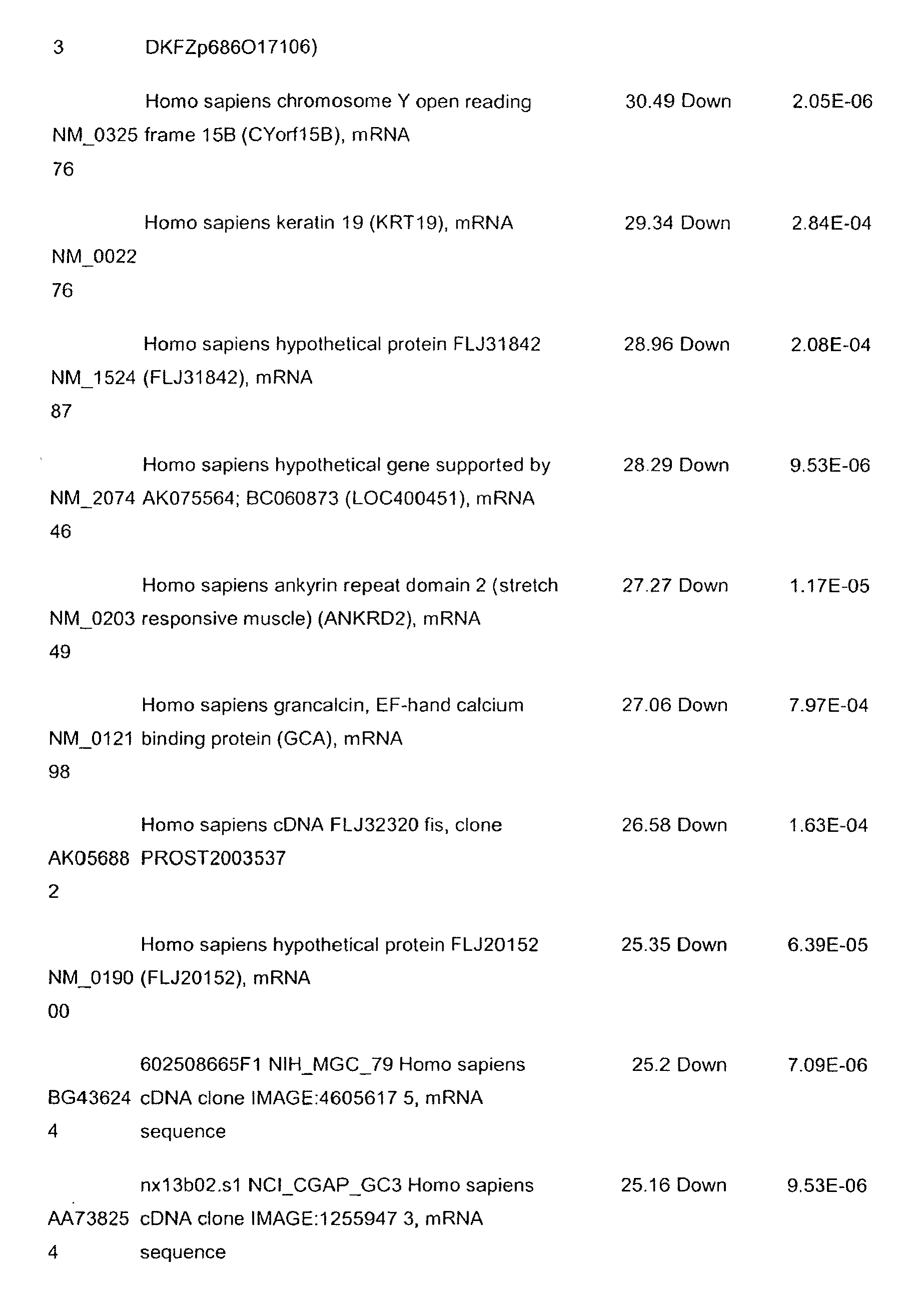

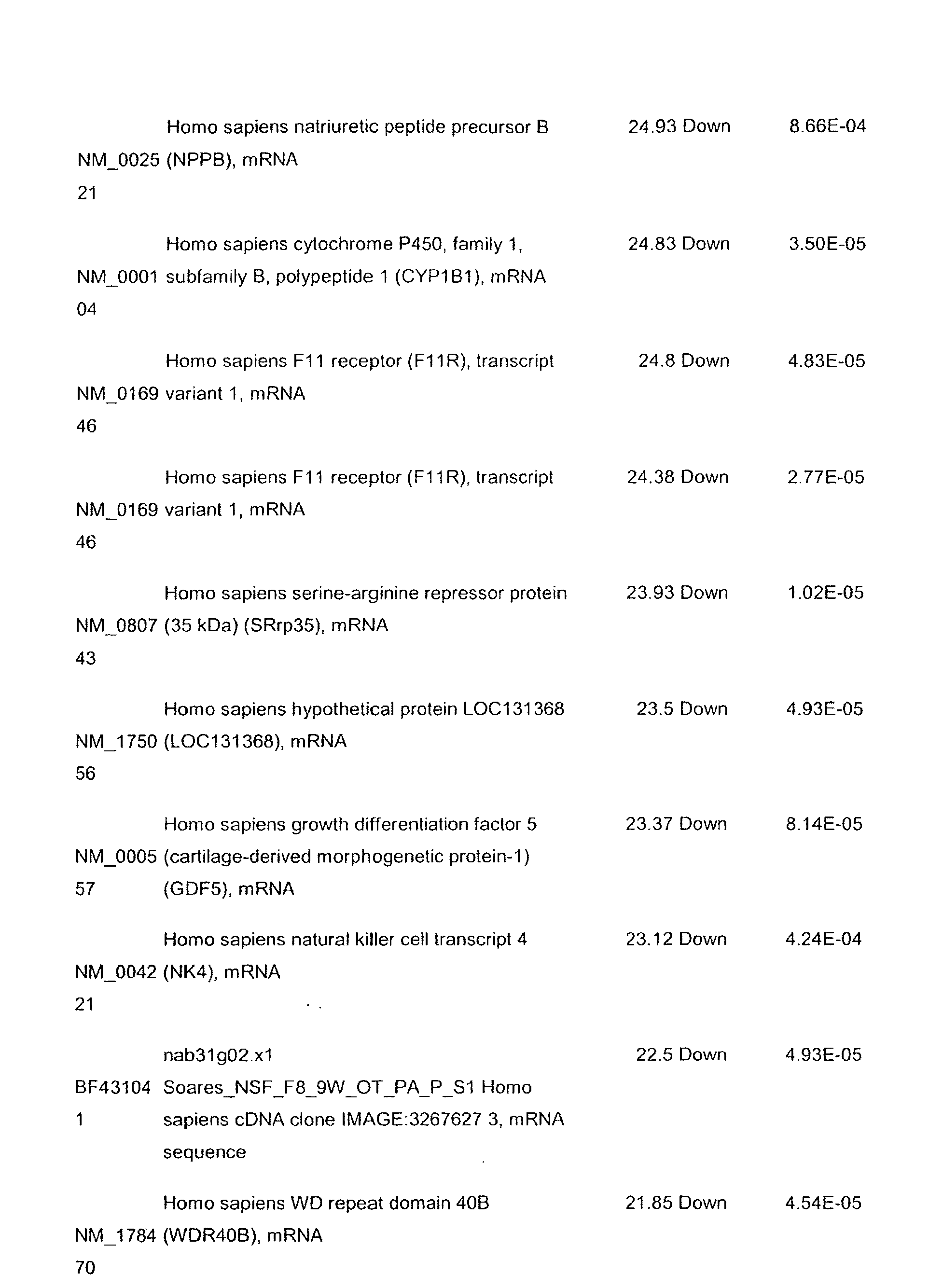

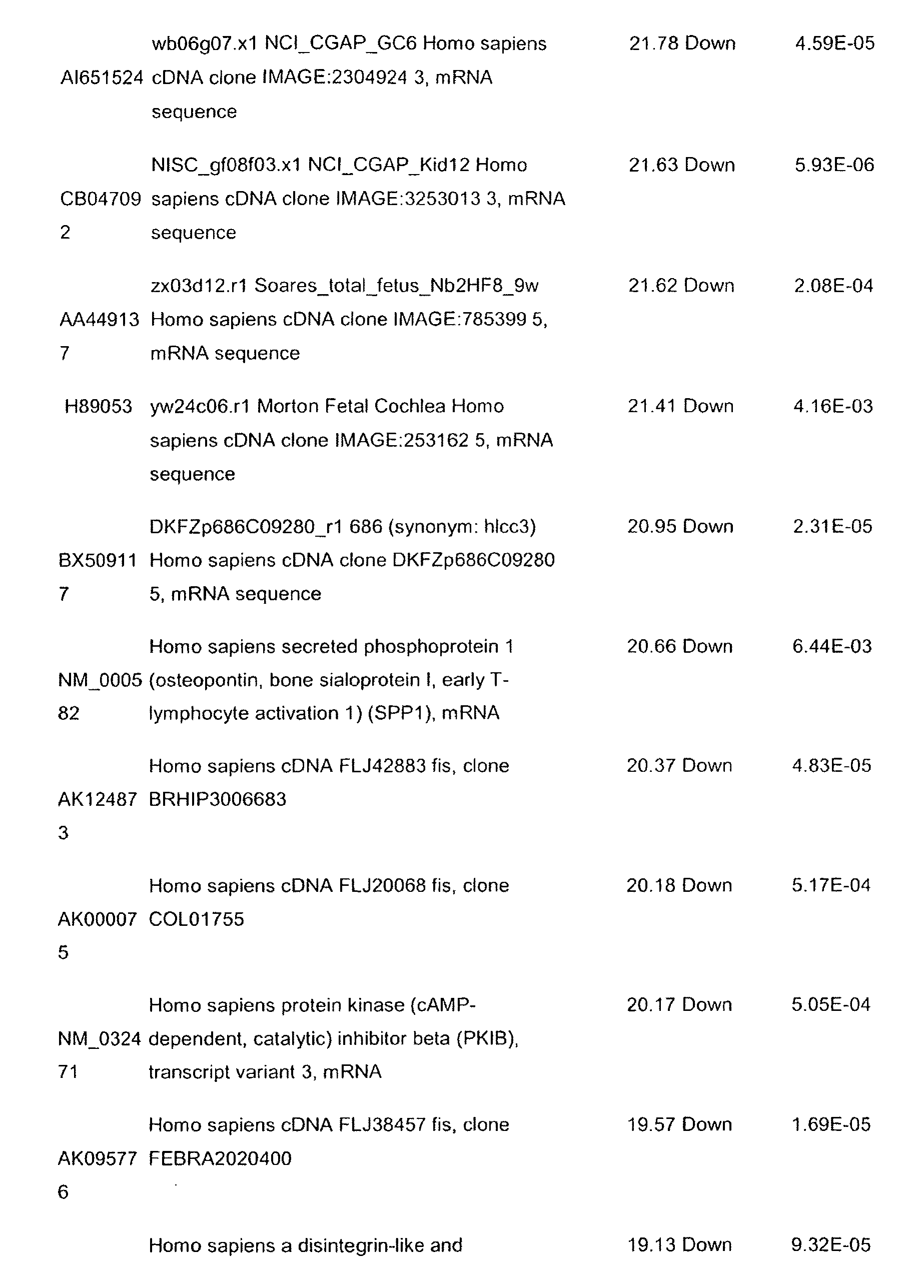

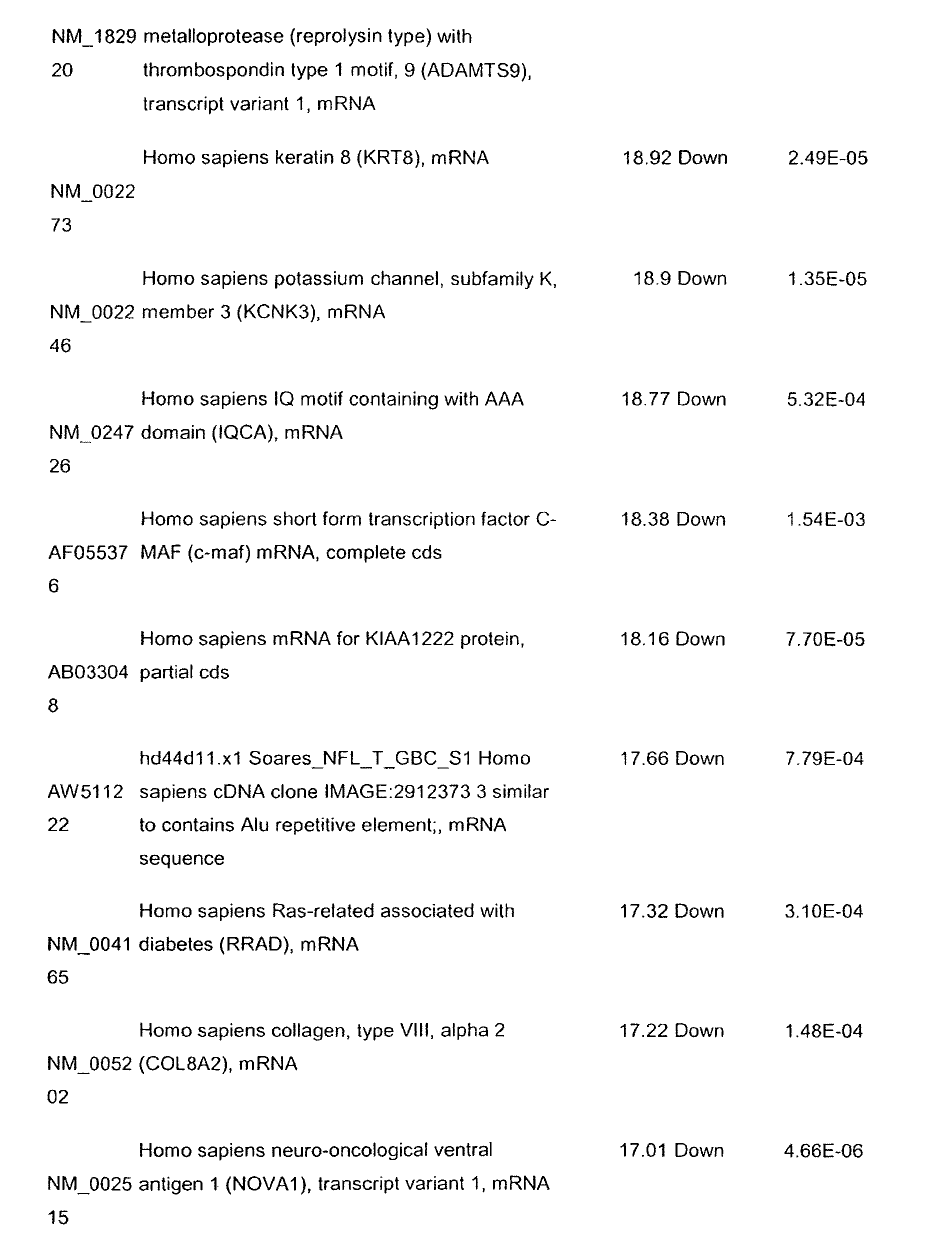

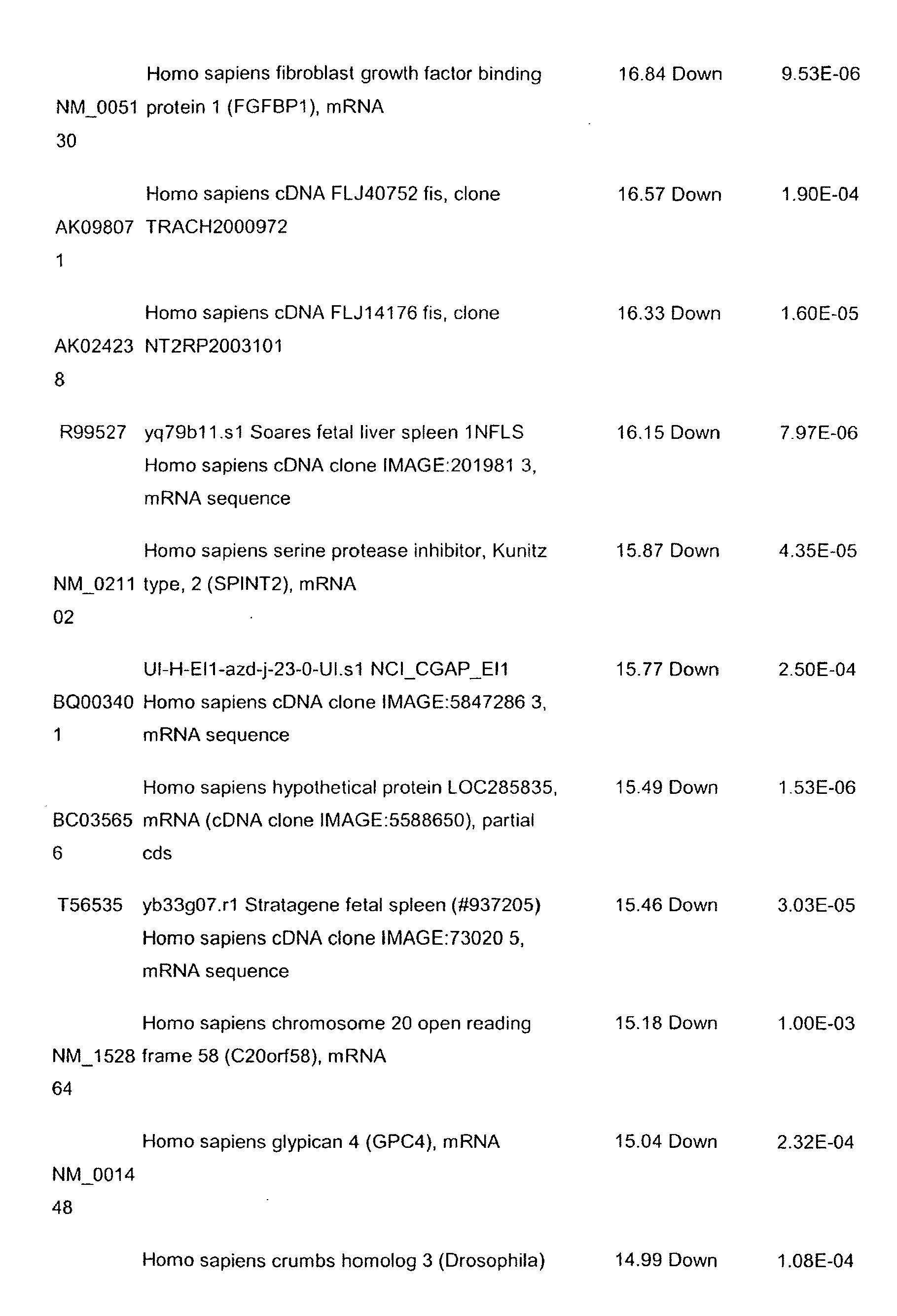

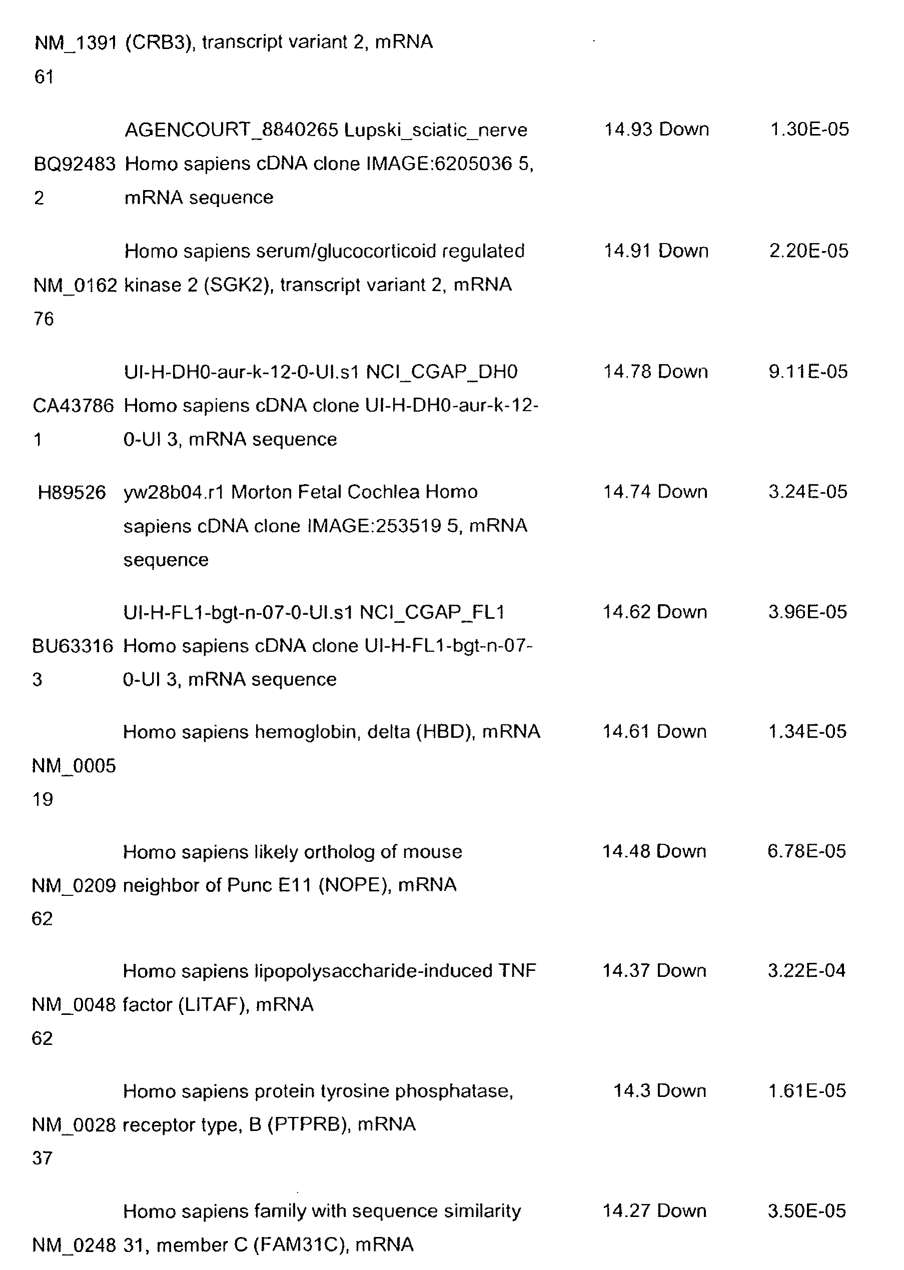

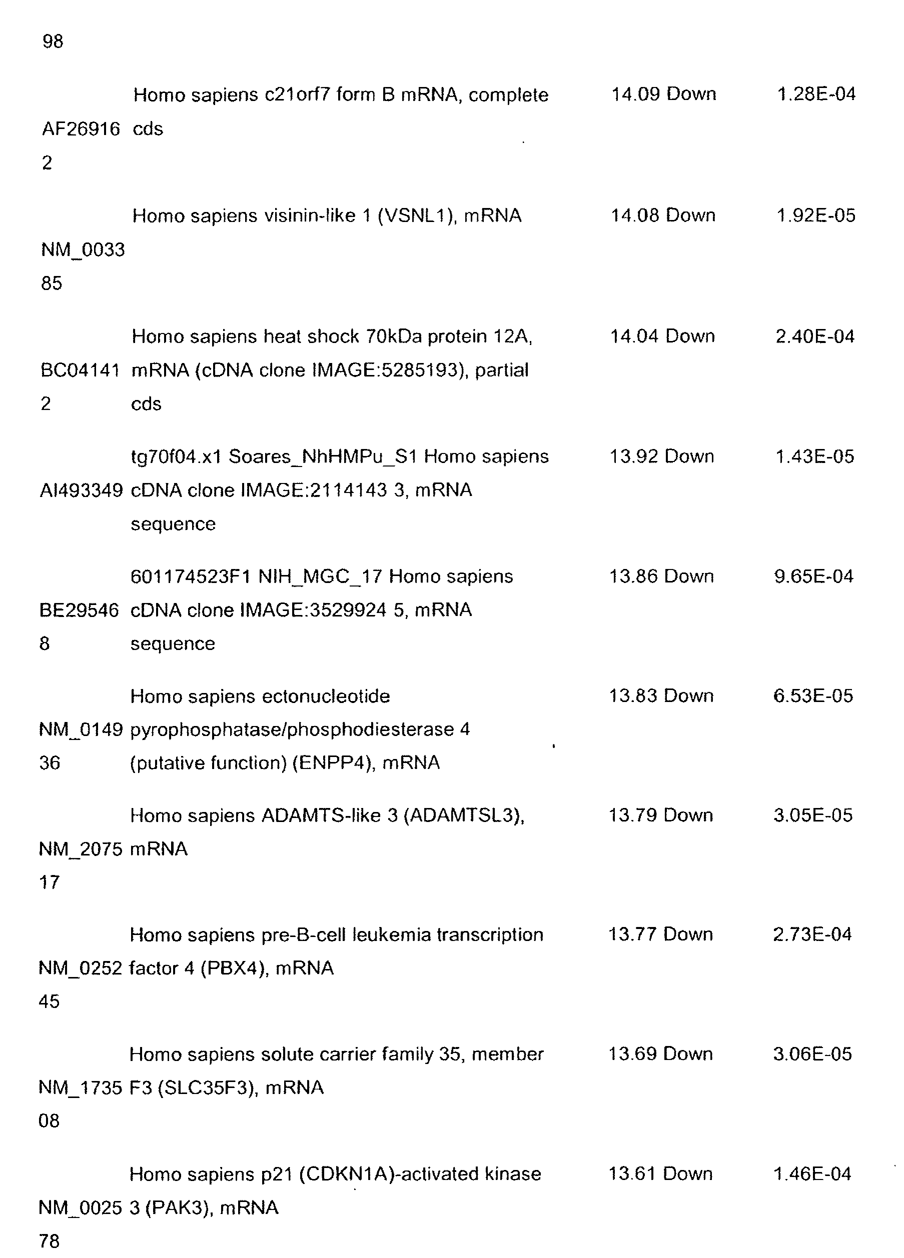

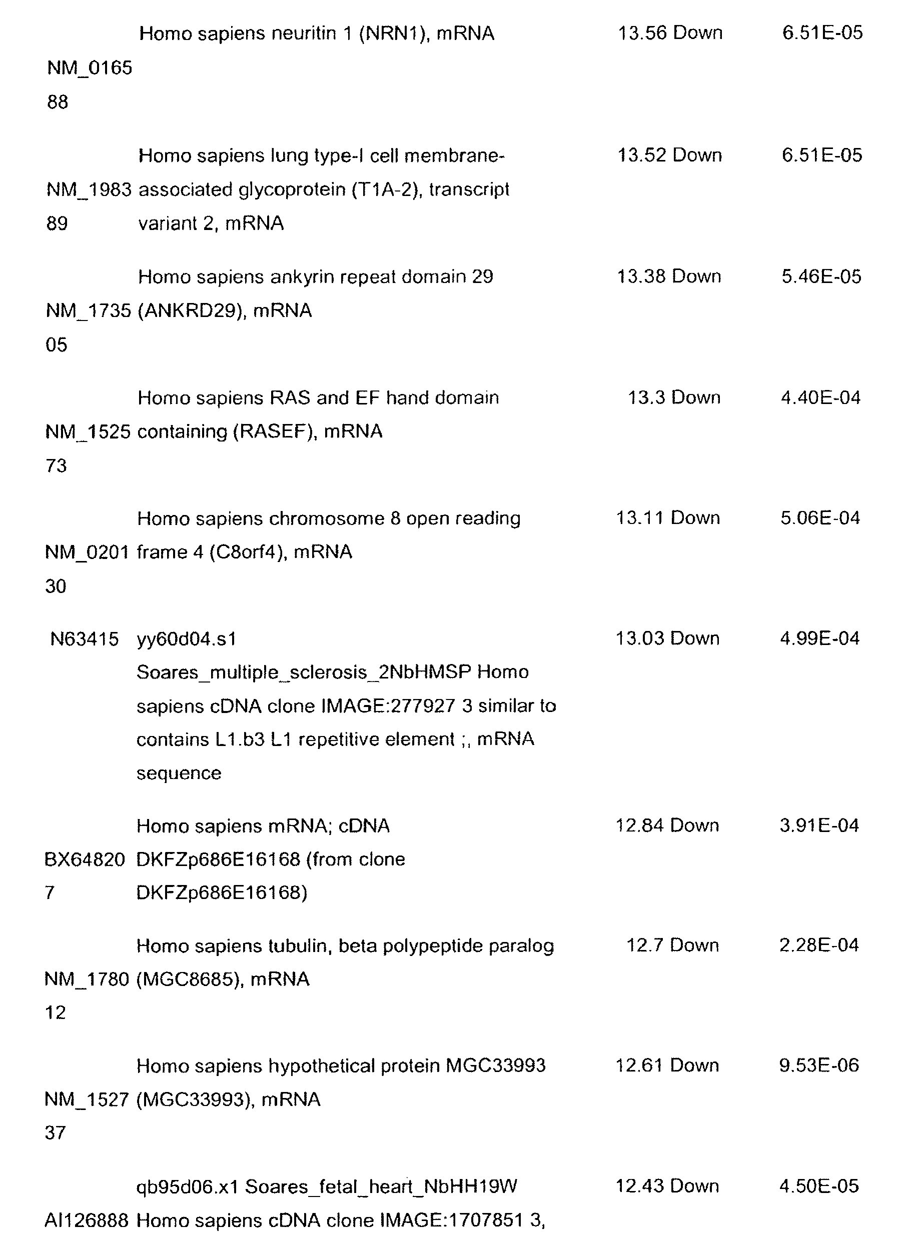

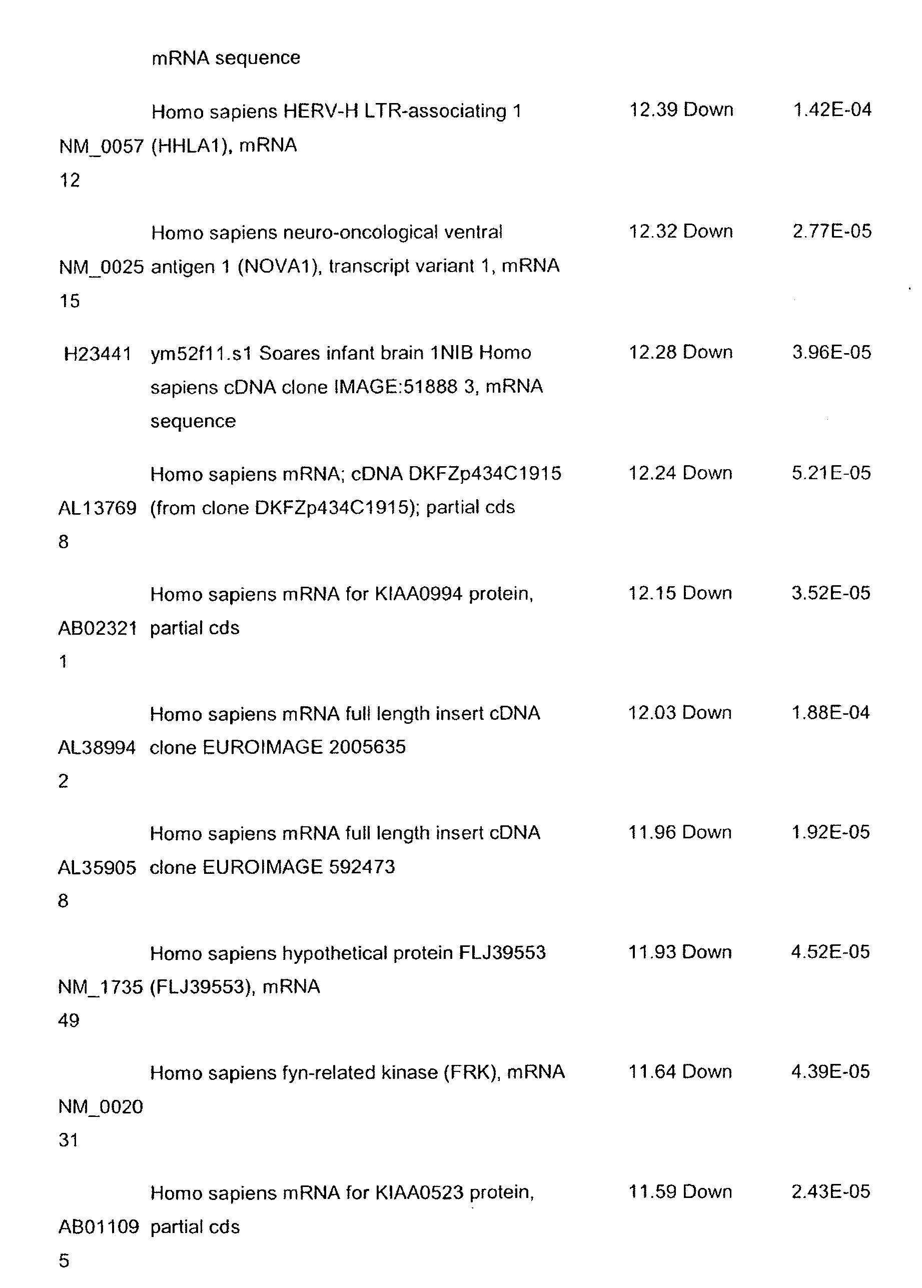

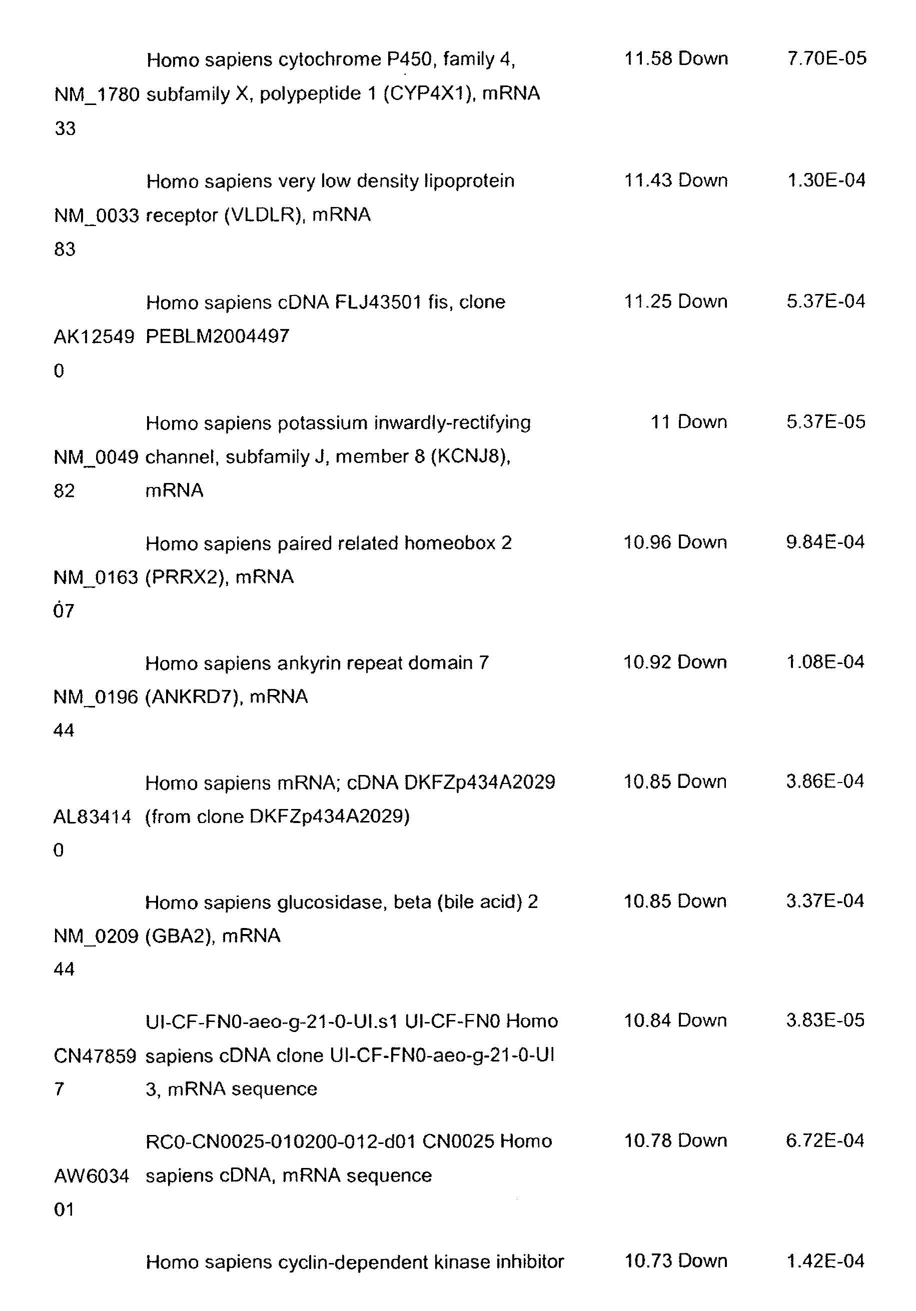

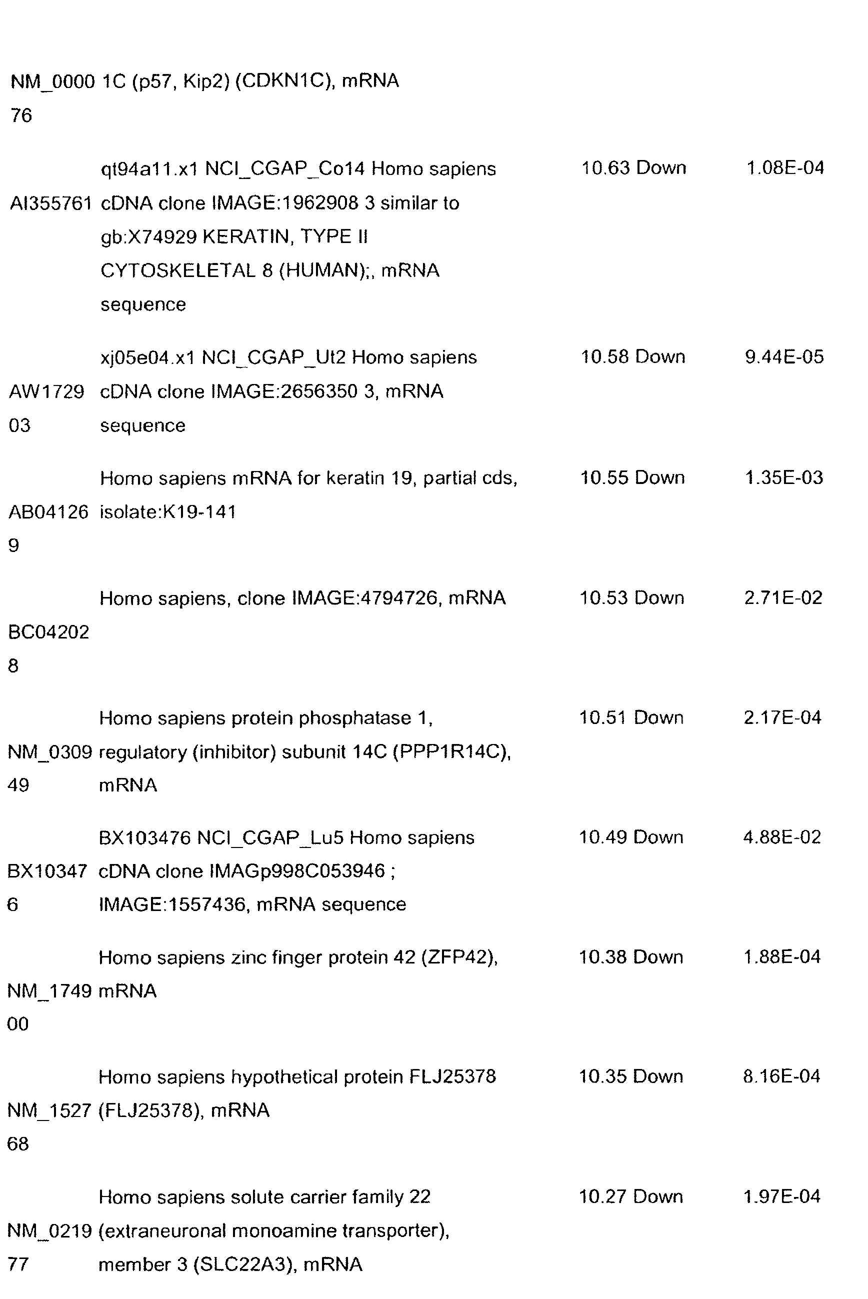

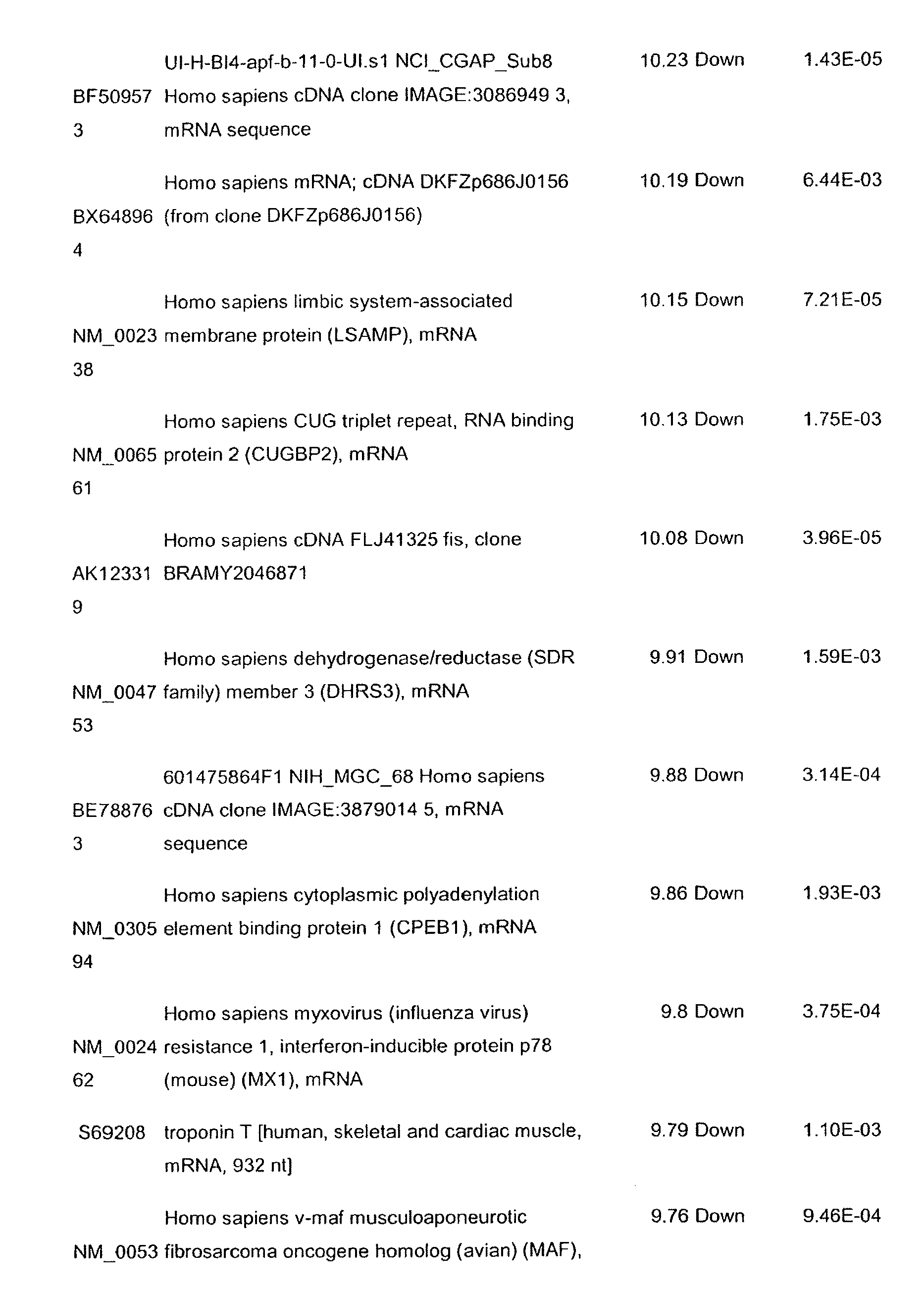

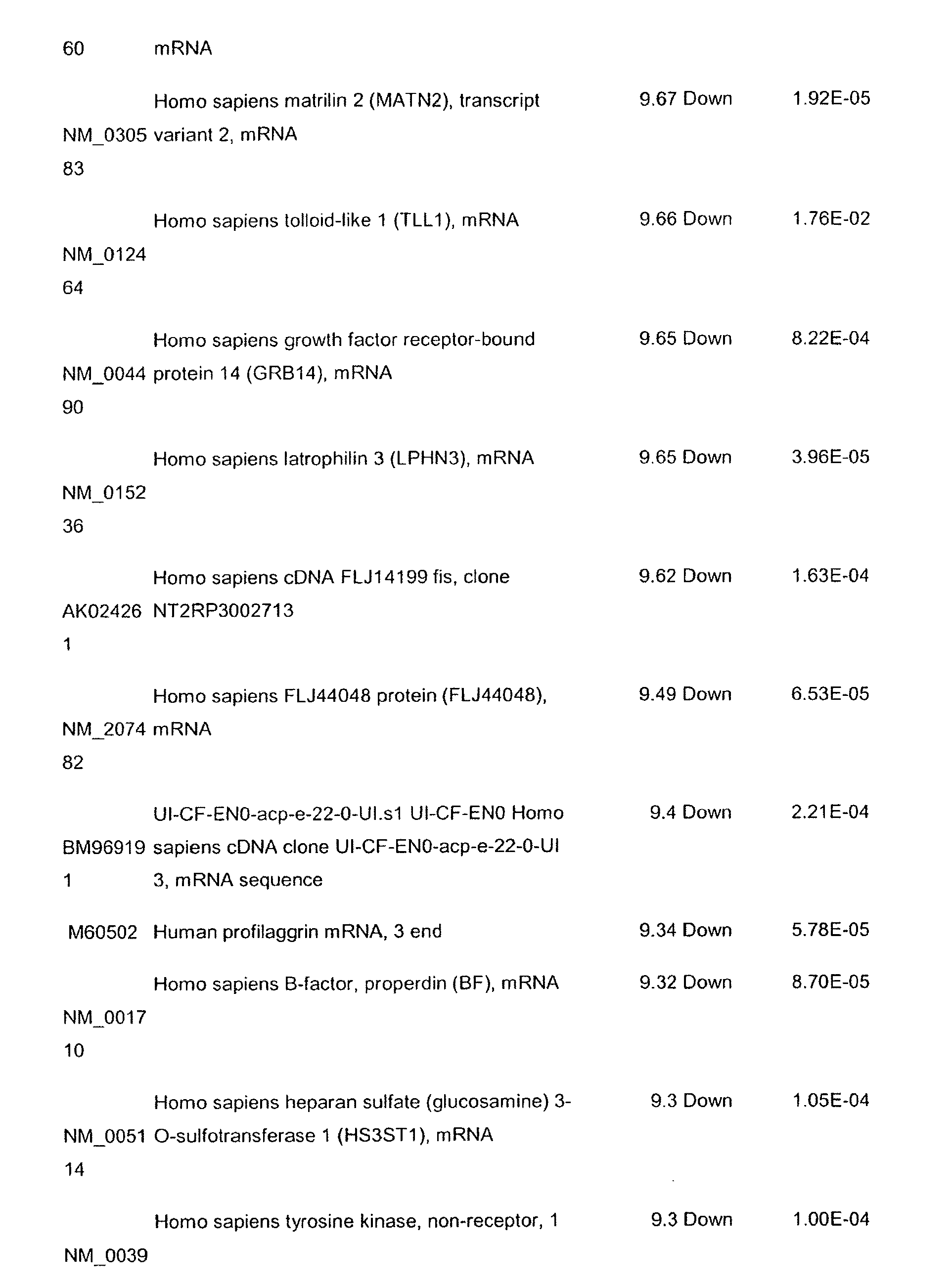

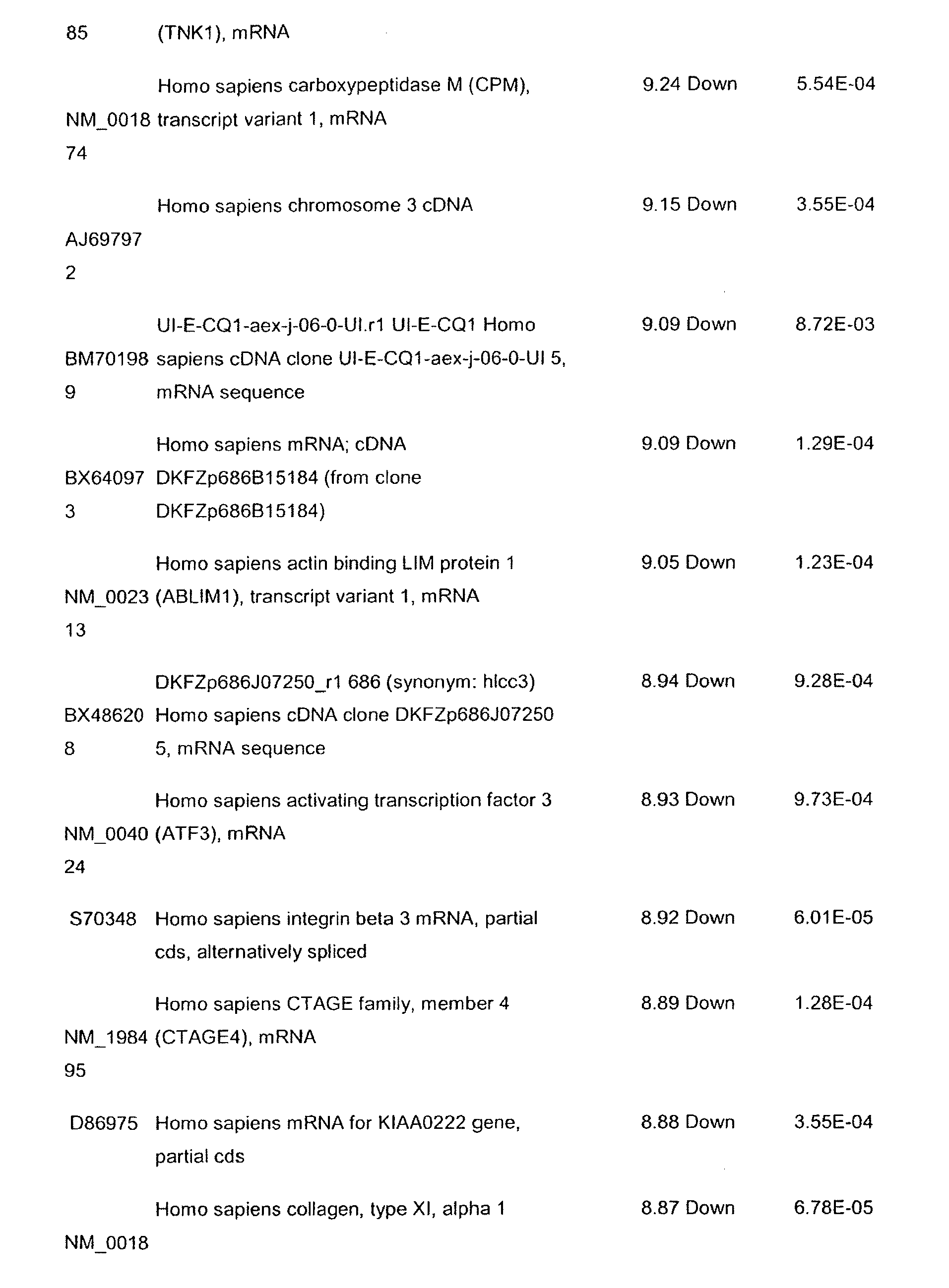

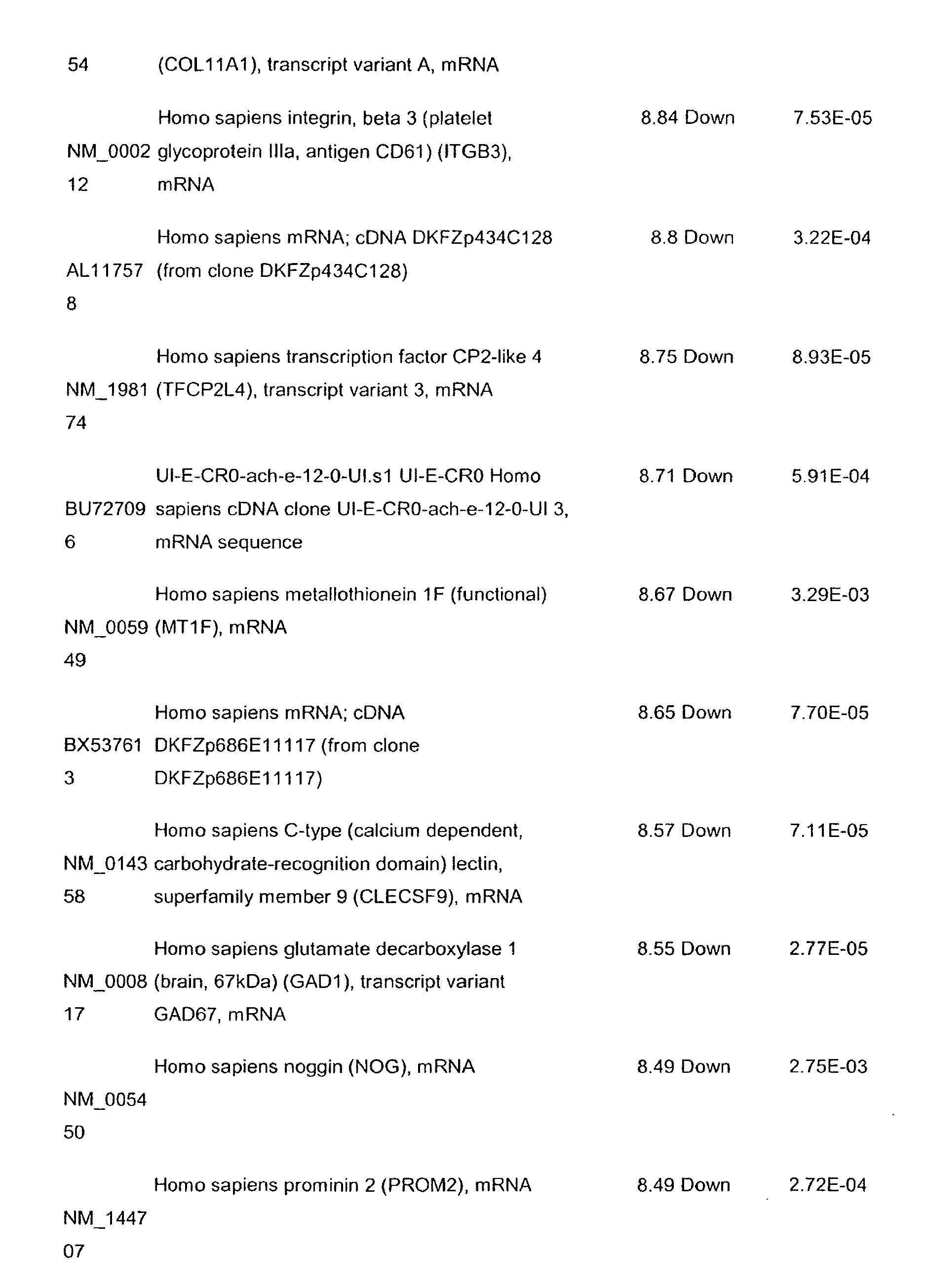

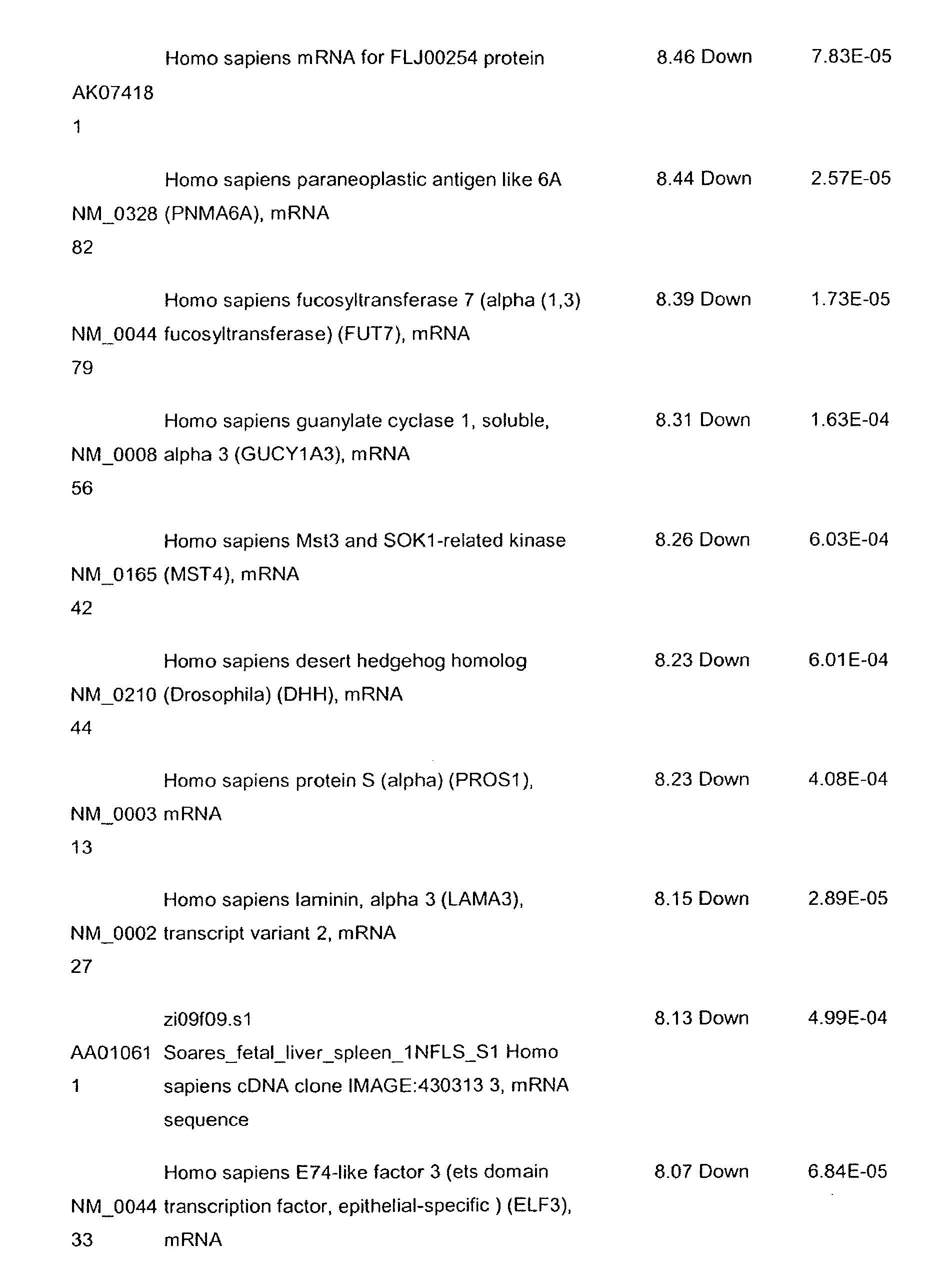

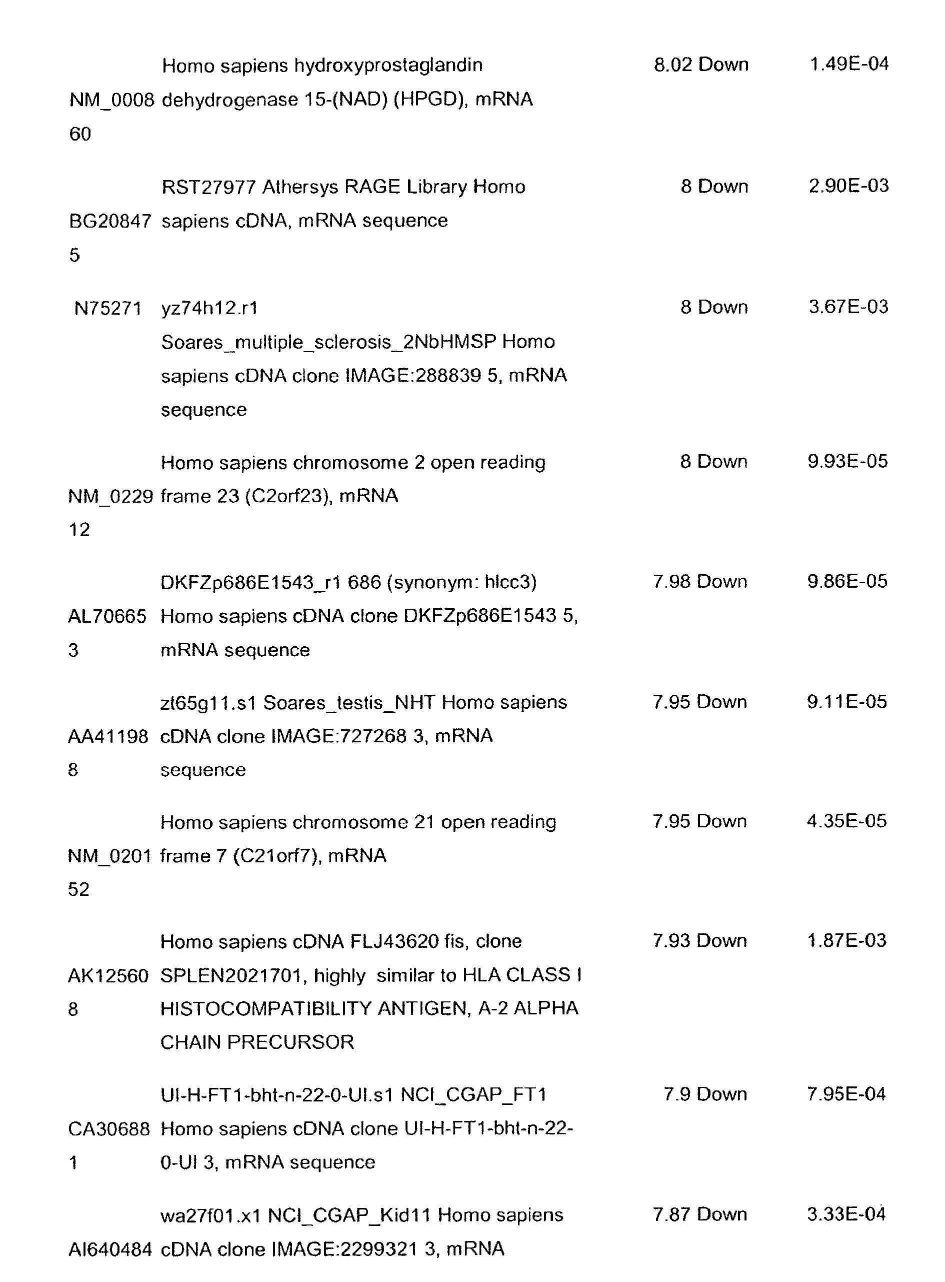

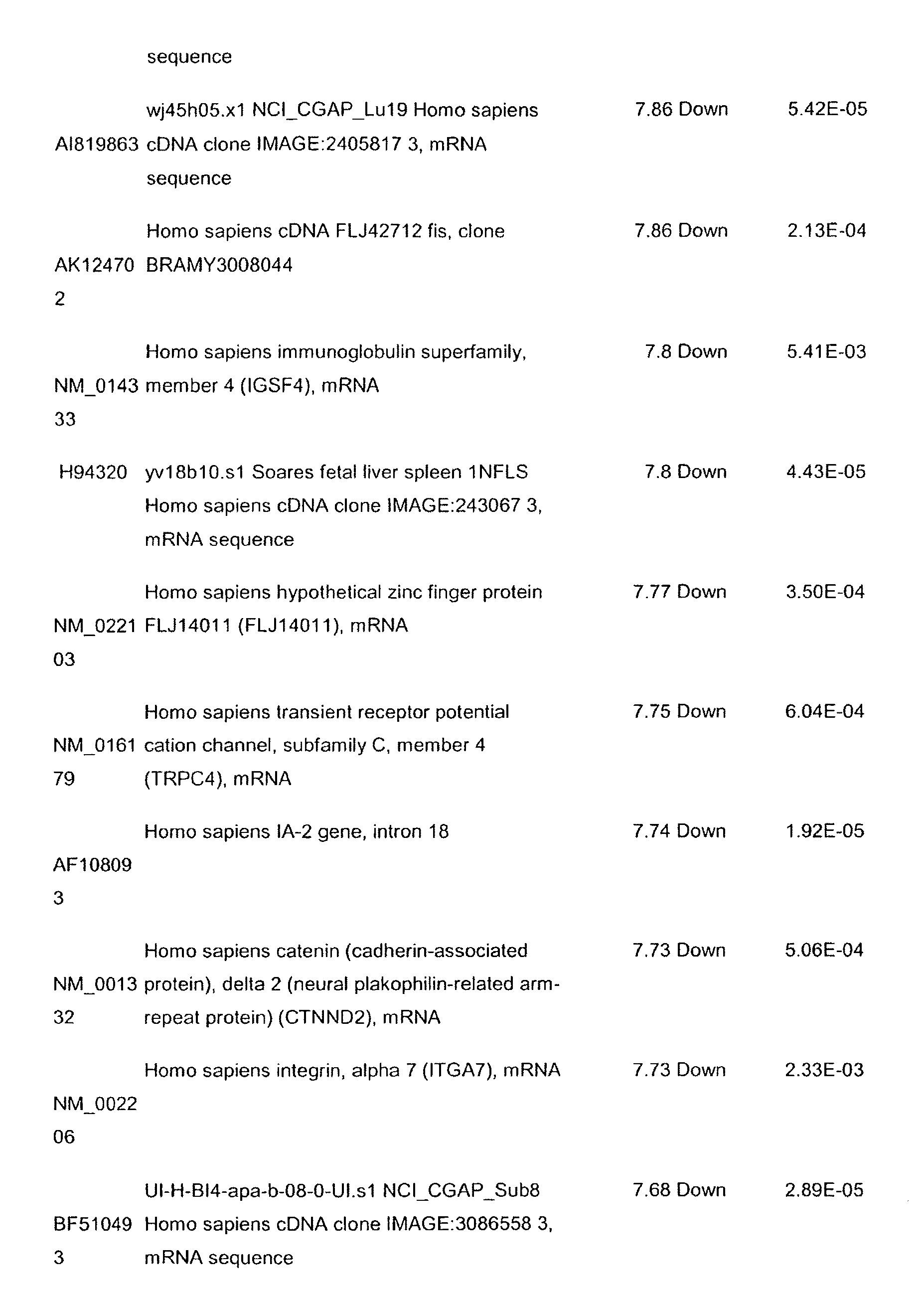

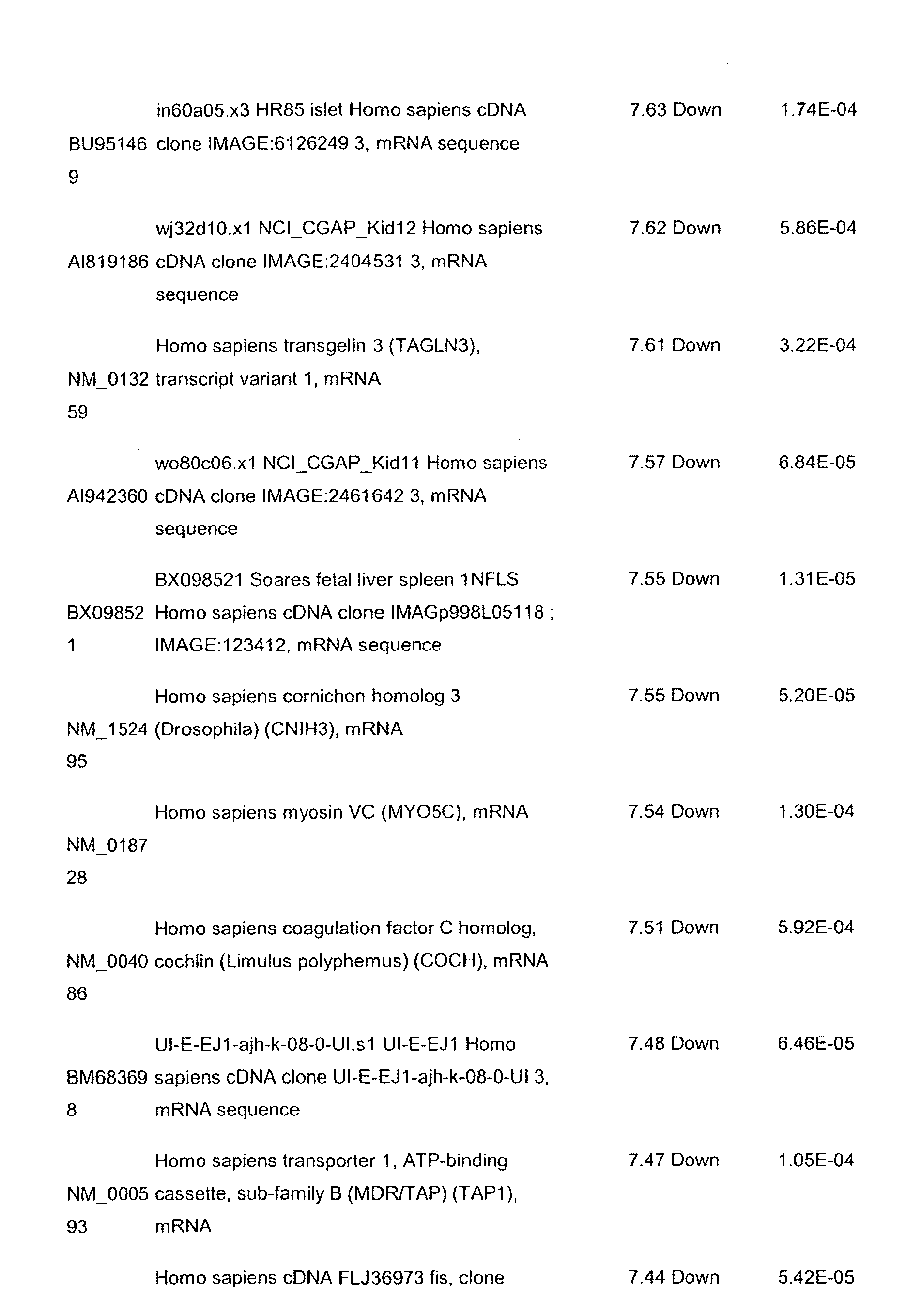

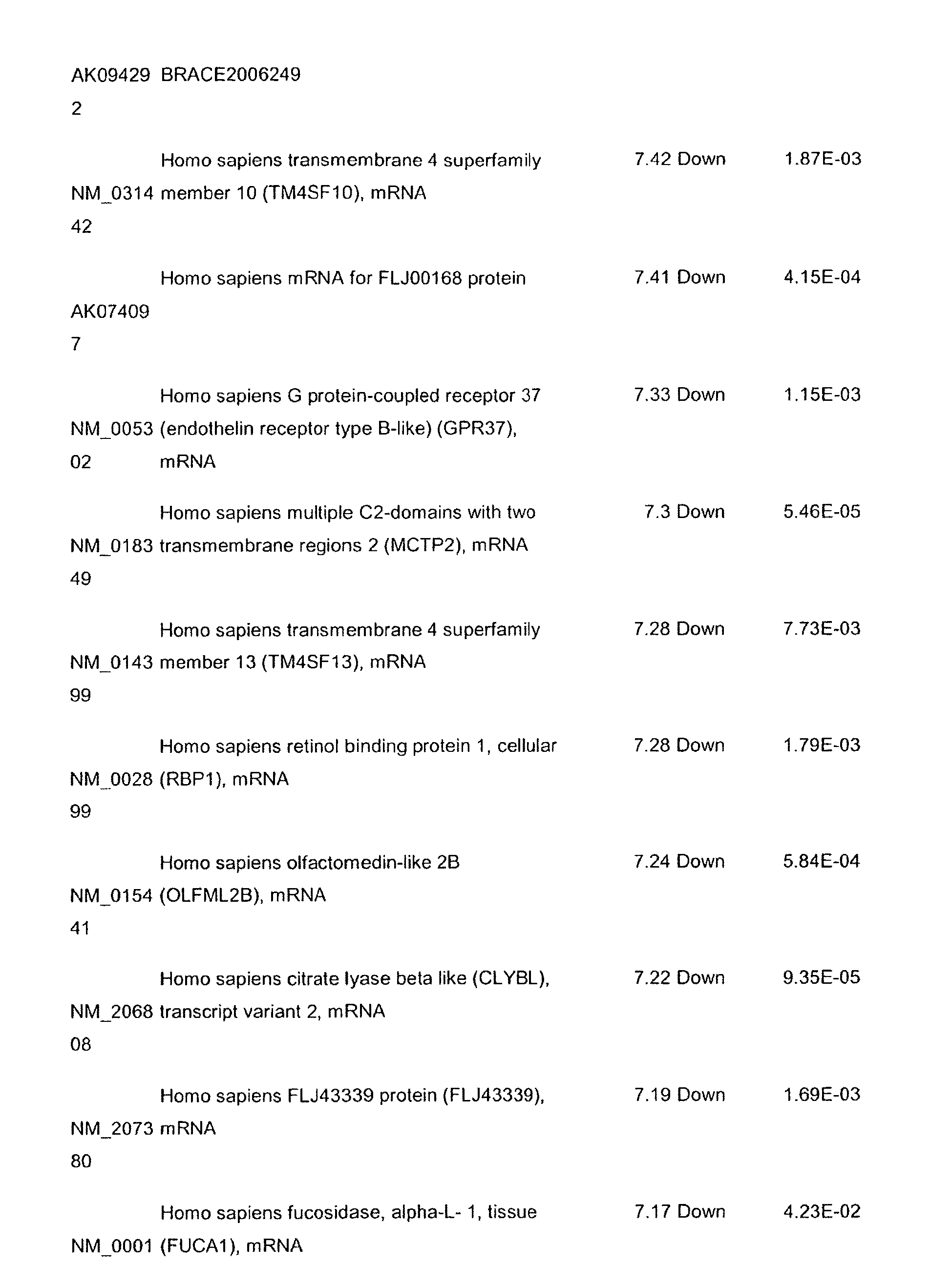

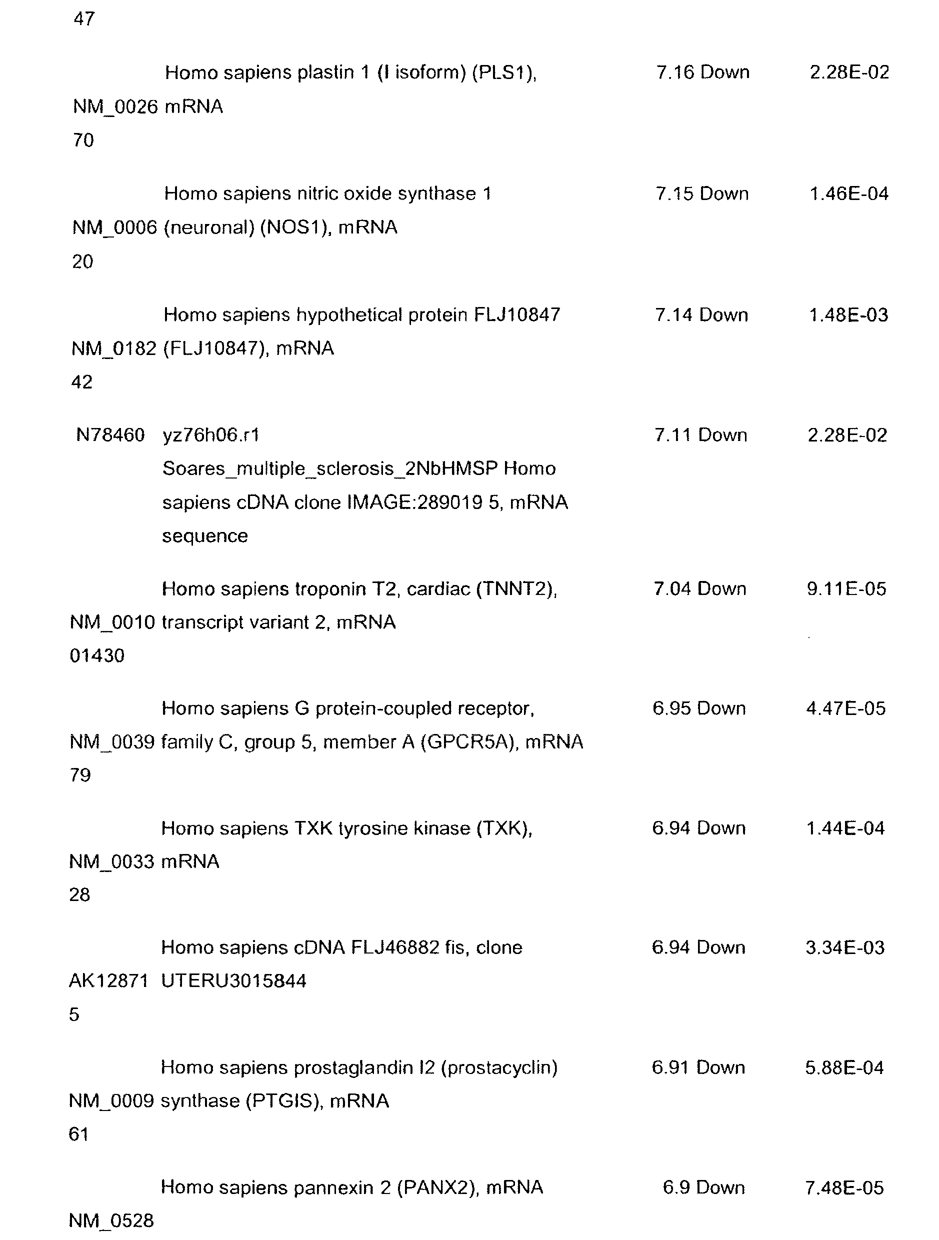

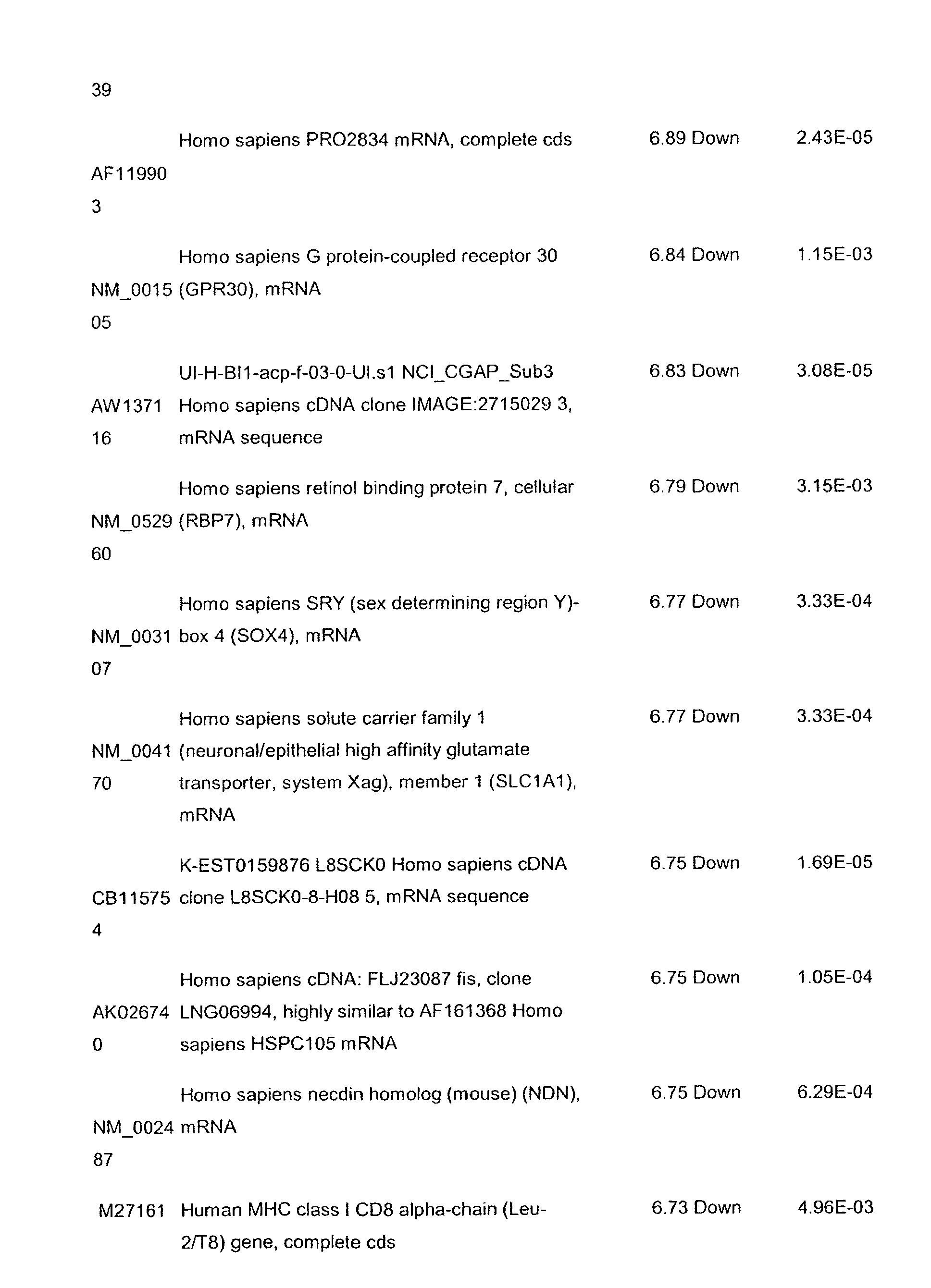

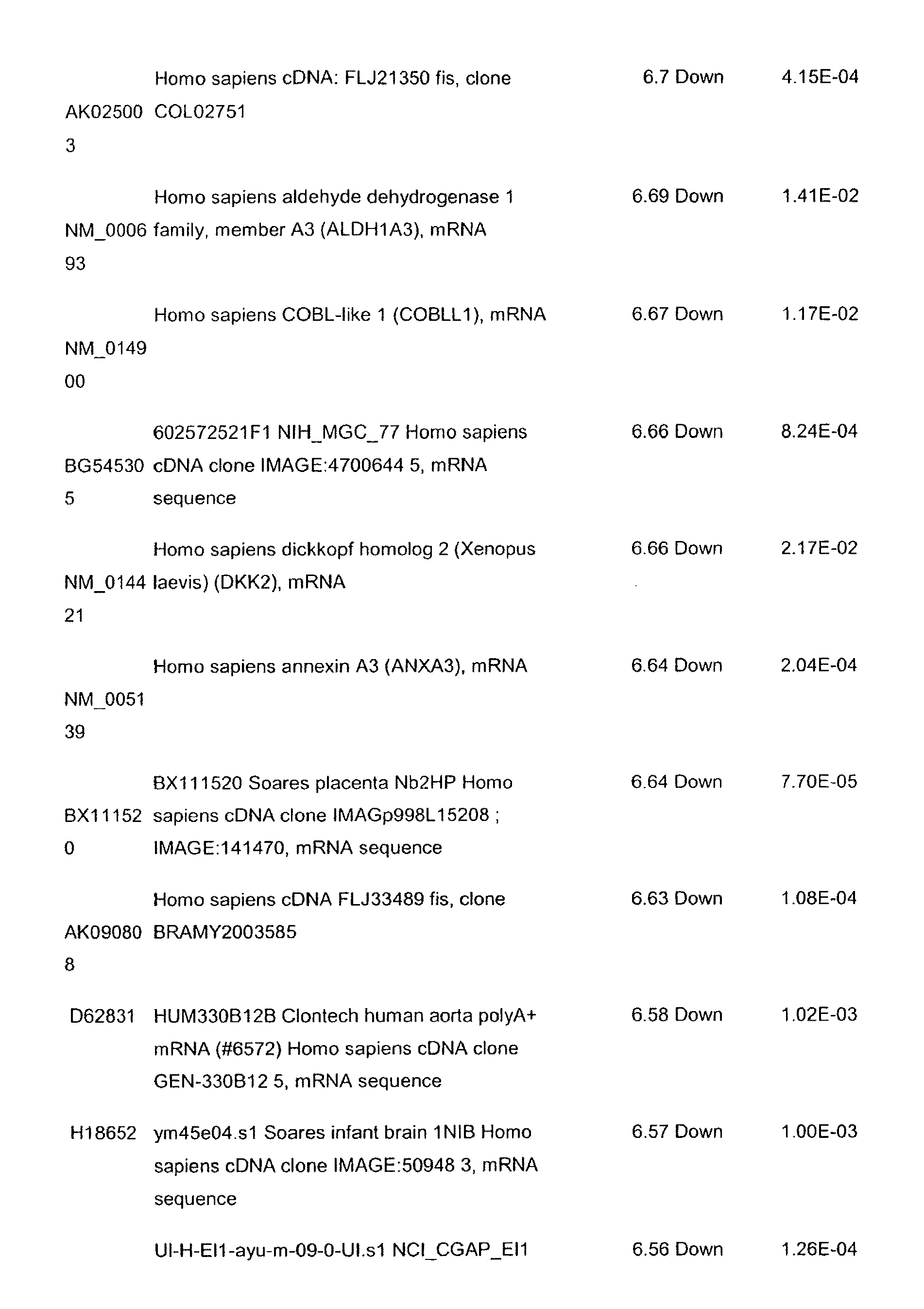

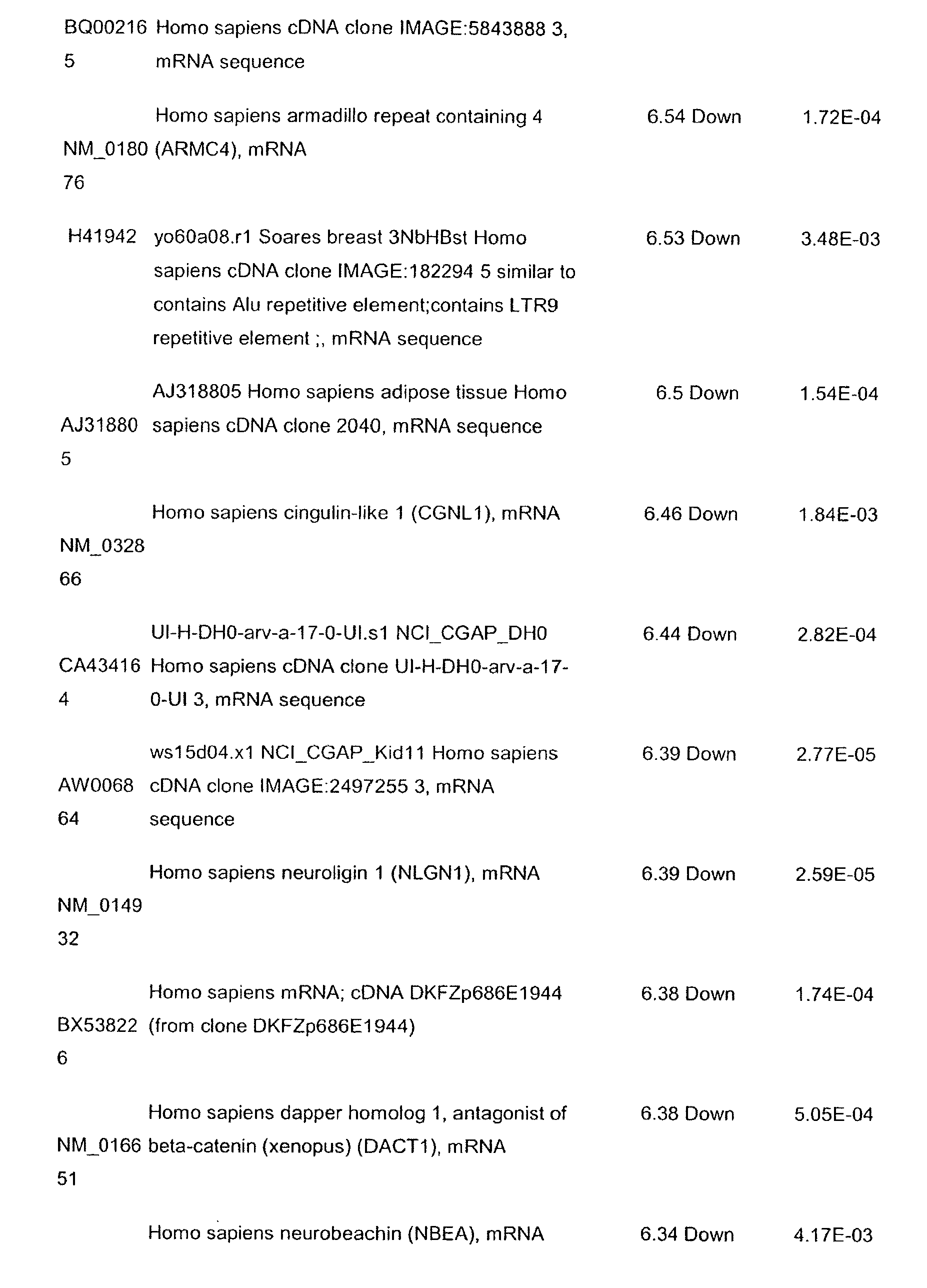

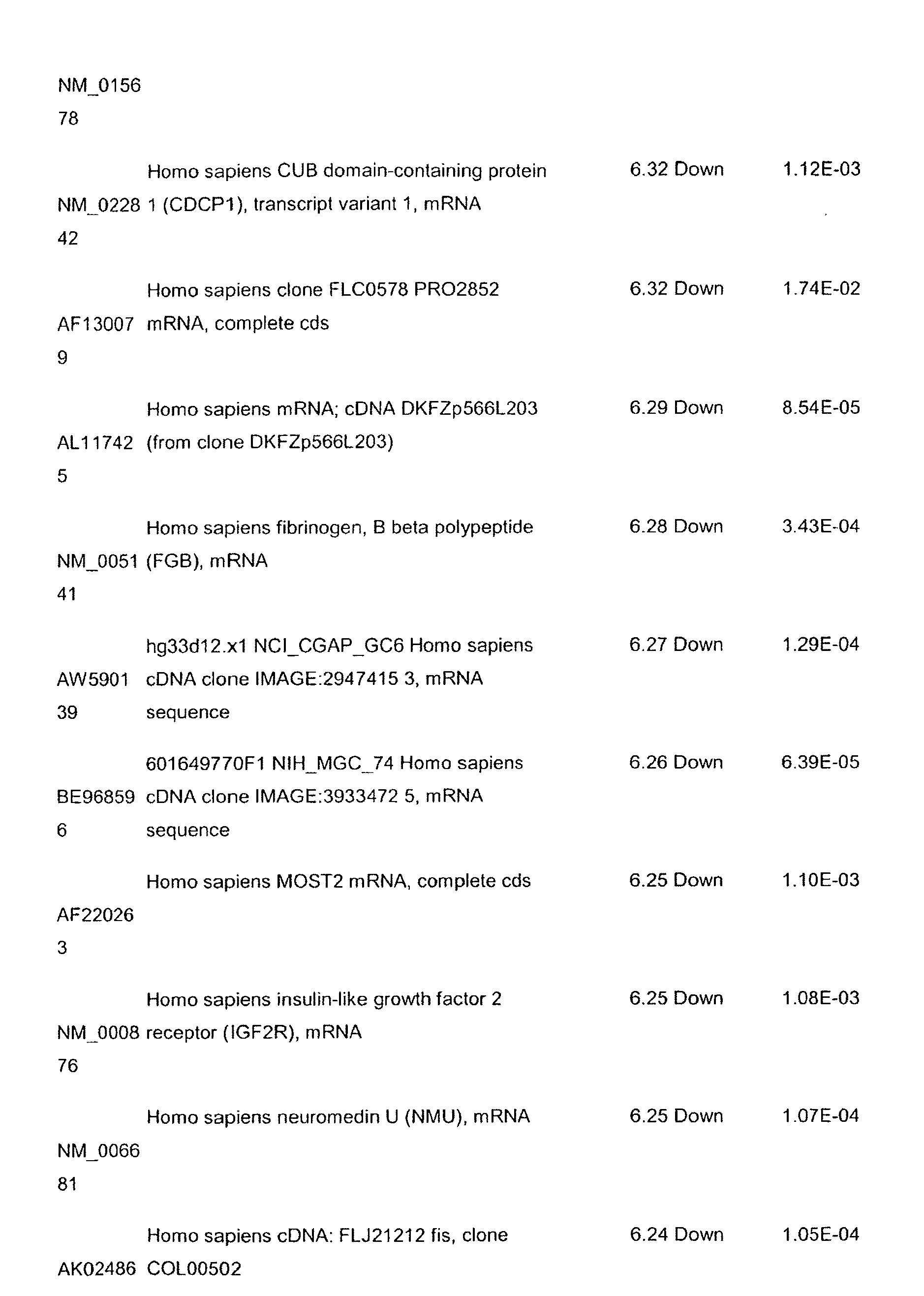

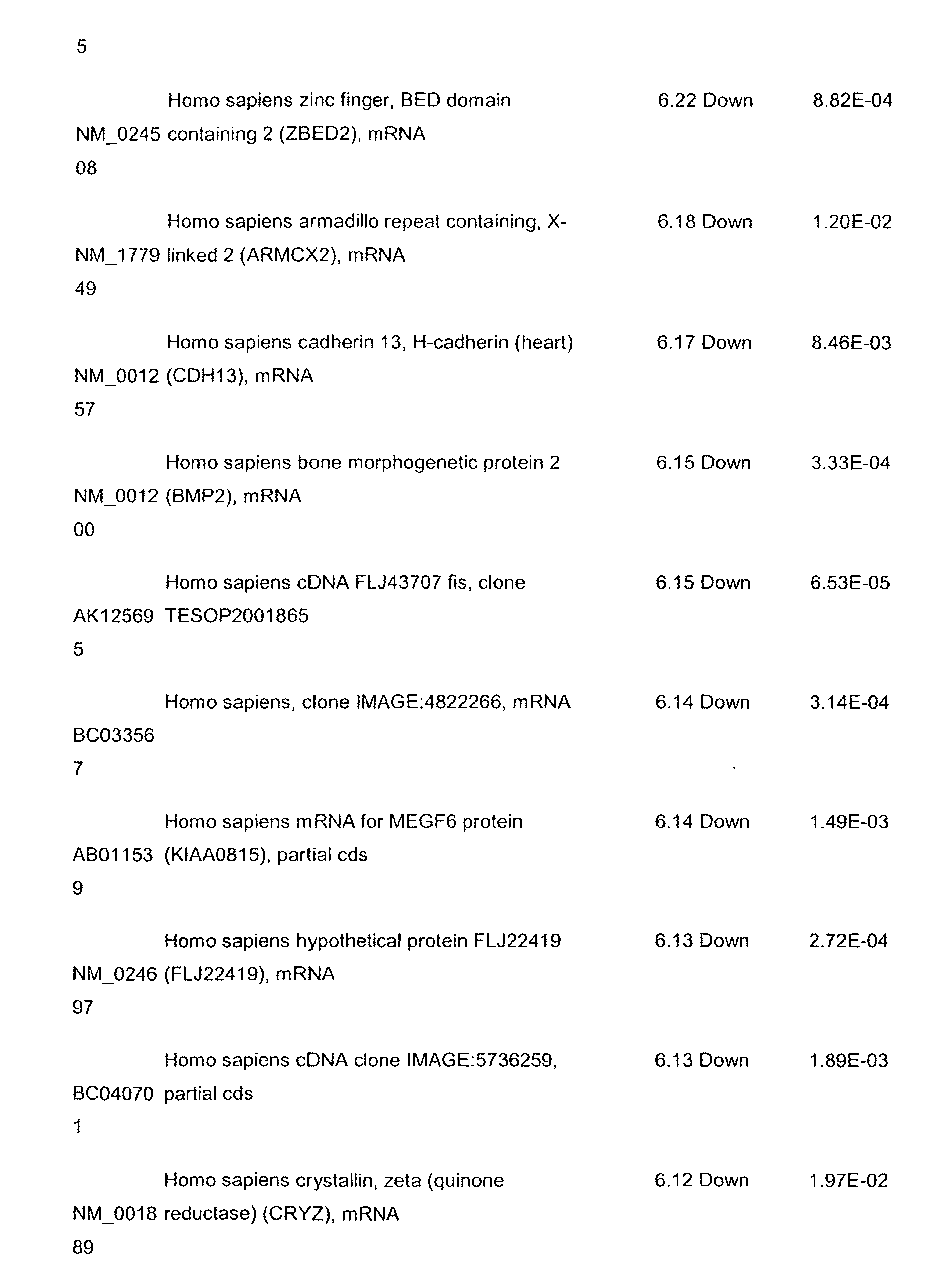

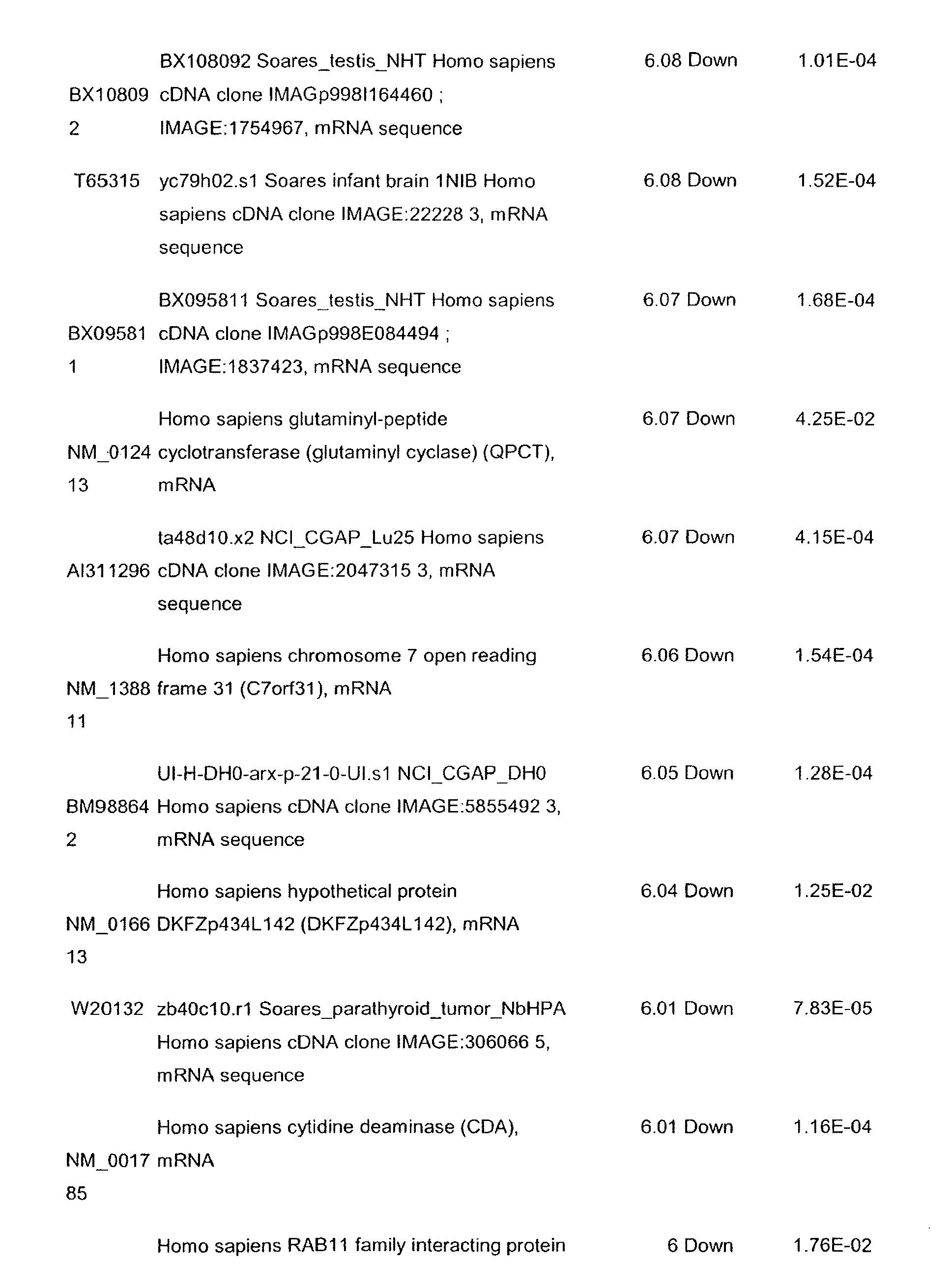

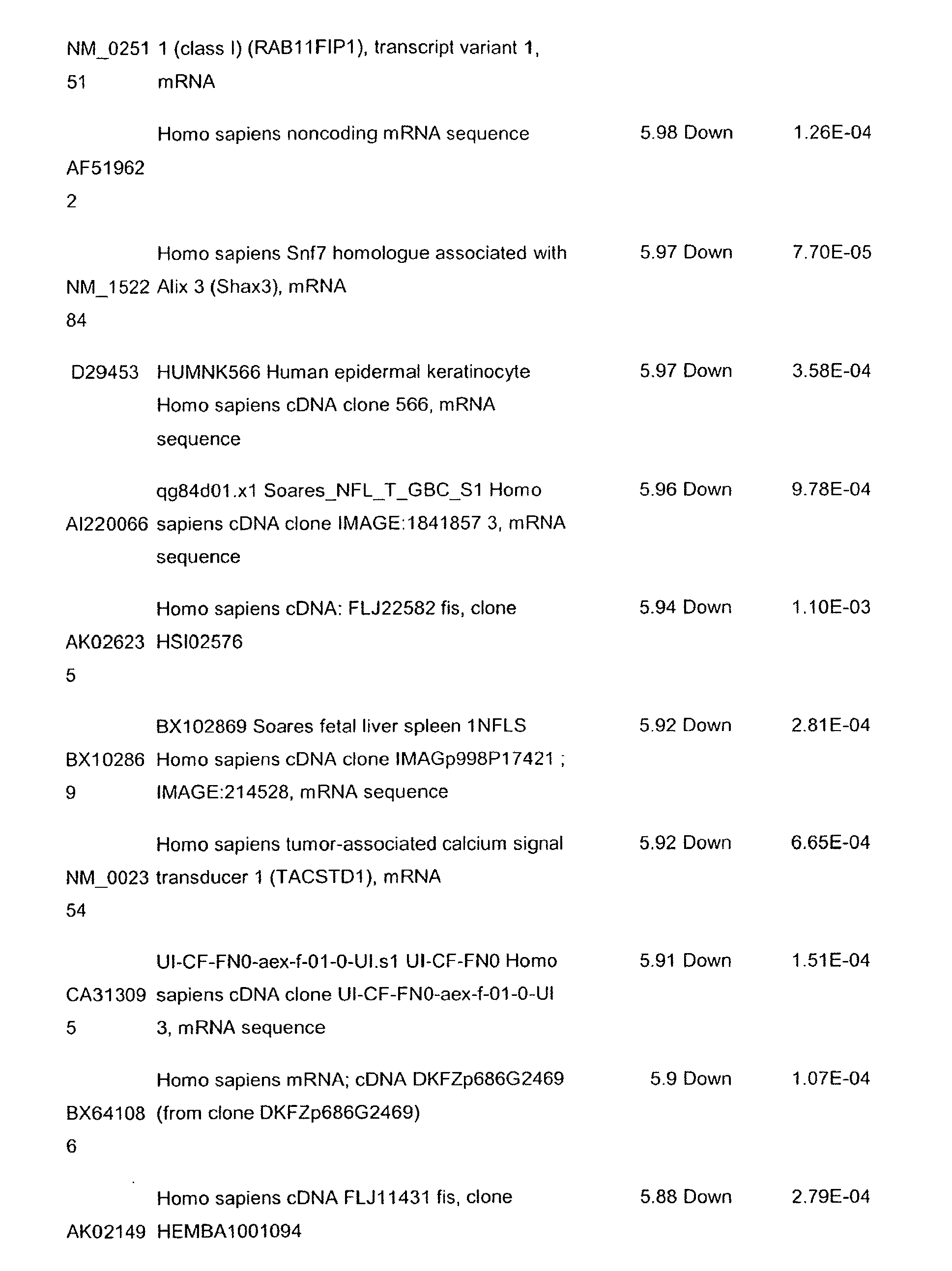

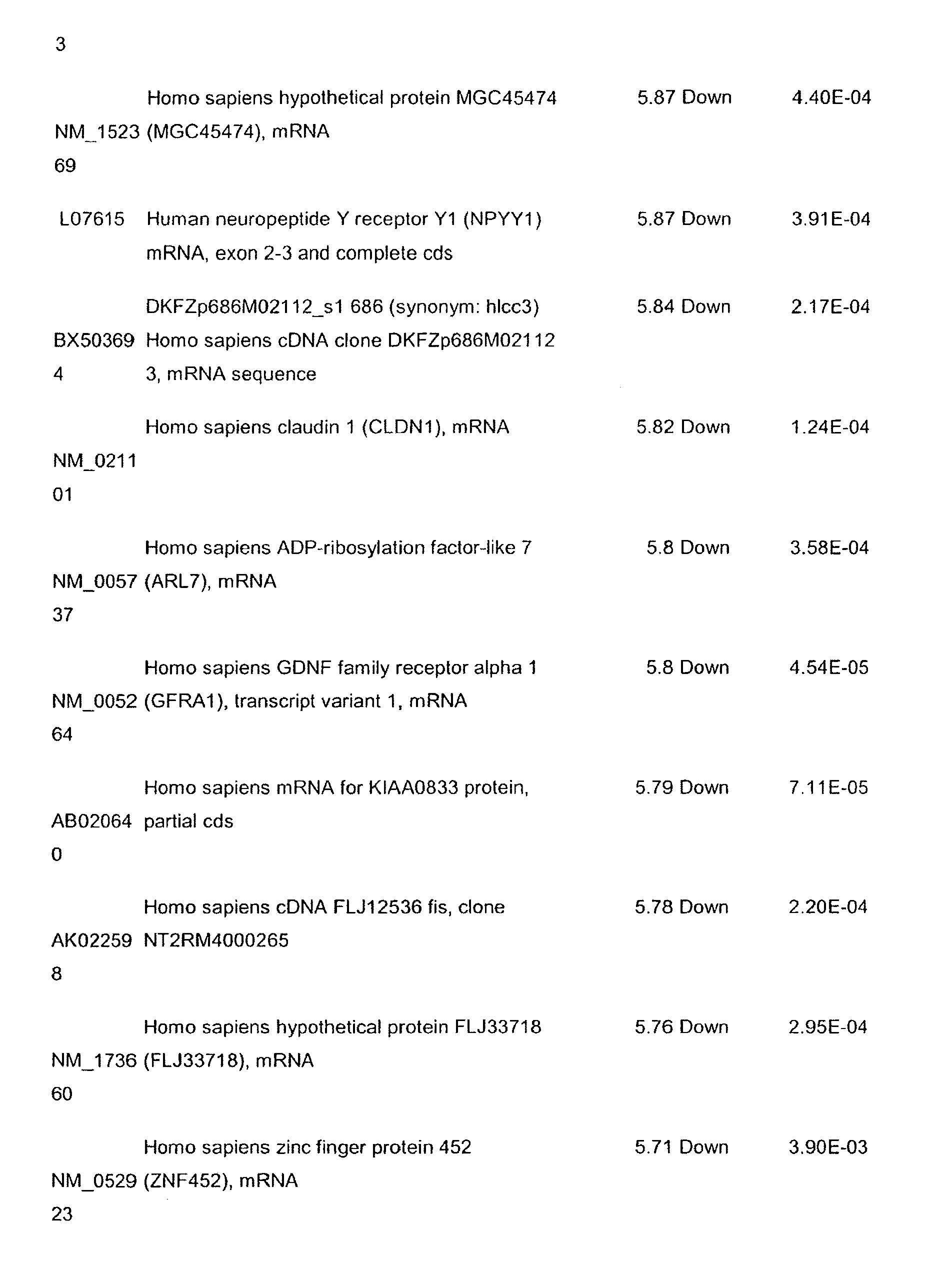

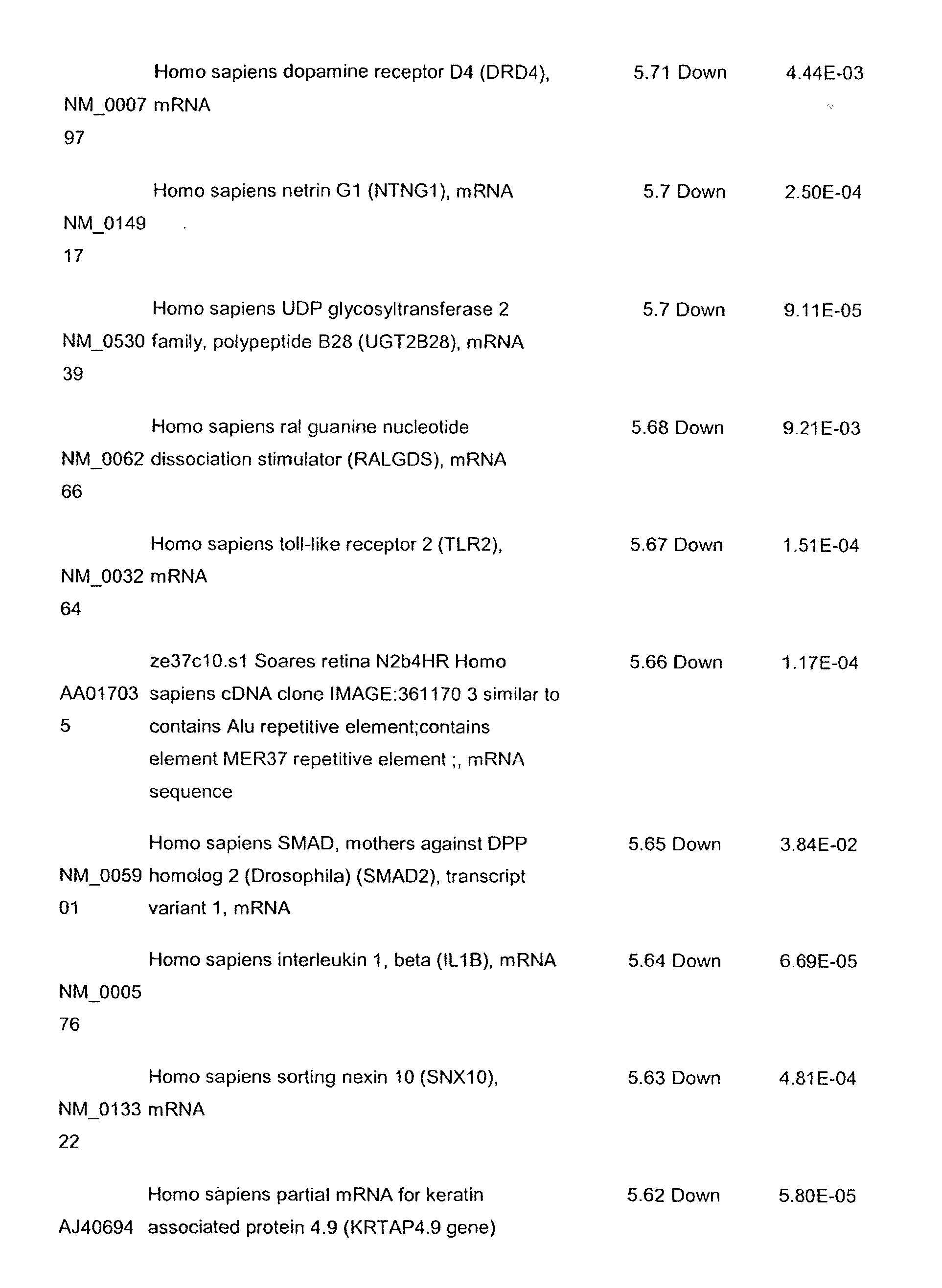

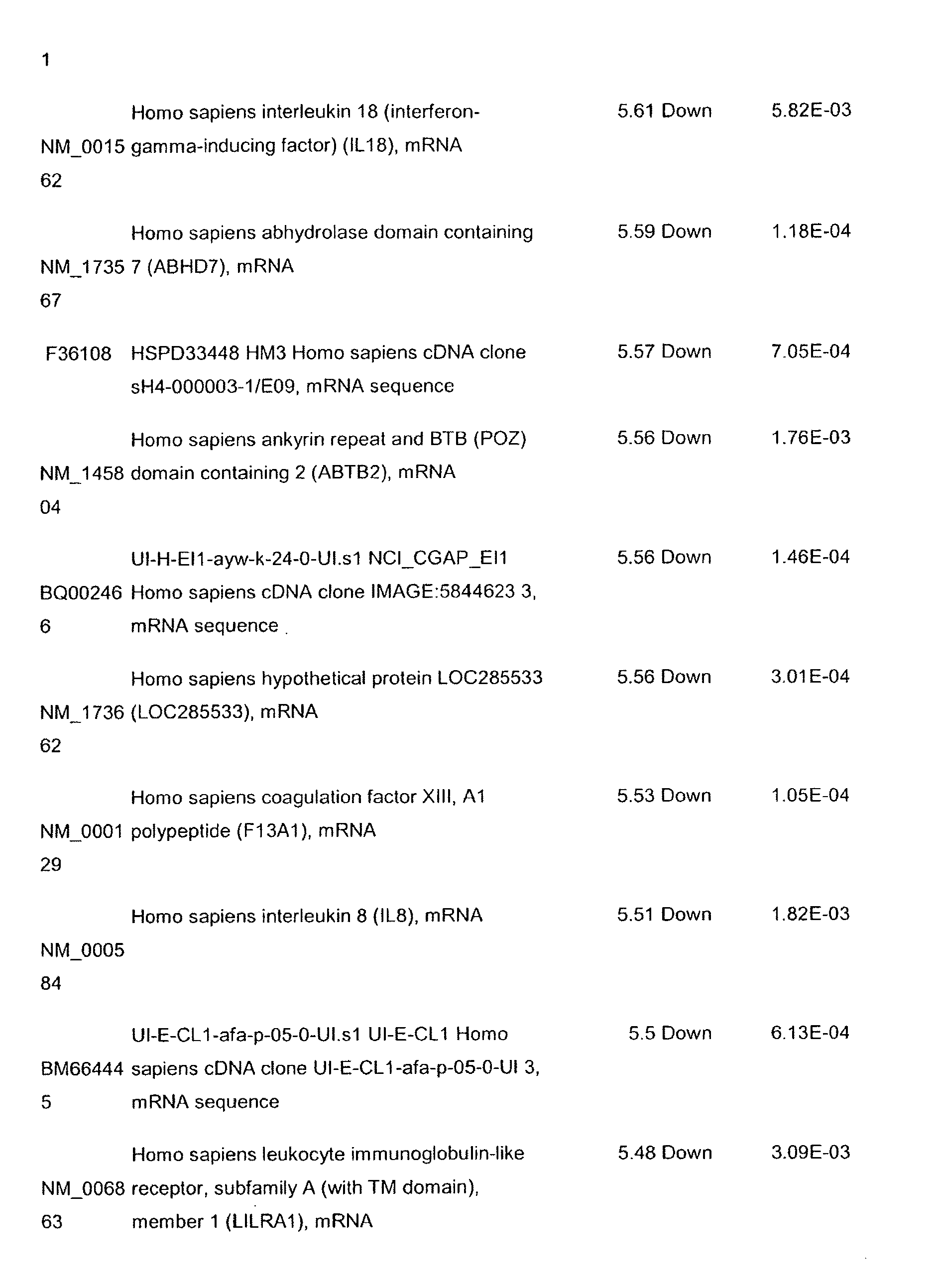

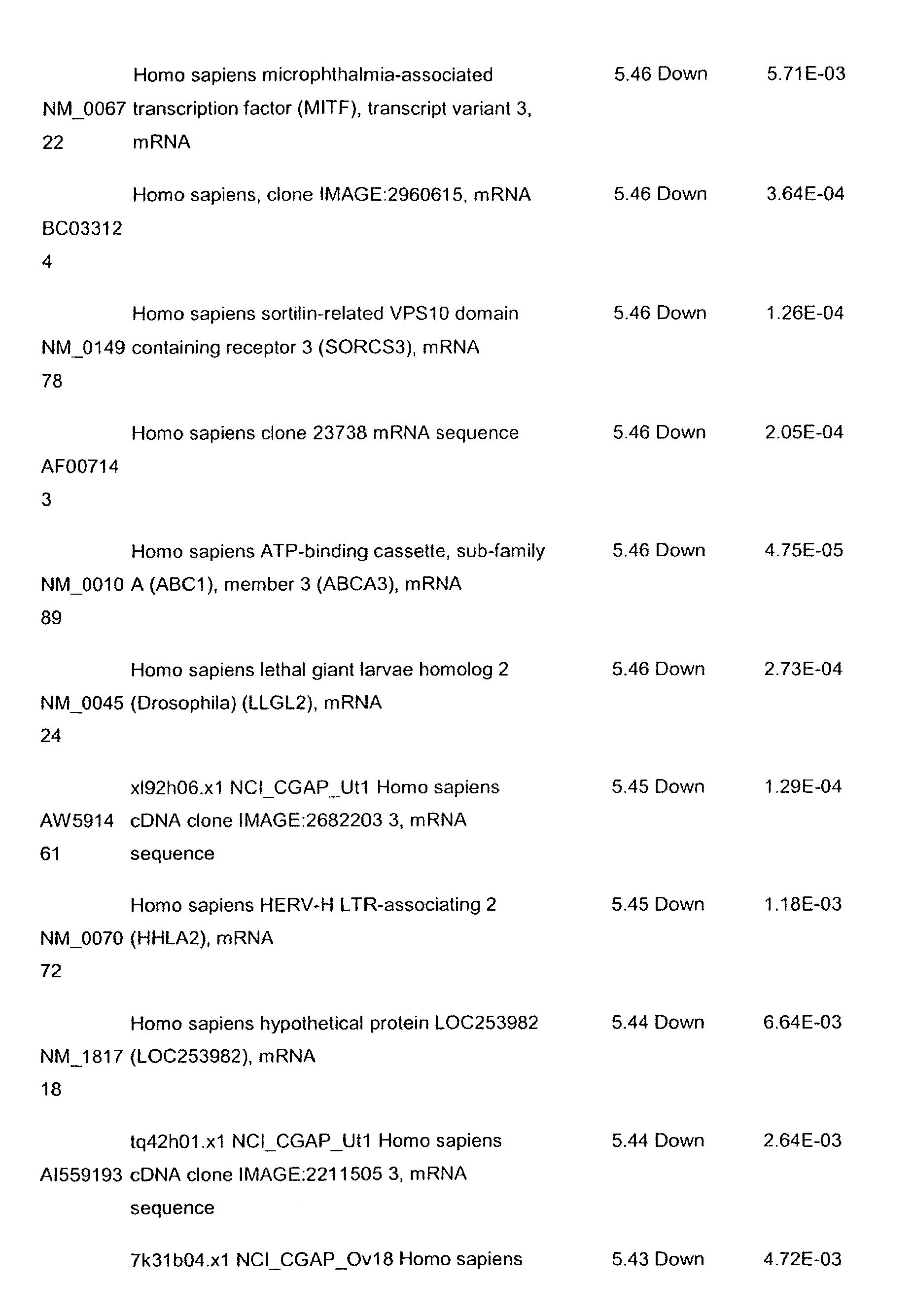

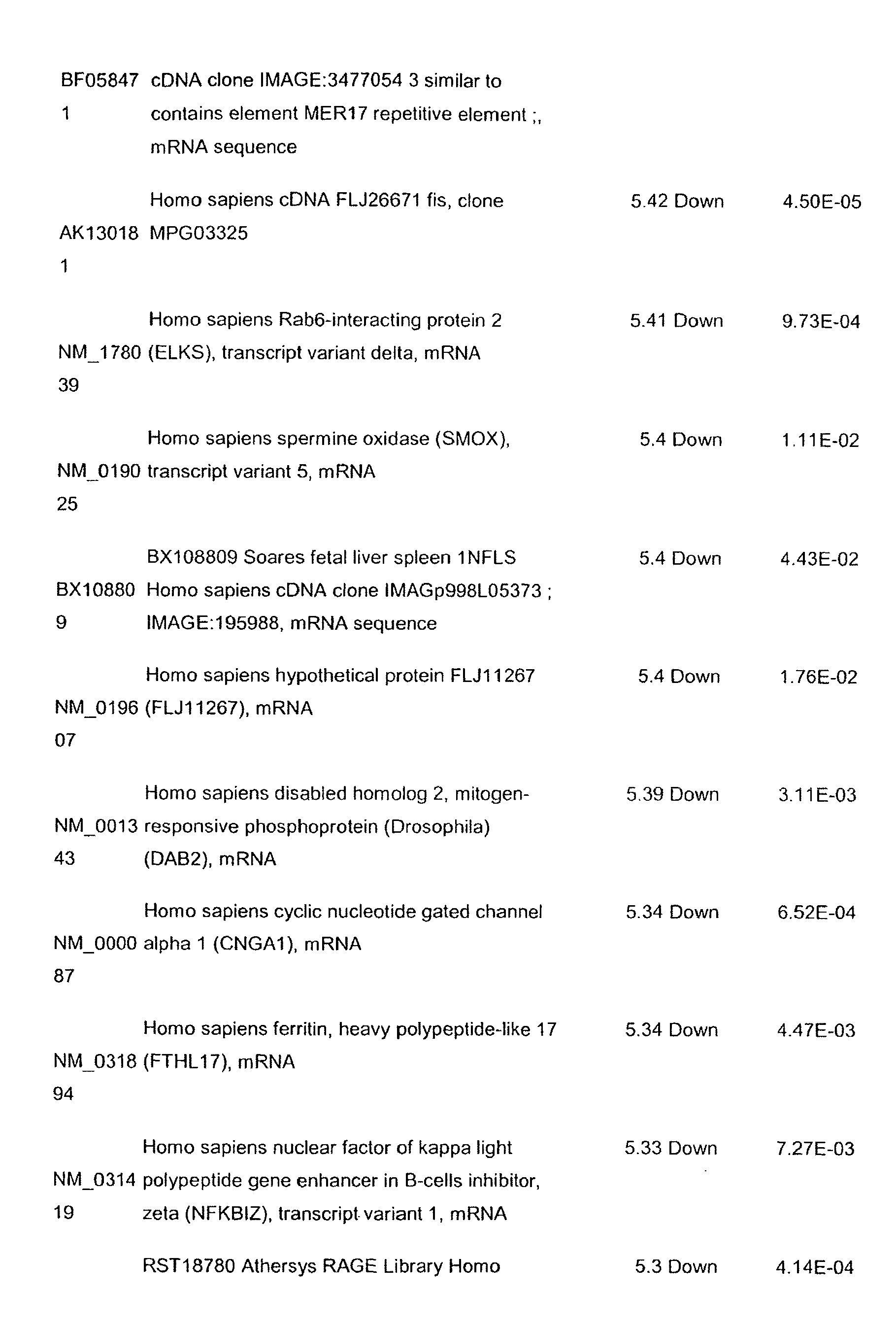

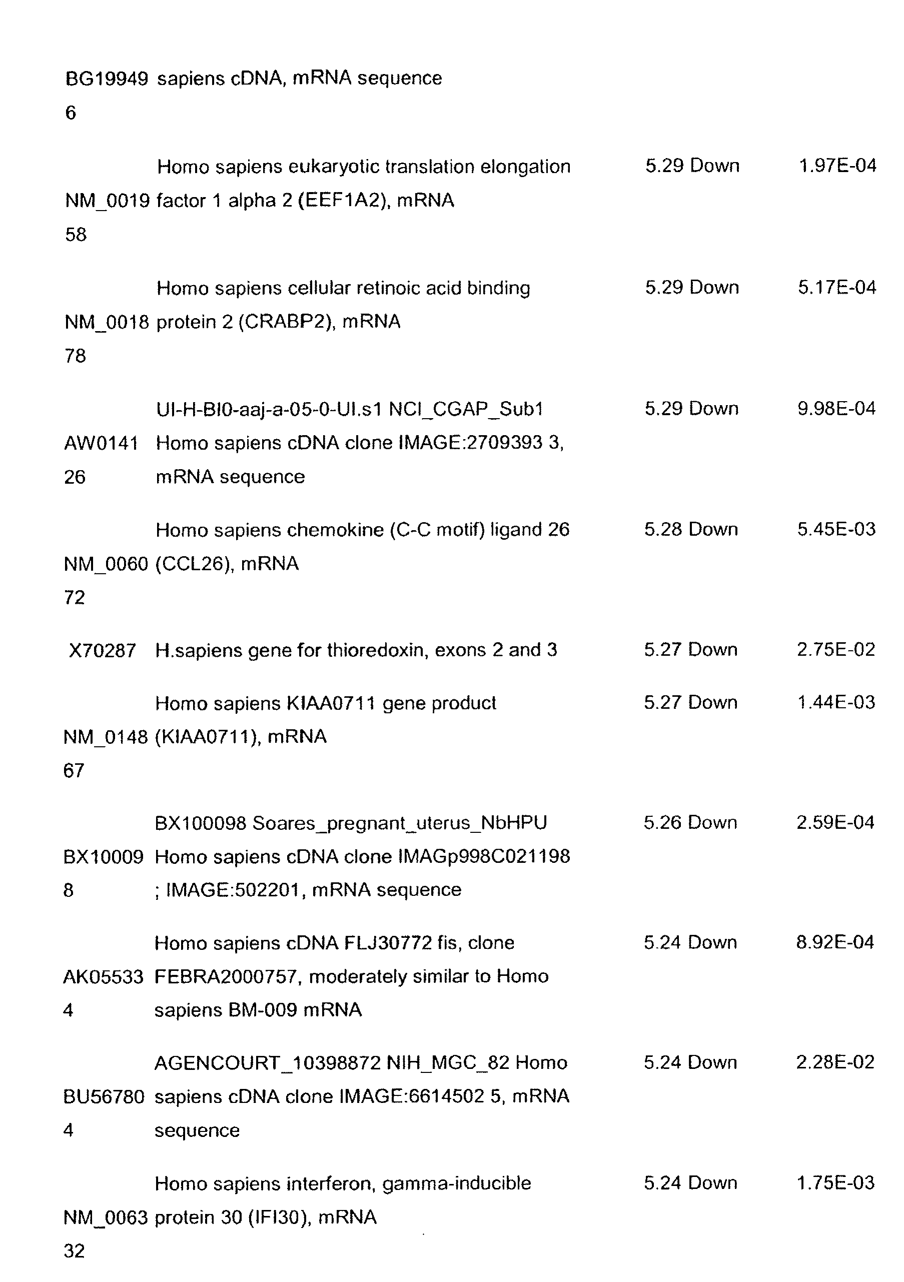

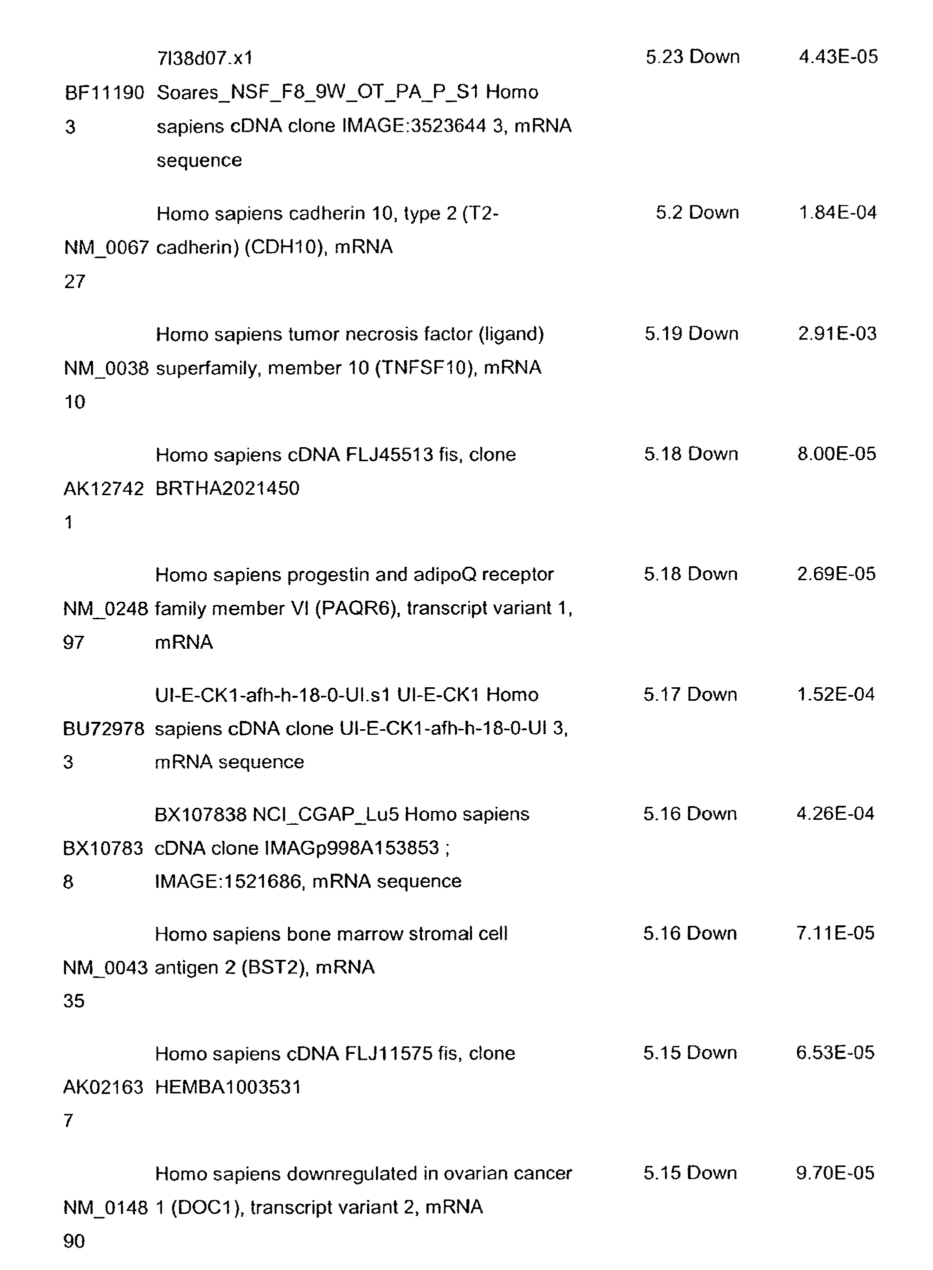

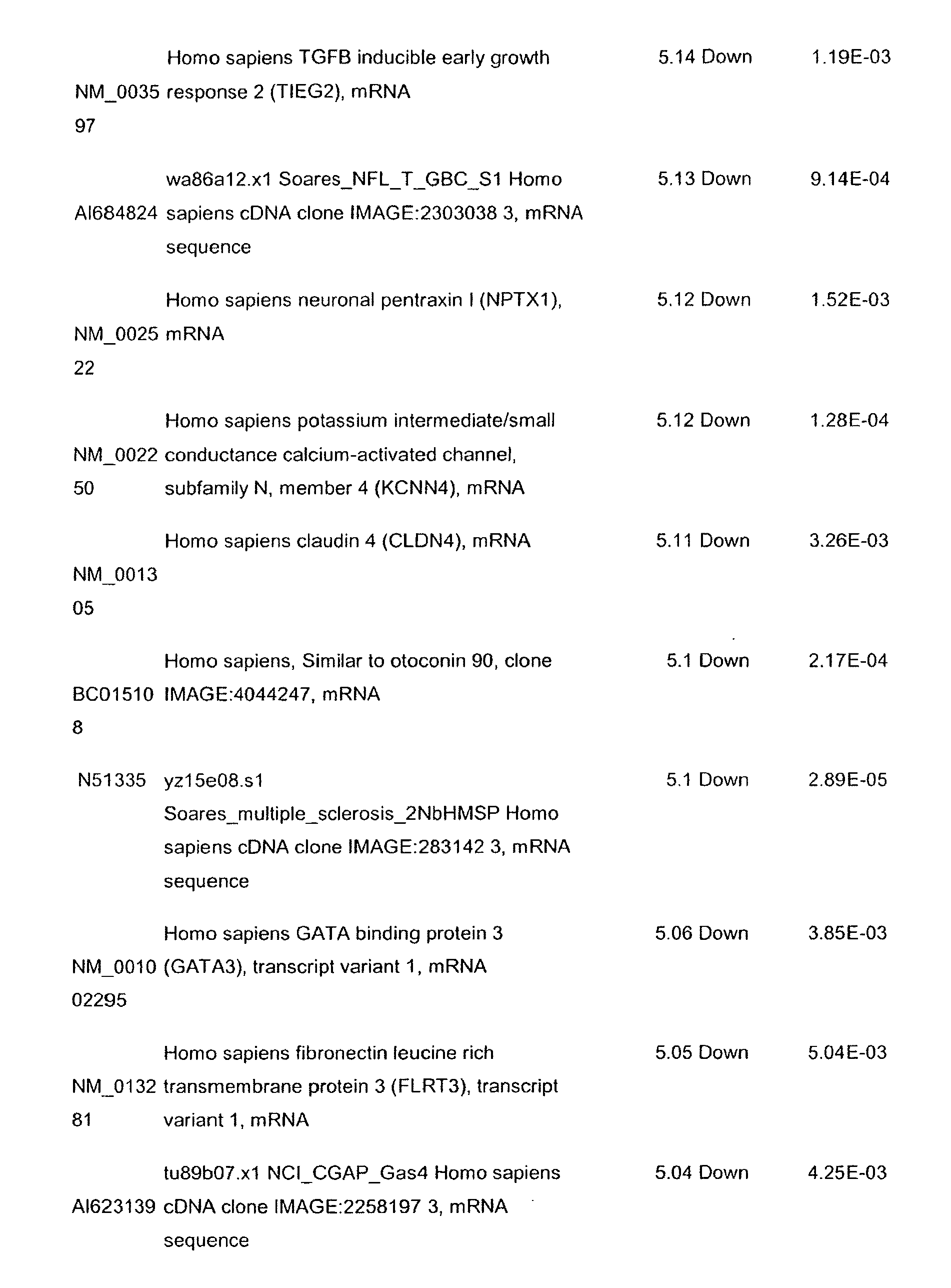

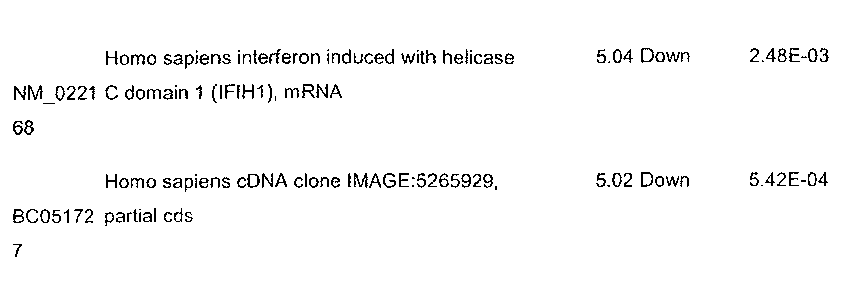

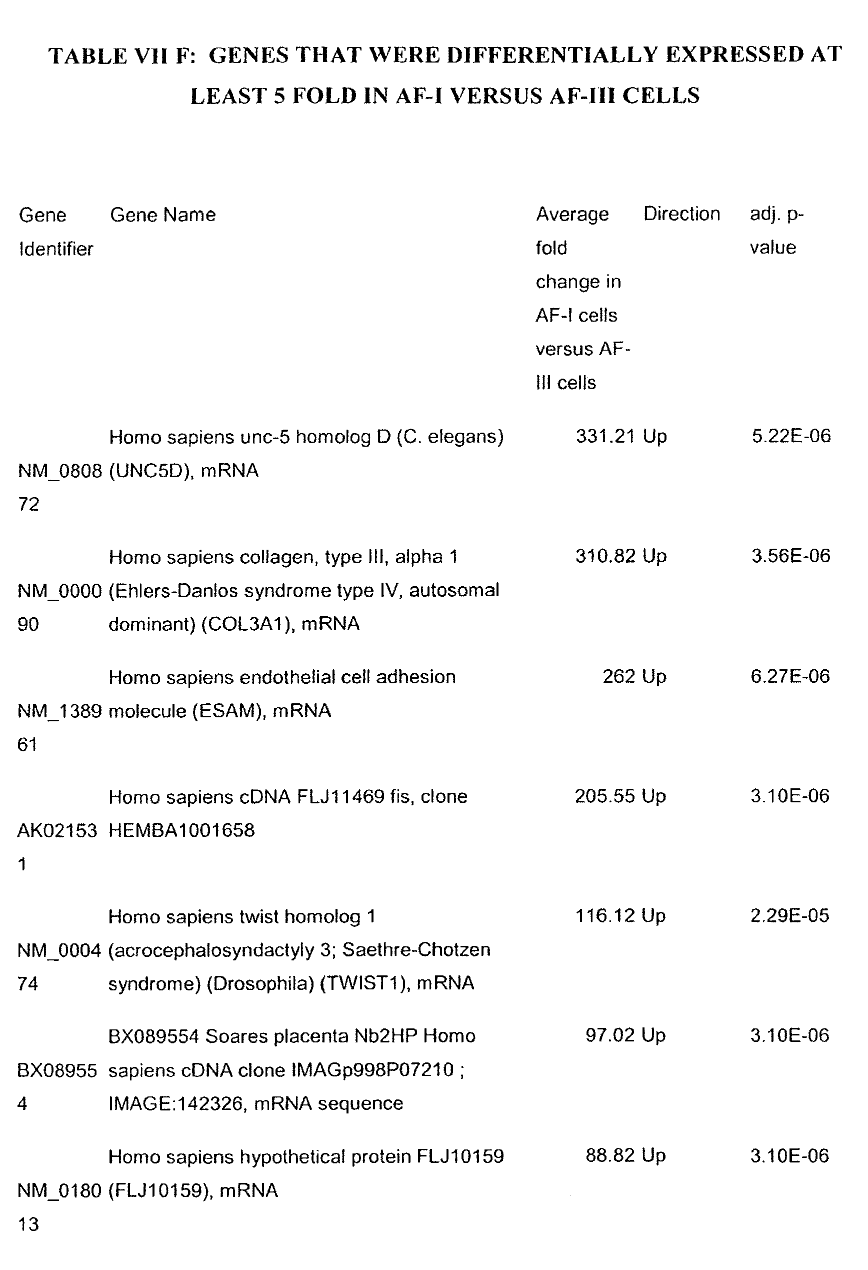

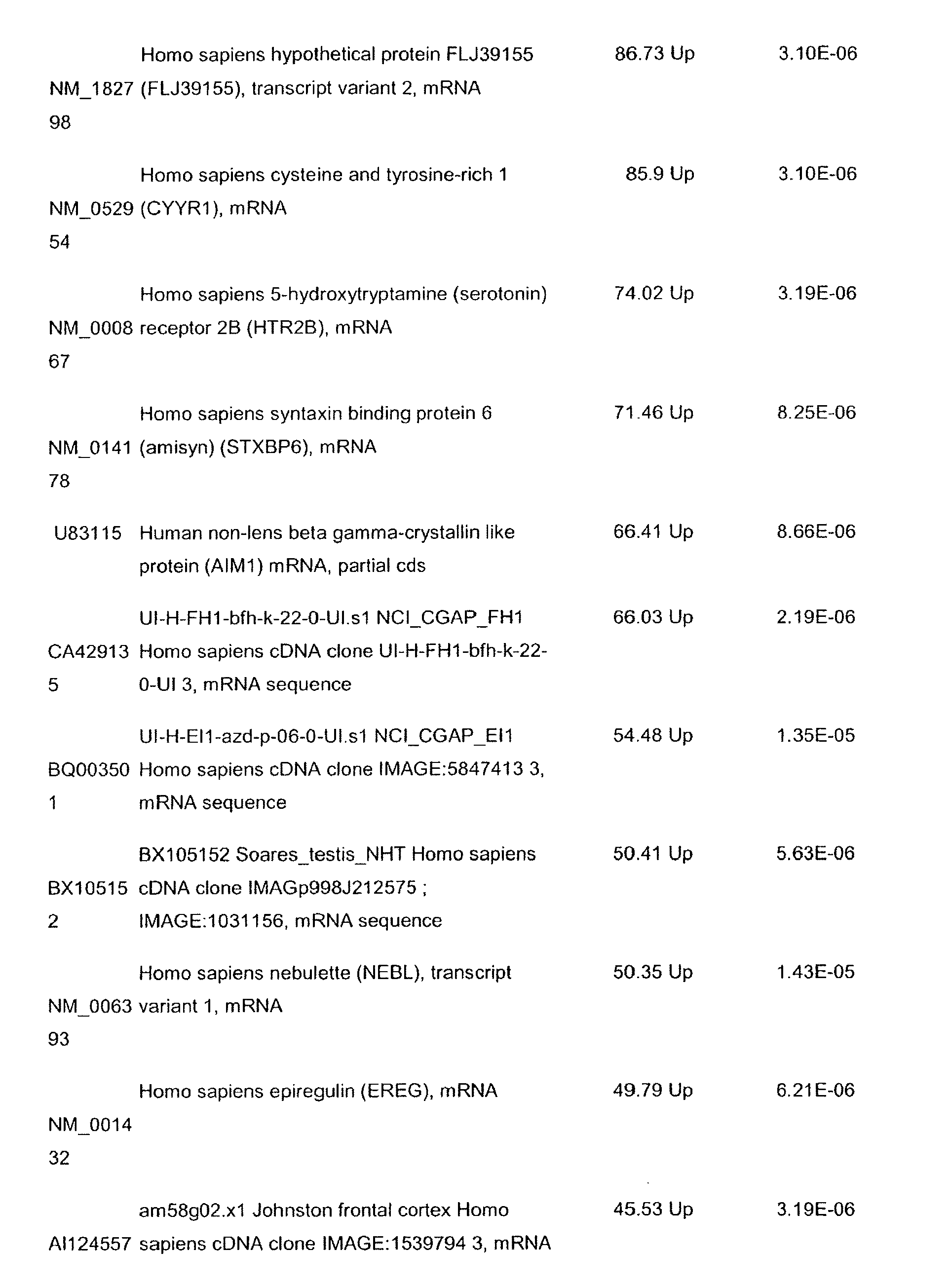

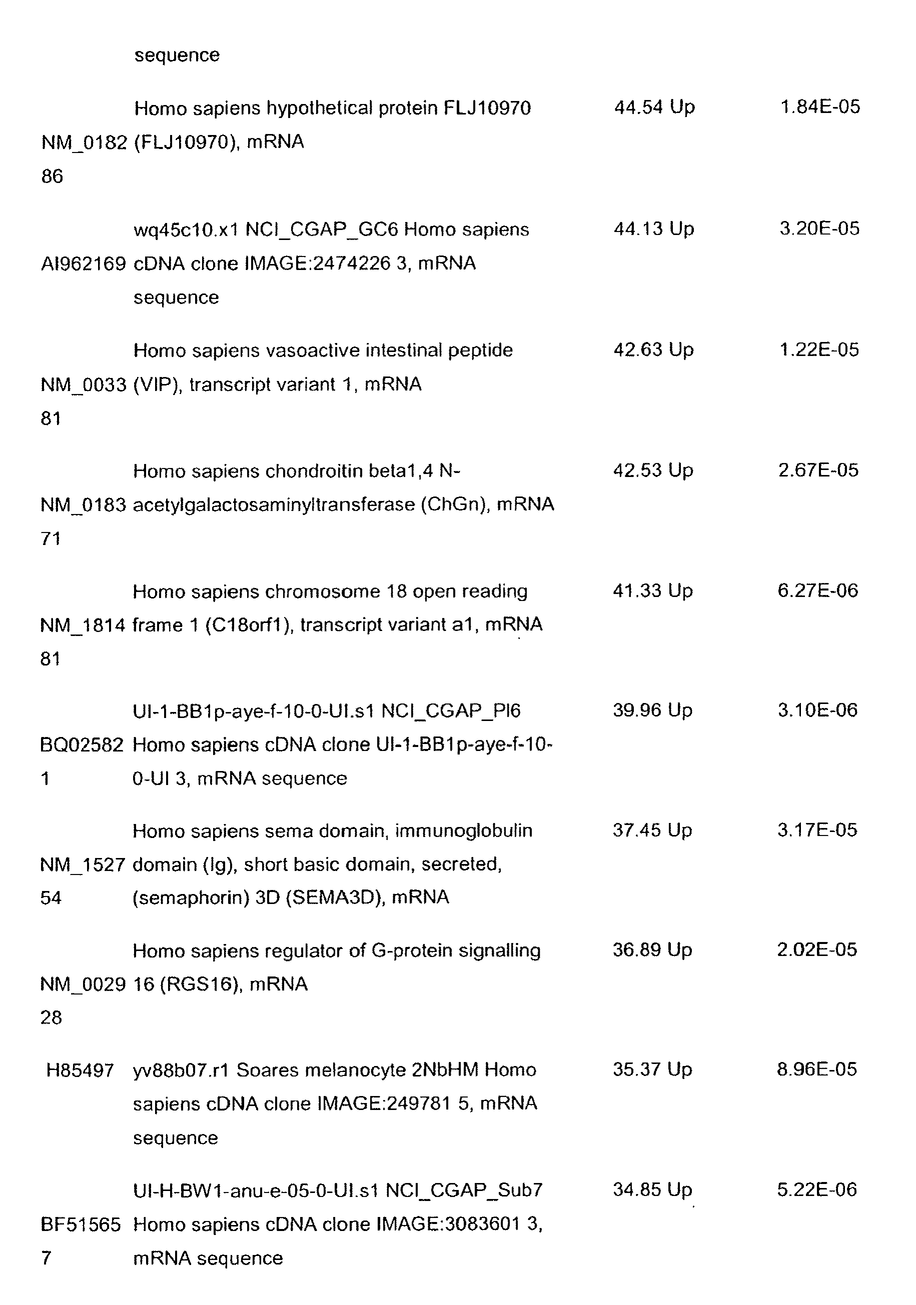

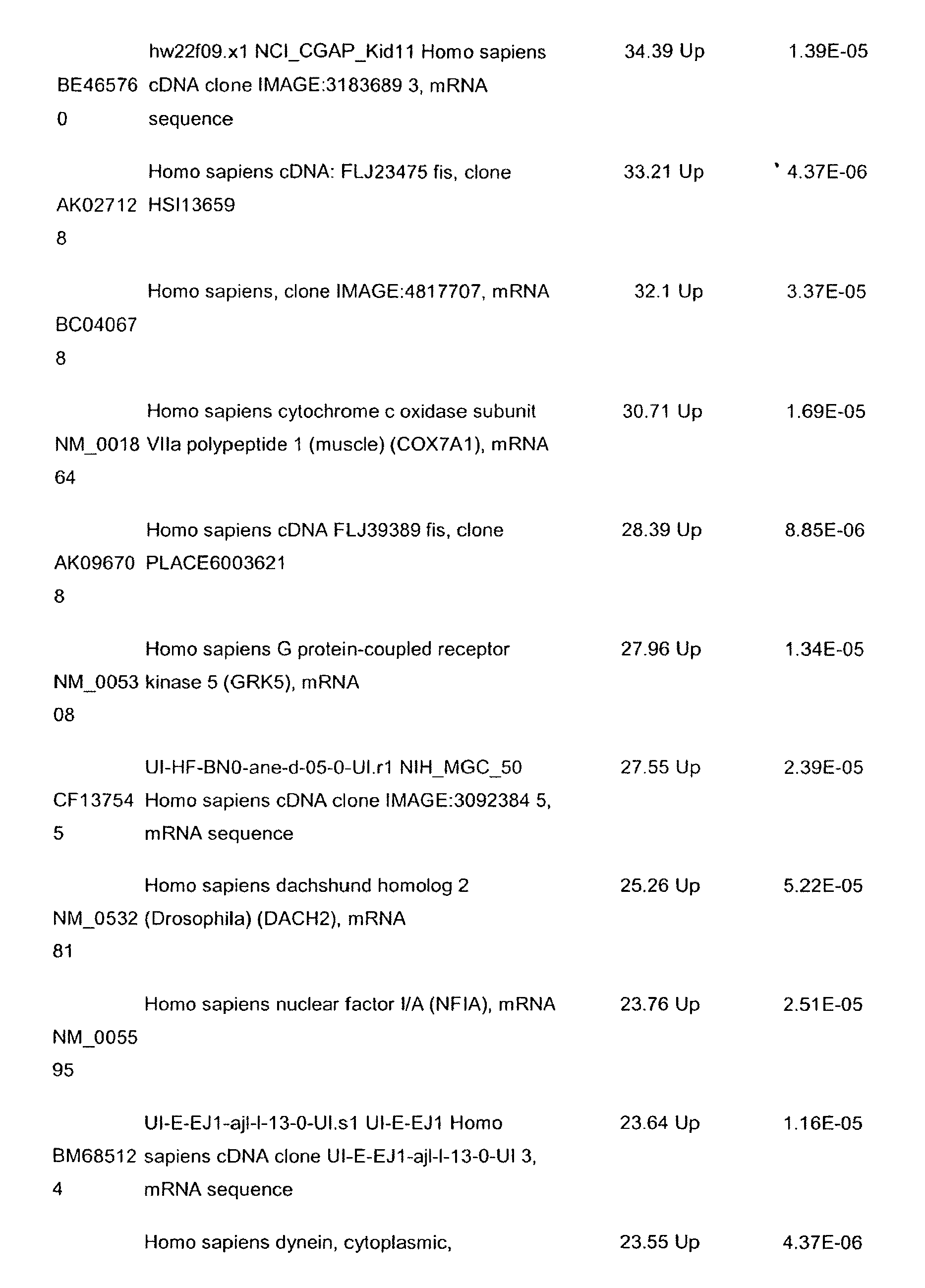

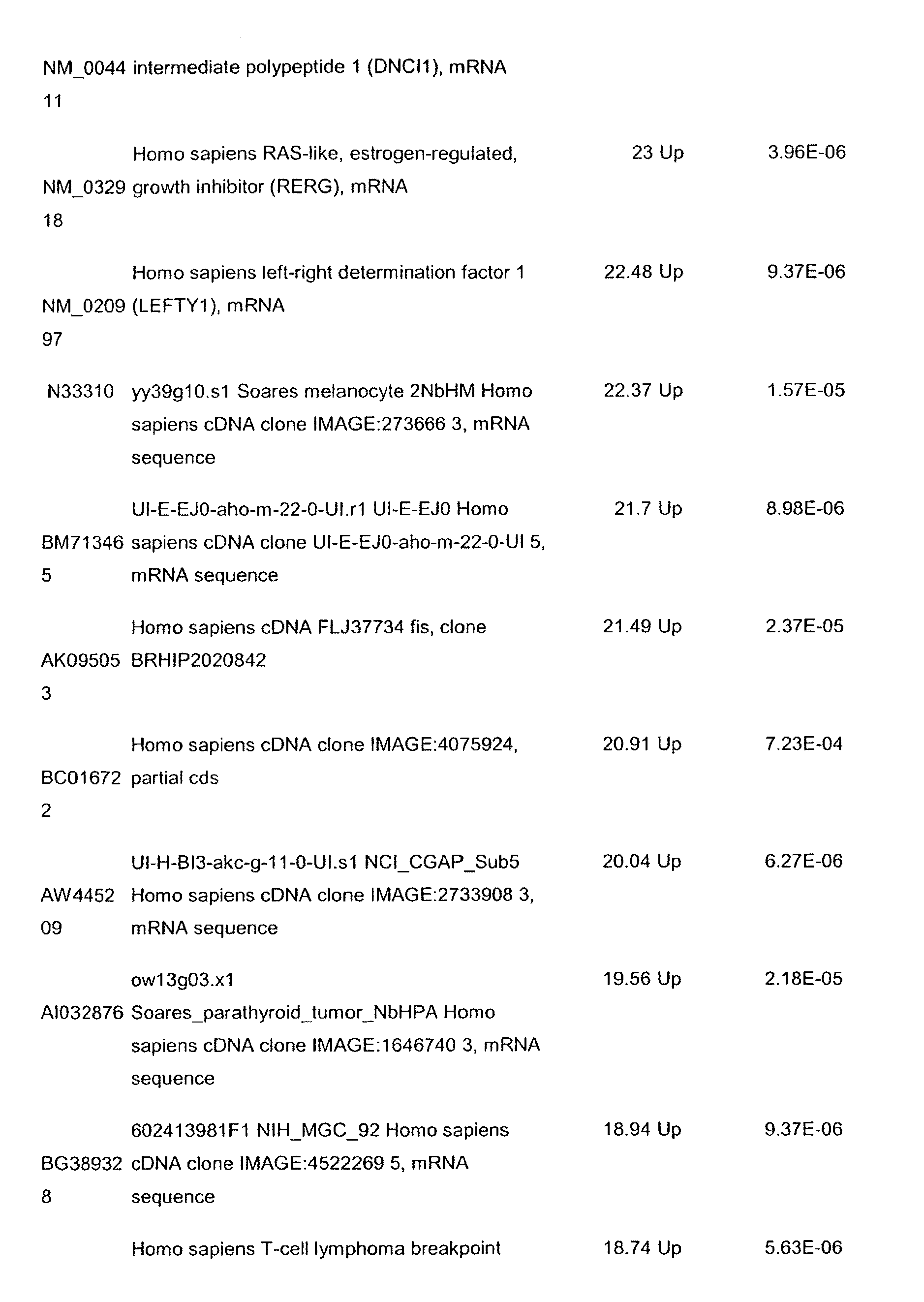

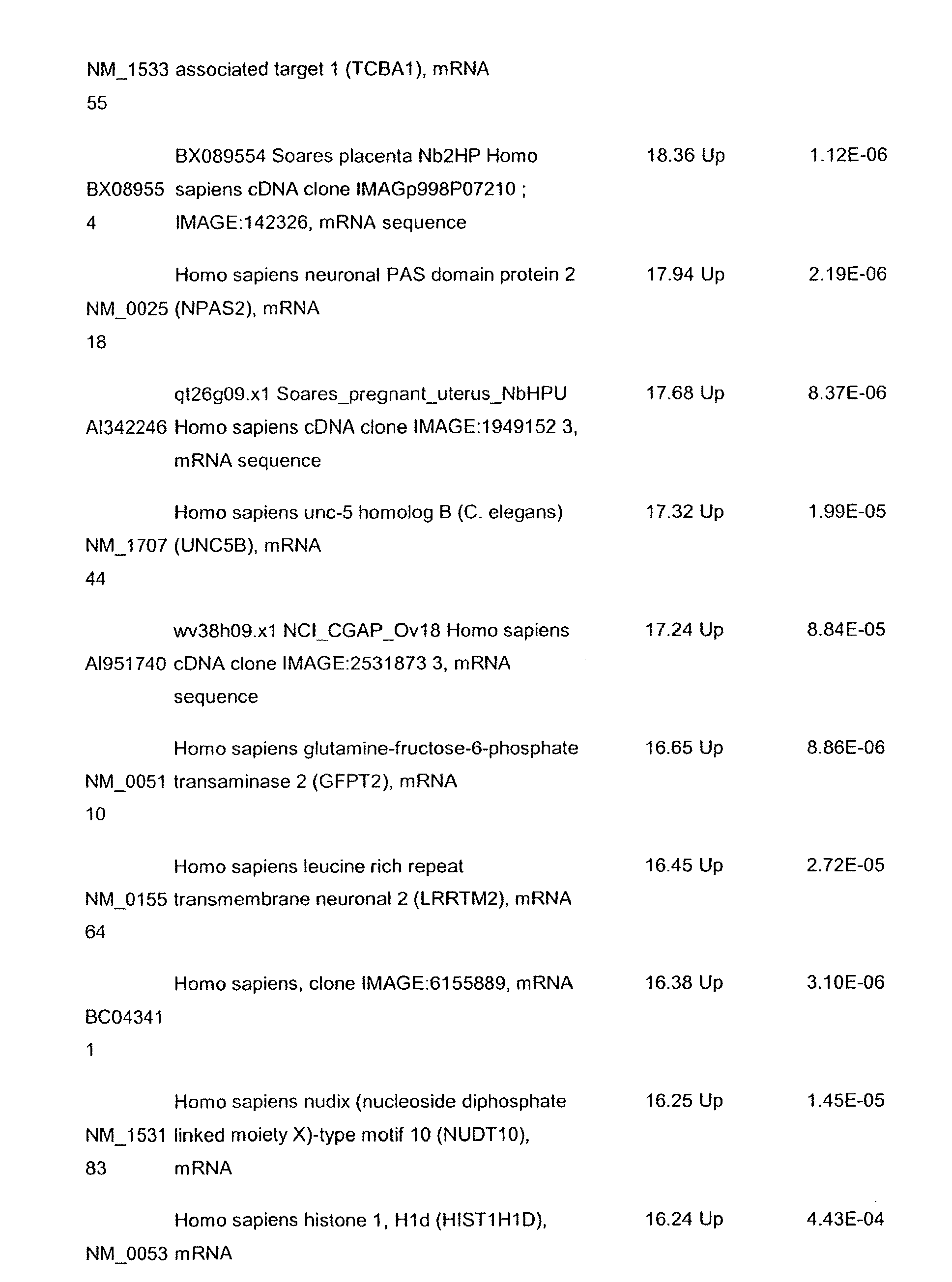

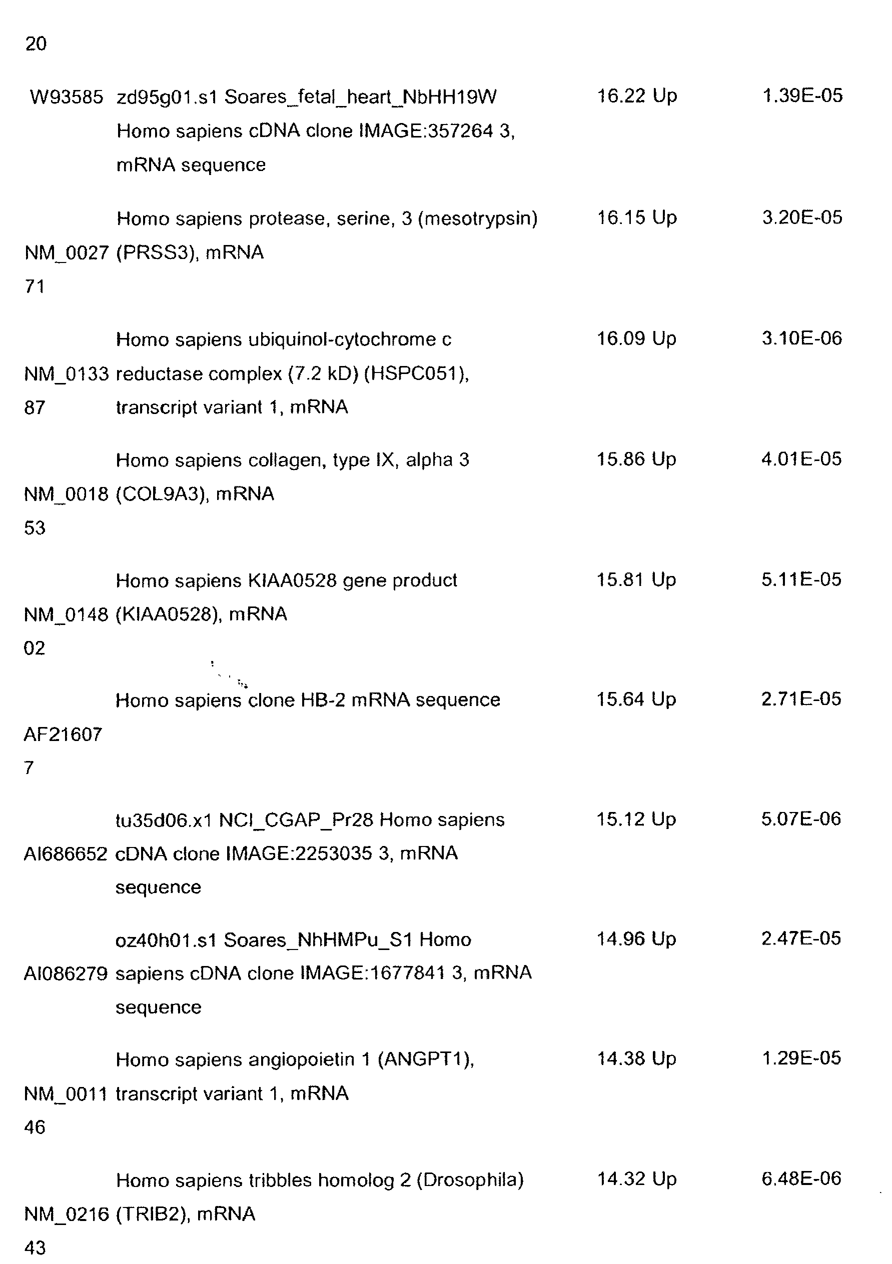

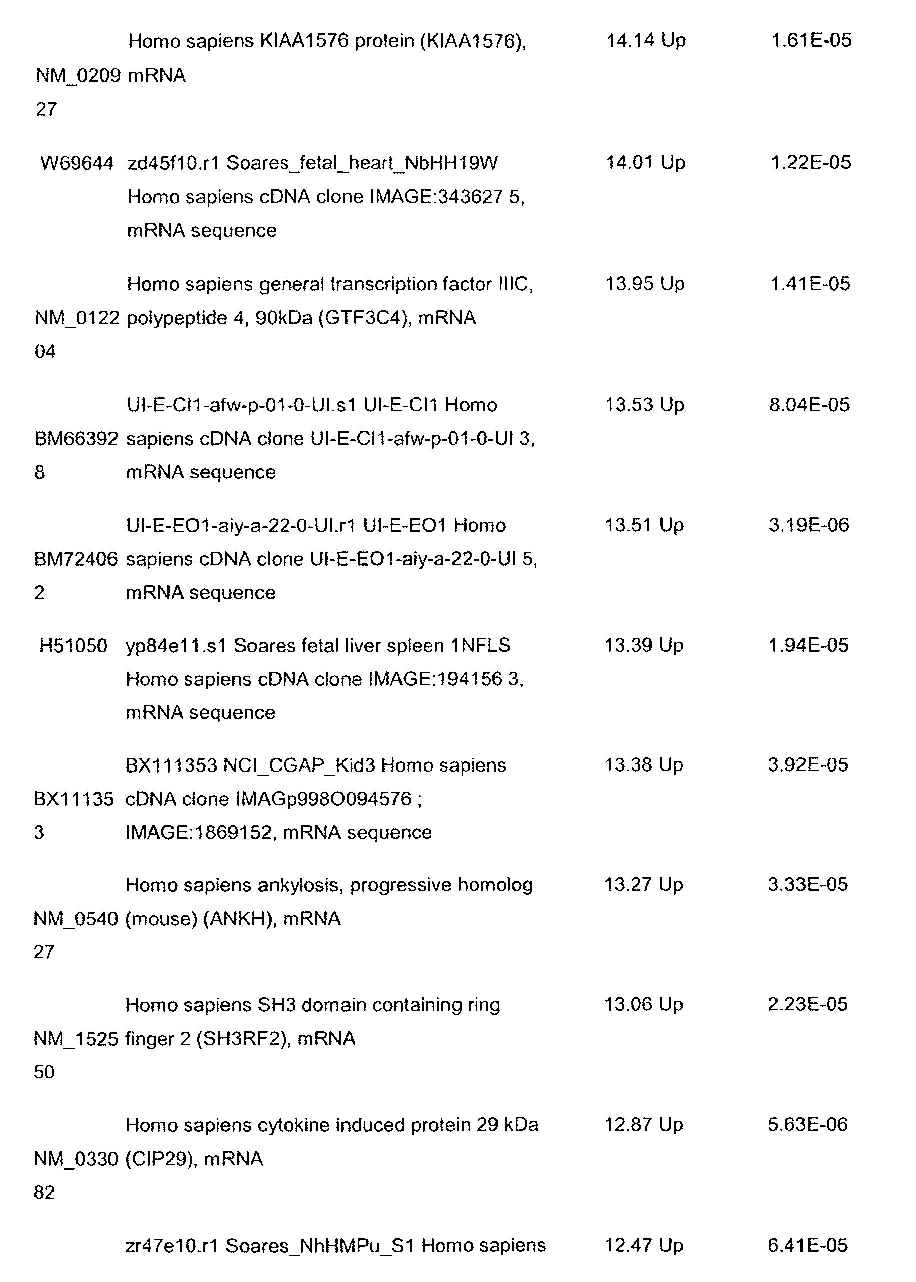

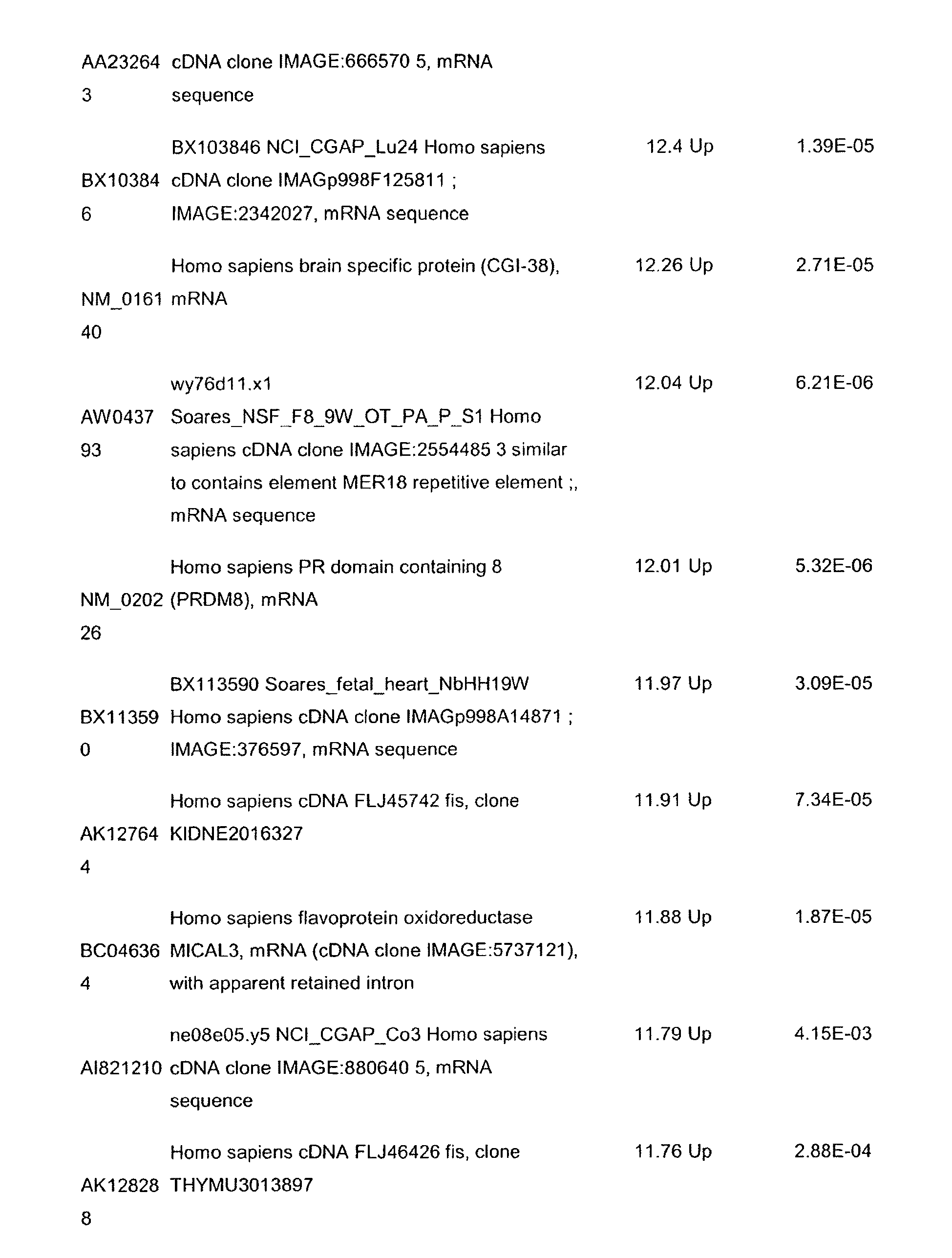

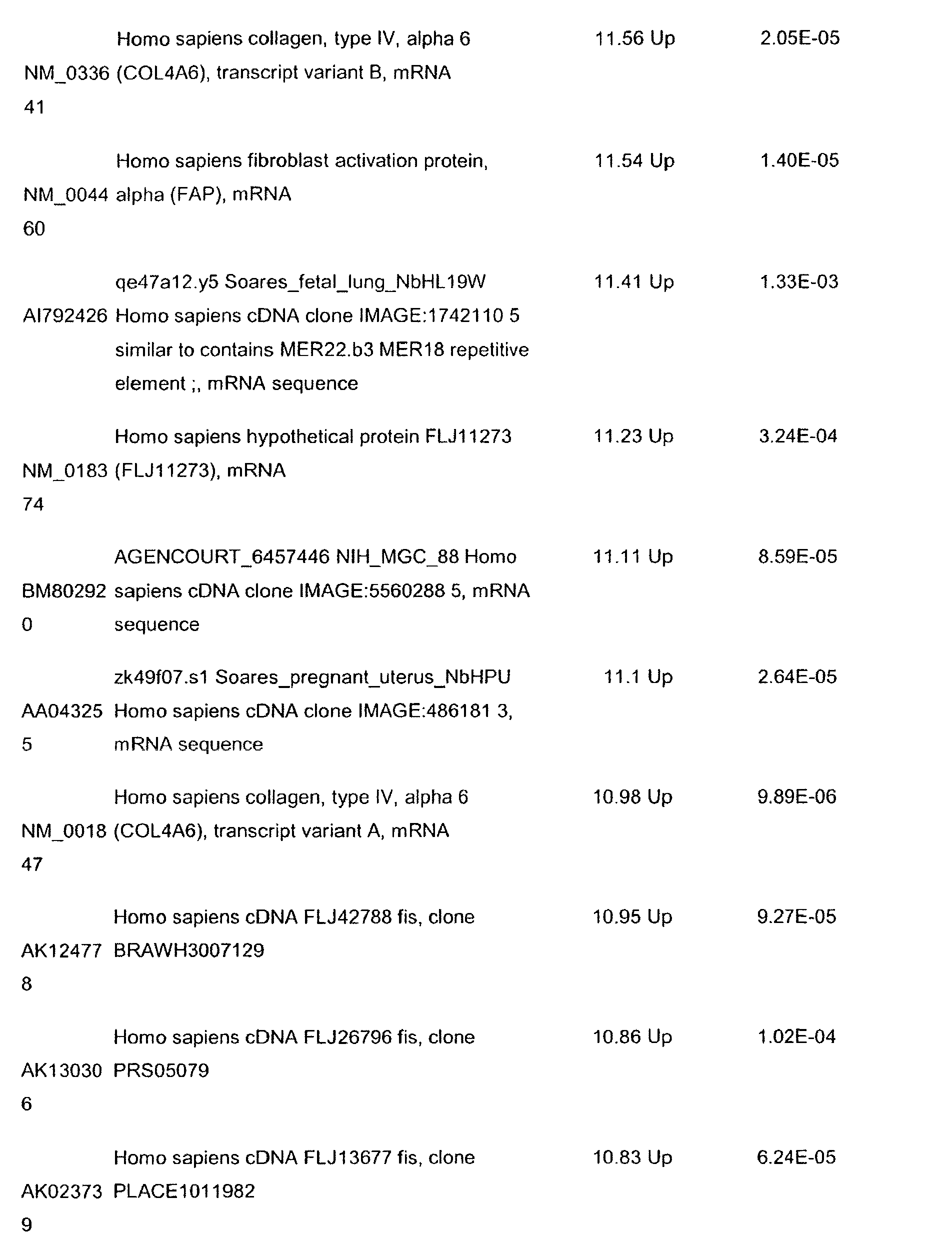

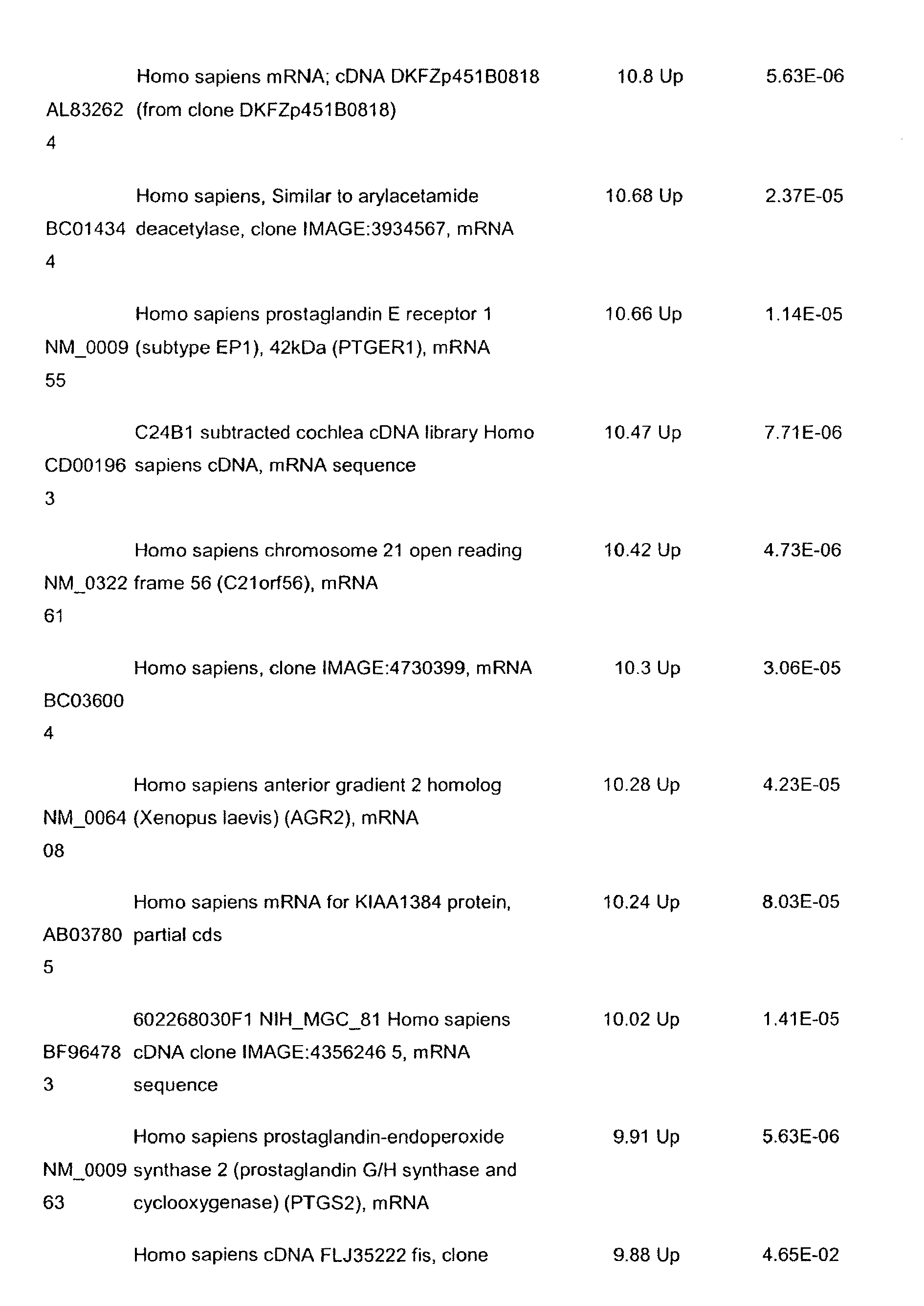

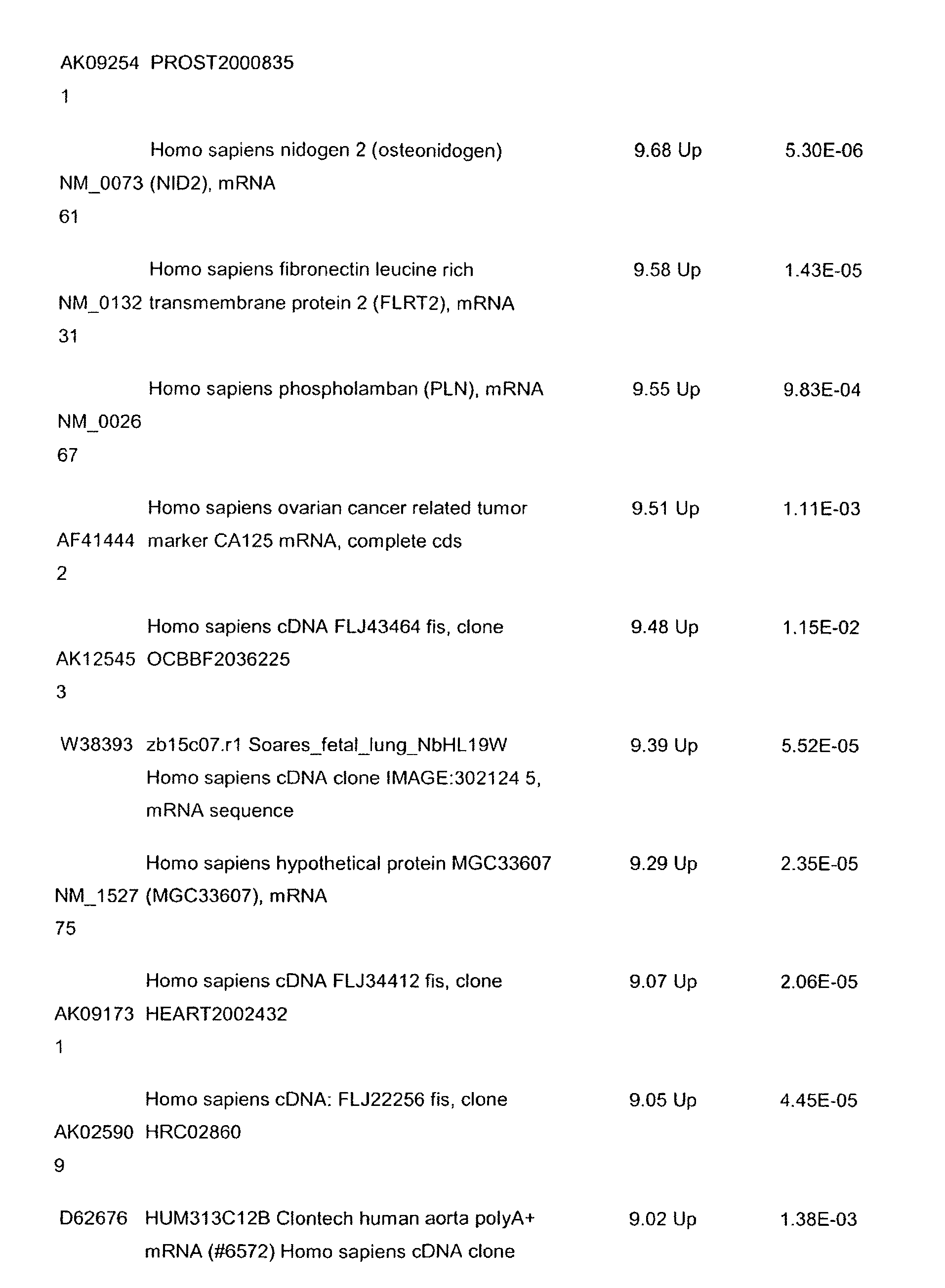

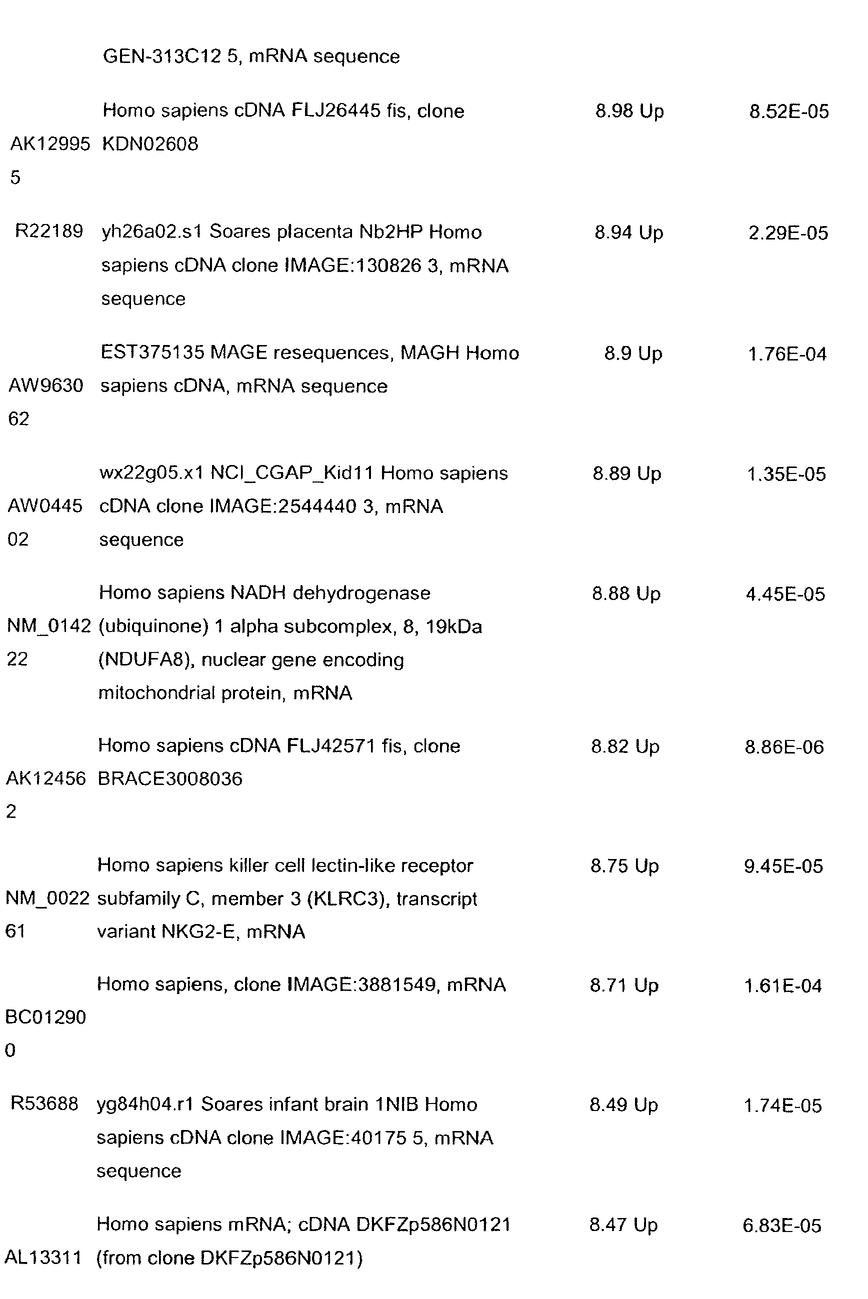

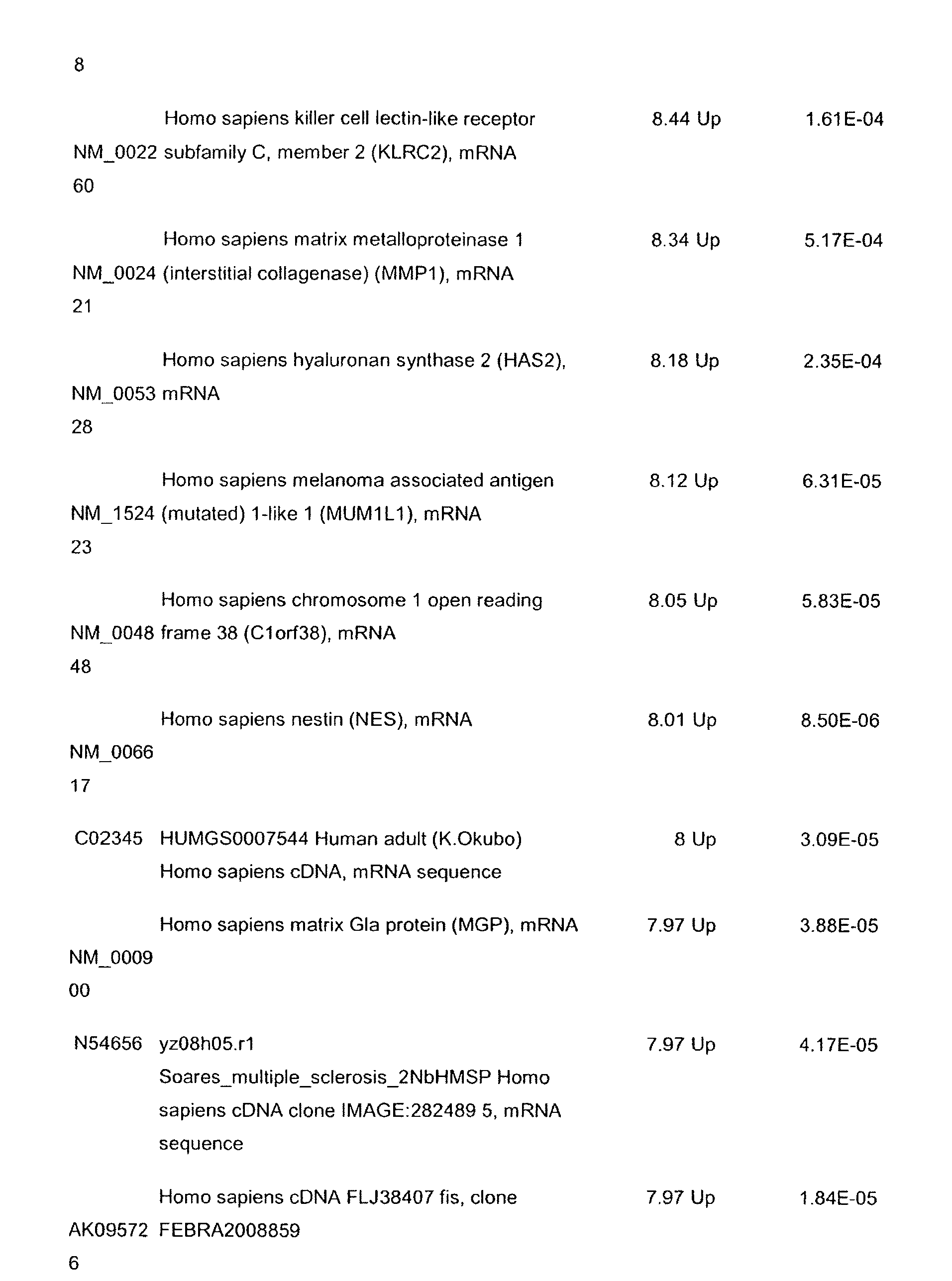

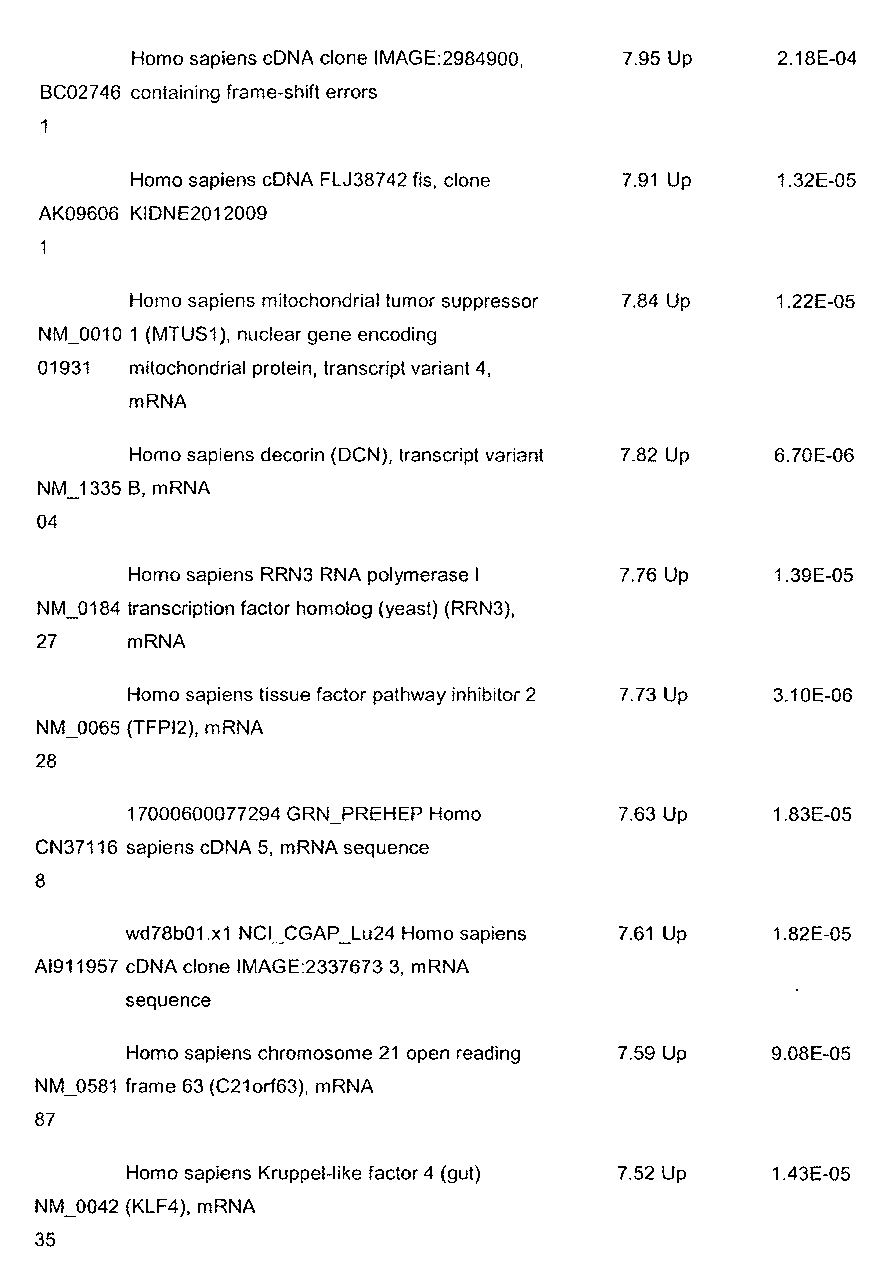

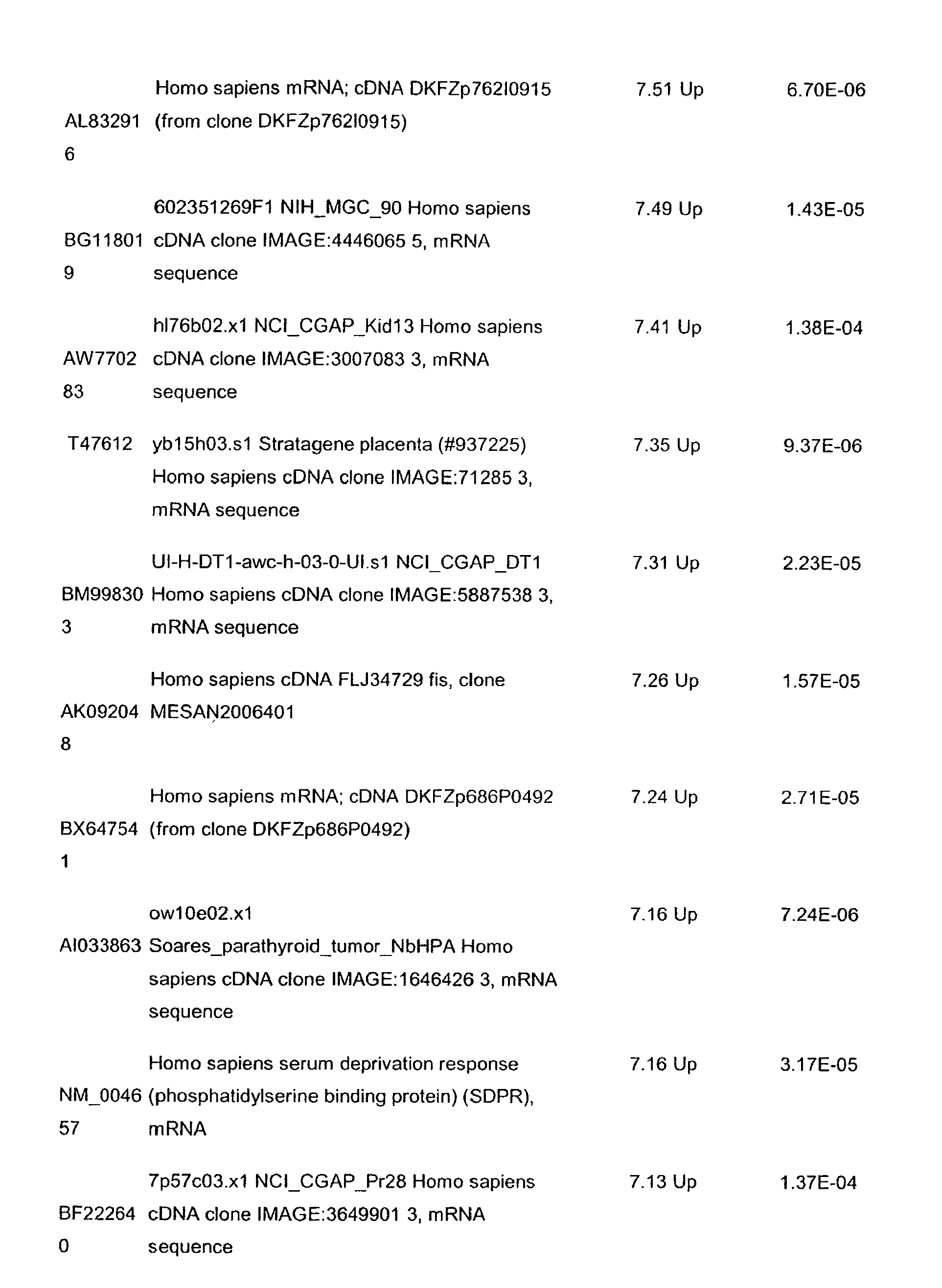

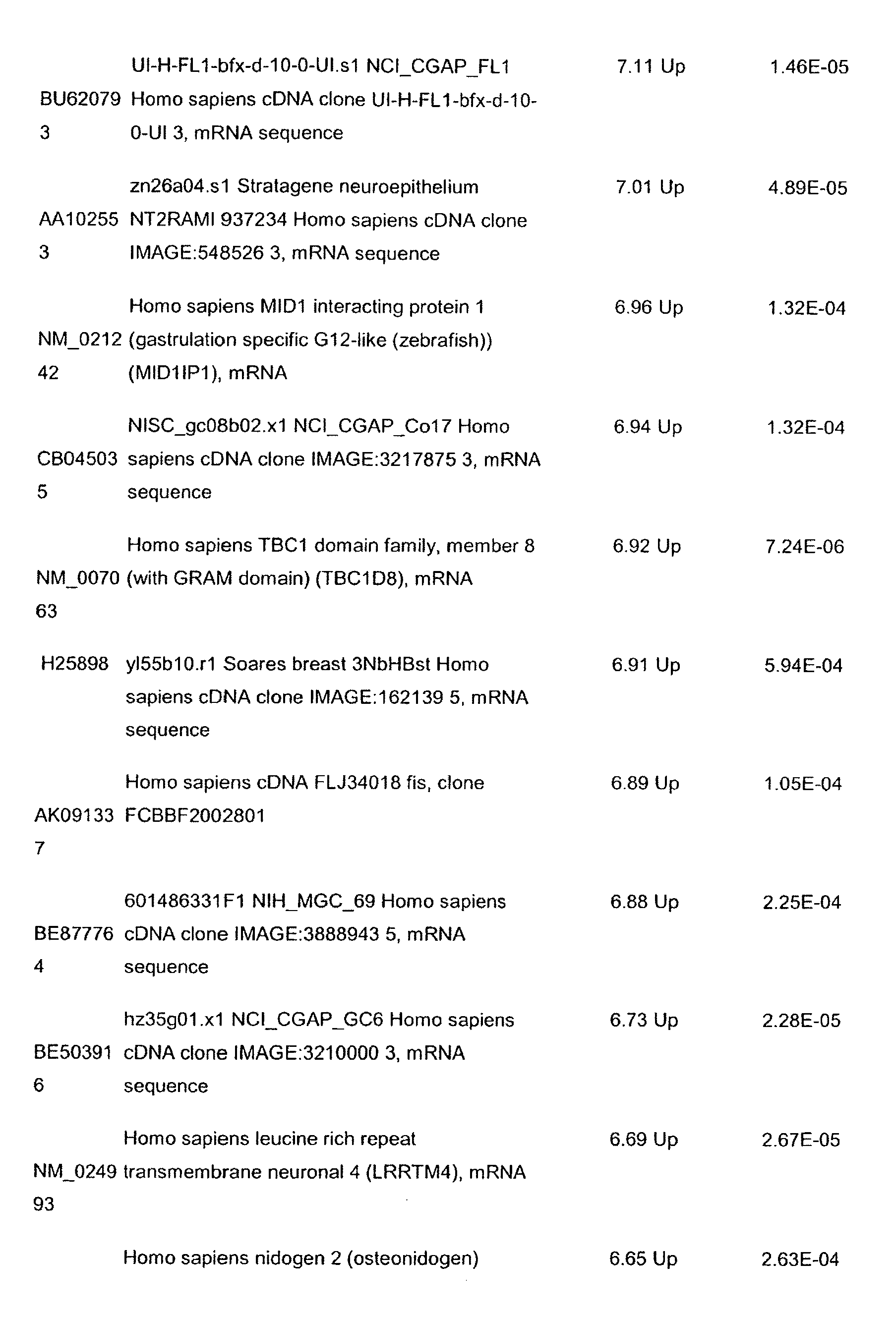

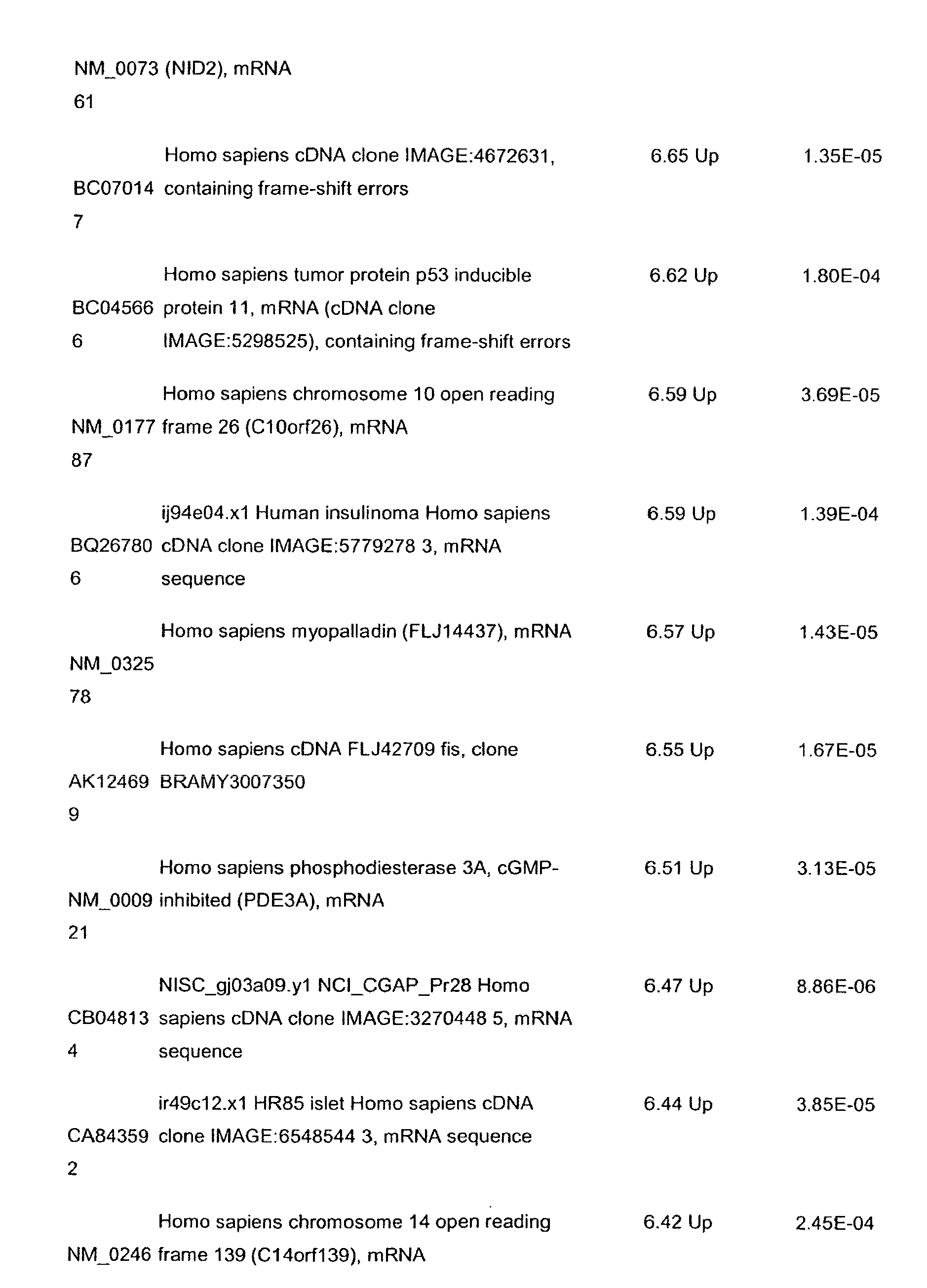

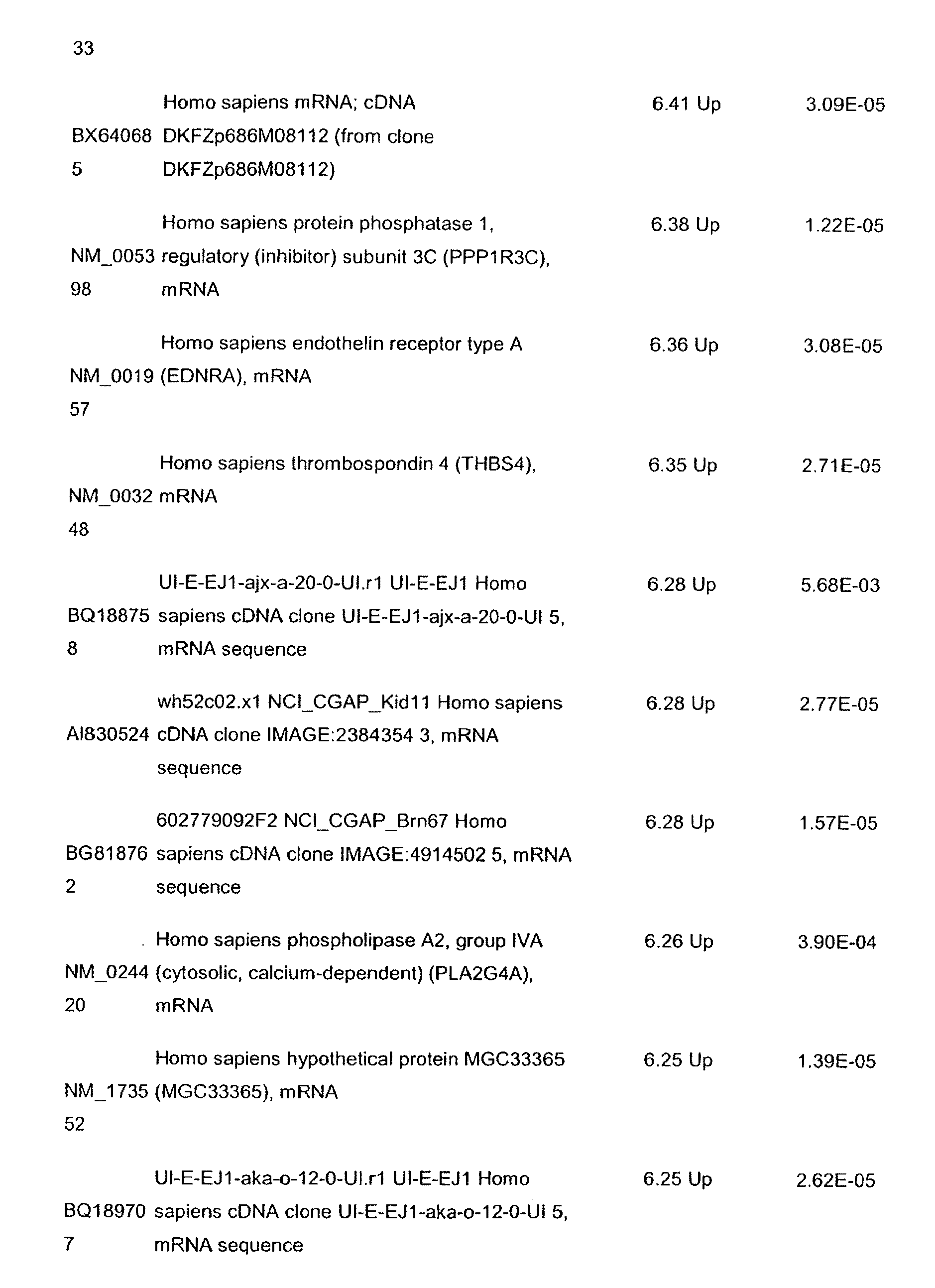

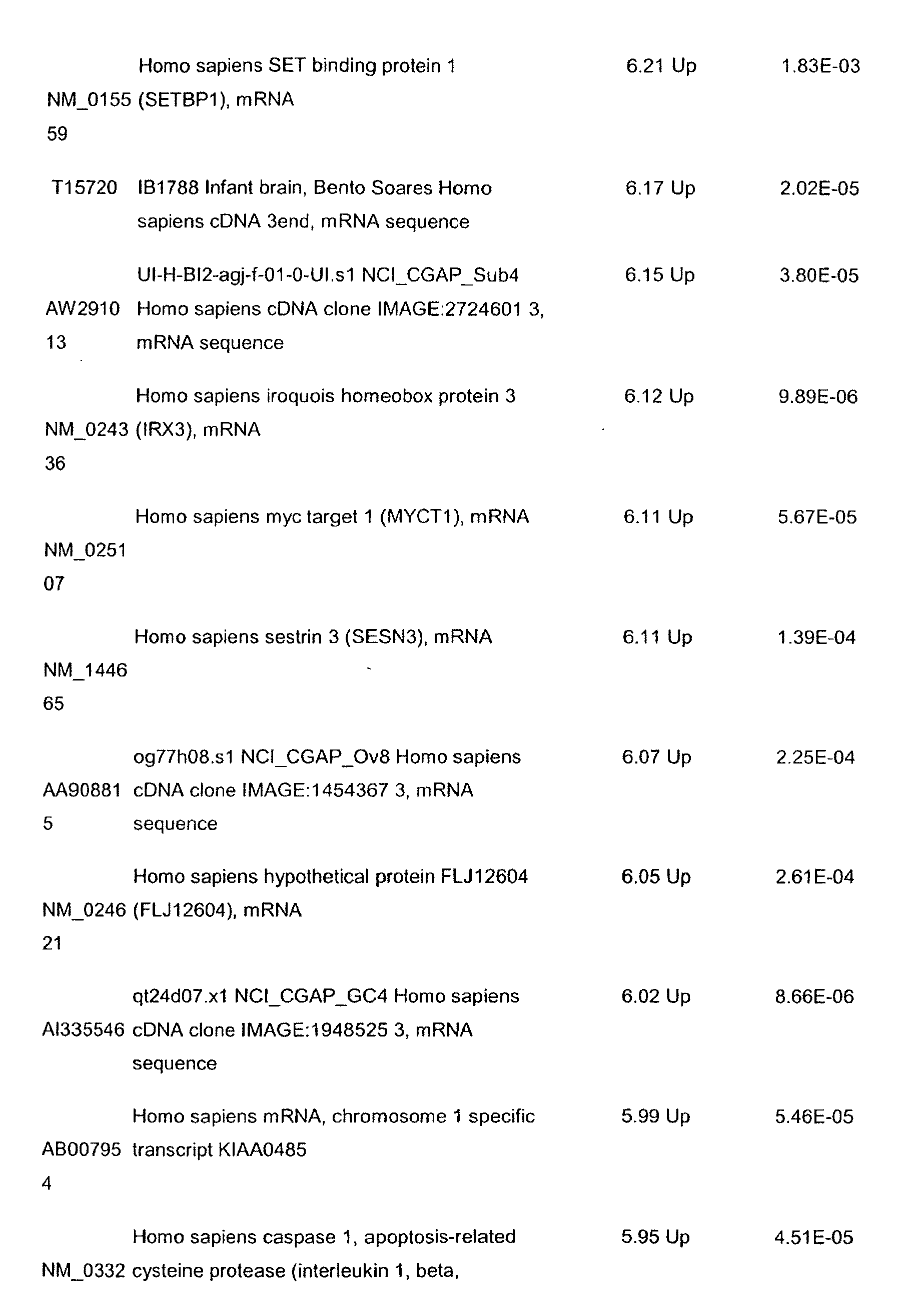

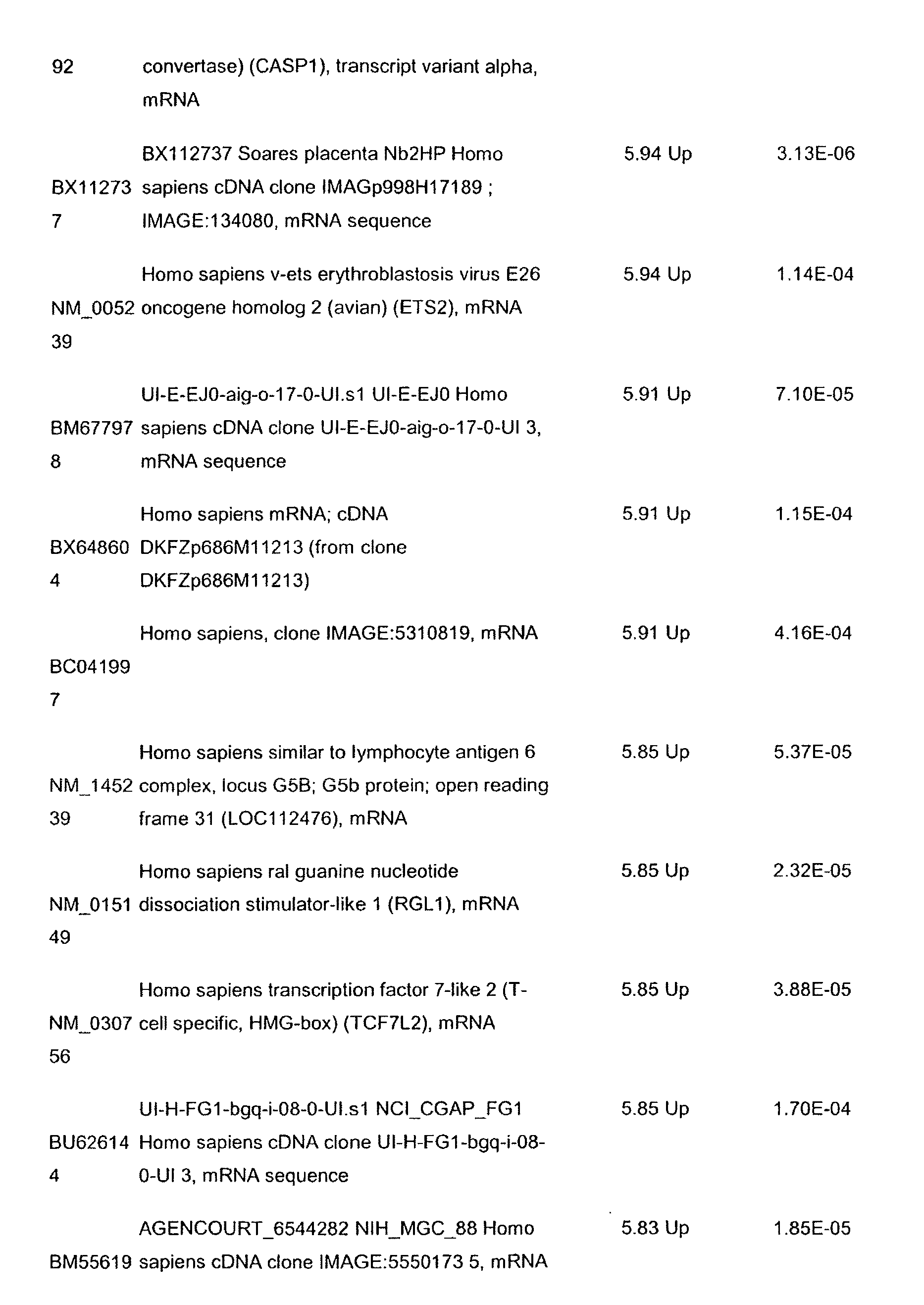

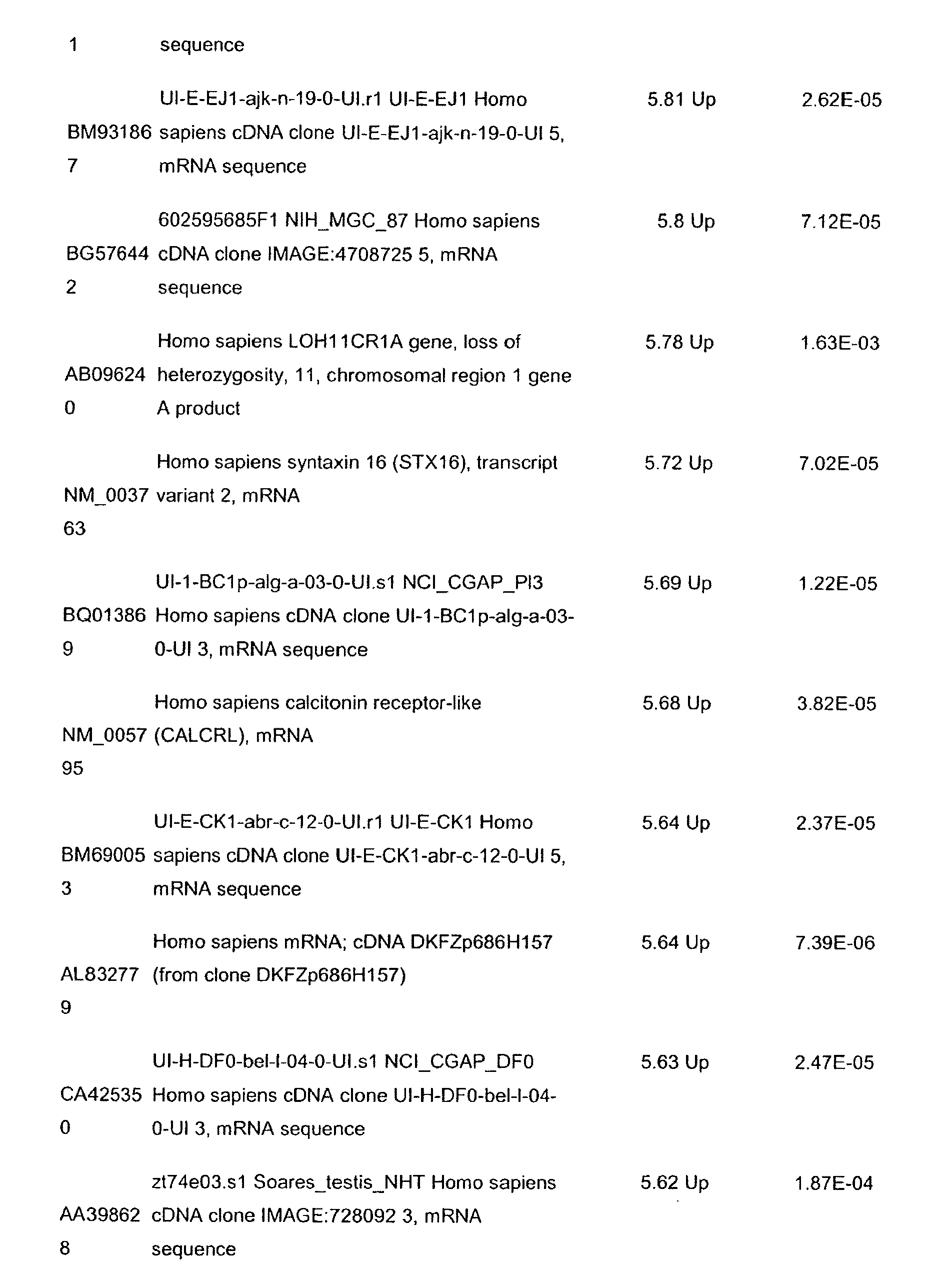

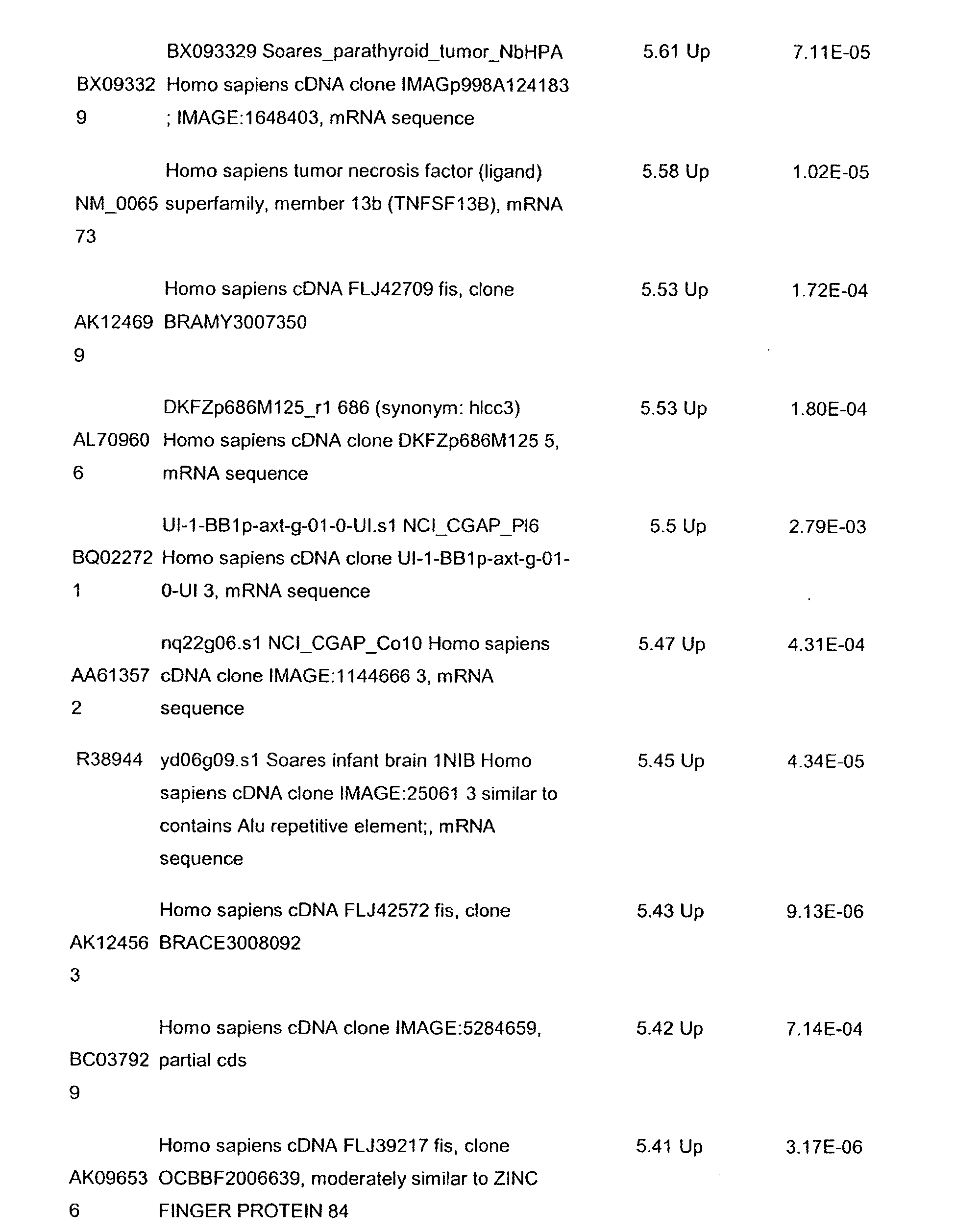

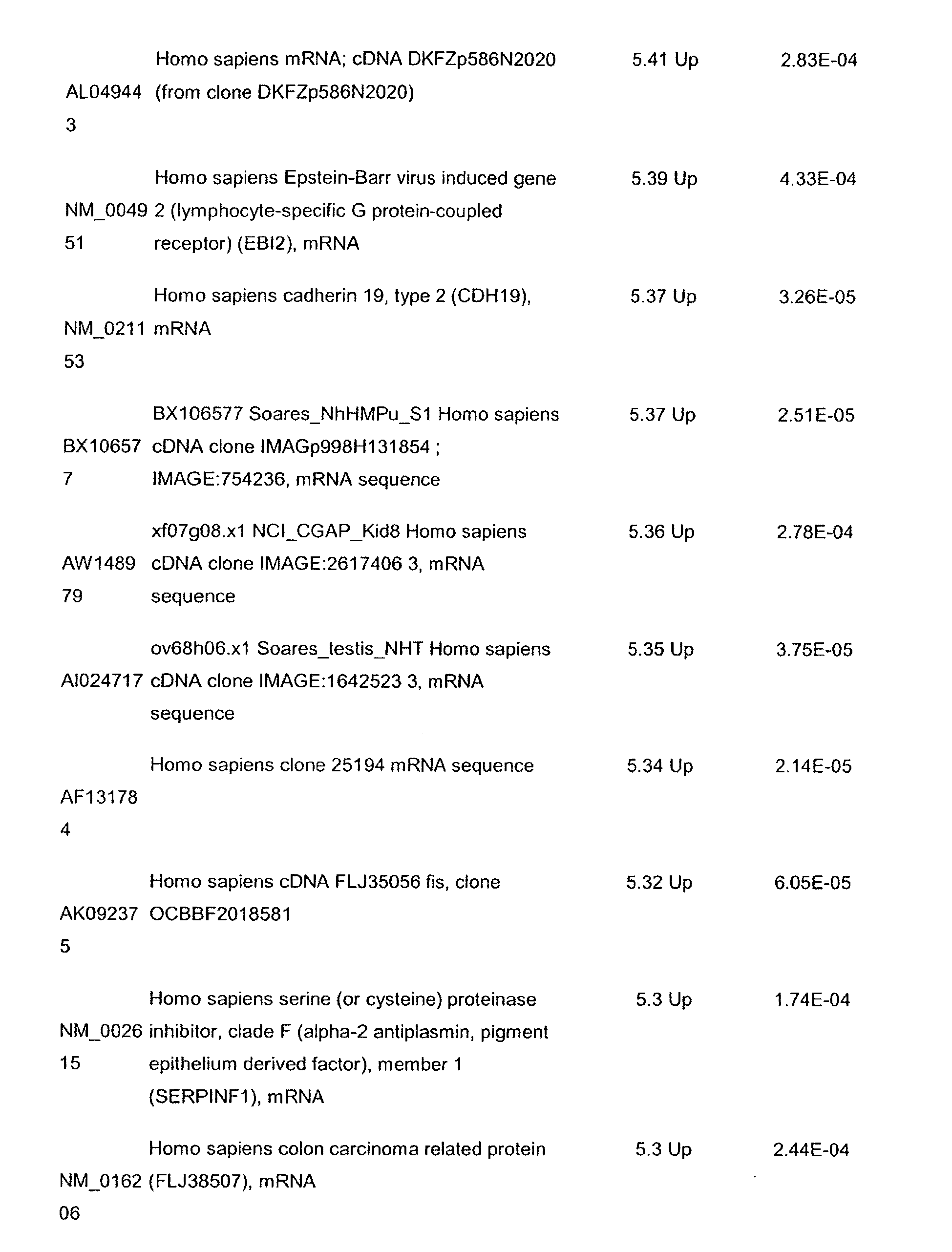

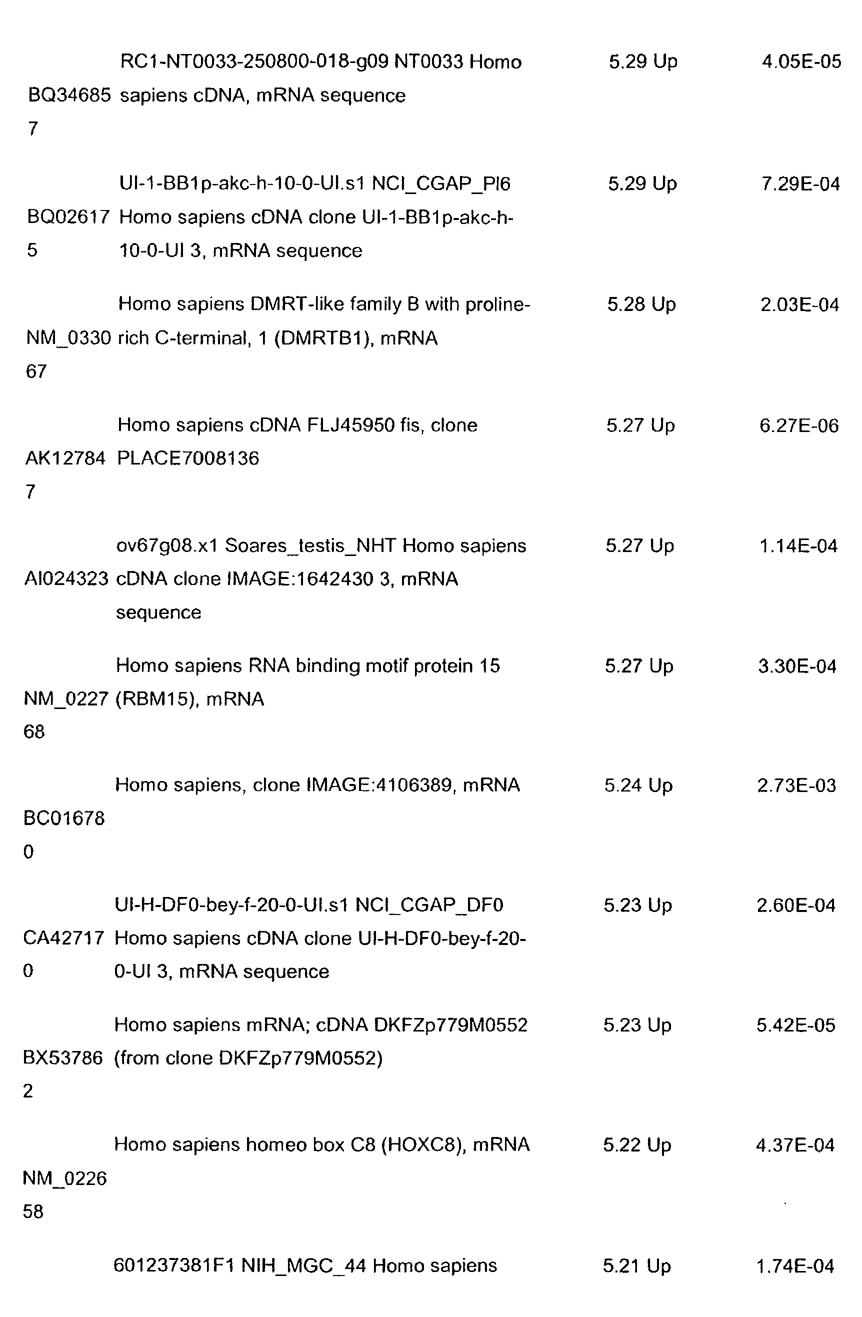

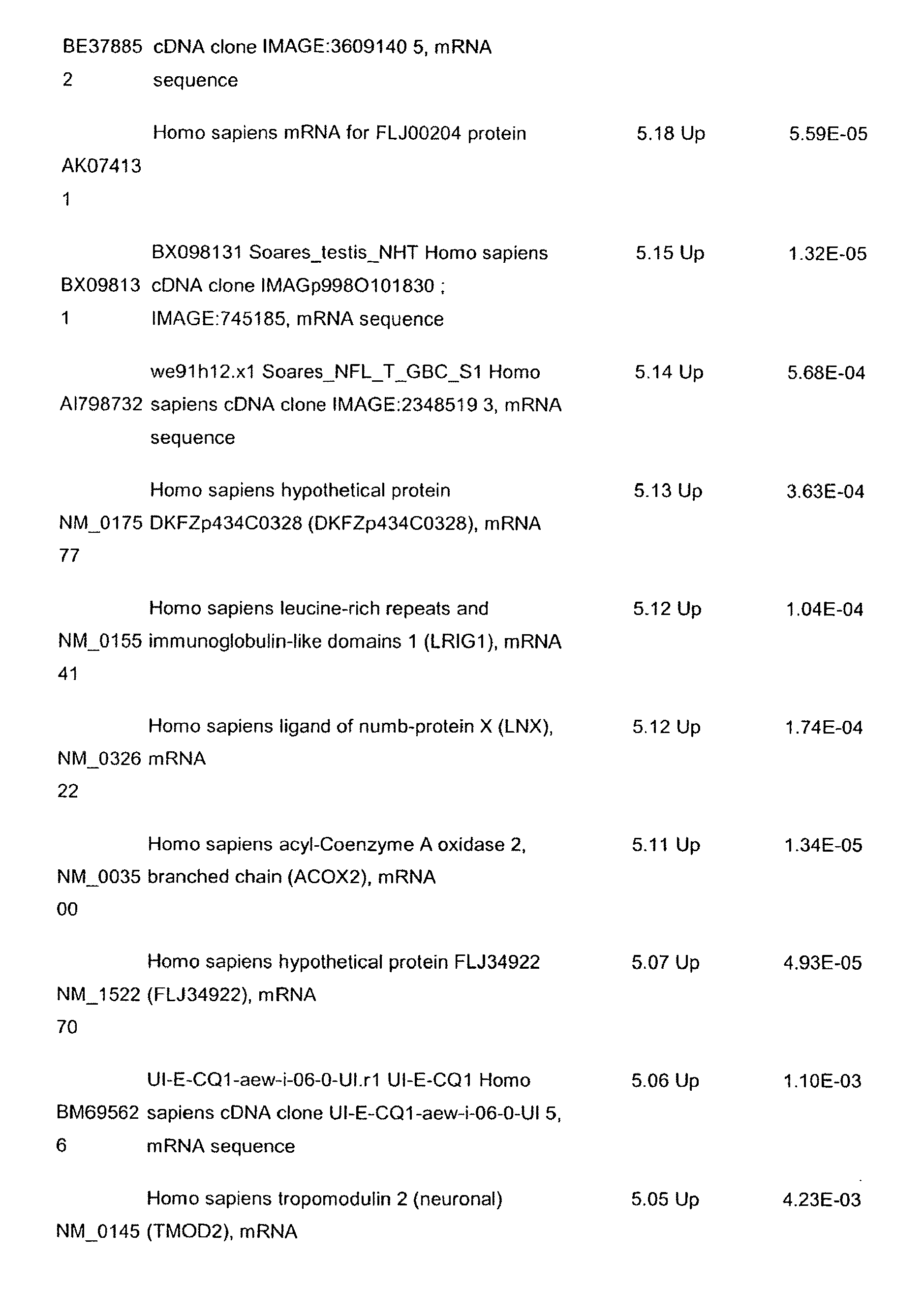

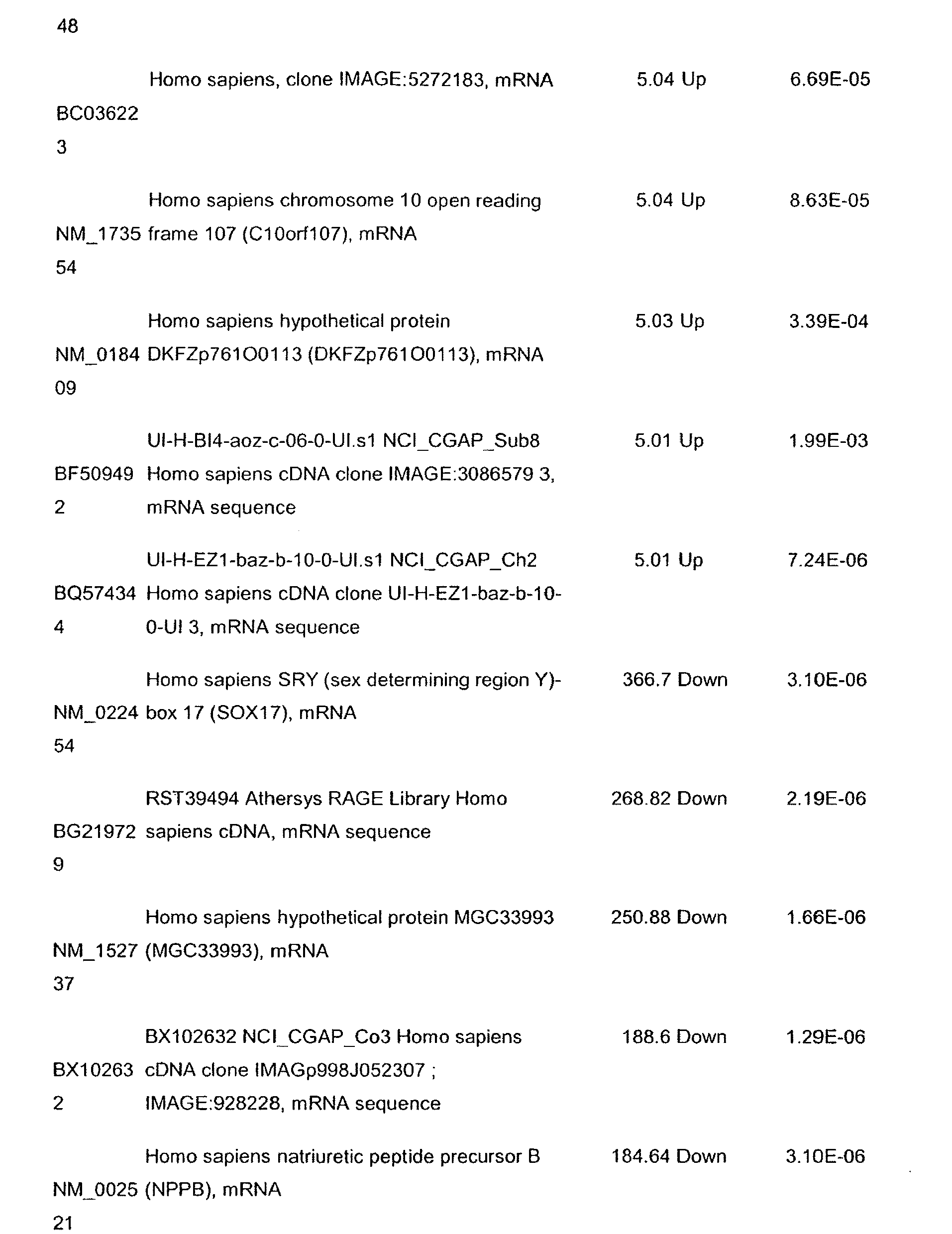

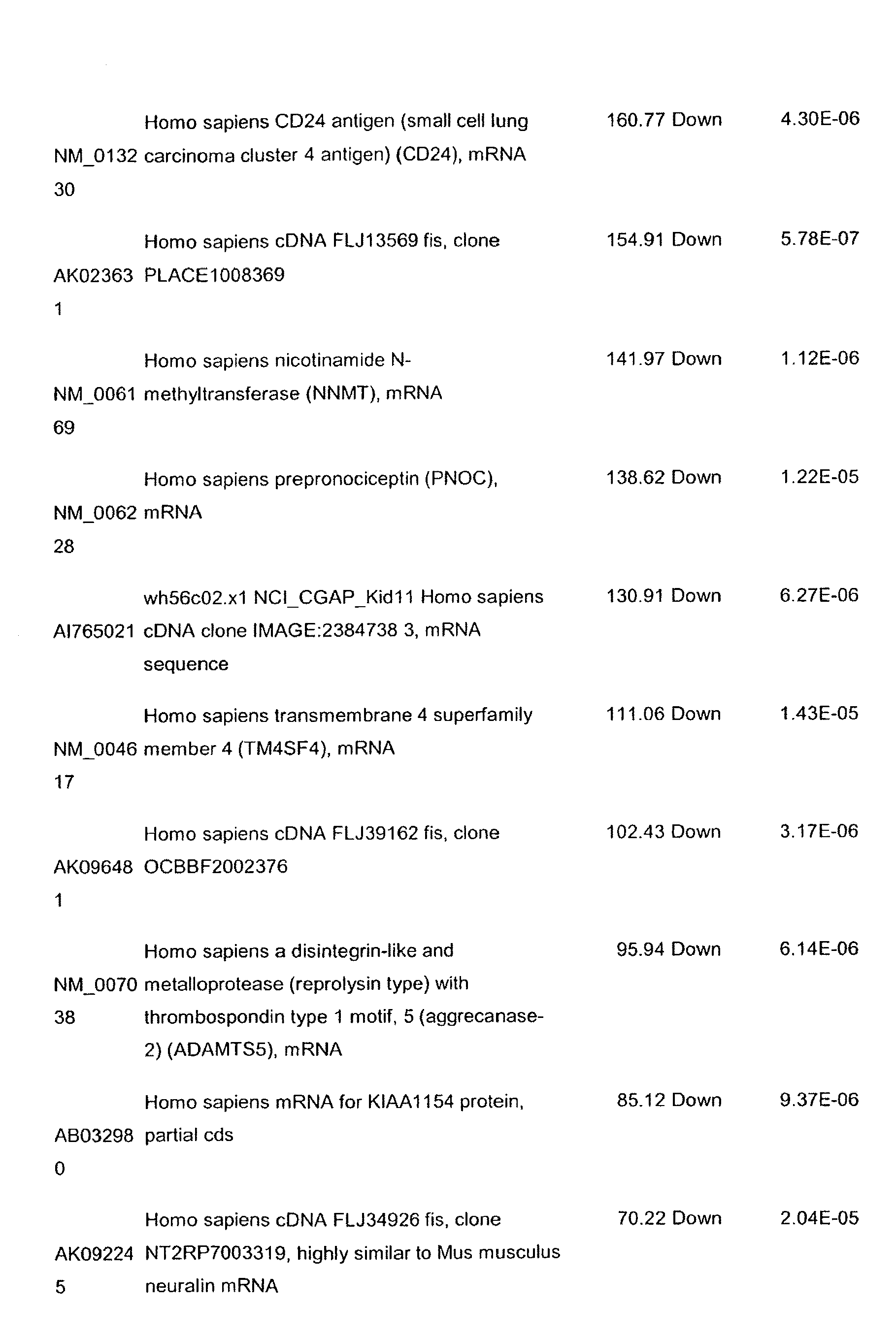

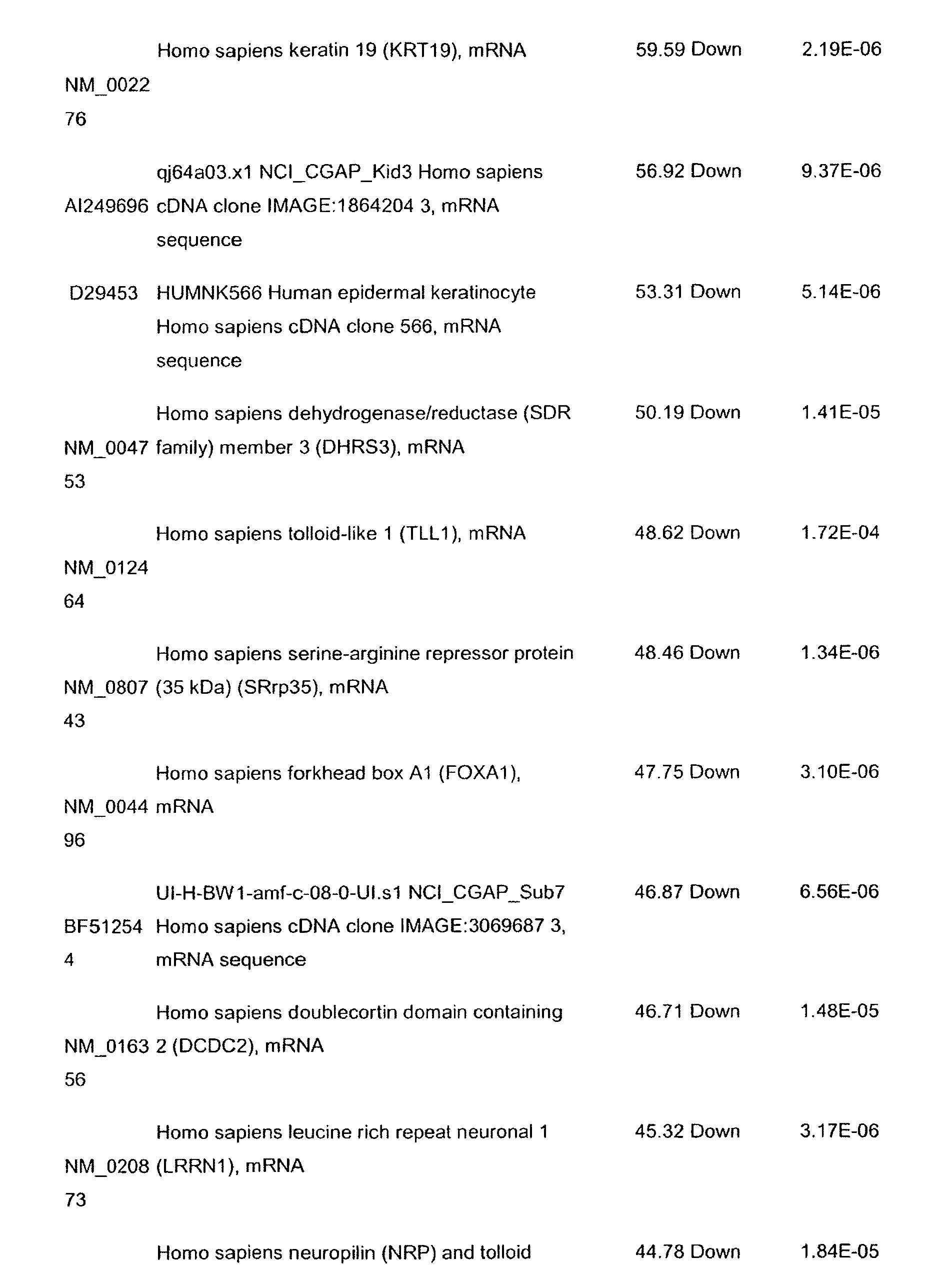

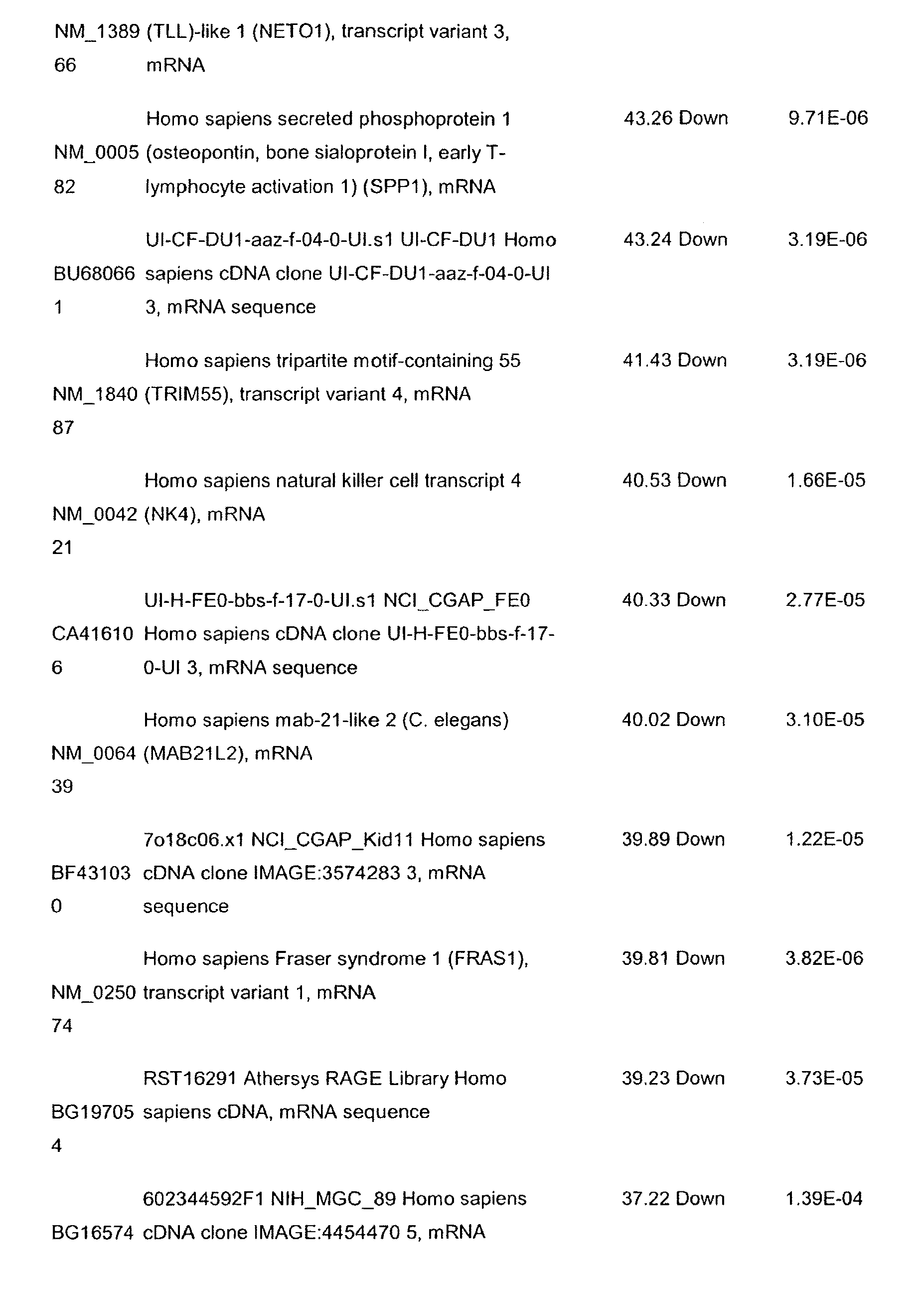

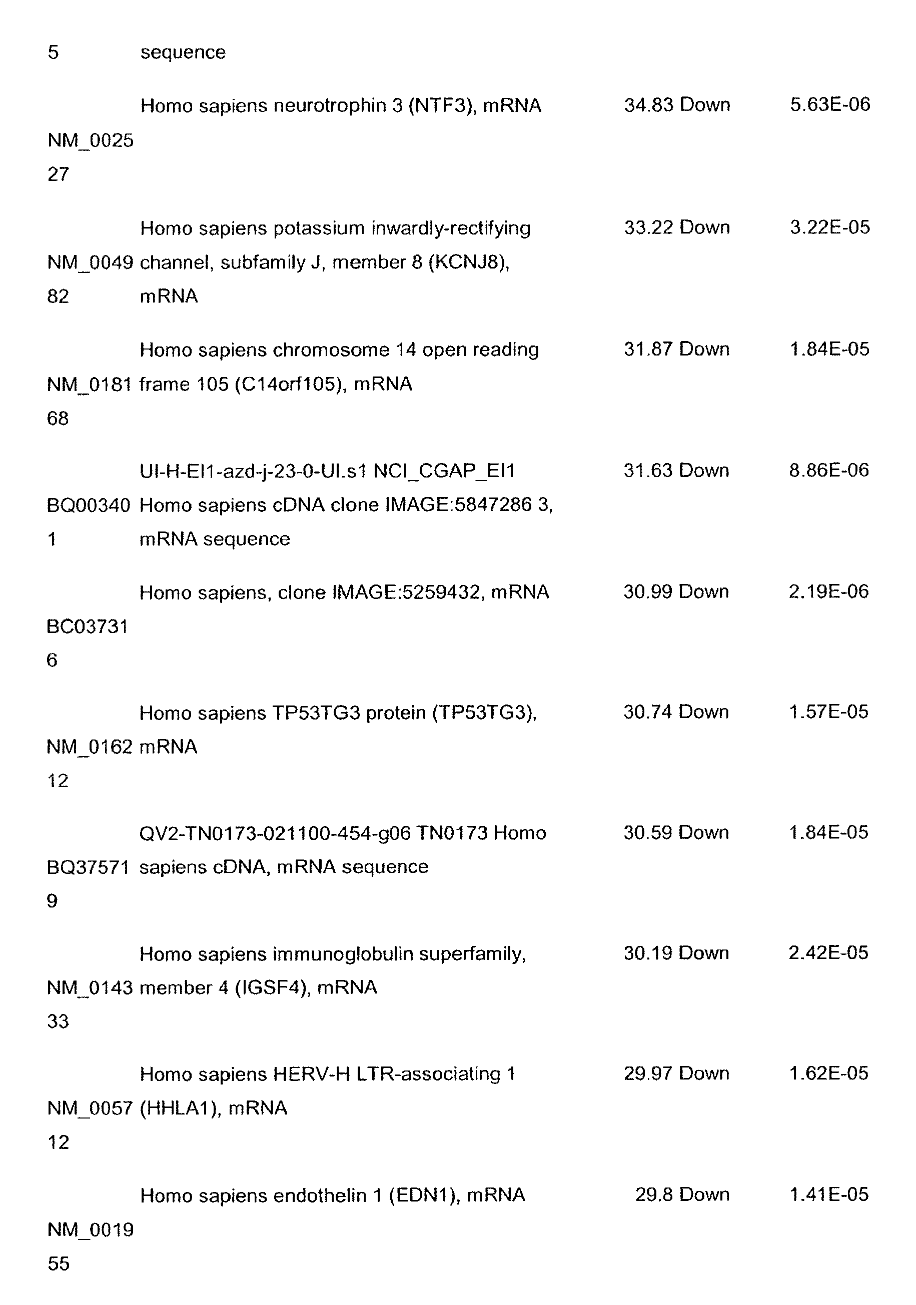

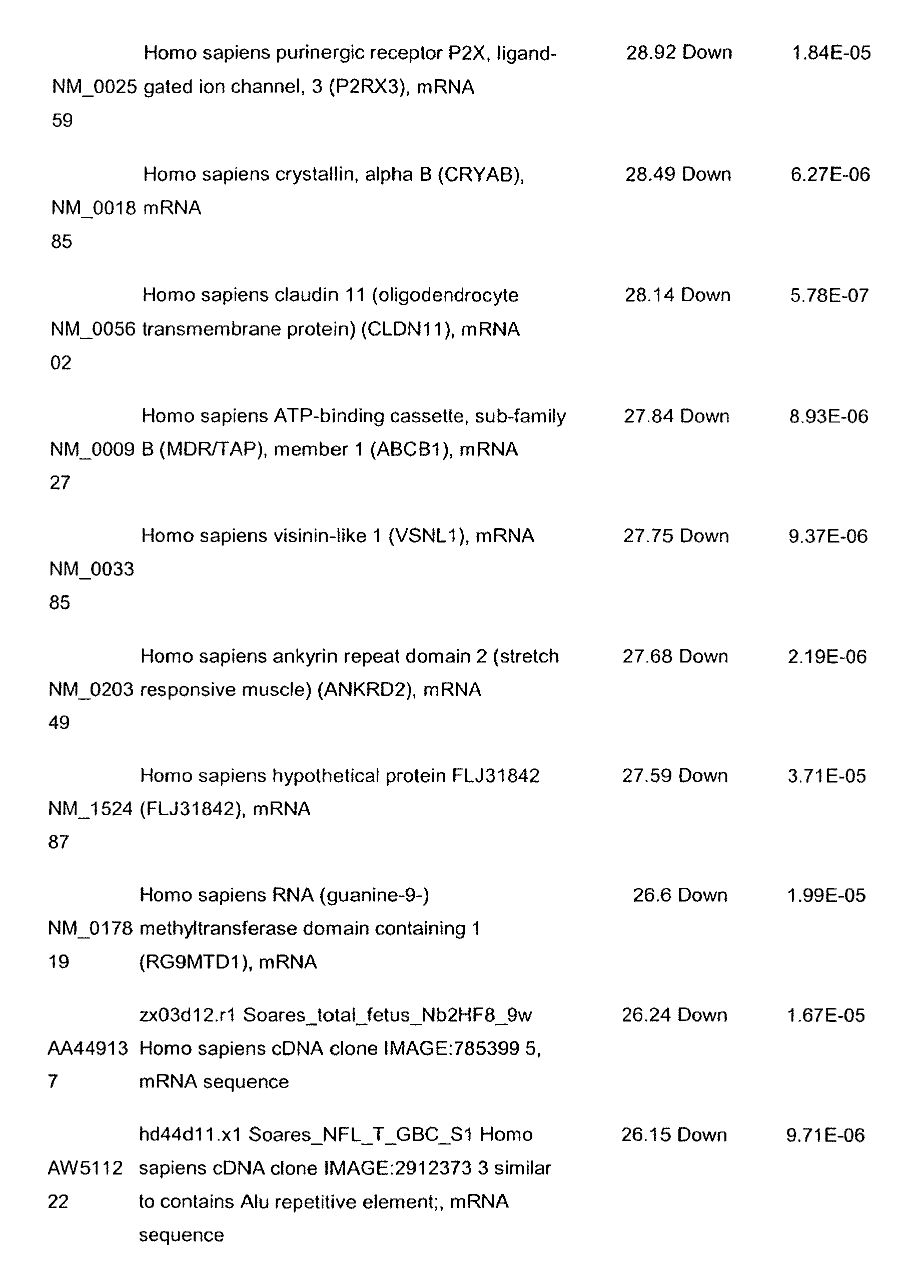

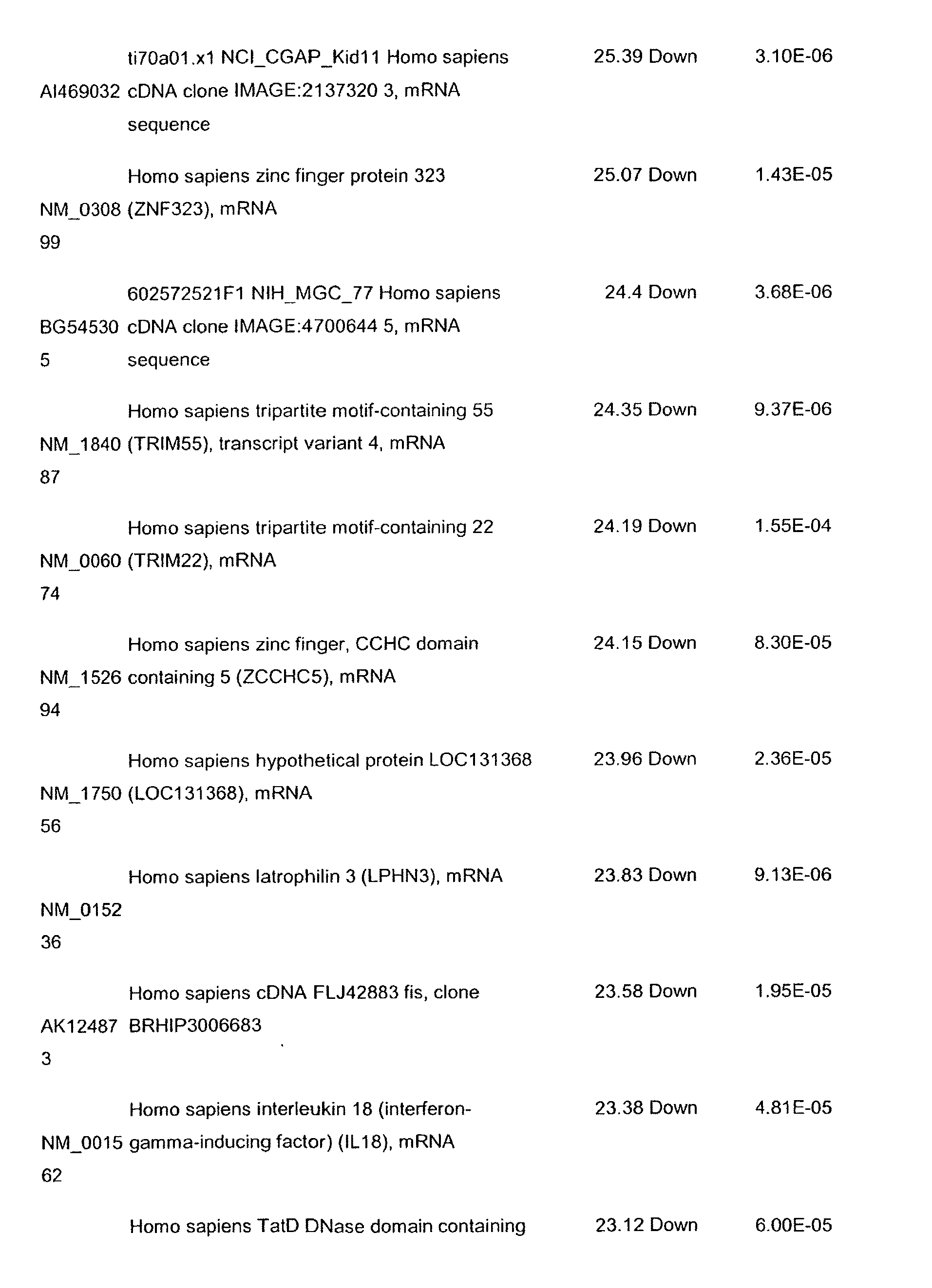

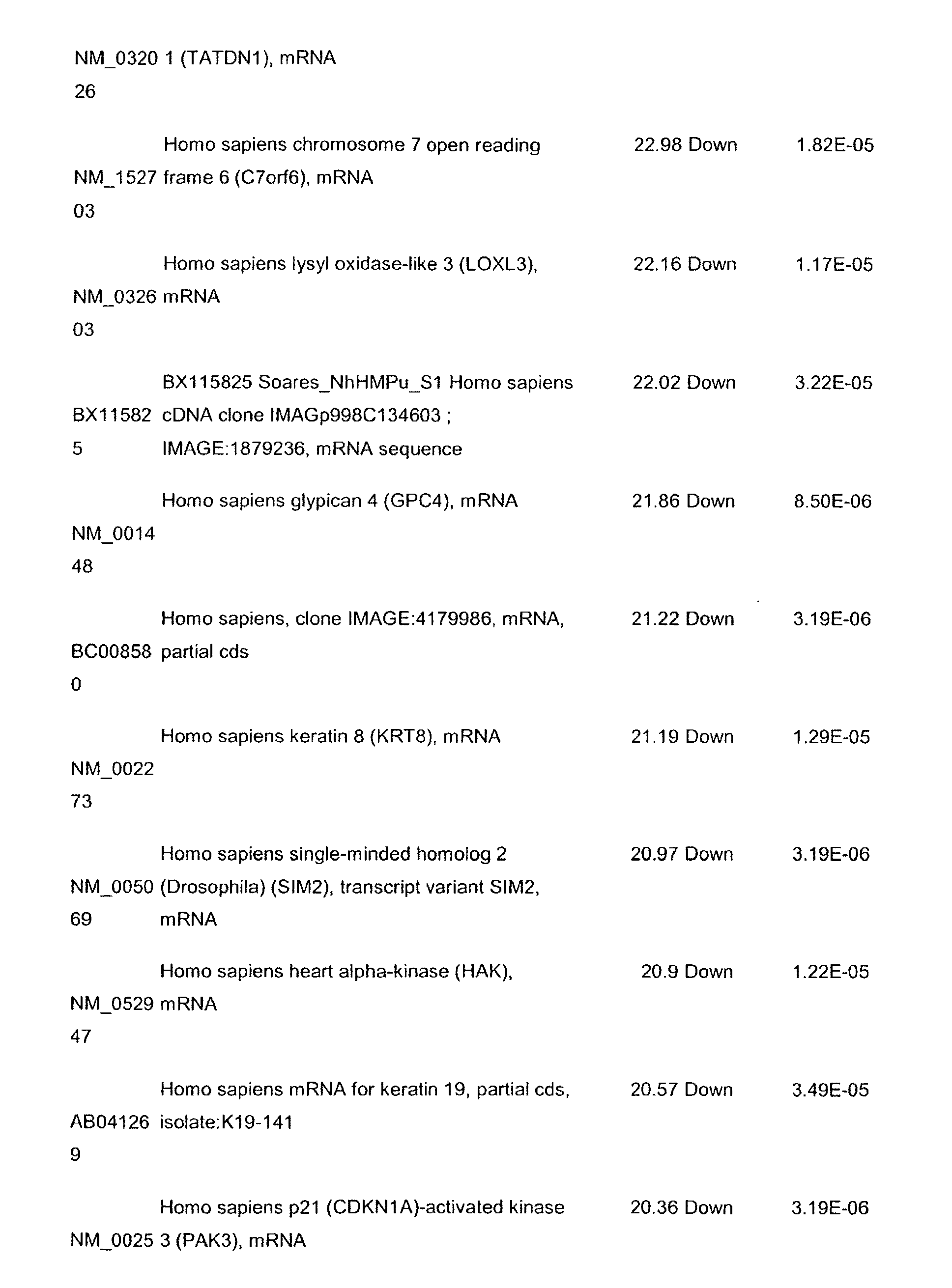

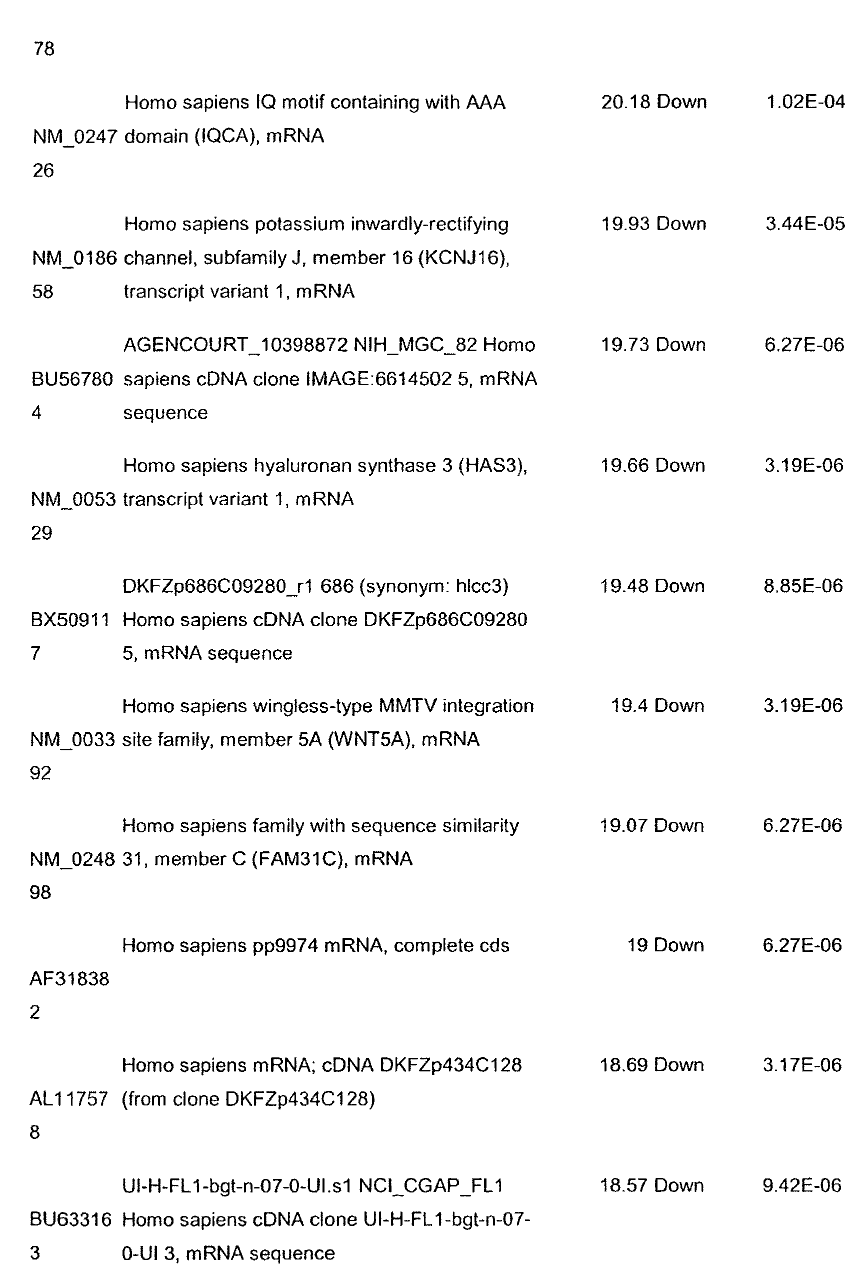

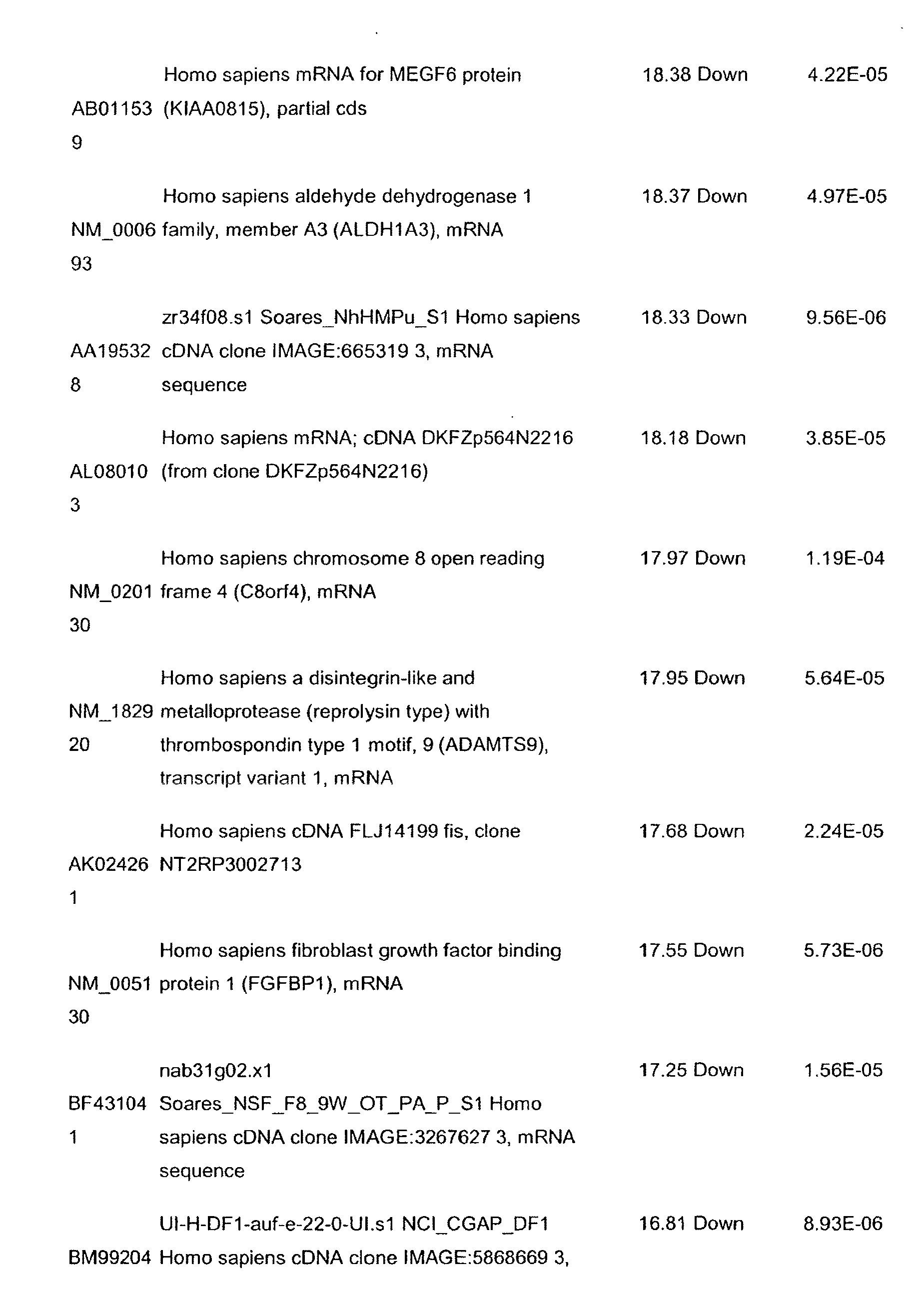

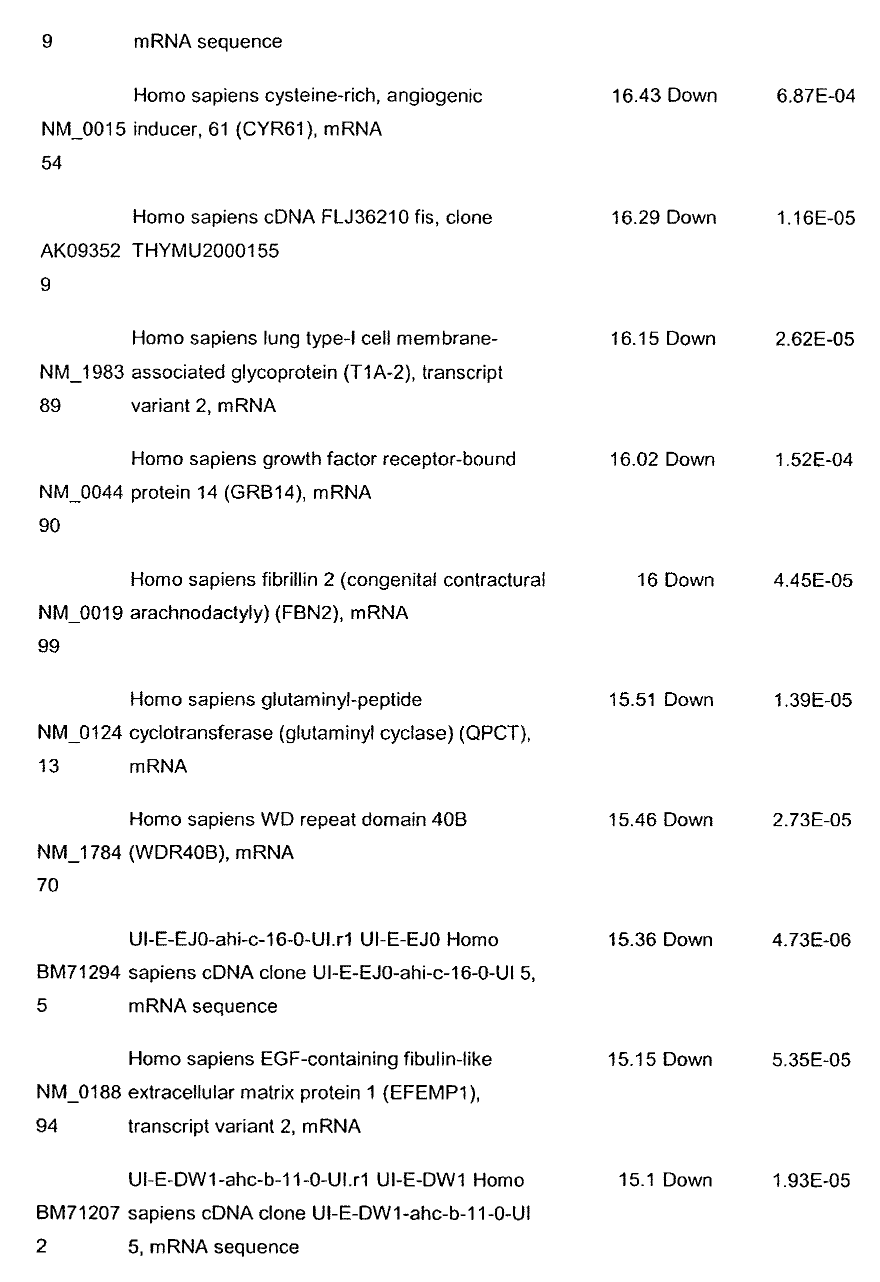

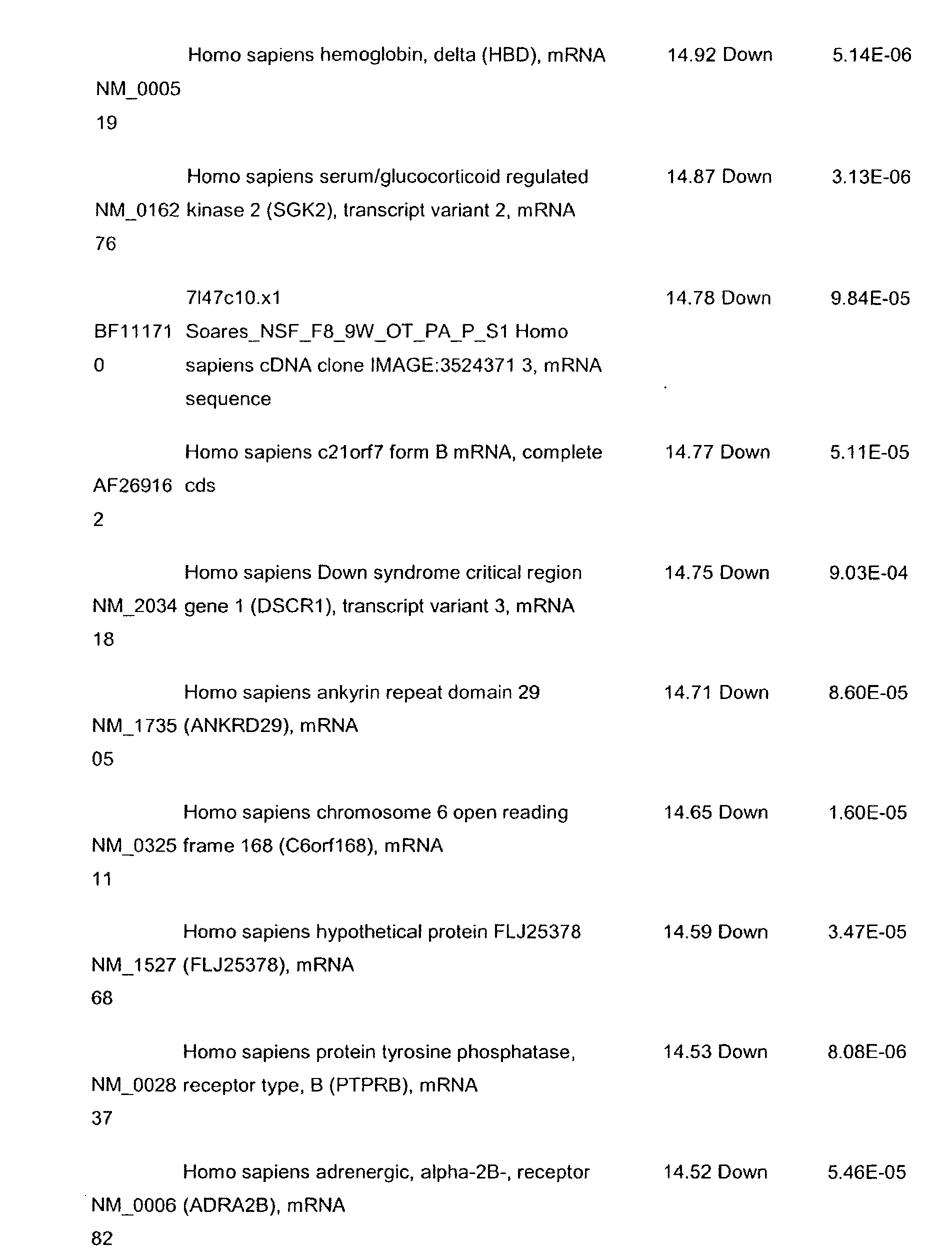

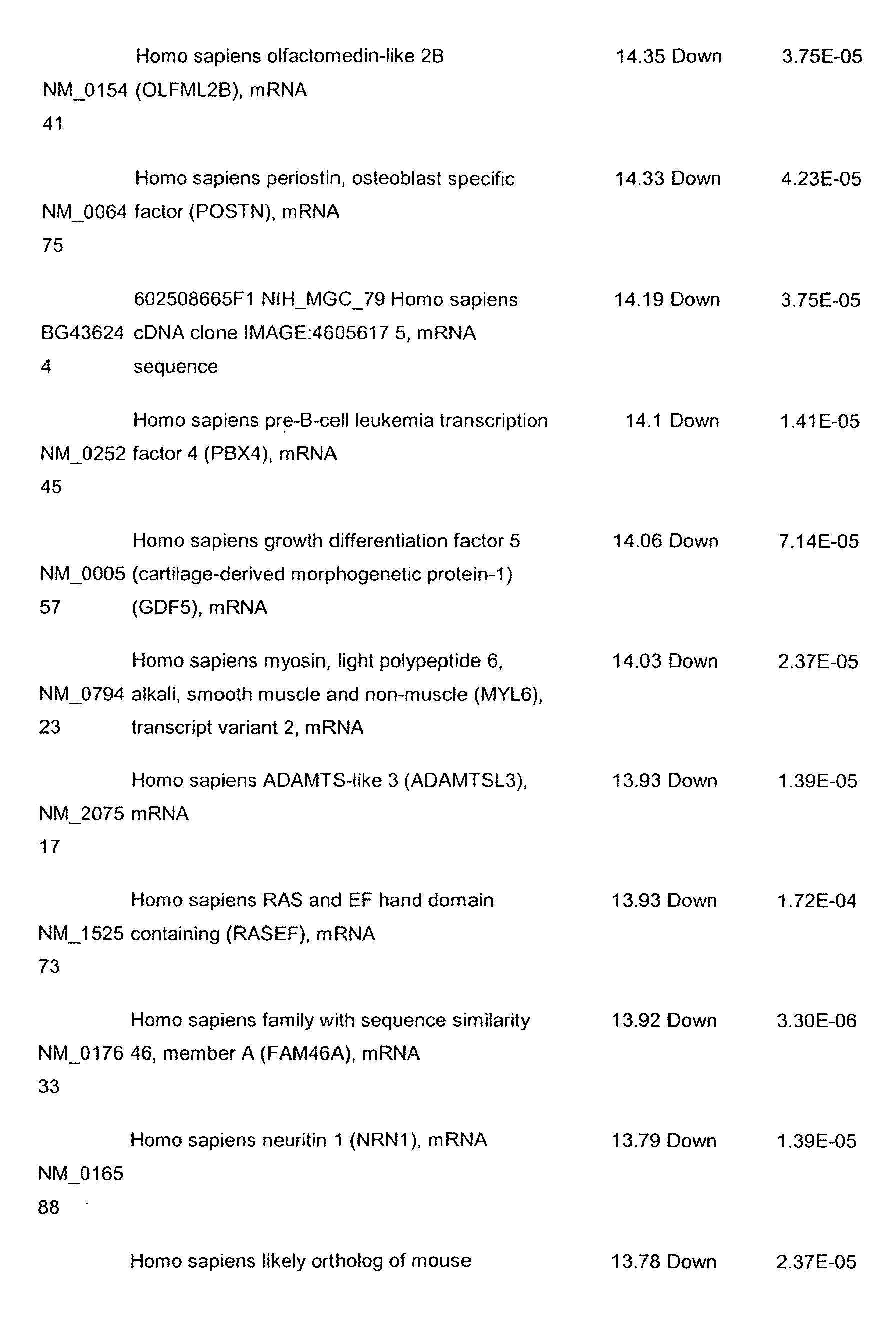

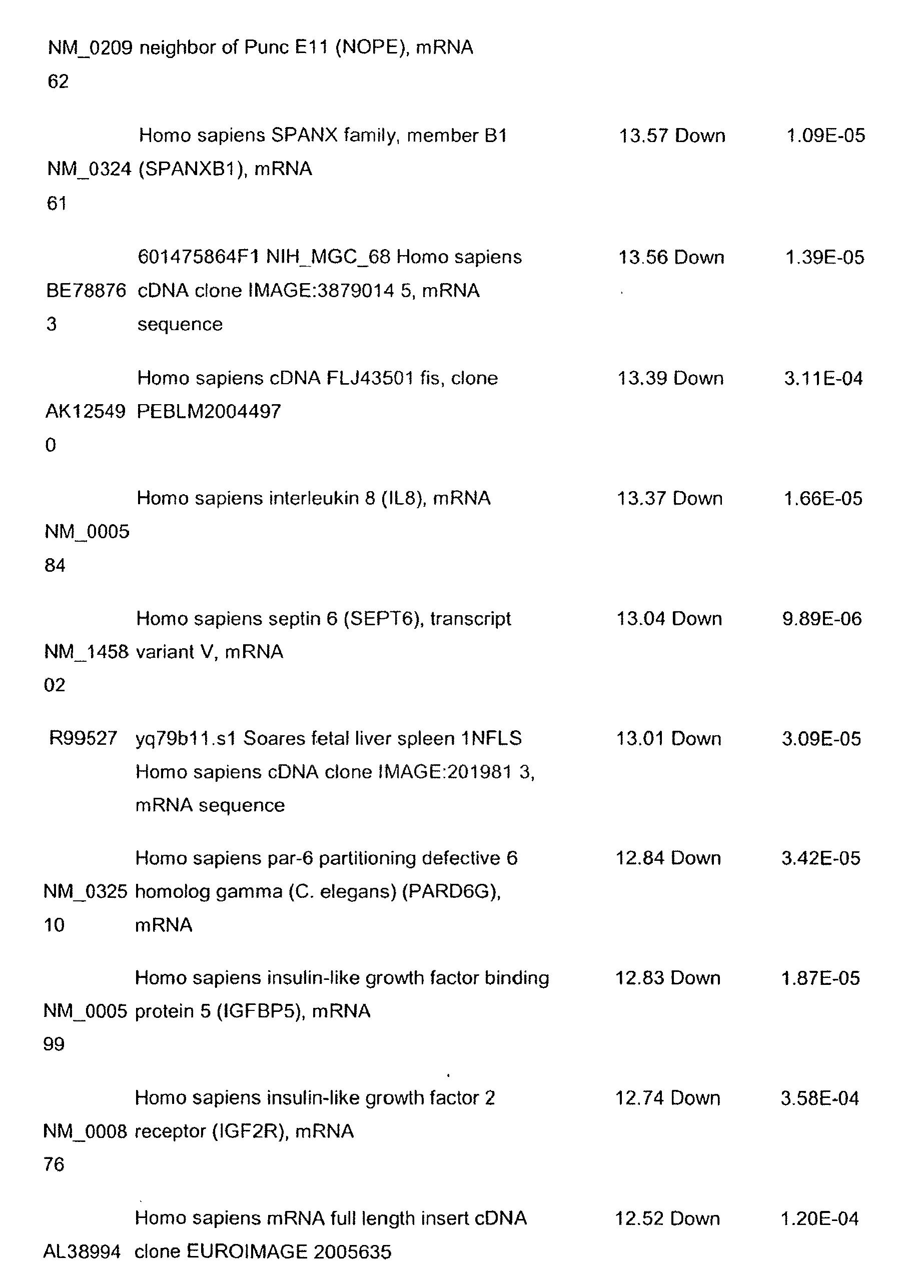

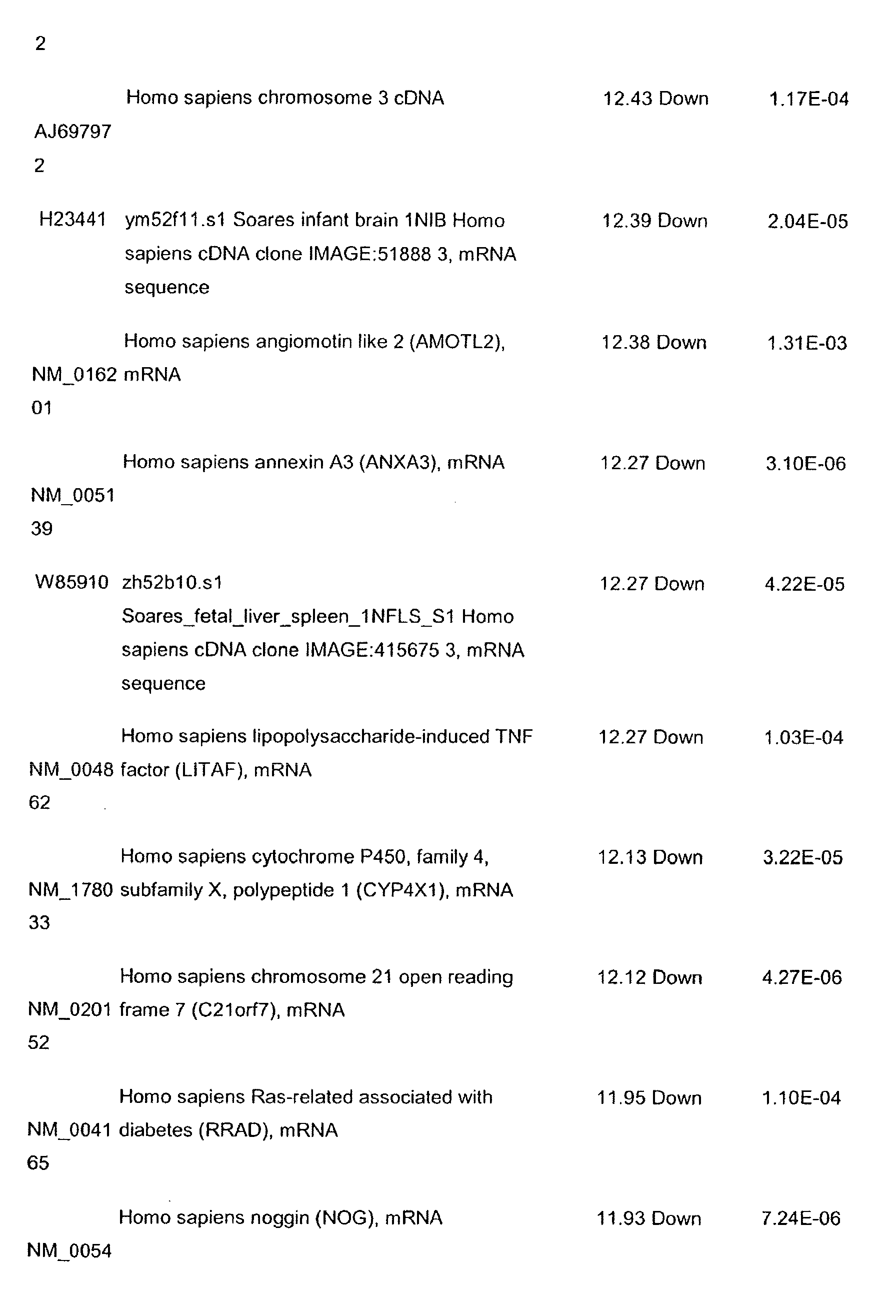

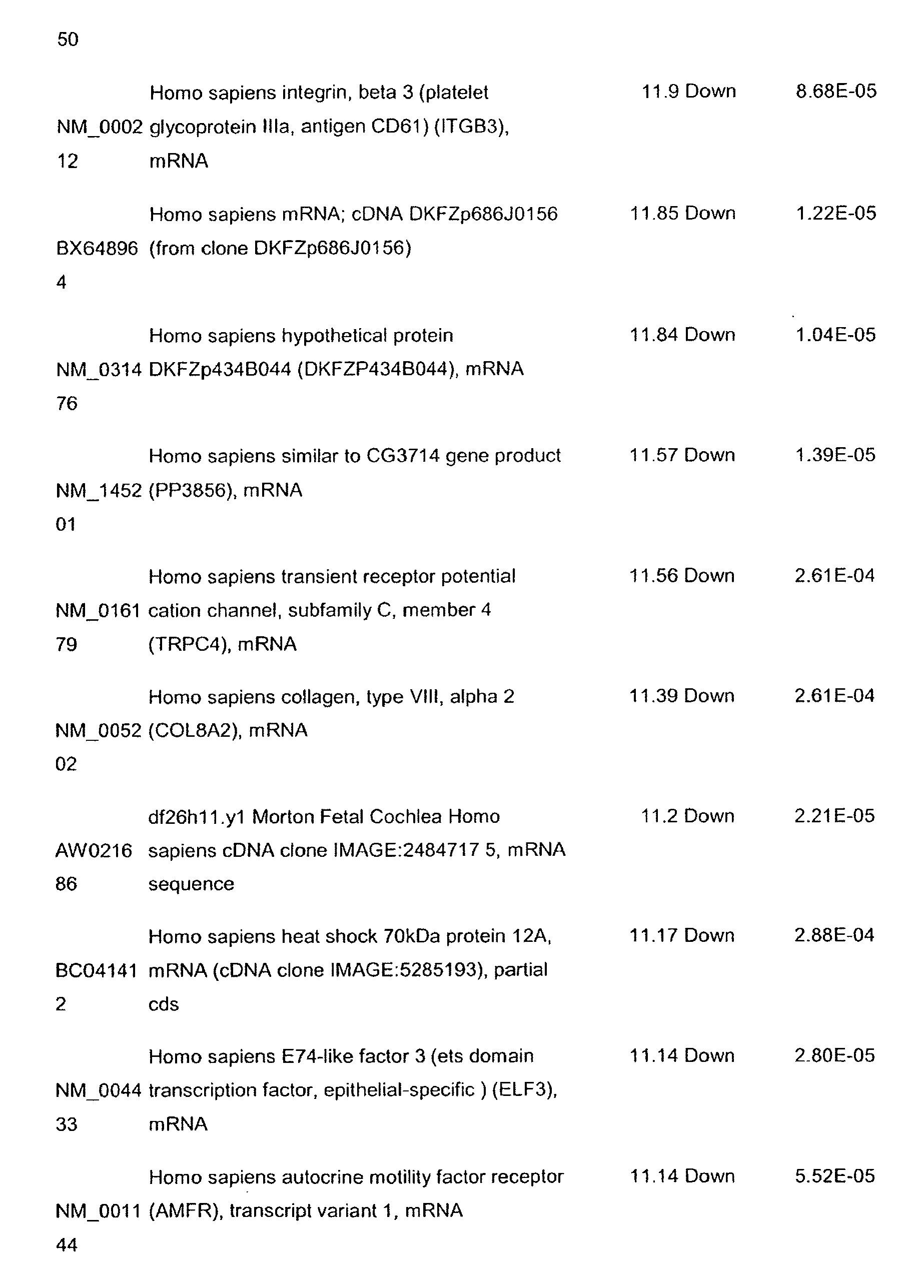

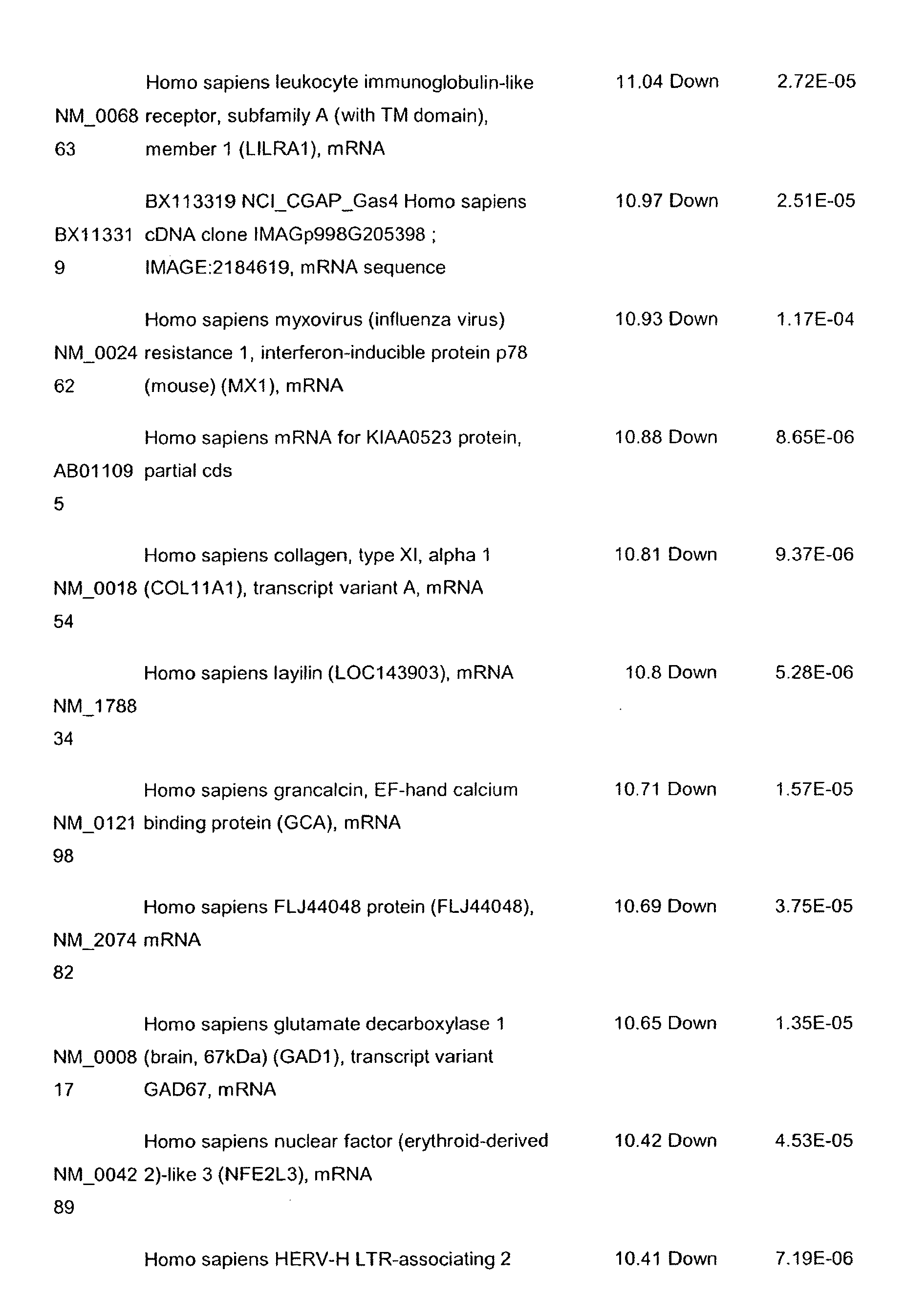

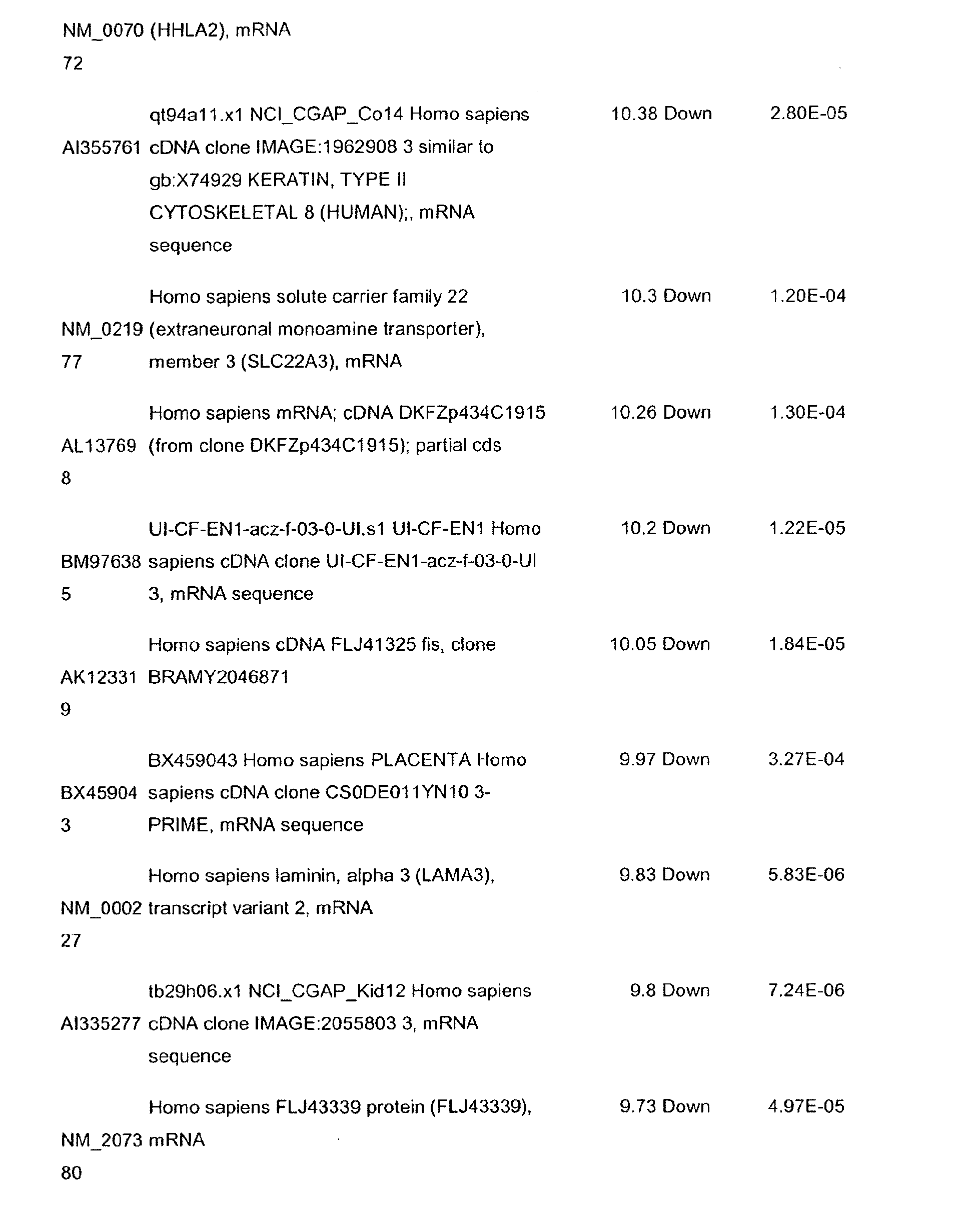

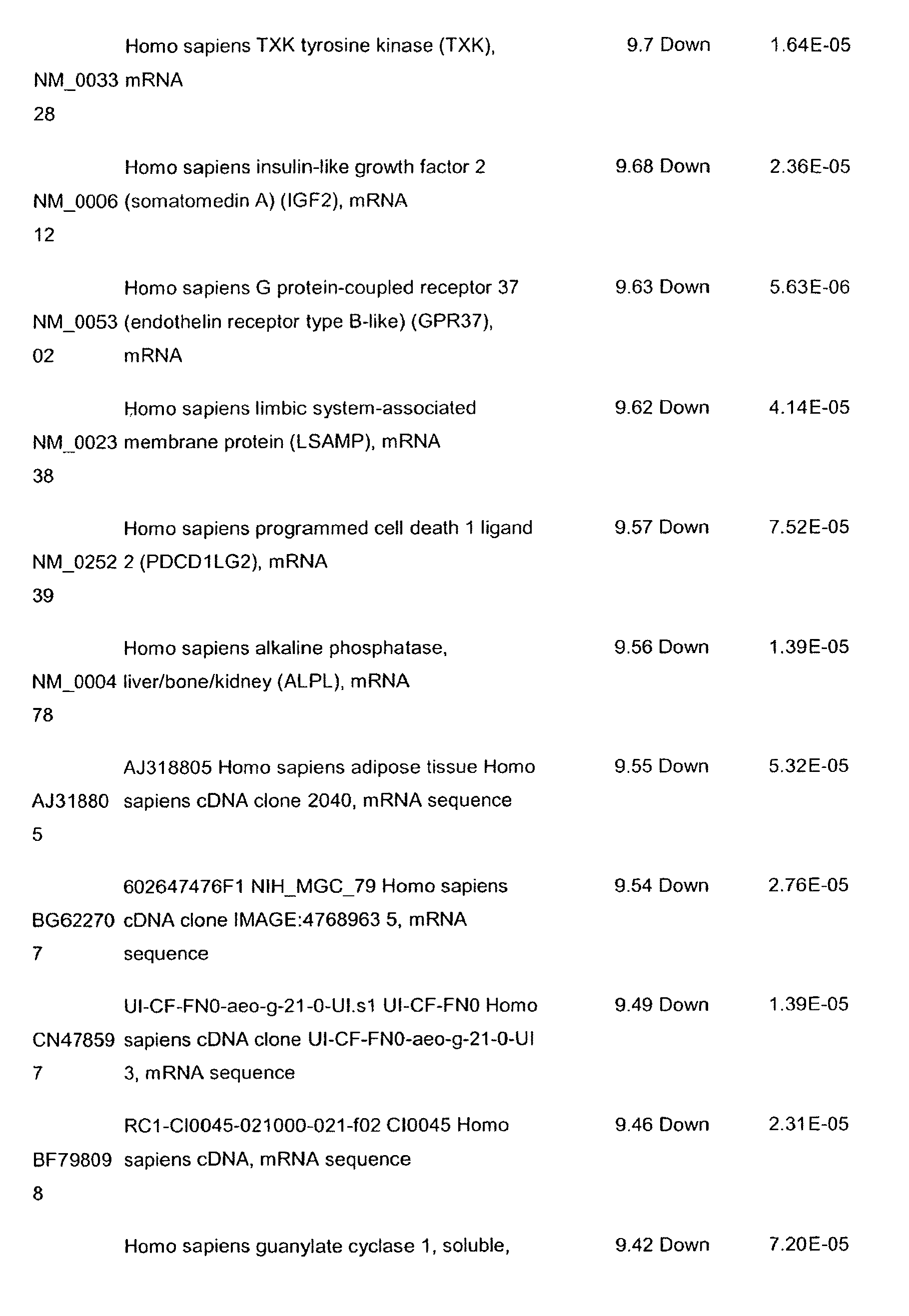

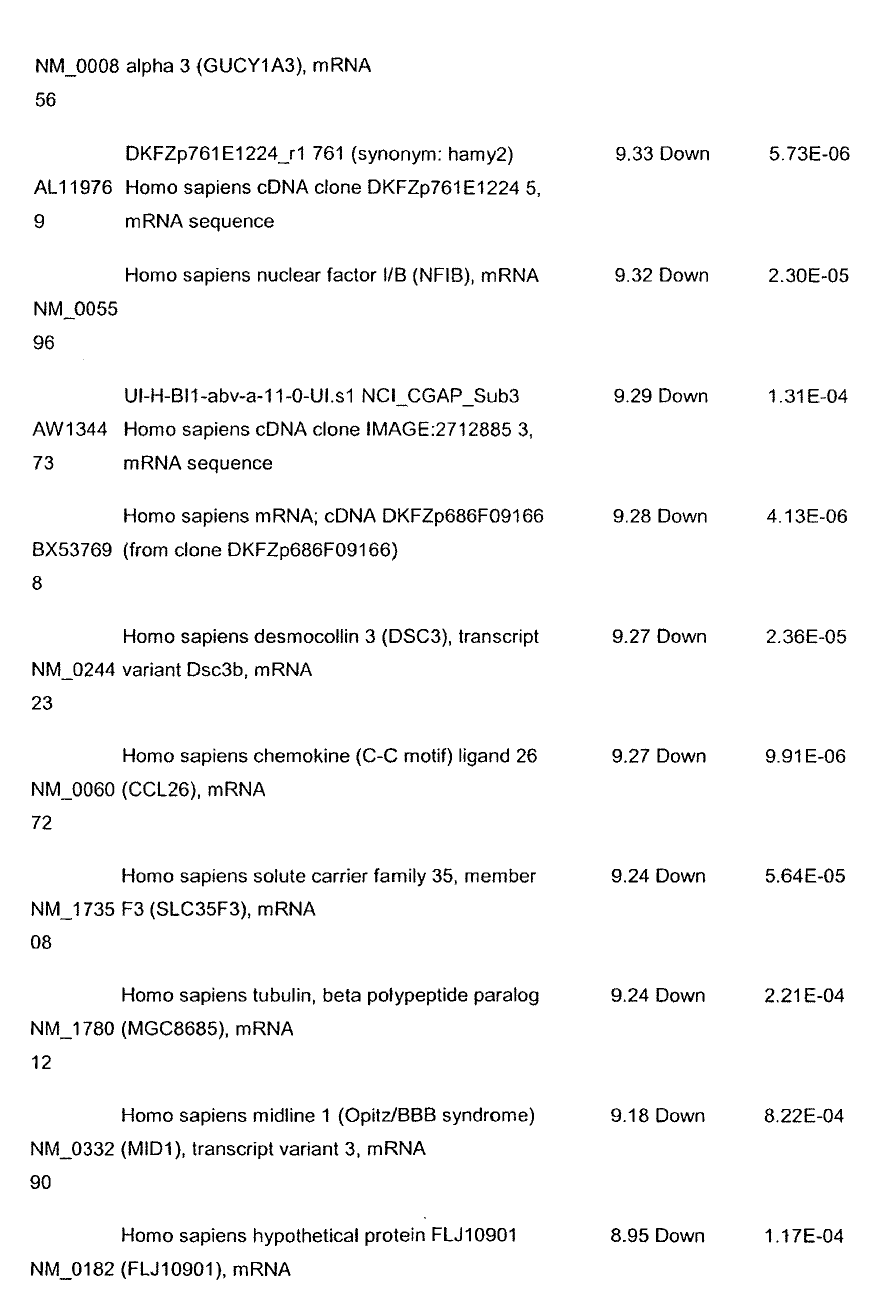

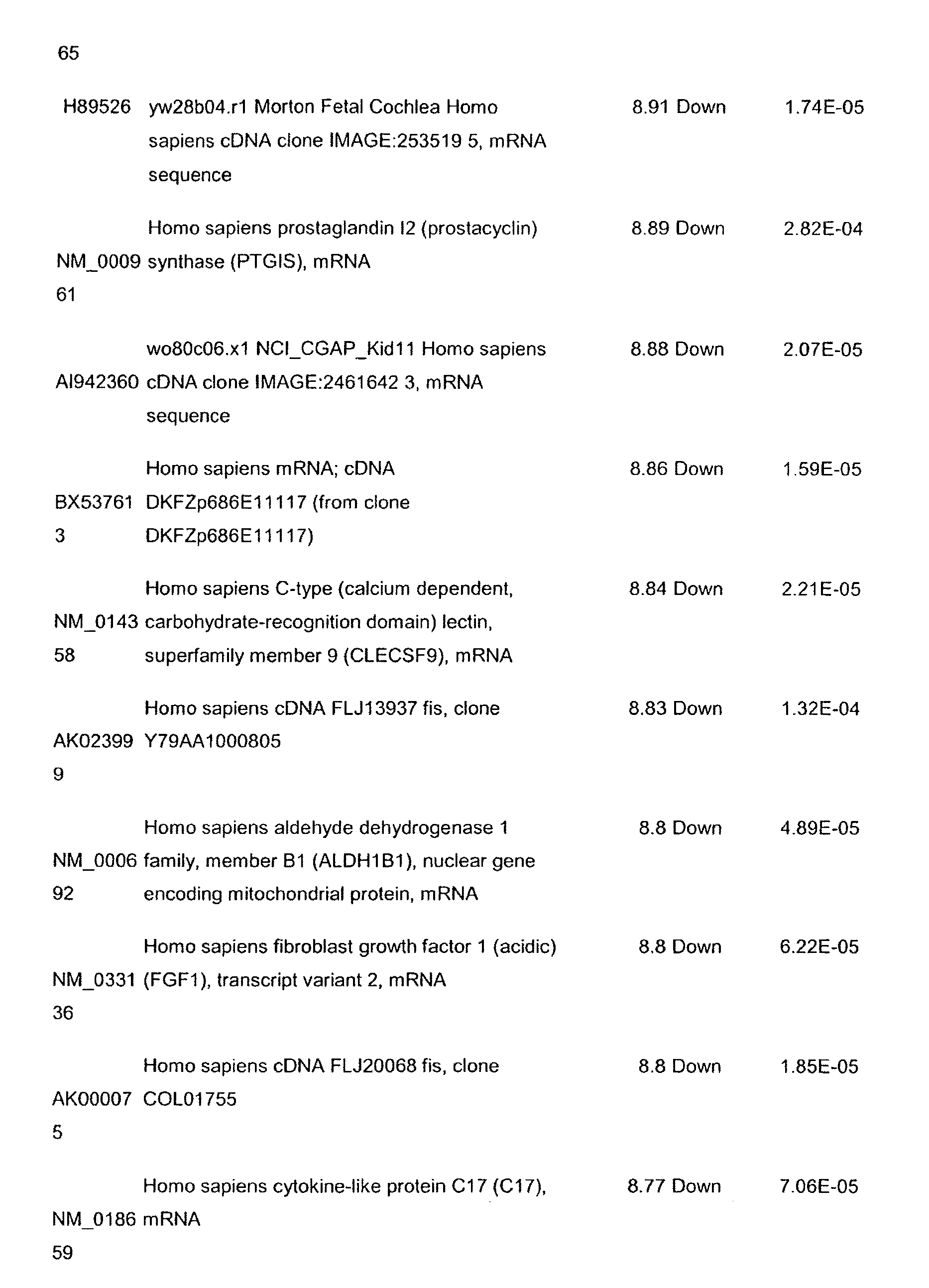

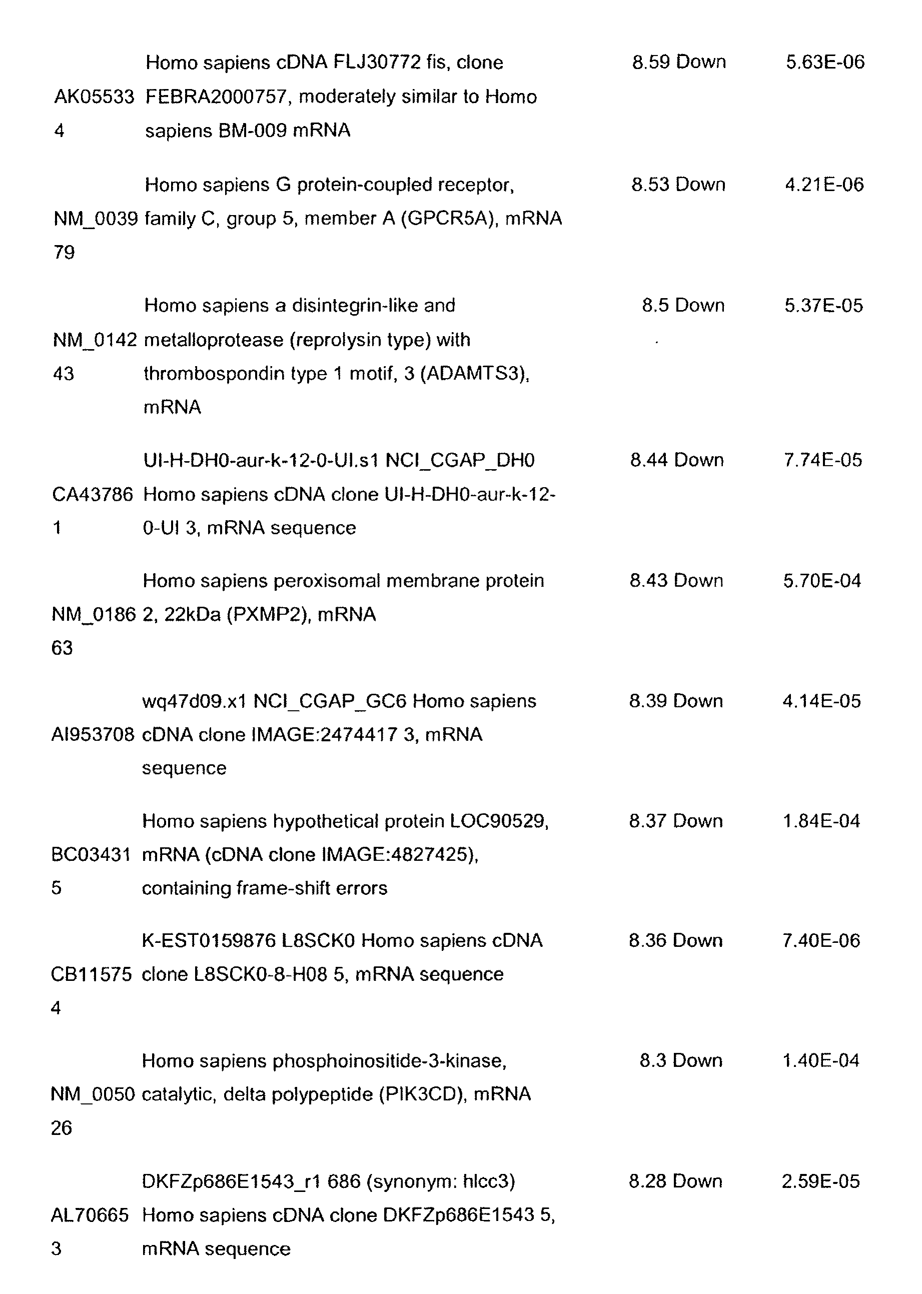

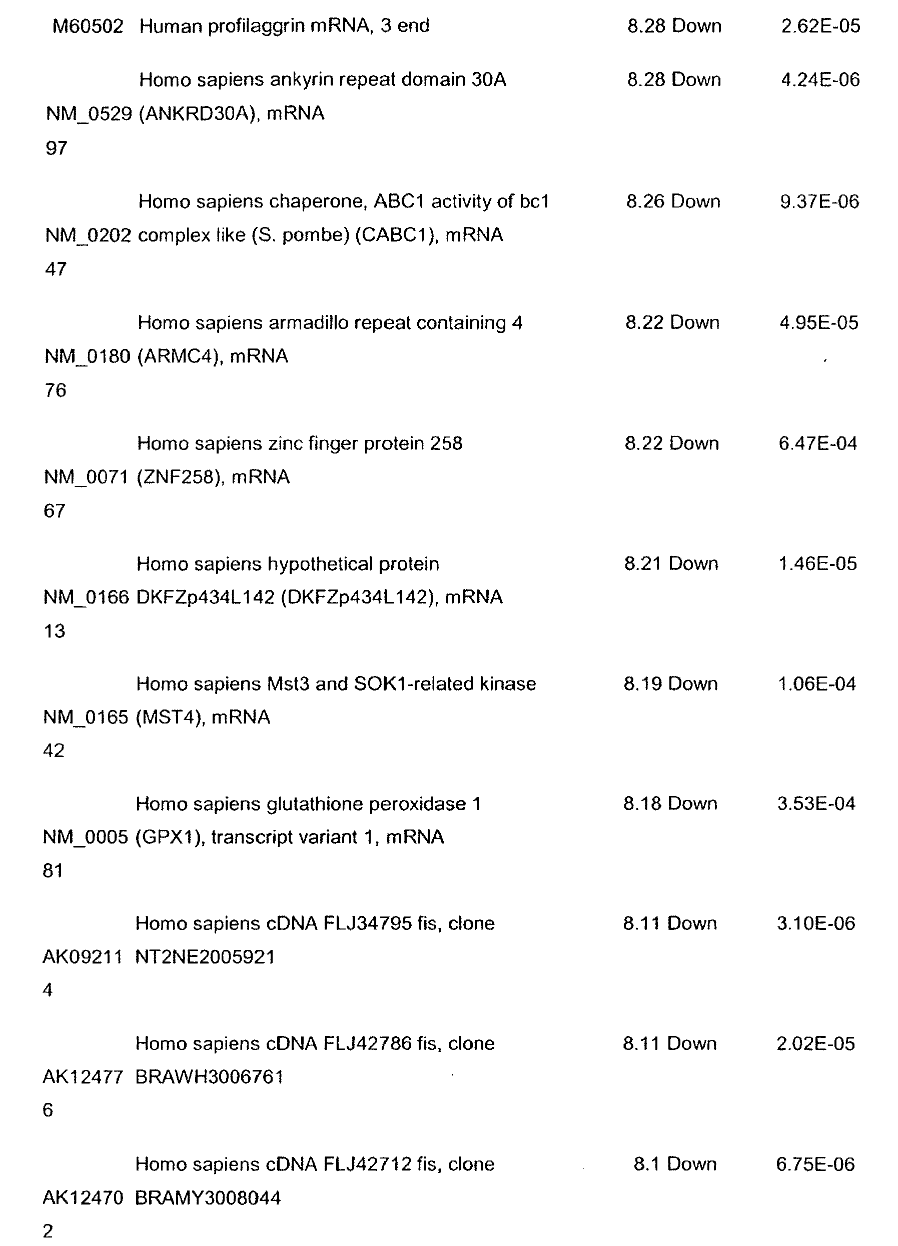

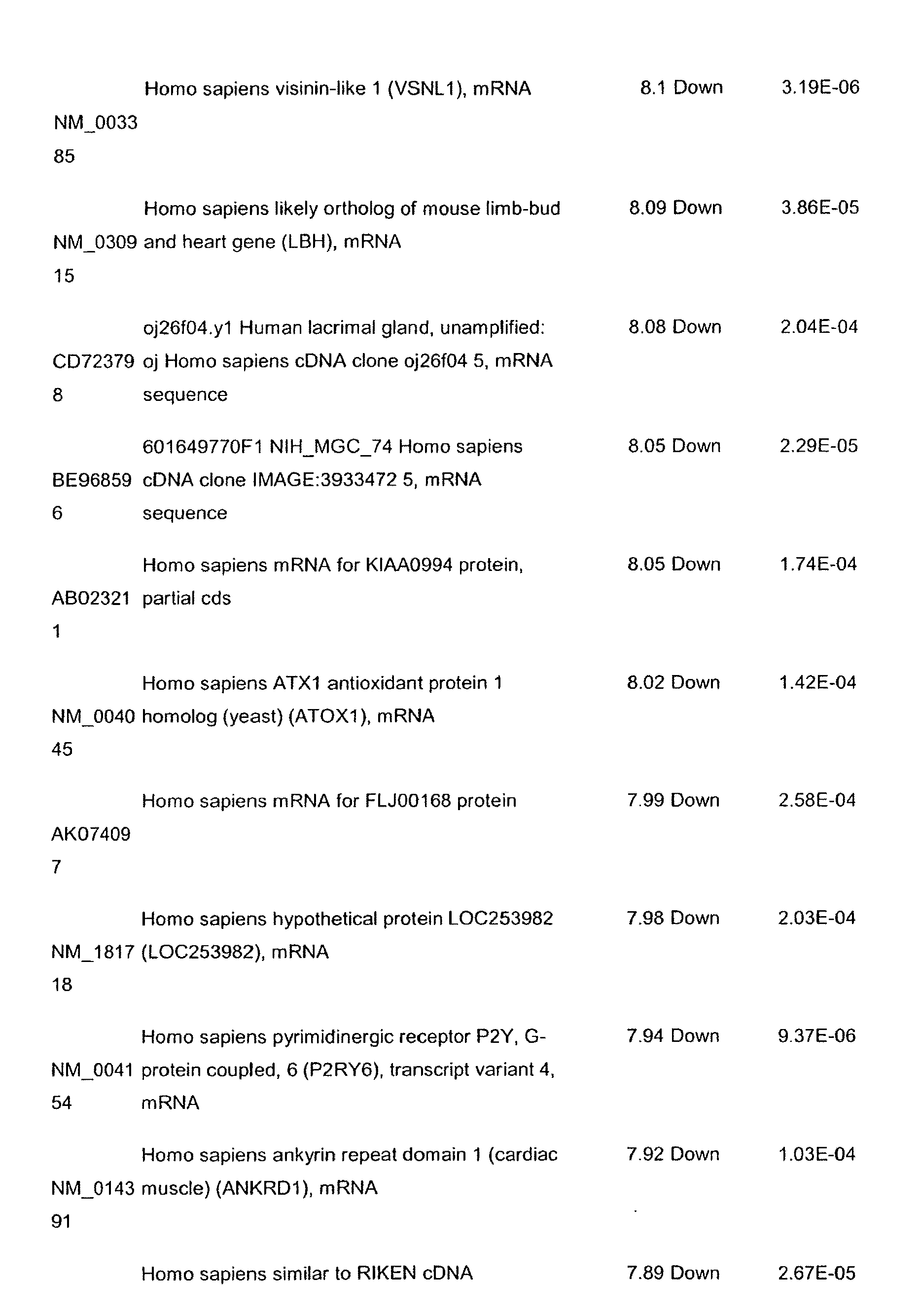

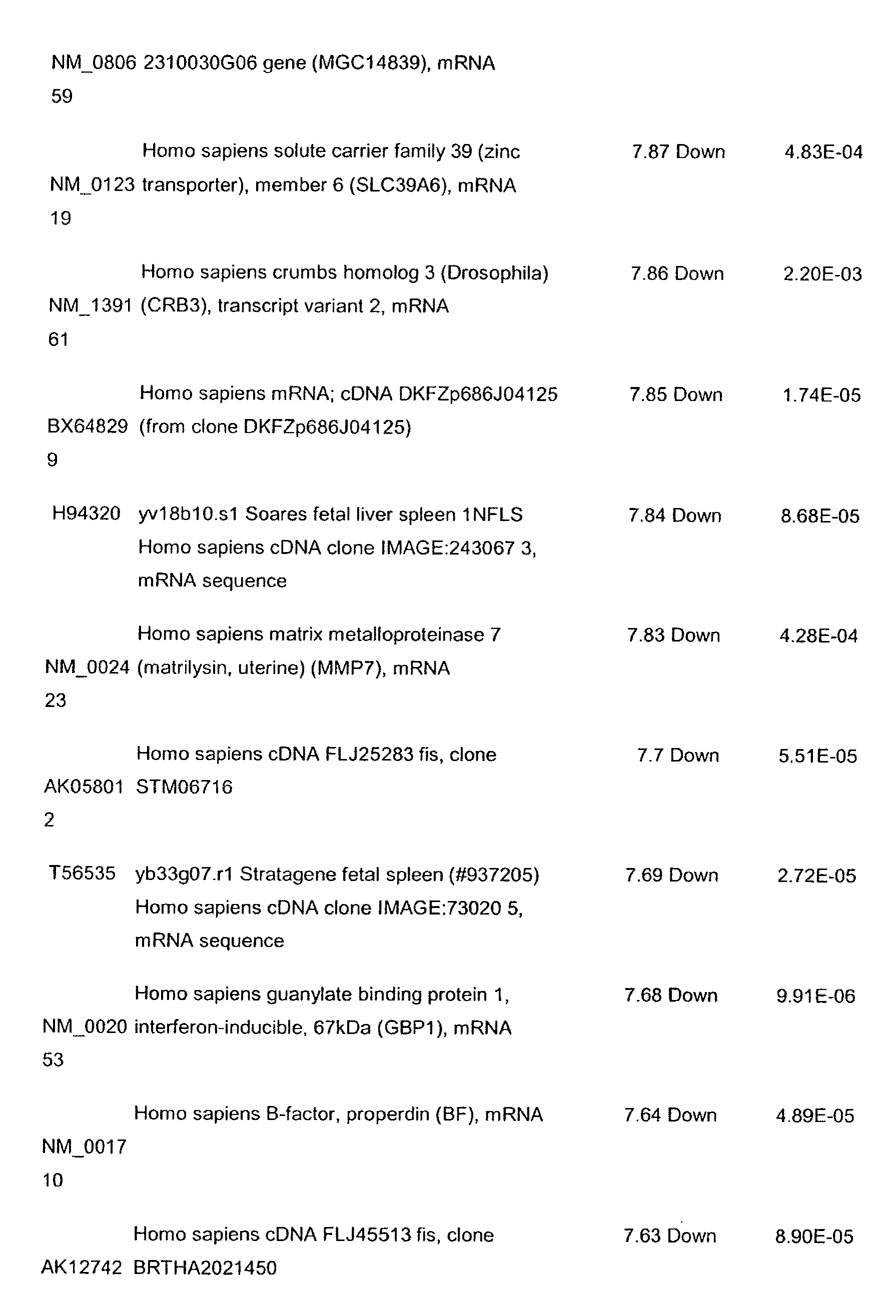

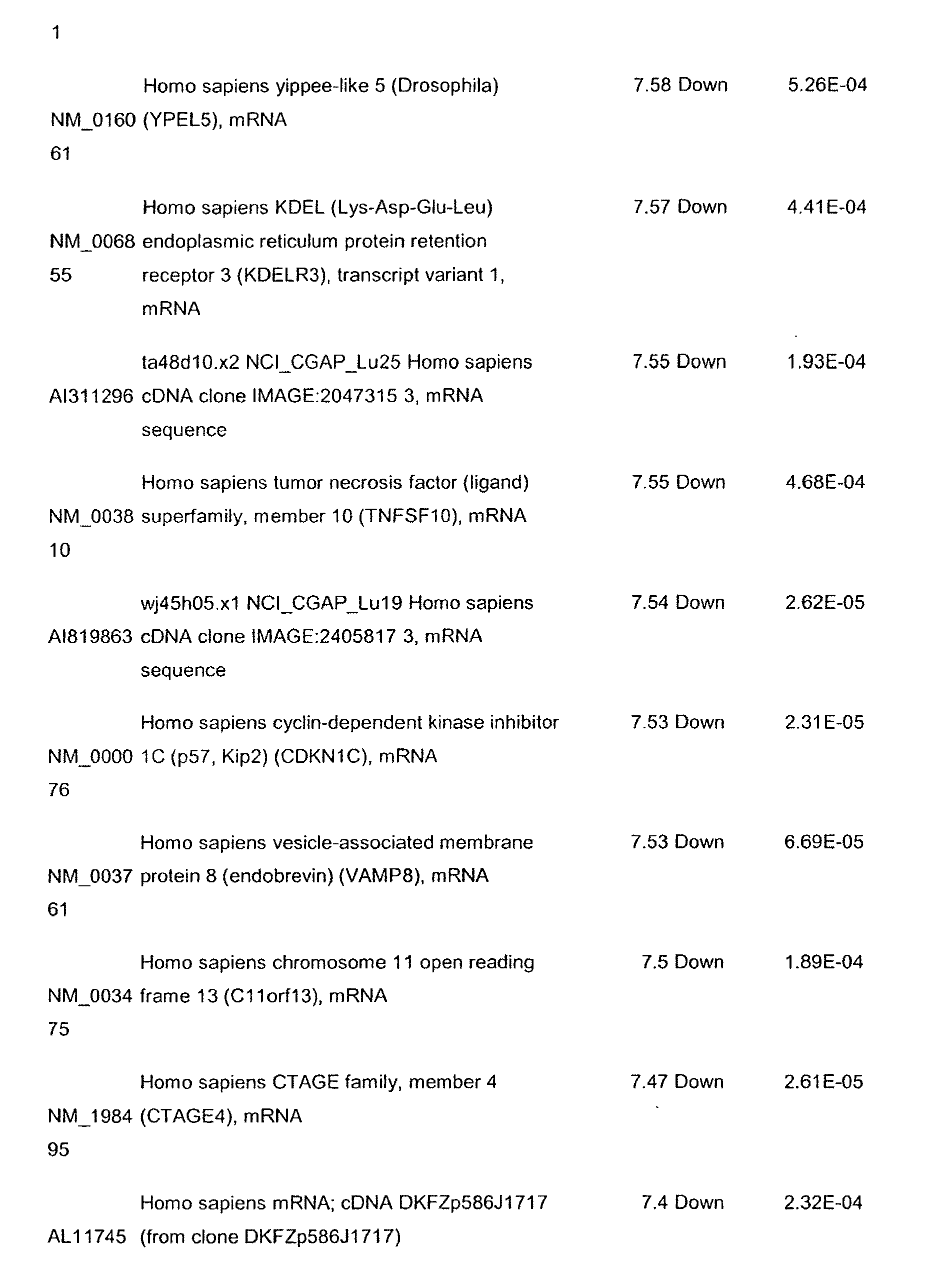

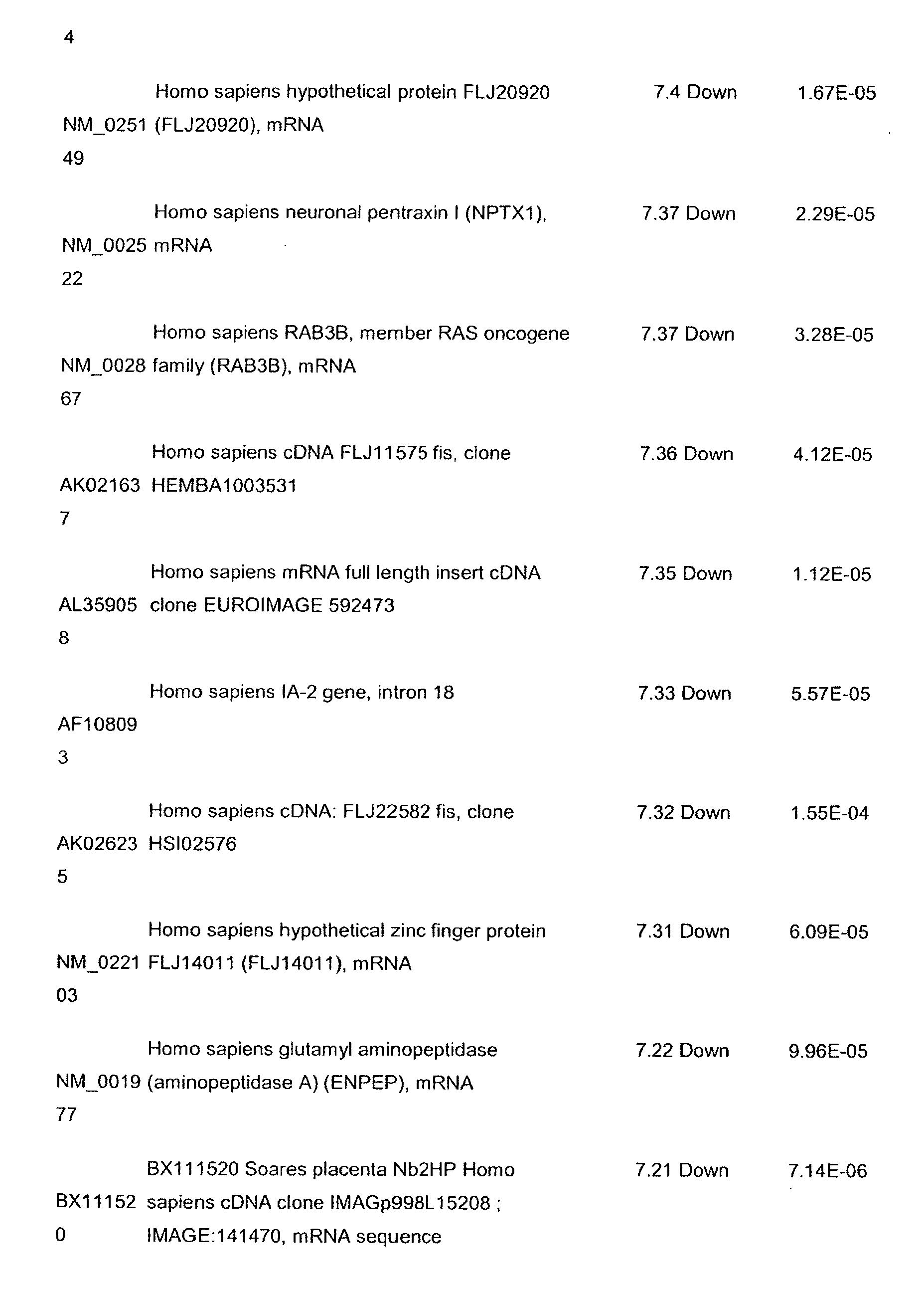

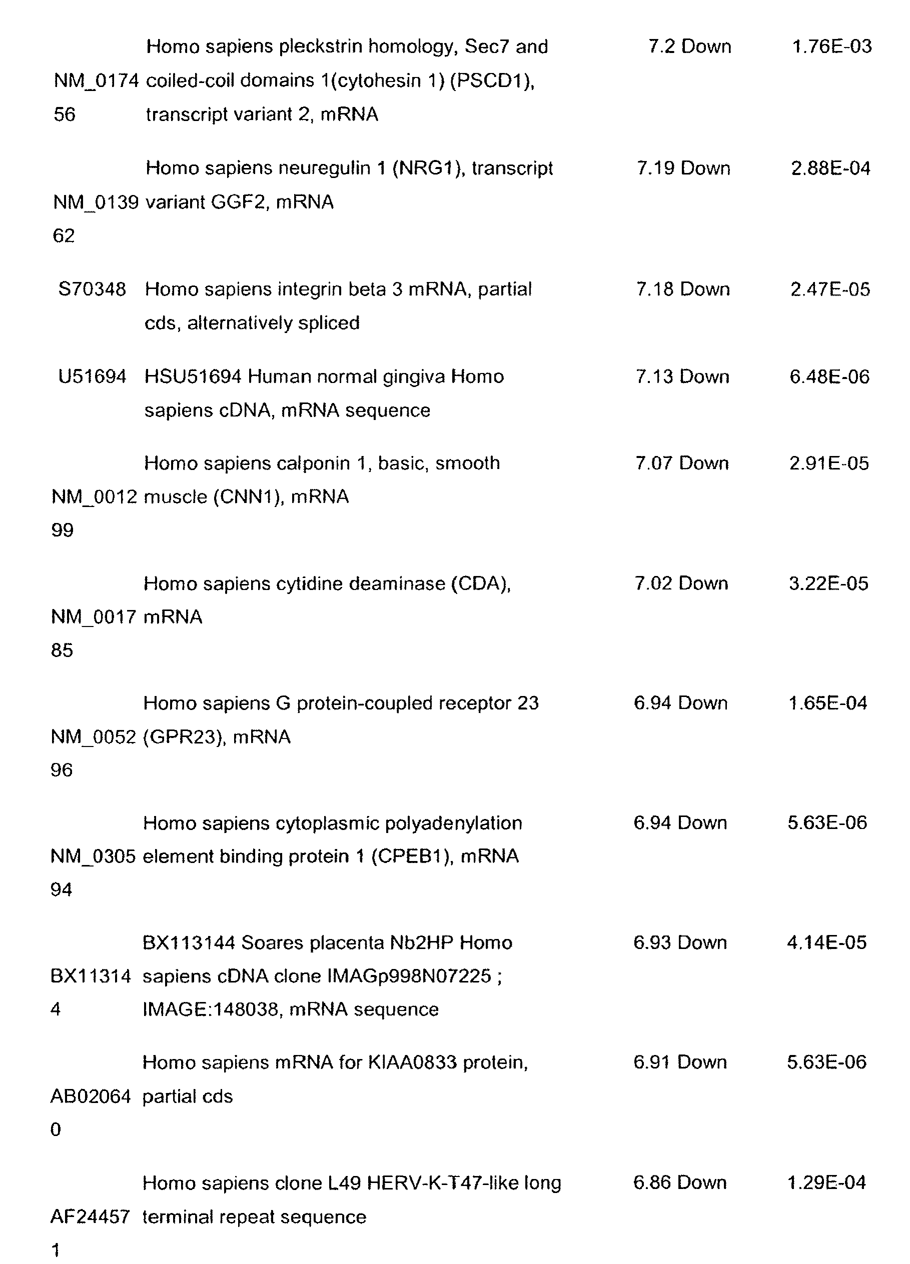

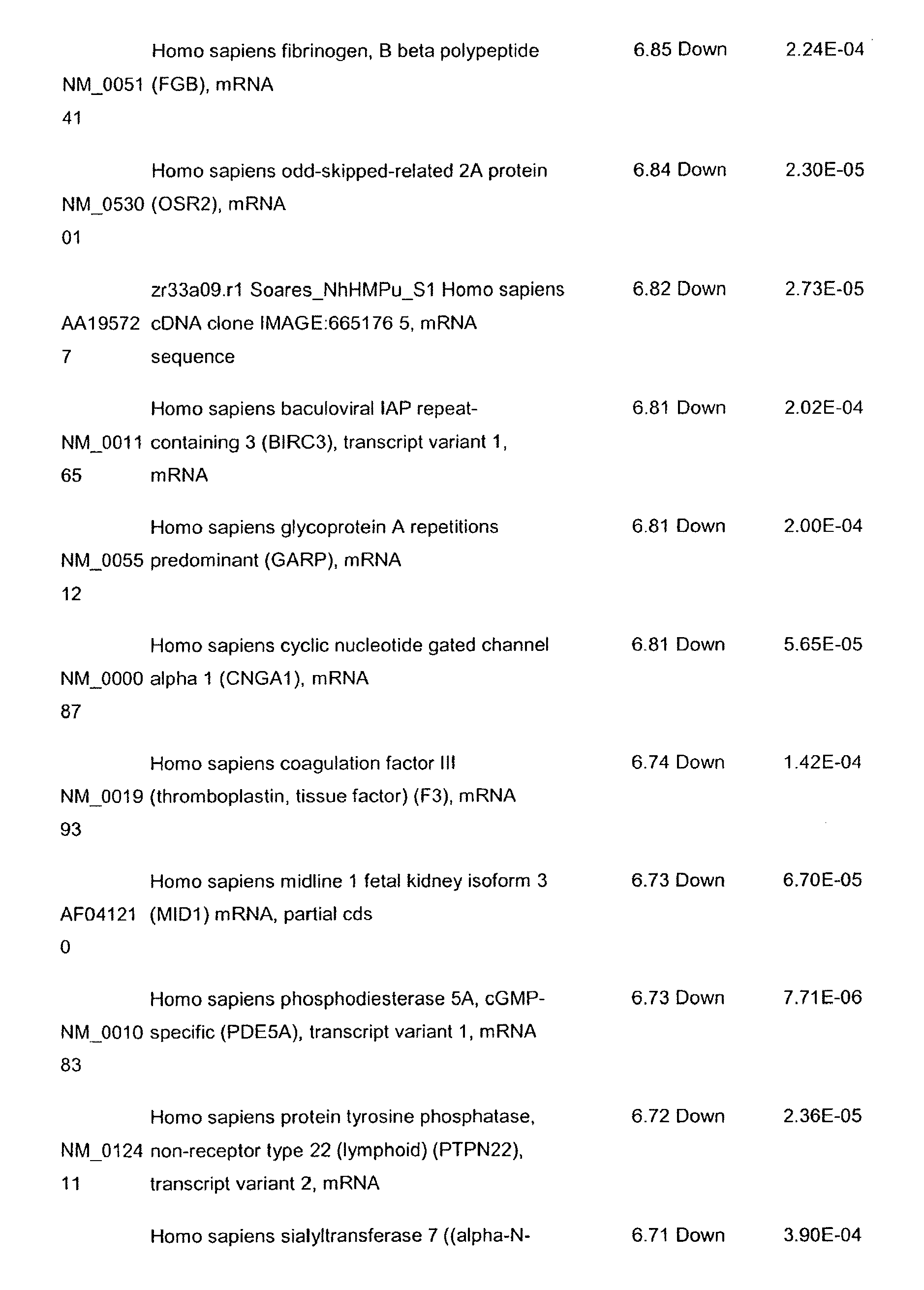

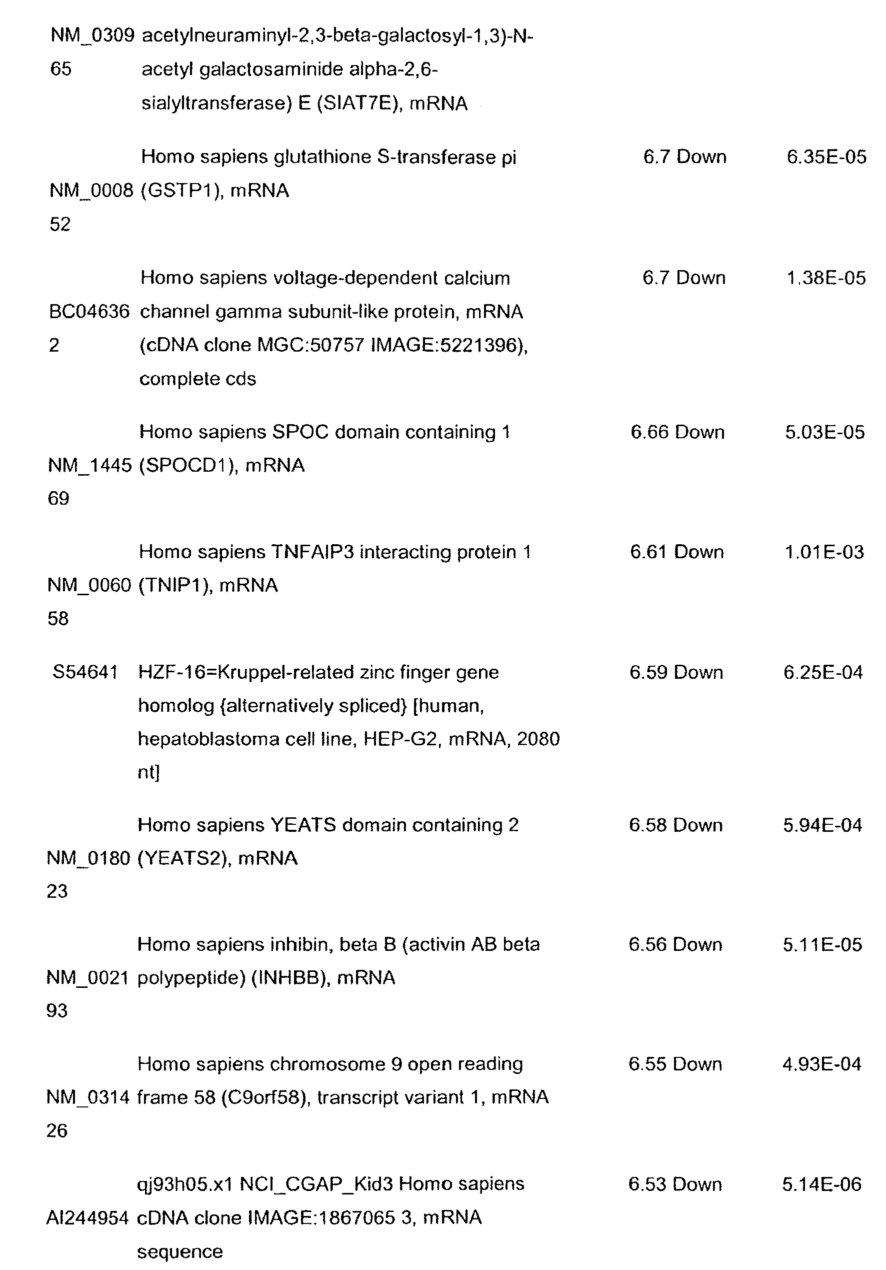

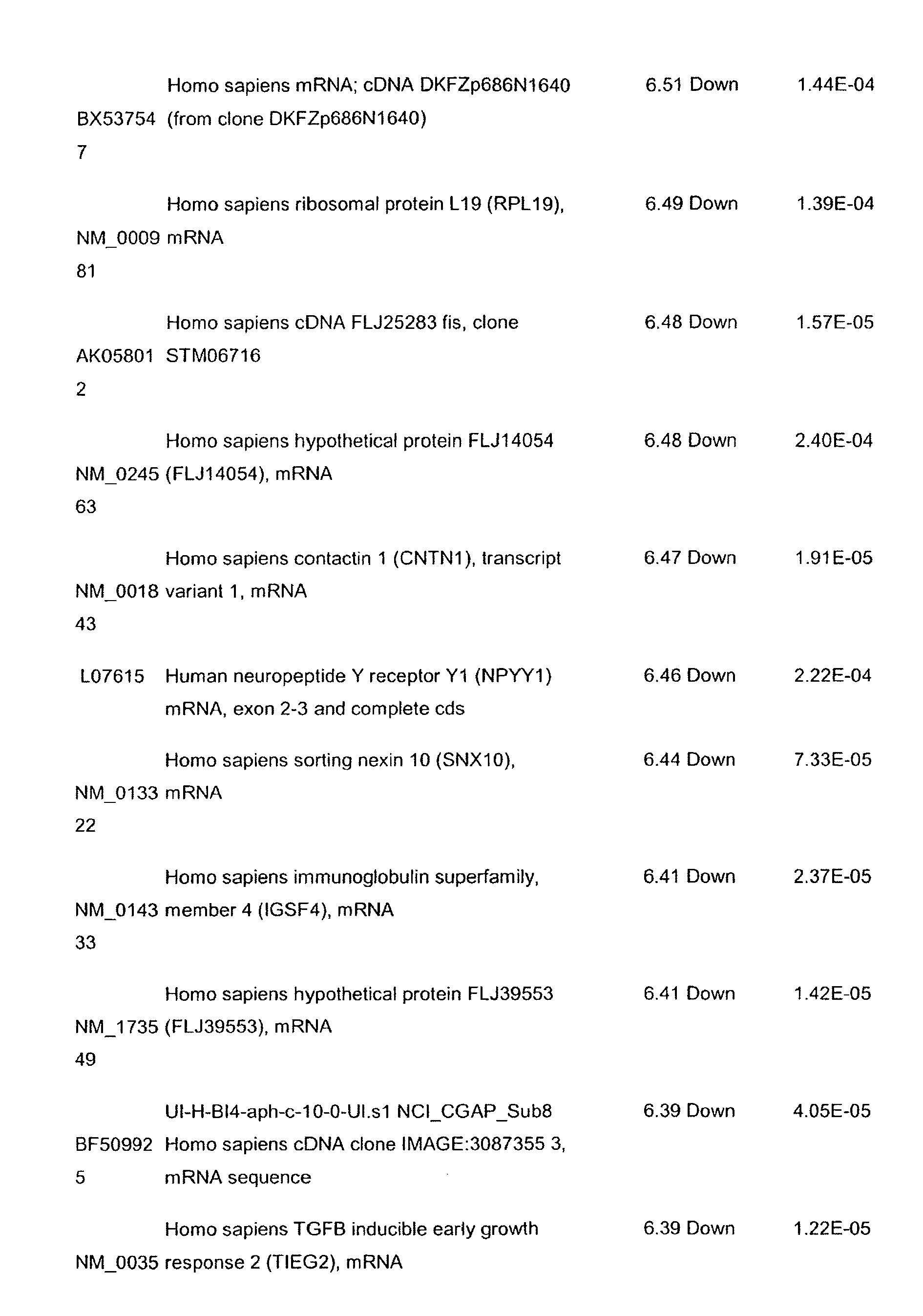

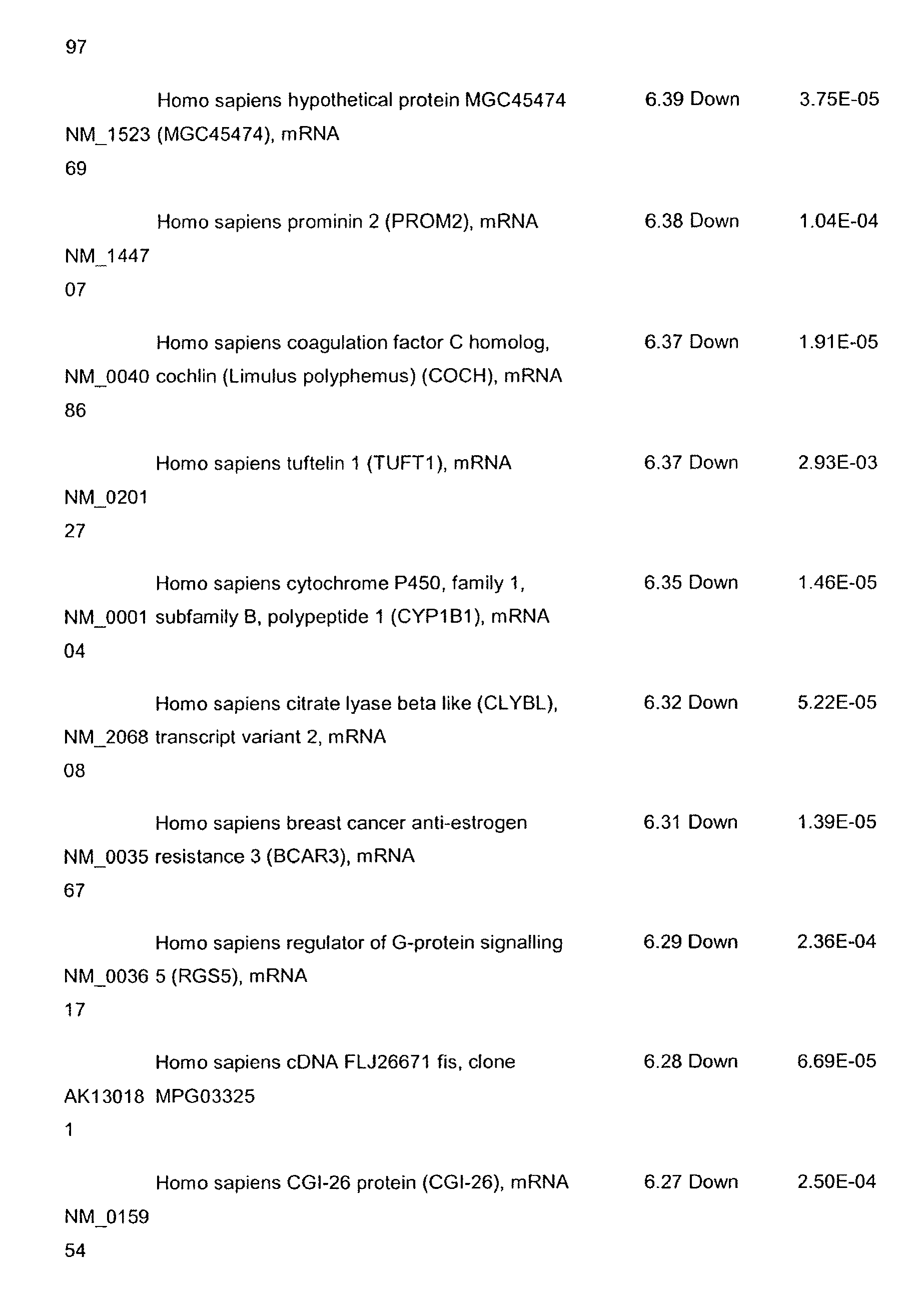

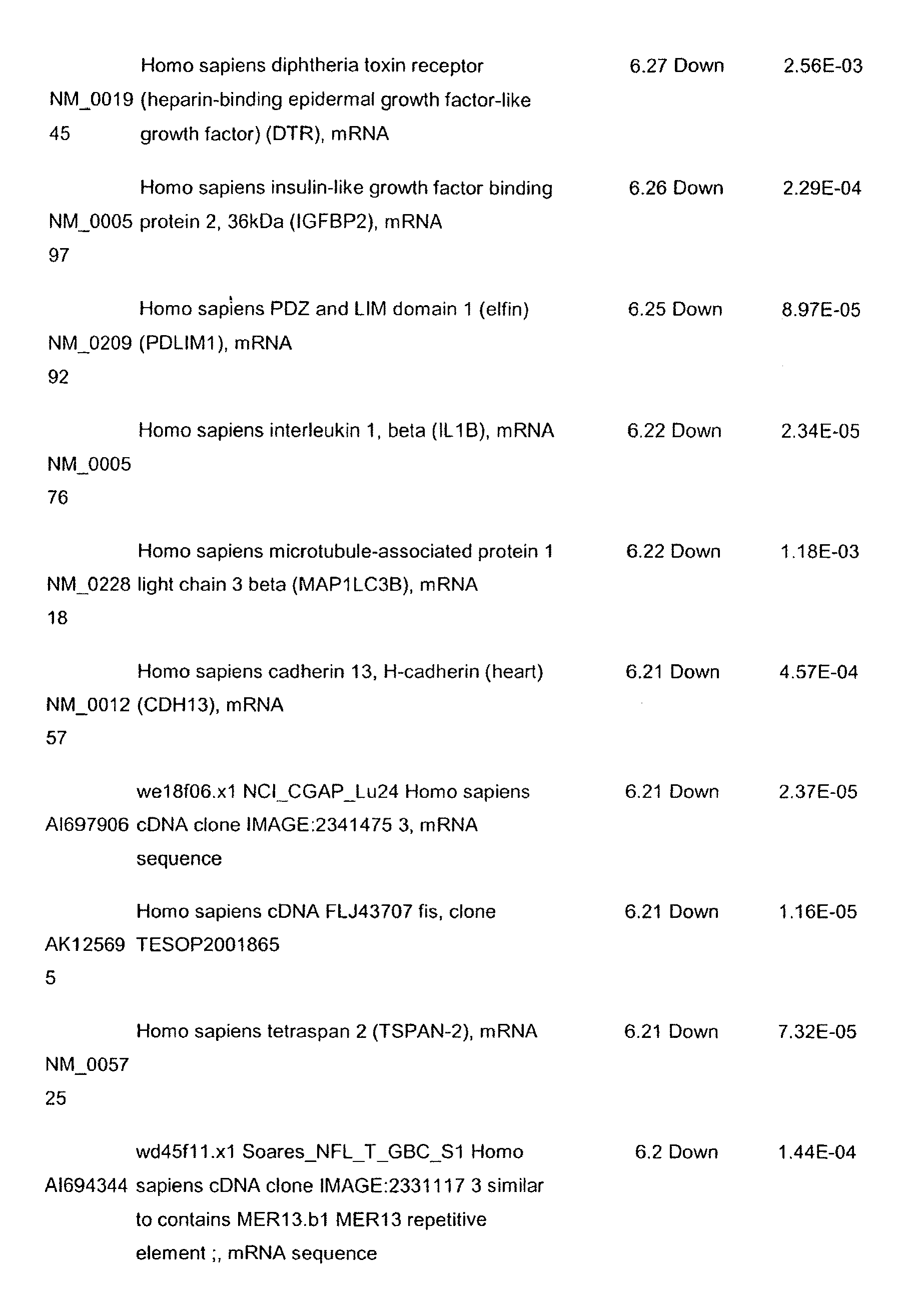

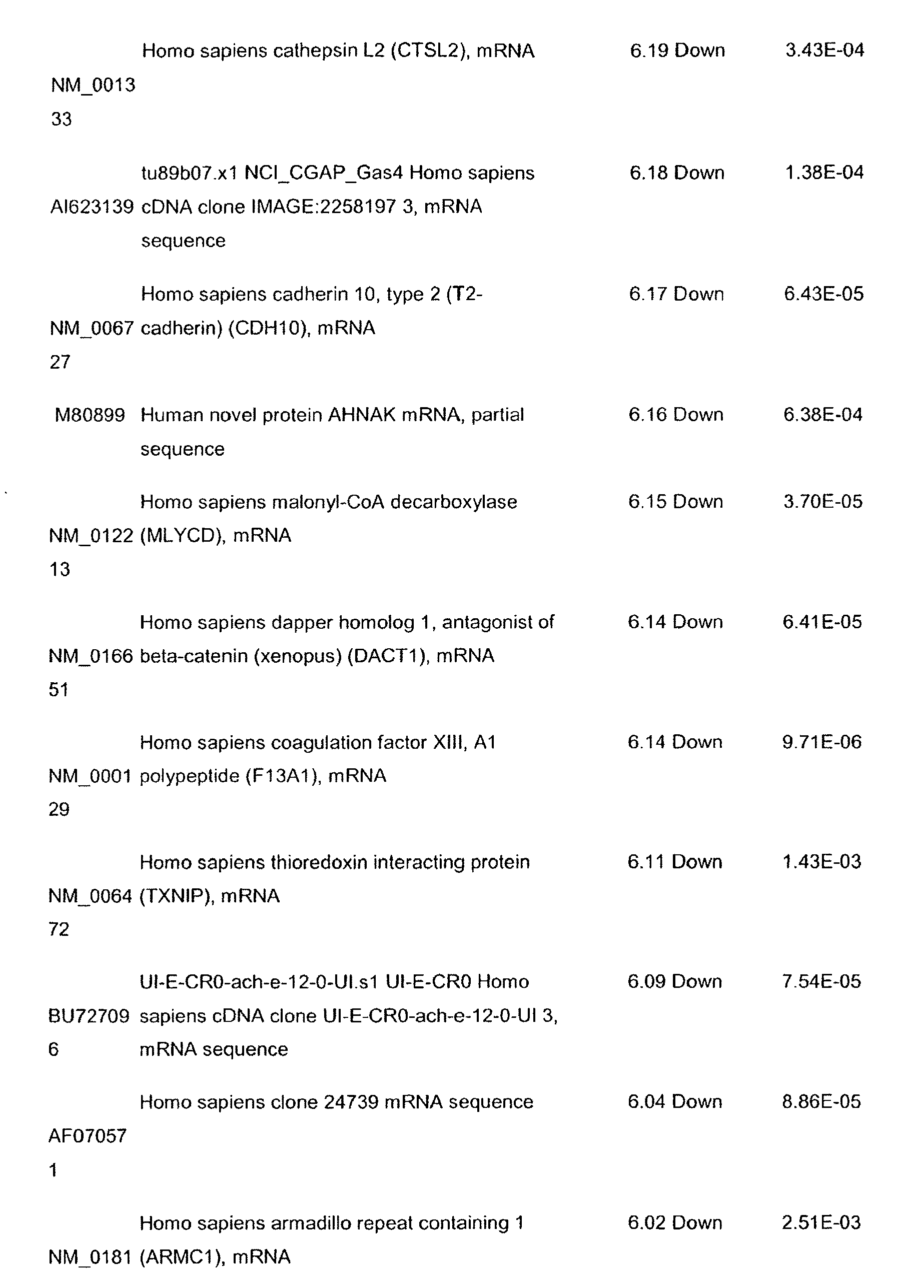

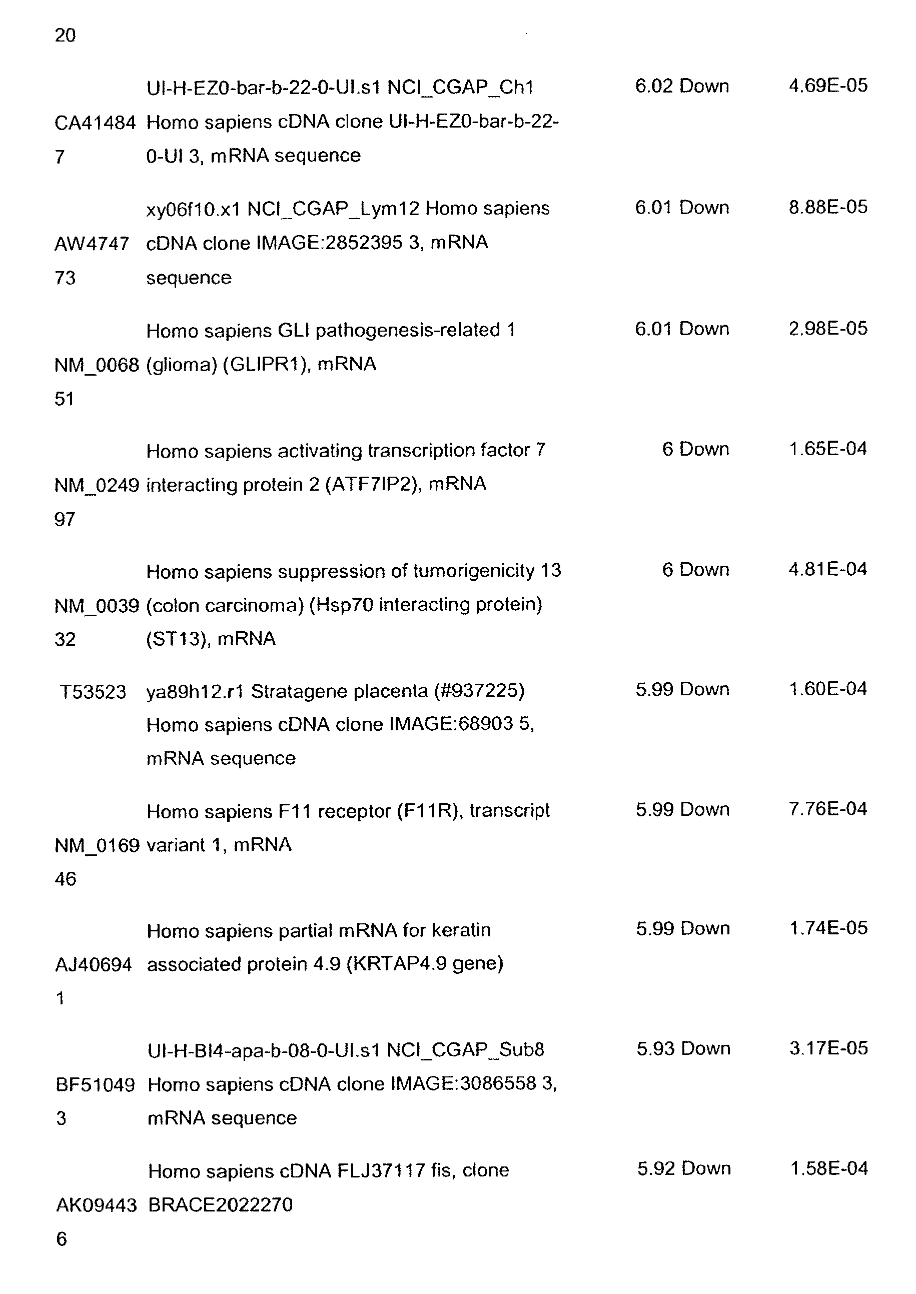

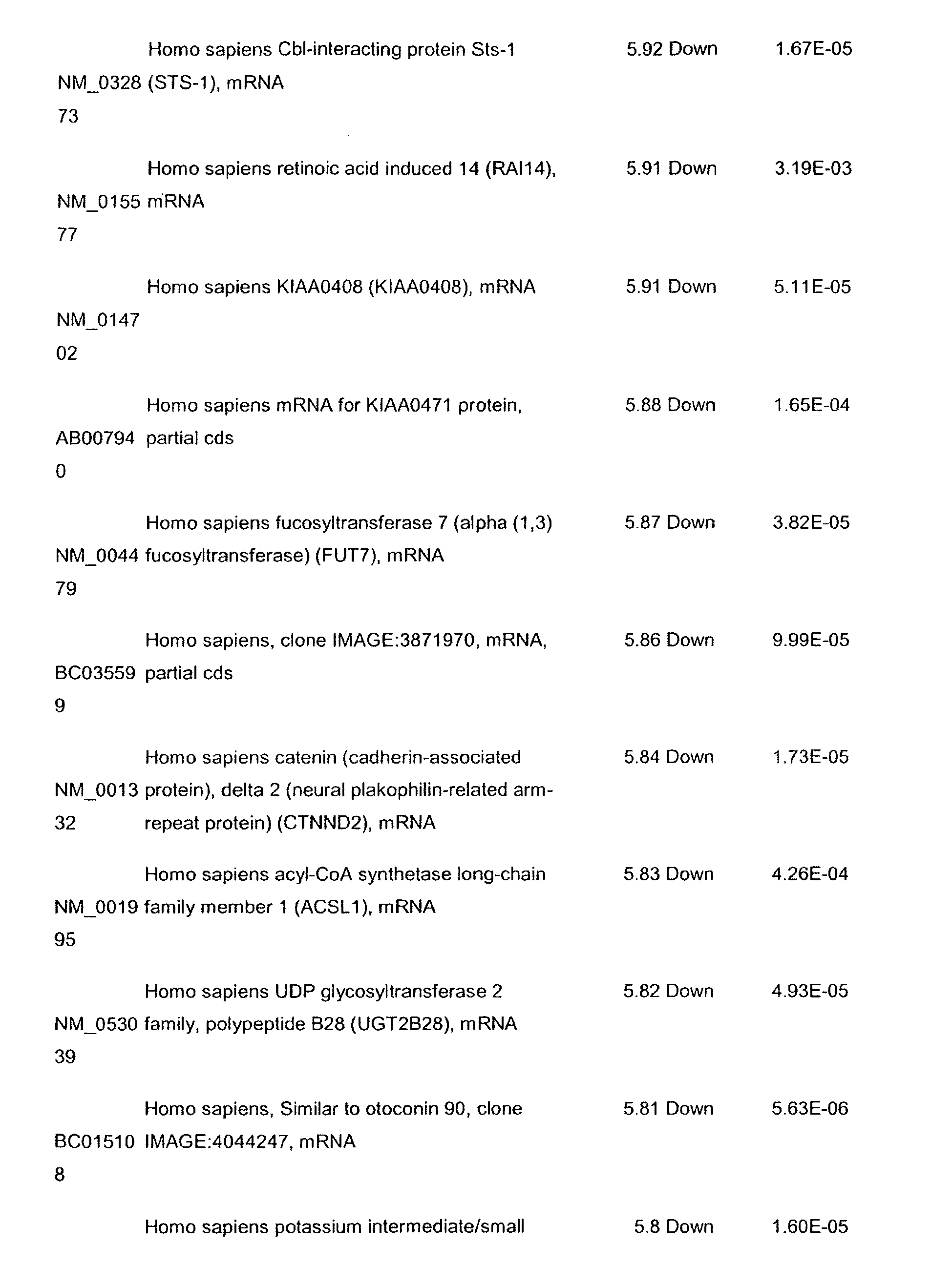

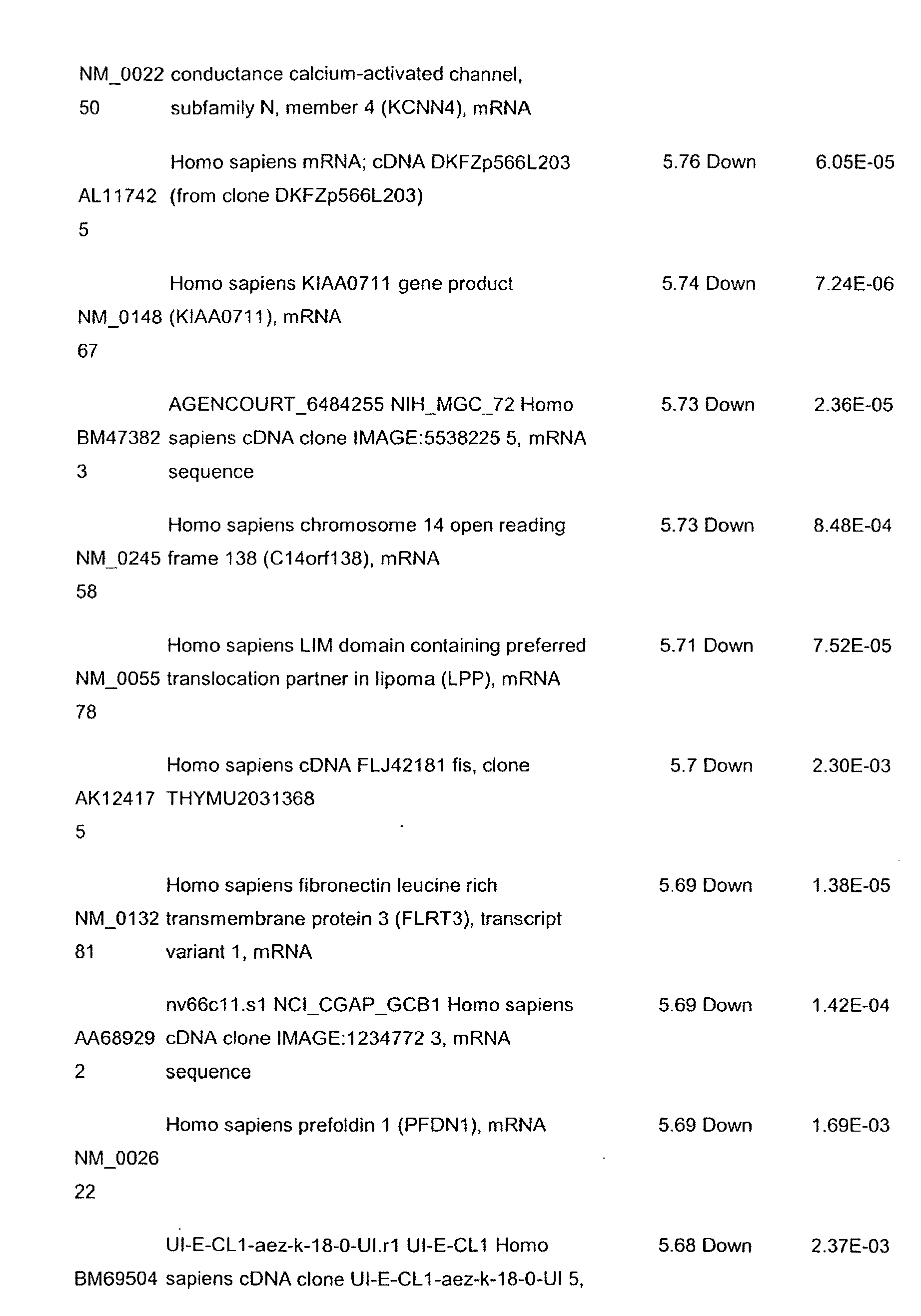

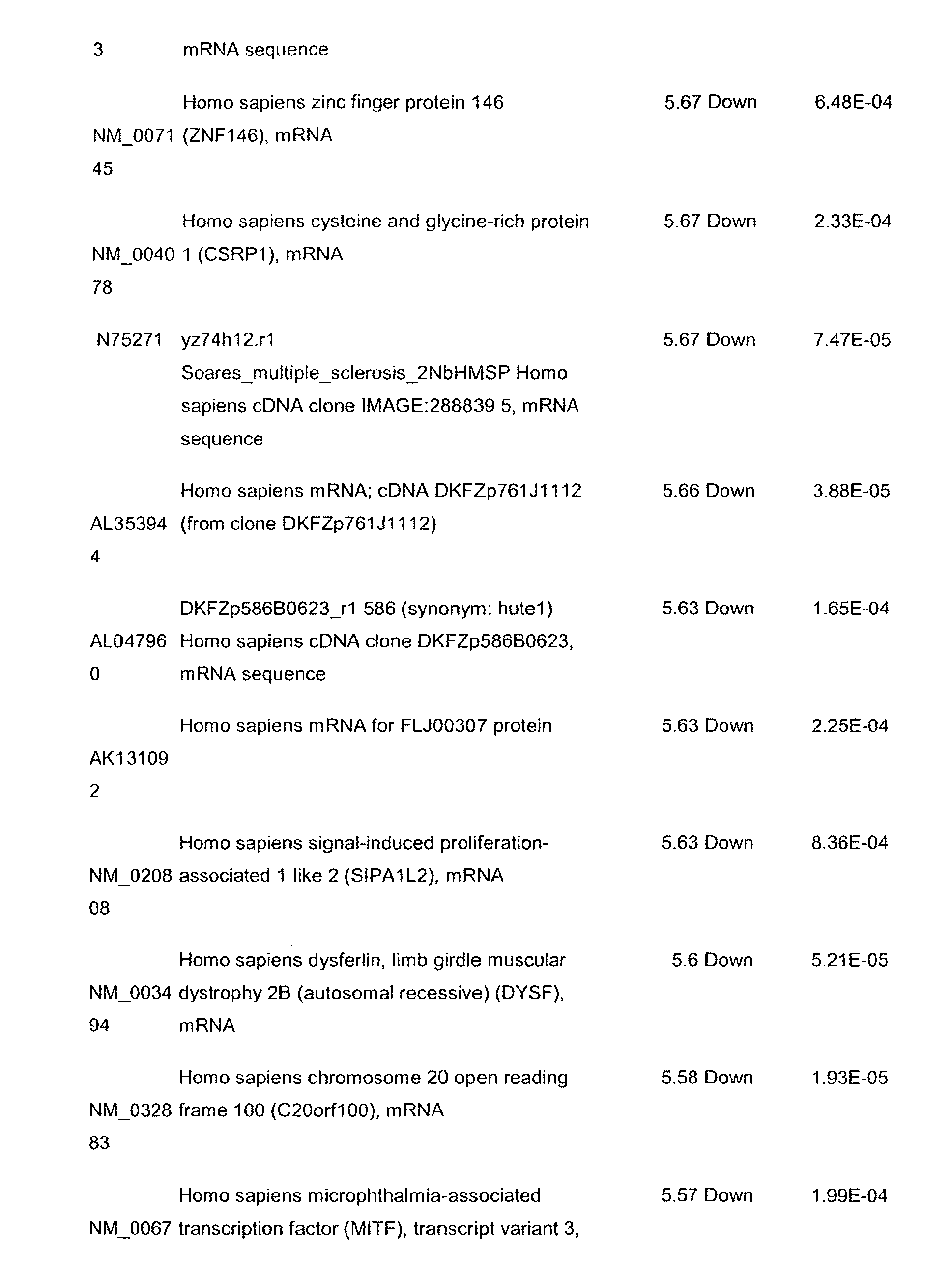

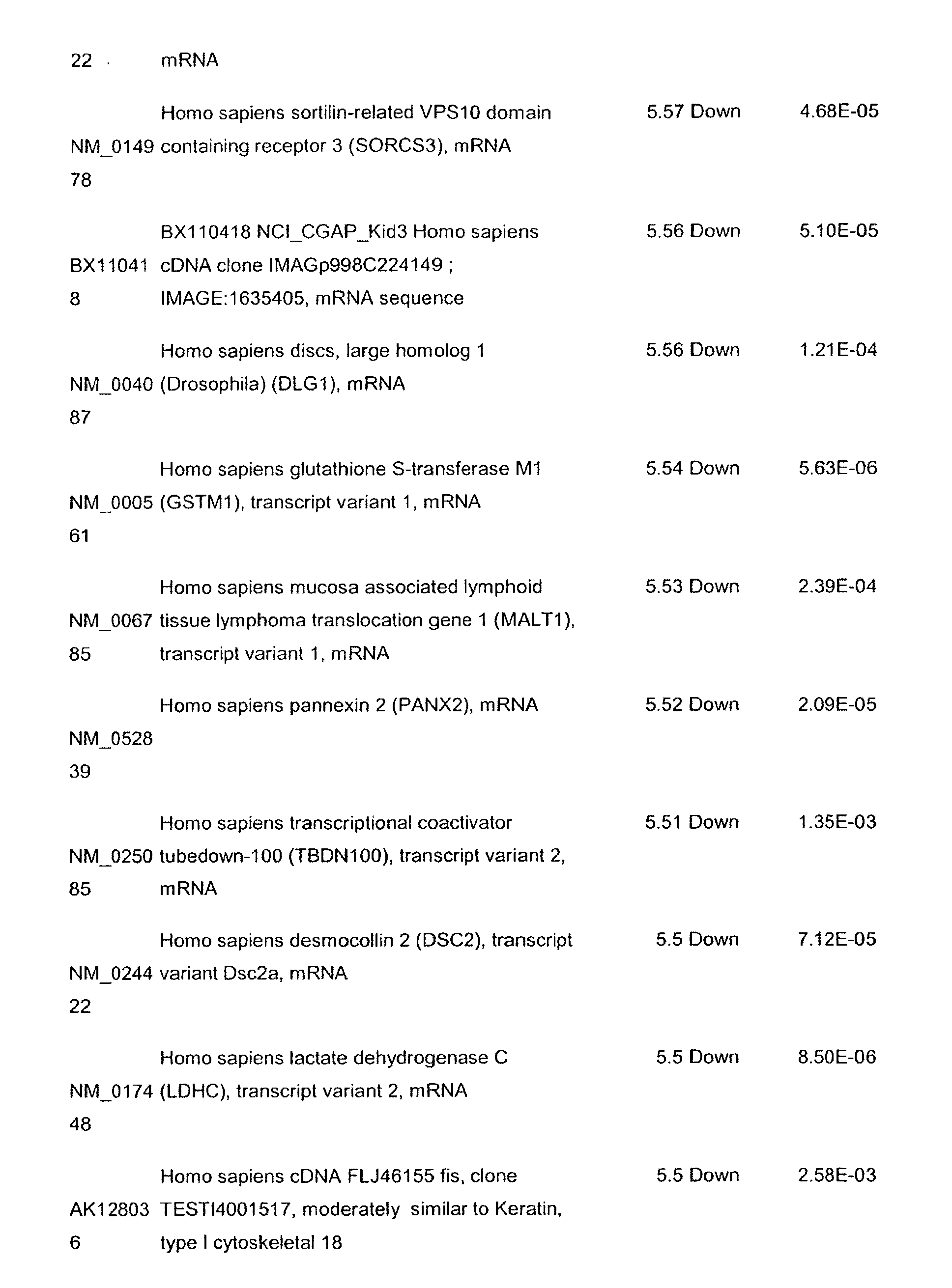

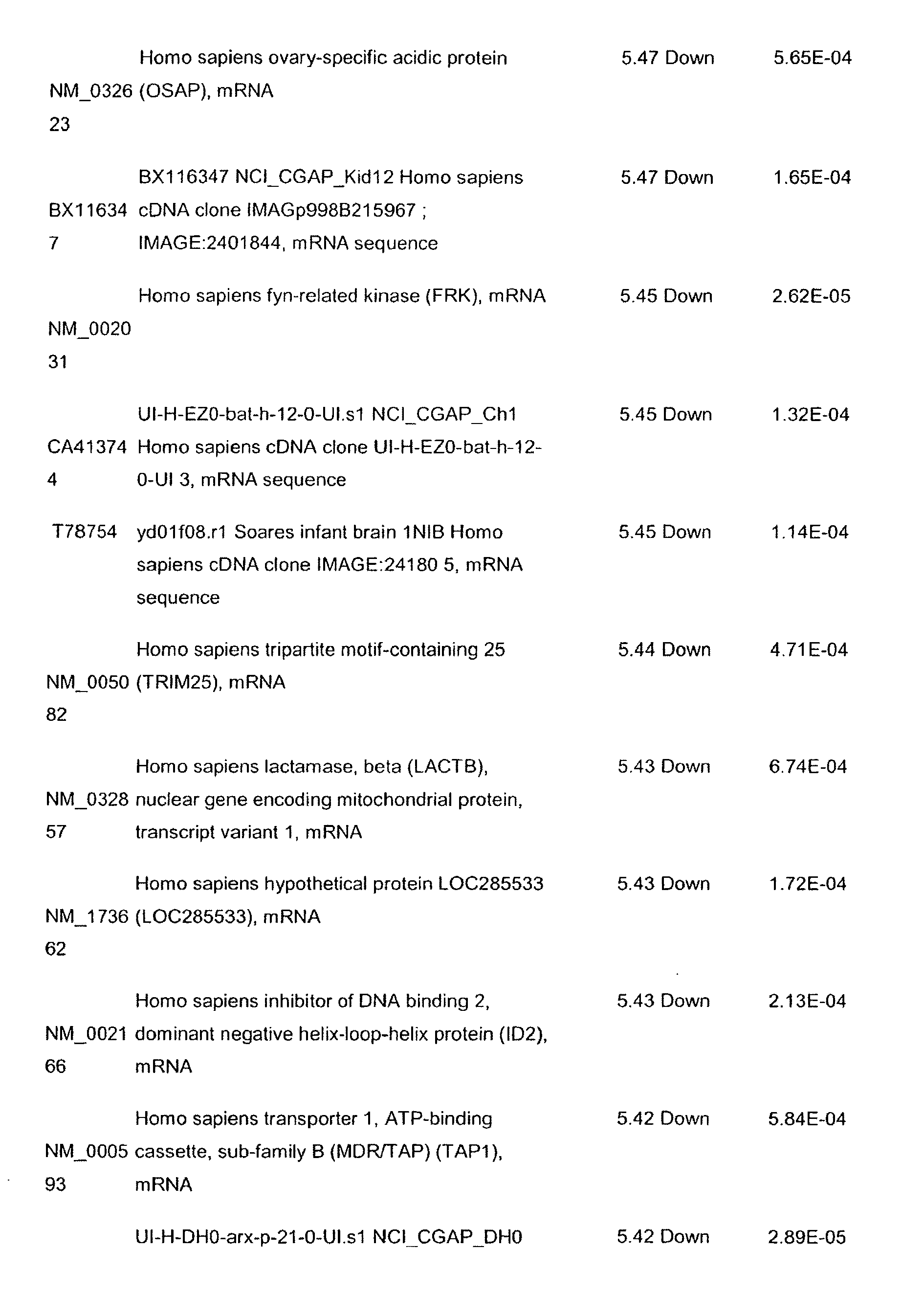

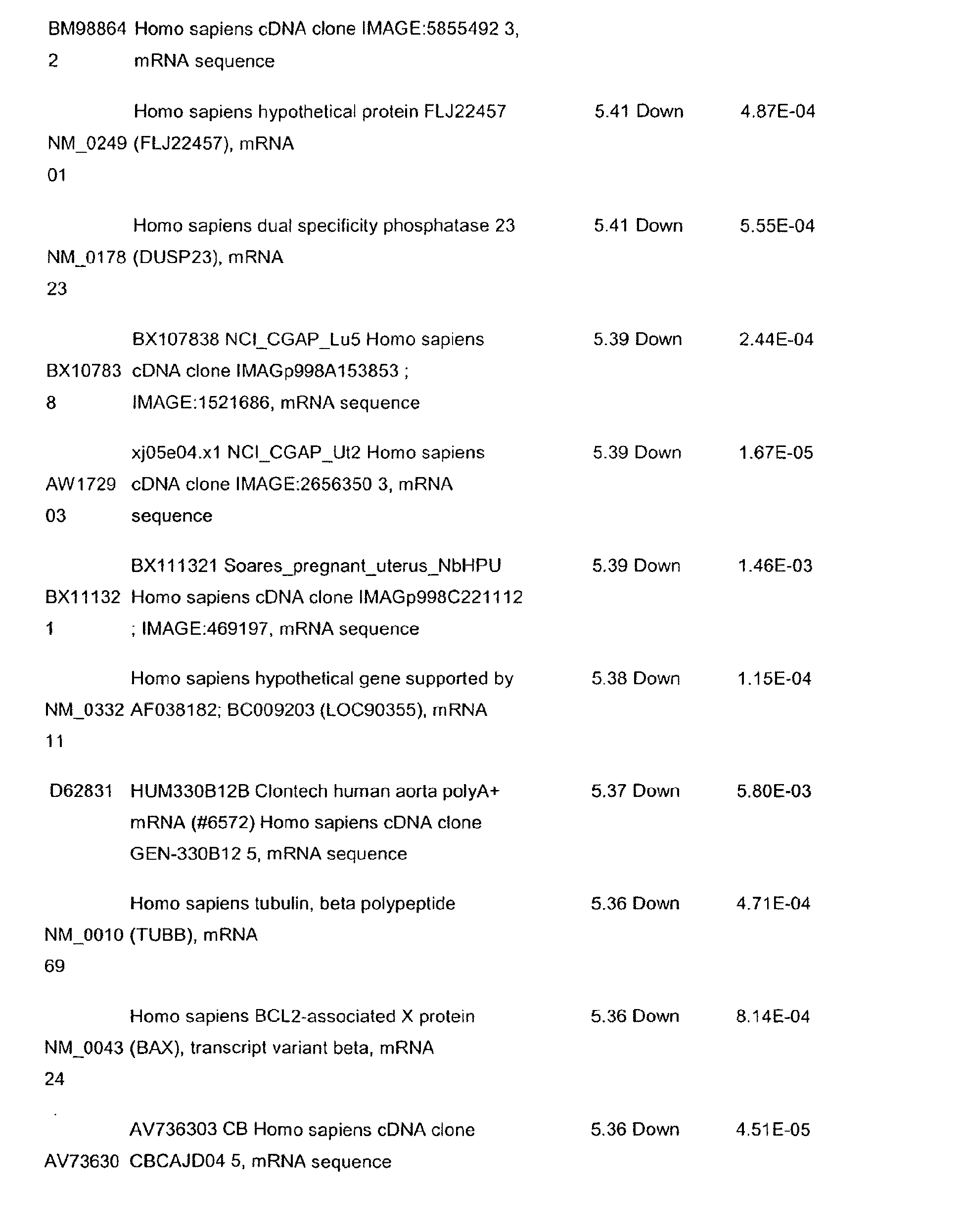

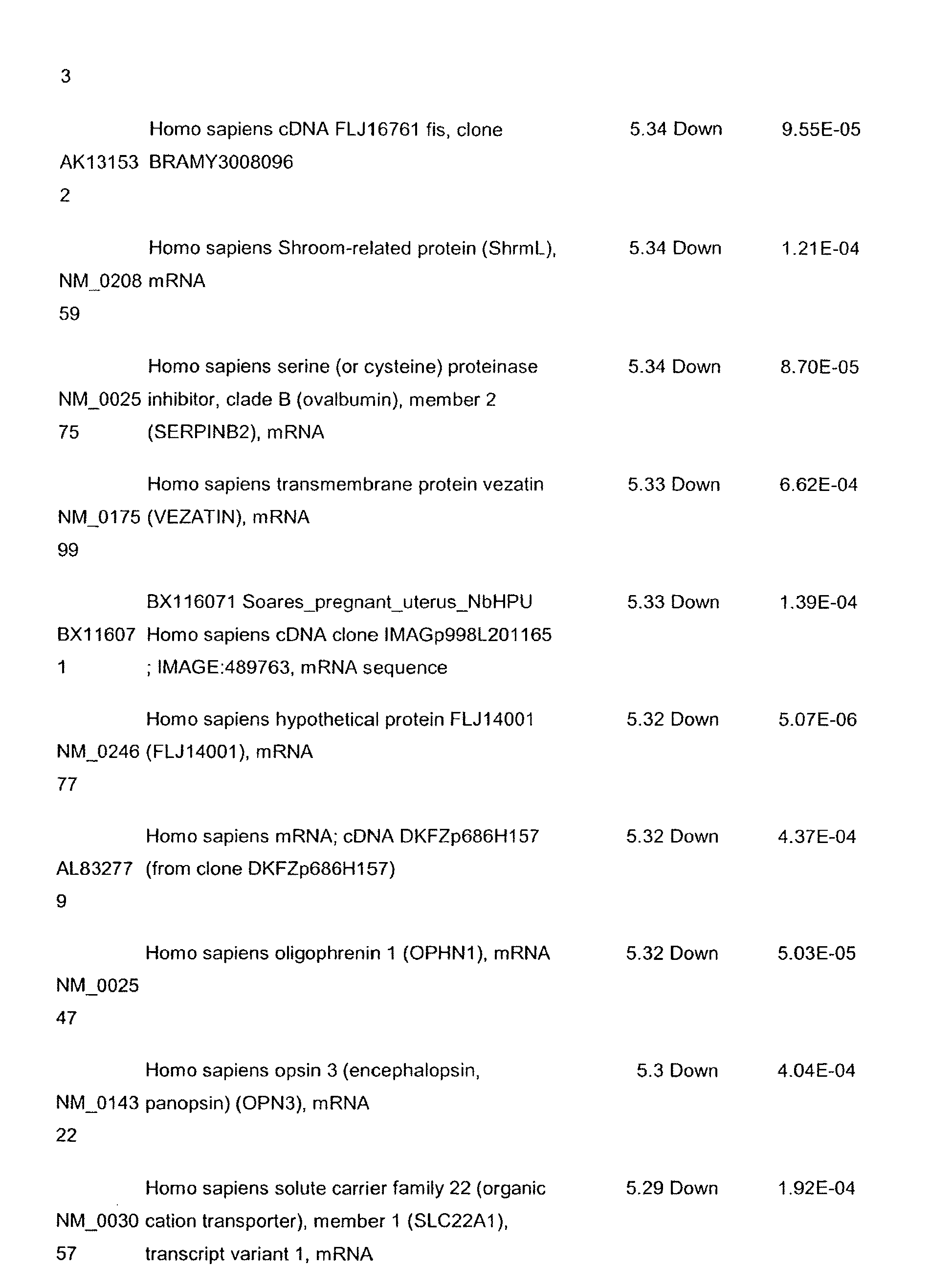

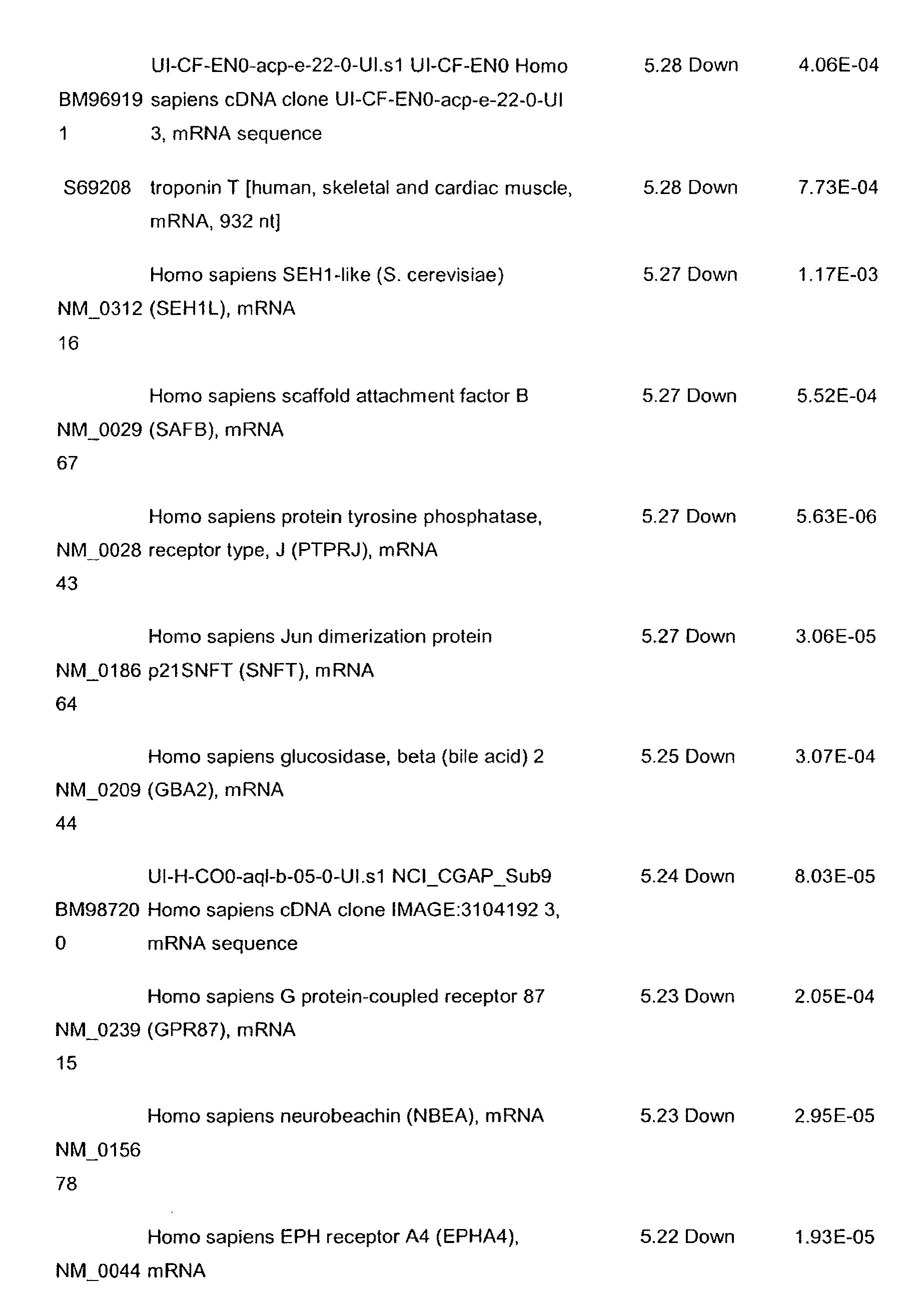

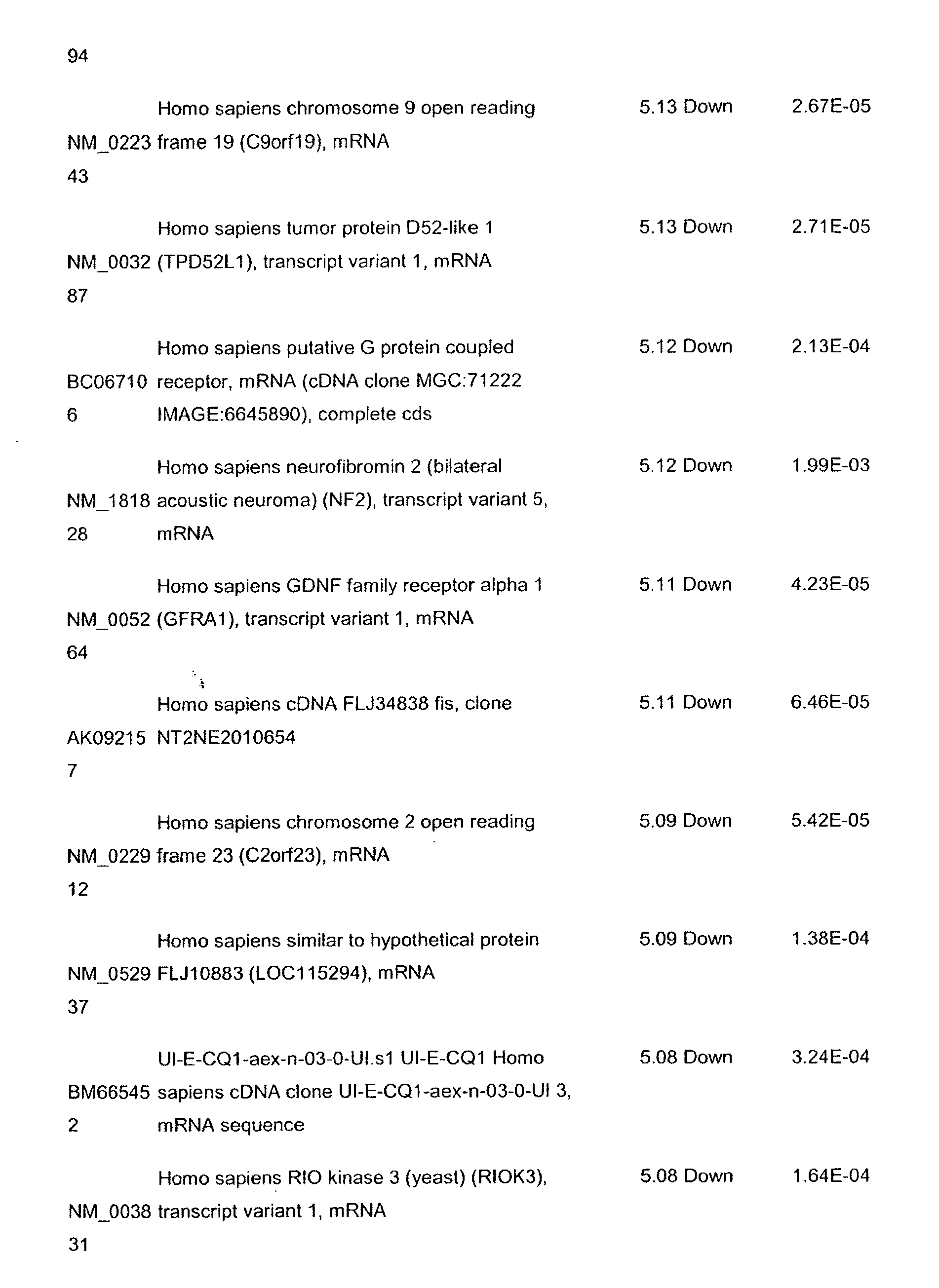

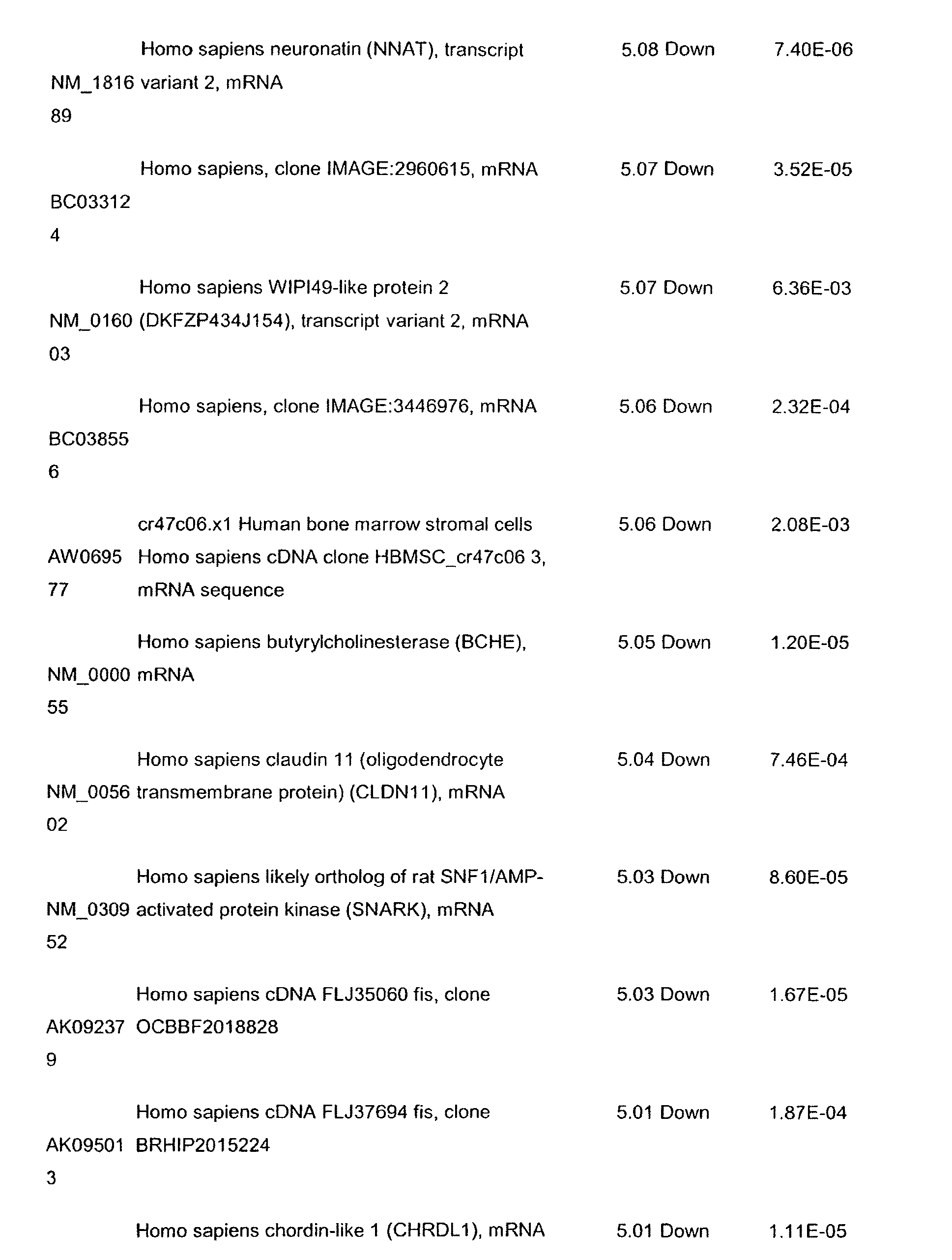

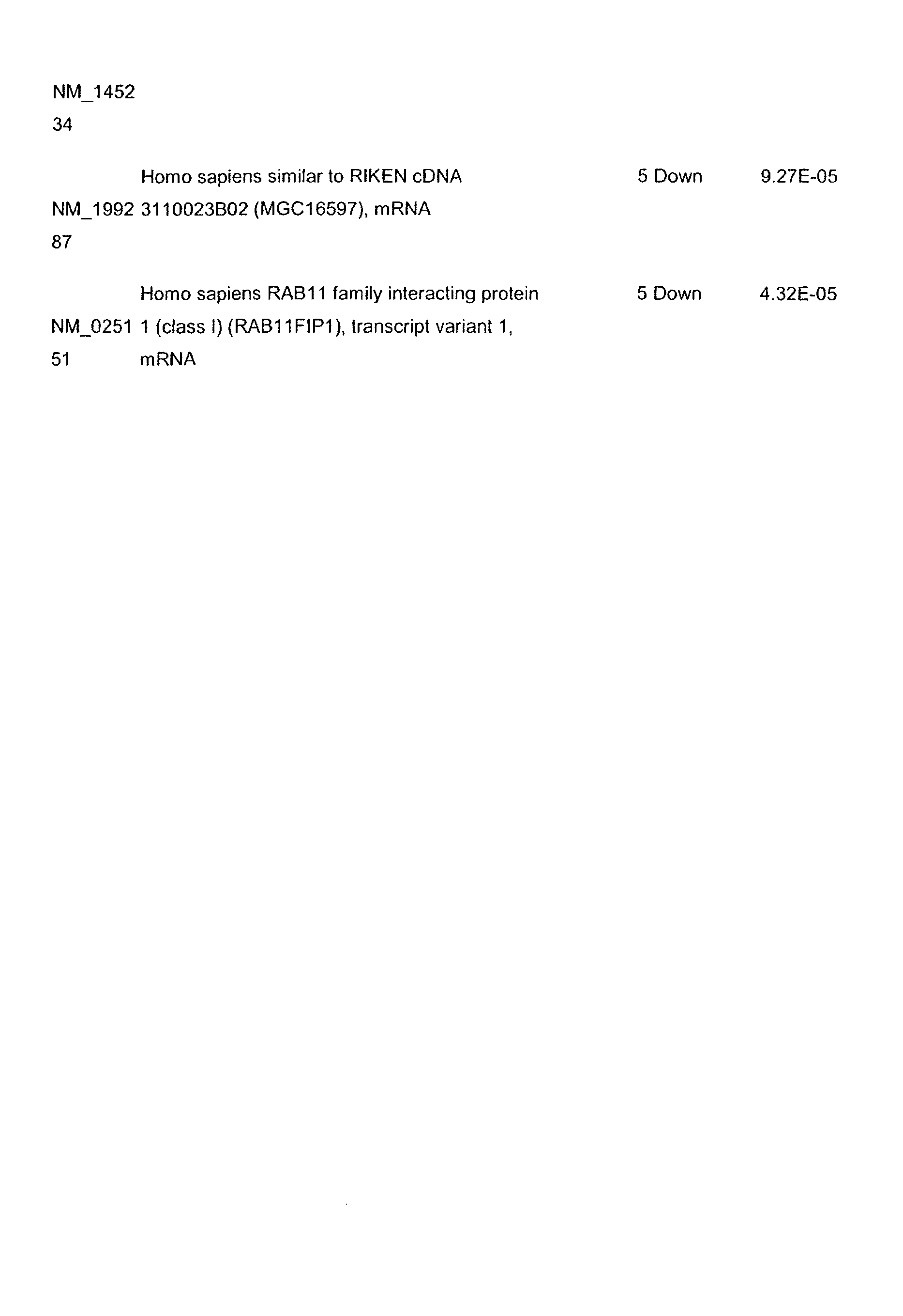

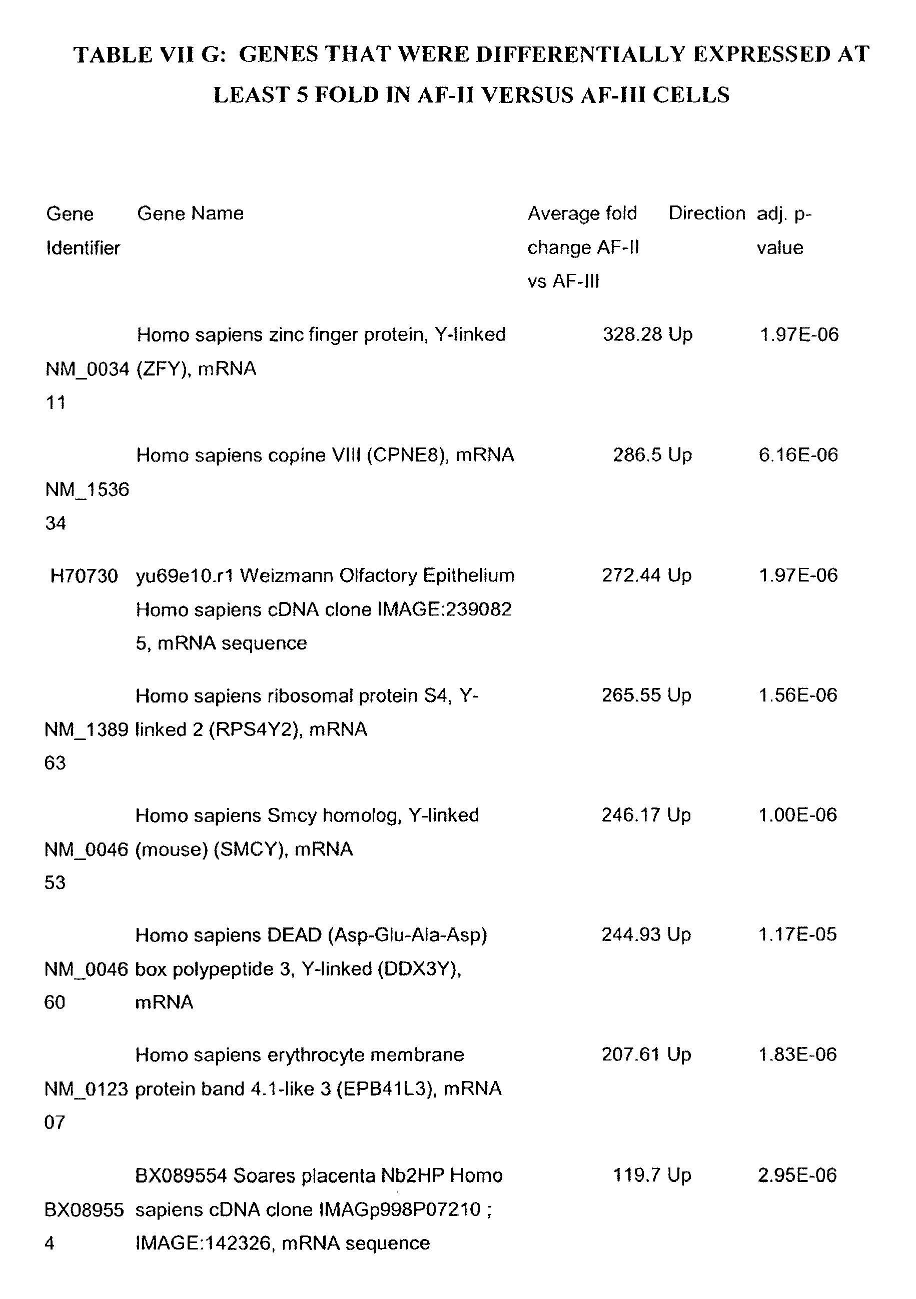

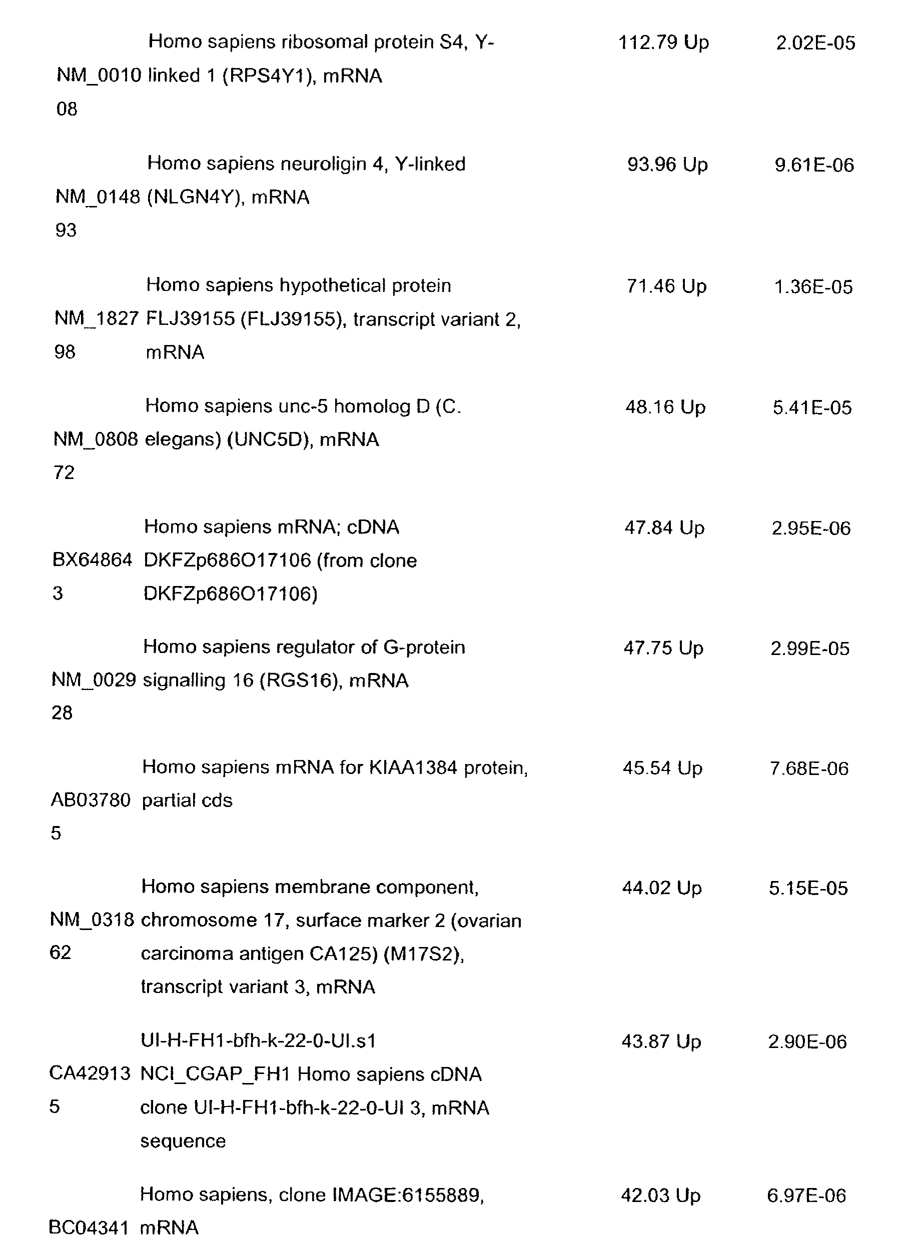

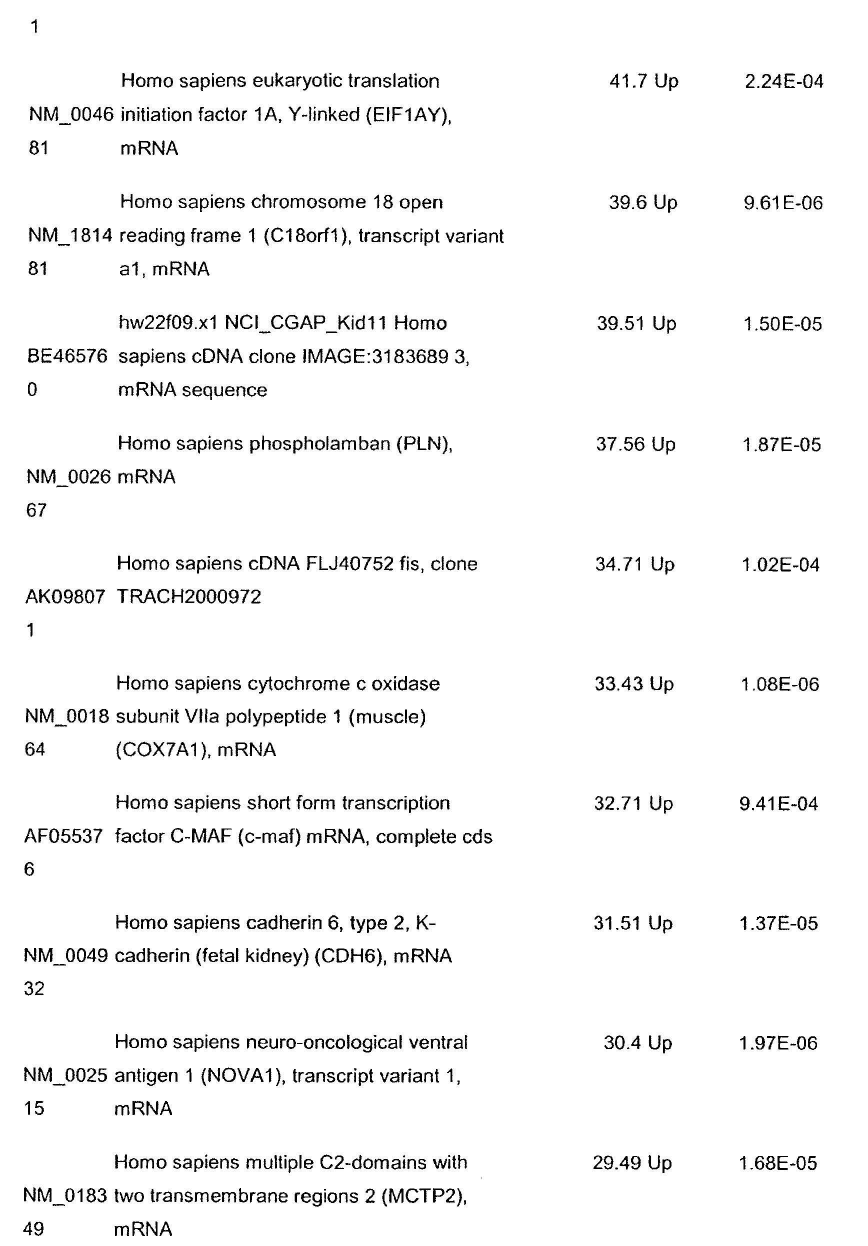

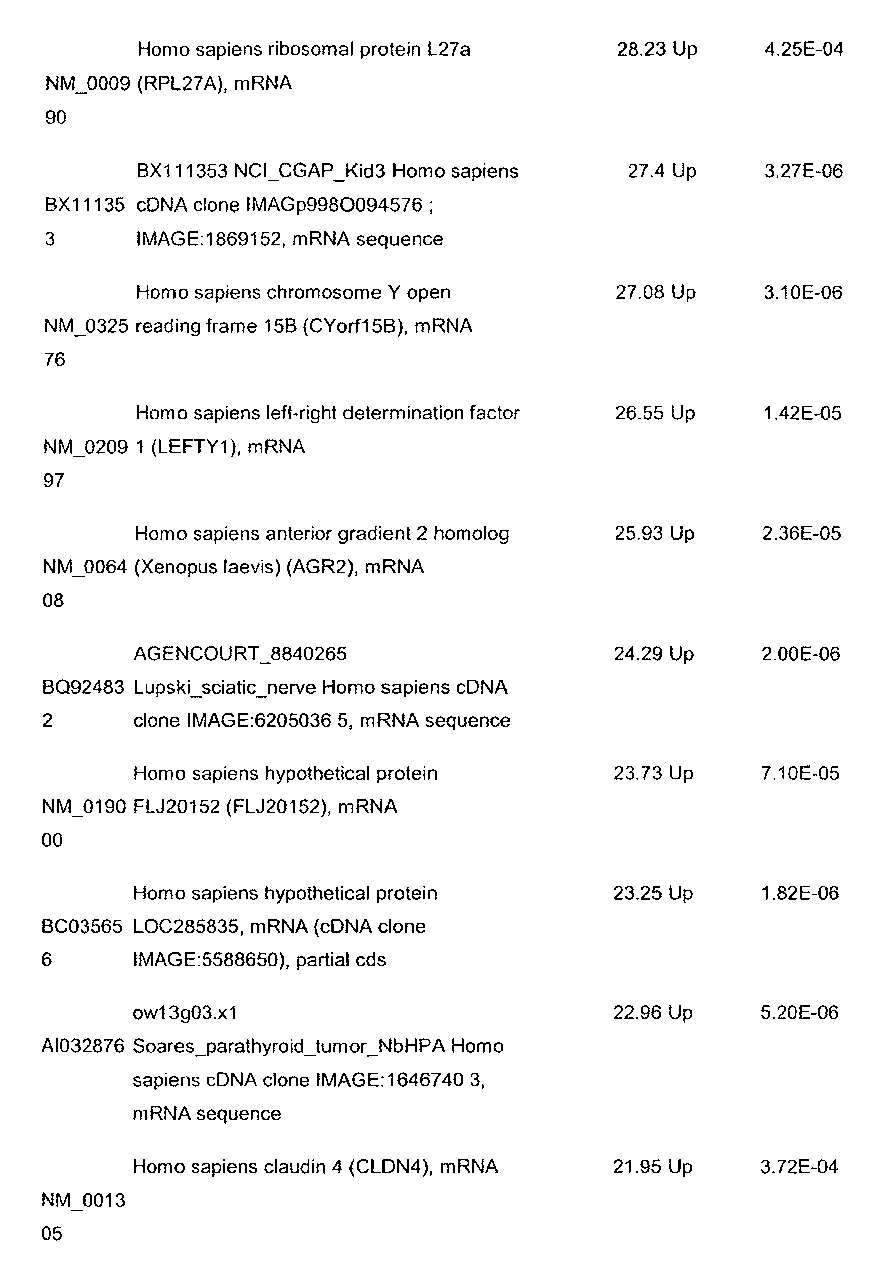

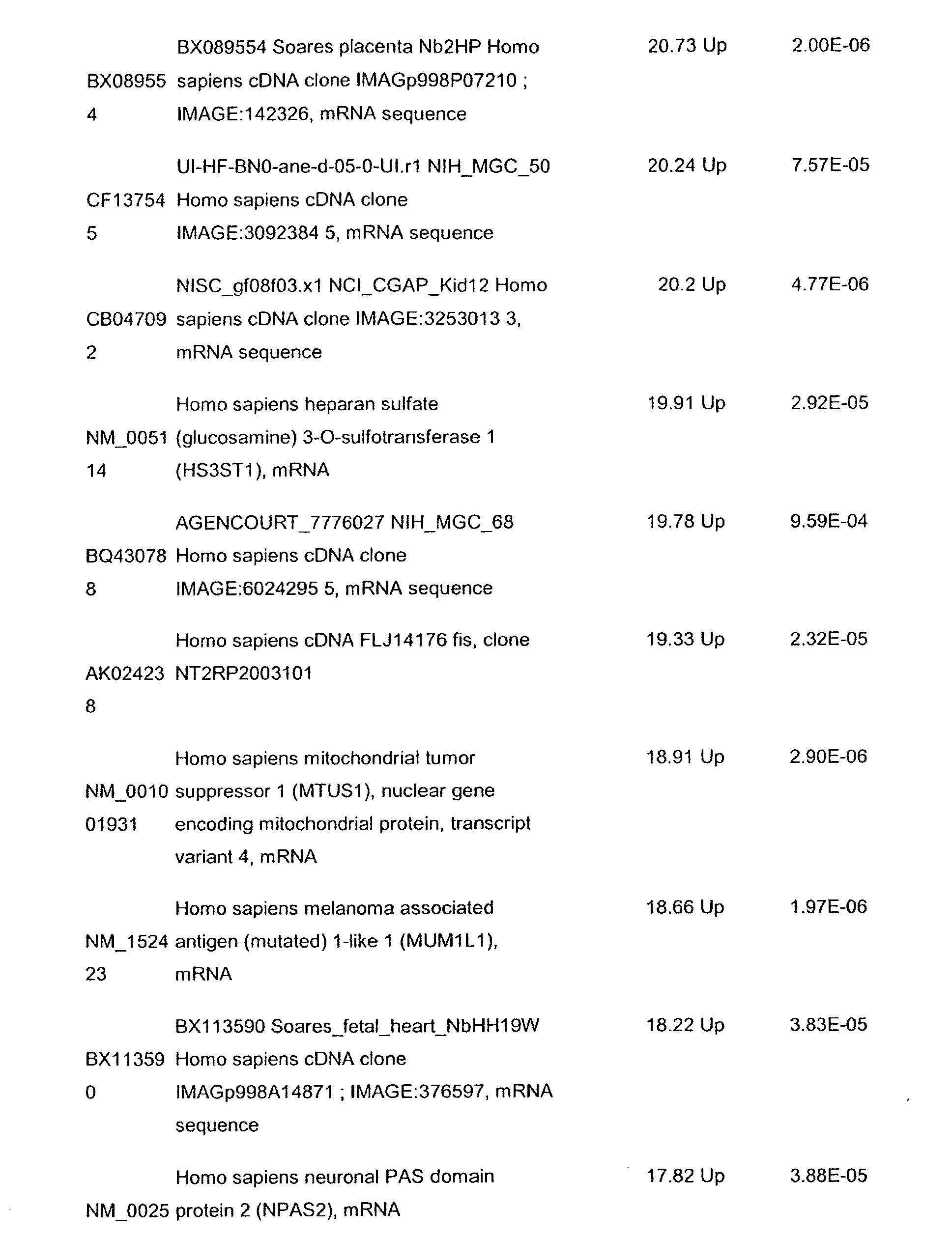

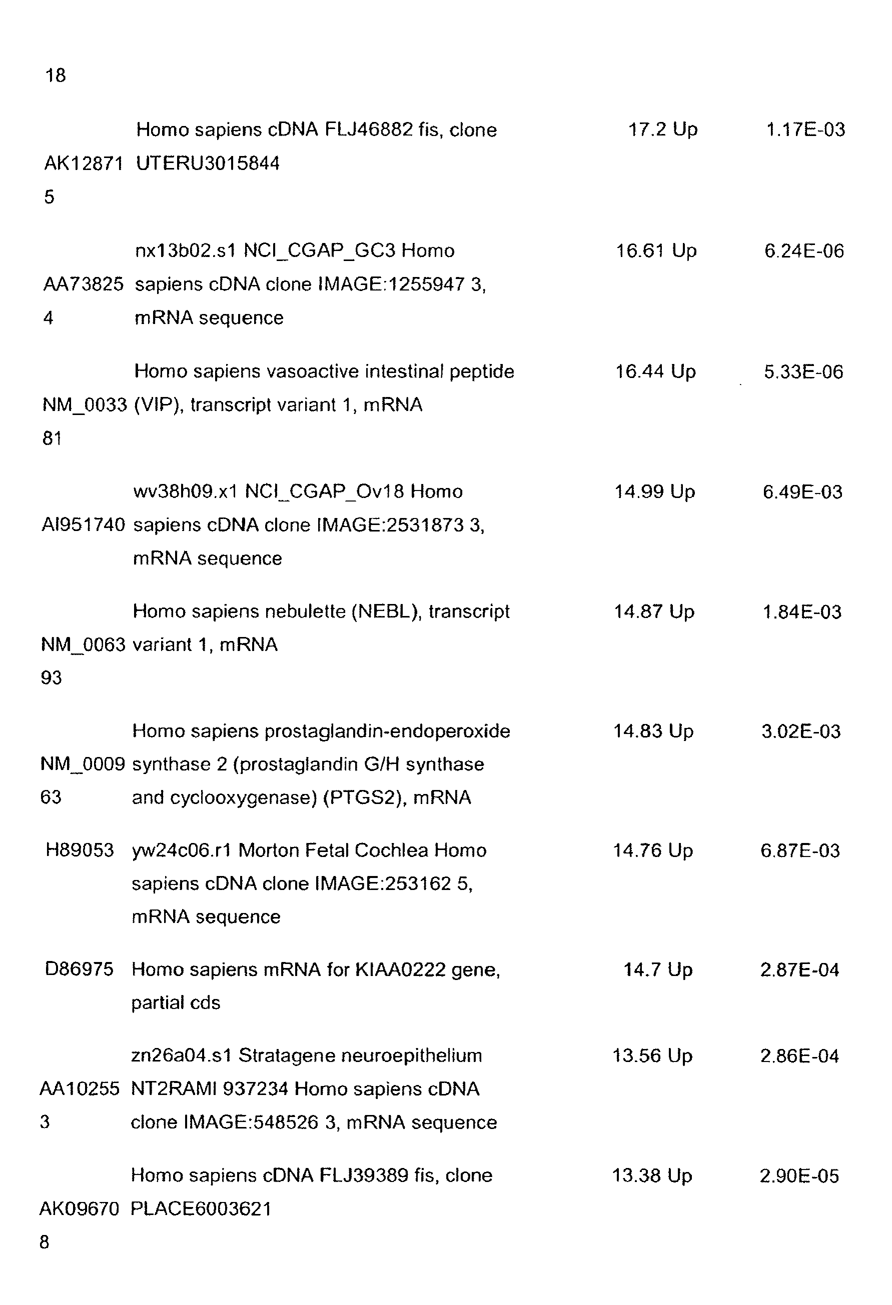

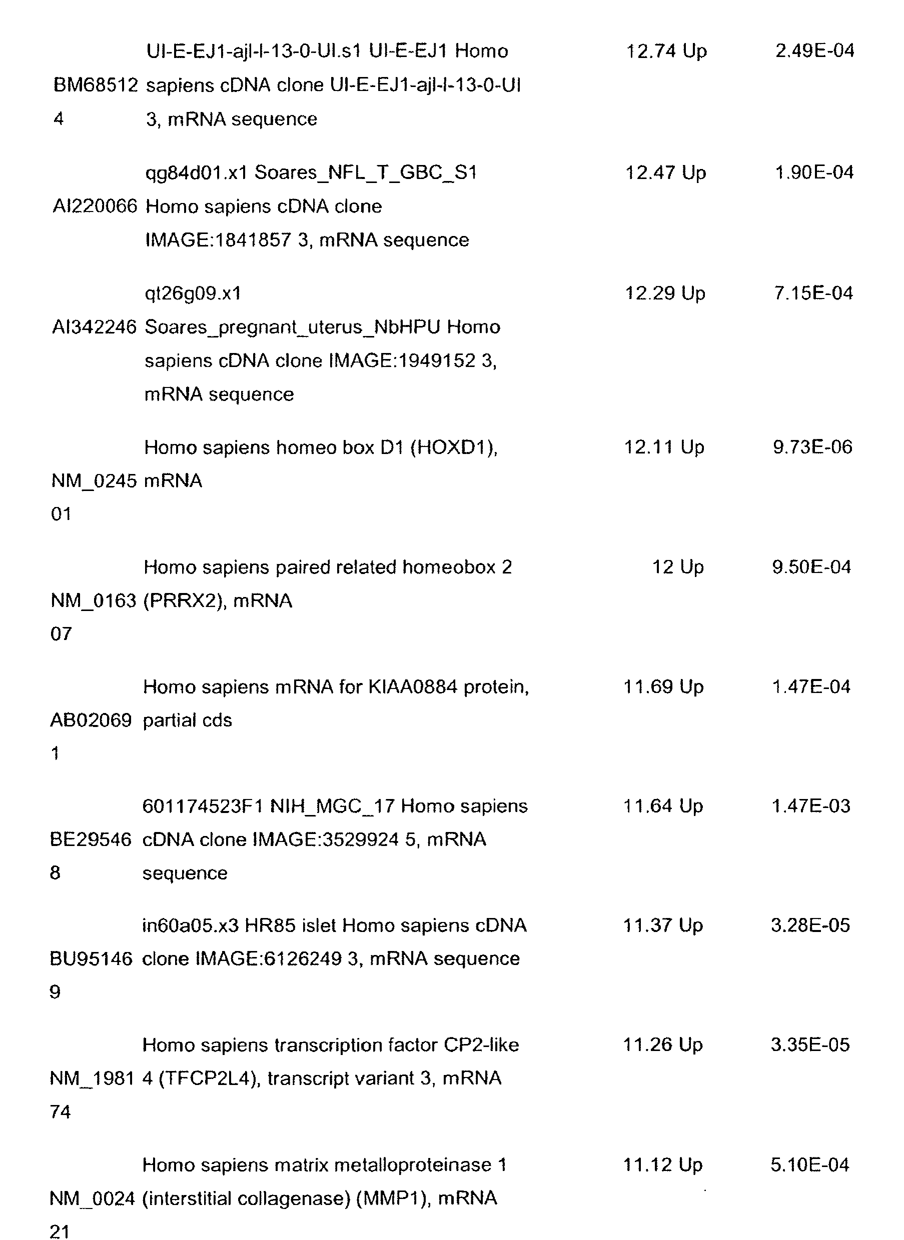

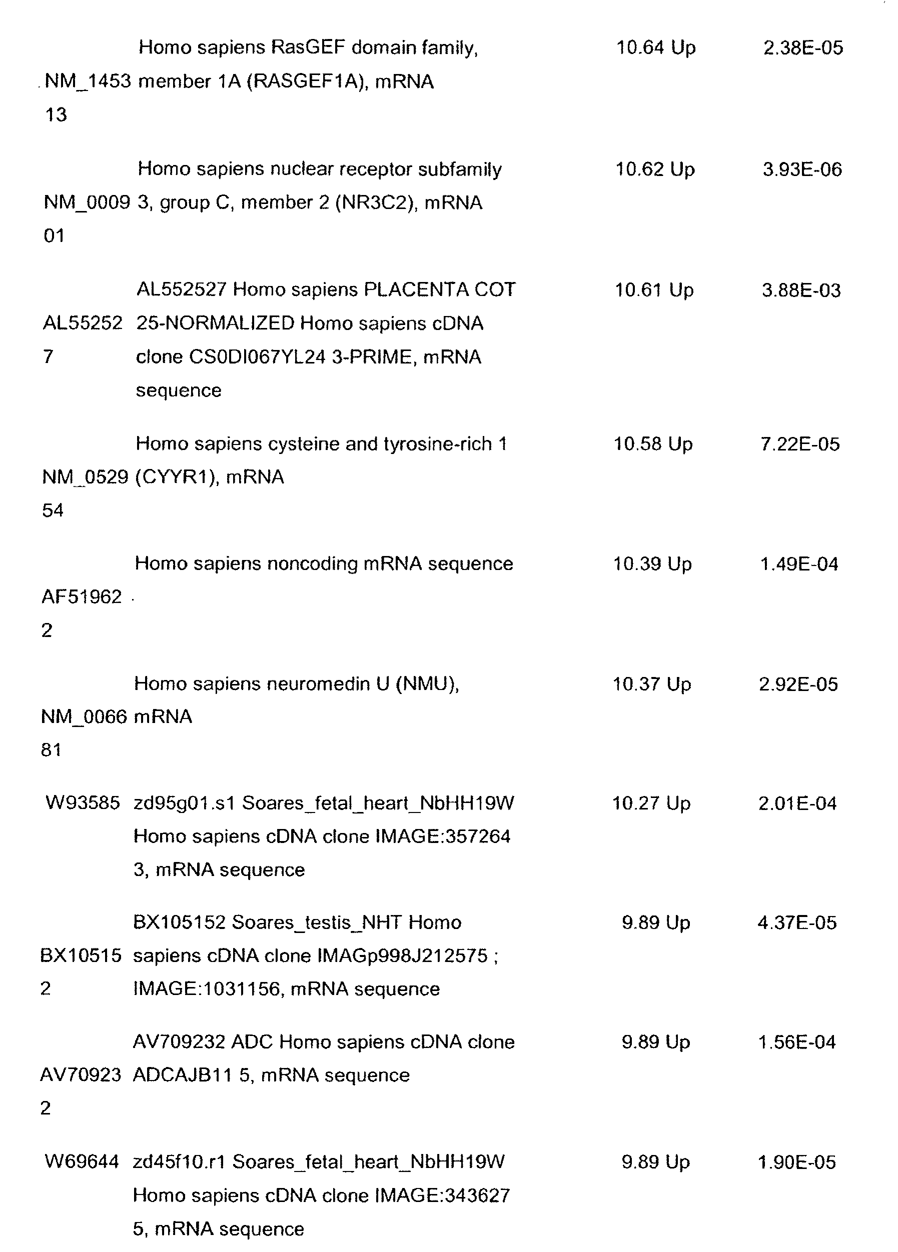

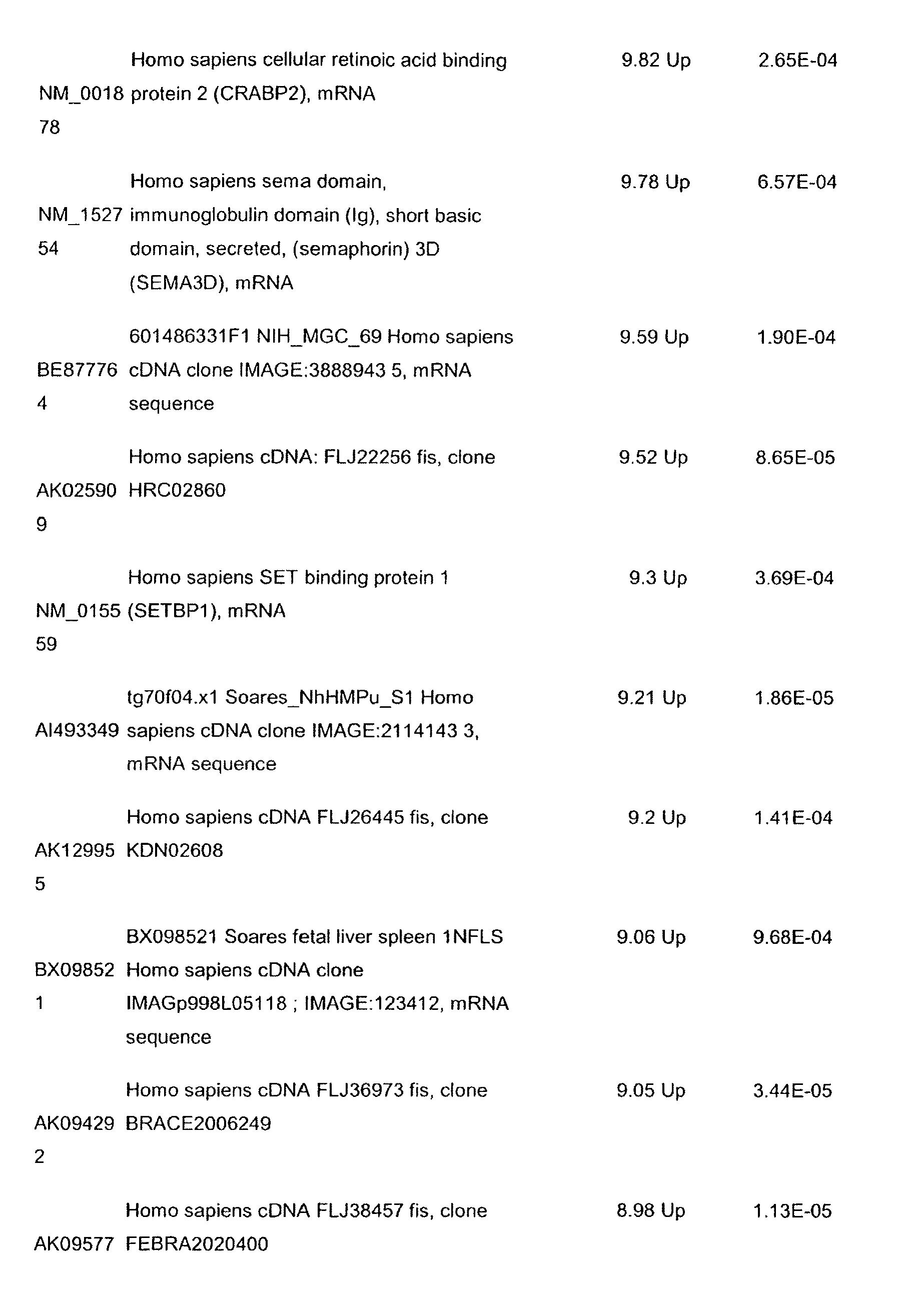

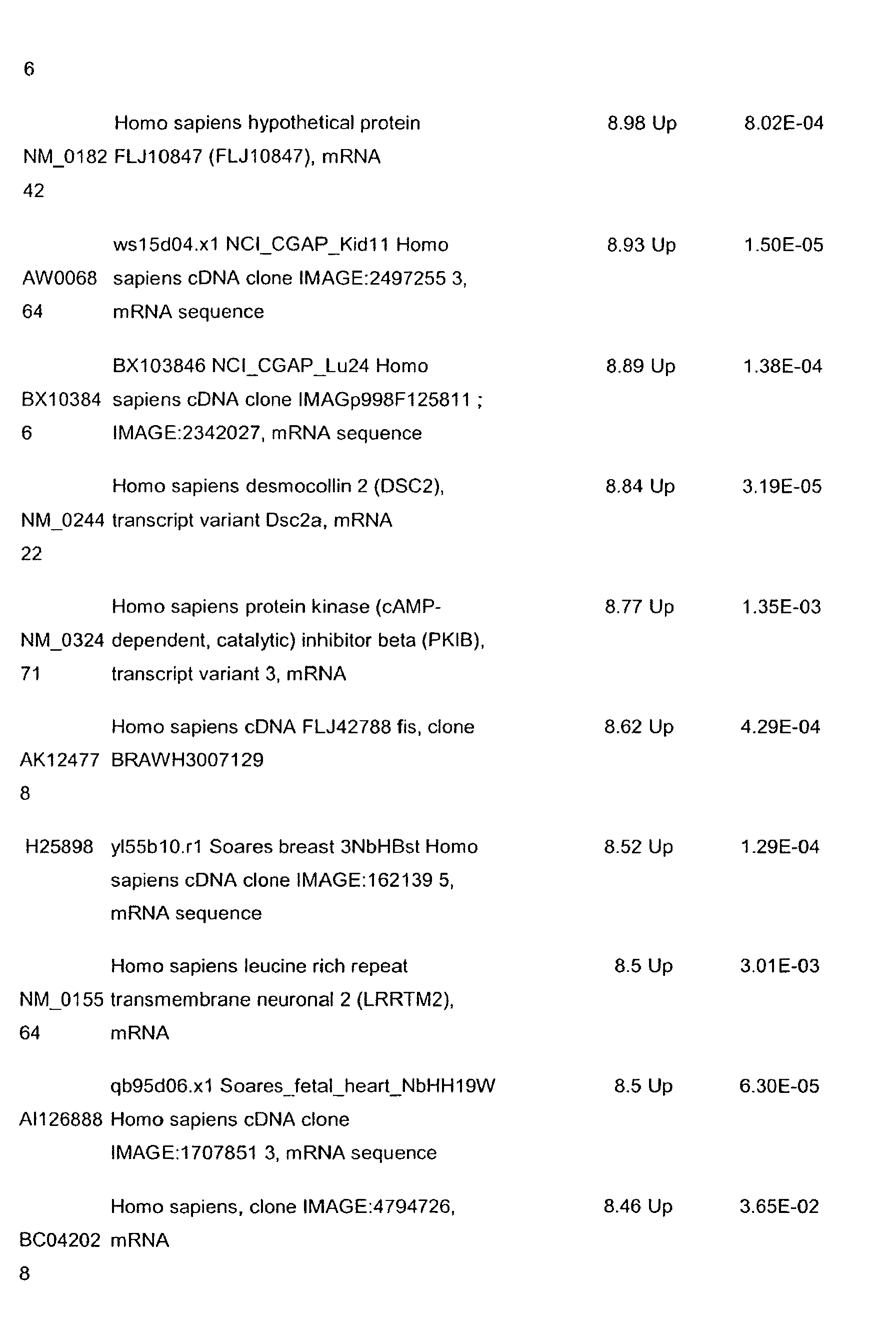

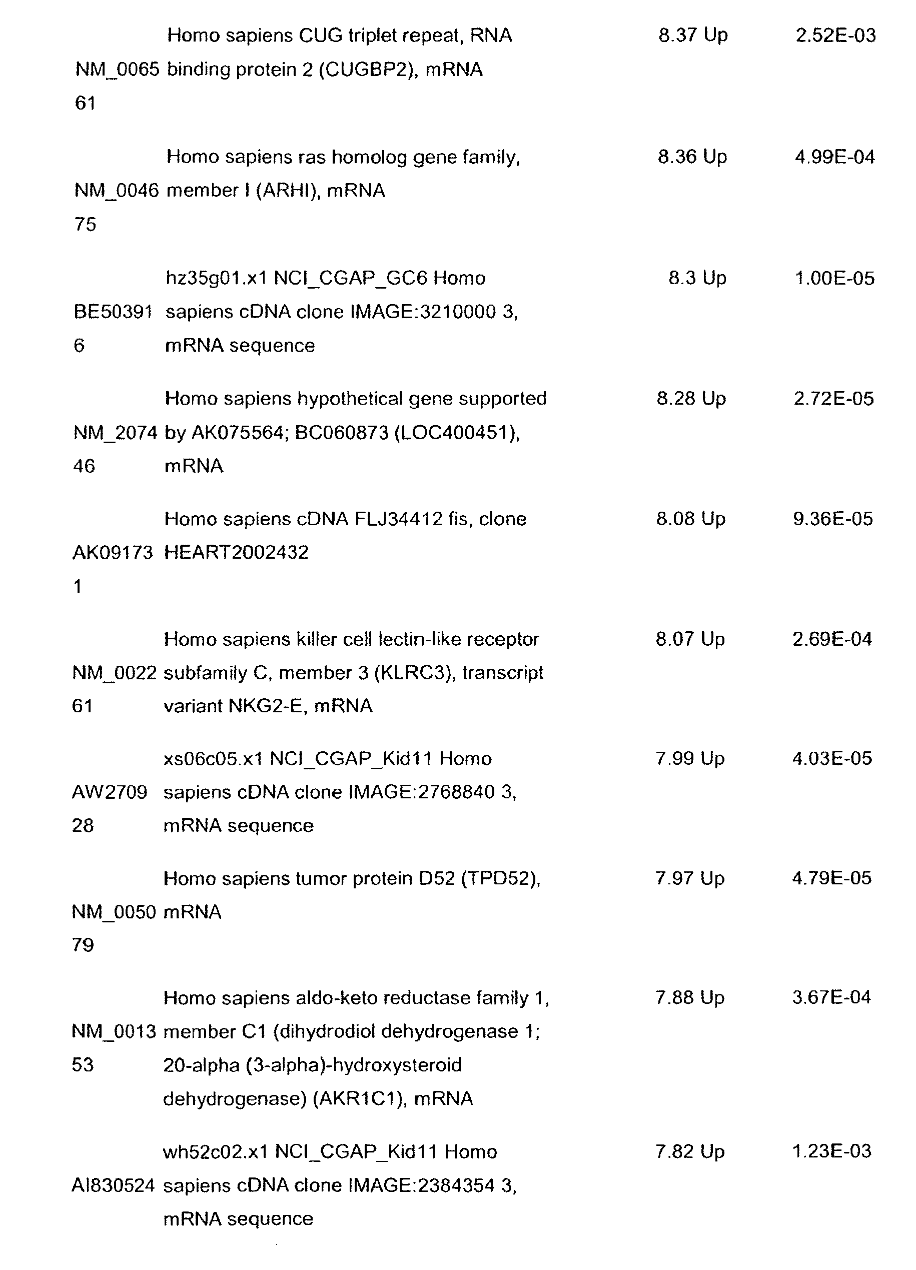

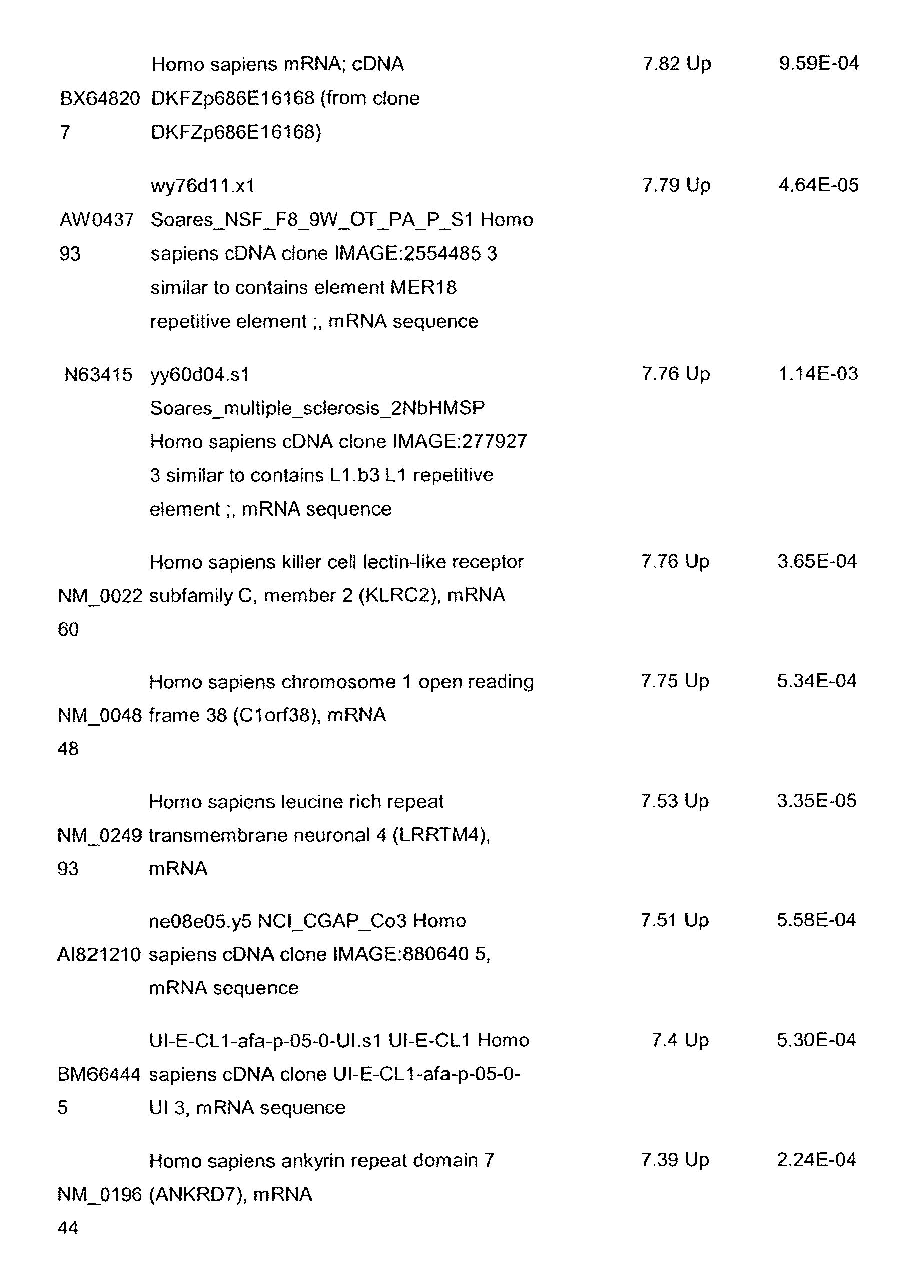

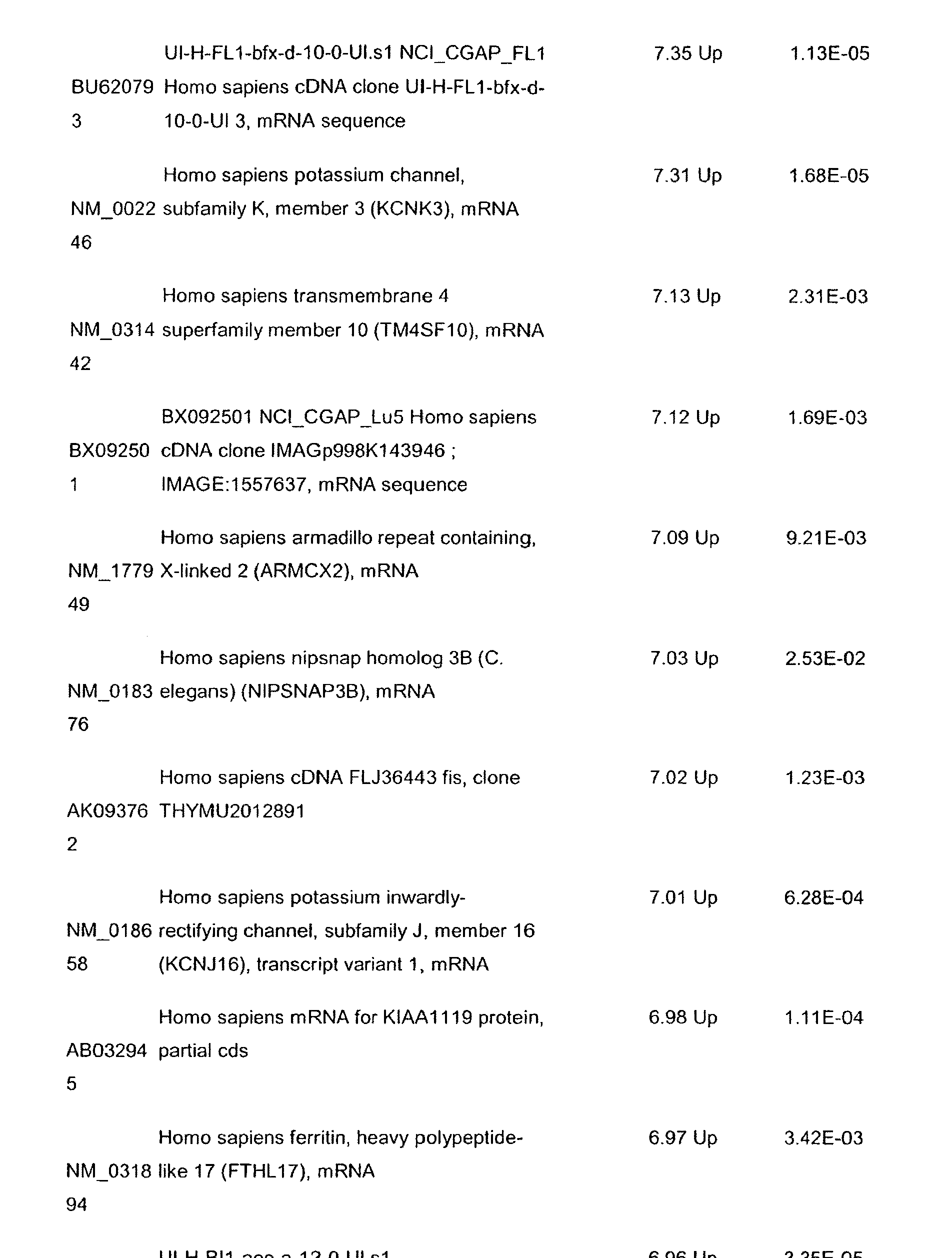

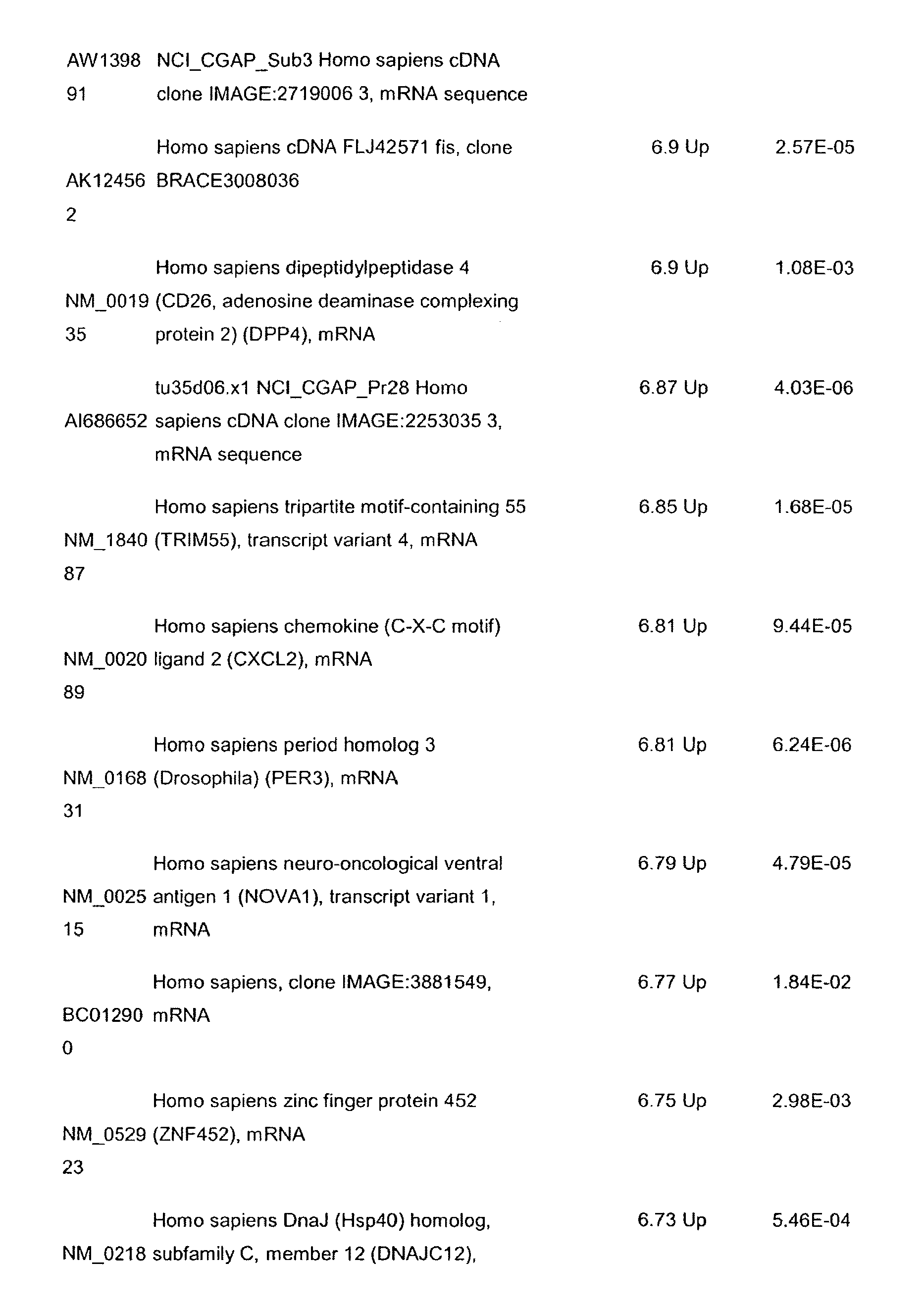

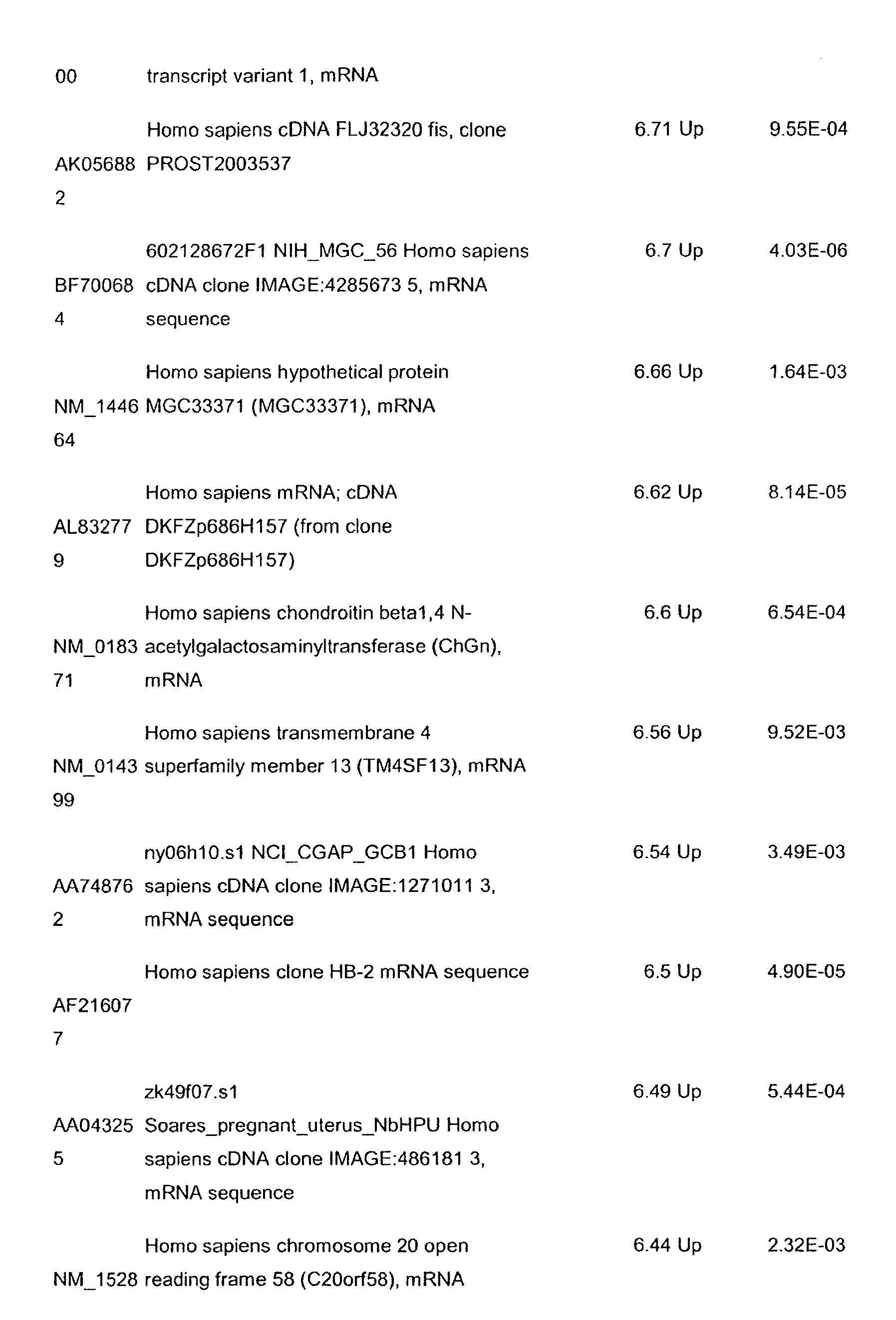

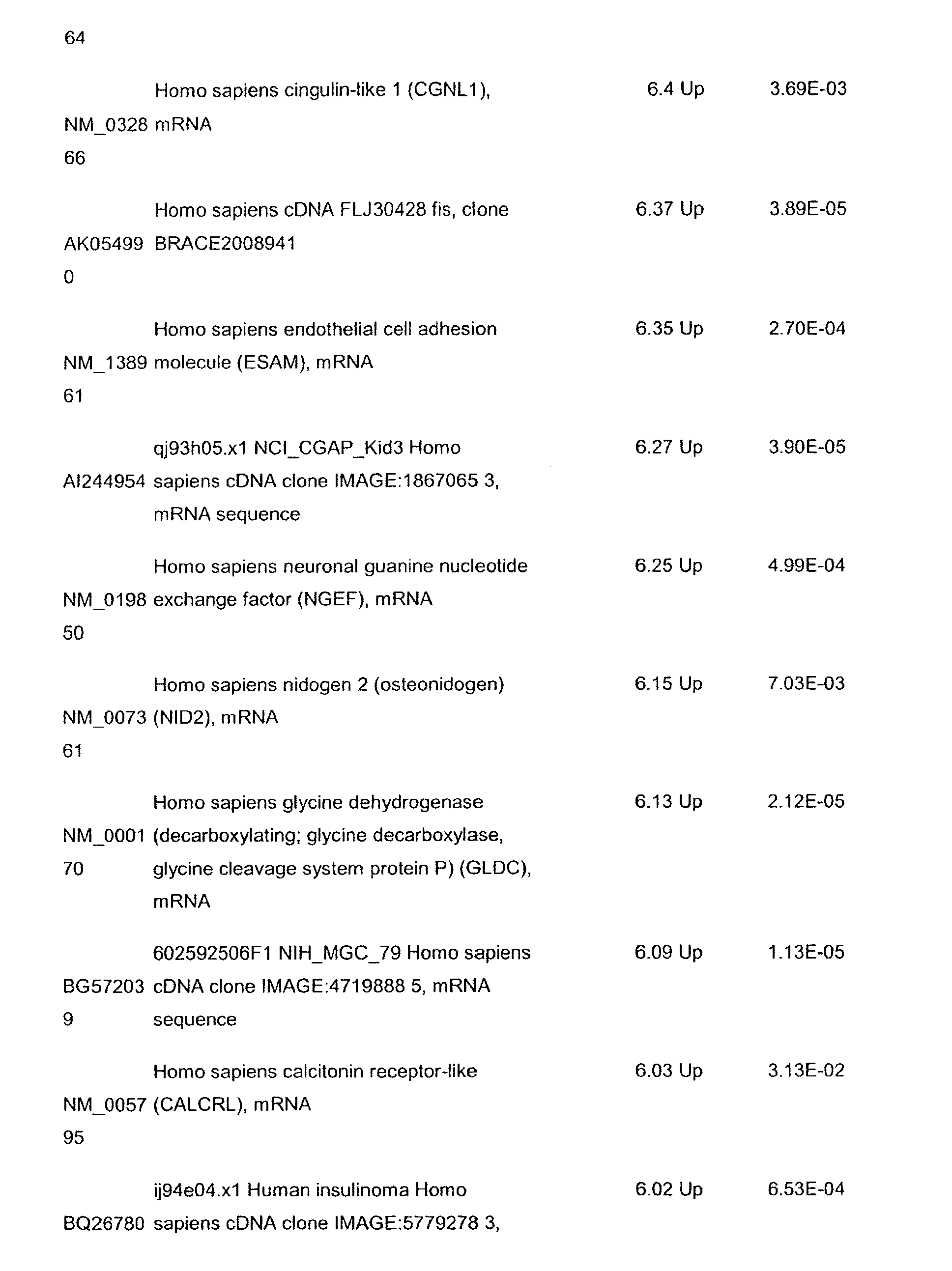

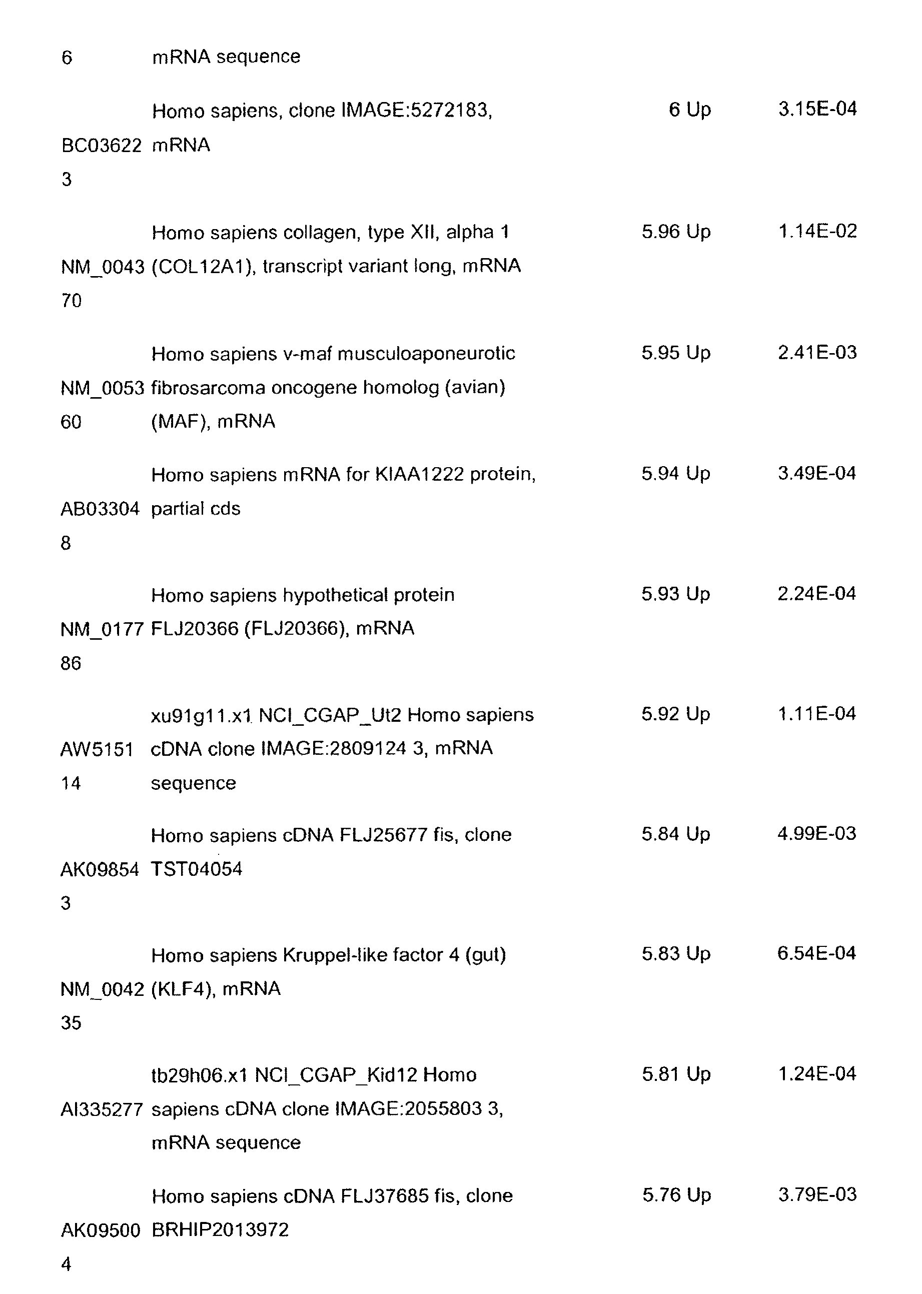

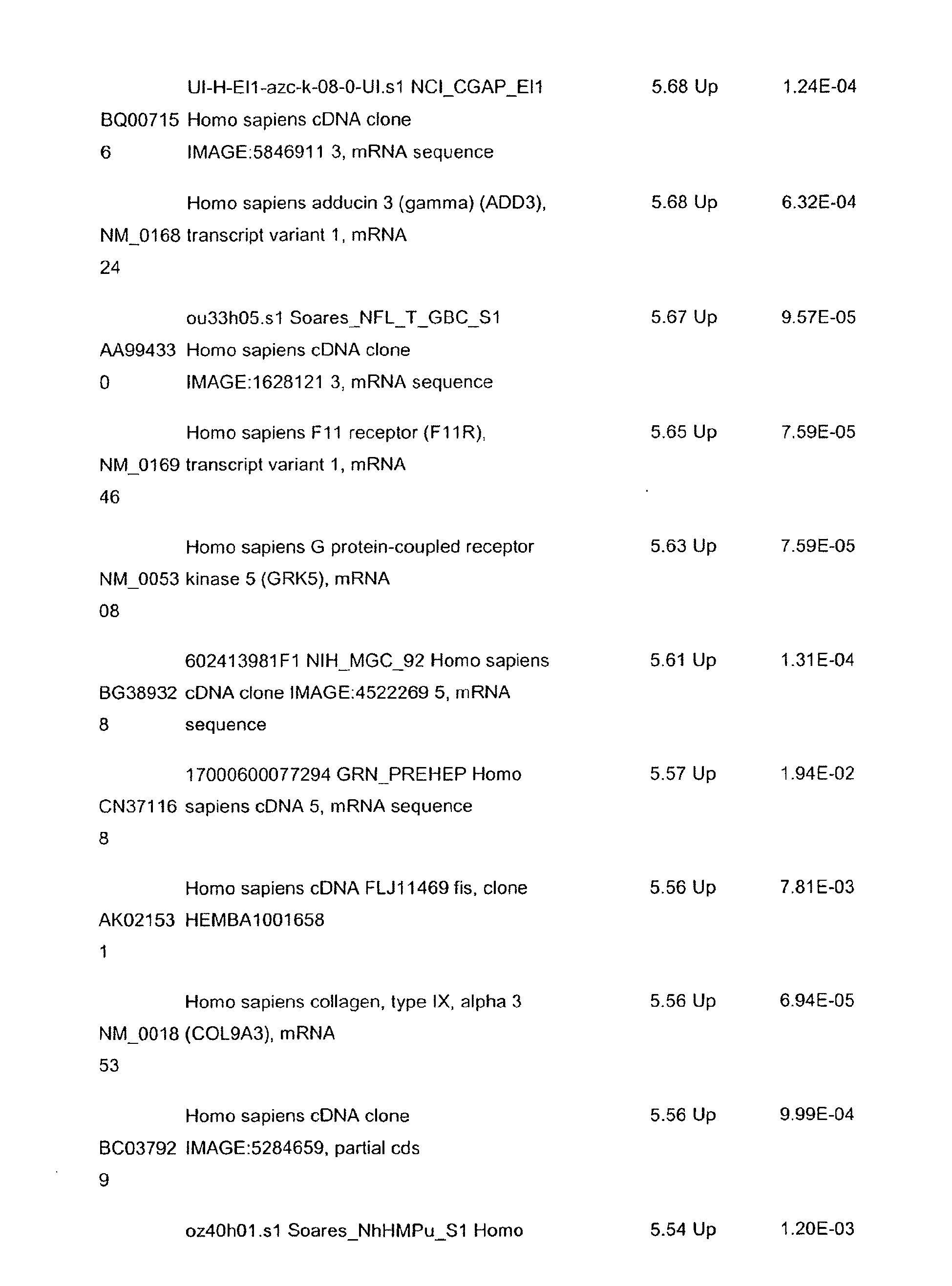

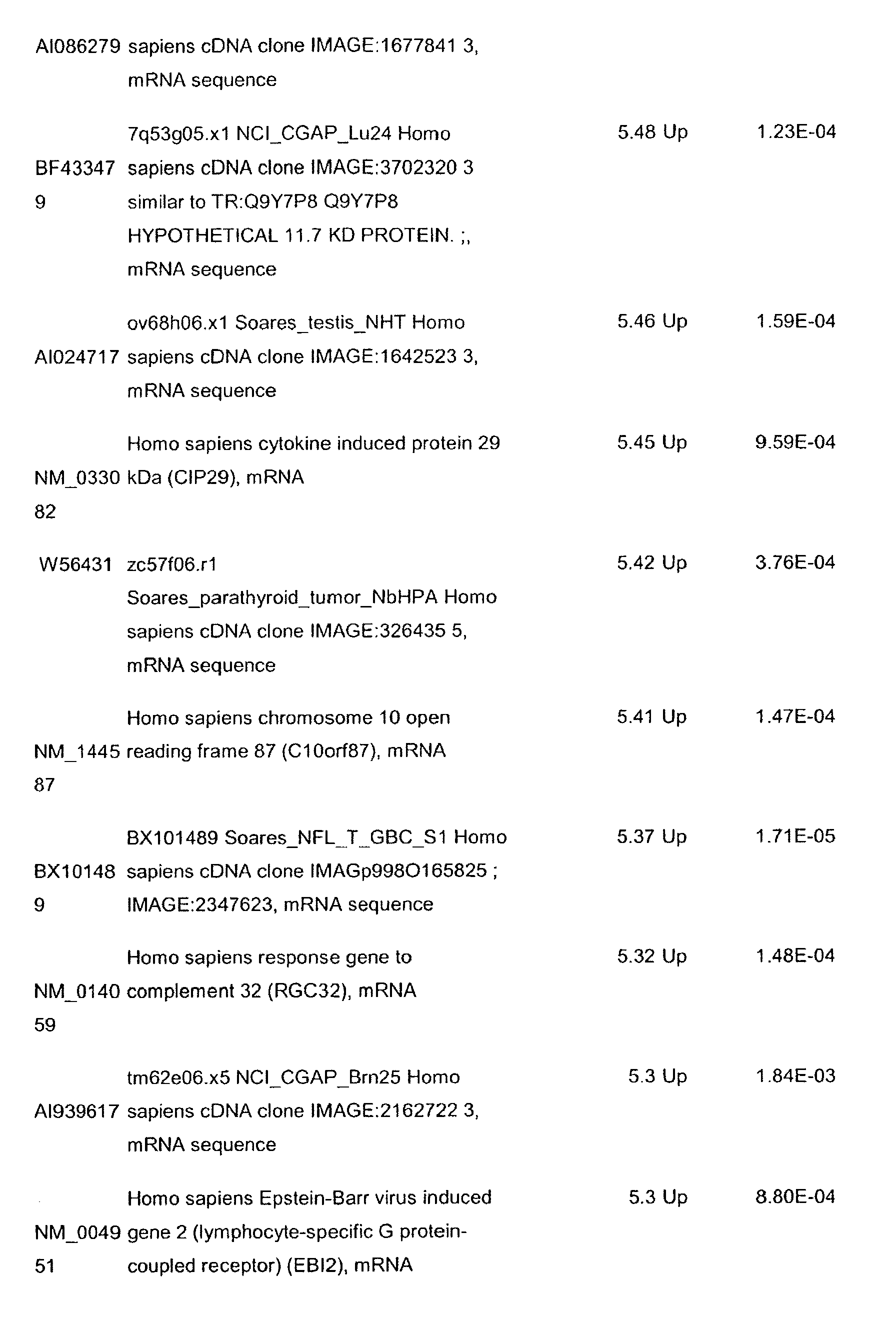

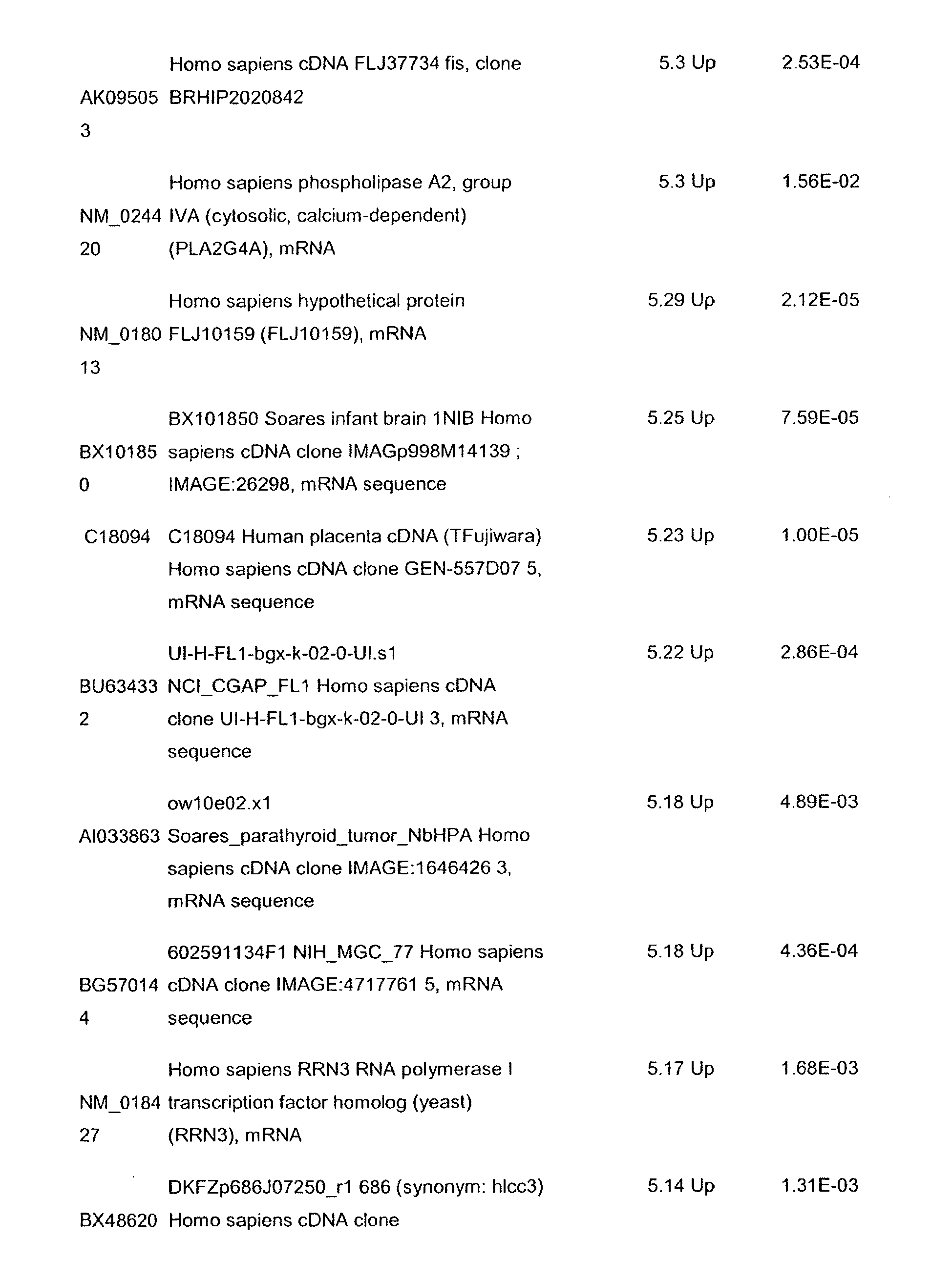

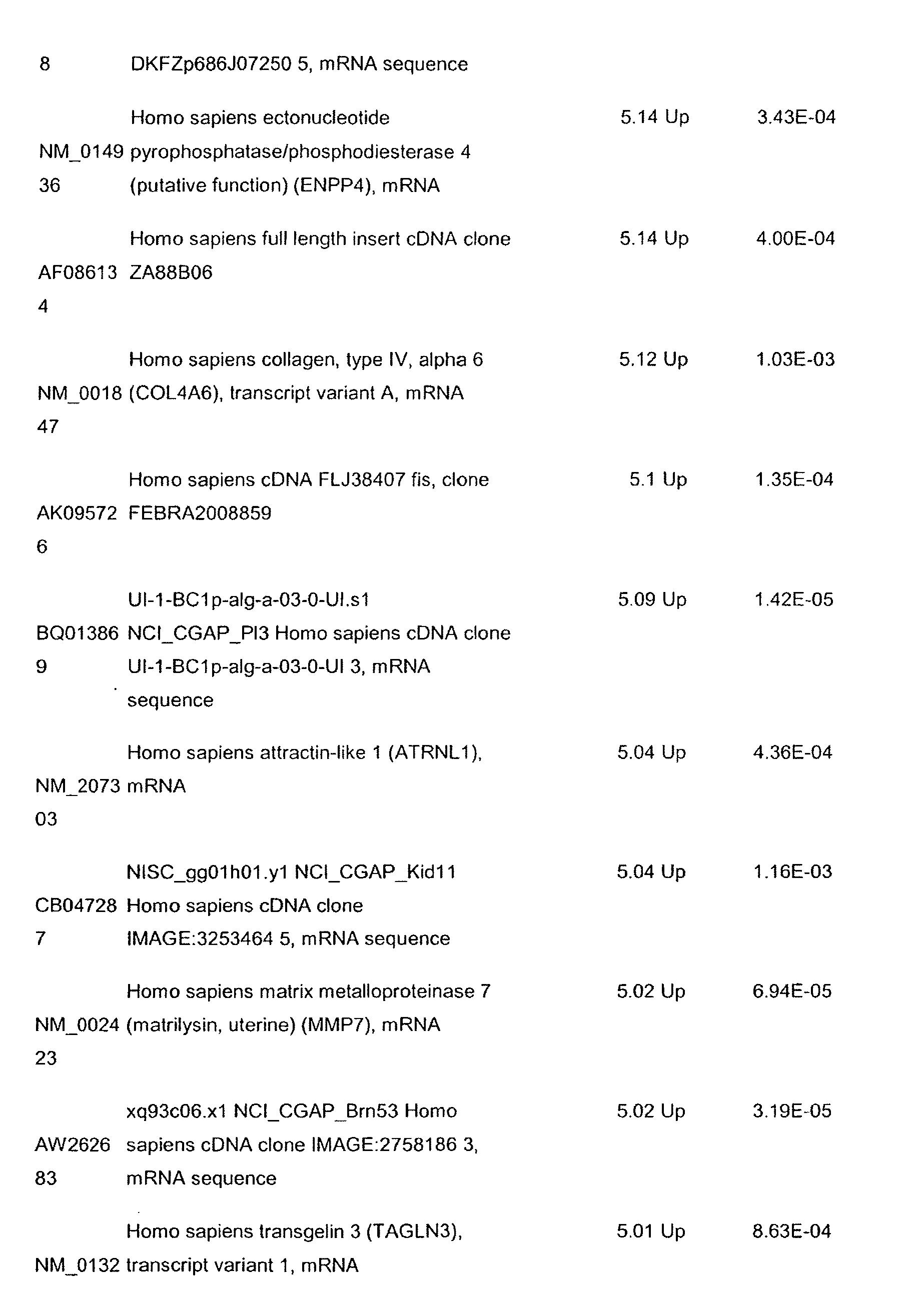

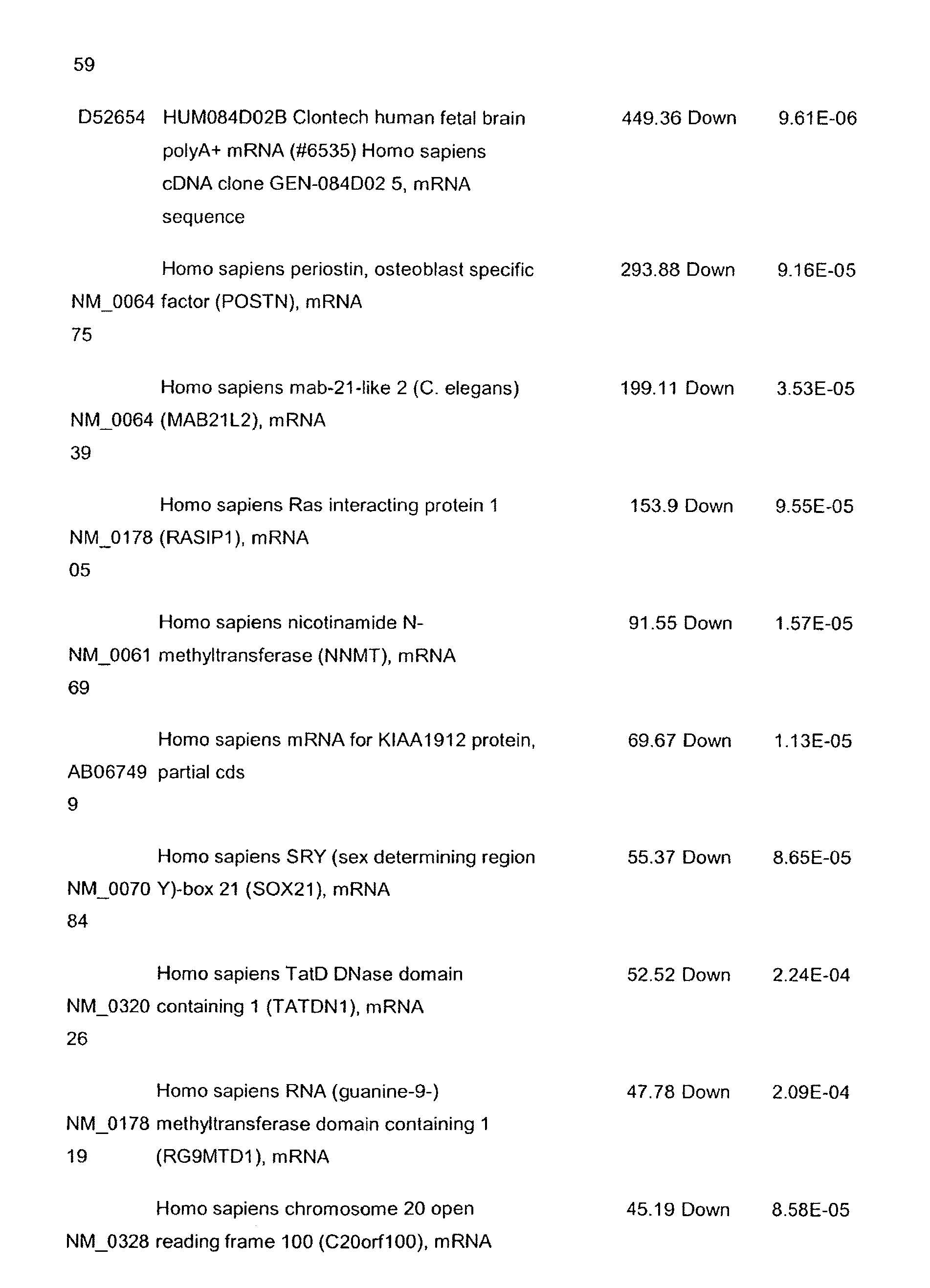

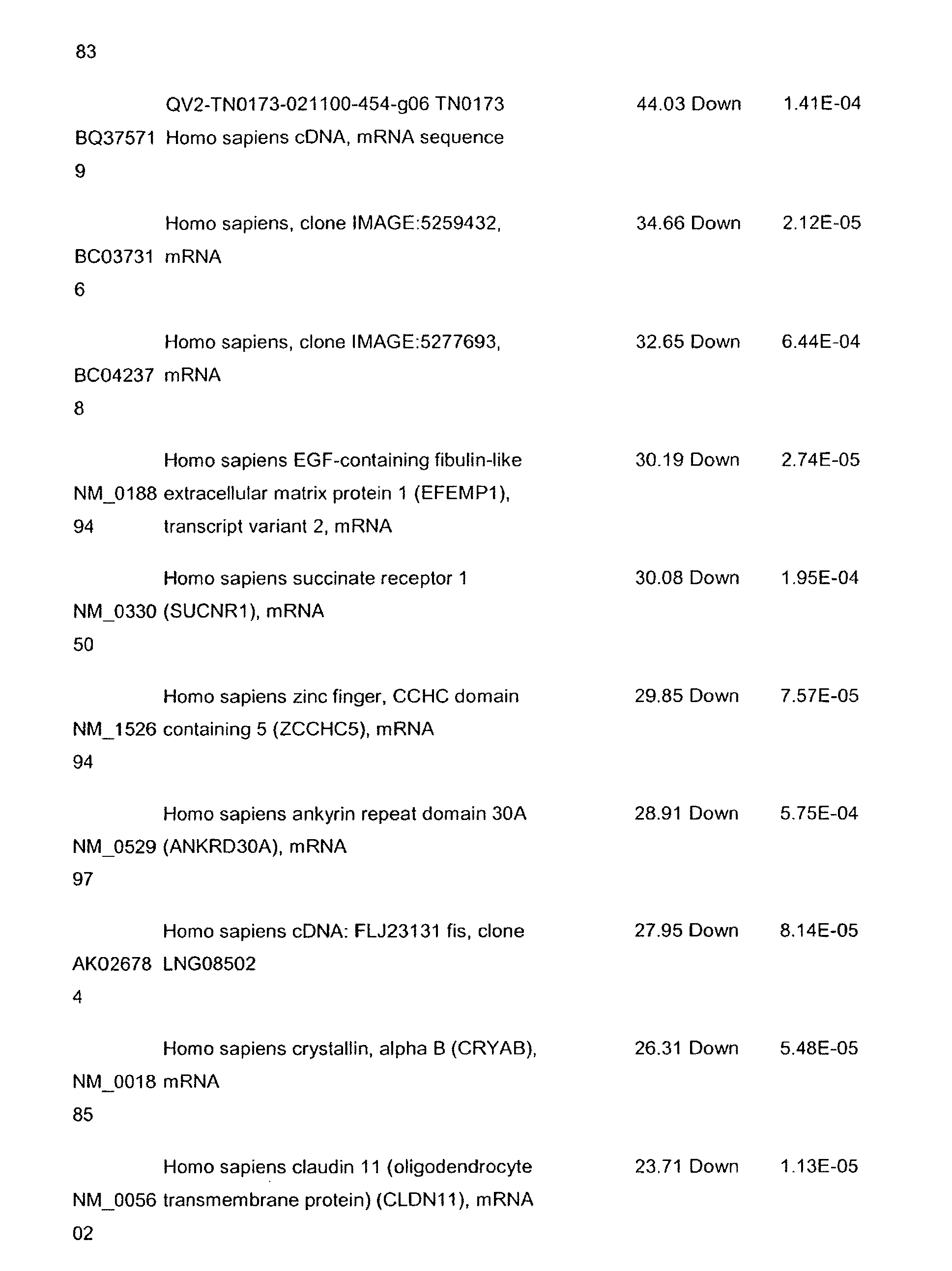

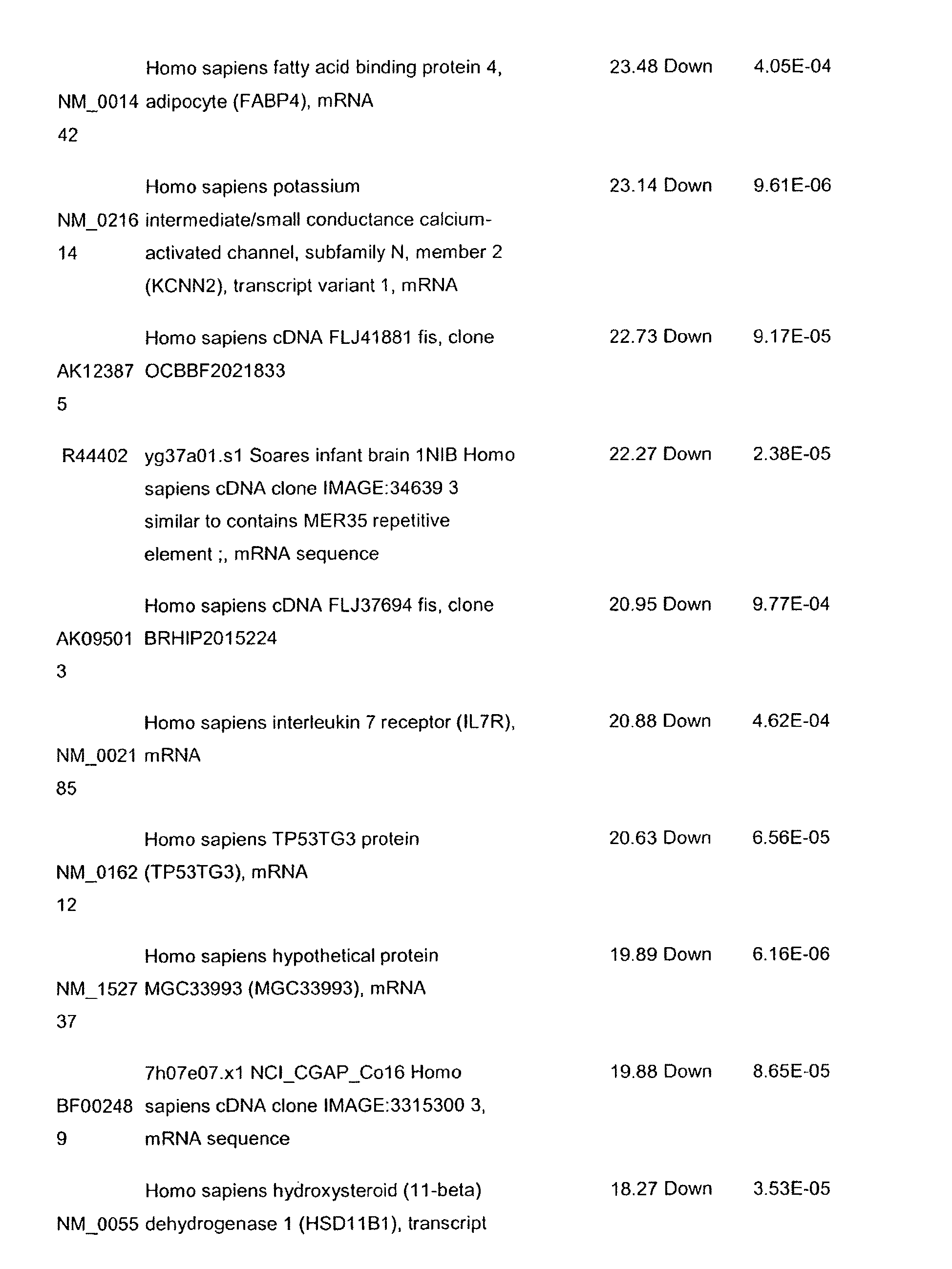

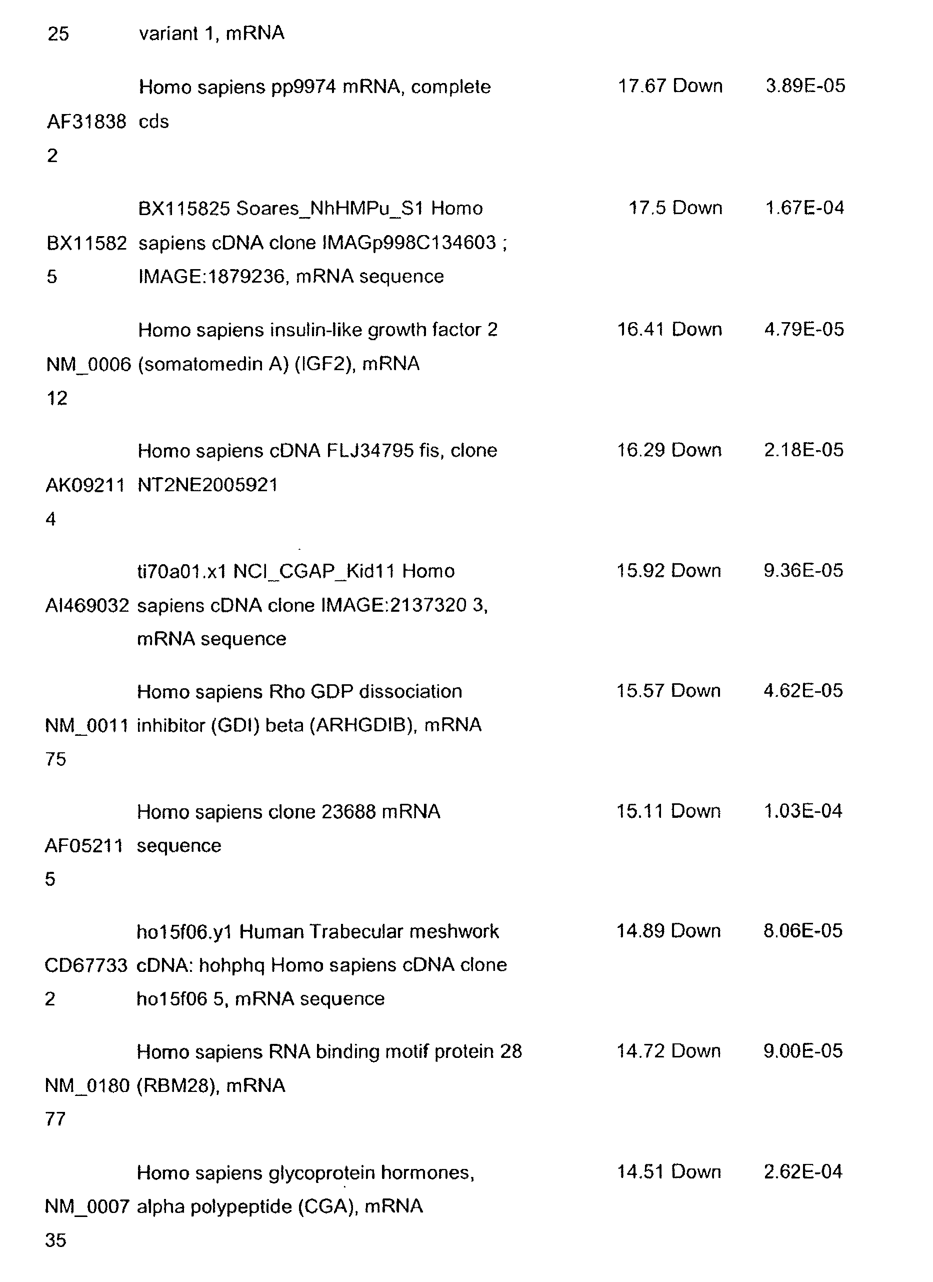

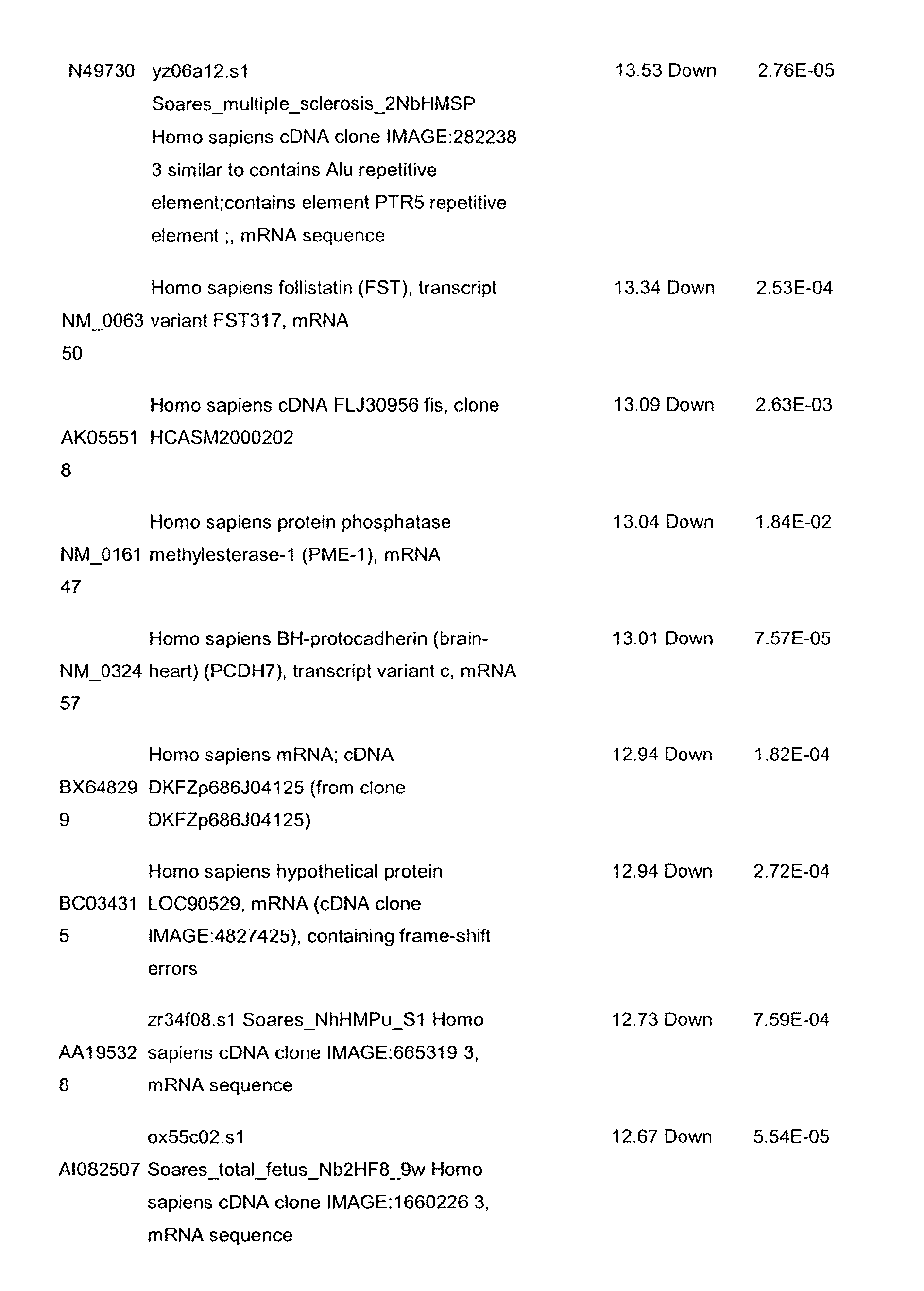

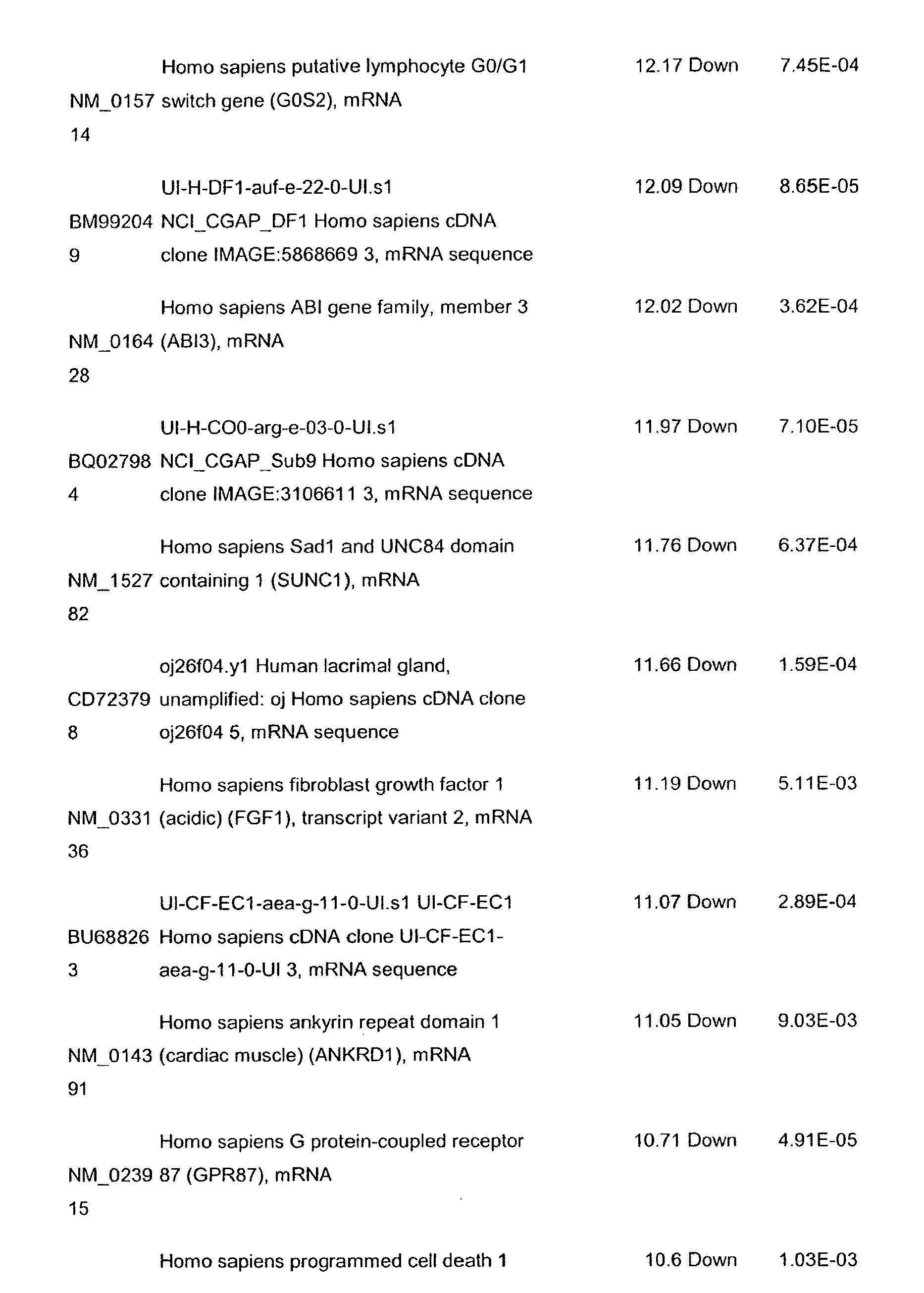

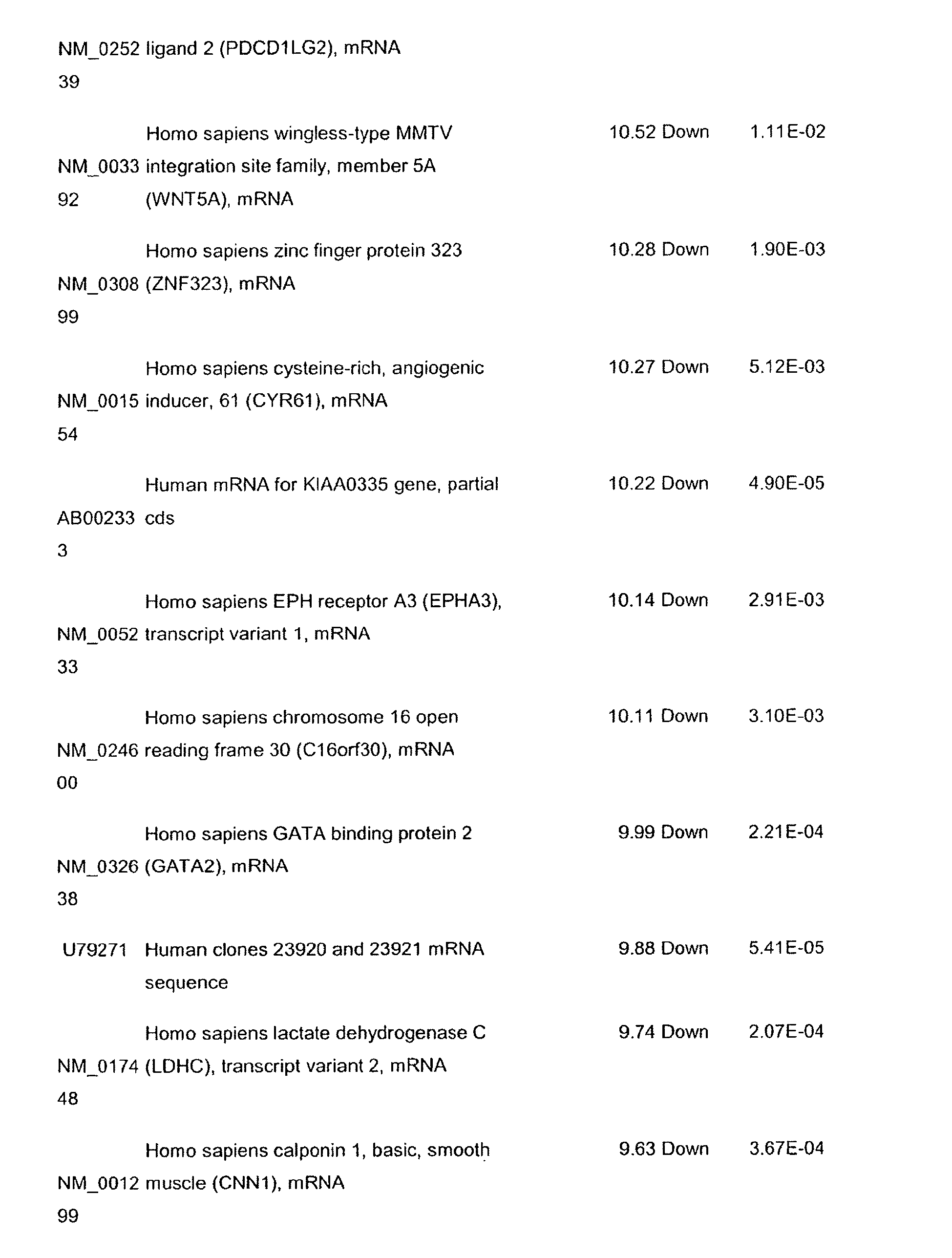

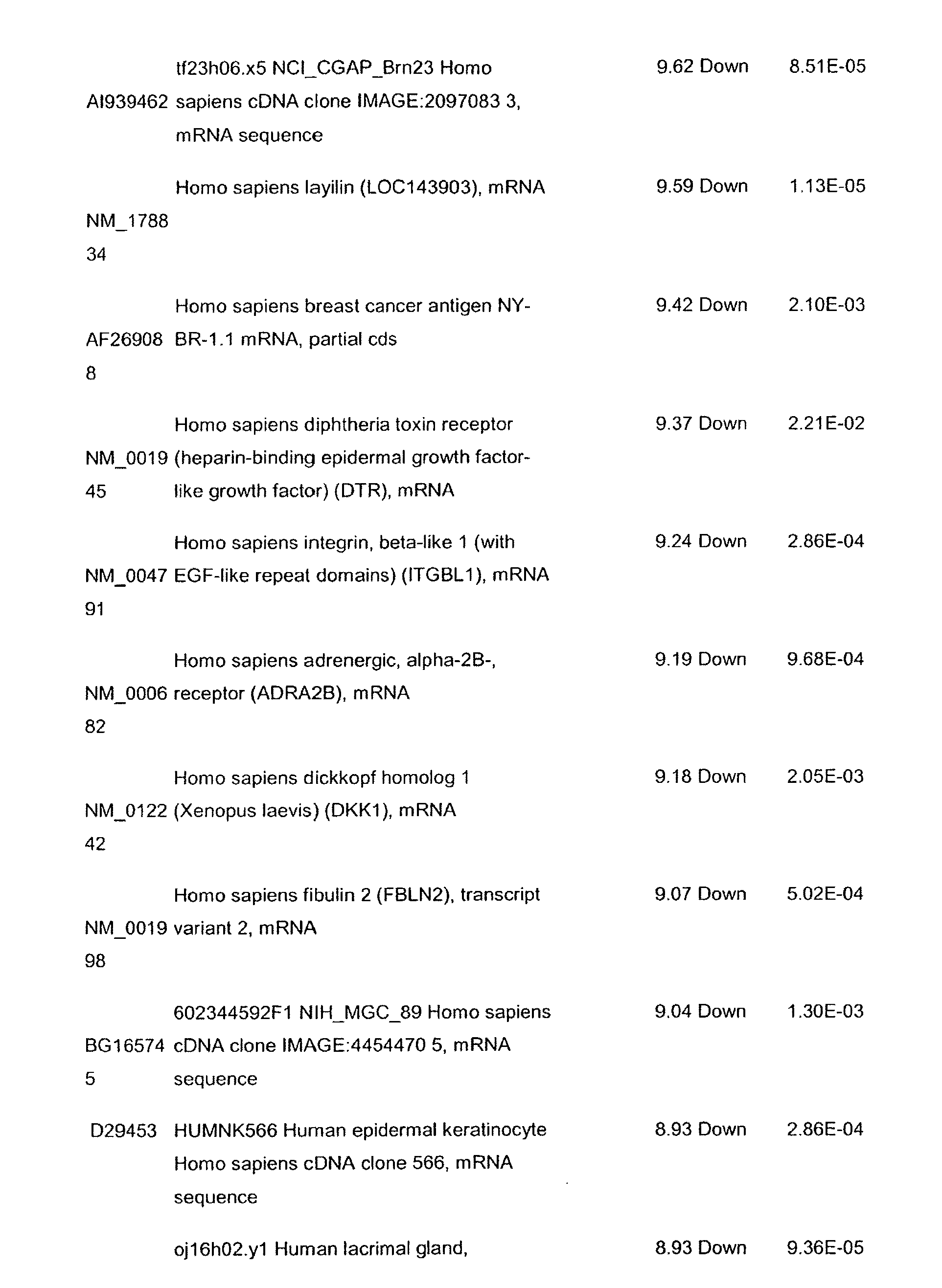

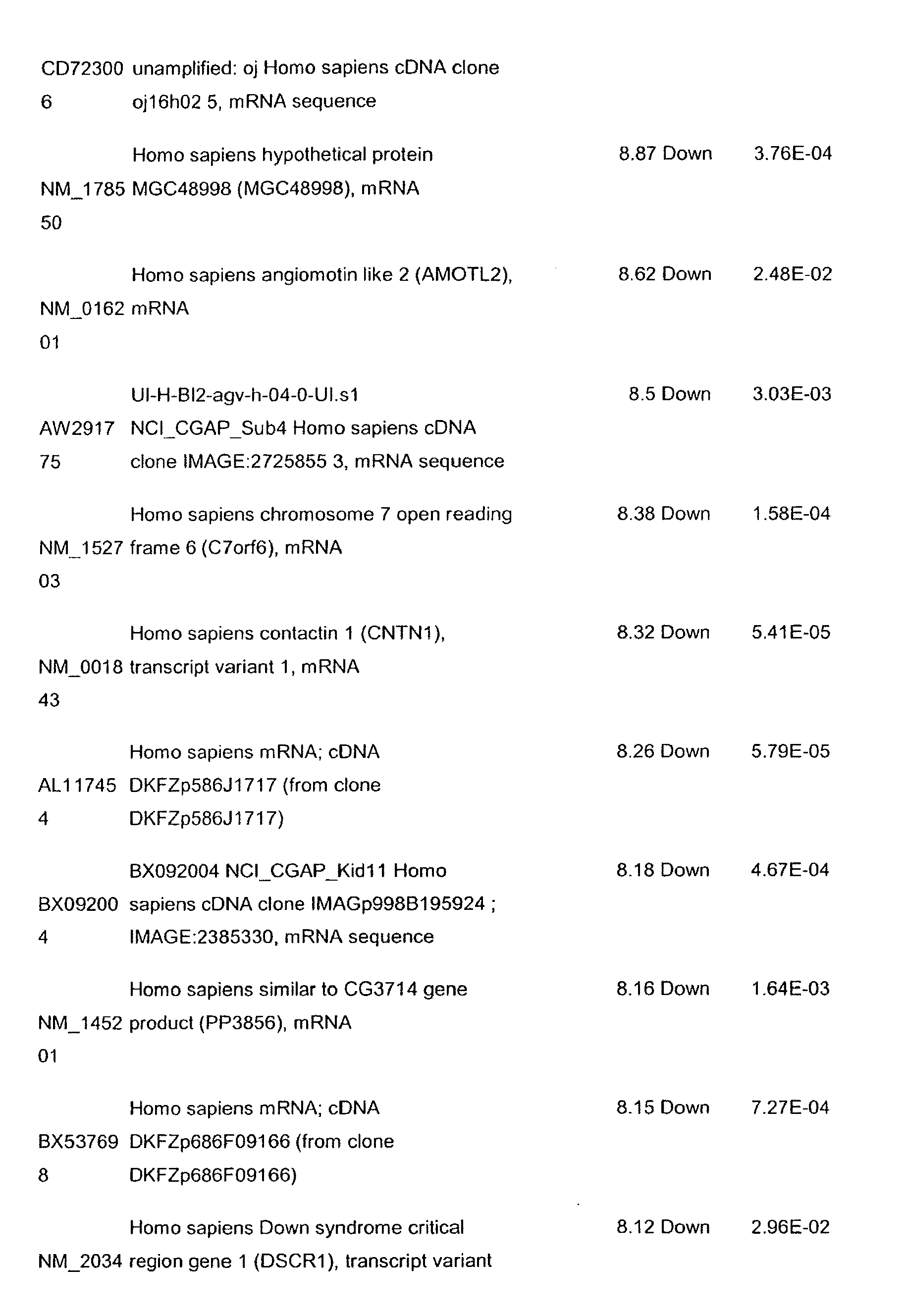

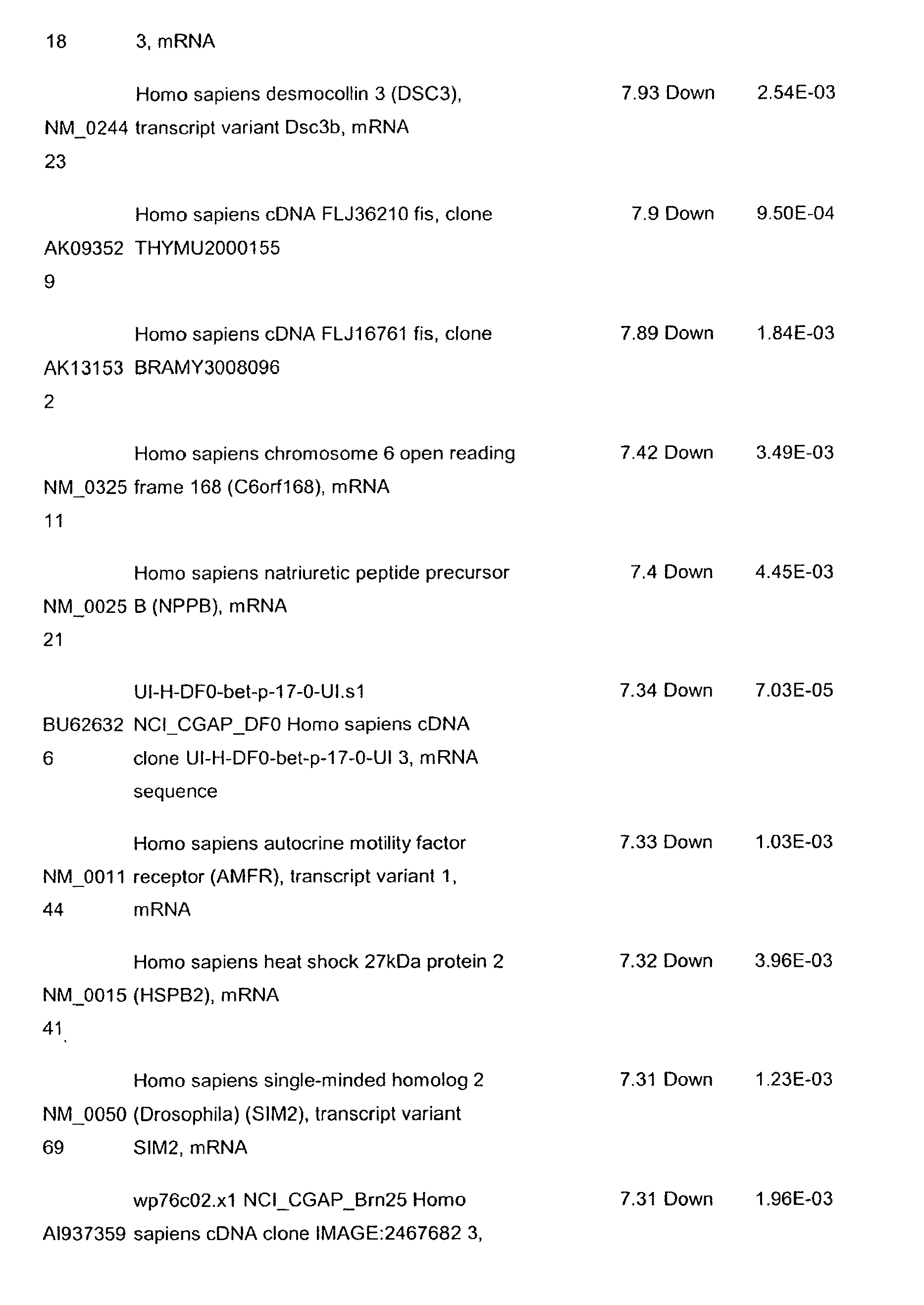

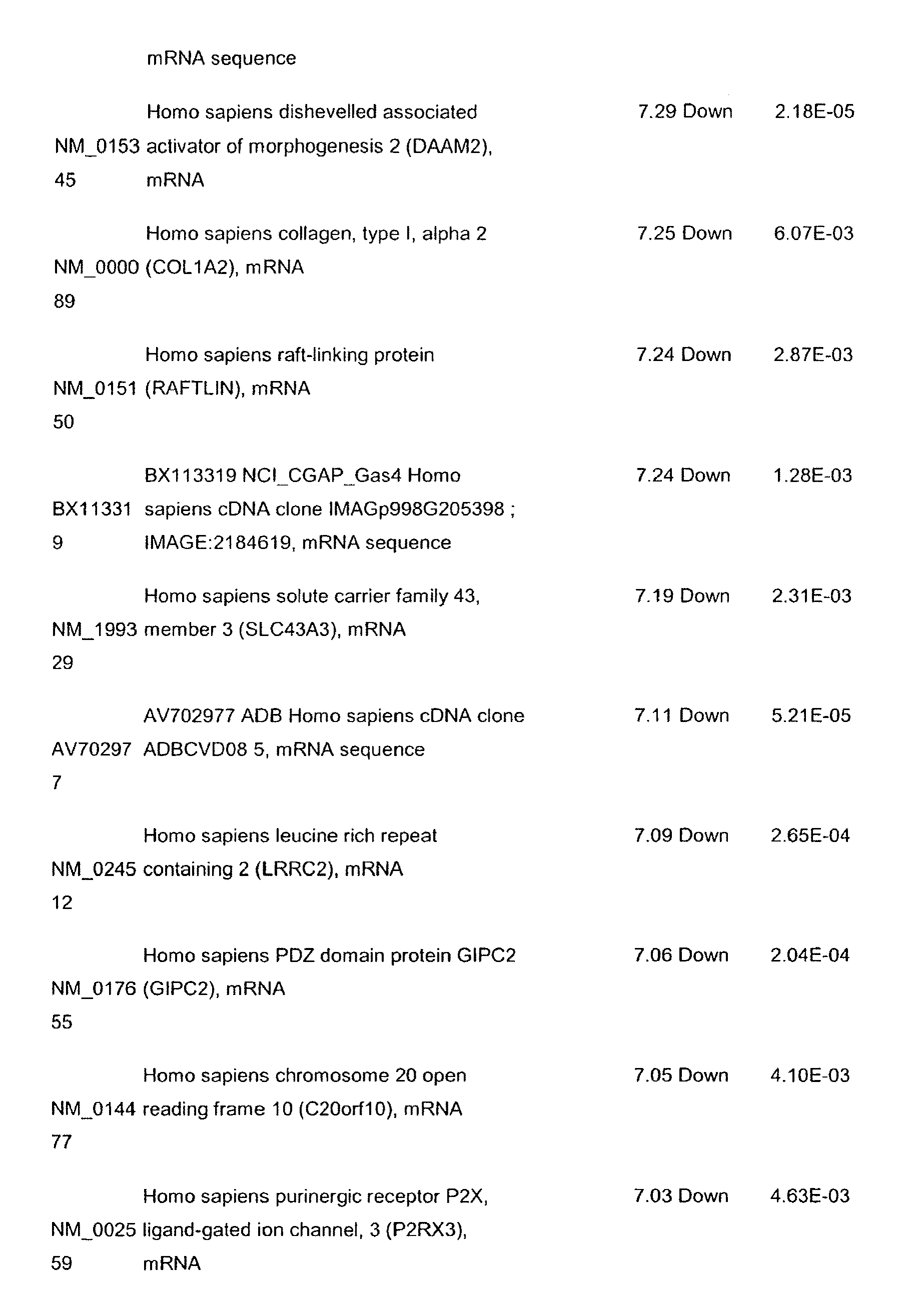

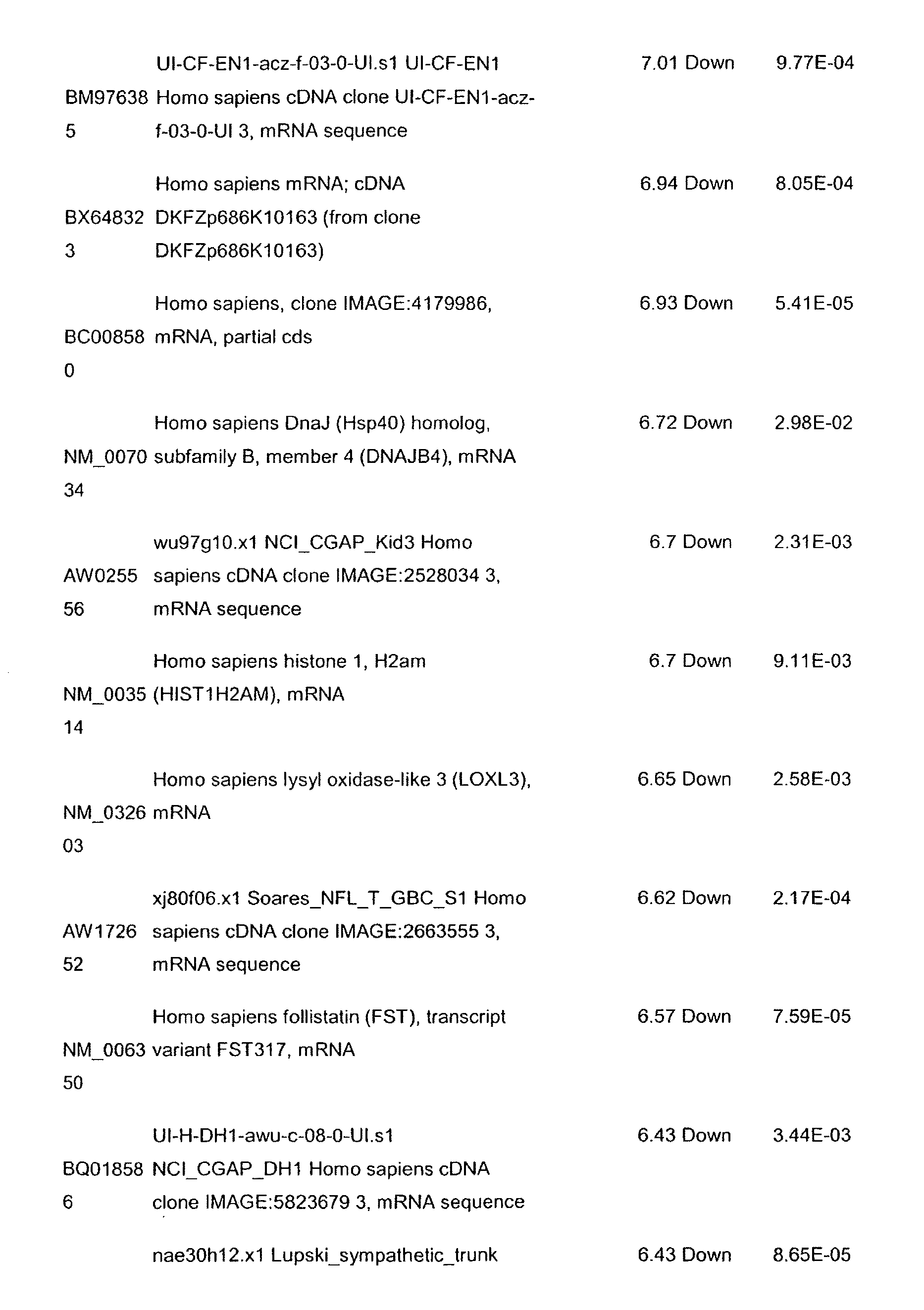

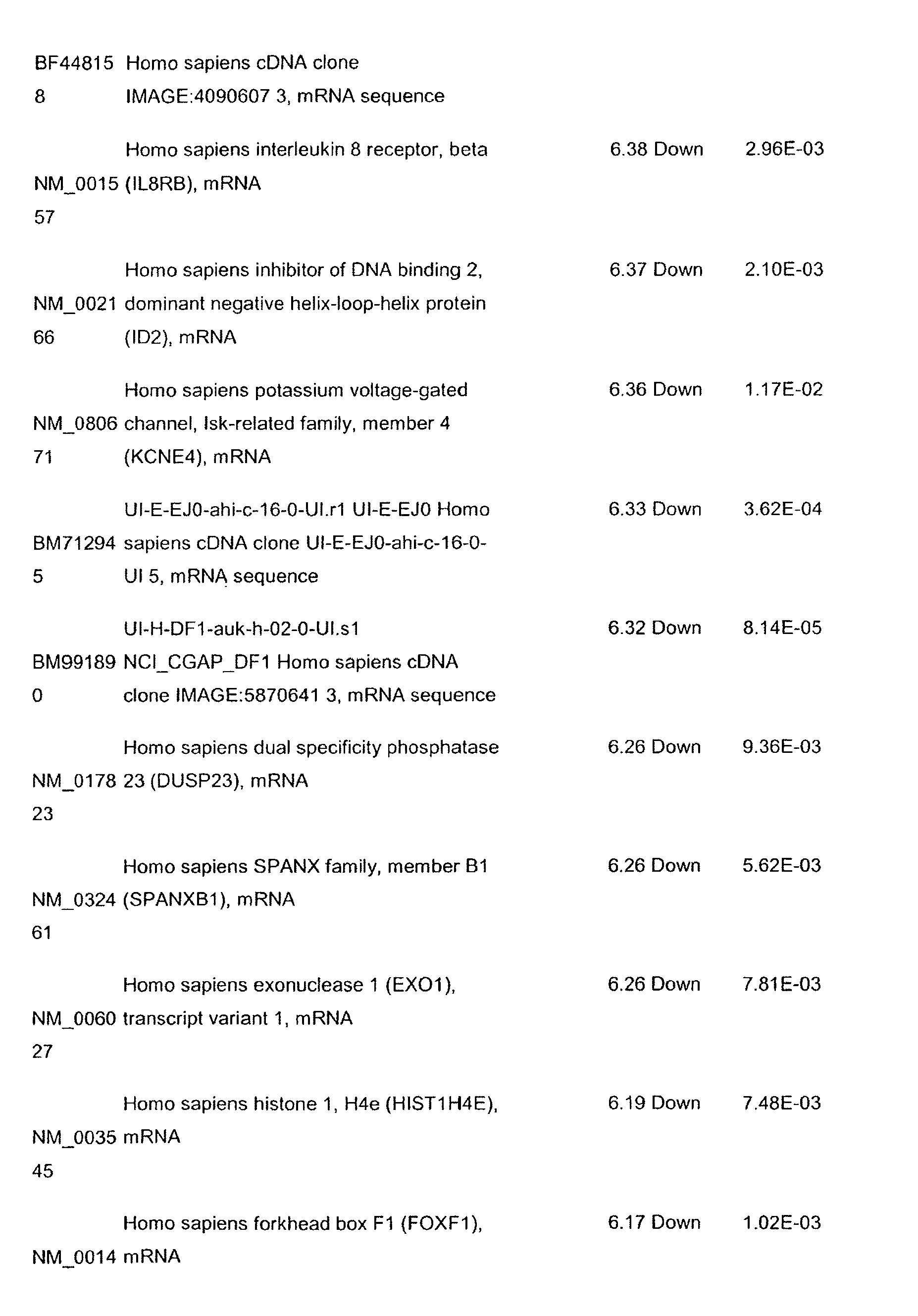

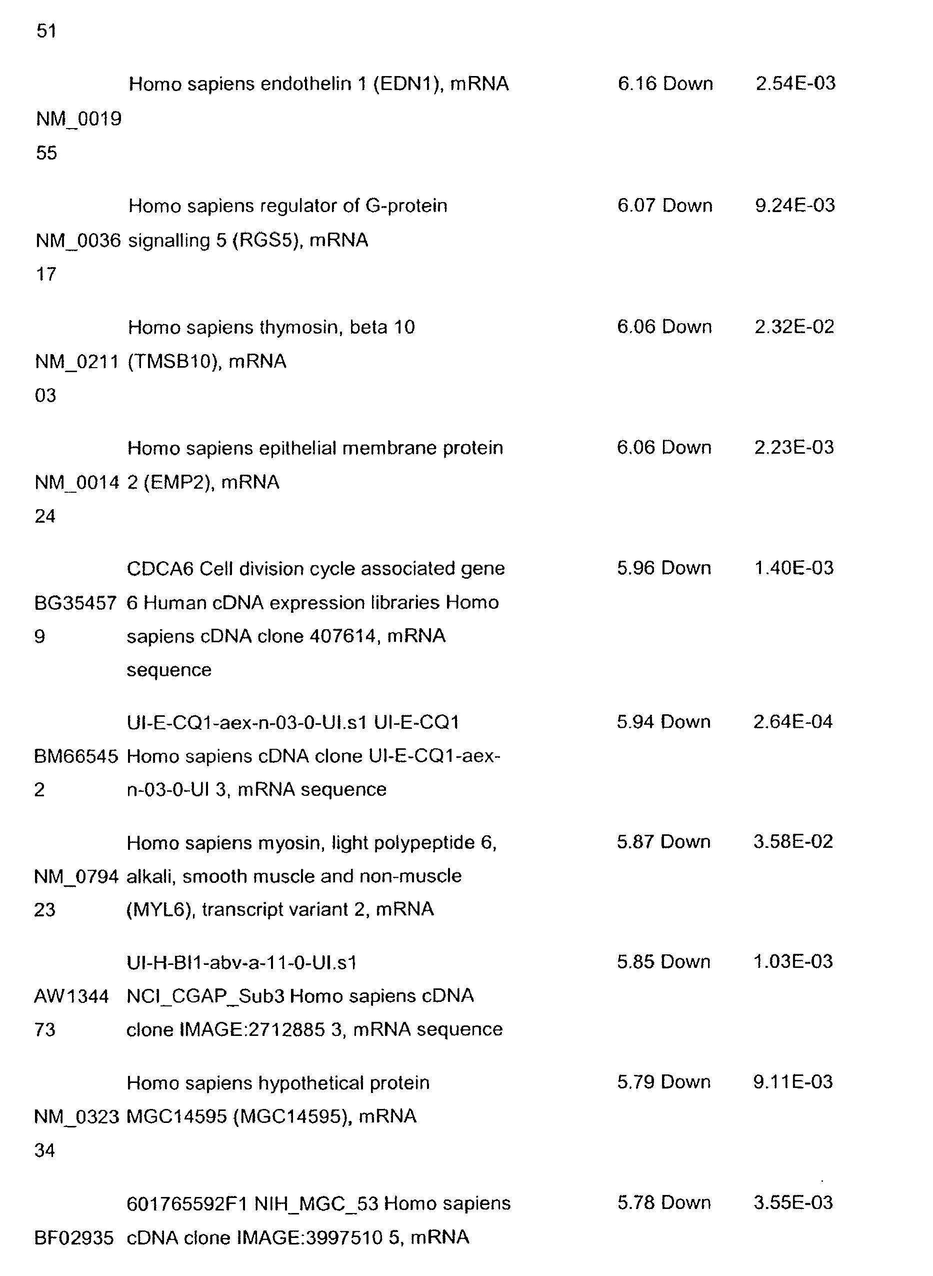

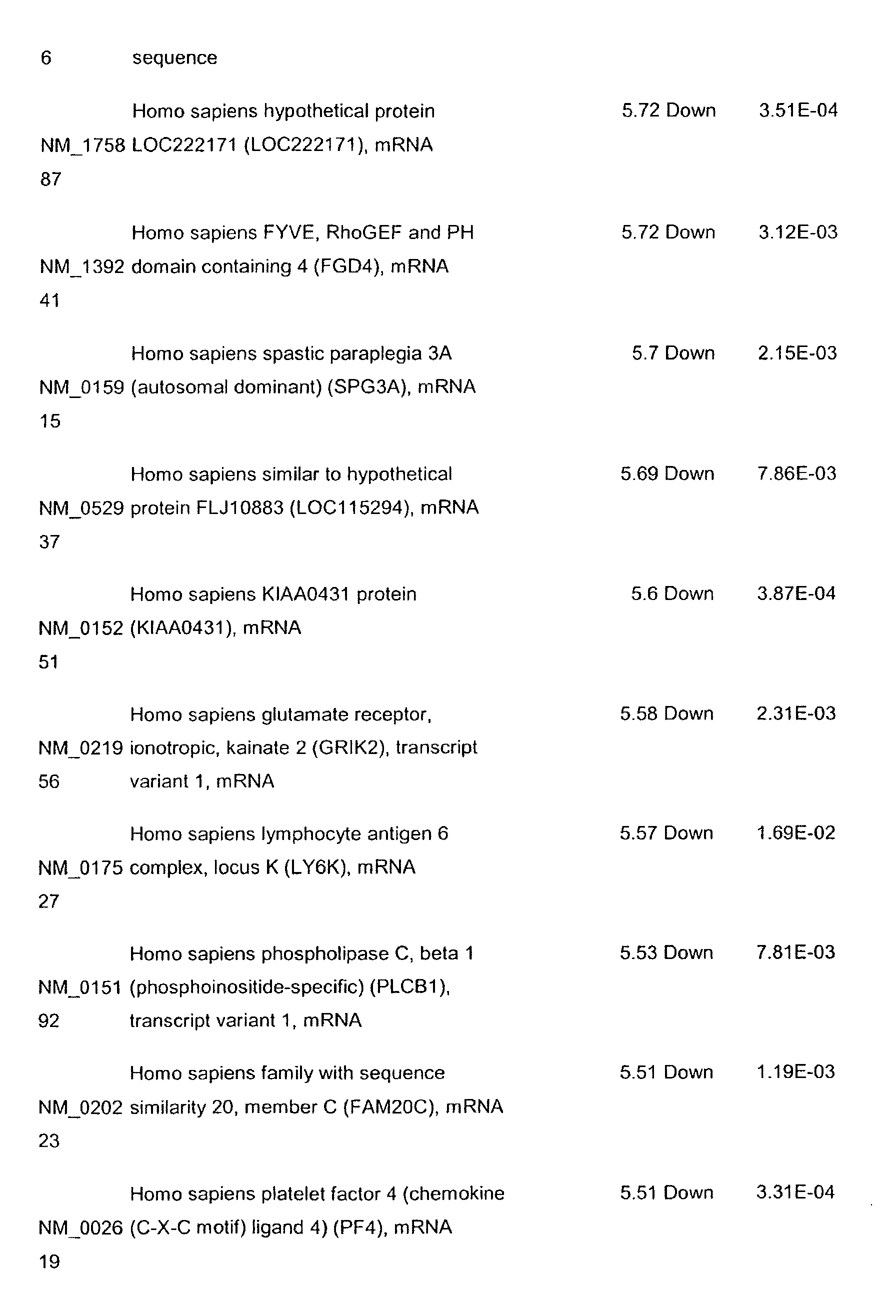

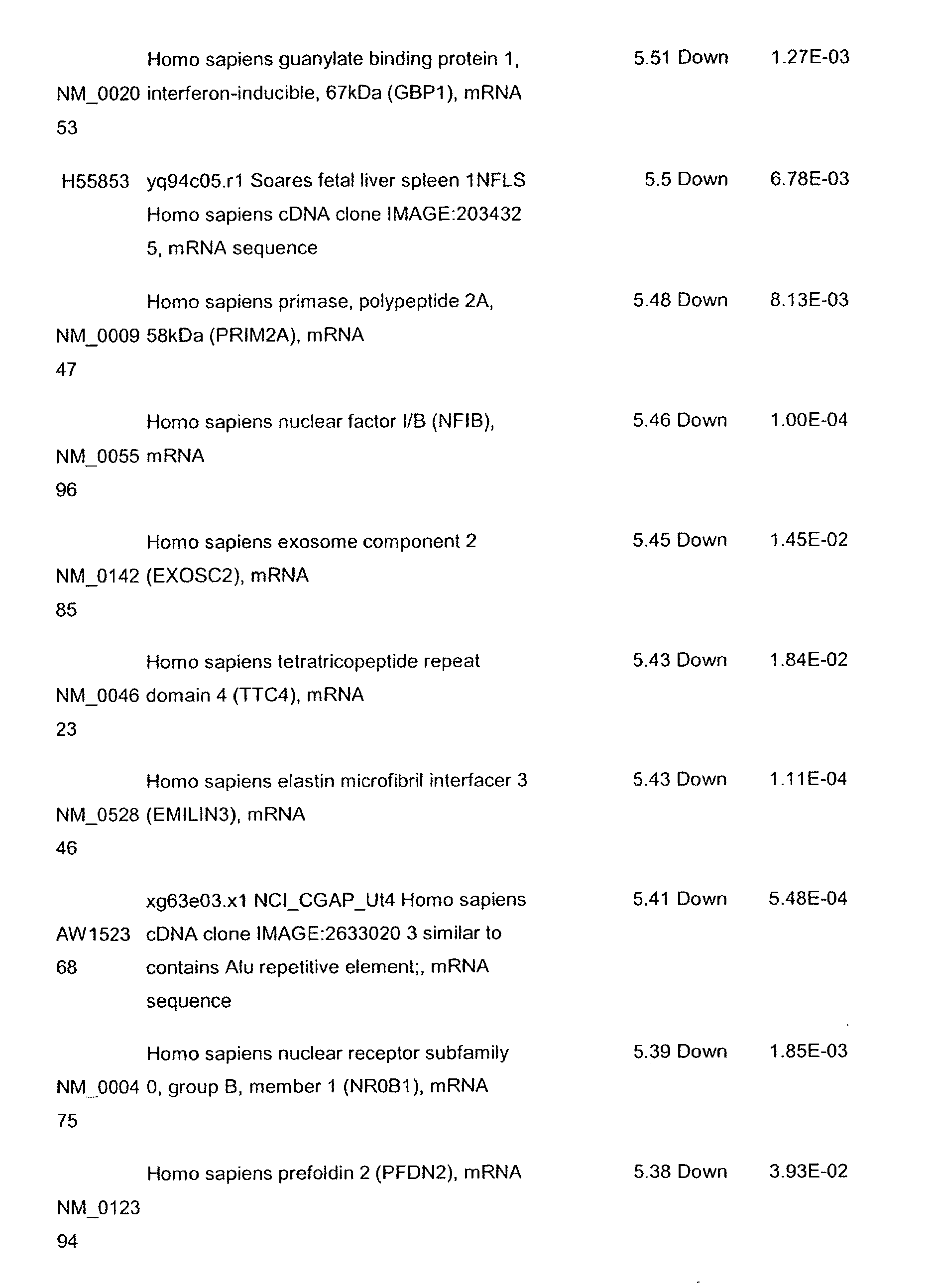

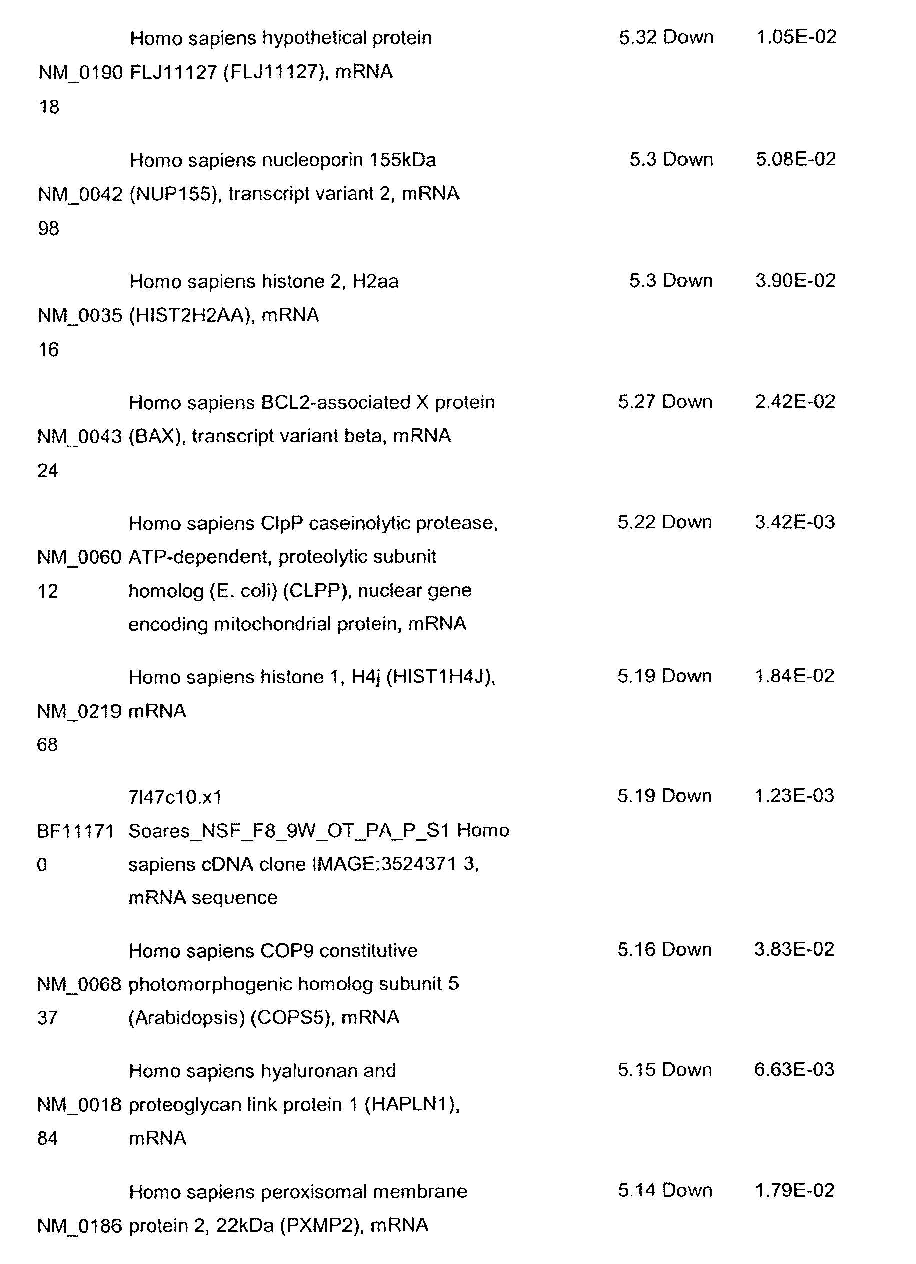

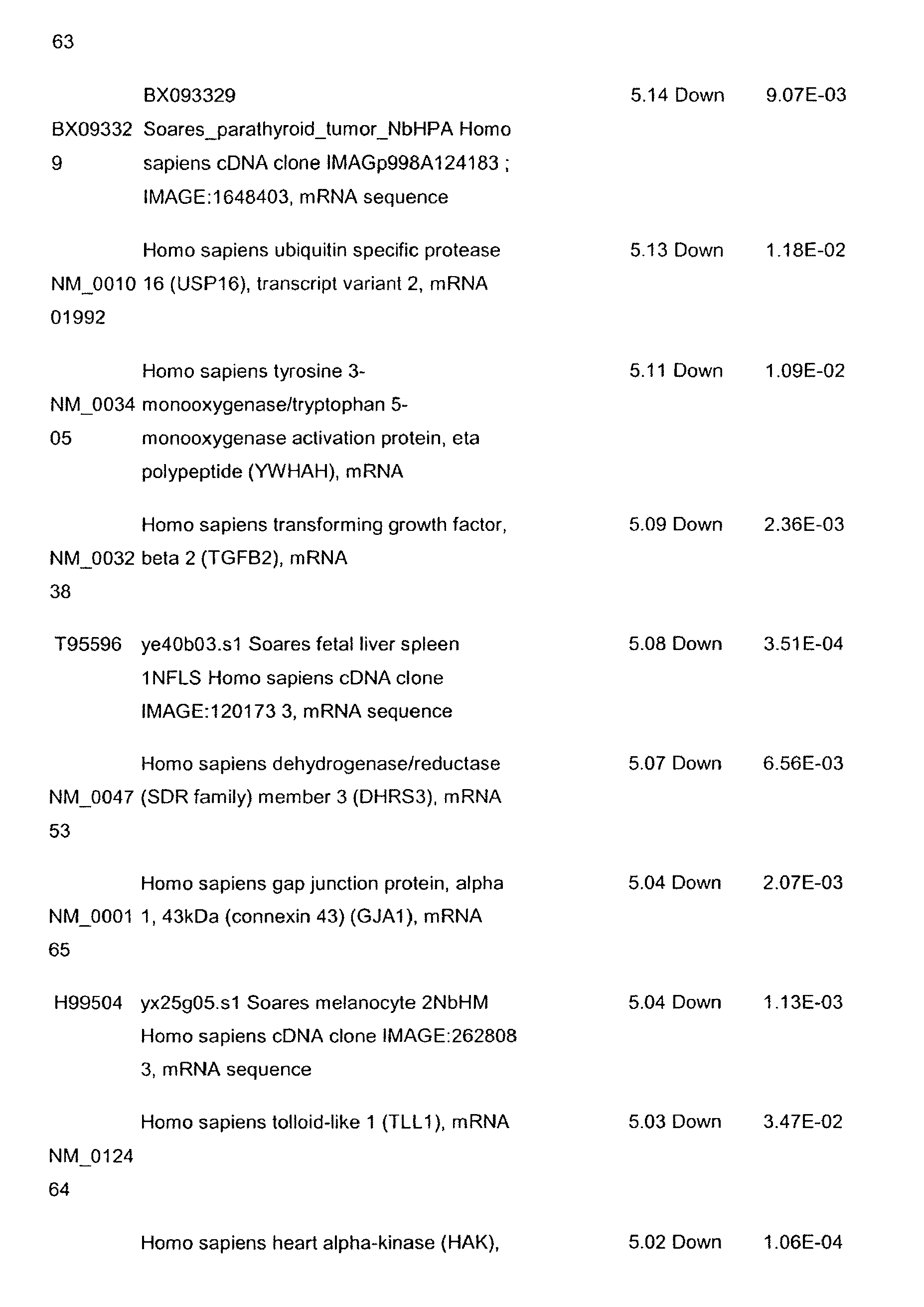

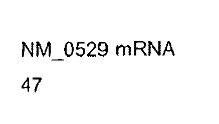

**TABLE VIII: REAL TIME PCR DATA FOR DIFFERENTIATED AF-I CELLS**

| Hormone | Ct values for AFCA007 Clone A cultured for 2 wks in Amniomax | Ct values for AFCA007 Clone A cultured for 2 wks in Amniomax + 10 micro molar retinoic acid | ΔΔCₜ for AFCA007 Clone A cultured for 2 wks in Amniomax | ΔΔCₜ for AFCA007 Clone A cultured for 2 wks in Amniomax + 10 micro molar retinoic acid |
|---|---|---|---|---|
| GIP | 34 | 31 | 0.00062 | 0.004 |
| Neurotensin | 37 | 35 | 0.000183 | 0.0004 |
| Gastrin | No | 36 | | 0.0004 |
| | expression | | | |
| Secretin | 32 | 32 | 0.02 | 0.014 |
| Somatostatin | 31 | 28 | 0.002 | 0.014 |
| CCK | 33 | 31 | 0.003 | 0.008 |
| GAPDH | 22 | 22 | | |

| | | | | |
|---|---|---|---|---|
| *: For ΔΔCₜ analysis, the positive control was human intestinal RNA | | | | |

**TABLE IX: PCR ANALYSIS OF THE EXPRESSION OF ENDODERM MARKERS IN AFCA007 (P8) AFTER 14-DAYS OF DIFFERENTIATION ASSAY**

| Treatment | HNF3β | Pdx-1 | GATA4 | Glu-2 | HNF1α | AFP |
|---|---|---|---|---|---|---|
| Untreated | - | - | - | + | - | - |
| FGF4 50 ng/ml | + | + | + | + | + | |
| All-trans RA | + | - | + | + | + | + |
| (10 µM) | | | | | | |
| L-685,458 | + | | + | + | + | |
| (10 µM) | | | | | | |
| Cyclopamine | | | + | + | | |
| (10 µM) | | | | | | |
| BMP5 | + | | + | + | + | |
| (5 ng/ml) | | | | | | |
| BMP6 | + | | + | + | + | |
| (50 ng/ml) | | | | | | |
| Excendin 4 | + | | + | + | + | |
| (10 nM) | | | | | | |
| BMP7 | | | + | + | + | |
| (10 ng/ml) | | | | | | |
| BMP4 | + | | | + | + | |
| (50 ng/ml) | | | | | | |

**TABLE X: CYTOKINE, CYTOKINE AND GROWTH FACTOR RECEPTOR EXPRESSION LEVEL FOR AFCA007 A (AF-I) AND AFCA015 C (AF-II) CELLS**

| | **AFCA007 A** | **AFCA007 A** | **AFCA015 C** | **AFCA015 C** |
|---|---|---|---|---|
| **POS** | 23,029.50 | 22,843.43 | 23,281.28 | 23,086.51 |
| **NEG** | 1.00 | 0.98 | 0.86 | 0.86 |
| **Angiogenin** | 44.50 | 89.98 | 101.17 | 26.71 |
| **BDNF** | 702.50 | 670.09 | 590.28 | 411.52 |
| **BLC** | 276.50 | 365.08 | 192.90 | 0.86 |
| **BMP-4** | 66.00 | 96.36 | 126.89 | 117.22 |
| **BMP-6** | 135.00 | 182.17 | 247.34 | 133.53 |
| **CK beta 8-1** | 139.50 | 100.28 | 80.16 | 43.87 |
| **CNTF** | 235.50 | 234.64 | 82.73 | 0.86 |
| **EGF** | 125.50 | 106.66 | 66.01 | 0.86 |
| **Eotaxin** | 90.00 | 95.87 | 152.61 | 99.21 |
| **Eotaxin-2** | 635.00 | 554.86 | 528.55 | 290.54 |
| **Eotaxin-3** | 174.50 | 245.43 | 246.48 | 170.42 |
| **FGF-6** | 422.00 | 411.67 | 318.07 | 213.32 |
| **FGF-7** | 378.50 | 348.41 | 225.05 | 15.12 |
| **Flt-3 Ligand** | 298.00 | 264.56 | 146.18 | 66.60 |
| **Fractalkine** | 545.50 | 399.41 | 330.50 | 105.21 |
| **GCP-2** | 323.00 | 266.52 | 182.18 | 0.86 |
| **GDNF** | 249.00 | 213.56 | 100.74 | 15.55 |
| **GM-CSF** | 1,003.50 | 687.75 | 663.15 | 343.31 |
| **I-309** | 108.00 | 90.47 | 91.74 | 36.57 |
| **IFN-gamma** | 227.50 | 189.04 | 217.34 | 7.40 |
| **IGFBP-1** | 156.00 | 197.37 | 182.61 | 37.00 |
| **IGFBP-2** | 627.00 | 415.10 | 383.66 | 281.53 |
| **IGFBP-4** | 154.00 | 136.57 | 103.74 | 0.86 |
| **IGF-I** | 121.00 | 200.32 | 218.62 | 191.01 |
| **IL-10** | 474.50 | 379.30 | 320.22 | 64.89 |
| **IL-13** | 768.00 | 627.43 | 636.57 | 184.58 |
| **IL-15** | 829.50 | 542.60 | 558.56 | 226.62 |
| **IL-16** | 243.50 | 235.62 | 146.18 | 2.25 |
| **IL-1alpha** | 1,206.00 | 1,058.47 | 1,048.95 | 662.05 |
| **IL-1beta** | 349.00 | 282.21 | 258.49 | 64.89 |
| **IL-1ra** | 278.50 | 337.13 | 225.05 | 111.65 |
| **IL-2** | 684.50 | 581.34 | 489.97 | 254.50 |
| **IL-3** | 1,052.50 | 1,066.31 | 1,075.53 | 904.86 |
| **IL-4** | 396.00 | 404.80 | 383.23 | 224.47 |
| **IL-5** | 763.00 | 676.96 | 754.89 | 303.41 |
| **IL-6** | 1,172.00 | 1,113.88 | 485.25 | 327.43 |
| **IL-7** | 675.00 | 567.61 | 473.68 | 241.63 |
| **Leptin** | 269.50 | 267.99 | 229.77 | 118.08 |
| **LIGHT** | 295.50 | 254.75 | 214.33 | 85.48 |
| **MCP-1** | 1,027.00 | 901.06 | 1,080.25 | 990.24 |
| **MCP-2** | 546.00 | 461.20 | 389.66 | 120.66 |
| **MCP-3** | 378.50 | 372.93 | 288.49 | 85.91 |
| **MCP-4** | 642.50 | 532.30 | 390.95 | 159.69 |
| **M-CSF** | 692.00 | 566.63 | 504.11 | 267.37 |
| **MDC** | 446.50 | 442.07 | 312.07 | 138.24 |
| **MIG** | 760.00 | 725.51 | 701.30 | 363.47 |
| **MIP-1-delta** | 290.50 | 347.92 | 295.78 | 117.65 |
| **MIP-3-alpha** | 406.50 | 399.90 | 311.64 | 64.46 |
| **NAP-2** | 216.50 | 241.51 | 208.33 | 44.29 |
| **NT-3** | 45.50 | 124.31 | 61.30 | 0.86 |
| **PARC** | 181.50 | 208.16 | 138.46 | 0.86 |
| **PDGF-BB** | 594.00 | 313.10 | 126.03 | 152.83 |
| **RANTES** | 418.50 | 350.86 | 248.63 | 108.21 |
| **SCF** | 307.50 | 152.26 | 84.45 | 45.58 |
| **SDF-1** | 654.50 | 594.09 | 237.48 | 116.37 |
| TARC | 227.50 | 264.56 | 148.75 | 2.25 |
| **TGF-beta** 1 | 824.50 | 750.02 | 693.59 | 407.23 |
| **TGF-beta** 3 | 278.00 | 341.54 | 277.35 | 57.16 |
| **TNF-alpha** | 804.50 | 968.73 | 992.37 | 552.23 |
| **TNF-beta** | 549.50 | 510.23 | 888.20 | 285.82 |
| **POS** | 24,267.79 | 23,873.40 | 25,002.74 | 25,119.43 |
| **NEG** | 1.00 | 0.89 | 0.78 | 0.78 |
| **Acrp30** | 29.13 | 0.89 | 100.74 | 98.12 |
| **AgRP** | 1.00 | 0.89 | 19.30 | 0.78 |
| **Angiopoietin-2** | 144.13 | 84.27 | 62.74 | 58.01 |
| **Amphiregulin** | 1.00 | 0.89 | 0.78 | 0.78 |
| **Axl** | 1,478.63 | 882.74 | 542.12 | 536.93 |
| **bFGF** | 5,455.13 | 4,386.44 | 4,419.49 | 7,225.00 |
| **b-NGF** | 94.13 | 8.27 | 69.72 | 58.79 |
| **BTC** | 1.00 | 0.89 | 0.78 | 0.78 |
| **CCL-28** | 141.13 | 76.23 | 0.78 | 0.78 |
| **CTACK** | 383.13 | 315.86 | 304.37 | 297.86 |
| **Dtk** | 90.13 | 51.19 | 48.77 | 48.67 |
| **EGF-R** | 1,730.13 | 851.89 | 1,363.21 | 1,485.42 |
| **ENA-78** | 207.13 | 109.31 | 514.97 | 192.34 |
| **Fas/TNFRSF6** | 4,740.63 | 1,570.79 | 1,026.94 | 575.87 |
| **FGF-4** | 342.13 | 141.50 | 156.21 | 88.77 |
| **FGF-9** | 267.13 | 166.98 | 140.69 | 100.07 |
| **GCSF** | 320.63 | 151.78 | 152.33 | 115.25 |
| **GITR-Ligand** | 183.63 | 112.44 | 170.17 | 81.77 |
| **GITR** | 211.13 | 116.91 | 63.51 | 18.30 |
| **GRO** | 10,097.13 | 9,584.54 | 2,380.16 | 1,317.60 |
| **GRO-alpha** | 1,061.13 | 754.43 | 356.73 | 124.60 |
| **HCC-4** | 90.63 | 18.55 | 67.00 | 8.18 |
| HGF | 1.00 | 0.89 | 265.19 | 333.29 |
| ICAM-1 | 11,257.63 | 6,308.40 | 2,116.03 | 1,415.33 |
| **ICAM-3** | 75.13 | 19.89 | 54.98 | 61.52 |
| **IGFBP-3** | 603.63 | 406.61 | 435.46 | 346.92 |
| **IGFBP-6** | 523.63 | 334.63 | 253.56 | 176.77 |
| **IGF-I SR** | 929.13 | 526.87 | 484.72 | 463.73 |
| **IL-1 R4/ST2** | 330.63 | 297.97 | 272.95 | 171.71 |
| **IL-1 RI** | 57.13 | 40.91 | 40.24 | 16.74 |
| **IL-11** | 192.13 | 106.63 | 79.80 | 19.86 |
| **IL-12 p40** | 134.13 | 113.33 | 99.19 | 93.06 |
| **IL-12 p70** | 183.63 | 165.64 | 166.29 | 85.27 |
| **IL-17** | 47.13 | 0.89 | 0.78 | 0.78 |
| **IL-2 Rapha** | 229.13 | 158.04 | 115.87 | 36.21 |
| **IL-6 R** | 158.63 | 30.62 | 55.75 | 46.72 |
| **IL-8** | 2,792.13 | 2,894.57 | 528.93 | 164.70 |
| **I-TAC** | 499.63 | 184.42 | 139.53 | 89.16 |
| **Lymphotactin** | 581.63 | 277.86 | 295.45 | 215.71 |
| **MIF** | 2,280.63 | 1,794.32 | 1,532.31 | 1,042.71 |
| **MIP-1alpha** | 557.13 | 448.19 | 319.49 | 261.65 |
| **MIP-1beta** | 193.13 | 133.45 | 182.58 | 225.44 |
| **MIP-3beta** | 360.13 | 203.19 | 126.34 | 101.23 |
| **MSP-alpha** | 251.13 | 174.13 | 121.69 | 89.94 |
| **NT-4** | 115.13 | 27.94 | 2.62 | 0.78 |
| **Osteoprotegerin** | 2,391.13 | 1,761.24 | 1,558.30 | 1,582.37 |
| **Oncostatin M** | 541.13 | 340.45 | 179.48 | 98.51 |
| **PIGF** | 88.13 | 50.74 | 40.24 | 11.29 |
| **sgp130** | 604.13 | 503.63 | 369.92 | 379.24 |
| **sTNF RII** | 17.63 | 0.89 | 0.78 | 8.57 |
| **sTNF-RI** | 903.63 | 551.91 | 685.63 | 469.18 |
| **TECK** | 255.63 | 91.87 | 140.69 | 100.46 |
| **TIMP-1** | 1,099.63 | 732.53 | 661.58 | 832.85 |
| **TIMP-2** | 11,468.63 | 7,205.68 | 14,022.74 | 14,546.57 |
| **Thrombopoietin** | 580.13 | 364.59 | 405.21 | 281.90 |
| **TRAIL R3** | 1,881.13 | 728.50 | 988.93 | 1,186.39 |
| **TRAIL R4** | 437.13 | 412.42 | 172.89 | 233.23 |
| **uPAR** | 1,055.13 | 631.49 | 668.56 | 960.95 |
| **VEGF** | 418.13 | 293.95 | 290.79 | 452.44 |
| **VEGF-D** | 39.13 | 0.89 | 20.85 | 12.46 |
| **POS** | 17,605.50 | 17,898.76 | 18,624.45 | 19,358.48 |
| **NEG** | 1.00 | 1.04 | 1.02 | 1.09 |
| **Activin A** | 7.00 | 106.51 | 131.02 | 216.38 |
| **ALCAM** | 227.00 | 372.00 | 229.19 | 194.01 |
| **B7-1(CD80)** | 18.00 | 138.72 | 100.34 | 3.55 |
| **BMP-5** | 42.50 | 129.37 | 90.63 | 42.29 |
| **BMP-7** | 72.00 | 136.12 | 111.59 | 1.09 |
| **Cardiotrophin-1** | 578.00 | 1,221.99 | 498.65 | 233.30 |
| **CD14** | 118.00 | 231.72 | 134.09 | 180.37 |
| **CXCL-16** | 1.00 | 187.56 | 201.07 | 175.45 |
| **DR6 (TNFRSF21)** | 107.00 | 382.39 | 190.84 | 1.09 |
| **Endoglin** | 1,213.50 | 735.17 | 1,168.45 | 1,359.16 |
| **ErbB3** | 183.50 | 269.13 | 226.63 | 167.81 |
| **E-Selectin** | 150.00 | 250.94 | 178.06 | 82.68 |
| **Fas Ligand** | 134.00 | 269.65 | 162.72 | 88.14 |
| **ICAM-2** | 364.50 | 416.16 | 273.16 | 203.83 |
| **IGF-II** | 172.00 | 206.26 | 140.22 | 89.77 |
| **IL-1R II** | 178.00 | 226.52 | 190.33 | 124.70 |
| **IL-10 Rbeta** | 124.50 | 111.18 | 91.14 | 194.56 |
| **IL-13 Ralpha2** | 94.50 | 117.94 | 80.40 | 1.09 |
| **IL-18 BPalpha** | 3.00 | 132.49 | 234.82 | 1.09 |
| **IL-18 Rbeta** | 59.50 | 191.71 | 112.61 | 1.09 |
| **IL-2 Ralpha** | 128.00 | 156.90 | 149.94 | 50.48 |
| **IL-2 Rbeta** | 1.00 | 57.15 | 196.98 | 1.09 |
| **IL-2 Rgamma** | 1.00 | 1.04 | 1.02 | 1.09 |
| **IL-21R** | 153.50 | 166.78 | 146.36 | 70.67 |
| **IL-5 Ralpha** | 133.00 | 166.78 | 150.96 | 21.01 |
| **IL-9** | 169.00 | 184.96 | 155.56 | 57.58 |
| **IP-10** | 91.50 | 211.46 | 161.19 | 94.14 |
| **LAP** | 3,066.00 | 2,460.08 | 5,775.28 | 6,813.80 |
| **Leptin** R | 225.50 | 223.41 | 168.35 | 230.03 |
| **LIF** | 93.50 | 181.84 | 495.07 | 217.48 |
| **L-Selectin** | 87.50 | 103.91 | 122.84 | 1.09 |
| **M-CSF R** | 1.00 | 193.27 | 65.57 | 167.27 |
| **MMP-1** | 206.50 | 170.93 | 135.11 | 379.56 |
| **MMP-13** | 158.50 | 149.11 | 119.26 | 57.58 |
| **MMP-9** | 1.00 | 137.16 | 56.37 | 167.81 |
| **MPIF-1** | 1.00 | 291.47 | 1.02 | 1.09 |
| **NGF R** | 1.00 | 123.13 | 388.21 | 1.09 |
| **PDGF AA** | 331.50 | 311.21 | 470.01 | 493.62 |
| **PDGF-AB** | 48.50 | 111.70 | 137.67 | 248.58 |
| **PDGF Ralpha** | 1.00 | 184.96 | 174.48 | 1.09 |
| **PDGF Rbeta** | 1,199.50 | 859.86 | 3,513.79 | 4,030.00 |
| **PECAM-1** | 41.50 | 138.72 | 280.32 | 190.74 |
| **Prolactin** | 285.00 | 272.77 | 195.44 | 59.76 |
| **SCF R** | 64.50 | 245.75 | 128.46 | 88.68 |
| **SDF-1beta** | 425.50 | 364.73 | 248.11 | 452.14 |
| **Siglec-5** | 152.50 | 153.79 | 1.02 | 1.09 |
| **TGF-alpha** | 69.50 | 227.04 | 140.74 | 166.72 |
| **TGF beta2** | 173.50 | 138.72 | 182.15 | 118.70 |
| **Tie-1** | 8.50 | 124.69 | 373.89 | 101.78 |
| **Tie-2** | 153.00 | 90.92 | 231.24 | 18.28 |
| **TIMP-4** | 49.50 | 97.68 | 93.70 | 17.74 |
| **VE-Cadherin** | 77.00 | 139.24 | 131.02 | 85.41 |
| **VEGF R2** | 59.50 | 164.70 | 101.37 | 78.86 |
| **VEGF R3** | 37.50 | 121.06 | 222.54 | 54.85 |

**TABLE XI: PROTEINS CAPABLE OF DETECTION BY ANTIBODY ARRAYS**

| **Cytokine** | **Full Name** |
|---|---|
| **Activin A** | EDF(erythroid differentiation factor), FRP(Follicle stimulating hormone releasing protein), Restrictin-P, WEHI-MIF(WEHI mesoderm inducing factor) |
| **AGRP** | Agouti related protein |
| **ALCAM** | Activated leukocyte cell adhesion molecule |
| **Amphiregulin** | Amphiregulin |
| **ANG** | Angiogenin |
| **Angiopoietin-1** | Angiopoietin-1 |
| **Angiopoietin-like** Factor | Angiopoietin-like Factor |
| **Angiostatin** | Angiostatin |
| **APRIL** | A proliferation-inducing ligand |
| **AXL** | A protein tyrosine kinase also called UFO or ark. The ligand for this receptor is GPF (growth-potentiating factor ). The ligand is called also axl stimulatory factor,which is identical with one of the gas genes , gas-6 |
| **B7-1(CD80)** | B7-1(CD80) |
| **BAFF** | B-cell-activating factor belonging to the TNF family |
| **BDNF** | Brain-derived neurotrophic factor |
| **Betacellulin** | Betacellulin |
| **beta-Galactosidase** | beta-Galactosidase |
| **beta-NGF** | Nerve growth factor-beta |
| **bFGF** | Basic fibroblast growth factor |
| **BLC** | B-lymphocyte chemoattractant |
| **BMP-2** | Bone morphogenetic proteins -2 |
| **BMP-4** | Bone morphogenetic proteins -4 |
| **BMP-5** | Bone morphogenetic proteins -5 |
| **BMP-6** | Bone morphogenetic proteins -6 |
| **BMP-7** | Bone morphogenetic proteins -7 |
| **BTC** | Betacellulin |
| **CCL28** | CCK-1 |
| **CD27** | Cluster of Differentiation 27 |
| **CD30** | Cluster of Differentiation 30 |
| **CD40** | Cluster of Differentiation 40 |
| **CD40 Ligand** | Cluster of Differentiation 40 ligand |
| **Ck beta 8-1** | Chemokine-beta-8 |
| **CNTF** | Ciliary neuronotrophic factor, ciliary neurotrophic factor |
| **Cripto-1** | CRGF (Cripto growth factor) |
| **CTACK** | CTAC (Cuteaneous T-Cell Attracting Chemokine); Skinkine; Eskine |
| **CTLA-4** | Cytotoxic T-lymphocyte associated antigen 4 |
| **CXCL16** | CXC Chemokine ligand 16 |
| **Dkk-4** | Dickkopf-4 |
| **DR6** | Death Receptor 6 |
| **Dtk** | Developmental tyrosine kinase |
| **EGF** | Epidermal growth factor |
| **EGR-R** | Epidermal growth factor related protein |
| **ENA-78** | Epithelial neutrophil-activating protein 78, epithelial cellderived neutrophil attractant-78 |
| **Endostatin** | Endostatin |
| **Eotaxin** | Eotaxin |
| **Eotaxin-2** | MPIF-2 (Myeloid progenitor inhibitory factor-2 ),Chemokine-beta-6 |
| **Eotaxin-3** | MIP-4-alpha (macrophage inflammatory protein-4-alpha ), TSC-1 (thymic stroma chemokine-1) |
| **Erythropoietin** | Erythropoietin |
| **E-Selectin** | E-Selectin |
| **Fas Ligand** | FasL, CD95 ligand, Apo-1 ligand |
| **Fas/TNFRSF6** | **Fas or cd95** |
| **FGF-2** | Fibroblast growth factor-2 |
| **FGF-4** | Fibroblast growth factor-4 |
| **FGF-6** | Fibroblast growth factor-6 |
| **FGF-7** | Fibroblast growth factor-7 |
| **FGF-8** | Fibroblast growth factor-8 |
| **FGF-9** | Fibroblast growth factor-9 |
| **FKN or FK** | **Fractalkine** |
| **Flt-3 Ligand** | fms-like tyrosine kinase-3 Ligand (also known as STK-1 Ligand) |
| **Follistatin** | Follicle stimulating hormone suppressing protein (FSP) |
| **GCP-2** | Granulocyte Chemotactic Protein 2 |
| **GCSF** | Granulocyte-colony Stimulating Factor |
| **GDF-15** | growth/differentiation factor-15 |
| **GDNF** | Glial-derived Neurotrophic Factor |
| **GITR** | Glucocorticoid induced tumor necrosis factor receptor |
| **GITR-Ligand** | Glucocorticoid induced tumor necrosis factor receptor |
| **GM-CSF** | Granulocyte-macrophage colony stimulating factor |
| **GRO** | Growth Related Oncogene |
| | |
| **GRO-a** | Growth Related Oncogene-Alpha |
| **HB-EGF** | Heparin-binding Epidermal Growth factor |
| **HCC-4** | Hemofiltrate CC Chemokine 4 |
| **HGF** | Hepatocyte growth factor |
| **HPTA** | Hepatopoeitin A |
| **HRG** | Heregulin/NDF/GGF/Neuregulin |
| **HRG-alpha** | Heregulin/NDF/GGF/Neuregulin |
| **HRG-beta** | Heregulin/NDF/GGF/Neuregulin |
| **HVEM** | Herpesvirus entry mediator |
| **I-309** | I-309 |
| **ICAM-1** | Intercellular Adhesion Molecule 1 |
| **ICAM-2** | Intercellular Adhesion Molecule 2 |
| **ICAM-3** | Intercellular Adhesion Molecule 3 |
| **IFN-alpha** | Interferon alpha |
| **IFN-beta** | Interferon beta |
| **IFN-gamma** | Interferon gamma |
| **IFN-omega** | Interferon omega |
| **IGFBP-1** | Insulin-like growth factor binding proteins 1 |
| **IGFBP-2** | Insulin-like growth factor binding proteins 2 |
| **IGFBP-3** | Insulin-like growth factor binding proteins 3 |
| **IGFBP-4** | Insulin-like growth factor binding proteins 4 |
| **IGFBP-6** | Insulin-like growth factor binding proteins 6 |
| **IGF-I SR** | Insulin like growth factor I soluble receptor |
| **IGF-I** | Insulin-like growth factor-1 |
| **IGF-II** | Insulin-like growth factor II |
| **IL-1 R1** | Interleukin 1 receptor-related protein-1 |
| **IL-1 R4/ST2** | Interleukin 1 receptor 4, also known as ST2 |
| **IL-10** | Interleukin 10 |
| **IL-10 R alpha** | Interleukin 10 soluble receptor alpha |
| **IL-10 R beta** | Interleukin 10 receptor beta |
| **IL-11** | Interleukin 11 |
| **IL-12** | Interleukin 12 |
| **IL-12 p40** | Interleukin 12p40 |
| **IL12p70** | Interleukin 12p70 |
| **IL-13** | Interleukin 13 |
| **IL-15** | Interleukin 15 |
| **IL-16** | Interleukin 16 |
| **IL-17** | Interleukin 17 |
| **IL-18 BPa** | Interleukin 18 binding protein A |
| **IL-18 R alpha** | Interleukin 18 receptor alpha |
| **IL-18 R beta** | Interleukin 18 receptor beta |
| **IL-18** | Interleukin 18 |
| **IL-1a** | Interleukin I alpha |
| **IL-1b** | Interleukin I beta |
| **IL-1ra** | Interleukin 1 receptor antagonist |
| **IL-2** | Interleukin 2 |
| **IL-2 R alpha** | Interleukin 2 soluble receptor alpha |
| **IL-2 R beta** | Interleukin 2 soluble receptor beta |
| **IL-2 R gamma** | Interleukin 2 receptor gamma |
| **IL-21 R** | Interleukin 21 receptor |
| **IL-3** | Interleukin 3 |
| **IL-4** | Interleukin 4 |
| **IL-5** | Interleukin 5 |
| **IL-5 R alpha** | Interleukin 5 receptor alpha |
| **IL-6** | Interleukin 6 |
| **IL-7** | Interleukin 7 |
| **IL-8** | Interleukin 8 |
| **IL-9** | Interleukin 9 |
| **IL-9 R** | Interleukin 9 receptor |
| **Inhibin A** | Inhibin A |
| **Inhibin B** | Inhibin B |
| **IP-10** | IFN - inducible Protein 10, Immune Protein 10 |
| **I-TAC** | Interferon-inducible T cell Alpha Chemoattractant |
| **LAP** | Liver activator protein |
| **TGF-LAP** | Tumor growth factor - latency associated peptide |
| **LT-BP-1** | Latent TGF-beta binding protein 1 |
| **LECT2** | Leukocyte cell-derived chemotaxin-2 |
| **LEPTIN R** | LEPTIN receptor |
| **LFA-1 alpha** | Lymphocyte function-associated antigen 1 alpha |
| **LIF** | Leukemia Inhibitoty Factor |
| **LIGHT** | LIGHT |
| **LIX** | LPS induced C-X-C chemokine |
| **L-Selectin** | L-Selectin |
| **Lymphotactin** | Lymphotactin |
| **MAC-1** | Macrophage galactose-specific lectin-1 |
| **MMP-13** | Matrix Metalloproteinase 13 |
| **MMP-19** | Matrix Metalloproteinase 19 |
| **MCP-1** | Monocyte Chemoattractant Protein 1 |
| **MCP-2** | Monocyte Chemoattractant Protein 2 |
| **MCP-3** | Monocyte Chemoattractant Protein 3 |
| **MCP-4** | Monocyte Chemoattractant Protein 4 |
| **MCSF** | Macrophage-colony Stimuating Factor |
| **M-CSF R** | Macrophage colony stimulating factor receptor |
| **MDC** | Macrophage-derived Chemokine |
| **MIF** | Macrophage Migration Inhibitory Factor |
| **MIG** | Monokine induced by gamma interferon |
| **MIP-1a** | Macrophage Inflammatory Protein 1 alpha |
| **MIP-1b** | Macrophage Inflammatory Protein 1 beta |
| **MIP-1d** | Macrophage Inflammatory Protein 1delta |
| **MIP-2** | Macrophage Inflammatory Protein 2 |
| **MIP-3 alpha** | Macrophage Inflammatory Protein 3 alpha |
| **MIP-3 beta** | Macrophage Inflammatory Protein 3 beta |
| **MMP-1** | Matrix Metalloproteinase 1 |
| **MMP-10** | Matrix Metalloproteinase 10 |
| **MMP-11 (Stromelysin-3)** | Matrix Metalloproteinase 11 |
| **MMP-12** | Matrix Metalloproteinase 12 |
| **MMP-13** | Matrix Metalloproteinase 13 |
| **MMP-14** | Matrix Metalloproteinase 14 |
| **MMP-2** | Matrix Metalloproteinase 2 |
| **MMP-20** | Matrix Metalloproteinase 20 |
| **MMP-3** | Matrix metalloproteinase 3 |
| **MMP-7** | Matrix Metalloproteinase 7 |
| **MMP-8** | Matrix Metalloproteinase 8 |
| **MMP-9** | Matrix Metalloproteinase 9 |
| **MPIF-1** | Myeloid Progenitor Inhibitory Factor 1 |
| **MSP a-chain** | Macrophage stimulating protein a-chain |
| **MSP b-chain** | Macrophage stimulating protein b-chain |
| **NAP-2** | Neutrophil Activating Peptide 2 |
| **NGF** | Nerve growth factor |
| **NGF R** | Nerve growth factor receptor |
| **NT-3** | Neurotrophin-4 |
| **NT-4** | Neurotrophin-3 |
| **OPG** | Osteoprotegerin |
| **OSM** | Oncostatin M |
| **PARC** | Pulmonary and Activation-Regulated Chemokine |
| **PDGF R alpha** | Platelet-derived growth factor receptor alpha |
| | |
| **PDGF R beta** | Platelet-derived growth factor receptor beta |
| **PDGF-AA** | Platelet-derived Growth Factor AA |
| **PDGF-BB** | Platelet-derived Growth Factor BB |
| **PECAM-1** | Platelet endothelial cell adhesion molecule |
| **PF4** | Platelet factor 4 |
| **PIGF** | Placenta growth Factor |
| **Prolactin** | Prolactin |
| **P-selectin** | P-selectin |
| **RANTES** | Regulated upon activation, normal T-cell expressed, and presumably secreted |
| **SCF** | Stem Call Factor |
| **SCF R** | Stem cell factor receptor |
| **SDF-1** | Stromal cell-derived factor |
| **SLPI** | Secretory leukocyte protease inhibitor |
| **SMDF** | Sensory and Motor Neuron-derived Factor |
| **SPARC** | Osteonectin |
| **ST2** | Interleukin 1 receptor 4 |
| **TARC** | Thymus and Activation-Regulated Chemokine |
| **TECK** | Thymus-expressed Chemokine |
| **TGFa** | Tumor Necrosis Factor Alpha |
| **TGF-a** | Transforming growth factor alpha |
| **TGF-beta 1** | Tumor Necrosis Facyor beta1 |
| **TGF-beta 2** | Tumor Necrosis Facyor beta2 |
| **TGF-beta 3** | Tumor Necrosis Facyor beta3 |
| **Thrombospondin (TSP)** | Thrombospondin |
| **Thymopoietin** | Thymopoietin (Tpo) |
| **Tie-1** | Tyrosine kinase with Ig and EGF homology domains 1 |
| **Tie-2** | Tyrosine kinase with immunoglobulin and epidermal growth factor homology domain 2 |
| **TIMP-1** | Tissue inhibitor of metalloproteinases-1 |
| **TIMP-2** | Tissue inhibitor of metalloproteinases-2 |
| **TIMP-3** | Tissue inhibitor of metalloproteinase-3 |
| **TIMP-3** | Tissue inhibitor of metalloproteinases-3 |
| **TIMP-4** | Tissue inhibitor of metalloproteinase-4 |
| **TNF-a** | Tumor necrosis factor-alpha |
| **TNF-b** | Tumor necrosis factor-beta |
| **TPO** | Thrombopoietin |
| **TRAIL R1** | TNF-related apoptosis-inducing ligand receptor 1 |
| **TRAIL R2** | TNF-related apoptosis-inducing ligand receptor 2 |
| **TRAIL** | TNF-related apoptosis inducing ligand |
| **TRANCE** | Tumor necrosis factor-related activation-induced cytokine |
| **TSLP** | Thymic stromal derived lymphopoietin |
| **uPA** | Urokinase plasminogen activator Ab |
| **uPAR** | Urokinase plasminogen activator receptor |
| **VCAM-1** | Vascular cell adhesion molecule (VCAM-1, CD106, or INCAM-110) |
| **VE-Cadherin** | Vascular epithelial cadherin |
| **VEGF** | Vascular Endothelial Growth Factor |
| **VEGF R2** | Vascular endothelial growth factor receptor 2 |
| **VEGF R3** | Vascular endothelial growth factor receptor 3 |
| **VEGF-B** | Vascular Endothelial Growth Factor B |
| **VEGF-C** | Vascular Endothelial Growth Factor C |
| **VEGF-D** | Vascular Endothelial Growth Factor D |

Publications cited throughout this document are hereby incorporated by reference in their entirety. Although the various aspects of the invention have been illustrated above by reference to examples and preferred embodiments, it will be appreciated that the scope of the invention is defined not by the foregoing description, but by the following claims properly construed under principles of patent law.

Embodiments disclosed herein:
1. A substantially pure population of amniotic fluid-derived cells.
2. The population of amniotic fluid-derived cells according to embodiment 1, wherein the cells of said population are substantially negative in the expression of at least one of the CD117, Oct-4 and Tra2-54 protein markers.
3. The population of amniotic fluid-derived cells according to embodiment 1 or embodiment 2, wherein the cells of said population are substantially positive for the expression of at least one of the HNF-1 beta, GATA-6 and Sox-17 protein markers.
4. The population of amniotic fluid-derived cells according to any one of embodiments 1 to 3, wherein the cells of said population also express at least one of the HNF-3 beta, Hes-1, GATA-4 and Musashi-1 genes.
5. The population of amniotic fluid-derived cells according to any one of embodiments 1 to 4, wherein the cells of said population are positive for the expression of at least one of the Liver Activator Protein, PDGF receptor β, AXL, bFGF, EGF receptor, Fas /TNFRSF6, GRO, GRO-alpha, ICAM-1, IL-lalpha, Il-3, Il-6, Il-8, MIF, Osteoprotegerin, TIMP-2 and TRAIL R3 protein markers.
6. The population of amniotic fluid-derived cells according to any one of embodiments 1 to 6, wherein the cells of said population are substantially negative in the expression of the protein marker Sox-17.
7. The population of amniotic fluid-derived cells according to embodiment 6, wherein the cells of said population are substantially negative in the expression of cytokeratin protein.
8. The population of amniotic fluid-derived cells according to embodiment 6 or embodiment 7, wherein the cells of said population are positive for the expression of at least one of the Liver Activator Protein, PDGF receptor β, bFGF, EGF receptor, GRO, GRO-alpha, ICAM-1, Il-3, Il-6, MIF, Osteoprotegerin, TIMP-2, and TRAIL R3 protein markers.
9. The population of amniotic fluid-derived cells according to any one of embodiments 1 to 9, wherein the cells of said population are substantially negative in the expression of the protein marker Sox-17 and also express at least one of the Hes-1 and Musashi-1 genes.
10. The population of amniotic fluid-derived cells according to any one of embodiments 1 to 9, wherein the cells of said population are substantially negative in the expression of CD117, Oct-4, Sox-17 and Tra2-54.
11. The population of pancreatic amniotic fluid-derived cells according to embodiment 1, capable of propagating *in vitro.*
12. The population of amniotic fluid-derived cells according to any one of embodiments 1 to 11, capable of propagating *in vitro* under hypoxic conditions.
13. The population of amniotic fluid-derived cells according to any one of embodiments 1 to 12, capable of differentiating into cells displaying the characteristics of the β-cell lineage.
14. The population of amniotic fluid-derived cells according to any one of embodiments 1 to 12, capable of differentiating into a gut hormone-producing cell.
15. A method of obtaining a population of cells from amniotic fluid, comprising:
   a. Isolating amniotic fluid,
   b. Isolating the cells from the amniotic fluid,
   c. Placing the cells in culture medium,
   d. Plating the cells in a culture vessel, and
   e. Allowing the cells to grow in said medium for at least about five days, thereby obtaining a population of amniotic fluid-derived cells.
16. The method according to embodiment 15, wherein the cells of said population are as characterised in any one of embodiments 1 to 14.
17. A method of obtaining a population of cells from amniotic fluid, comprising:
   a. Isolating amniotic fluid,
   b. Isolating the cells from the amniotic fluid,
   c. Selecting cells that express at least one of the SSEA-4, SSEA-3, TRA1-60 and TRA1-81 markers,
   d. Placing the cells in culture medium,
   e. Plating the cells in a culture vessel, and
   f. Allowing the cells to grow in said medium for at least about five days, thereby obtaining a population of amniotic fluid-derived cells.
18. The method according to embodiment 17, wherein the cells of said population are as characterised in any one of embodiments 1 to 14.
19. A population of amniotic fluid-derived cells as defined in any one of embodiments 1 to 14 or as obtained by the method of any one of embodiments 15 to 18, for use in treating a patient with diabetes mellitus or at risk of developing diabetes.
20. The cells for use according to embodiment 19, wherein the cells of said population are differentiated into pancreatic hormone producing cells.

## Claims

1. A substantially pure population of amniotic fluid-derived cells.

2. The population of amniotic fluid-derived cells according to claim 1, wherein
the cells of said population are
negative in the expression of at least one of the CD117, Oct-4 and Tra2-54 protein markers;
positive for the expression of at least one of the HNF-1 beta, GATA-6 and Sox-17 protein markers;
positive for the expression of at least one of the HNF-3 beta, Hes-1, GATA-4 and Musashi-1 genes;
positive for the expression of at least one of the Liver Activator Protein, PDGF receptor, AXL, bFGF, EGF receptor, Fas/TNFRSF6, GRO, GRO-alpha, ICAM-1, IL-lalpha, Il-3, Il-6, Il-8, MIF, Osteoprotegerin, TIMP-2 and TRAIL R3 protein markers; and/or
negative in the expression of the protein marker Sox-17.

3. The population of amniotic fluid-derived cells according to claim 2, wherein the cells of said population are substantially negative in the expression of the protein marker Sox-17 and of the cytokeratin protein.

4. The population of amniotic fluid-derived cells according to claim 3, wherein the cells of said population are positive for the expression of at least one of the Liver Activator Protein, PDGF receptor, bFGF, EGF receptor, GRO, GRO-alpha, ICAM-1, Il-3, Il-6, MIF, Osteoprotegerin, TIMP-2, and TRAIL R3 protein markers.

5. The population of amniotic fluid-derived cells according to any one of claims 1 to 4, wherein the cells of said population are substantially negative in the expression of the protein marker Sox-17 and also express at least one of the Hes-1 and Musashi-1 genes.

6. The population of amniotic fluid-derived cells according to any one of claims 1 to 9, wherein the cells of said population are substantially negative in the expression of CD117, Oct-4, Sox-17 and Tra2-54.

7. The population of amniotic fluid-derived cells according to any of the preceding claims, capable of propagating in vitro.

8. The population of amniotic fluid-derived cells according to any of the preceding claims, capable of propagating in vitro under hypoxic conditions.

9. The population of amniotic fluid-derived cells according to any one of claims 1 to 12, capable of differentiating into cells displaying the characteristics of the β-cell lineage.

10. The population of amniotic fluid-derived cells according to any of the preceding claims, capable of differentiating into a gut hormone-producing cell.

11. An *in vitro* method of obtaining a population of cells from isolated amniotic fluid, comprising:
a. Isolating the cells from the amniotic fluid,
b. Placing the cells in culture medium,
c. Plating the cells in a culture vessel, and
d. Allowing the cells to grow in said medium for at least about five days, thereby obtaining a population of amniotic fluid-derived cells.

12. An *in vitro* method according to claim 11, comprising selecting cells that express at least one of the SSEA-4, SSEA-3, TRA1-60 and TRA1-81 markers, before step (b) of claim 11.

13. The method according to claims 11 or 12, wherein the cells of said population are as **characterised in** any one of claims 1 to 10.

14. A population of amniotic fluid-derived cells as defined in any one of claims 1 to 9 or 13, or as obtained by the method of any one of claims 11 or 12, for use in treating a patient with diabetes mellitus or at risk of developing diabetes.

15. The cells for use according to claim 14, wherein the cells of said population are differentiated into pancreatic hormone producing cells.


SEQUENCE LISTING

<110>  Lifescan Inc.

<120>  Amniotic Fluid Derived Cells

<130>  P055528EP

<150>  US60/685,607
<151>  2005-05-27

<150>  US60/743,821
<151>  2006-03-27

<160>  11

<170>  PatentIn version 3.3

<210>  1
<211>  20
<212>  DNA
<213>  Homo sapiens

<400>  1
cgaccatctg ccgctttgag                                                  20


<210>  2
<211>  20
<212>  DNA
<213>  Homo sapiens

<400>  2
ccccctgtcc cccattccta                                                  20


<210>  3
<211>  24
<212>  DNA
<213>  Homo sapiens

<400>  3
cagatcctaa acagctcgca gaat                                             24


<210>  4
<211>  23
<212>  DNA
<213>  Homo sapiens

<400>  4
gcgtacgcaa attaaactcc aga                                              23


<210>  5
<211>  17
<212>  DNA
<213>  Homo sapiens

<400>  5
tggcgcagca gatacca                                                     17

<210>  6
<211>  18
<212>  DNA
<213>  Homo sapiens

<400>  6
agcgccttcc acgacttg                                                    18


<210>  7
<211>  23
<212>  DNA
<213>  Homo sapiens

<400>  7
ccagcatctt gctcaactcg gcg                                              23


<210>  8
<211>  21
<212>  DNA
<213>  Homo sapiens

<400>  8
gtttatccgt ggtgtgtctg g                                                21


<210>  9
<211>  20
<212>  DNA
<213>  Homo sapiens

<400>  9
ctgagctata gaggcctggg                                                  20


<210>  10
<211>  19
<212>  DNA
<213>  Homo sapiens

<400>  10
atgcaccgct acgacgtga                                                   19


<210>  11
<211>  20
<212>  DNA
<213>  Homo sapiens

<400>  11
cttttgcacc cctcccattt                                                  20